(19) Europäisches Patentamt / European Patent Office / Office européen des brevets

(11) **EP 3 452 937 B1**

(12) **EUROPEAN PATENT SPECIFICATION**

(45) Date of publication and mention
of the grant of the patent:
**22.10.2025 Bulletin 2025/43**

(21) Application number: **17720140.7**

(22) Date of filing: **28.04.2017**

(51) International Patent Classification (IPC):
*G16B 20/10* (2019.01)   *G16B 20/20* (2019.01)
*G16B 20/40* (2019.01)   *G16B 25/10* (2019.01)
*G16B 40/30* (2019.01)   *C12Q 1/6809* (2018.01)

(52) Cooperative Patent Classification (CPC):
(C-Sets available)
**G16B 25/10; C12Q 1/6809; G16B 20/00;
G16B 20/10; G16B 20/20; G16B 20/40;
G16B 40/30**                    (Cont.)

(86) International application number:
**PCT/EP2017/060298**

(87) International publication number:
**WO 2017/191076 (09.11.2017 Gazette 2017/45)**

(54) **METHOD OF CHARACTERISING A DNA SAMPLE**

VERFAHREN ZUR CHARAKTERISIERUNG EINER DNA-PROBE

PROCÉDÉ DE CARACTÉRISATION D'UN ÉCHANTILLON D'ADN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.05.2016 GB 201607635
10.03.2017 GB 201703903**

(43) Date of publication of application:
**13.03.2019 Bulletin 2019/11**

(73) Proprietor: **Genome Research Limited
Cambridge, Cambridgeshire CB10 1SA (GB)**

(72) Inventors:
 • **NIK-ZAINAL, Serena
  Cambridge
  Cambridgeshire CB10 1SA (GB)**
 • **DAVIES, Helen
  Cambridge
  Cambridgeshire CB10 1SA (GB)**
 • **GLODZIK, Dominik
  Cambridge
  Cambridgeshire CB10 1SA (GB)**
 • **MORGANELLA, Sandro
  Cambridge
  Cambridgeshire CB10 1SA (GB)**

(74) Representative: **Mewburn Ellis LLP
Aurora Building
Counterslip
Bristol BS1 6BX (GB)**

(56) References cited:
**WO-A1-2013/096843**

• **DEGASPERI ANDREA ET AL: "A practical
framework and online tool for mutational
signature analyses show intertissue variation
and driver dependencies", NATURE CANCER,
vol. 1, no. 2, 14 February 2020 (2020-02-14),
pages 249 - 263, XP055936184, Retrieved from
the Internet <URL:https://www.nature.com/
articles/s43018-020-0027-5.pdf> DOI: 10.1038/
s43018-020-0027-5**
• **HELEN DAVIES ET AL: "HRDetect is a predictor
of BRCA1 and BRCA2 deficiency based on
mutational signatures", NATURE MEDICINE, vol.
23, no. 4, 13 March 2017 (2017-03-13), New York,
pages 517 - 525, XP055386173, ISSN: 1078-8956,
DOI: 10.1038/nm.4292**

**EP 3 452 937 B1**

(Cont. next page)

- LUDMIL B. ALEXANDROV ET AL: "Deciphering Signatures of Mutational Processes Operative in Human Cancer", CELL REPORTS, vol. 3, no. 1, 1 January 2013 (2013-01-01), US, pages 246 - 259, XP055391929, ISSN: 2211-1247, DOI: 10.1016/j.celrep.2012.12.008
- ALEXANDROV, L.B: "Deciphering signatures of mutational processes from mutational catalogues of cancer genomes", 8 September 2014 (2014-09-08), XP055392072, Retrieved from the Internet <URL:ftp://ftp.sanger.ac.uk/pub/resources/theses/la2/chapter2.pdf> [retrieved on 20170719]
- KORNELIUS SCHULZE ET AL: "Exome sequencing of hepatocellular carcinomas identifies new mutational signatures and potential therapeutic targets", NATURE GENETICS., vol. 47, no. 5, 30 March 2015 (2015-03-30), NEW YORK, US, pages 505 - 511, XP055392299, ISSN: 1061-4036, DOI: 10.1038/ng.3252
- SERENA NIK-ZAINAL ET AL: "Landscape of somatic mutations in 560 breast cancer whole-genome sequences", NATURE, vol. 534, no. 7605, 2 May 2016 (2016-05-02), pages 47 - 54, XP055379838, ISSN: 0028-0836, DOI: 10.1038/nature17676

(52) Cooperative Patent Classification (CPC): (Cont.)

C-Sets
C12Q 1/6809, C12Q 2535/122, C12Q 2537/165

**Description**

*FIELD OF INVENTION*

The present invention relates to a method of characterising a DNA sample from a tumour, to determine a prognosis for the tumour, determine the suitability of a treatment for the tumour, select a patient with a tumour for a clinical trial of a cancer therapy, classify patients undergoing treatment for cancer or participating in a clinical trial, and classifying patients who have completed a clinical trial or course of treatment. It is particularly, but not exclusively, concerned with a method for characterising the properties of cancer based on a DNA sample from a tumour.

*BACKGROUND TO THE INVENTION*

The mutational theory of cancer proposes that changes in DNA sequence, termed "driver" mutations, confer proliferative advantage upon a cell, leading to outgrowth of a neoplastic clone [1]. Some driver mutations are inherited in the germline, but most arise in somatic cells during the lifetime of the cancer patient, together with many "passenger" mutations not implicated in cancer development [1]. Multiple mutational processes, including endogenous and exogenous mutagen exposures, aberrant DNA editing, replication errors and defective DNA maintenance, are responsible for generating these mutations [1-3].

Over the past five decades, several waves of technology have advanced the characterisation of mutations in cancer genomes. Karyotype analysis revealed rearranged chromosomes and copy number alterations. Subsequently, loss of heterozygosity analysis, hybridisation of cancer-derived DNA to microarrays and other approaches provided higher resolution insights into copy number changes [4-8]. Recently, DNA sequencing has enabled systematic characterisation of the full repertoire of mutation types including base substitutions, small insertions/deletions, rearrangements and copy number changes [9-13], yielding substantial insights into the mutated cancer genes and mutational processes operative in human cancer.

Alexandrov et al. (Cell Reports vol. 3, no. 1, 1 January 2013, pages 246-259) describes a computational framework to characterise mutational signatures from cancer-derived somatic mutational catalogues, by: (1) classifying single base substitutions (SBS) into six subtypes (C:G>A:T, C:G>G:C, C:G>T:A, T:A>A:T, T:A>C:G and T:A>G:C) then further based on the 5' and 3' sequence context of each mutated base; and (2) identifying mutational signatures as operative processes associated with distributions over the categories of SBS such that the mutations in each category in each of a plurality of cancer genomes can be approximately expressed as the sum of the respective category of all operative processes multiplied by their exposure in the respective cancer genome.

WO 2013/096843 A describes methods for assessing samples for the presence of a loss of heterozygosity (LOH) signature and for predicting the status of homology directed repair (HDR) in a cancer cell, by counting the number of LOH regions within "Indicator LOH regions" that are above a predetermined length but shorter than the entire length of the chromosome in which the LOH regions is located.

As for many cancer classes, most currently available breast cancer sequences target protein-coding exons [8, 11-15]. Consequently, there has been limited consideration of mutations in untranslated, intronic and intergenic regions, leaving central questions pertaining to the molecular pathogenesis of the disease unresolved. First, the role of activating driver rearrangements [16-18] forming chimeric (fusion) genes/proteins or relocating genes adjacent to new regulatory regions as observed in renal [19] and haemopoietic malignancies. Second, the role of driver substitutions and indels in non-coding regions of the genome [20, 21]. Common inherited variants conferring susceptibility to human disease are generally in non-coding regulatory regions and the possibility that similar mechanisms operate somatically in cancer was highlighted by the discovery of somatic driver substitutions in the TERT gene promoter [22, 23]. Third, which mutational processes generate the somatic mutations found in breast cancer [2, 24]. Addressing this question has been constrained because exome sequences do not inform on genome rearrangements and capture relatively few base substitution mutations, thus limiting statistical power to extract the mutational signatures imprinted on the genome by these processes [24, 25].

The present inventors have analysed the complete genome sequences of 560 cases in order to address these and other questions and to pave the way to a comprehensive understanding of the origins and consequences of somatic mutations in breast cancer.

From this analysis, it has been possible to determine certain characteristics of a cancer tumour based on the mutations found in DNA obtained from that tumour.

*STATEMENTS OF INVENTION*

The present invention provides a computer-implemented method of determining a prognosis of a tumour or determining the suitability of a treatment for a tumour, the method including characterising a DNA sample obtained from said tumour, by performing three or more of the following steps a) to e):

a) determining a catalogue of base substitution signatures which are present in the sample;
b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
c) determining a catalogue of insertion/deletion signatures which are present in the sample
d) determining the overall copy number profile in the sample
e) identifying putative driver mutations present in the sample
and

based on said determinations and identifications, constructing an interpreted profile of the tumour,
wherein the three or more steps a) to e) include at least steps a), b) and c); steps a), b) and d); steps a), b) and e); steps b), c) and d); steps b), d) and e); or steps b), c) and e); and

determining the prognosis or suitability of the treatment from the interpreted profile.

The present invention also provides a computer-implemented method of selecting a patient for a clinical trial of a cancer therapy, the method including:

characterising a DNA sample obtained from a tumour in said patient, by performing three or more of the following steps a) to e):

a) determining a catalogue of base substitution signatures which are present in the sample;
b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
c) determining a catalogue of insertion/deletion signatures which are present in the sample
d) determining the overall copy number profile in the sample
e) identifying putative driver mutations present in the sample
and

based on said determinations and identifications, constructing an interpreted profile of the tumour,
wherein the three or more steps a) to e) include at least steps a), b) and c); steps a), b) and d); steps a), b) and e); steps b), c) and d); steps b), d) and e); or steps b), c) and e); and
determining whether or not the patient is suitable for the clinical trial on the basis of the profile constructed for that patient.

The invention also provides a method of classifying a plurality of patients undergoing treatment for cancer, or participating in a clinical trial, the method including allocating patients to groups based on a profile constructed from a DNA sample obtained from a tumour in each patient by a method comprising the steps of:

characterising a DNA sample obtained from said tumour, by performing three or more of the following steps a) to e):

a) determining a catalogue of base substitution signatures which are present in the sample;
b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
c) determining a catalogue of insertion/deletion signatures which are present in the sample
d) determining the overall copy number profile in the sample
e) identifying putative driver mutations present in the sample
and

based on said determinations and identifications, constructing an interpreted profile of the tumour,
wherein the three or more steps a) to e) include at least steps a), b) and c); steps a), b) and d); steps a), b) and e); steps b), c) and d); steps b), d) and e); or steps b), c) and e).

The invention also provides a method of classifying patients who have completed a clinical trial or course of treatment, the method including:

by performing three or more of the following steps a) to e):

a) determining a catalogue of base substitution signatures which are present in the sample;
b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
c) determining a catalogue of insertion/deletion signatures which are present in the sample
d) determining the overall copy number profile in the sample
e) identifying putative driver mutations present in the sample
and

based on said determinations and identifications, constructing an interpreted profile of the tumour,
wherein the three or more steps a) to e) include at least steps a), b) and c); steps a), b) and d); steps a), b) and e); steps b), c) and d); steps b), d) and e); or steps b), c) and e); and correlating the interpreted profile obtained for each patient with the clinical outcome of the trial or treatment.

A further exemplary embodiment of the present invention provides a computer program product containing non-transitory memory storing a computer program which, when run on a computer, performs the steps of any embodiment of any method of the invention.

A further exemplary aspect of the present invention provides a computer having a processor, wherein the processor is configured to perform the steps of any embodiment of any method of the invention.

*BRIEF DESCRIPTION OF THE FIGURES*

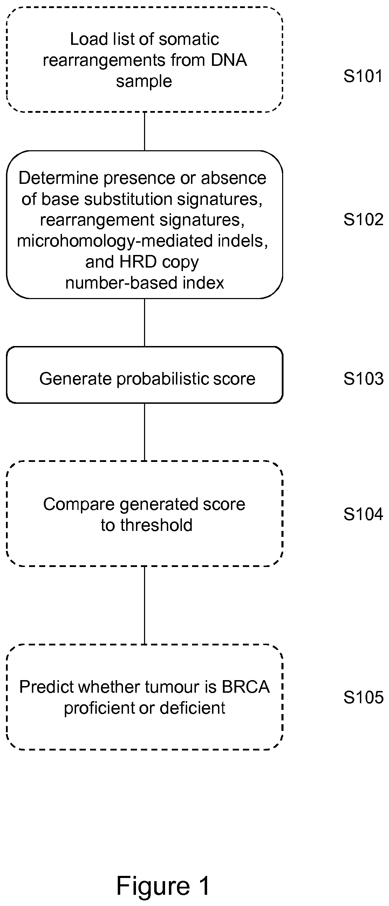

Figure 1 is a flow diagram showing, in schematic form, a method of characterising a DNA sample according to an embodiment of the present invention;

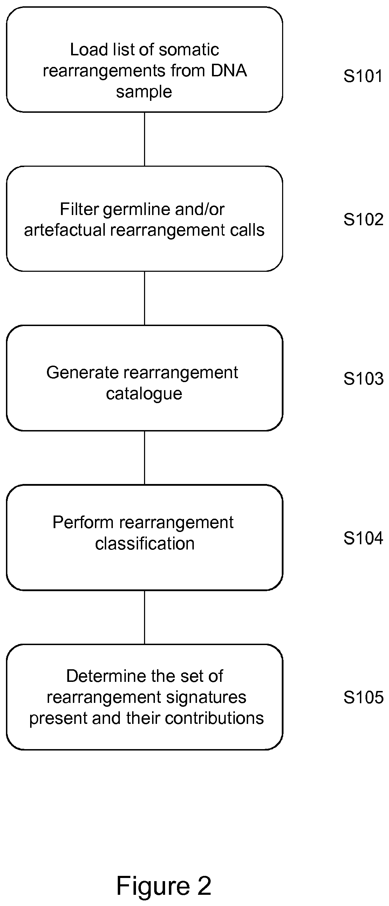

Figure 2 is a flow diagram showing, in schematic form, a method of determining the likelihood of a DNA sample being HR-deficient or not;

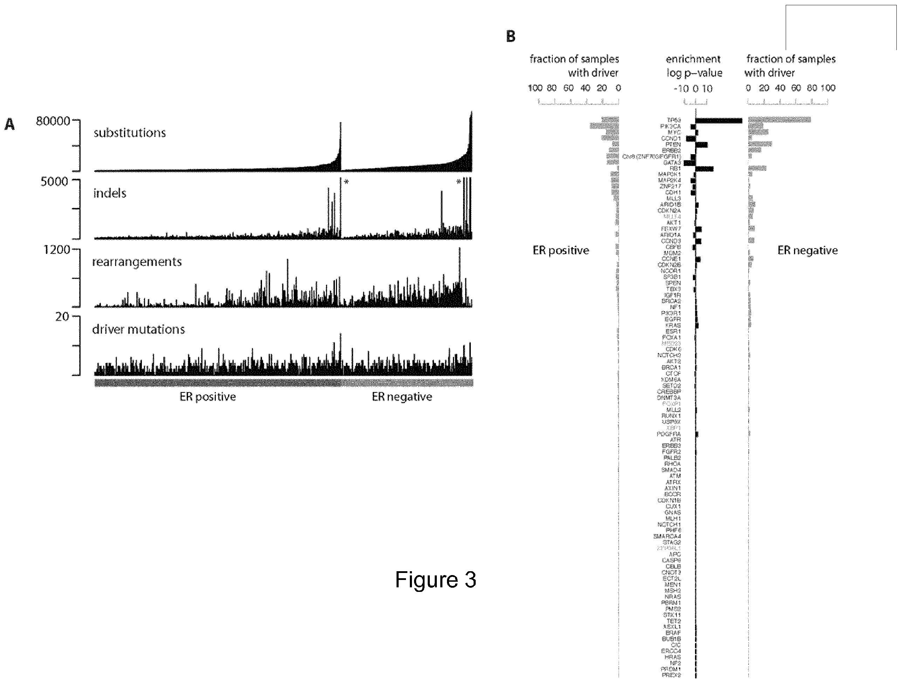

Figure 3 shows the catalogue of somatic mutations in 560 breast cancers; Figure 3A is a catalogue of base substitutions, insertions/deletions, rearrangements and driver mutations in the 560 breast cancers (sorted by total substitution burden) with the indel axis limited to 5,000(*); Figure 3B is a complete list of curated driver genes sorted by frequency (descending) and showing the fraction of ER positive (left, total 366) and ER negative (right, total 194) samples carrying a mutation in the relevant driver gene presented in grey along with the log10 p-value of enrichment of each driver gene towards the ER positive or ER negative cohort in black;

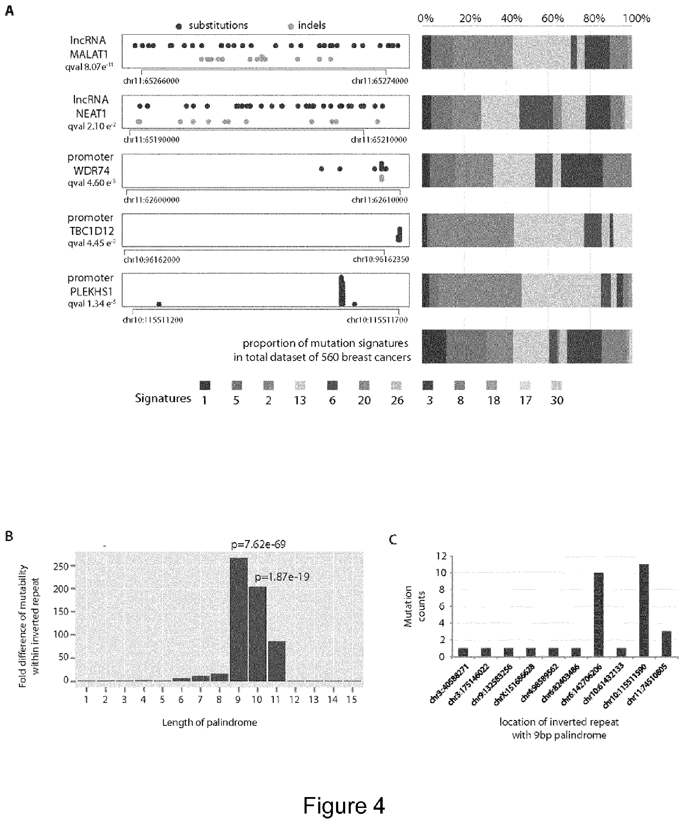

Figure 4 shows non-coding analyses of breast cancer genomes; Figure 4A shows the distributions of substitution (darker dots, top lines) and indel (lighter dots, bottom lines) mutations within the footprint of five regulatory regions identified as being more significantly mutated than expected is provided on the left, along with the proportion of base substitution mutation signatures associated with corresponding samples carrying mutations in each of these non-coding regions on the right; Figure 4B shows the mutability of TGAACA/TGTTCA motifs within inverted repeats of varying flanking palindromic sequence length compared to motifs not within an inverted repeat; Figure 4C shows the variation in mutability between loci of TGAACA/TGTTCA inverted repeats with 9bp palindromes;

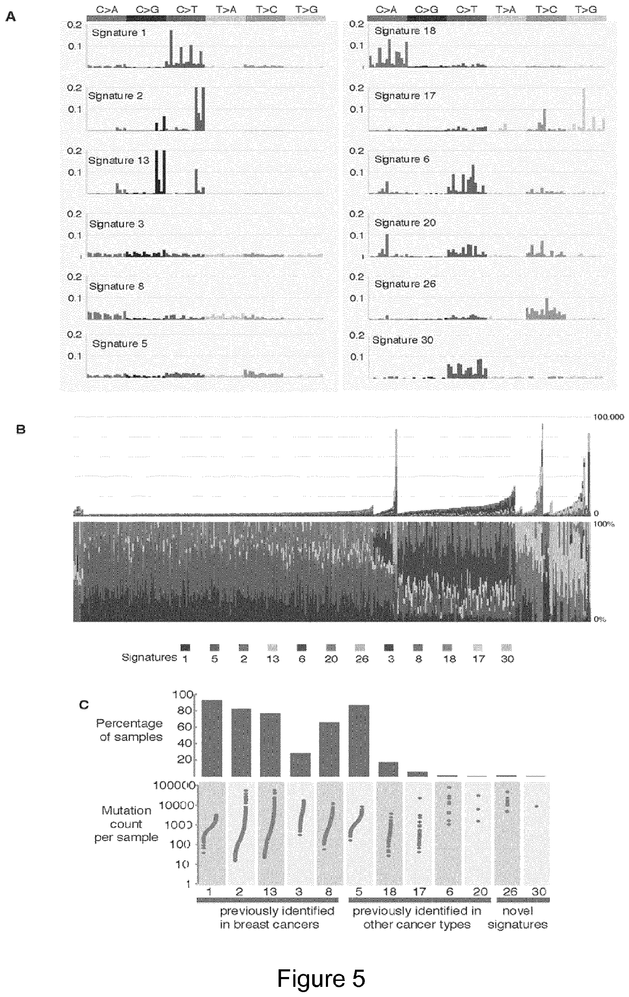

Figure 5 shows the extraction and contributions of base substitution signatures in 560 breast cancers; Figure 5A shows twelve mutation signatures extracted using Non-Negative Matrix Factorization with each signature ordered by mutation class (C>A/G>T, C>G/G>C, C>T/G>A, T>A/A>T, T>C/A>G, T>G/A>C), taking immediate flanking sequence into account. For each class, mutations are ordered by 5' base (A,C,G,T) first before 3' base (A,C,G,T); Figure 5B shows the spectrum of base substitution signatures within 560 breast cancers, the mutation signatures are ordered according to broad biological groups: Signatures 1 and 5 are correlated with age of diagnosis, Signatures 2 and 13 are putatively APOBEC-related, Signatures 6, 20 and 26 are associated with MMR deficiency, Signatures 3 and 8 are associated with HR deficiency, Signatures 18, 17 and 30 have unknown etiology; Figure 5C shows the distribution of mutation counts for each signature in relevant breast cancer samples with the percentage of samples carrying each signature provided above each signature;

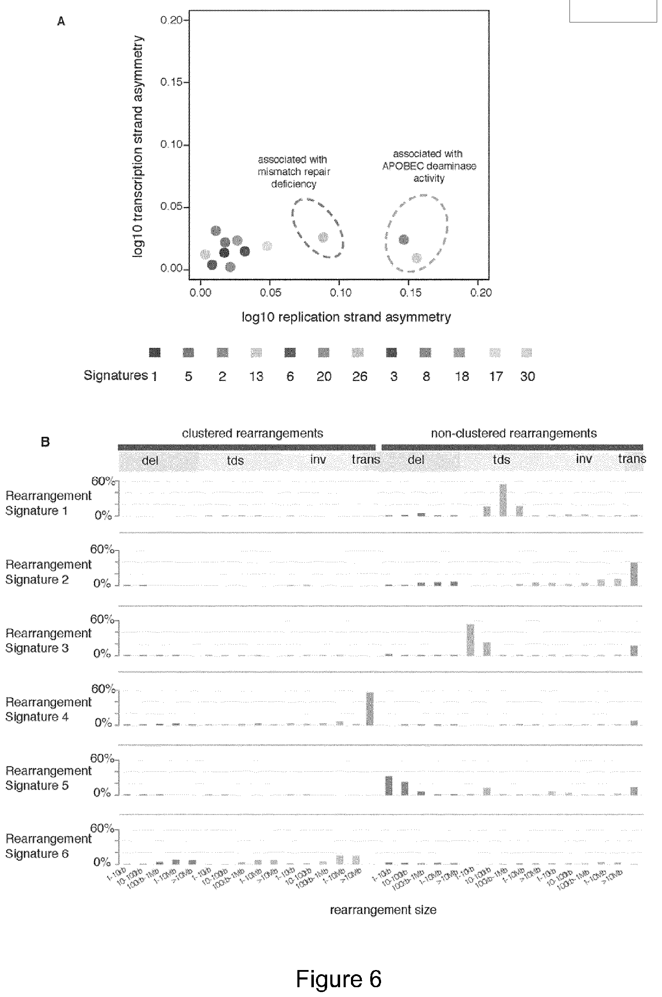

Figure 6 shows the distribution of base substitution signatures in 560 breast cancers; Figure 6A shows the contrasting transcriptional strand asymmetry and replication strand asymmetry between twelve base substitution signatures; Figure 6B shows the six rearrangement signatures extracted using Non-Negative Matrix Factorization with the probability of rearrangement element on y-axis and the rearrangement size on x-axis.

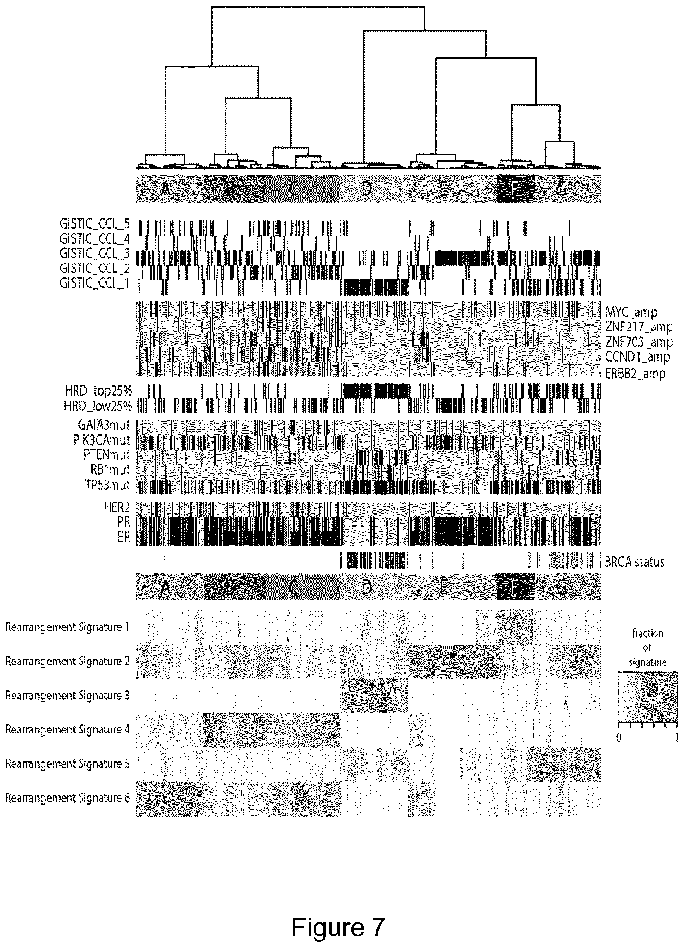

Figure 7 shows the rearrangement signatures in the 560 breast cancers as a heatmap of rearrangement signatures (RS) following unsupervised hierarchical clustering based on proportions of RS in each cancer; 7 cluster groups (A-G) were noted and relationships with expression (AIMS) subtype, immunohistopathology status (ER, PR, HER2 status - black=positive), abrogation of BRCA1 and BRCA2 (whether germline, somatic or through promoter hypermethylation), presence of 3 or more foci of kataegis (black=positive), HRD index (top 25% or lowest 25% - black=positive), GISTIC cluster group (black=positive) and driver mutations in cancer genes and miRNA cluster groups with the contribution of base substitution signatures in these 7 cluster groups provided in the lowermost panel;

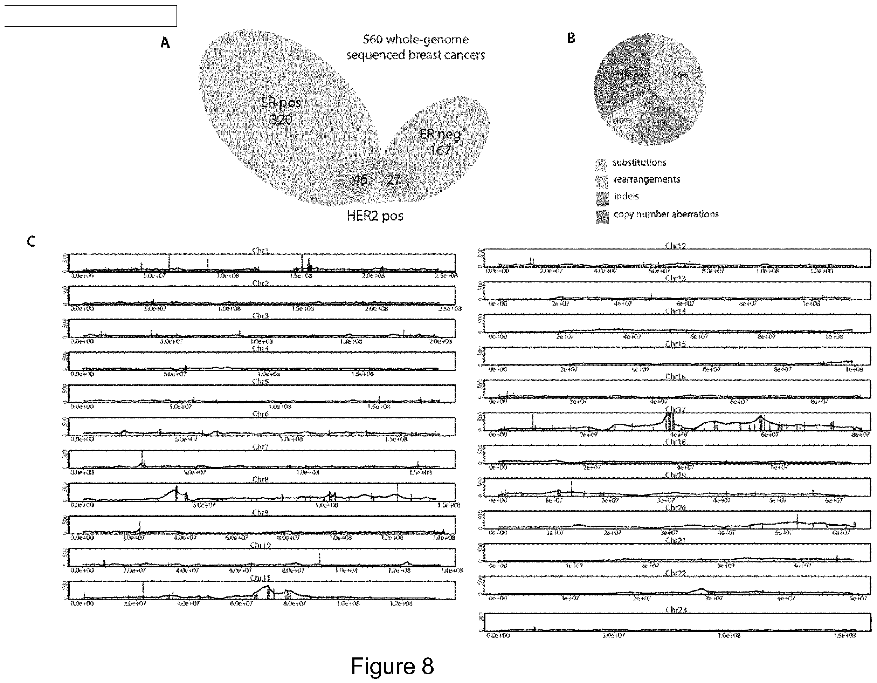

Figure 8 shows a landscape of driver mutations; Figure 8A shows the frequency of driver mutations in breast cancer genes; Figure 8B shows driver mutations by mutation type; Figure 8C shows the distribution of rearrangements throughout the genome including background rearrangement density (continuous line) based on rearrangement breakpoints (in intergenic regions only) and frequency of rearrangement within breast cancer genes (spikes);

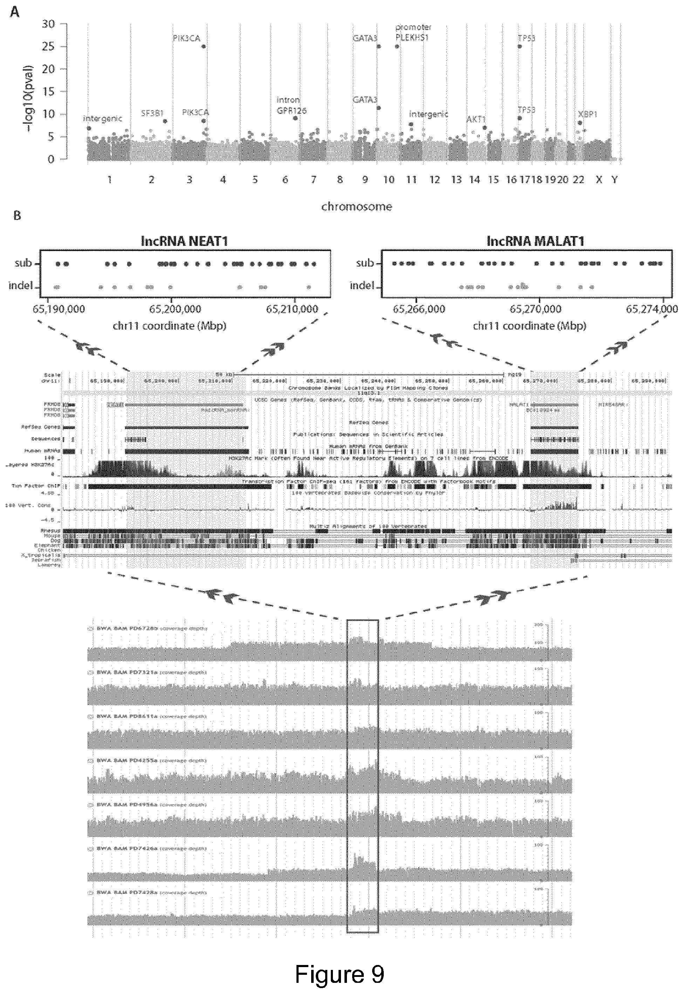

Figure 9 shows recurrent non-coding events in breast cancers; Figure 9A is a Manhattan plot demonstrating sites with most significant p-values as identified by binning analysis with highlighted sites which were also detected by the method seeking recurrence when partitioned by genomic features; Figure 9B shows the locus at chr11:65Mb which was identified by independent analyses as being more mutated than expected by chance and, in the lowermost panel, a rearrangement hotspot analysis identified this region as a tandem duplication hotspot, with nested tandem duplications noted at this site; partitioning the genome into different regulatory elements, an analysis of substitutions and indels identified lncRNAs MALAT1 and NEAT1 (topmost panels) with significant p-values;

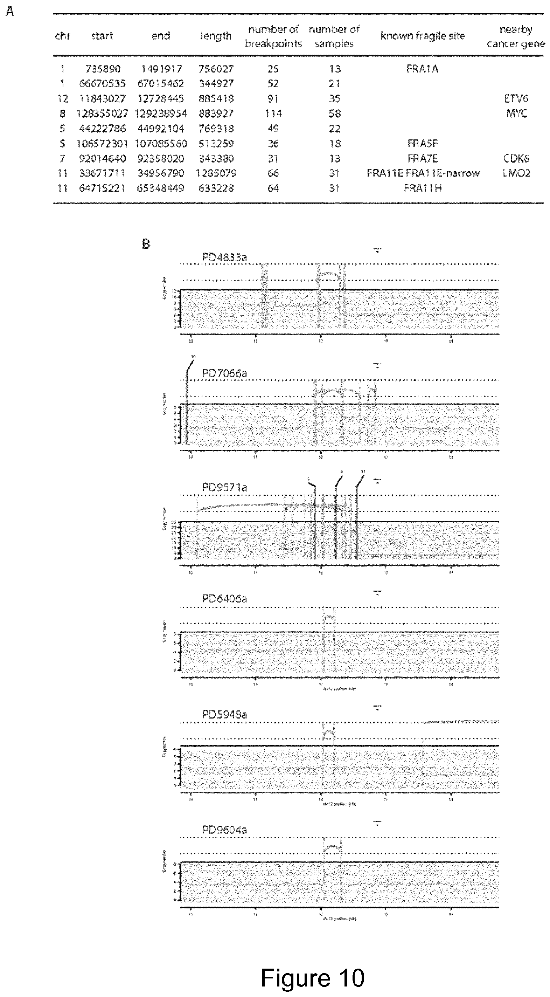

Figure 10 shows hotspots of tandem duplications; Figure 10A lists hotspots of tandem duplications; Figure 10B shows ETV6 tandem duplication hotspot occurring in 6 different patients;

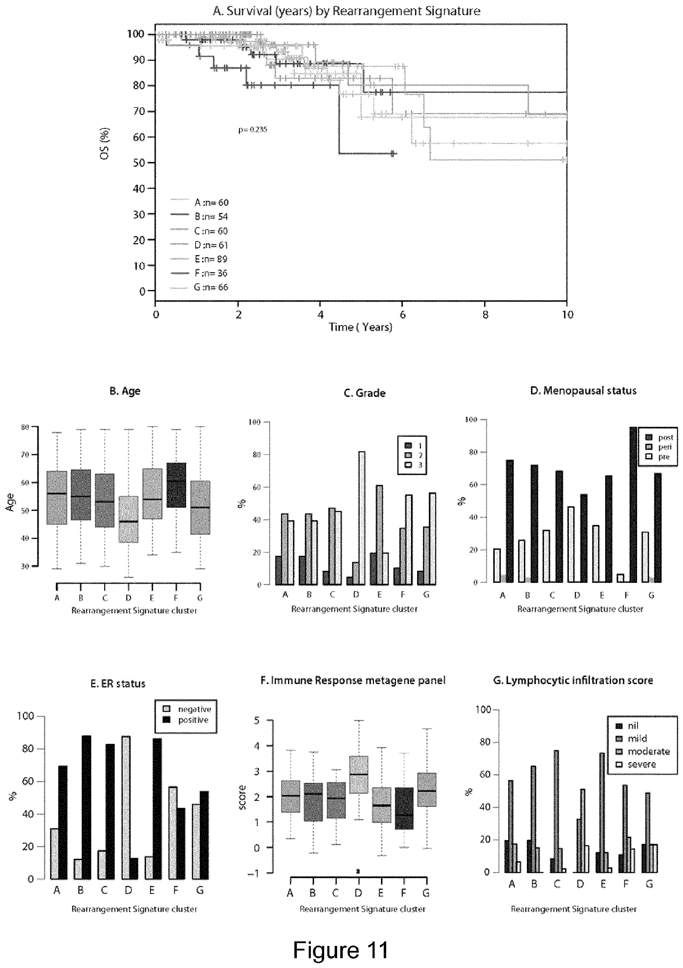

Figure 11 shows the rearrangement cluster groups and associated features, including Figure 11A, overall survival by rearrangement cluster group; Figure 11B, age of diagnosis; Figure 11C, tumour grade; Figure 11D, menopausal status; Figure 11E, ER status; Figure 11F, immune response metagene panel; Figure 11G, lymphocytic infiltration score;

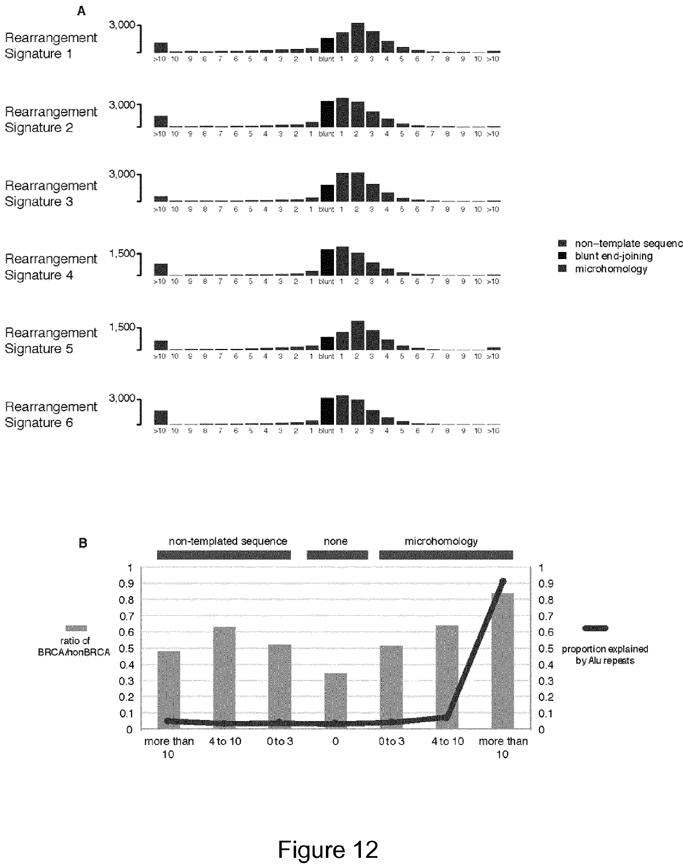

Figure 12 shows the rearrangement breakpoint junctions; Figure 12A is the breakpoint features of rearrangements in the 560 breast cancers sorted by rearrangement signature with non-template sequences to the left of the "blunt" marking and microhomology to the right; Figure 12B shows the breakpoint features in BRCA and non-BRCA cancers; and

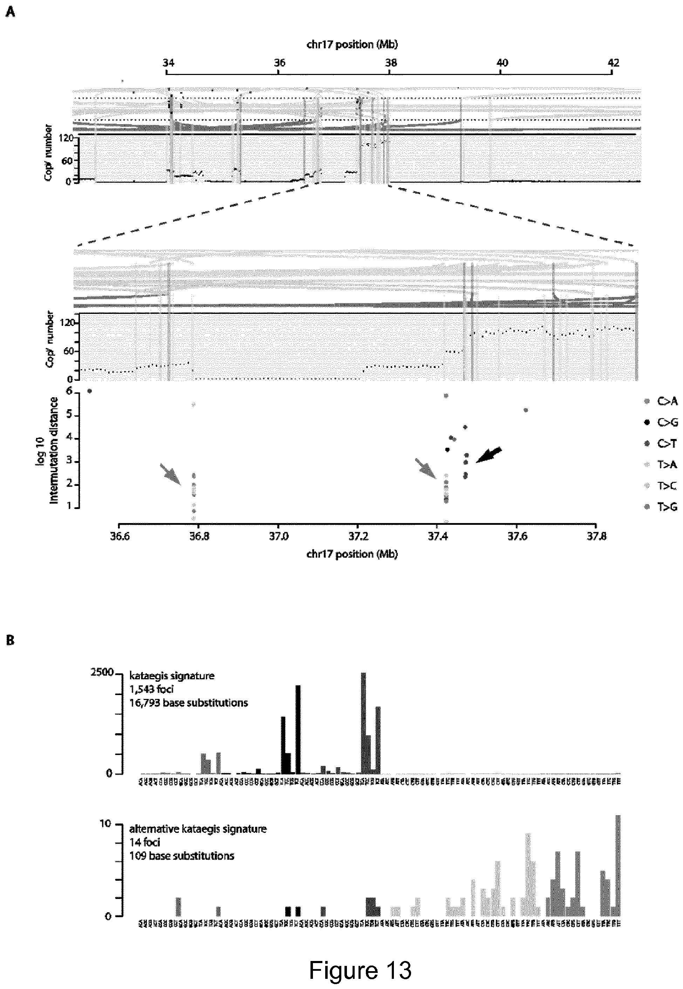

Figure 13 shows signatures of focal hypermutation; Figure 13A shows kataegis and alternative kataegis occurring at the same locus (ERBB2 amplicon in PD13164a) with the copy number

(y-axis) depicted as black dots; lines represent rearrangements breakpoints with the topmost panel showing a ~10Mb region including the ERBB2 locus; the second panel from top zooms in 10-fold to a ~1Mb window highlighting co-occurrence of rearrangement breakpoints, with copy number changes and three different kataegis loci; the third panel from top demonstrates kataegis loci in more detail with log10 intermutation distance on y axis and black arrow highlighting kataegis and light grey blue arrows highlighting alternative kataegis; Figure 13B shows sequence context of kataegis and alternative kataegis identified in this dataset.

*BRIEF DESCRIPTION OF THE TABLES*

Table 1 sets out a quantitative definition of a number of rearrangement signatures;

Table 2 is a summary of the somatic mutation catalogue;

Table 3 is a summary of the hunt for novel driver mutations using base substitutions only

Table 4 is a summary of the hunt for novel driver mutations using insertions/deletions only

Table 5 is a summary of the hunt for novel driver mutations using a combination of base substitutions and insertions/deletions

Table 6 is a curated cancer gene list for all cancer types

Table 7 is a summary of the hunt for driver mutations from amongst rearrangements

Table 8 is the list of driver mutations identified in 560 breast cancers

Table 9 is a summary of an analysis searching for predicted in-frame gene fusions, recurrent donors and acceptors

Table 10 is a summary of the analysis for recurrent non-coding events.

Table 11 is a summary of base substitution and rearrangement mutational signatures and kataegis extracted from 560 breast cancer genomes

*DETAILED DESCRIPTION*

The methods of the invention comprise characterising a DNA sample obtained from a tumour, the method including three or more of the following steps a) to e):

a) determining a catalogue of base substitution signatures which are present in the sample;
b) determining a catalogue of rearrangement signatures which are present in the sample, using a rearrangement classification that classifies rearrangements: (i) between clustered and non-clustered, (ii) between deletions, inversions and tandem duplications, and (iii) by size of the rearrangement;
c) determining a catalogue of insertion/deletion signatures which are present in the sample
d) determining the overall copy number profile in the sample
e) identifying putative driver mutations present in the sample
and

based on said determinations and identifications, constructing an interpreted profile of the tumour.
For the avoidance of doubt, the method may include any combination of three or more of the steps a) to e), being: steps a), b) and c); steps a), b) and d); steps a), b) and e); steps b), c) and d); steps b), d) and e); steps c), b) and e); steps a), b), c) and d); steps b), c), d) and e); or all of the steps a) to e).
In certain embodiments the method further includes the steps of:

f) identifying putative recurrently mutated non-coding sites, and
using the identified putative recurrently mutated non-coding sites in constructing the interpreted profile.

In certain embodiments the method further includes the steps of:

g) obtaining a biologically useful over-arching summary of the sample, and
using the obtained summary in constructing the interpreted profile.

In certain of those embodiments, step g) includes identifying whether the sample has a high or low likelihood of being homologous recombination (HR)-deficient by performing the steps of: determining the presence or absence of a plurality of base substitution signatures, rearrangement signatures and insertion/deletion (indel) signatures in the sample and copy number profiles for the sample; generating, from the presence or absence of said plurality of base substitution signatures, rearrangement signatures and indel signatures in the sample and the copy number profiles for the sample, a probabilistic score; and based on said probabilistic score, identifying whether said sample has a high or low likelihood of being homologous recombination (HR)-deficient.
Alternatively, step g) may include identifying whether the sample has a high or low likelihood of being homologous recombination (HR)-deficient by performing the steps of: performing two or more of the following steps:

a) determining the presence or absence of at least one base substitution signature in the sample
b) determining the presence or absence of at least one rearrangement signature in the sample
c) determining the presence or absence of at least one indel signature in the sample; and
d) determining a copy number profile for the sample;

generating, from the above determinations, a probabilistic score; and based on said probabilistic score, identifying whether said sample has a high or low likelihood of being homologous recombination (HR) -deficient.

Preferably three or more and more preferably all four of the determining steps of this aspect are performed. The probabilistic score and the weighting of the determinations in generating that probabilistic score may vary depending on which determining steps are performed and/or depending on the number of determining steps performed and/or the number of signatures or profiles used in each determining step.

Where base substitution signatures are considered, preferably the plurality of base substitution signatures include either base substitution signature 3 or base substitution signature 8 or both.

Where rearrangement signatures are considered, preferably the plurality of rearrangement signatures includes either rearrangement signature 5 or rearrangement signature 3 or both.

Where indel signatures are considered, preferably the plurality of indel signatures include microhomology-mediated indels.

Preferably the copy number profiles, if considered, include the HRD copy number-based index.

In particular embodiments of the present invention, the plurality of base substitution signatures, the plurality of rearrangement signatures and the plurality of indel signatures consist of base substitution signature 3, base substitution signature 8, rearrangement signature 5 and rearrangement signature 3 and microhomology-mediated indels. Following an extensive study of WGS from breast cancers, these five factors, together with the copy number profile, have been found to have the greatest influence on whether a tumour is HR-deficient or not.

Preferably in such embodiments, the probabilistic score is a weighted score which gives weight to the factors in the following precedence (greatest first): microhomology-mediated indels, base substitution signature 3, rearrangement signature 5, the HRD copy number-based index, rearrangement signature 3 and base substitution signature 8. The study of WGS from breast cancers found that the above order was indicative of the importance of these six factors.

The method may further include the step of cataloguing the somatic mutations in said sample to produce a mutational catalogue for that sample, wherein the presence or absence of said base substitution signatures, rearrangement signatures and/or indel signatures as required, is derived from said mutational catalogue.

When such a catalogue has been obtained, the method may further include the step of determining the number of mutations in the mutational catalogue which are attributable to each of the base substitution signatures, rearrangement signatures and/or indel signatures, as required, which are determined to be present.

Generating the probabilistic score may include the sub steps of: log-transforming the number of mutations attributed to each of the signatures; normalising the log-transformed number of mutations for each signature and the copy number profile; and weighting each of said normalised values by a predetermined weighting factor which represents the likelihood of the signature or profile associated with that value causing the tumour to be HR deficient.

By log-transforming the number of mutations and normalising all of the features, an accurate balance between the influence of the various factors can be obtained.

In one particular embodiment, the probabilistic score is generated as

$$P(C_i = BRCA) = \frac{1}{1 + e^{-(\beta_0 + x_i^T \beta)}}$$

where

$C_i$ is the variable encoding the status of i$^{th}$ sample
$\beta_0$ is the intercept weight
$x_i^T$ is the vector encoding features of i$^{th}$ sample; and
$\beta$ is the vector of weights.

For embodiments wherein the features consist of the six features set out above, the vector of weights $\beta$ may be as set out in the table below, or within a variation of $\pm 10\%$, preferably $\pm 5\%$ of these weights:

| Feature | weight β |
|---|---|
| Proportion of indels with micro-homology | 2.129 |
| Number of base substitutions of signature 3 | 1.239 |
| Number of rearrangement signature 5 rearrangements | 0.978 |
| HRD index | 0.613 |
| Number of rearrangement signature 3 rearrangements | 0.588 |
| Number of base substitutions of signature 8 | 0.444 |

For other embodiments wherein the features consist of the six features set out above, the vector of weights $\beta$ may be as set out below in the table below, or within a variation of $\pm 10\%$, preferably $\pm 5\%$ of these weights:

| Feature | weight β |
|---|---|
| Proportion of indels with micro-homology | 2.398 |
| Number of base substitutions of signature 3 | 1.611 |
| Number of rearrangement signature 5 rearrangements | 0.847 |
| HRD index | 0.667 |
| Number of rearrangement signature 3 rearrangements | 1.153 |
| Number of base substitutions of signature 8 | 0.091 |

For embodiments wherein the features consist of a subset of the six features set out above, the vector of weights $\beta$ may be as set out in the table below, or within a variation of $\pm 10\%$, preferably $\pm 5\%$ of these weights:

| Feature 1 | Weight | Feature 2 | Weight |
|---|---|---|---|
| Number of base substitutions of signature 3 | 2.371 | Number of rearrangement signature 3 rearrangements | 1.835 |
| Number of base substitutions of signature 3 | 1.876 | Number of rearrangement signature 5 rearrangements | 2.989 |
| Number of base substitutions of signature 3 | 2.931 | Proportion of indels with micro-homology | 3.984 |
| Number of base substitutions of signature 3 | 2.429 | HRD index | 2.051 |
| Number of rearrangement signature 3 rearrangements | 3.559 | Proportion of indels with micro-homology | 4.819 |
| Number of rearrangement signature 3 rearrangements | 1.650 | HRD index | 1.895 |
| Number of rearrangement signature 3 rearrangements | 2.297 | Number of base substitutions of signature 8 | 0.676 |
| Number of rearrangement signature 5 rearrangements | 3.026 | Proportion of indels with micro-homology | 1.933 |
| Number of rearrangement signature 5 rearrangements | 3.715 | HRD index | 1.017 |
| Proportion of indels with micro-homology | 2.523 | HRD index | 1.894 |
| Proportion of indels with micro-homology | 3.223 | Number of base substitutions of signature 8 | 0.807 |
| HRD index | 2.813 | Number of base substitutions of signature 8 | 0.357 |

The step of identifying may include comparing said score to a predetermined threshold and performing said identification based on said comparison. The threshold may be set based on clinical parameters. For example, the weighted score may be compared to a threshold and, from that comparison, a clinical decision as to how to treat a tumour from which the DNA sample was taken can be made.

The predetermined threshold may be selected in a number of ways. In particular, different thresholds for this determination may be set depending on the context and the desired certainty of the outcome.

In some embodiments, the threshold will be an absolute number of rearrangements from the rearrangement catalogue of the DNA sample which are determined to be associated with a particular rearrangement signature. If this number is exceeded, then it can be determined that a particular rearrangement signature is present in the DNA sample.

The rearrangement signatures are generally "additive" with respect to each other (i.e. a tumour may be affected by the underlying mutational processes associated with more than one signature and, if this is the case, a sample from that tumour will generally display a higher overall number of rearrangements (being the sum of the separate rearrangements associated with each of the underlying processes), but with the proportion of rearrangements spread over the signatures which are present). As a result, in determining the presence or absence of a particular signature, attention may focus on the absolute number of rearrangements associated with a particular signature in the sample (which may be calculated by the methods described below in other aspects of the invention). Such thresholds are generally better in situations where multiple signatures are present in a sample.

In these embodiments, a signature may be determined to be present if at least 5 and preferably at least 10 informative rearrangements are associated with it.

In other embodiments, the threshold combines the total number of rearrangements detected in the sample (which may be set to ensure that the analysis is representative) along with a proportion of the rearrangements which are associated with a particular signature (again, as determined by the methods described below in other aspects of the invention).

For example, the requirements for determination that a signature is present may be that there are at least 20, preferably at least 40, more preferably at least 50 informative rearrangements and a signature may be deemed to be present if a proportion of at least 10%, preferably at least 20%, more preferably at least 30% of the rearrangements are associated with it. The higher the number of rearrangements present in a sample, the lower the proportional threshold for detection of a specific signature may be.

The proportional thresholds may be adjusted depending on the number of other signatures which make up a significant portion of the rearrangements found in the sample (e.g., if 4 signatures are each present with 20-25% of the rearrangements, then it may be determined that all 4 signatures are present, rather than no signatures at all are present), even if the threshold determined under the present embodiments is 30%.

The above thresholds are based on data obtained from genomes sequenced to 30-40 fold depth. If data is obtained from genomes sequenced at lower coverages, then the number of rearrangements detected overall is likely to be lower, and the thresholds will need to be adjusted accordingly.

The method may further include the steps of:

> h) identifying the presence of mis-match repair (MMR) deficiency in the sample by using the presence or absence of base substitution signatures and indel signatures, and
> using this identification in constructing the interpreted profile.

The method may further include the steps of:

> i) identifying mutational characteristics in the sample that are informative of pathophysiological processes that are targetable including signatures relating to the immunological responses or to other DNA damage response processes, and
> using the identified characteristics in constructing the interpreted profile.

Preferably the catalogue of base substitution signatures, if obtained, is obtained by: cataloguing the somatic mutations in said sample to produce a mutational catalogue for that sample; determining the contributions of known mutational signatures to said mutational catalogue by determining a scalar factor for each of a plurality of said known mutational signatures which together minimize a function representing the difference between the mutations in said catalogue and the mutations expected from a combination of said plurality of known mutational signatures scaled by said scalar factors; and if the scalar factor corresponding to any one of said mutational signatures exceeds a predetermined threshold, including said mutation signature in the catalogue of base substitution signatures for the sample.

Preferably the method of this aspect includes the further step of, prior to said step of determining, filtering the mutations in said catalogue to remove either residual germline mutations or known sequencing artefacts or both. Such filtering can be highly advantageous to remove mutations from the catalogue which are known to arise from mechanisms other than somatic mutation, and may therefore cloud or obscure the contributions of the mutational signatures, or lead to false positive results.

For example, the filtering may use a list of known germline polymorphisms and remove somatic mutations resulting from those polymorphisms from the catalogue prior to determining the contributions of the mutational signatures.

As a further example, the filtering may use BAM files of unmatched normal human tissue sequenced by the same process as the DNA sample and discard any somatic mutation which is present in at least two well-mapping reads in at least two of said BAM files. This approach can remove artefacts resulting from the sequencing technology used to obtain the sample.

The method may further include the step of selecting said plurality of known mutational signatures as a subset of all known mutational signatures. By selecting a subset, for example, based on prior knowledge about the sample, the number of possible signatures contributing to the mutational catalogue is reduced, which is likely to increase the accuracy of the determining step.

For example, the subset of mutational signatures may be selected based on biological knowledge about the DNA sample or the mutational signatures or both. Thus, it may be immediately apparent that a certain DNA sample cannot have resulted from a particular mutational signature as a result of characteristics of the DNA sample and the particular mutational signature. Further possibilities are described in more detail in the embodiments below.

In particular embodiments, the step of determining may determine the scalars $E_i$ which minimize the Frobenius norm:

$$\min \left\| \vec{M} - \sum_{i=1}^{q} (\vec{S_i} \times E_i) \right\|_{2}^{F}$$

wherein $\vec{S_i}$ and $\vec{M}$ are equally-sized vectors with nonnegative components being, respectively, a consensus mutational signature and the mutational catalogue and q is the number of signatures in said plurality of known mutational signatures,

and wherein $E_i$ are further constrained by the requirements that $0 \le E_i \le \|\vec{S_i}\|_1$, $i$ = 1..$q$, and $\sum_{i=1}^{q} E_i = \left\| \vec{S_i} \right\|_1$.

Preferably the catalogue of rearrangement signatures, if obtained, is obtained by:
cataloguing the somatic mutations in said sample to produce a rearrangement catalogue for that sample which classifies identified rearrangement mutations in the sample into a plurality of categories using said rearrangement classification; determining the contributions of known rearrangement signatures to said rearrangement catalogue by computing the cosine similarity between the rearrangement mutations in said catalogue and the known rearrangement signatures; and if the number or proportion of rearrangements in the rearrangement catalogue which are determined to be associated with one of said rearrangement signatures exceeds a predetermined threshold, including said rearrangement signature in the catalogue of rearrangement signatures for the sample.

Preferably the method includes the further step of, prior to said step of determining, filtering the mutations in said catalogue to remove either residual germline structural variations or known sequencing artefacts or both. Such filtering can be highly advantageous to remove rearrangements from the catalogue which are known to arise from mechanisms other than somatic mutation, and may therefore cloud or obscure the contributions of the rearrangement signatures, or lead to false positive results.

For example, the filtering may use a list of known germline rearrangement or copy number polymorphisms and remove somatic mutations resulting from those polymorphisms from the catalogue prior to determining the contributions of the rearrangement signatures.

As a further example, the filtering may use BAM files of unmatched normal human tissue sequenced by the same process as the DNA sample and discards any somatic mutation which is present in at least two well-mapping reads in at least two of said BAM files. This approach can remove artefacts resulting from the sequencing technology used to obtain the sample.

The classification of the rearrangement mutations includes identifying mutations as being clustered or non-clustered. This may be determined by a piecewise-constant fitting ("PCF") algorithm which is a method of segmentation of sequential data. In particular embodiments, rearrangements may be identified as being clustered if the average density of rearrangement breakpoints within a segment is a certain factor greater than the whole genome average density of rearrangements for an individual patient's sample. For example the factor may be at least 8 times, preferably at least 9 times and in particular embodiments is 10 times. The inter-rearrangement distance is the distance from a rearrangement breakpoint to the one immediately preceding it in the reference genome. For any given breakpoint, this measurement is already known.

The classification of the rearrangement mutations includes identifying rearrangements as one of: tandem duplications, deletions, inversions or translocations. Such classifications of rearrangement mutations are already known.

The classification of the rearrangement mutations further includes grouping mutations identified as tandem duplications, deletions or inversions by size. For example, the mutations may be grouped into a plurality of size groups by the number of bases in the rearrangement. Preferably the size groups are logarithmically based, for example 1-10kb, 10-100kb, 100kb-1Mb, 1Mb-10Mb and greater than 10Mb. Translocations cannot be classified by size.

In particular embodiments, in each DNA sample the number of rearrangements $E_i$ associated with the *i*th mutational signature $\vec{S}_i$ is determined as proportional to the cosine similarity ($\vec{C}_i$) between the catalogue of this sample $\vec{M}$ and $\vec{S}_i$ :

$$\vec{C}_i = \frac{\vec{S}_i \cdot \vec{M}}{\| \vec{S}_i \| \| \vec{M} \|}$$

wherein:

$$E_i = \frac{\vec{C}_i}{\sum_{i=1}^{q} \vec{C}_i} \sum_{j=1}^{36} \vec{M}^j$$

wherein $\vec{S}_i$ and $\vec{M}$ are equally-sized vectors with nonnegative components being, respectively, a known rearrangement signature and the mutational catalogue and $q$ is the number of signatures in said plurality of known rearrangement signatures.

The method may further include the step of filtering the number of rearrangements determined to be assigned to each signature by reassigning one or more rearrangements from signatures that are less correlated with the catalogue to signatures that are more correlated with the catalogue. Such filtering can serve to reassign rearrangements from a signature which has only a few rearrangements associated with it (and so is probably not present) to a signature which has a greater number of rearrangement associated with it. This can have the effect of reducing "noise" in the assignment process.

In one embodiment, the step of filtering uses a greedy algorithm to iteratively find an alternative assignment of rearrangements to signatures that improves or does not change the cosine similarity between the catalogue $\vec{M}$ and the reconstructed catalogue $\vec{M}' = S \times \vec{E}'_{ij}$ , wherein $\vec{E}'_{ij}$ is the version of the vector $\vec{E}$ obtained by moving the mutations from the signature i to signature *j,* wherein, in each iteration, the effects of all possible movements between signatures are estimated, and the filtering step terminates when all of these possible reassignments have a negative impact on the cosine similarity.

The subject may be a cancer patient or a suspected cancer patient. For example, the method may be used in the determination or identification of a rearrangement sequence to predict whether the subject has cancer or not or what type of cancer a patient has, or to select the subject for a particular form of treatment.

The method may further include the step of determining if the number or proportion of rearrangements in the rearrangement catalogue which are determined to be associated with one or more of said rearrangement signatures each or in combination exceeds a predetermined threshold and, if so, determining that said rearrangement signature is present in the sample.

Preferably the step of identifying driver mutations present in the sample determines whether one or more, preferably at least 50%, preferably 75%, more preferably all of the breast cancer genes from the list of 93 cancer genes in Fig 3C/Table 8 are present or not.

The DNA samples are preferably obtained from both tumour and normal tissues obtained from the patient, e.g. blood sample from the patient and tumour tissue obtained by a biopsy. Somatic mutations in the tumour sample are detected, standardly, by comparing its genomic sequences with the one of the normal tissue.

The patient to be treated is preferably a human patient.

Further aspects of the present invention include computer programs for running on computer systems which carry out the method of the above aspect.

A further aspect of the present invention provides a computer program product containing non-transitory memory storing a computer program which, when run on a computer, performs three or more of the following steps a) to e):

a) determining a catalogue of base substitution signatures which are present in the sample;

b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;

c) determining a catalogue of insertion/deletion signatures which are present in the sample

d) determining the overall copy number profile in the sample

e) identifying putative driver mutations present in the sample

and

based on said determinations and identifications, constructs an interpreted profile of the tumour, wherein the three or more steps a) to e) include at least steps a), b) and c); steps a), b) and d); steps a), b) and e); steps b), c) and d); steps b), d) and e); or steps b), c) and e); and

determining the prognosis of the tumour or suitability of a treatment for the tumour from the interpreted profile, or determining whether or not the patient is suitable for a clinical trial of a cancer therapy on the basis of the interpreted profile; or

classifying a plurality of patients undergoing treatment for cancer, or participating in a clinical trial, the method including allocating patients to groups based on an interpreted profile constructed from a DNA sample obtained from a tumour in each of said plurality of patients; or

classifying patients who have completed a clinical trial or course of treatment, by correlating the interpreted profile obtained for each patient with the clinical outcome of the trial or treatment.

A further aspect of the present invention provides a computer having a processor, wherein the processor is configured to:

perform three or more of the following steps a) to e):

a) determining a catalogue of base substitution signatures which are present in the sample;

b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;

c) determining a catalogue of insertion/deletion signatures which are present in the sample

d) determining the overall copy number profile in the sample

e) identifying putative driver mutations present in the sample

and

based on said determinations and identifications, to construct an interpreted profile of the tumour; , wherein the three or more steps a) to e) include at least steps a), b) and c); steps a), b) and d); steps a), b) and e); steps b), c) and d); steps b), d) and e); or steps b), c) and e); and

determine the prognosis of the tumour or suitability of a treatment for the tumour from the interpreted profile, or determining whether or not the patient is suitable for a clinical trial of a cancer therapy on the basis of the interpreted profile; or

classify a plurality of patients undergoing treatment for cancer, or participating in a clinical trial, the method including allocating patients to groups based on an interpreted profile constructed from a DNA sample obtained from a tumour in each of said plurality of patients; or

classify patients who have completed a clinical trial or course of treatment, by correlating the interpreted profile obtained for each patient with the clinical outcome of the trial or treatment.

The computer program and the processor of the above two aspects may also carry out some or all of the optional or preferred steps described above in relation to the first aspect.

These and other aspects of the invention are described in further detail below.

## Uses of Predictor Outcome

The characterisation (or "genomic profiling") of DNA samples from tumours according to the present disclosure has many uses.

In particular, it is envisaged that this characterisation will allow for significant advances in the interpretation of clinical outcomes and clinical trials data by allowing the identification of common features amongst those patients who respond to a particular treatment and those who do not. At present little is known about what causes two apparently similar tumours to respond or not to a particular therapy. If the tumours are characterised, either before or after the treatment, differences in the tumours may be identified which allow a profile to be developed of the tumours which respond and which do not.

Similarly, the characterisation may be used to select or stratify patients for clinical trials by profiling the tumour in advance and conducting the trial only on patients with particular types of tumour, or ensuring that patients with particular types of tumour are considered as a cohort within the trial and their results considered separately from patients with tumours which exhibit different features.

The characterisation may also be used to guide treatment decisions or in prognosis. In particular, if tumours with particular features or combinations of features are found to respond particularly well or particularly poorly to a certain treatment as a result of studies such as those described in the previous paragraphs, the characterisation may in future be performed in advance of commencing treatment on a patient in order to determine the suitability of that treatment for the patient in question.

Specifically, aspects of the present invention provide methods of classifying of patients undergoing treatment for cancer, or participating in a clinical trial, based on the profile constructed from a DNA sample obtained from a tumour in the patient by a method according to the above described aspects, including some, all or none of the optional or preferred features of those aspects as described above.

Further aspects of the present invention provide methods of selecting a patient for a clinical trial of a cancer therapy, the selection being made on the basis of the profile constructed from a DNA sample obtained from a tumour in the patient by a method according to the above described aspects, including some, all or none of the optional or preferred features of those aspects as described above.

Further aspects of the present invention provide a method of classifying patients who have completed a clinical trial or course of treatment, the method including: characterising a DNA sample obtained from a tumour in each of said patients using a method according to the above described aspects, including some, all or none of the optional or preferred features of those aspects; and correlating the interpreted profile obtained for each patient with the clinical outcome of the trial or treatment as well as a method of determining a prognosis of a tumour, the method including characterising a DNA sample obtained from said tumour using a method according to the above described aspects, including some, all or none of the optional or preferred features of those aspects; and determining the prognosis from the interpreted profile.

### Genomic profiling to characterise tumour DNA

The complete genomes of 560 breast cancers and non-neoplastic tissue from different individuals (556 female and four male) were sequenced. 3,479,652 somatic base substitutions, 371,993 small indels and 77,695 rearrangements were detected, with substantial variation in the number of each between individual samples (Figure 3A, Supplementary Table 3). Transcriptome sequence, microRNA expression, array based copy number and DNA methylation data were obtained from subsets of cases.

To identify new cancer genes, the inventors combined somatic substitutions and indels in protein-coding exons with data from other series [12-15, 26], constituting a total of 1,332 breast cancers, and searched for mutation clustering in each gene beyond that expected by chance. Five cancer genes were found for which evidence was previously absent or equivocal *(MED23, FOXP1, MLLT4, XBP1, ZFP36L1)*, or for which the mutations indicate the gene acts as a recessive cancer gene in breast cancer rather than in a dominant fashion, as previously reported in other cancer types (Supplementary Methods section 7.4 for detailed descriptions). From published reports on all cancer types (http://cancer.sanger. ac.uk/census), a list of 727 human cancer genes was compiled (Supplementary Table 12). Based on driver mutations found previously, conservative rules were defined for somatic driver base substitutions and indel mutations in each gene and sought mutations conforming to these rules in the 560 breast cancers. 916 likely driver mutations of these classes were identified (Figure 3B).

To explore the role of genomic rearrangements as driver mutations [16, 18, 19, 27], the inventors sought predicted in-frame fusion genes that might create activated, dominant cancer genes. 1,278 unique and 39 infrequently recurrent in-frame gene fusions were identified (Supplementary Table 15). Many of the latter, however, were in regions of high rearrangement density, including amplicons [28] and fragile sites, and their recurrence is likely attributable to chance [27]. Furthermore, transcriptome sequences from 260 cancers did not show expression of these fusions and generally confirmed the rarity of recurrent in-frame fusion genes. By contrast, recurrent rearrangements interrupting the gene footprints of *CDKN2A, RB1, MAP3K1, PTEN, MAP2K4, ARID1B, FBXW7, MLLT4* and *TP53* were found beyond the numbers expected from local background rearrangement rates, indicating that they contribute to the driver mutation burden of recessive cancer genes. Several other recurrently rearranged genomic regions were observed, including dominantly-acting cancer genes *ETV6* and *ESR1* without consistent elevation in expression levels, L1-retrotransposition sites [29] and fragile sites.

Incorporation of recurrent copy number changes, including homozygous deletions and amplifications, generated a final tally of 1,628 likely driver mutations in 93 cancer genes (Fig.1C). At least one driver was identifiable in 95% of cancers. The 10 most frequently mutated genes were *TP53, PIK3CA, MYC, CCND1, PTEN, ERBB2, chr8:ZNF703/FGFR1 locus, GATA3, RB1* and *MAP3K1* (Figure 3B, Figure 8) and accounted for 62% of drivers.

**Recurrent somatic mutations in non-coding genomic regions**

To investigate non-coding somatic driver substitutions and indels, the inventors searched for non-coding genomic regions with more mutations than expected by chance (Figure 4A, Supplementary Table 16, Figure 9). The promoter of *PLEKHS1* (pleckstrin homology domain containing, family S member 1) exhibited recurrent mutations at two genomic positions [30] (Fig.2A), the underlined bases in the sequence CAGCAAGC TGAACA GCTTGCTG (as previously reported [30]). The two mutated bases are flanked on either side by 9bp of palindromic sequence forming inverted repeats [31]. Most cancers with these mutations showed many base substitutions of mutational signatures 2 and 13 that have been attributed to activity of APOBEC DNA-editing proteins that target the TCN sequence motif. One of the mutated bases is a cytosine in a TCA sequence context (shown above as the reverse complement, TGA) at which predominantly C>T substitutions were found. The other is a cytosine in ACA context which showed both C>T and C>G mutations.

The TGAACA core sequence was mutated at the same two positions at multiple locations elsewhere in the genome (Figure 4B) where the TGAACA core was also flanked by palindromes (inverted repeat), albeit of different sequences and lengths (Figure 4B). These mutations were also usually found in cancers with many signature 2 and 13 mutations (Figure 4A). TGAACA core sequences with longer flanking palindromes generally exhibited a higher mutation rate, and TGAACA sequences flanked by 9bp palindromes exhibited a ~265-fold higher mutation rate than sequences without them (Figure 4C). However, additional factors must influence the mutation rate because it varied markedly between TGAACA core sequences with different palindromes of the same length (Figure 4D). Some TGAACA-inverted repeat sites were in regulatory regions but others were intronic or intergenic without functional annotation (examples in Figure 4B) or exonic. The propensity for mutation recurrence at specific positions in a distinctive sequence motif in cancers with numerous mutations of particular signatures renders it plausible that these are hypermutable hotspots [32-34], perhaps through formation of DNA hairpin structures [35], which are single stranded at their tips enabling attack by APOBEC enzymes, rather than driver mutations.

Two recurrently mutated sites were also observed in the promoter of *TBC1D12* (TBC1 domain family, member 12) (q-value $4.5e^{-2}$) (Figure 4A). The mutations were characteristic of signatures 2 and 13 and enriched in cancers with many signature 2 and 13 mutations (Figure 4A). The mutations were within the *TBC1D12* Kozak consensus sequence (CCCCAG**ATG**GTGGG)) shifting it away from the consensus [36]. The association with particular mutational signatures suggests that these may also be in a region of hypermutability rather than drivers.

The *WDR74* (WD repeat domain 74) promoter showed base substitutions and indels (q-value $4.6e^{-3}$) forming a cluster of overlapping mutations (Figure 4A) [20]. Coding sequence driver mutations in *WDR74* have not been reported. No differences were observed in *WDR74* transcript levels between cancers with *WDR74* promoter mutations compared to those without. Nevertheless, the pattern of this non-coding mutation cluster, with overlapping and different mutation types, is more compatible with the possibility of the mutations being drivers.

Two long non-coding RNAs, *MALAT1* (q-value $8.7e^{-11}$, as previously reported [12]) and *NEAT1* (q-value $2.1e^{-2}$) were enriched with mutations. Transcript levels were not significantly different between mutated and non-mutated samples. Whether these mutations are drivers, or result from local hypermutability, is unclear.

**Mutational signatures**

Mutational processes generating somatic mutations imprint particular patterns of mutations on cancer genomes, termed signatures [2, 24, 37]. Applying a mathematical approach [25] to extract mutational signatures previously revealed five base substitution signatures in breast cancer; signatures 1, 2, 3, 8 and 13 [2, 24]. Using this method in the 560 cases revealed 12 signatures, including those previously observed and a further seven, of which five have formerly been detected in other cancer types (signatures 5, 6, 17, 18 and 20) and two are new (signatures 26 and 30) (Figure 5A-B, Figure 6A, Supplementary Table 21A-C, Supplementary Methods 15 for further details). Two indel signatures were also found [2, 24].

In embodiments of the present invention, detection of the presence of base substitution signatures in a DNA sample obtained from a tumour is carried out using the methods disclosed in [58].

Signatures of rearrangement mutational processes have not previously been formally investigated. To enable this we adopted a rearrangement classification incorporating 32 subclasses. In many cancer genomes, large numbers of rearrangements are regionally clustered, for example in zones of gene amplification. Therefore, the rearrangements were first classified into those inside and outside clusters, further subclassified into deletions, inversions and tandem duplications, and then according to the size of the rearranged segment. The final category in both groups was interchromosomal translocations.

Application of the mathematical framework used for base substitution signatures [2, 24, 25] extracted six rearrangement signatures (Figure 7A, Supplementary Table 21). Unsupervised hierarchical clustering on the basis of the proportion of rearrangements attributed to each signature in each breast cancer yielded seven major subgroups exhibiting distinct associations with other genomic, histological or gene expression features.

Rearrangement Signature 1 (9% of all rearrangements) and Rearrangement Signature 3 (18% rearrangements) were characterised predominantly by tandem duplications (Figure 7A). Tandem duplications associated with Rearrangement Signature 1 were mostly >100kb (Figure 7B), and those with Rearrangement Signature 3 <10kb (Figure 7C). More than 95% of Rearrangement Signature 3 tandem duplications were concentrated in 15% of cancers (Cluster D, Figure 7), many with several hundred rearrangements of this type. Almost all cancers (91%) with *BRCA1* mutations or promoter hypermethylation were in this group, which was enriched for basal-like, triple negative cancers and copy number classification of a high Homologous Recombination Deficiency (HRD) index [38-40]. Thus, inactivation of *BRCA1,* but not *BRCA2,* may be responsible for the Rearrangement Signature 3 small tandem duplication mutator phenotype.

More than 35% of Rearrangement Signature 1 tandem duplications were found in just 8.5% of the breast cancers and some cases had hundreds of these (Cluster F, Figure 7). The cause of this large tandem duplication mutator phenotype (Figure 7B) is unknown. Cancers exhibiting it are frequently TP53-mutated, relatively late diagnosis, triple-negative breast cancers, showing enrichment for base substitution signature 3 and a high Homologous Recombination Deficiency (HRD) index (Figure 7) but do not have *BRCA1/2* mutations or *BRCA1* promoter hypermethylation.

Rearrangement Signature 1 (Figure 7B) and 3 (Figure 7C) tandem duplications were generally evenly distributed over the genome. However, there were nine locations at which recurrence of tandem duplications was found across the breast cancers and which often showed multiple, nested tandem duplications in individual cases (Figure 10). These may be mutational hotspots specific for these tandem duplication mutational processes although we cannot exclude the possibility that they represent driver events.

Rearrangement Signature 5 (accounting for 14% rearrangements) was characterised by deletions <100kb. It was strongly associated with the presence of *BRCA1* mutations or promoter hypermethylation (Cluster D, Figure 7, *BRCA2* mutations (Cluster G, Figure 7) and with Rearrangement Signature 1 large tandem duplications (Cluster F, Figure 7).

Rearrangement Signature 2 (accounting for 22% rearrangements) was characterised by non-clustered deletions (>100kb), inversions and interchromosomal translocations, was present in most cancers but was particularly enriched in ER positive cancers with quiet copy number profiles (Cluster E, GISTIC Cluster 3, Figure 8). Rearrangement Signature 4 (accounting for 18% of rearrangements) was characterised by clustered interchromosomal translocations while Rearrangement Signature 6 (19% of rearrangements) by clustered inversions and deletions (Clusters A, B, C, Figure 8).

Short segments (1-5bp) of overlapping microhomology characteristic of alternative methods of end joining repair were found at most rearrangements [2, 14]. Rearrangement Signatures 2, 4 and 6 were characterised by a peak at 1bp of microhomology while Rearrangement Signatures 1, 3 and 5, associated with homologous recombination DNA repair deficiency, exhibited a peak at 2bp (Figure 12). Thus, different end-joining mechanisms may operate with different rearrangement processes. A proportion of breast cancers showed Rearrangement Signature 5 deletions with longer (>10bp) microhomologies involving sequences from short-interspersed nuclear elements (SINEs), most commonly AluS (63%) and AluY (15%) family repeats (Figure 12). Long segments (more than 10bp) of non-templated sequence were particularly enriched amongst clustered rearrangements.

As a result of the above, a method for determining the number of rearrangements detected in a DNA sample which are associated with a particular rearrangement signature has also been developed, as set out in more detail in PCT/EP2017/060279. In embodiments of the present invention, the detection of rearrangement signatures in a DNA sample from a tumour are carried out according to the methods set out in that application.

### Microhomology-mediated indels

The determination of the presence or absence of microhomology-mediated indels (also called "microhomology-mediated deletions" as, of the overall range of insertions and deletions, only deletions are ever classified as microhomology-mediated) can be performed as follows.

First, indels are identified using cgpPindel, as described in [59] and [60].

For each insertion/deletion (indel), more than or equal to 25bp of flanking sequence is identified using the Ensembl API.

Only deletions are taken into consideration for the rest of the analysis. If the first few nucleotides but not all of the nucleotides of the deletion motif matches the first few nucleotides of the immediate 3' flanking sequence, then this is referred to as "microhomology-mediated deletion" or "microhomology-mediated indel".

### Localised hypermutation: *kataegis*

Focal base substitution hypermutation, termed *kataegis,* is generally characterised by substitutions with characteristic features of signatures 2 and 13 [2, 24]. *Kataegis* was observed in 49% breast cancers, with 4% exhibiting 10 or more foci (Supplementary Table 21C). *Kataegis* colocalises with clustered rearrangements characteristic of rearrangement signatures 4 and 6 (Figure 8). Cancers with tandem duplications or deletions of rearrangement signatures 1, 3 and 5 did not usually demonstrate *kataegis*. However, there must be additional determinants of *kataegis* since only 2% of rearrangements are associated with it. A rare (14/1,557 foci, 0.9%), alternative form of *kataegis* colocalising with rearrangements but with a base substitution pattern characterised by T>G and T>C mutations predominantly at NTT and NTA sequences was also observed. This pattern of base substitutions most closely matches Signature 9 (http://cancer.sanger.ac.uk/cosmic/signatures), previously observed in B lymphocyte neoplasms and attributed to polymerase eta activity [41].

**Mutational signatures associated with *BRCA1* and *BRCA2* mutations**

Of the 560 breast cancers, 90 had germline (60) or somatic (14) inactivating mutations in *BRCA1* (35) or *BRCA2* (39) or showed methylation of the *BRCA1* promoter (16). Loss of the wild-type chromosome 17 or 13 was observed in 80/90 cases. The latter exhibited many base substitution mutations of signature 3, accompanied by deletions of >3bp with microhomology at rearrangement breakpoints, and signature 8 together with CC>AA double nucleotide substitutions. Cases in which the wild type chromosome 17 or 13 was retained did not show these signatures. Thus signature 3 and, to a lesser extent, signature 8 are associated with absence of BRCA1 and BRCA2 functions.

Cancers with inactivating *BRCA1* or *BRCA2* mutations usually carry many genomic rearrangements. Cancers with *BRCA1,* but not *BRCA2*, mutations exhibit large numbers of Rearrangement Signature 3 small tandem duplications. Cancers with *BRCA1* or *BRCA2* mutations show substantial numbers of Rearrangement Signature 5 deletions. No other Rearrangement Signatures were associated with *BRCA1* or *BRCA2* null cases. Some breast cancers without identifiable *BRCA1/2* mutations or *BRCA1* promoter methylation showed these features and segregated with *BRCA1/2* null cancers in hierarchical clustering analysis (Figure 7). In such cases, the *BRCA 1/2* mutations may have been missed or other mutated or promoter methylated genes may be exerting similar effects (Please see http://cancer.sanger.ac.uk/cosmic/sample/genomes for examples of whole genome profiles of typical BRCA1 null (e.g. PD6413a, PD7215a) and BRCA2 null tumours (e.g. PD4952a, PD4955a)).

A further subset of cancers (Cluster F, Figure 7) show similarities in mutational pattern to BRCA1/2 null cancers, with many Rearrangement Signature 5 deletions and enrichment for base substitution signatures 3 and 8. However, these do not segregate together with *BRCA1/2* null cases in hierarchical clustering analysis, have Rearrangement Signature 1 large tandem duplications and do not show *BRCA1/2* mutations. Somatic and germline mutations in genes associated with the DNA double-strand break repair pathway including *ATM, ATR, PALB2, RAD51C, RAD50, TP53, CHEK2* and *BRIP1,* were sought in these cancers. We did not observe any clear-cut relationships between mutations in these genes and these mutational patterns.

Cancers with *BRCA1/2* mutations are particularly responsive to cisplatin and PARP inhibitors [43-45]. Combinations of base substitution, indel and rearrangement mutational signatures may be better biomarkers of defective homologous recombination based DNA double strand break repair and responsiveness to these drugs [46] than *BRCA1/2* mutations or promoter methylation alone and thus may constitute the basis of future diagnostics.

As a development of these observations, a method for determining the likelihood of a tumour being HR deficient was developed, as set out in more detail in PCT/EP2017/060294. In embodiments of the present invention, the "BRCAness predictor" methods of that application can form an additional part of the characterisation of the tumour.

**METHODS**

**Sample selection**

DNA was extracted from 560 breast cancers and normal tissue (peripheral blood lymphocytes, adjacent normal breast tissue or skin) from the same individuals. Samples were subjected to pathology review and only samples assessed as being composed of > 70% tumor cells, were accepted for inclusion in the study.

**Massively-parallel sequencing and alignment**

Short insert 500bp genomic libraries were constructed, flowcells prepared and sequencing clusters generated according to Illumina library protocols [47]. 108 base/100 base (genomic), or 75 base (transcriptomic) paired-end sequencing were performed on Illumina GAIIx, Hiseq 2000 or Hiseq 2500 genome analyzers in accordance with the Illumina Genome Analyzer operating manual. The average sequence coverage was 40.4 fold for tumour samples and 30.2 fold for normal samples.

Short insert paired-end reads were aligned to the reference human genome (GRCh37) using Burrows-Wheeler Aligner, BWA (v0.5.9) [48].

**Processing of genomic data**

CaVEMan (Cancer Variants Through Expectation Maximization: http://cancerit.github.io/CaVEMan/) was used for calling somatic substitutions.

Indels in the tumor and normal genomes were called using a modified Pindel version 2.0. (http://cancerit.github.io/cgpPindel/) on the NCBI37 genome build [49].

Structural variants were discovered using a bespoke algorithm, BRASS (BReakpoint AnalySiS) (https://github.com/cancerit/BRASS) through discordantly mapping paired-end reads. Next, discordantly mapping read pairs that were likely to span breakpoints, as well as a selection of nearby properly-paired reads, were grouped for each region of interest. Using the Velvet de novo assembler [50], reads were locally assembled within each of these regions to produce a contiguous consensus sequence of each region. Rearrangements, represented by reads from the rearranged derivative as well as the corresponding non-rearranged allele were instantly recognisable from a particular pattern of five vertices in the de Bruijn graph (a mathematical method used in de novo assembly of (short) read sequences) of component of Velvet. Exact coordinates and features of junction sequence (e.g. microhomology or non-templated sequence) were derived from this, following aligning to the reference genome, as though they were split reads.

Supplementary Table 3 for summary of somatic variants. Annotation was according to ENSEMBL version 58.

Single nucleotide polymorphism (SNP) array hybridization using the Affymetrix SNP6.0 platform was performed according to Affymetrix protocols. Allele-specific copy number analysis of tumors was performed using ASCAT (v2.1.1), to generate integral allele-specific copy number profiles for the tumor cells [51]. ASCAT was also applied to NGS data directly with highly comparable results.

**Identification of novel breast cancer genes**

To identify recurrently mutated driver genes, a dN/dS method that considers the mutation spectrum, the sequence of each gene, the impact of coding substitutions (synonymous, missense, nonsense, splice site) and the variation of the mutation rate across genes [52, 53] was used for substitutions (Supplementary Table 9). Owing to the lack of a neutral reference for the indel rate in coding sequences, a different approach was required (Supplementary Table 10, Supplementary Methods for details). To detect genes under significant selective pressure by either point mutations or indels, for each gene the P-values from the dN/dS analysis of substitutions and from the recurrence analysis of indels were combined using Fisher's method. Multiple testing correction (Benjamini-Hochberg FDR) was performed separately for the 600+ putative driver genes and for all other genes, stratifying the FDR correction to increase sensitivity (as described in Sun *et al.* 2006 [54]). To achieve a low false discovery rate a conservative q-value cutoff of <0.01 was used for significance (Supplementary Table 11).

This analysis was applied to the 560 whole genome sequenced breast cancers as well as a further 772 breast cancers that have been sequenced previously by other institutions.

Please see Supplementary Methods for detailed explanations of these methods.

**Recurrence in the non-coding regions**

*Partitioning the genome into functional regulatory elements/gene features*

To identify non-coding regions with significant recurrence, the inventors used a method similar to the one described for searching for novel indel drivers.

The genome was partitioned according to different sets of regulatory elements/gene features, with a separate analysis performed for each set of elements, including exons (n=20,245 genes), core promoters (n=20,245 genes, where a core promoter is the interval [-250,+250] bp from any transcription start site (TSS) of a coding transcript of the gene, excluding any overlap with coding regions), 5' UTR (n=9,576 genes), 3' UTR (n=19,502 genes), intronic regions flanking exons (n=20,212 genes, represents any intronic sequence within 75bp from an exon, excluding any base overlapping with any of the above elements. This attempts to capture recurrence in essential splice site or proximal splicing-regulatory elements), any other sequence within genes (n=18,591 genes, for every protein-coding gene, this contains any region within the start and end of transcripts not included in any of the above categories), ncRNAs (n=10,684, full length lincRNAs, miRNAs or rRNAs), enhancers (n=194,054) [55], ultra-conserved regions (n=187,057, a collection of regions under negative selection based on 1,000 genomes data [20].

Every element set listed above was analysed separately to allow for different mutation rates across element types and to stratify the FDR correction [54]. Within each set of elements, a negative binomial regression approach was used to learn the underlying variation of the mutation rate across elements. The offset reflects the expected number of mutations in each element assuming uniform mutation rates across them *(i.e. $E_{subs,element} = \sum_{j \in \{1,2,...,192\}} (t \cdot r_j \cdot S_j)$, and, $E_{indels,element} = \mu_{indel} \cdot S_{indel,element}$ ).* As covariate here the local density of mutations in neighbouring non-coding regions was used, corrected for sequence composition and trinucleotide mutation rates, that is, the t parameter of the dN/dS equations. Normalised local rates were pre-calculated for 100kb non-overlapping bins of the genome and used in all analyses. Other covariates (expression, replication time or HiC) were not used here as they were not found to substantially improve the model once the local mutation rate was used as a covariate. A separate regression analysis was performed for substitutions and indels, to account for the different level of uncertainty in the distribution of substitution and indel rates across elements.

$$\text{model}_{subs} = \text{glm.nb}(\text{formula} = n_{subs} \sim \text{offset}(\log(E_{subs})) + \mu_{local,subs})$$

$$\text{model}_{indels} = \text{glm.nb}(\text{formula} = n_{indels} \sim \text{offset}(\log(E_{indels})) + \mu_{local,indels})$$

The observed counts for each element ($n_{subs,element}$ and $n_{indels,element}$) are compared to the background distributions using a negative binomial test, with the estimated overdispersion parameters ($\theta_{subs}$ and $\theta_{indels}$) estimated by the negative binomial regression, yielding P-values for substitution and indel recurrence for each element. These P-values were combined using Fisher's method and corrected for multiple testing using FDR (Supplementary Table 16A).

### Partitioning the genome into discrete bins

A genome-wide screening of recurrence in 1kb non-overlapping bins was performed. The method described in the earlier section was employed, using as covariate the local mutation rate calculated from 5Mb up and downstream from the bin of interest and excluding any low-coverage region from the estimate (Supplementary Table 16B, Figure 9A for example). Significant hits were subjected to manual curation to remove false positives caused by sequencing or mapping artefacts.

### Mutational signatures analysis

Mutational signatures analysis was performed following a three-step process: (i) hierarchical *de novo* extraction based on somatic substitutions and their immediate sequence context, (ii) updating the set of consensus signatures using the mutational signatures extracted from breast cancer genomes, and (iii) evaluating the contributions of each of the updated consensus signatures in each of the breast cancer samples. These three steps are discussed in more detail in the next sections.

### Hierarchical de novo extraction of mutational signatures

The mutational catalogues of the 560 breast cancer whole genomes were analysed for mutational signatures using a hierarchical version of the Wellcome Trust Sanger Institute mutational signatures framework [25]. Briefly, we converted all mutation data into a matrix, *M*, that is made up of 96 features comprising mutations counts for each mutation type (C>A, C>G, C>T, T>A, T>C, and T>G; all substitutions are referred to by the pyrimidine of the mutated Watson-Crick base pair) using each possible 5' (C, A, G, and T) and 3' (C, A, G, and T) context for all samples. After conversion, the previously developed algorithm was applied in a hierarchical manner to the matrix *M* that contains *K* mutation types and *G* samples. The algorithm deciphers the minimal set of mutational signatures that optimally explains the proportion of each mutation type and then estimates the contribution of each signature across the samples. More specifically, the algorithm makes use of a well-known blind source separation technique, termed nonnegative matrix factorization (NMF). NMF identifies the matrix of mutational signature, *P*, and the matrix of the exposures of these signatures, *E*, by minimizing a Frobenius norm while maintaining non-negativity:

$$\min_{P \in \mathbb{M}_{\mathbb{R}_+}^{(K,N)} \; E \in \mathbb{M}_{\mathbb{R}_+}^{(N,G)}} ||M - P \times E||_F^2$$

The method for deciphering mutational signatures, including evaluation with simulated data and list of limitations, can be found in [25]. The framework was applied in a hierarchical manner to increase its ability to find mutational signatures present in few samples as well as mutational signatures exhibiting a low mutational burden. More specifically, after application to the original matrix Mcontaining 560 samples, we evaluated the accuracy of explaining the mutational patterns of each of the 560 breast cancers with the extracted mutational signatures. All samples that were well explained by the extracted mutational signatures were removed and the framework was applied to the remaining sub-matrix of *M*. This procedure was repeated until the extraction process did not reveal any new mutational signatures. Overall, the approach extracted 12 unique mutational signatures operative across the 560 breast cancers (Figure 5, Supplementary Table 21).

### Updating the set of consensus mutational signatures

The 12 hierarchically extracted breast cancer signatures were compared to the census of consensus mutational signatures [25]. 11 of the 12 signatures closely resembled previously identified mutational patterns. The patterns of these 11 signatures, weighted by the numbers of mutations contributed by each signature in the breast cancer data, were used to update the set of consensus mutational signatures as previously done in [25]. 1 of the 12 extracted signatures is novel and at present, unique for breast cancer. This novel signature is consensus signature 30 (http://cancer.sanger.ac.uk/cosmic/signatures).

### Evaluating the contributions of consensus mutational signatures in 560 breast cancers

The complete compendium of consensus mutational signatures that was found in breast cancer includes: signatures 1, 2, 3, 5, 6, 8, 13, 17, 18, 20, 26, and 30. We evaluated the presence of all these signatures in the 560 breast cancer genomes by re-introducing them into each sample. More specifically, the updated set of consensus mutational signatures was used to minimize the constrained linear function for each sample:

$$\min_{Exposures_i \geq 0} ||SampleMutations - \sum_{i=1}^{N} (\overrightarrow{Signature_i} * Exposure_i)||_F^2$$

Here, $\overrightarrow{Signature_i}$ represents a vector with 96 components (corresponding to a consensus mutational signature with its six somatic substitutions and their immediate sequencing context) and $Exposure_i$ is a nonnegative scalar reflecting the number of mutations contributed by this signature. $N$ is equal to 12 and it reflects the number of all possible signatures that can be found in a single breast cancer sample. Mutational signatures that did not contribute large numbers (or proportions) of mutations or that did not significantly improve the correlation between the original mutational pattern of the sample and the one generated by the mutational signatures were excluded from the sample. This procedure reduced over-fitting the data and allowed only the essential mutational signatures to be present in each sample (Supplementary Table 21B).

### Kataegis

Kataegis or foci of localized hypermutation has been previously defined [25] as 6 or more consecutive mutations with an average intermutation distance of less than or equal to 1,000 bp. Kataegis were sought in 560 whole-genome sequenced breast cancers from high-quality base substitution data using the method described previously [25]. This method likely misses some foci of kataegis sacrificing sensitivity of detection for a higher positive predictive value of kataegic foci (Supplementary Table 21C).

### Rearrangement signatures

### Clustered vs non-clustered rearrangements

Rearrangements that occurred as focal catastrophic events or focal driver amplicons were separated from genome-wide rearrangement mutagenesis using a piecewise constant fitting (PCF) method. For each sample, both breakpoints of each rearrangement were considered individually and all breakpoints were ordered by chromosomal position. The inter-rearrangement distance, defined as the number of base pairs from one rearrangement breakpoint to the one immediately preceding it in the reference genome, was calculated. Putative regions of clustered rearrangements were identified as having an average inter-rearrangement distance that was at least 10 times greater than the whole genome average for the individual sample. PCF parameters used were $\gamma$ = 25 and kmin = 10. The respective partner breakpoint of all breakpoints involved in a clustered region are likely to have arisen at the same mechanistic instant and so were considered as being involved in the cluster even if located at a distant chromosomal site. Extended Data Table 4A summarises the rearrangements within clusters ("clustered") and not within clusters ("non-clustered").

### Classification - types and size

In both classes of rearrangements, clustered and non-clustered, rearrangements were subclassified into deletions, inversions and tandem duplications, and then further subclassified according to size of the rearranged segment (1-10kb, 10kb-100kb, 100kb-1Mb, 1Mb-10Mb, more than 10Mb). The final category in both groups was interchromosomal translocations.

*Rearrangement signatures by NNMF*

The classification produces a matrix of 32 distinct categories of structural variants across 544 breast cancer genomes. This matrix was decomposed using the previously developed approach for deciphering mutational signatures by searching for the optimal number of mutational signatures that best explains the data without over-fitting the data [25] (Supplementary Table 21D-E).

*Consensus clustering of rearrangement signatures*

To identify subgroups of samples sharing similar combinations of six identified rearrangement signatures derived from whole genome sequencing analysis consensus clustering was performed using the ConsensusClusterPlus R package [56]. Input data for each sample (n=544, a subset of the full sample cohort) was the proportion of rearrangements assigned to each of the six signatures. Thus, each sample has 6 data values, with a total sum of 1. Proportions for each signature were mean-centred across samples prior to clustering. The following settings were used in the consensus clustering:

- Number of repetitions: 1000

- pItem = 0.9 (resampling frequency samples)

- pFeature = 0.9 (resampling frequency)

- Pearson distance metric

- Ward linkage method

**Individual patient whole genome profiles**

Breast cancer whole genome profiles were adapted from the R Circos package [57]. Features depicted in circos plots from outermost rings heading inwards: Karyotypic ideogram outermost. Base substitutions next, plotted as rainfall plots (log10 intermutation distance on radial axis, dot colours: blue=C>A, black=C>G, red=C>T, grey=T>A, green=T>C, pink=T>G). Ring with short green lines = insertions, ring with short red lines = deletions. Major copy number allele (green = gain) ring, minor copy number allele ring (pink=loss), Central lines represent rearrangements (green= tandem duplications, pink=deletions, blue=inversions and gray=interchromosomal events. Top right hand panel displays the number of mtations contributing to each mutation signature extracted using NNMF in individual cancers. Middle right hand panel represents indels. Bottom right corner shows histogram of rearrangements present in this cancer. Bottom left corner shows all curated driver mutations, top and middle left panels show clinical and pathology data respectively.

The systems and methods of the above embodiments may be implemented in a computer system (in particular in computer hardware or in computer software) in addition to the structural components and user interactions described.

The term "computer system" includes the hardware, software and data storage devices for embodying a system or carrying out a method according to the above described embodiments. For example, a computer system may comprise a central processing unit (CPU), input means, output means and data storage. Preferably the computer system has a monitor to provide a visual output display (for example in the design of the business process). The data storage may comprise RAM, disk drives or other computer readable media. The computer system may include a plurality of computing devices connected by a network and able to communicate with each other over that network.

The methods of the above embodiments may be provided as computer programs or as computer program products or computer readable media carrying a computer program which is arranged, when run on a computer, to perform the method(s) described above.

The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

TABLE 1

| Type | Class | Size | Probability | | | | | |
|------|-------|------|-------------|---|---|---|---|---|
| | | | Signature 1 | Signature 2 | Signature 3 | Signature 4 | Signature 5 | Signature 6 |
| clustered | deletion | 1-10kb | 0% | 0% | 0% | 1% | 0% | 1% |
| clustered | deletion | 10-100kb | 0% | 0% | 0% | 1% | 0% | 1% |
| clustered | deletion | 100kb-1-Mb | 0% | 0% | 0% | 2% | 0% | 3% |
| clustered | deletion | 1Mb-10-Mb | 0% | 0% | 0% | 3% | 0% | 7% |
| clustered | deletion | >10Mb | 0% | 0% | 0% | 1% | 0% | 7% |
| clustered | tandem duplication | 1-10kb | 0% | 0% | 0% | 0% | 0% | 0% |
| clustered | tandem duplication | 10-100kb | 0% | 0% | 0% | 1% | 0% | 1% |
| clustered | tandem du-plication | 100kb-1-Mb | 1% | 0% | 0% | 1% | 0% | 3% |
| clustered | tandem du-plication | 1Mb-10-Mb | 0% | 0% | 0% | 3% | 0% | 7% |
| clustered | tandem duplication | >10Mb | 0% | 0% | 0% | 1% | 0% | 7% |
| clustered | inversion | 1-10kb | 0% | 0% | 0% | 3% | 0% | 2% |
| clustered | inversion | 10-100kb | 0% | 0% | 0% | 2% | 0% | 2% |
| clustered | inversion | 100kb-1-Mb | 0% | 0% | 0% | 3% | 0% | 5% |
| clustered | inversion | 1Mb-10-Mb | 0% | 0% | 0% | 6% | 0% | 15% |
| clustered | inversion | >10Mb | 0% | 0% | 0% | 2% | 0% | 14% |
| clustered | translocation | | 0% | 0% | 0% | 56% | 0% | 0% |
| non-clustered | deletion | 1-10kb | 0% | 2% | 2% | 0% | 32% | 3% |
| non-clustered | deletion | 10-100kb | 1% | 1% | 0% | 0% | 22% | 2% |
| non-clus-tered | deletion | 100kb-1-Mb | 4% | 5% | 0% | 0% | 5% | 2% |
| non-clus-tered | deletion | 1Mb-10-Mb | 1% | 6% | 0% | 1% | 1% | 2% |
| non-clustered | deletion | >10Mb | 0% | 6% | 1% | 0% | 1% | 2% |
| non-clus-tered | tandem du-plication | 1-10kb | 0% | 0% | 53% | 0% | 1% | 0% |
| non-clus-tered | tandem du-plication | 10-100kb | 16% | 0% | 22% | 0% | 12% | 0% |
| non-clus-tered | tandem du-plication | 100kb-1-Mb | 54% | 0% | 1% | 0% | 1% | 0% |
| non-clus-tered | tandem du-plication | 1Mb-10-Mb | 17% | 2% | 0% | 1% | 0% | 1% |

(continued)

| Type | Class | Size | Probability | | | | | |
|---|---|---|---|---|---|---|---|---|
| | | | Signature 1 | Signature 2 | Signature 3 | Signature 4 | Signature 5 | Signature 6 |
| non-clustered | tandem duplication | >10Mb | 0% | 5% | 1% | 0% | 1% | 1% |
| non-clustered | inversion | 1-10kb | 1% | 5% | 1% | 1% | 5% | 1% |
| non-clustered | inversion | 10-100kb | 2% | 2% | 0% | 0% | 3% | 1% |
| non-clustered | inversion | 100kb-1-Mb | 2% | 4% | 0% | 0% | 0% | 1% |
| non-clustered | inversion | 1Mb-10-Mb | 0% | 10% | 0% | 1% | 0% | 4% |
| non-clustered | inversion | >10Mb | 1% | 12% | 1% | 0% | 2% | 3% |
| non-clustered | translocation | | 1% | 39% | 16% | 7% | 13% | 1% |

**Table 2**

| sample | substitutions | insertion/deletions | rearrangements |
|---|---|---|---|
| PD10010a | 2431 | 205 | 108 |
| PD10011a | 8272 | 246 | 144 |
| PD10014a | 12125 | 633 | 221 |
| PD11326a | 9628 | 491 | 360 |
| PD11327a | 12715 | 350 | 274 |
| PD11336a | 5585 | 215 | 74 |
| PD11337a | 3073 | 213 | 77 |
| PD11338a | 2300 | 221 | 2 |
| PD11339a | 2529 | 300 | 63 |
| PD11340a | 5064 | 445 | 349 |
| PD11341a | 1652 | 81 | 40 |
| PD11342a | 3305 | 173 | 17 |
| PD11343a | 2944 | 148 | 122 |
| PD11344a | 11028 | 122 | 165 |
| PD11345a | 10351 | 495 | 451 |
| PD11346a | 2292 | 174 | 471 |
| PD11347a | 2031 | 146 | 146 |
| PD11348a | 5010 | 262 | 130 |
| PD11349a | 7449 | 424 | 451 |
| PD11352a | 2586 | 221 | 2 |
| PD11355a | 1838 | 192 | 42 |
| PD11357a | 3379 | 435 | 18 |
| PD11358a | 4327 | 304 | 0 |

(continued)

| sample | substitutions | insertion/deletions | rearrangements |
|---|---|---|---|
| PD11359a | 2666 | 245 | 1 |
| PD11360a | 3116 | 274 | 87 |
| PD11361a | 1723 | 111 | 5 |
| PD11364a | 2339 | 214 | 23 |
| PD11365a | 13652 | 3088 | 27 |
| PD11366a | 3005 | 306 | 32 |
| PD11367a | 8164 | 418 | 104 |
| PD11368a | 6828 | 495 | 333 |
| PD11369a | 6134 | 438 | 308 |
| PD11370a | 1279 | 106 | 3 |
| PD11372a | 13793 | 370 | 57 |
| PD11374a | 2383 | 157 | 70 |
| PD11375a | 1934 | 146 | 7 |
| PD11376a | 3152 | 293 | 64 |
| PD11379a | 19382 | 436 | 115 |
| PD11380a | 2720 | 314 | 32 |
| PD11381a | 2097 | 191 | 29 |
| PD11383a | 2579 | 243 | 4 |
| PD11384a | 1725 | 131 | 5 |
| PD11385a | 1887 | 172 | 76 |
| PD11386a | 3382 | 209 | 16 |
| PD11388a | 4989 | 270 | 156 |
| PD11389a | 1677 | 205 | 1 |
| PD11391a | 1274 | 105 | 2 |
| PD11393a | 4669 | 299 | 157 |
| PD11394a | 2209 | 212 | 26 |
| PD11395a | 3792 | 366 | 25 |
| PD11396a | 2435 | 234 | 46 |
| PD11397a | 4827 | 223 | 100 |
| PD11398a | 2347 | 150 | 43 |
| PD11399a | 3496 | 185 | 125 |
| PD11402a | 2636 | 236 | 31 |
| PD11462a | 2839 | 392 | 149 |
| PD11464a | 4497 | 144 | 154 |
| PD11465a | 22551 | 762 | 208 |
| PD11740a | 2334 | 184 | 15 |
| PD11741a | 1976 | 104 | 34 |
| PD11742a | 7005 | 581 | 334 |
| PD11743a | 3309 | 192 | 82 |

(continued)

| sample | substitutions | insertion/deletions | rearrangements |
|--------|---------------|---------------------|----------------|
| PD11744a | 1882 | 139 | 1 |
| PD11745a | 3018 | 169 | 154 |
| PD11748a | 11761 | 462 | 401 |
| PD11750a | 10078 | 264 | 217 |
| PD11751a | 16648 | 1074 | 513 |
| PD11752a | 7636 | 458 | 246 |
| PD11753a | 6858 | 372 | 10 |
| PD11755a | 2776 | 117 | 130 |
| PD11756a | 1771 | 113 | 17 |
| PD11757a | 1952 | 40 | 94 |
| PD11760a | 4091 | 338 | 76 |
| PD11761a | 2707 | 163 | 70 |
| PD11762a | 1734 | 105 | 16 |
| PD11765a | 2002 | 106 | 42 |
| PD11766a | 1695 | 90 | 81 |
| PD11767a | 869 | 48 | 6 |
| PD11769a | 1722 | 196 | 53 |
| PD11816a | 4011 | 222 | 57 |
| PD11818a | 1605 | 108 | 301 |
| PD11819a | 1686 | 101 | 2 |
| PD13162a | 5404 | 198 | 136 |
| PD13163a | 5353 | 649 | 172 |
| PD13164a | 2722 | 213 | 85 |
| PD13165a | 6341 | 371 | 324 |
| PD13166a | 2331 | 274 | 172 |
| PD13167a | 2405 | 158 | 242 |
| PD13168a | 2528 | 116 | 178 |
| PD13296a | 18582 | 970 | 1221 |
| PD13297a | 14982 | 947 | 322 |
| PD13298a | 8367 | 536 | 233 |
| PD13299a | 10735 | 497 | 278 |
| PD13302a | 4896 | 425 | 267 |
| PD13304a | 1517 | 179 | 0 |
| PD13306a | 2026 | 131 | 18 |
| PD13307a | 5353 | 213 | 78 |
| PD13310a | 1831 | 191 | 1 |
| PD13311a | 4510 | 91 | 145 |
| PD13312a | 2034 | 278 | 21 |
| PD13416a | 1939 | 182 | 0 |

(continued)

| sample | substitutions | insertion/deletions | rearrangements |
|---|---|---|---|
| PD13418a | 6808 | 288 | 49 |
| PD13419a | 1092 | 74 | 25 |
| PD13420a | 1862 | 125 | 16 |
| PD13422a | 1805 | 178 | 10 |
| PD13424a | 3954 | 259 | 217 |
| PD13425a | 34502 | 719 | 617 |
| PD13426a | 2699 | 239 | 0 |
| PD13427a | 1134 | 90 | 2 |
| PD13428a | 3422 | 283 | 49 |
| PD13602a | 5914 | 446 | 231 |
| PD13603a | 3064 | 224 | 309 |
| PD13604a | 93102 | 586 | 148 |
| PD13605a | 1238 | 108 | 162 |
| PD13606a | 5893 | 589 | 336 |
| PD13607a | 3288 | 264 | 40 |
| PD13608a | 4107 | 186 | 175 |
| PD13609a | 4706 | 143 | 478 |
| PD13618a | 1833 | 107 | 0 |
| PD13619a | 2112 | 188 | 2 |
| PD13620a | 4598 | 640 | 156 |
| PD13622a | 4355 | 258 | 256 |
| PD13623a | 3800 | 198 | 10 |
| PD13625a | 11239 | 714 | 242 |
| PD13626a | 1076 | 101 | 38 |
| PD13627a | 8442 | 276 | 184 |
| PD13629a | 2029 | 176 | 2 |
| PD13630a | 4107 | 216 | 85 |
| PD13631a | 2603 | 146 | 19 |
| PD13752a | 2809 | 231 | 58 |
| PD13753a | 7995 | 124 | 11 |
| PD13754a | 2506 | 137 | 20 |
| PD13755a | 1936 | 155 | 1 |
| PD13756a | 2083 | 205 | 0 |
| PD13757a | 1487 | 48 | 24 |
| PD13758a | 2299 | 188 | 25 |
| PD13760a | 4634 | 186 | 10 |
| PD13761a | 1688 | 165 | 1 |
| PD13762a | 1653 | 145 | 1 |
| PD13763a | 2563 | 199 | 67 |

(continued)

| sample | substitutions | insertion/deletions | rearrangements |
|---|---|---|---|
| PD13764a | 4830 | 166 | 989 |
| PD13765a | 5479 | 386 | 108 |
| PD13766a | 3837 | 121 | 67 |
| PD13767a | 2638 | 206 | 25 |
| PD13768a | 1352 | 140 | 1 |
| PD13770a | 2079 | 106 | 34 |
| PD13771a | 13089 | 845 | 280 |
| PD14432a | 1870 | 192 | 13 |
| PD14433a | 3582 | 231 | 54 |
| PD14435a | 3716 | 273 | 200 |
| PD14437a | 4363 | 258 | 217 |
| PD14439a | 1316 | 154 | 5 |
| PD14441a | 4662 | 255 | 32 |
| PD14442a | 1746 | 116 | 2 |
| PD14450a | 1298 | 102 | 45 |
| PD14453a | 9916 | 498 | 318 |
| PD14454a | 2510 | 122 | 129 |
| PD14456a | 1977 | 139 | 26 |
| PD14457a | 4567 | 262 | 182 |
| PD14458a | 1513 | 153 | 0 |
| PD14459a | 2086 | 164 | 31 |
| PD14460a | 4524 | 220 | 58 |
| PD14461a | 1436 | 137 | 38 |
| PD14462a | 2628 | 152 | 29 |
| PD14465a | 3097 | 257 | 99 |
| PD14467a | 1624 | 112 | 52 |
| PD14468a | 1549 | 152 | 15 |
| PD14471a | 3270 | 245 | 115 |
| PD14472a | 2298 | 220 | 7 |
| PD14473a | 1543 | 90 | 38 |
| PD17973a | 2025 | 141 | 14 |
| PD17981a | 6964 | 318 | 44 |
| PD17991a | 1222 | 121 | 3 |
| PD17994a | 1306 | 62 | 7 |
| PD18017a | 2108 | 114 | 60 |
| PD18020a | 17772 | 336 | 446 |
| PD18022a | 1602 | 74 | 113 |
| PD18024a | 11104 | 340 | 667 |
| PD18031a | 2769 | 173 | 93 |

(continued)

| sample | substitutions | insertion/deletions | rearrangements |
|---|---|---|---|
| PD18037a | 5971 | 455 | 197 |
| PD18045a | 19468 | 506 | 570 |
| PD18046a | 2901 | 528 | 39 |
| PD18047a | 2344 | 246 | 88 |
| PD18048a | 4786 | 316 | 198 |
| PD18049a | 3392 | 231 | 50 |
| PD18050a | 3566 | 185 | 299 |
| PD18100a | 1531 | 90 | 8 |
| PD18101a | 863 | 67 | 9 |
| PD18116a | 1544 | 112 | 9 |
| PD18149a | 2570 | 293 | 21 |
| PD18188a | 2236 | 139 | 52 |
| PD18189a | 4272 | 239 | 73 |
| PD18247a | 9136 | 110 | 3 |
| PD18251a | 3487 | 175 | 125 |
| PD18257a | 2973 | 130 | 51 |
| PD18258a | 1142 | 119 | 8 |
| PD18259a | 6639 | 419 | 78 |
| PD18264a | 6680 | 122 | 40 |
| PD18269a | 1387 | 94 | 2 |
| PD18728a | 1769 | 166 | 2 |
| PD18730a | 1901 | 94 | 20 |
| PD18733a | 3019 | 258 | 33 |
| PD18734a | 3828 | 188 | 318 |
| PD18748a | 2958 | 152 | 19 |
| PD18749a | 4661 | 150 | 2 |
| PD18751a | 1907 | 119 | 71 |
| PD18754a | 2593 | 176 | 21 |
| PD18756a | 1635 | 101 | 2 |
| PD18768a | 2556 | 236 | 2 |
| PD18769a | 2982 | 76 | 21 |
| PD18771a | 1776 | 120 | 8 |
| PD18775a | 1330 | 84 | 11 |
| PD18776a | 1629 | 115 | 13 |
| PD22036a | 1047 | 34 | 0 |
| PD22251a | 1505 | 124 | 39 |
| PD22355a | 9761 | 632 | 473 |
| PD22357a | 7719 | 298 | 276 |
| PD22358a | 6158 | 123 | 61 |

(continued)

| sample | substitutions | insertion/deletions | rearrangements |
|---|---|---|---|
| PD22359a | 1817 | 76 | 33 |
| PD22360a | 8510 | 150 | 154 |
| PD22361a | 5899 | 236 | 129 |
| PD22362a | 1949 | 59 | 158 |
| PD22363a | 8905 | 332 | 132 |
| PD22364a | 2402 | 152 | 40 |
| PD22365a | 1906 | 60 | 49 |
| PD22366a | 9809 | 388 | 260 |
| PD23550a | 2866 | 113 | 271 |
| PD23554a | 6899 | 102 | 131 |
| PD23558a | 10134 | 620 | 148 |
| PD23559a | 5382 | 204 | 84 |
| PD23560a | 3006 | 117 | 77 |
| PD23561a | 58028 | 2535 | 74 |
| PD23562a | 7348 | 448 | 221 |
| PD23563a | 8223 | 317 | 215 |
| PD23564a | 83447 | 66764 | 9 |
| PD23565a | 8852 | 252 | 174 |
| PD23566a | 6667 | 289 | 265 |
| PD23567a | 7198 | 381 | 255 |
| PD23569a | 1055 | 33 | 21 |
| PD23570a | 2245 | 120 | 28 |
| PD23574a | 13094 | 386 | 326 |
| PD23577a | 3970 | 309 | 199 |
| PD23578a | 6840 | 195 | 220 |
| PD23579a | 87311 | 55849 | 9 |
| PD24182a | 9537 | 293 | 196 |
| PD24186a | 7488 | 238 | 113 |
| PD24189a | 29590 | 35971 | 8 |
| PD24190a | 9808 | 381 | 226 |
| PD24191a | 6120 | 656 | 56 |
| PD24192a | 17827 | 1017 | 225 |
| PD24193a | 11237 | 4282 | 2 |
| PD24194a | 13695 | 220 | 51 |
| PD24195a | 2240 | 77 | 14 |
| PD24196a | 2620 | 81 | 142 |
| PD24197a | 20215 | 423 | 319 |
| PD24199a | 2854 | 64 | 131 |
| PD24200a | 974 | 19 | 47 |

(continued)

| sample | substitutions | insertion/deletions | rearrangements |
|---|---|---|---|
| PD24201a | 20478 | 1095 | 389 |
| PD24202a | 7779 | 332 | 292 |
| PD24204a | 2795 | 201 | 5 |
| PD24205a | 8928 | 781 | 83 |
| PD24206a | 3610 | 164 | 104 |
| PD24207a | 4745 | 201 | 347 |
| PD24208a | 25624 | 130 | 62 |
| PD24209a | 10169 | 209 | 73 |
| PD24212a | 10055 | 807 | 235 |
| PD24214a | 3079 | 212 | 213 |
| PD24215a | 6177 | 678 | 63 |
| PD24216a | 9339 | 232 | 59 |
| PD24217a | 15305 | 44 | 110 |
| PD24218a | 3288 | 350 | 95 |
| PD24219a | 3319 | 169 | 100 |
| PD24220a | 3611 | 273 | 383 |
| PD24221a | 2293 | 103 | 12 |
| PD24223a | 3428 | 185 | 12 |
| PD24224a | 2212 | 90 | 43 |
| PD24225a | 1821 | 51 | 31 |
| PD24302a | 1268 | 88 | 5 |
| PD24303a | 4500 | 69 | 165 |
| PD24304a | 6853 | 185 | 91 |
| PD24306a | 3336 | 81 | 107 |
| PD24307a | 1594 | 89 | 50 |
| PD24308a | 4752 | 290 | 221 |
| PD24314a | 3870 | 182 | 143 |
| PD24318a | 2546 | 162 | 94 |
| PD24320a | 15695 | 3840 | 61 |
| PD24322a | 4868 | 202 | 98 |
| PD24325a | 10387 | 479 | 200 |
| PD24326a | 23620 | 252 | 61 |
| PD24327a | 16185 | 253 | 247 |
| PD24329a | 2111 | 152 | 94 |
| PD24332a | 1920 | 104 | 79 |
| PD24333a | 22884 | 396 | 49 |
| PD24335a | 3948 | 246 | 434 |
| PD24336a | 3339 | 180 | 81 |
| PD24337a | 5707 | 360 | 460 |

(continued)

| sample | substitutions | insertion/deletions | rearrangements |
|--------|---------------|---------------------|----------------|
| PD3851a | 1819 | 84 | 32 |
| PD3890a | 5584 | 170 | 166 |
| PD3904a | 5668 | 649 | 161 |
| PD3905a | 4603 | 124 | 139 |
| PD3945a | 10551 | 500 | 104 |
| PD3989a | 2238 | 149 | 14 |
| PD4005a | 6041 | 151 | 184 |
| PD4006a | 9575 | 288 | 351 |
| PD4069a | 2085 | 205 | 3 |
| PD4072a | 27931 | 135 | 1 |
| PD4076a | 2091 | 123 | 2 |
| PD4085a | 2704 | 110 | 66 |
| PD4086a | 1951 | 68 | 49 |
| PD4088a | 1691 | 77 | 171 |
| PD4103a | 5218 | 266 | 613 |
| PD4107a | 9874 | 389 | 291 |
| PD4109a | 10313 | 840 | 181 |
| PD4115a | 9752 | 821 | 129 |
| PD4116a | 8170 | 486 | 256 |
| PD4192a | 4292 | 72 | 102 |
| PD4194a | 1428 | 34 | 23 |
| PD4198a | 4517 | 185 | 220 |
| PD4199a | 6948 | 133 | 85 |
| PD4225a | 1955 | 185 | 46 |
| PD4248a | 2196 | 136 | 141 |
| PD4252a | 3773 | 431 | 150 |
| PD4255a | 4554 | 383 | 311 |
| PD4261a | 1796 | 138 | 24 |
| PD4264a | 2118 | 148 | 70 |
| PD4266a | 2388 | 137 | 7 |
| PD4267a | 2119 | 113 | 0 |
| PD4315a | 3388 | 195 | 209 |
| PD4604a | 9323 | 354 | 201 |
| PD4605a | 2650 | 187 | 98 |
| PD4606a | 1129 | 72 | 48 |
| PD4607a | 25953 | 157 | 18 |
| PD4613a | 2195 | 109 | 24 |
| PD4826a | 3818 | 260 | 293 |
| PD4833a | 6401 | 626 | 540 |

(continued)

| sample | substitutions | insertion/deletions | rearrangements |
|---|---|---|---|
| PD4836a | 4303 | 317 | 80 |
| PD4841a | 7020 | 461 | 607 |
| PD4844a | 12810 | 1065 | 216 |
| PD4845a | 5735 | 439 | 155 |
| PD4847a | 11042 | 1314 | 252 |
| PD4872a | 4810 | 224 | 90 |
| PD4874a | 9709 | 240 | 222 |
| PD4875a | 6805 | 310 | 138 |
| PD4876a | 4908 | 325 | 95 |
| PD4951a | 2779 | 200 | 33 |
| PD4952a | 12033 | 1069 | 333 |
| PD4953a | 6946 | 355 | 143 |
| PD4954a | 5764 | 371 | 127 |
| PD4955a | 6070 | 736 | 102 |
| PD4956a | 11582 | 917 | 236 |
| PD4957a | 3279 | 171 | 74 |
| PD4958a | 9338 | 486 | 102 |
| PD4959a | 4611 | 370 | 103 |
| PD4962a | 6843 | 143 | 85 |
| PD4965a | 2706 | 176 | 33 |
| PD4967a | 3227 | 113 | 9 |
| PD4968a | 4780 | 327 | 213 |
| PD4969a | 1032 | 63 | 18 |
| PD4970a | 2410 | 126 | 31 |
| PD4971a | 2765 | 110 | 383 |
| PD4972a | 1145 | 86 | 0 |
| PD4975a | 7506 | 234 | 76 |
| PD4976a | 6275 | 471 | 118 |
| PD4977a | 42258 | 500 | 16 |
| PD4978a | 3736 | 155 | 555 |
| PD4980a | 3796 | 364 | 87 |
| PD4981a | 1663 | 116 | 2 |
| PD4982a | 1474 | 156 | 0 |
| PD4983a | 1763 | 128 | 0 |
| PD4985a | 2004 | 173 | 2 |
| PD4986a | 1976 | 499 | 9 |
| PD5925a | 8780 | 276 | 92 |
| PD5928a | 7114 | 571 | 80 |
| PD5930a | 3946 | 144 | 140 |

(continued)

| sample | substitutions | insertion/deletions | rearrangements |
|--------|---------------|---------------------|----------------|
| PD5932a | 10060 | 550 | 320 |
| PD5934a | 11579 | 611 | 453 |
| PD5935a | 9671 | 636 | 335 |
| PD5936a | 2782 | 282 | 130 |
| PD5937a | 76097 | 19436 | 8 |
| PD5942a | 5990 | 384 | 161 |
| PD5944a | 2609 | 303 | 24 |
| PD5945a | 8833 | 497 | 434 |
| PD5946a | 3912 | 312 | 403 |
| PD5947a | 1779 | 102 | 151 |
| PD5948a | 15172 | 1052 | 489 |
| PD5950a | 2190 | 137 | 229 |
| PD5951a | 3193 | 215 | 116 |
| PD5953a | 2136 | 533 | 19 |
| PD5956a | 4085 | 445 | 692 |
| PD5959a | 2504 | 128 | 309 |
| PD5960a | 4045 | 274 | 62 |
| PD5961a | 2314 | 152 | 104 |
| PD5964a | 1590 | 92 | 69 |
| PD6016a2 | 1911 | 95 | 1 |
| PD6041a | 1771 | 152 | 2 |
| PD6042a | 7119 | 274 | 163 |
| PD6043a | 22764 | 938 | 217 |
| PD6044a | 2573 | 244 | 61 |
| PD6045a | 2333 | 141 | 32 |
| PD6046a | 2976 | 281 | 212 |
| PD6047a | 9442 | 827 | 142 |
| PD6048a | 3102 | 257 | 343 |
| PD6404a | 6553 | 247 | 139 |
| PD6405a | 35317 | 628 | 214 |
| PD6406a | 5026 | 106 | 185 |
| PD6409a | 8810 | 183 | 163 |
| PD6410a | 7313 | 245 | 194 |
| PD6411a | 6675 | 147 | 187 |
| PD6412a | 25187 | 24891 | 82 |
| PD6413a | 4588 | 157 | 321 |
| PD6414a | 1761 | 117 | 7 |
| PD6415a | 9545 | 449 | 363 |
| PD6416a | 3331 | 100 | 72 |

(continued)

| sample | substitutions | insertion/deletions | rearrangements |
|--------|--------------|---------------------|----------------|
| PD6417a | 1546 | 134 | 137 |
| PD6418a | 2020 | 204 | 2 |
| PD6422a | 5181 | 293 | 362 |
| PD6466b | 1809 | 180 | 61 |
| PD6684a | 13677 | 663 | 297 |
| PD6711a2 | 1914 | 207 | 21 |
| PD6719a | 4971 | 153 | 107 |
| PD6720a | 2018 | 202 | 74 |
| PD6721a | 2231 | 138 | 45 |
| PD6722a | 10110 | 236 | 196 |
| PD6727b | 6679 | 410 | 271 |
| PD6728b | 2578 | 133 | 113 |
| PD6729a2 | 4872 | 372 | 120 |
| PD6730b | 10089 | 356 | 97 |
| PD6731a2 | 8549 | 134 | 99 |
| PD6732b | 2915 | 116 | 178 |
| PD6733b | 4676 | 202 | 93 |
| PD7066a | 3638 | 122 | 321 |
| PD7067a | 5980 | 144 | 226 |
| PD7069a | 5131 | 146 | 145 |
| PD7199a | 1130 | 88 | 69 |
| PD7201a | 1584 | 140 | 12 |
| PD7202a | 5141 | 371 | 452 |
| PD7203a | 1564 | 47 | 177 |
| PD7204a | 2176 | 175 | 44 |
| PD7205a | 7858 | 208 | 519 |
| PD7206a | 4740 | 181 | 127 |
| PD7207a | 2854 | 167 | 6 |
| PD7209a | 1665 | 136 | 1 |
| PD7210a | 1319 | 103 | 1 |
| PD7211a | 8286 | 544 | 605 |
| PD7214a | 1493 | 72 | 15 |
| PD7215a | 6384 | 244 | 259 |
| PD7217a | 9169 | 381 | 124 |
| PD7218a | 1474 | 131 | 1 |
| PD7219a | 11621 | 145 | 70 |
| PD7220a | 2709 | 231 | 39 |
| PD7221a | 2040 | 106 | 9 |
| PD7238a | 2217 | 219 | 46 |

(continued)

| sample | substitutions | insertion/deletions | rearrangements |
|---|---|---|---|
| PD7240a | 5188 | 261 | 150 |
| PD7243a | 6774 | 496 | 203 |
| PD7248a | 13042 | 613 | 367 |
| PD7249a | 4584 | 419 | 181 |
| PD7250a | 7890 | 430 | 344 |
| PD7304a | 2003 | 148 | 154 |
| PD7305a | 4756 | 309 | 330 |
| PD7306a | 6863 | 416 | 410 |
| PD7307a | 8424 | 135 | 329 |
| PD7316a | 5647 | 125 | 188 |
| PD7321a | 6055 | 488 | 452 |
| PD7322a | 2015 | 215 | 70 |
| PD7341a | 3090 | 84 | 245 |
| PD7344a | 722 | 38 | 38 |
| PD7426a | 18497 | 653 | 485 |
| PD7428a | 15950 | 694 | 386 |
| PD8609a | 5087 | 451 | 60 |
| PD8610a | 5098 | 217 | 142 |
| PD8611a | 6828 | 230 | 416 |
| PD8612a | 1968 | 146 | 43 |
| PD8614a | 1439 | 77 | 17 |
| PD8615a | 4270 | 121 | 77 |
| PD8617a | 1899 | 181 | 59 |
| PD8618a | 1506 | 132 | 8 |
| PD8619a | 3571 | 219 | 248 |
| PD8620a | 2657 | 141 | 2 |
| PD8621a | 8062 | 873 | 271 |
| PD8622a | 1809 | 348 | 49 |
| PD8623a | 2020 | 295 | 29 |
| PD8652a2 | 15856 | 476 | 387 |
| PD8660a2 | 26128 | 672 | 591 |
| PD8828a | 2061 | 61 | 12 |
| PD8830a | 8246 | 324 | 129 |
| PD8832a | 26409 | 1512 | 377 |
| PD8964a | 11151 | 976 | 247 |
| PD8965a | 4451 | 449 | 105 |
| PD8969a | 6820 | 492 | 126 |
| PD8973a | 5908 | 149 | 83 |
| PD8977a | 4046 | 427 | 32 |

(continued)

| sample | substitutions | insertion/deletions | rearrangements |
|--------|---------------|---------------------|----------------|
| PD8978a | 6670 | 876 | 133 |
| PD8979a | 4037 | 210 | 742 |
| PD8980a | 15459 | 803 | 544 |
| PD8981a | 1748 | 171 | 172 |
| PD8982a | 21557 | 690 | 511 |
| PD8984a | 9152 | 368 | 163 |
| PD8995a | 7586 | 634 | 117 |
| PD8996a | 3230 | 434 | 201 |
| PD8997a | 2831 | 159 | 338 |
| PD8998a | 4769 | 68 | 95 |
| PD8999a | 1347 | 138 | 85 |
| PD9000a | 9510 | 727 | 208 |
| PD9001a | 2705 | 289 | 38 |
| PD9002a | 8476 | 916 | 159 |
| PD9004a | 10625 | 564 | 284 |
| PD9009a | 15210 | 254 | 94 |
| PD9063a | 50045 | 233 | 85 |
| PD9064a | 12351 | 769 | 428 |
| PD9065a | 2210 | 154 | 29 |
| PD9067a | 1554 | 92 | 9 |
| PD9193a | 1682 | 78 | 306 |
| PD9464a | 5251 | 332 | 349 |
| PD9467a | 2463 | 161 | 33 |
| PD9539a | 3580 | 233 | 223 |
| PD9541a | 2706 | 184 | 43 |
| PD9544a | 1389 | 121 | 3 |
| PD9567a | 3512 | 164 | 118 |
| PD9568a | 30395 | 1259 | 215 |
| PD9569a | 2141 | 139 | 63 |
| PD9570a | 1406 | 80 | 2 |
| PD9571a | 4700 | 318 | 315 |
| PD9572a | 4083 | 129 | 17 |
| PD9573a | 1616 | 159 | 40 |
| PD9574a | 1634 | 102 | 21 |
| PD9575a | 6500 | 591 | 97 |
| PD9576a | 17838 | 1007 | 511 |
| PD9577a | 1624 | 161 | 60 |
| PD9578a | 1088 | 54 | 2 |
| PD9579a | 1634 | 53 | 47 |

(continued)

| sample | substitutions | insertion/deletions | rearrangements |
|---|---|---|---|
| PD9581a | 1616 | 254 | 0 |
| PD9582a | 2740 | 99 | 245 |
| PD9584a | 2753 | 177 | 146 |
| PD9585a | 10284 | 865 | 533 |
| PD9589a | 1413 | 91 | 16 |
| PD9591a | 1296 | 69 | 15 |
| PD9592a | 8496 | 412 | 228 |
| PD9593a | 1617 | 113 | 23 |
| PD9595a | 9722 | 626 | 223 |
| PD9597a | 2911 | 47 | 14 |
| PD9599a | 3295 | 272 | 131 |
| PD9600a | 2019 | 114 | 38 |
| PD9604a | 10669 | 4035 | 119 |
| PD9605a | 3373 | 255 | 61 |
| PD9606a | 1222 | 107 | 0 |
| PD9694a | 1712 | 65 | 50 |
| PD9696a | 5865 | 127 | 332 |
| PD9702a | 7861 | 395 | 470 |
| PD9752a | 3476 | 178 | 293 |
| PD9754a | 1897 | 203 | 46 |
| PD9755a | 1710 | 138 | 58 |
| PD9756a | 4212 | 167 | 52 |
| PD9759a | 2192 | 150 | 54 |
| PD9760a | 5006 | 209 | 141 |
| PD9761a | 2103 | 163 | 2 |
| PD9842a | 3201 | 215 | 1 |
| PD9843a | 2010 | 108 | 0 |
| PD9844a | 507 | 80 | 0 |
| PD9845a | 3147 | 208 | 29 |
| PD9847a | 2229 | 133 | 4 |
| **Grand Total** | **3479652** | **371993** | **77695** |

**Table 3**

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| TP53 | 4 | 263 | 70 | 32 | 123.409217 | 548.572852 | 386.126191 | 0 | 0 | 0 | 0 |
| CDH1 | 2 | 12 | 19 | 11 | 3.67105513 | 127.588633 | 101.520381 | 0.04043973 | 0 | 4.00E-14 | 0 |
| MAP3K1 | 4 | 19 | 19 | 0 | 2.5854755 | 38.7790438 | 0 | 0.07882551 | 0 | 1 | 0 |
| PTEN | 1 | 15 | 9 | 5 | 9.42779802 | 88.7665823 | 76.4774799 | 1.24E-05 | 1.97E-11 | 5.58E-06 | 0 |
| MAP2K4 | 0 | 19 | 6 | 4 | 12.9141653 | 68.2495193 | 50.0217428 | 1.49E-07 | 4.67E-07 | 0.00055925 | 0 |
| CBFB | 2 | 16 | 4 | 8 | 17.7603098 | 71.0968958 | 164.882381 | 2.21E-09 | 4.35E-05 | 3.04E-12 | 0 |
| PIK3CA | 3 | 386 | 0 | 0 | 74.1273212 | 0 | 0 | 0 | 1 | 1 | 0 |
| AKT1 | 1 | 36 | 0 | 0 | 19.5479087 | 0 | 0 | 0 | 1 | 1 | 0 |
| RB1 | 2 | 8 | 12 | 5 | 2.03072816 | 36.4530852 | 22.4514658 | 0.20472683 | 1.06E-10 | 0.0013377 | 2.67E-14 |
| NCOR1 | 3 | 13 | 17 | 3 | 1.43999904 | 23.881301 | 9.35105626 | 0.20472683 | 1.10E-11 | 0.3481657 | 1.48E-12 |
| MLL3 | 10 | 37 | 26 | 3 | 1.36703885 | 12.1558035 | 4.29517328 | 0.20472683 | 2.82E-12 | 1 | 1.56E-12 |
| CTCF | 4 | 17 | 5 | 2 | 4.48323755 | 23.8534585 | 22.2135963 | 0.00237053 | 0.00034414 | 0.24953143 | 1.99E-08 |
| ARID1A | 2 | 11 | 9 | 0 | 1.71418369 | 21.8857681 | 0 | 0.20472683 | 1.74E-06 | 1 | 1.46E-05 |
| MED23 | 0 | 8 | 6 | 2 | 1.83213623 | 19.8090684 | 10.900486 | 0.20472683 | 0.00034414 | 0.80456104 | 5.60E-05 |
| FOXA1 | 2 | 20 | 1 | 0 | 7.84632165 | 13.0148587 | 0 | 1.04E-05 | 0.6764284 | 1 | 5.78E-05 |
| TBX3 | 2 | 10 | 4 | 1 | 2.6915495 | 33.7765523 | 14.4769794 | 0.16306328 | 0.00071801 | 1 | 9.24E-05 |
| CDKN1B | 0 | 2 | 4 | 0 | 3.08919531 | 94.2490409 | 0 | 0.20472683 | 2.11E-05 | 1 | 0.00010703 |
| NF1 | 1 | 12 | 6 | 2 | 1.67695415 | 12.9892263 | 6.54641509 | 0.20472683 | 0.00222702 | 1 | 0.00065812 |
| CASP8 | 0 | 9 | 3 | 0 | 5.63994454 | 25.6984512 | 0 | 0.01639242 | 0.01295529 | 1 | 0.00066763 |
| BRCA2 | 0 | 11 | 7 | 1 | 1.47428032 | 13.2394614 | 7.99258957 | 0.20472683 | 0.00091508 | 1 | 0.00070737 |
| SF3B1 | 2 | 27 | 1 | 0 | 5.4321554 | 2.94257435 | 0 | 9.96E-05 | 1 | 1 | 0.0013107 |
| GATA3 | 6 | 12 | 2 | 1 | 3.50034998 | 13.6513113 | 15.1745466 | 0.03567495 | 0.22813069 | 1 | 0.0023884 |
| FOXP1 | 1 | 3 | 2 | 3 | 1.10155054 | 9.41674698 | 21.8435391 | 0.20661747 | 0.34760338 | 0.05815745 | 0.00427114 |
| BRCA1 | 1 | 10 | 3 | 2 | 1.93899891 | 8.67722623 | 15.8286107 | 0.20472683 | 0.2122473 | 0.40227659 | 0.00579244 |
| SPEN | 7 | 13 | 10 | 0 | 0.82303968 | 9.50219676 | 0 | 1 | 0.00012824 | 1 | 0.00611285 |
| RUNX1 | 3 | 8 | 1 | 2 | 2.93288755 | 7.41649088 | 26.08342 | 0.153771 | 0.75085288 | 0.22954759 | 0.00889968 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| ERBB2 | 3 | 23 | 0 | 0 | 5.0021794 | 0 | 0 | 0.00041745 | 1 | 1 | 0.01212771 |
| MLLT4 | 0 | 9 | 5 | 0 | 1.80716102 | 14.0674079 | 0 | 0.20472683 | 0.00554054 | 1 | 0.01430864 |
| KDM6A | 1 | 5 | 2 | 3 | 1.14023998 | 5.53242957 | 16.5897928 | 0.20472683 | 0.61733926 | 0.12099836 | 0.01451691 |
| HIST1H3B | 3 | 7 | 1 | 0 | 6.52866262 | 23.7358283 | 0 | 0.01293597 | 0.47568053 | 1 | 0.01457274 |
| TBL1XR1 | 2 | 5 | 1 | 2 | 2.14913055 | 7.38261034 | 15.1382362 | 0.20472683 | 0.73800513 | 0.40227659 | 0.04077154 |
| PTPRD | 3 | 21 | 4 | 1 | 2.05719041 | 6.07464902 | 2.62253245 | 0.18005426 | 0.21331233 | 1 | 0.05574899 |
| PRRX1 | 1 | 7 | 1 | 0 | 5.21213104 | 13.6262664 | 0 | 0.03652454 | 0.62861306 | 1 | 0.08611263 |
| ZFHX3 | 5 | 11 | 7 | 0 | 0.85983687 | 8.91210596 | 0 | 1 | 0.00337867 | 1 | 0.09726602 |
| FBXW7 | 0 | 8 | 2 | 0 | 3.41568879 | 10.3356307 | 0 | 0.11784311 | 0.32207162 | 1 | 0.10261303 |
| C11orf30 | 1 | 13 | 2 | 0 | 3.25441313 | 6.77166602 | 0 | 0.06969207 | 0.50732929 | 1 | 0.10939934 |
| KRAS | 0 | 6 | 0 | 0 | 9.13712049 | 0 | 0 | 0.00716467 | 1 | 1 | 0.11016706 |
| STK11 | 0 | 2 | 2 | 0 | 1.53241712 | 37.7720978 | 0 | 0.21676652 | 0.04760294 | 1 | 0.15403046 |
| PREX2 | 3 | 27 | 1 | 0 | 3.07810424 | 1.97038719 | 0 | 0.02044305 | 1 | 1 | 0.16753773 |
| ATM | 1 | 17 | 3 | 1 | 2.08141162 | 4.77413651 | 2.77855073 | 0.20453581 | 0.47568053 | 1 | 0.17134935 |
| ARNT | 0 | 5 | 2 | 0 | 2.37454195 | 14.5658769 | 0 | 0.20472683 | 0.21414141 | 1 | 0.20541186 |
| ERBB3 | 1 | 16 | 0 | 0 | 4.20153426 | 0 | 0 | 0.01557192 | 1 | 1 | 0.20541186 |
| UNC13C | 6 | 16 | 6 | 0 | 1.06007311 | 4.9405991 | 0 | 0.20472683 | 0.11548229 | 1 | 0.21415908 |
| ADAM29 | 2 | 11 | 2 | 0 | 2.52674122 | 7.08901824 | 0 | 0.153771 | 0.47568053 | 1 | 0.21415908 |
| NTRK1 | 1 | 12 | 0 | 0 | 4.78083356 | 0 | 0 | 0.01625294 | 1 | 1 | 0.21415908 |
| JAK1 | 1 | 4 | 2 | 1 | 1.01998718 | 9.56835531 | 6.24527888 | 0.20672869 | 0.35128699 | 1 | 0.2159993 |
| USP6 | 0 | 5 | 2 | 1 | 1.39266438 | 7.70204701 | 6.83180869 | 0.20472683 | 0.47568053 | 1 | 0.2159993 |
| COL2A1 | 0 | 4 | 0 | 3 | 1.02792734 | 0 | 11.8662958 | 0.20472683 | 1 | 0.24953143 | 0.2159993 |
| GRB7 | 0 | 9 | 0 | 0 | 5.63291284 | 0 | 0 | 0.01695079 | 1 | 1 | 0.22126512 |
| LIFR | 1 | 4 | 3 | 0 | 1.06522391 | 9.77240591 | 0 | 0.20472683 | 0.15592649 | 1 | 0.22739029 |
| PHF6 | 0 | 5 | 1 | 0 | 4.13649516 | 7.82519621 | 0 | 0.11784311 | 0.68272016 | 1 | 0.22739029 |
| PIK3R1 | 1 | 10 | 0 | 1 | 3.19300568 | 0 | 9.23867134 | 0.10284024 | 1 | 1 | 0.22739029 |

EP 3 452 937 B1

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| SMAD4 | 0 | 3 | 2 | 0 | 1.6258355 | 16.5862558 | 0 | 0.20472683 | 0.20343671 | 1 | 0.24860092 |
| SETD2 | 2 | 11 | 4 | 0 | 1.28764393 | 5.93994693 | 0 | 0.20472683 | 0.21414141 | 1 | 0.26855769 |
| MLH1 | 0 | 5 | 1 | 1 | 2.18961237 | 7.00705052 | 7.55444152 | 0.20472683 | 0.75085288 | 1 | 0.26855769 |
| AXL | 0 | 11 | 0 | 0 | 4.78808167 | 0 | 0 | 0.02301607 | 1 | 1 | 0.26855769 |
| EIF1AX | 0 | 2 | 1 | 0 | 3.46419437 | 30.5350059 | 0 | 0.20472683 | 0.37419133 | 1 | 0.28380774 |
| FLI1 | 2 | 7 | 0 | 1 | 3.1216072 | 0 | 13.1262857 | 0.153771 | 1 | 1 | 0.28380774 |
| RHOA | 1 | 5 | 0 | 0 | 7.26421487 | 0 | 0 | 0.02590215 | 1 | 1 | 0.28592545 |
| BCL2L2 | 0 | 1 | 0 | 1 | 1.68783089 | 0 | 119.560574 | 0.20672869 | 1 | 0.3481657 | 0.28592545 |
| PRKAR1A | 3 | 1 | 3 | 0 | 0.49145225 | 18.6842305 | 0 | 1 | 0.02452862 | 1 | 0.29117581 |
| EPS15 | 0 | 4 | 2 | 0 | 1.66473657 | 13.4808169 | 0 | 0.20472683 | 0.23750321 | 1 | 0.29216296 |
| FGFR2 | 1 | 12 | 0 | 0 | 4.13308918 | 0 | 0 | 0.02726226 | 1 | 1 | 0.29438728 |
| MLLT10 | 1 | 9 | 2 | 0 | 2.42197682 | 5.71722368 | 0 | 0.19565254 | 0.56753458 | 1 | 0.30074836 |
| PRKCG | 3 | 10 | 1 | 0 | 2.96929239 | 6.78471102 | 0 | 0.12241875 | 0.77298454 | 1 | 0.30074836 |
| PMS1 | 0 | 9 | 1 | 0 | 3.21188286 | 4.83820387 | 0 | 0.11784311 | 0.83151916 | 1 | 0.30376051 |
| AKT3 | 1 | 3 | 2 | 0 | 1.25961789 | 11.6539172 | 0 | 0.20472683 | 0.26984993 | 1 | 0.31767708 |
| NIN | 2 | 11 | 2 | 1 | 1.65574553 | 3.59677111 | 5.63863303 | 0.20472683 | 0.74470128 | 1 | 0.31767708 |
| NF2 | 1 | 3 | 1 | 1 | 1.46086989 | 7.19269678 | 9.3486614 | 0.20472683 | 0.74470128 | 1 | 0.31767708 |
| EIF4A2 | 1 | 5 | 0 | 1 | 3.02589708 | 0 | 11.3448132 | 0.18005426 | 1 | 1 | 0.31767708 |
| ETV5 | 0 | 5 | 0 | 1 | 3.04974655 | 0 | 13.9203481 | 0.19565254 | 1 | 1 | 0.31767708 |
| MAP3K13 | 2 | 7 | 1 | 1 | 1.76914009 | 3.39608314 | 10.6938068 | 0.20472683 | 1 | 1 | 0.31767708 |
| HSP90AA1 | 1 | 4 | 2 | 0 | 1.44592203 | 11.8227763 | 0 | 0.20472683 | 0.27713211 | 1 | 0.3435939 |
| CDKN2A | 0 | 2 | 1 | 0 | 2.67074502 | 40.5494038 | 0 | 0.20472683 | 0.33813743 | 1 | 0.34771013 |
| IL7R | 0 | 5 | 1 | 0 | 3.37287669 | 10.3170748 | 0 | 0.18746598 | 0.68272016 | 1 | 0.34771013 |
| CHEK2 | 0 | 8 | 0 | 0 | 4.67537773 | 0 | 0 | 0.03795388 | 1 | 1 | 0.34771013 |
| GRIN2A | 1 | 15 | 1 | 0 | 2.84361705 | 4.09289858 | 0 | 0.11784311 | 1 | 1 | 0.36072142 |
| STAG2 | 4 | 8 | 3 | 0 | 1.14853221 | 5.69862825 | 0 | 0.20472683 | 0.33813743 | 1 | 0.37104828 |

(continued)

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| ROS1 | 3 | 14 | 2 | 1 | 1.71492044 | 3.49103189 | 3.08697903 | 0.20472683 | 0.76070569 | 1 | 0.37104828 |
| IL6ST | 0 | 8 | 1 | 0 | 3.04007392 | 5.05575506 | 0 | 0.153771 | 0.83151916 | 1 | 0.37104828 |
| FAS | 0 | 3 | 0 | 1 | 2.74746074 | 0 | 17.9321636 | 0.20472683 | 1 | 1 | 0.37104828 |
| SOX9 | 0 | 6 | 1 | 0 | 3.15064971 | 11.0884376 | 0 | 0.20453581 | 0.68272016 | 1 | 0.37652905 |
| ATP1A1 | 0 | 4 | 1 | 1 | 1.34201001 | 6.22349871 | 8.5009375 | 0.20472683 | 0.80759426 | 1 | 0.37652905 |
| JAK2 | 0 | 6 | 2 | 0 | 1.67365283 | 7.61428363 | 0 | 0.20472683 | 0.47568053 | 1 | 0.39240665 |
| TPM3 | 0 | 5 | 0 | 0 | 5.57768864 | 0 | 0 | 0.05366873 | 1 | 1 | 0.42694914 |
| SMAD2 | 1 | 5 | 1 | 0 | 2.91577327 | 6.36375135 | 0 | 0.19565254 | 0.74470128 | 1 | 0.43193114 |
| PDE4DIP | 2 | 16 | 1 | 1 | 2.15974024 | 1.61330786 | 3.21656256 | 0.19565254 | 1 | 1 | 0.43644694 |
| FANCD2 | 1 | 7 | 2 | 0 | 1.72649533 | 7.17031336 | 0 | 0.20472683 | 0.47568053 | 1 | 0.44351306 |
| AURKA | 0 | 4 | 1 | 0 | 2.7993547 | 8.39693863 | 0 | 0.20472683 | 0.6943602 | 1 | 0.45063262 |
| PALB2 | 1 | 6 | 2 | 0 | 1.51017053 | 7.42572446 | 0 | 0.20472683 | 0.47568053 | 1 | 0.45932694 |
| ATP2B3 | 2 | 15 | 0 | 0 | 3.26822605 | 0 | 0 | 0.0619751 | 1 | 1 | 0.45932694 |
| ROBO1 | 3 | 12 | 0 | 2 | 1.51888532 | 0 | 6.78774211 | 0.20472683 | 1 | 1 | 0.47297386 |
| CACNA1D | 3 | 16 | 2 | 0 | 1.92661036 | 4.27860834 | 0 | 0.20472683 | 0.71101049 | 1 | 0.47463194 |
| SSX1 | 0 | 1 | 1 | 0 | 1.39709267 | 27.7090115 | 0 | 0.20472683 | 0.4315215 | 1 | 0.47646861 |
| ATRX | 2 | 16 | 1 | 1 | 1.78699962 | 1.38405373 | 4.02598676 | 0.20472683 | 1 | 1 | 0.47646861 |
| CHD1L | 0 | 7 | 1 | 0 | 2.63802918 | 5.53714438 | 0 | 0.20453581 | 0.81353232 | 1 | 0.52315235 |
| EWSR1 | 3 | 7 | 1 | 0 | 2.32601823 | 4.75897125 | 0 | 0.20453581 | 0.84094642 | 1 | 0.52988202 |
| SMAD3 | 0 | 3 | 1 | 0 | 2.33519009 | 16.9655707 | 0 | 0.20472683 | 0.56753458 | 1 | 0.54163533 |
| CASC5 | 1 | 10 | 2 | 0 | 1.64393956 | 4.85798153 | 0 | 0.20472683 | 0.6764284 | 1 | 0.54399602 |
| MYB | 0 | 6 | 1 | 0 | 2.56946819 | 5.80572794 | 0 | 0.20472683 | 0.80759426 | 1 | 0.55313016 |
| ARID1B | 1 | 11 | 2 | 0 | 1.74567493 | 5.67387497 | 0 | 0.20472683 | 0.62861306 | 1 | 0.56526095 |
| SET | 0 | 1 | 1 | 0 | 1.03015 | 17.4377849 | 0 | 0.20472683 | 0.53910493 | 1 | 0.56636268 |
| NFE2L2 | 0 | 4 | 1 | 0 | 2.25210294 | 8.25545806 | 0 | 0.20472683 | 0.71101049 | 1 | 0.56636268 |
| MYH11 | 4 | 11 | 0 | 2 | 1.39873964 | 0 | 6.49686646 | 0.20472683 | 1 | 1 | 0.5946251 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| PTPRJ | 0 | 8 | 1 | 0 | 2.40698411 | 5.60419427 | 0 | 0.20472683 | 0.83151916 | 1 | 0.59856783 |
| MAPK9 | 1 | 3 | 0 | 1 | 1.88804069 | 0 | 12.4750784 | 0.20472683 | 1 | 1 | 0.59856783 |
| BCOR | 4 | 10 | 1 | 1 | 1.28913352 | 2.34896929 | 8.17986816 | 0.20472683 | 1 | 1 | 0.59856783 |
| DCUN1D1 | 0 | 1 | 1 | 0 | 1.05968856 | 12.1039551 | 0 | 0.20472683 | 0.61772969 | 1 | 0.61434745 |
| SMC3 | 1 | 8 | 0 | 1 | 2.03232386 | 0 | 5.11294512 | 0.20472683 | 1 | 1 | 0.61434745 |
| STAT5B | 1 | 5 | 0 | 1 | 2.07343611 | 0 | 10.0543131 | 0.20472683 | 1 | 1 | 0.62494518 |
| FCGR2B | 1 | 2 | 1 | 0 | 1.70962138 | 16.7520167 | 0 | 0.20472683 | 0.56753458 | 1 | 0.62820373 |
| IDH1 | 2 | 4 | 1 | 0 | 1.93227338 | 8.1737129 | 0 | 0.20472683 | 0.72034472 | 1 | 0.63344113 |
| KIT | 0 | 7 | 0 | 1 | 2.2122265 | 0 | 6.68234948 | 0.20472683 | 1 | 1 | 0.63344113 |
| PSIP1 | 1 | 4 | 0 | 1 | 1.78392185 | 0 | 8.62896147 | 0.20472683 | 1 | 1 | 0.63344113 |
| DNMT3A | 2 | 8 | 1 | 0 | 2.13773608 | 5.24829371 | 0 | 0.20472683 | 0.85456061 | 1 | 0.65605864 |
| CREBBP | 1 | 9 | 2 | 0 | 1.53166011 | 4.98065821 | 0 | 0.20472683 | 0.6764284 | 1 | 0.66524218 |
| LCK | 3 | 5 | 1 | 0 | 2.0858371 | 7.39782952 | 0 | 0.20472683 | 0.74470128 | 1 | 0.66524218 |
| RAD21 | 1 | 8 | 0 | 0 | 3.16782164 | 0 | 0 | 0.11784311 | 1 | 1 | 0.66524218 |
| NOTCH2 | 8 | 11 | 4 | 1 | 0.73892116 | 4.84860909 | 2.52658666 | 1 | 0.29133126 | 1 | 0.67059674 |
| EPHA3 | 4 | 6 | 2 | 0 | 1.16677158 | 5.27362269 | 0 | 0.20472683 | 0.62861306 | 1 | 0.67773946 |
| RPS6KB1 | 2 | 3 | 0 | 1 | 1.401991 | 0 | 11.0139043 | 0.20472683 | 1 | 1 | 0.67773946 |
| ARID2 | 2 | 10 | 2 | 0 | 1.53818288 | 3.67783211 | 0 | 0.20472683 | 0.74366664 | 1 | 0.68859793 |
| NCKIPSD | 0 | 3 | 0 | 1 | 1.51198597 | 0 | 14.5084318 | 0.20472683 | 1 | 1 | 0.68859793 |
| AKT2 | 1 | 3 | 1 | 0 | 1.58002086 | 12.9714835 | 0 | 0.20472683 | 0.67375119 | 1 | 0.70126771 |
| NCOA2 | 3 | 9 | 2 | 0 | 1.30032993 | 4.42263686 | 0 | 0.20472683 | 0.68272016 | 1 | 0.70126771 |
| STAT4 | 3 | 7 | 1 | 0 | 2.07418966 | 3.6404623 | 0 | 0.20472683 | 0.98788102 | 1 | 0.70126771 |
| ARAF | 3 | 6 | 0 | 1 | 1.87634431 | 0 | 7.21440404 | 0.20472683 | 1 | 1 | 0.70295601 |
| TTL | 0 | 2 | 1 | 0 | 1.61903485 | 11.7614378 | 0 | 0.20472683 | 0.6764284 | 1 | 0.71190429 |
| TPR | 3 | 21 | 0 | 0 | 2.20239869 | 0 | 0 | 0.14421919 | 1 | 1 | 0.72861369 |
| USP9X | 2 | 13 | 2 | 0 | 1.50054624 | 3.10744157 | 0 | 0.20472683 | 0.81353232 | 1 | 0.72949689 |

EP 3 452 937 B1

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| PRDM1 | 4 | 8 | 1 | 0 | 1.92046689 | 5.20399356 | 0 | 0.20472683 | 0.871227 | 1 | 0.72949689 |
| CBLB | 2 | 12 | 0 | 0 | 2.57831368 | 0 | 0 | 0.14465511 | 1 | 1 | 0.72949689 |
| PLCG1 | 3 | 7 | 1 | 1 | 1.23300946 | 2.79066007 | 3.96485129 | 0.20472683 | 1 | 1 | 0.72949689 |
| FNBP1 | 0 | 2 | 0 | 1 | 1.06187844 | 0 | 10.8224303 | 0.20472683 | 1 | 1 | 0.72949689 |
| SETBP1 | 2 | 4 | 1 | 1 | 0.62113745 | 2.91036407 | 34.6193707 | 1 | 1 | 1 | 0.72949689 |
| NTRK2 | 1 | 7 | 1 | 0 | 1.979398 | 5.47183291 | 0 | 0.20472683 | 0.84316548 | 1 | 0.73045244 |
| SKP2 | 1 | 3 | 1 | 0 | 1.73101793 | 8.26981042 | 0 | 0.20472683 | 0.72034472 | 1 | 0.73713626 |
| INHBA | 3 | 2 | 2 | 0 | 0.74059212 | 15.2048904 | 0 | 1 | 0.21331233 | 1 | 0.74340443 |
| MYOC | 0 | 3 | 1 | 0 | 1.78238555 | 8.02925528 | 0 | 0.20472683 | 0.73038584 | 1 | 0.74340443 |
| LASP1 | 0 | 4 | 0 | 0 | 4.67269139 | 0 | 0 | 0.153771 | 1 | 1 | 0.74340443 |
| MAP3K4 | 5 | 4 | 4 | 0 | 0.3971713 | 5.70381124 | 0 | 1 | 0.21414141 | 1 | 0.74444973 |
| CUX1 | 4 | 4 | 3 | 0 | 0.59571172 | 7.44145638 | 0 | 1 | 0.23750321 | 1 | 0.74444973 |
| AFF3 | 5 | 8 | 2 | 0 | 1.06968697 | 4.56292785 | 0 | 0.21676652 | 0.68272016 | 1 | 0.74444973 |
| LZTR1 | 0 | 1 | 1 | 1 | 0.43014891 | 10.2234832 | 9.40744028 | 1 | 0.6943602 | 1 | 0.74444973 |
| CREB3L2 | 0 | 3 | 1 | 0 | 1.77083063 | 8.97991145 | 0 | 0.20472683 | 0.71101049 | 1 | 0.74444973 |
| CCNE1 | 2 | 3 | 1 | 0 | 1.44646895 | 8.23361097 | 0 | 0.20472683 | 0.72743076 | 1 | 0.74444973 |
| ATIC | 1 | 3 | 1 | 0 | 1.31327411 | 8.26447144 | 0 | 0.20472683 | 0.73038584 | 1 | 0.74444973 |
| EP300 | 2 | 9 | 2 | 0 | 1.28924796 | 3.60255916 | 0 | 0.20472683 | 0.74470128 | 1 | 0.74444973 |
| TGFBR2 | 2 | 4 | 1 | 0 | 1.39224095 | 7.87203425 | 0 | 0.20472683 | 0.74470128 | 1 | 0.74444973 |
| EXT1 | 0 | 4 | 1 | 0 | 1.64467413 | 6.88617448 | 0 | 0.20472683 | 0.76070569 | 1 | 0.74444973 |
| GNAS | 1 | 6 | 1 | 0 | 1.61045981 | 6.60610387 | 0 | 0.20472683 | 0.80226622 | 1 | 0.74444973 |
| DDX3X | 2 | 4 | 1 | 0 | 1.21384809 | 5.35857172 | 0 | 0.20472683 | 0.81353232 | 1 | 0.74444973 |
| BCL9 | 0 | 7 | 1 | 0 | 1.84062623 | 4.98772118 | 0 | 0.20472683 | 0.87321542 | 1 | 0.74444973 |
| IL21R | 0 | 6 | 0 | 0 | 3.52075575 | 0 | 0 | 0.153771 | 1 | 1 | 0.74444973 |
| WSB1 | 0 | 5 | 0 | 0 | 3.4859859 | 0 | 0 | 0.153771 | 1 | 1 | 0.74444973 |
| RSPO2 | 0 | 4 | 0 | 0 | 3.79848364 | 0 | 0 | 0.153771 | 1 | 1 | 0.74444973 |

EP 3 452 937 B1

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| EXT2 | 0 | 8 | 0 | 0 | 3.10606888 | 0 | 0 | 0.153771 | 1 | 1 | 0.74444973 |
| ERG | 2 | 7 | 0 | 0 | 3.0305369 | 0 | 0 | 0.153771 | 1 | 1 | 0.74444973 |
| CEP76 | 0 | 6 | 0 | 0 | 3.10681624 | 0 | 0 | 0.15819813 | 1 | 1 | 0.74444973 |
| ACVR2A | 1 | 7 | 0 | 0 | 2.8880028 | 0 | 0 | 0.15819813 | 1 | 1 | 0.74444973 |
| NCOA3 | 0 | 10 | 0 | 0 | 2.65033842 | 0 | 0 | 0.15819813 | 1 | 1 | 0.74444973 |
| ETV1 | 3 | 4 | 0 | 1 | 1.15584898 | 0 | 6.74440644 | 0.20472683 | 1 | 1 | 0.74444973 |
| PTPRC | 4 | 12 | 1 | 0 | 1.81404485 | 2.27616036 | 0 | 0.20472683 | 1 | 1 | 0.74444973 |
| TRIP11 | 2 | 8 | 0 | 1 | 1.15713999 | 0 | 6.64572893 | 0.20472683 | 1 | 1 | 0.74444973 |
| WHSC1L1 | 0 | 7 | 1 | 0 | 1.88720069 | 3.44866578 | 0 | 0.20472683 | 1 | 1 | 0.74444973 |
| ACSL6 | 0 | 3 | 0 | 1 | 1.35260534 | 0 | 8.25358052 | 0.20472683 | 1 | 1 | 0.74444973 |
| MED12 | 5 | 19 | 1 | 0 | 1.86458167 | 1.51725303 | 0 | 0.20472683 | 1 | 1 | 0.74444973 |
| ROBO2 | 1 | 12 | 1 | 0 | 1.95364231 | 2.30985546 | 0 | 0.20472683 | 1 | 1 | 0.74444973 |
| TRRAP | 2 | 18 | 1 | 0 | 2.07069181 | 2.04173666 | 0 | 0.20472683 | 1 | 1 | 0.74444973 |
| KEAP1 | 0 | 2 | 1 | 0 | 1.11088485 | 10.0208329 | 0 | 0.22624434 | 0.6943602 | 1 | 0.75763727 |
| GAS7 | 1 | 3 | 1 | 0 | 1.39485512 | 8.14498027 | 0 | 0.20661747 | 0.73800513 | 1 | 0.75763727 |
| EPAS1 | 1 | 4 | 1 | 0 | 1.44074043 | 7.24045787 | 0 | 0.20472683 | 0.76070569 | 1 | 0.77222715 |
| EZH2 | 1 | 3 | 1 | 0 | 1.03697156 | 5.42793249 | 0 | 0.20472683 | 0.81353232 | 1 | 0.77222715 |
| CHIC2 | 0 | 3 | 0 | 0 | 4.80823004 | 0 | 0 | 0.17200778 | 1 | 1 | 0.77222715 |
| NUP98 | 1 | 9 | 1 | 0 | 1.77881626 | 2.93933206 | 0 | 0.20472683 | 1 | 1 | 0.77222715 |
| FCRL4 | 2 | 4 | 1 | 0 | 1.57289988 | 5.28741462 | 0 | 0.20472683 | 0.83122311 | 1 | 0.77626485 |
| EML4 | 0 | 4 | 1 | 0 | 1.41649012 | 4.35215562 | 0 | 0.20472683 | 0.87321542 | 1 | 0.77626485 |
| MSH2 | 1 | 5 | 1 | 0 | 1.58846474 | 4.06445651 | 0 | 0.20472683 | 0.92981812 | 1 | 0.77626485 |
| BRIP1 | 3 | 7 | 0 | 1 | 1.24402033 | 0 | 5.10098058 | 0.20472683 | 1 | 1 | 0.77626485 |
| CDK12 | 3 | 10 | 1 | 0 | 1.75396847 | 2.46406312 | 0 | 0.20472683 | 1 | 1 | 0.77626485 |
| ZMYM2 | 0 | 6 | 1 | 0 | 1.57089296 | 2.95251222 | 0 | 0.20472683 | 1 | 1 | 0.78018764 |
| TRAF7 | 1 | 3 | 0 | 1 | 1.38376138 | 0 | 8.09124483 | 0.21918258 | 1 | 1 | 0.79181216 |

EP 3 452 937 B1

44

| gene_name | n_syn | n_mis | n_non | n_splice | wMIS3 | wNON3 | wSPLICE3 | qMIS3 | qNON3 | qSPLICE3 | qALL3 |
|-----------|-------|-------|-------|----------|-------|-------|----------|-------|-------|----------|-------|
| ATR | 4 | 12 | 1 | 1 | 1.14263956 | 1.3022085 | 2.52896692 | 0.20472683 | 1 | 1 | 0.81031132 |
| ESR1 | 1 | 7 | 0 | 0 | 2.89076417 | 0 | 0 | 0.18962835 | 1 | 1 | 0.81138156 |
| ABL2 | 2 | 7 | 1 | 0 | 1.6435028 | 3.42806395 | 0 | 0.20472683 | 1 | 1 | 0.81138156 |
| PPM1D | 1 | 3 | 1 | 0 | 1.26902436 | 5.37830062 | 0 | 0.20472683 | 0.81353232 | 1 | 0.81675904 |
| AR | 2 | 6 | 1 | 0 | 1.64648706 | 4.71963902 | 0 | 0.20472683 | 0.91735223 | 1 | 0.81847467 |
| IKBKB | 1 | 4 | 1 | 0 | 1.55291101 | 4.67640839 | 0 | 0.20472683 | 0.87321542 | 1 | 0.82146439 |
| PTPN13 | 3 | 11 | 2 | 0 | 1.13902169 | 2.65892486 | 0 | 0.20472683 | 0.90688301 | 1 | 0.82552546 |
| NUMA1 | 1 | 10 | 1 | 0 | 1.75128981 | 2.5004244 | 0 | 0.20472683 | 1 | 1 | 0.82552546 |
| NTRK3 | 2 | 5 | 0 | 1 | 1.286395 | 0 | 6.31160771 | 0.20672869 | 1 | 1 | 0.82552546 |
| MAP3K6 | 1 | 3 | 2 | 0 | 0.78565881 | 11.1605344 | 0 | 1 | 0.31627616 | 1 | 0.84351041 |
| MAP3K7 | 1 | 2 | 1 | 1 | 0.75189508 | 5.52527097 | 7.41980481 | 1 | 0.81353232 | 1 | 0.86212037 |
| MLL | 4 | 9 | 3 | 1 | 0.73221553 | 3.34890398 | 4.5577968 | 1 | 0.68272016 | 1 | 0.86975173 |
| RABEP1 | 0 | 2 | 2 | 0 | 0.81276481 | 9.56907507 | 0 | 1 | 0.33813743 | 1 | 0.8844798 |
| FGFR4 | 5 | 3 | 2 | 0 | 0.71237731 | 9.812343 | 0 | 1 | 0.33813743 | 1 | 0.8844798 |
| CIC | 2 | 7 | 1 | 0 | 1.35430909 | 4.46192142 | 0 | 0.20472683 | 0.96595393 | 1 | 0.8844798 |
| BRAF | 1 | 4 | 1 | 0 | 1.34694025 | 3.83616397 | 0 | 0.20472683 | 0.96595393 | 1 | 0.8844798 |
| THRAP3 | 0 | 7 | 0 | 0 | 2.42352468 | 0 | 0 | 0.20453581 | 1 | 1 | 0.8844798 |
| ATF1 | 0 | 3 | 0 | 0 | 3.63137964 | 0 | 0 | 0.20453581 | 1 | 1 | 0.8844798 |
| RAB25 | 1 | 3 | 0 | 0 | 3.97209378 | 0 | 0 | 0.20453581 | 1 | 1 | 0.8844798 |
| TSC1 | 1 | 9 | 0 | 0 | 2.36825858 | 0 | 0 | 0.20453581 | 1 | 1 | 0.8844798 |
| SPRED1 | 0 | 5 | 0 | 0 | 2.69303714 | 0 | 0 | 0.20453581 | 1 | 1 | 0.8844798 |
| GATA1 | 2 | 5 | 0 | 0 | 2.98270909 | 0 | 0 | 0.20472683 | 1 | 1 | 0.8844798 |
| TSC2 | 2 | 8 | 1 | 0 | 1.53241379 | 4.22378365 | 0 | 0.20472683 | 1 | 1 | 0.8844798 |
| PTPRS | 0 | 5 | 0 | 1 | 1.14606189 | 0 | 6.91095082 | 0.22624434 | 1 | 1 | 0.8844798 |
| ZNF217 | 4 | 8 | 1 | 0 | 1.47122982 | 3.33523889 | 0 | 0.20472683 | 1 | 1 | 0.89217818 |
| GRM3 | 7 | 12 | 1 | 0 | 1.5825747 | 2.3910278 | 0 | 0.20472683 | 1 | 1 | 0.89527903 |

| gene_name | n_syn | n_mis | n_non | n_splice | wMIS3 | wNON3 | wSPLICE3 | qMIS3 | qNON3 | qSPLICE3 | qALL3 |
|-----------|-------|-------|-------|----------|-------|-------|----------|-------|-------|----------|-------|
| NOTCH4 | 4 | 12 | 1 | 0 | 1.56020579 | 2.44281474 | 0 | 0.20472683 | 1 | 1 | 0.89527903 |
| BCL11A | 3 | 5 | 1 | 0 | 1.08147177 | 5.53774514 | 0 | 0.22106077 | 0.85224876 | 1 | 0.90373276 |
| SMARCA4 | 6 | 8 | 2 | 1 | 0.97027283 | 4.87688933 | 3.25770264 | 1 | 0.6764284 | 1 | 0.91377736 |
| PIK3C2B | 3 | 9 | 1 | 0 | 1.49100025 | 3.02615297 | 0 | 0.20472683 | 1 | 1 | 0.91377736 |
| NUP214 | 3 | 8 | 0 | 1 | 1.22521391 | 0 | 4.25100476 | 0.20472683 | 1 | 1 | 0.91907483 |
| STAT3 | 1 | 6 | 0 | 0 | 2.62876566 | 0 | 0 | 0.20472683 | 1 | 1 | 0.91907483 |
| MDM2 | 2 | 5 | 0 | 0 | 2.3417939 | 0 | 0 | 0.20472683 | 1 | 1 | 0.92791035 |
| CNOT3 | 0 | 6 | 0 | 0 | 2.67099463 | 0 | 0 | 0.20472683 | 1 | 1 | 0.93086038 |
| COL1A1 | 3 | 8 | 0 | 1 | 1.37665615 | 0 | 2.74901823 | 0.20472683 | 1 | 1 | 0.93086038 |
| HLF | 1 | 4 | 0 | 0 | 2.92013347 | 0 | 0 | 0.20472683 | 1 | 1 | 0.93086038 |
| RPL10 | 1 | 3 | 0 | 0 | 3.01378253 | 0 | 0 | 0.20472683 | 1 | 1 | 0.93086038 |
| EPHB1 | 8 | 11 | 0 | 1 | 1.25466803 | 0 | 3.94378913 | 0.20472683 | 1 | 1 | 0.93086038 |
| WIF1 | 0 | 4 | 0 | 0 | 2.97782629 | 0 | 0 | 0.20472683 | 1 | 1 | 0.93086038 |
| LRIG3 | 1 | 5 | 1 | 0 | 1.15436002 | 3.68065105 | 0 | 0.20544691 | 1 | 1 | 0.93865644 |
| AFF4 | 4 | 6 | 1 | 0 | 1.10983646 | 2.43466067 | 0 | 0.20472683 | 1 | 1 | 0.96051349 |
| SMC1A | 2 | 6 | 1 | 0 | 1.16814928 | 2.80884569 | 0 | 0.20472683 | 1 | 1 | 0.96051349 |
| HOXC13 | 0 | 1 | 1 | 0 | 0.87874729 | 23.8449957 | 0 | 1 | 0.47568053 | 1 | 1 |
| DDIT3 | 0 | 0 | 1 | 0 | 0 | 19.9559932 | 0 | 1 | 0.47568053 | 1 | 1 |
| JUN | 0 | 1 | 1 | 0 | 0.87579853 | 21.3973124 | 0 | 1 | 0.50732929 | 1 | 1 |
| NGFR | 0 | 1 | 1 | 0 | 0.74173157 | 17.7049839 | 0 | 1 | 0.56753458 | 1 | 1 |
| HNRNPA2B1 | 0 | 1 | 1 | 0 | 0.87494583 | 15.5435859 | 0 | 1 | 0.56753458 | 1 | 1 |
| TRIM24 | 2 | 3 | 2 | 0 | 0.67624588 | 5.85550565 | 0 | 1 | 0.56753458 | 1 | 1 |
| PPP6C | 0 | 1 | 1 | 0 | 0.95758492 | 14.7033778 | 0 | 1 | 0.58895562 | 1 | 1 |
| ALK | 3 | 1 | 2 | 0 | 0.14126076 | 5.49290293 | 0 | 1 | 0.62861306 | 1 | 1 |
| PAX8 | 1 | 1 | 1 | 0 | 0.60739358 | 11.770135 | 0 | 1 | 0.6764284 | 1 | 1 |
| NOV | 1 | 0 | 1 | 0 | 0 | 11.2462981 | 0 | 1 | 0.6764284 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| PTPRK | 2 | 3 | 2 | 0 | 0.48174946 | 4.76014934 | 0 | 1 | 0.6764284 | 1 | 1 |
| PICALM | 0 | 1 | 1 | 0 | 0.53099269 | 10.6466901 | 0 | 1 | 0.68272016 | 1 | 1 |
| MECOM | 4 | 4 | 2 | 0 | 0.56622945 | 4.6132961 | 0 | 1 | 0.68272016 | 1 | 1 |
| RNF213 | 7 | 14 | 3 | 0 | 0.86992752 | 3.3741188 | 0 | 1 | 0.68272016 | 1 | 1 |
| MAPK8 | 0 | 1 | 1 | 0 | 0.62210686 | 8.92295876 | 0 | 1 | 0.6943602 | 1 | 1 |
| FLCN | 1 | 1 | 1 | 0 | 0.49992863 | 9.95910664 | 0 | 1 | 0.6943602 | 1 | 1 |
| C2orf44 | 0 | 2 | 1 | 0 | 0.989641 | 8.81809012 | 0 | 1 | 0.71101049 | 1 | 1 |
| MYH9 | 6 | 9 | 2 | 1 | 0.98585333 | 4.1761022 | 2.72759631 | 1 | 0.72034472 | 1 | 1 |
| PCM1 | 2 | 5 | 2 | 0 | 0.70165511 | 3.66492225 | 0 | 1 | 0.73800513 | 1 | 1 |
| EBF1 | 2 | 2 | 1 | 0 | 0.65490236 | 7.78522783 | 0 | 1 | 0.74470128 | 1 | 1 |
| WAS | 2 | 2 | 1 | 0 | 0.89130007 | 6.70821643 | 0 | 1 | 0.76070569 | 1 | 1 |
| ARHGEF12 | 4 | 4 | 2 | 0 | 0.52795498 | 3.34585548 | 0 | 1 | 0.76070569 | 1 | 1 |
| ELF4 | 2 | 1 | 1 | 0 | 0.37302294 | 6.91749603 | 0 | 1 | 0.76093523 | 1 | 1 |
| RASAL1 | 1 | 2 | 1 | 0 | 0.70433531 | 6.8711391 | 0 | 1 | 0.76093523 | 1 | 1 |
| BAG4 | 2 | 1 | 1 | 0 | 0.52168931 | 5.79912226 | 0 | 1 | 0.76093523 | 1 | 1 |
| ZNF331 | 1 | 1 | 1 | 0 | 0.47077783 | 5.15247363 | 0 | 1 | 0.81353232 | 1 | 1 |
| MKL1 | 2 | 2 | 1 | 0 | 0.63854144 | 5.80304947 | 0 | 1 | 0.82955317 | 1 | 1 |
| PMS2 | 1 | 3 | 1 | 0 | 0.97204522 | 4.4632183 | 0 | 1 | 0.90688301 | 1 | 1 |
| ECT2L | 1 | 3 | 1 | 0 | 0.92285321 | 4.28852689 | 0 | 1 | 0.92981812 | 1 | 1 |
| TRIM33 | 1 | 3 | 1 | 0 | 0.84453296 | 4.14355159 | 0 | 1 | 0.95416617 | 1 | 1 |
| BCR | 2 | 3 | 1 | 0 | 0.59677172 | 4.36832747 | 0 | 1 | 0.98925204 | 1 | 1 |
| PIK3C2A | 1 | 7 | 0 | 0 | 1.37885191 | 0 | 0 | 0.20472683 | 1 | 1 | 1 |
| GAB2 | 0 | 4 | 0 | 0 | 1.8638869 | 0 | 0 | 0.20472683 | 1 | 1 | 1 |
| GOPC | 0 | 4 | 0 | 0 | 2.44975255 | 0 | 0 | 0.20472683 | 1 | 1 | 1 |
| SNX29 | 1 | 5 | 0 | 0 | 1.55128901 | 0 | 0 | 0.20472683 | 1 | 1 | 1 |
| ELN | 3 | 5 | 0 | 0 | 1.87158294 | 0 | 0 | 0.20472683 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|-----------|-------|-------|-------|----------|-------|-------|----------|-------|-------|----------|-------|
| RAD51 | 1 | 2 | 0 | 0 | 1.6507903 | 0 | 0 | 0.20472683 | 1 | 1 | 1 |
| PIK3CB | 1 | 7 | 0 | 0 | 1.91616143 | 0 | 0 | 0.20472683 | 1 | 1 | 1 |
| SMARCD1 | 0 | 2 | 0 | 0 | 1.28492239 | 0 | 0 | 0.20472683 | 1 | 1 | 1 |
| TFE3 | 3 | 4 | 0 | 0 | 1.32993783 | 0 | 0 | 0.20472683 | 1 | 1 | 1 |
| BCL3 | 0 | 3 | 0 | 0 | 2.06236742 | 0 | 0 | 0.20472683 | 1 | 1 | 1 |
| TFRC | 1 | 3 | 0 | 0 | 1.2684809 | 0 | 0 | 0.20472683 | 1 | 1 | 1 |
| NT5C2 | 0 | 2 | 0 | 0 | 1.22507772 | 0 | 0 | 0.20472683 | 1 | 1 | 1 |
| MAP2K7 | 0 | 3 | 0 | 0 | 2.14662606 | 0 | 0 | 0.20472683 | 1 | 1 | 1 |
| ERC1 | 2 | 5 | 0 | 0 | 1.08097272 | 0 | 0 | 0.20472683 | 1 | 1 | 1 |
| XPO1 | 2 | 5 | 0 | 0 | 1.22029739 | 0 | 0 | 0.20472683 | 1 | 1 | 1 |
| NCOA1 | 2 | 6 | 0 | 0 | 1.13803848 | 0 | 0 | 0.20472683 | 1 | 1 | 1 |
| FUS | 0 | 3 | 0 | 0 | 1.83145807 | 0 | 0 | 0.20472683 | 1 | 1 | 1 |
| FH | 0 | 3 | 0 | 0 | 1.64479168 | 0 | 0 | 0.20472683 | 1 | 1 | 1 |
| CRKL | 1 | 2 | 0 | 0 | 1.74042756 | 0 | 0 | 0.20472683 | 1 | 1 | 1 |
| SOX10 | 1 | 3 | 0 | 0 | 1.81438532 | 0 | 0 | 0.20472683 | 1 | 1 | 1 |
| DICER1 | 0 | 9 | 0 | 0 | 1.67527459 | 0 | 0 | 0.20472683 | 1 | 1 | 1 |
| TCL1A | 0 | 1 | 0 | 0 | 2.08373392 | 0 | 0 | 0.20472683 | 1 | 1 | 1 |
| CCNB1IP1 | 0 | 1 | 0 | 0 | 1.01812531 | 0 | 0 | 0.20472683 | 1 | 1 | 1 |
| CHD8 | 4 | 11 | 0 | 0 | 1.01453315 | 0 | 0 | 0.20472683 | 1 | 1 | 1 |
| BIRC7 | 0 | 3 | 0 | 0 | 3.22514884 | 0 | 0 | 0.20472683 | 1 | 1 | 1 |
| ARFRP1 | 0 | 2 | 0 | 0 | 3.18228699 | 0 | 0 | 0.20472683 | 1 | 1 | 1 |
| FLT1 | 1 | 6 | 0 | 0 | 1.30033175 | 0 | 0 | 0.20472683 | 1 | 1 | 1 |
| NBN | 0 | 3 | 0 | 0 | 1.32738611 | 0 | 0 | 0.20472683 | 1 | 1 | 1 |
| UBR5 | 6 | 15 | 1 | 0 | 1.05058658 | 0.9053676 | 0 | 0.20472683 | 1 | 1 | 1 |
| DYRK1B | 0 | 3 | 0 | 0 | 1.45112962 | 0 | 0 | 0.20472683 | 1 | 1 | 1 |
| TFPT | 0 | 1 | 0 | 0 | 1.03779242 | 0 | 0 | 0.20472683 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|-----------|-------|-------|-------|----------|-------|-------|----------|-------|-------|----------|-------|
| JAK3 | 2 | 6 | 0 | 0 | 1.53208284 | 0 | 0 | 0.20472683 | 1 | 1 | 1 |
| ELL | 0 | 4 | 0 | 0 | 2.19166509 | 0 | 0 | 0.20472683 | 1 | 1 | 1 |
| PIK3CG | 3 | 8 | 0 | 0 | 1.49224078 | 0 | 0 | 0.20472683 | 1 | 1 | 1 |
| MET | 0 | 7 | 0 | 0 | 2.02222049 | 0 | 0 | 0.20472683 | 1 | 1 | 1 |
| RHEB | 0 | 1 | 0 | 0 | 1.87425794 | 0 | 0 | 0.20472683 | 1 | 1 | 1 |
| SUFU | 0 | 3 | 0 | 0 | 2.09893183 | 0 | 0 | 0.20472683 | 1 | 1 | 1 |
| MAP3K8 | 1 | 3 | 0 | 0 | 1.63355924 | 0 | 0 | 0.20472683 | 1 | 1 | 1 |
| CCDC6 | 0 | 3 | 0 | 0 | 1.70214719 | 0 | 0 | 0.20472683 | 1 | 1 | 1 |
| YWHAE | 0 | 2 | 0 | 0 | 2.45524374 | 0 | 0 | 0.20472683 | 1 | 1 | 1 |
| MAP2K6 | 1 | 2 | 0 | 0 | 1.4327404 | 0 | 0 | 0.20472683 | 1 | 1 | 1 |
| MAPK10 | 0 | 2 | 0 | 0 | 1.24435016 | 0 | 0 | 0.20472683 | 1 | 1 | 1 |
| IL2 | 0 | 1 | 0 | 0 | 1.9959952 | 0 | 0 | 0.20472683 | 1 | 1 | 1 |
| WHSC1 | 2 | 8 | 0 | 0 | 1.72699776 | 0 | 0 | 0.20472683 | 1 | 1 | 1 |
| ZBTB16 | 1 | 5 | 0 | 0 | 1.86967924 | 0 | 0 | 0.20472683 | 1 | 1 | 1 |
| DDX6 | 0 | 2 | 0 | 0 | 1.51059284 | 0 | 0 | 0.20472683 | 1 | 1 | 1 |
| FANCE | 0 | 4 | 0 | 0 | 2.72713077 | 0 | 0 | 0.20472683 | 1 | 1 | 1 |
| PTP4A1 | 2 | 2 | 0 | 0 | 2.47504888 | 0 | 0 | 0.20472683 | 1 | 1 | 1 |
| CCND3 | 0 | 2 | 0 | 0 | 2.17581734 | 0 | 0 | 0.20472683 | 1 | 1 | 1 |
| ITK | 2 | 6 | 0 | 0 | 1.66120069 | 0 | 0 | 0.20472683 | 1 | 1 | 1 |
| BCL6 | 0 | 3 | 0 | 0 | 1.2817988 | 0 | 0 | 0.20472683 | 1 | 1 | 1 |
| TFG | 0 | 2 | 0 | 0 | 1.51618907 | 0 | 0 | 0.20472683 | 1 | 1 | 1 |
| MSH6 | 1 | 6 | 0 | 0 | 1.24494369 | 0 | 0 | 0.20472683 | 1 | 1 | 1 |
| SFPQ | 0 | 3 | 0 | 0 | 1.26063808 | 0 | 0 | 0.20472683 | 1 | 1 | 1 |
| SDHB | 0 | 1 | 0 | 0 | 1.1355039 | 0 | 0 | 0.20472683 | 1 | 1 | 1 |
| MPL | 0 | 4 | 0 | 0 | 2.45200274 | 0 | 0 | 0.20472683 | 1 | 1 | 1 |
| CREB1 | 0 | 2 | 0 | 0 | 1.98184666 | 0 | 0 | 0.20472683 | 1 | 1 | 1 |

EP 3 452 937 B1

| gene_name | n_syn | n_mis | n_non | n_splice | wMIS3 | wNON3 | wSPLICE3 | qMIS3 | qNON3 | qSPLICE3 | qALL3 |
|-----------|-------|-------|-------|----------|-------|-------|----------|-------|-------|----------|-------|
| NR4A3 | 3 | 5 | 0 | 0 | 1.52563297 | 0 | 0 | 0.20472683 | 1 | 1 | 1 |
| CSF3R | 0 | 4 | 0 | 0 | 1.845598 | 0 | 0 | 0.20472683 | 1 | 1 | 1 |
| KDSR | 0 | 2 | 0 | 0 | 1.94817249 | 0 | 0 | 0.20472683 | 1 | 1 | 1 |
| SPOP | 1 | 3 | 0 | 0 | 2.11068056 | 0 | 0 | 0.20472683 | 1 | 1 | 1 |
| ZNF639 | 0 | 2 | 0 | 0 | 1.09711271 | 0 | 0 | 0.20472683 | 1 | 1 | 1 |
| FLT3 | 3 | 7 | 0 | 0 | 1.50380752 | 0 | 0 | 0.20472683 | 1 | 1 | 1 |
| TBX22 | 1 | 4 | 0 | 0 | 1.99344757 | 0 | 0 | 0.20472683 | 1 | 1 | 1 |
| RPL5 | 0 | 1 | 0 | 0 | 1.06375051 | 0 | 0 | 0.20472683 | 1 | 1 | 1 |
| ACSL3 | 0 | 5 | 0 | 0 | 2.30633556 | 0 | 0 | 0.20472683 | 1 | 1 | 1 |
| CDKN1A | 0 | 1 | 0 | 0 | 1.67648476 | 0 | 0 | 0.20472683 | 1 | 1 | 1 |
| DEK | 0 | 2 | 0 | 0 | 1.6237832 | 0 | 0 | 0.20472683 | 1 | 1 | 1 |
| MAX | 2 | 1 | 0 | 0 | 1.1940898 | 0 | 0 | 0.20472683 | 1 | 1 | 1 |
| KTN1 | 5 | 7 | 1 | 0 | 1.0431394 | 1.64255713 | 0 | 0.20472683 | 1 | 1 | 1 |
| OMD | 0 | 3 | 0 | 0 | 2.04873341 | 0 | 0 | 0.20472683 | 1 | 1 | 1 |
| AKAP9 | 6 | 26 | 0 | 0 | 1.43397803 | 0 | 0 | 0.20472683 | 1 | 1 | 1 |
| POT1 | 0 | 5 | 0 | 0 | 2.31914089 | 0 | 0 | 0.20472683 | 1 | 1 | 1 |
| CHN1 | 2 | 3 | 0 | 0 | 1.287652 | 0 | 0 | 0.20472683 | 1 | 1 | 1 |
| RARA | 2 | 3 | 0 | 0 | 1.62768016 | 0 | 0 | 0.20472683 | 1 | 1 | 1 |
| RAF1 | 0 | 3 | 0 | 0 | 1.62671068 | 0 | 0 | 0.20472683 | 1 | 1 | 1 |
| H3F3B | 1 | 2 | 0 | 0 | 3.42798575 | 0 | 0 | 0.20472683 | 1 | 1 | 1 |
| MUTYH | 3 | 4 | 0 | 0 | 1.67375777 | 0 | 0 | 0.20472683 | 1 | 1 | 1 |
| CDK8 | 0 | 2 | 0 | 0 | 1.19824896 | 0 | 0 | 0.20472683 | 1 | 1 | 1 |
| CDC73 | 0 | 4 | 0 | 0 | 2.24648712 | 0 | 0 | 0.20472683 | 1 | 1 | 1 |
| DDB2 | 0 | 2 | 0 | 0 | 1.71071107 | 0 | 0 | 0.20472683 | 1 | 1 | 1 |
| PDGFRA | 2 | 6 | 0 | 0 | 1.26063678 | 0 | 0 | 0.20472683 | 1 | 1 | 1 |
| APC | 3 | 13 | 0 | 0 | 1.20864913 | 0 | 0 | 0.20472683 | 1 | 1 | 1 |

EP 3 452 937 B1

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| HNF1A | 1 | 5 | 0 | 0 | 2.47974519 | 0 | 0 | 0.20472683 | 1 | 1 | 1 |
| PAX3 | 1 | 4 | 0 | 0 | 1.77033807 | 0 | 0 | 0.20472683 | 1 | 1 | 1 |
| LCP1 | 0 | 3 | 0 | 0 | 1.43635151 | 0 | 0 | 0.20472683 | 1 | 1 | 1 |
| ABI1 | 0 | 3 | 0 | 0 | 1.83590799 | 0 | 0 | 0.20472683 | 1 | 1 | 1 |
| KLF4 | 0 | 3 | 0 | 0 | 1.78031373 | 0 | 0 | 0.20472683 | 1 | 1 | 1 |
| XPA | 0 | 1 | 0 | 0 | 1.01483701 | 0 | 0 | 0.20472683 | 1 | 1 | 1 |
| TET1 | 3 | 8 | 1 | 0 | 1.10747017 | 1.84833114 | 0 | 0.20472683 | 1 | 1 | 1 |
| RAP1GDS1 | 0 | 5 | 0 | 0 | 2.25709826 | 0 | 0 | 0.20472683 | 1 | 1 | 1 |
| ETV6 | 0 | 4 | 0 | 0 | 2.24936244 | 0 | 0 | 0.20472683 | 1 | 1 | 1 |
| PIK3C2G | 2 | 7 | 0 | 0 | 1.05642052 | 0 | 0 | 0.20472683 | 1 | 1 | 1 |
| CDH11 | 2 | 7 | 0 | 0 | 1.50518162 | 0 | 0 | 0.20472683 | 1 | 1 | 1 |
| CLTC | 3 | 7 | 0 | 0 | 1.00516294 | 0 | 0 | 0.20472683 | 1 | 1 | 1 |
| SS18 | 2 | 3 | 0 | 0 | 1.41434611 | 0 | 0 | 0.20472683 | 1 | 1 | 1 |
| BCL10 | 0 | 1 | 0 | 0 | 1.21510081 | 0 | 0 | 0.20472683 | 1 | 1 | 1 |
| SDHC | 1 | 2 | 0 | 0 | 3.24386547 | 0 | 0 | 0.20472683 | 1 | 1 | 1 |
| LPP | 1 | 4 | 0 | 0 | 1.57711801 | 0 | 0 | 0.20472683 | 1 | 1 | 1 |
| SLIT2 | 3 | 10 | 0 | 0 | 1.13746298 | 0 | 0 | 0.20472683 | 1 | 1 | 1 |
| FIP1L1 | 2 | 4 | 0 | 0 | 1.26999645 | 0 | 0 | 0.20472683 | 1 | 1 | 1 |
| EGFR | 5 | 11 | 0 | 0 | 1.79614495 | 0 | 0 | 0.20472683 | 1 | 1 | 1 |
| MSN | 1 | 4 | 0 | 0 | 1.7024442 | 0 | 0 | 0.20472683 | 1 | 1 | 1 |
| ZMYM3 | 4 | 8 | 0 | 0 | 1.1867982 | 0 | 0 | 0.20472683 | 1 | 1 | 1 |
| NONO | 1 | 3 | 0 | 0 | 1.47918044 | 0 | 0 | 0.20472683 | 1 | 1 | 1 |
| GPC3 | 0 | 4 | 0 | 0 | 2.13231465 | 0 | 0 | 0.20472683 | 1 | 1 | 1 |
| NOTCH 1 | 1 | 9 | 0 | 0 | 1.62166113 | 0 | 0 | 0.20472683 | 1 | 1 | 1 |
| PAK1 | 0 | 3 | 0 | 0 | 1.65993377 | 0 | 0 | 0.20472683 | 1 | 1 | 1 |
| MSI2 | 0 | 3 | 0 | 0 | 2.69606881 | 0 | 0 | 0.20472683 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| GNAQ | 0 | 2 | 0 | 0 | 1.39348912 | 0 | 0 | 0.20472683 | 1 | 1 | 1 |
| CREB3L1 | 1 | 3 | 0 | 0 | 1.81076134 | 0 | 0 | 0.20472683 | 1 | 1 | 1 |
| SLC34A2 | 1 | 5 | 0 | 0 | 1.92438902 | 0 | 0 | 0.20472683 | 1 | 1 | 1 |
| SLC45A3 | 2 | 4 | 0 | 0 | 1.89426429 | 0 | 0 | 0.20472683 | 1 | 1 | 1 |
| TGM7 | 1 | 5 | 0 | 0 | 2.06130059 | 0 | 0 | 0.20472683 | 1 | 1 | 1 |
| U2AF1 | 0 | 1 | 0 | 0 | 1.01134263 | 0 | 0 | 0.20472683 | 1 | 1 | 1 |
| IKZF3 | 1 | 2 | 0 | 0 | 1.01359241 | 0 | 0 | 0.20472683 | 1 | 1 | 1 |
| DDR2 | 3 | 8 | 0 | 0 | 2.01982589 | 0 | 0 | 0.20472683 | 1 | 1 | 1 |
| H3F3A | 2 | 2 | 0 | 0 | 2.76676186 | 0 | 0 | 0.20472683 | 1 | 1 | 1 |
| ERCC3 | 1 | 5 | 0 | 0 | 1.92480778 | 0 | 0 | 0.20472683 | 1 | 1 | 1 |
| PRKCI | 0 | 2 | 0 | 0 | 1.01493384 | 0 | 0 | 0.20472683 | 1 | 1 | 1 |
| GMPS | 0 | 3 | 0 | 0 | 1.42945175 | 0 | 0 | 0.20472683 | 1 | 1 | 1 |
| RPN1 | 1 | 3 | 0 | 0 | 1.4199228 | 0 | 0 | 0.20472683 | 1 | 1 | 1 |
| BAP1 | 1 | 5 | 0 | 0 | 2.0622479 | 0 | 0 | 0.20472683 | 1 | 1 | 1 |
| PBRM1 | 0 | 6 | 0 | 0 | 1.4764561 | 0 | 0 | 0.20472683 | 1 | 1 | 1 |
| RICTOR | 5 | 10 | 0 | 0 | 1.11612985 | 0 | 0 | 0.20472683 | 1 | 1 | 1 |
| COX6C | 0 | 1 | 0 | 0 | 3.05953848 | 0 | 0 | 0.20472683 | 1 | 1 | 1 |
| YWHAZ | 1 | 2 | 0 | 0 | 2.15553021 | 0 | 0 | 0.20472683 | 1 | 1 | 1 |
| PIGA | 1 | 4 | 0 | 0 | 2.10690303 | 0 | 0 | 0.20472683 | 1 | 1 | 1 |
| TSHR | 1 | 6 | 0 | 0 | 2.21987591 | 0 | 0 | 0.20472683 | 1 | 1 | 1 |
| YWHAB | 0 | 1 | 0 | 0 | 1.23608564 | 0 | 0 | 0.20472683 | 1 | 1 | 1 |
| KLK2 | 1 | 2 | 0 | 0 | 1.9290159 | 0 | 0 | 0.20472683 | 1 | 1 | 1 |
| HOOK3 | 0 | 3 | 0 | 0 | 1.30179344 | 0 | 0 | 0.20472683 | 1 | 1 | 1 |
| MAP2K1 | 0 | 3 | 0 | 0 | 2.62242965 | 0 | 0 | 0.20472683 | 1 | 1 | 1 |
| MN1 | 0 | 6 | 0 | 0 | 1.77106112 | 0 | 0 | 0.20472683 | 1 | 1 | 1 |
| CNBP | 1 | 1 | 0 | 0 | 1.34129603 | 0 | 0 | 0.20472683 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| AFF1 | 3 | 6 | 0 | 0 | 1.10468163 | 0 | 0 | 0.20472683 | 1 | 1 | 1 |
| HRAS | 1 | 2 | 0 | 0 | 2.63260136 | 0 | 0 | 0.20472683 | 1 | 1 | 1 |
| ERCC4 | 1 | 7 | 0 | 0 | 2.14619413 | 0 | 0 | 0.20472683 | 1 | 1 | 1 |
| ERBB4 | 4 | 11 | 0 | 0 | 1.25154629 | 0 | 0 | 0.20472683 | 1 | 1 | 1 |
| SUZ12 | 0 | 4 | 0 | 0 | 1.80533192 | 0 | 0 | 0.20472683 | 1 | 1 | 1 |
| GPC5 | 3 | 6 | 0 | 0 | 1.54031955 | 0 | 0 | 0.20472683 | 1 | 1 | 1 |
| IDH2 | 1 | 3 | 0 | 0 | 1.80829649 | 0 | 0 | 0.20472683 | 1 | 1 | 1 |
| FAM46C | 1 | 3 | 0 | 0 | 1.82843548 | 0 | 0 | 0.20472683 | 1 | 1 | 1 |
| FOXO4 | 2 | 5 | 0 | 0 | 2.12001952 | 0 | 0 | 0.20472683 | 1 | 1 | 1 |
| Sep-05 | 0 | 3 | 0 | 0 | 2.44475487 | 0 | 0 | 0.20472683 | 1 | 1 | 1 |
| MUC1 | 0 | 2 | 0 | 0 | 1.58747332 | 0 | 0 | 0.20472683 | 1 | 1 | 1 |
| PBX1 | 0 | 3 | 0 | 0 | 1.834101 | 0 | 0 | 0.20472683 | 1 | 1 | 1 |
| TCEA1 | 1 | 2 | 0 | 0 | 1.59127438 | 0 | 0 | 0.20472683 | 1 | 1 | 1 |
| FANCA | 1 | 8 | 0 | 0 | 2.0480114 | 0 | 0 | 0.20472683 | 1 | 1 | 1 |
| NACA | 1 | 4 | 0 | 0 | 1.574128 | 0 | 0 | 0.20472683 | 1 | 1 | 1 |
| LPAR1 | 1 | 3 | 0 | 0 | 1.77913728 | 0 | 0 | 0.20472683 | 1 | 1 | 1 |
| CARD11 | 4 | 9 | 0 | 0 | 1.4829135 | 0 | 0 | 0.20472683 | 1 | 1 | 1 |
| CTNND1 | 2 | 5 | 0 | 0 | 1.16048291 | 0 | 0 | 0.20472683 | 1 | 1 | 1 |
| MDM4 | 2 | 3 | 0 | 0 | 1.45701523 | 0 | 0 | 0.20472683 | 1 | 1 | 1 |
| SMURF1 | 0 | 4 | 0 | 0 | 1.86726236 | 0 | 0 | 0.20472683 | 1 | 1 | 1 |
| MTOR | 6 | 15 | 1 | 0 | 1.27118195 | 1.37131116 | 0 | 0.20472683 | 1 | 1 | 1 |
| SSX4 | 0 | 1 | 0 | 0 | 1.37669596 | 0 | 0 | 0.20472683 | 1 | 1 | 1 |
| RANBP17 | 2 | 9 | 0 | 0 | 1.93846614 | 0 | 0 | 0.20472683 | 1 | 1 | 1 |
| ZBTB10 | 3 | 7 | 0 | 0 | 1.66692169 | 0 | 0 | 0.20472683 | 1 | 1 | 1 |
| NRAS | 0 | 2 | 0 | 0 | 3.12082935 | 0 | 0 | 0.20472683 | 1 | 1 | 1 |
| FANCG | 1 | 3 | 0 | 0 | 1.51670963 | 0 | 0 | 0.20472683 | 1 | 1 | 1 |

EP 3 452 937 B1

(continued)

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| ITGB3 | 1 | 4 | 0 | 0 | 1.55202368 | 0 | 0 | 0.20472683 | 1 | 1 | 1 |
| TNFAIP3 | 5 | 5 | 0 | 0 | 1.10912008 | 0 | 0 | 0.20510127 | 1 | 1 | 1 |
| YAP1 | 0 | 2 | 0 | 0 | 1.24536059 | 0 | 0 | 0.20510127 | 1 | 1 | 1 |
| MAML2 | 3 | 5 | 1 | 0 | 1.07785311 | 2.22387933 | 0 | 0.20537274 | 1 | 1 | 1 |
| MAP3K3 | 0 | 2 | 0 | 0 | 1.01169131 | 0 | 0 | 0.20537274 | 1 | 1 | 1 |
| MYCL1 | 0 | 2 | 0 | 0 | 1.51584822 | 0 | 0 | 0.20544691 | 1 | 1 | 1 |
| PPARG | 1 | 2 | 0 | 0 | 1.07455934 | 0 | 0 | 0.20544691 | 1 | 1 | 1 |
| PRCC | 0 | 2 | 0 | 0 | 1.47609462 | 0 | 0 | 0.20544691 | 1 | 1 | 1 |
| MLL2 | 8 | 20 | 1 | 1 | 1.02591639 | 0.79114785 | 2.50487985 | 0.20544691 | 1 | 1 | 1 |
| PTPRB | 9 | 14 | 2 | 0 | 1.00069045 | 2.14991472 | 0 | 0.20661747 | 1 | 1 | 1 |
| SH3GL1 | 0 | 2 | 0 | 0 | 1.77157082 | 0 | 0 | 0.20661747 | 1 | 1 | 1 |
| TPM4 | 0 | 1 | 0 | 0 | 1.02019166 | 0 | 0 | 0.20661747 | 1 | 1 | 1 |
| FHIT | 0 | 1 | 0 | 0 | 1.21734057 | 0 | 0 | 0.20661747 | 1 | 1 | 1 |
| MAP3K9 | 0 | 4 | 0 | 0 | 1.24006062 | 0 | 0 | 0.20672869 | 1 | 1 | 1 |
| KDR | 4 | 7 | 0 | 0 | 1.00233471 | 0 | 0 | 0.20672869 | 1 | 1 | 1 |
| YWHAQ | 0 | 1 | 0 | 0 | 1.27435445 | 0 | 0 | 0.2093365 | 1 | 1 | 1 |
| FANCF | 0 | 2 | 0 | 0 | 1.1397 | 0 | 0 | 0.21012998 | 1 | 1 | 1 |
| NFKB2 | 0 | 3 | 0 | 0 | 1.2671814 | 0 | 0 | 0.21047216 | 1 | 1 | 1 |
| ZNF384 | 1 | 2 | 0 | 0 | 1.13915676 | 0 | 0 | 0.21047216 | 1 | 1 | 1 |
| VTI1A | 0 | 1 | 0 | 0 | 1.1832564 | 0 | 0 | 0.21047216 | 1 | 1 | 1 |
| GRB2 | 1 | 1 | 0 | 0 | 1.26997017 | 0 | 0 | 0.21148356 | 1 | 1 | 1 |
| CALR | 2 | 2 | 0 | 0 | 1.08546268 | 0 | 0 | 0.21177317 | 1 | 1 | 1 |
| FOXO3 | 0 | 3 | 0 | 0 | 1.35951653 | 0 | 0 | 0.21330744 | 1 | 1 | 1 |
| HIP1 | 2 | 4 | 0 | 0 | 1.05443687 | 0 | 0 | 0.21401794 | 1 | 1 | 1 |
| CRTC3 | 0 | 2 | 0 | 0 | 1.18775967 | 0 | 0 | 0.21448992 | 1 | 1 | 1 |
| PDGFB | 0 | 1 | 0 | 0 | 1.15699376 | 0 | 0 | 0.2149146 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| U2AF2 | 0 | 2 | 0 | 0 | 1.29373004 | 0 | 0 | 0.21676652 | 1 | 1 | 1 |
| PDGFRB | 1 | 4 | 0 | 0 | 1.16291973 | 0 | 0 | 0.21676652 | 1 | 1 | 1 |
| KIAA1549 | 3 | 8 | 0 | 0 | 1.16230175 | 0 | 0 | 0.21676652 | 1 | 1 | 1 |
| RAC1 | 0 | 1 | 0 | 0 | 1.41571317 | 0 | 0 | 0.21676652 | 1 | 1 | 1 |
| IGF1R | 3 | 7 | 1 | 0 | 1.16673614 | 3.39529936 | 0 | 0.21676652 | 1 | 1 | 1 |
| MRAS | 0 | 1 | 0 | 0 | 1.35023238 | 0 | 0 | 0.21676652 | 1 | 1 | 1 |
| PAX5 | 1 | 2 | 0 | 0 | 1.19452276 | 0 | 0 | 0.21676652 | 1 | 1 | 1 |
| TRIM27 | 0 | 2 | 0 | 0 | 1.09755081 | 0 | 0 | 0.21676652 | 1 | 1 | 1 |
| SYK | 2 | 3 | 0 | 0 | 1.03707885 | 0 | 0 | 0.21695009 | 1 | 1 | 1 |
| AXIN2 | 0 | 3 | 0 | 0 | 1.05454289 | 0 | 0 | 0.21762707 | 1 | 1 | 1 |
| GATA2 | 0 | 2 | 0 | 0 | 1.44384046 | 0 | 0 | 0.21771126 | 1 | 1 | 1 |
| MCL1 | 1 | 2 | 0 | 0 | 1.50318682 | 0 | 0 | 0.21918258 | 1 | 1 | 1 |
| LHFP | 0 | 1 | 0 | 0 | 1.28944601 | 0 | 0 | 0.21918258 | 1 | 1 | 1 |
| AXIN1 | 1 | 3 | 0 | 0 | 1.06758587 | 0 | 0 | 0.219265 | 1 | 1 | 1 |
| RHOH | 1 | 1 | 0 | 0 | 1.39130707 | 0 | 0 | 0.22078719 | 1 | 1 | 1 |
| CD79B | 1 | 1 | 0 | 0 | 1.08651441 | 0 | 0 | 0.22106077 | 1 | 1 | 1 |
| CD74 | 0 | 1 | 0 | 0 | 1.17932934 | 0 | 0 | 0.22106077 | 1 | 1 | 1 |
| HEY1 | 2 | 2 | 0 | 0 | 1.2257582 | 0 | 0 | 0.22523613 | 1 | 1 | 1 |
| LYL1 | 0 | 1 | 0 | 0 | 1.11152141 | 0 | 0 | 0.22617735 | 1 | 1 | 1 |
| SMO | 1 | 3 | 0 | 0 | 1.10305151 | 0 | 0 | 0.22624434 | 1 | 1 | 1 |
| VHL | 0 | 1 | 0 | 0 | 1.2965381 | 0 | 0 | 0.22624434 | 1 | 1 | 1 |
| TAL1 | 1 | 2 | 0 | 0 | 1.31708904 | 0 | 0 | 0.22624434 | 1 | 1 | 1 |
| SOX2 | 0 | 2 | 0 | 0 | 1.17286825 | 0 | 0 | 0.22786021 | 1 | 1 | 1 |
| LMO2 | 0 | 1 | 0 | 0 | 1.1417039 | 0 | 0 | 0.22821956 | 1 | 1 | 1 |
| FADD | 0 | 1 | 0 | 0 | 1.32097679 | 0 | 0 | 0.22845649 | 1 | 1 | 1 |
| MYC | 2 | 2 | 0 | 0 | 1.01201362 | 0 | 0 | 0.23350724 | 1 | 1 | 1 |

EP 3 452 937 B1

| gene_name | n_syn | n_mis | n_non | n_splice | wMIS3 | wNON3 | wSPLICE3 | qMIS3 | qNON3 | qSPLICE3 | qALL3 |
|-----------|-------|-------|-------|----------|-------|-------|----------|-------|-------|----------|-------|
| TNFRSF14 | 0 | 1 | 0 | 0 | 1.16911065 | 0 | 0 | 0.24033568 | 1 | 1 | 1 |
| CANT1 | 2 | 2 | 0 | 0 | 1.15670588 | 0 | 0 | 0.24337128 | 1 | 1 | 1 |
| FOXL2 | 0 | 2 | 0 | 0 | 1.42654966 | 0 | 0 | 0.24514993 | 1 | 1 | 1 |
| MAP2K2 | 3 | 2 | 0 | 0 | 1.05139795 | 0 | 0 | 0.24745362 | 1 | 1 | 1 |
| BCL2 | 0 | 1 | 0 | 0 | 1.04483791 | 0 | 0 | 0.24745362 | 1 | 1 | 1 |
| HOXD11 | 1 | 2 | 0 | 0 | 1.06248832 | 0 | 0 | 0.25235805 | 1 | 1 | 1 |
| PAX7 | 0 | 1 | 0 | 0 | 0.54592498 | 0 | 0 | 1 | 1 | 1 | 1 |
| FYN | 3 | 1 | 0 | 0 | 0.37250789 | 0 | 0 | 1 | 1 | 1 | 1 |
| MAP4K3 | 2 | 2 | 0 | 0 | 0.54023598 | 0 | 0 | 1 | 1 | 1 | 1 |
| DDX11 | 10 | 4 | 0 | 0 | 0.46844279 | 0 | 0 | 1 | 1 | 1 | 1 |
| WWTR1 | 1 | 0 | 0 | 0 | 0 | 0 | 0 | 1 | 1 | 1 | 1 |
| MRE11A | 1 | 3 | 0 | 0 | 0.9672297 | 0 | 0 | 1 | 1 | 1 | 1 |
| BIRC3 | 0 | 1 | 0 | 0 | 0.56367938 | 0 | 0 | 1 | 1 | 1 | 1 |
| FLT4 | 3 | 2 | 1 | 0 | 0.35574705 | 3.91542185 | 0 | 1 | 1 | 1 | 1 |
| RRM2B | 0 | 1 | 0 | 0 | 0.82095255 | 0 | 0 | 1 | 1 | 1 | 1 |
| HERPUD1 | 0 | 1 | 0 | 0 | 0.90342086 | 0 | 0 | 1 | 1 | 1 | 1 |
| POU1F1 | 0 | 1 | 0 | 0 | 0.89695254 | 0 | 0 | 1 | 1 | 1 | 1 |
| GOLGA5 | 0 | 2 | 0 | 0 | 0.88678911 | 0 | 0 | 1 | 1 | 1 | 1 |
| KLF6 | 0 | 1 | 0 | 0 | 0.98036368 | 0 | 0 | 1 | 1 | 1 | 1 |
| FGFR3 | 3 | 0 | 0 | 0 | 0 | 0 | 0 | 1 | 1 | 1 | 1 |
| MAP4K4 | 4 | 4 | 0 | 0 | 0.5949114 | 0 | 0 | 1 | 1 | 1 | 1 |
| TCF3 | 0 | 1 | 0 | 0 | 0.55174918 | 0 | 0 | 1 | 1 | 1 | 1 |
| SDHA | 3 | 0 | 0 | 0 | 0 | 0 | 0 | 1 | 1 | 1 | 1 |
| SMARCE1 | 0 | 1 | 0 | 1 | 0.77700506 | 0 | 17.7304065 | 1 | 1 | 1 | 1 |
| KDM5A | 3 | 2 | 0 | 0 | 0.27532831 | 0 | 0 | 1 | 1 | 1 | 1 |
| NOTCH3 | 7 | 5 | 1 | 0 | 0.41964071 | 1.87754614 | 0 | 1 | 1 | 1 | 1 |

EP 3 452 937 B1

(continued)

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| PAK3 | 1 | 2 | 0 | 0 | 0.89179386 | 0 | 0 | 1 | 1 | 1 | 1 |
| FGFR1 | 5 | 3 | 0 | 0 | 0.63664631 | 0 | 0 | 1 | 1 | 1 | 1 |
| PIK3C3 | 1 | 4 | 0 | 0 | 0.90211582 | 0 | 0 | 1 | 1 | 1 | 1 |
| HOXA9 | 0 | 1 | 0 | 0 | 0.98759182 | 0 | 0 | 1 | 1 | 1 | 1 |
| TP73 | 3 | 2 | 0 | 1 | 0.63251601 | 0 | 9.3278045 | 1 | 1 | 1 | 1 |
| RUNX1T1 | 2 | 3 | 0 | 0 | 0.75852992 | 0 | 0 | 1 | 1 | 1 | 1 |
| DNM2 | 3 | 3 | 0 | 0 | 0.81508269 | 0 | 0 | 1 | 1 | 1 | 1 |
| TRPM3 | 6 | 9 | 1 | 0 | 0.85388998 | 1.56538139 | 0 | 1 | 1 | 1 | 1 |
| CYLD | 1 | 2 | 0 | 0 | 0.54261425 | 0 | 0 | 1 | 1 | 1 | 1 |
| GNA11 | 1 | 1 | 0 | 0 | 0.6943319 | 0 | 0 | 1 | 1 | 1 | 1 |
| EZR | 3 | 2 | 0 | 0 | 0.73431655 | 0 | 0 | 1 | 1 | 1 | 1 |
| CDC6 | 1 | 1 | 0 | 0 | 0.58206674 | 0 | 0 | 1 | 1 | 1 | 1 |
| HSP90AB1 | 3 | 2 | 0 | 0 | 0.54801854 | 0 | 0 | 1 | 1 | 1 | 1 |
| ABL1 | 2 | 4 | 0 | 1 | 0.99323909 | 0 | 14.2406062 | 1 | 1 | 1 | 1 |
| MAPK1 | 2 | 1 | 0 | 0 | 0.62314354 | 0 | 0 | 1 | 1 | 1 | 1 |
| PATZ1 | 1 | 0 | 0 | 0 | 0 | 0 | 0 | 1 | 1 | 1 | 1 |
| EEF1A2 | 1 | 1 | 0 | 0 | 0.55527024 | 0 | 0 | 1 | 1 | 1 | 1 |
| PTK6 | 4 | 1 | 0 | 0 | 0.40910022 | 0 | 0 | 1 | 1 | 1 | 1 |
| NDRG1 | 3 | 2 | 0 | 0 | 0.96544826 | 0 | 0 | 1 | 1 | 1 | 1 |
| ERCC2 | 0 | 2 | 0 | 0 | 0.93024085 | 0 | 0 | 1 | 1 | 1 | 1 |
| CD79A | 1 | 0 | 0 | 0 | 0 | 0 | 0 | 1 | 1 | 1 | 1 |
| PPP2R1A | 1 | 1 | 0 | 1 | 0.37624809 | 0 | 8.97657874 | 1 | 1 | 1 | 1 |
| CDK6 | 1 | 1 | 0 | 0 | 0.73339956 | 0 | 0 | 1 | 1 | 1 | 1 |
| HOXA13 | 2 | 1 | 0 | 0 | 0.51815986 | 0 | 0 | 1 | 1 | 1 | 1 |
| MLLT6 | 4 | 3 | 0 | 0 | 0.66713856 | 0 | 0 | 1 | 1 | 1 | 1 |
| RNF43 | 0 | 2 | 0 | 0 | 0.88389479 | 0 | 0 | 1 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|-----------|-------|-------|-------|----------|-------|-------|----------|-------|-------|----------|-------|
| DDX5 | 2 | 2 | 0 | 1 | 0.82305172 | 0 | 11.3325684 | 1 | 1 | 1 | 1 |
| PHOX2B | 2 | 1 | 0 | 0 | 0.65825919 | 0 | 0 | 1 | 1 | 1 | 1 |
| HSPA8 | 0 | 2 | 0 | 0 | 0.98099208 | 0 | 0 | 1 | 1 | 1 | 1 |
| CCND1 | 0 | 1 | 0 | 0 | 0.92384463 | 0 | 0 | 1 | 1 | 1 | 1 |
| BIRC2 | 2 | 0 | 0 | 0 | 0 | 0 | 0 | 1 | 1 | 1 | 1 |
| CBL | 4 | 3 | 0 | 0 | 0.69310234 | 0 | 0 | 1 | 1 | 1 | 1 |
| ELK3 | 2 | 1 | 0 | 0 | 0.53627653 | 0 | 0 | 1 | 1 | 1 | 1 |
| SH2B3 | 0 | 2 | 0 | 0 | 0.98626378 | 0 | 0 | 1 | 1 | 1 | 1 |
| ALDH2 | 1 | 0 | 0 | 0 | 0 | 0 | 0 | 1 | 1 | 1 | 1 |
| RAB23 | 1 | 0 | 0 | 0 | 0 | 0 | 0 | 1 | 1 | 1 | 1 |
| E2F3 | 1 | 0 | 0 | 0 | 0 | 0 | 0 | 1 | 1 | 1 | 1 |
| TFEB | 1 | 0 | 0 | 0 | 0 | 0 | 0 | 1 | 1 | 1 | 1 |
| VEGFA | 1 | 0 | 0 | 0 | 0 | 0 | 0 | 1 | 1 | 1 | 1 |
| RAD50 | 0 | 2 | 0 | 0 | 0.54142213 | 0 | 0 | 1 | 1 | 1 | 1 |
| ACVR1 | 1 | 1 | 0 | 0 | 0.41081609 | 0 | 0 | 1 | 1 | 1 | 1 |
| RPL22 | 1 | 0 | 0 | 0 | 0 | 0 | 0 | 1 | 1 | 1 | 1 |
| TRIM62 | 0 | 1 | 0 | 0 | 0.62125812 | 0 | 0 | 1 | 1 | 1 | 1 |
| CCND2 | 1 | 0 | 0 | 0 | 0 | 0 | 0 | 1 | 1 | 1 | 1 |
| CD274 | 1 | 1 | 0 | 0 | 0.8491144 | 0 | 0 | 1 | 1 | 1 | 1 |
| KCNJ5 | 0 | 1 | 0 | 0 | 0.63919195 | 0 | 0 | 1 | 1 | 1 | 1 |
| HOXC11 | 1 | 1 | 0 | 0 | 0.8287603 | 0 | 0 | 1 | 1 | 1 | 1 |
| STIL | 2 | 3 | 1 | 0 | 0.61119778 | 3.08930025 | 0 | 1 | 1 | 1 | 1 |
| Sep-06 | 3 | 1 | 0 | 0 | 0.43172096 | 0 | 0 | 1 | 1 | 1 | 1 |
| FOXA2 | 1 | 1 | 0 | 0 | 0.44511267 | 0 | 0 | 1 | 1 | 1 | 1 |
| KDM5C | 4 | 7 | 0 | 0 | 0.94834 | 0 | 0 | 1 | 1 | 1 | 1 |
| SBDS | 1 | 0 | 0 | 0 | 0 | 0 | 0 | 1 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| BCL11B | 2 | 2 | 0 | 0 | 0.48501038 | 0 | 0 | 1 | 1 | 1 | 1 |
| TRAF2 | 0 | 1 | 0 | 1 | 0.71167884 | 0 | 20.6943622 | 1 | 1 | 1 | 1 |
| HOXD13 | 1 | 1 | 0 | 0 | 0.55041374 | 0 | 0 | 1 | 1 | 1 | 1 |
| MYOD1 | 0 | 1 | 0 | 0 | 0.69104551 | 0 | 0 | 1 | 1 | 1 | 1 |
| CBFA2T3 | 0 | 1 | 0 | 0 | 0.5074803 | 0 | 0 | 1 | 1 | 1 | 1 |
| MLLT1 | 0 | 1 | 0 | 0 | 0.58886425 | 0 | 0 | 1 | 1 | 1 | 1 |
| MNX1 | 0 | 1 | 0 | 0 | 0.66894426 | 0 | 0 | 1 | 1 | 1 | 1 |
| BTG1 | 1 | 0 | 0 | 0 | 0 | 0 | 0 | 1 | 1 | 1 | 1 |
| MEN1 | 1 | 2 | 0 | 1 | 0.97081723 | 0 | 21.26029 | 1 | 1 | 1 | 1 |
| MYCN | 0 | 1 | 0 | 0 | 0.40593584 | 0 | 0 | 1 | 1 | 1 | 1 |
| CDK4 | 1 | 1 | 0 | 0 | 0.87054328 | 0 | 0 | 1 | 1 | 1 | 1 |
| NKX2-1 | 0 | 1 | 0 | 0 | 0.59456398 | 0 | 0 | 1 | 1 | 1 | 1 |
| PIM1 | 1 | 1 | 0 | 0 | 0.82345935 | 0 | 0 | 1 | 1 | 1 | 1 |
| MAP2K5 | 1 | 1 | 0 | 1 | 0.60306135 | 0 | 5.84538856 | 1 | 1 | 1 | 1 |
| FBXO11 | 0 | 3 | 0 | 0 | 0.95549973 | 0 | 0 | 1 | 1 | 1 | 1 |
| NCOA4 | 0 | 1 | 0 | 0 | 0.50186514 | 0 | 0 | 1 | 1 | 1 | 1 |
| NDST4 | 3 | 1 | 0 | 0 | 0.18933331 | 0 | 0 | 1 | 1 | 1 | 1 |
| MAP3K12 | 1 | 2 | 0 | 1 | 0.72671743 | 0 | 13.7563936 | 1 | 1 | 1 | 1 |
| TCF12 | 0 | 2 | 0 | 0 | 0.81536295 | 0 | 0 | 1 | 1 | 1 | 1 |
| BCL2A1 | 1 | 0 | 0 | 0 | 0 | 0 | 0 | 1 | 1 | 1 | 1 |
| PML | 2 | 1 | 0 | 0 | 0.27622897 | 0 | 0 | 1 | 1 | 1 | 1 |
| GATA6 | 2 | 2 | 0 | 0 | 0.70072179 | 0 | 0 | 1 | 1 | 1 | 1 |
| RPTOR | 4 | 6 | 0 | 0 | 0.99183682 | 0 | 0 | 1 | 1 | 1 | 1 |
| BRD4 | 2 | 3 | 0 | 0 | 0.62217729 | 0 | 0 | 1 | 1 | 1 | 1 |
| CBLC | 0 | 1 | 0 | 0 | 0.76243989 | 0 | 0 | 1 | 1 | 1 | 1 |
| PRDM16 | 3 | 4 | 0 | 0 | 0.70051296 | 0 | 0 | 1 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| IKBKE | 5 | 3 | 0 | 0 | 0.76046468 | 0 | 0 | 1 | 1 | 1 | 1 |
| CXCR7 | 3 | 1 | 0 | 0 | 0.47443424 | 0 | 0 | 1 | 1 | 1 | 1 |
| ARHGAP26 | 0 | 2 | 0 | 0 | 0.78992014 | 0 | 0 | 1 | 1 | 1 | 1 |
| RSPO3 | 0 | 1 | 0 | 0 | 0.94896328 | 0 | 0 | 1 | 1 | 1 | 1 |
| MTDH | 2 | 2 | 0 | 0 | 0.84538789 | 0 | 0 | 1 | 1 | 1 | 1 |
| NFIB | 2 | 2 | 0 | 0 | 0.82601197 | 0 | 0 | 1 | 1 | 1 | 1 |
| TCF7L2 | 0 | 2 | 0 | 0 | 0.99899763 | 0 | 0 | 1 | 1 | 1 | 1 |
| CHEK1 | 0 | 1 | 0 | 0 | 0.63116992 | 0 | 0 | 1 | 1 | 1 | 1 |
| ARID5B | 4 | 4 | 0 | 0 | 0.68195307 | 0 | 0 | 1 | 1 | 1 | 1 |
| FOXO1 | 0 | 1 | 0 | 0 | 0.45678353 | 0 | 0 | 1 | 1 | 1 | 1 |
| JAZF1 | 1 | 0 | 0 | 0 | 0 | 0 | 0 | 1 | 1 | 1 | 1 |
| CHD1 | 3 | 5 | 0 | 0 | 0.57985051 | 0 | 0 | 1 | 1 | 1 | 1 |
| XPC | 2 | 2 | 0 | 0 | 0.53410492 | 0 | 0 | 1 | 1 | 1 | 1 |
| BUB1B | 1 | 2 | 0 | 0 | 0.62435575 | 0 | 0 | 1 | 1 | 1 | 1 |
| FANCC | 2 | 2 | 0 | 0 | 0.80628498 | 0 | 0 | 1 | 1 | 1 | 1 |
| RALGDS | 0 | 2 | 0 | 0 | 0.82997155 | 0 | 0 | 1 | 1 | 1 | 1 |
| PCSK7 | 2 | 2 | 0 | 1 | 0.67959604 | 0 | 9.20500384 | 1 | 1 | 1 | 1 |
| SHC1 | 1 | 1 | 0 | 0 | 0.51709229 | 0 | 0 | 1 | 1 | 1 | 1 |
| FUBP1 | 1 | 2 | 0 | 0 | 0.89043778 | 0 | 0 | 1 | 1 | 1 | 1 |
| RBM15 | 1 | 2 | 0 | 0 | 0.52335221 | 0 | 0 | 1 | 1 | 1 | 1 |
| REL | 2 | 2 | 0 | 0 | 0.72501986 | 0 | 0 | 1 | 1 | 1 | 1 |
| FEV | 1 | 0 | 0 | 0 | 0 | 0 | 0 | 1 | 1 | 1 | 1 |
| TERT | 2 | 2 | 0 | 0 | 0.45082189 | 0 | 0 | 1 | 1 | 1 | 1 |
| ZNF704 | 3 | 0 | 0 | 0 | 0 | 0 | 0 | 1 | 1 | 1 | 1 |
| WRN | 2 | 3 | 0 | 0 | 0.48419197 | 0 | 0 | 1 | 1 | 1 | 1 |
| CDX2 | 1 | 1 | 0 | 0 | 0.71579819 | 0 | 0 | 1 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| NSD1 | 4 | 8 | 1 | 0 | 0.78844077 | 1.35719174 | 0 | 1 | 1 | 1 | 1 |
| RET | 5 | 6 | 0 | 0 | 0.95928189 | 0 | 0 | 1 | 1 | 1 | 1 |
| MAPK7 | 2 | 0 | 0 | 0 | 0 | 0 | 0 | 1 | 1 | 1 | 1 |
| CTNNB1 | 2 | 3 | 0 | 0 | 0.8565748 | 0 | 0 | 1 | 1 | 1 | 1 |
| SF1 | 3 | 1 | 0 | 0 | 0.33433942 | 0 | 0 | 1 | 1 | 1 | 1 |
| LRP1B | 9 | 24 | 3 | 0 | 0.80821514 | 1.57975586 | 0 | 1 | 1 | 1 | 1 |
| TET2 | 3 | 8 | 1 | 0 | 0.95966569 | 1.56028794 | 0 | 1 | 1 | 1 | 1 |
| ZRSR2 | 1 | 1 | 0 | 0 | 0.48940116 | 0 | 0 | 1 | 1 | 1 | 1 |
| ASPSCR1 | 0 | 1 | 0 | 0 | 0.57190038 | 0 | 0 | 1 | 1 | 1 | 1 |
| BRD3 | 1 | 1 | 0 | 0 | 0.39506356 | 0 | 0 | 1 | 1 | 1 | 1 |
| MAP3K2 | 2 | 1 | 0 | 0 | 0.39787791 | 0 | 0 | 1 | 1 | 1 | 1 |
| KIF5B | 3 | 4 | 1 | 0 | 0.92470654 | 2.87801998 | 0 | 1 | 1 | 1 | 1 |
| CHCHD7 | 1 | 0 | 0 | 0 | 0 | 0 | 0 | 1 | 1 | 1 | 1 |
| ASXL1 | 3 | 4 | 1 | 0 | 0.67459046 | 2.76634138 | 0 | 1 | 1 | 1 | 1 |
| BCL2L1 | 1 | 0 | 0 | 0 | 0 | 0 | 0 | 1 | 1 | 1 | 1 |
| BPTF | 5 | 9 | 0 | 1 | 0.77447259 | 0 | 4.1890599 | 1 | 1 | 1 | 1 |
| PLEKHG5 | 0 | 2 | 0 | 0 | 0.72033819 | 0 | 0 | 1 | 1 | 1 | 1 |
| GPHN | 1 | 2 | 0 | 0 | 0.57445945 | 0 | 0 | 1 | 1 | 1 | 1 |
| CAMTA1 | 4 | 5 | 0 | 0 | 0.69205268 | 0 | 0 | 1 | 1 | 1 | 1 |
| MLLT3 | 1 | 1 | 0 | 0 | 0.4269138 | 0 | 0 | 1 | 1 | 1 | 1 |
| MALT1 | 0 | 2 | 0 | 0 | 0.88668132 | 0 | 0 | 1 | 1 | 1 | 1 |
| CLP1 | 1 | 0 | 0 | 0 | 0 | 0 | 0 | 1 | 1 | 1 | 1 |
| TAF15 | 1 | 2 | 0 | 0 | 0.88265182 | 0 | 0 | 1 | 1 | 1 | 1 |
| MYD88 | 2 | 0 | 0 | 0 | 0 | 0 | 0 | 1 | 1 | 1 | 1 |
| MAP3K11 | 4 | 0 | 0 | 0 | 0 | 0 | 0 | 1 | 1 | 1 | 1 |
| PTPRM | 7 | 4 | 0 | 1 | 0.39802391 | 0 | 2.15596499 | 1 | 1 | 1 | 1 |

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| CHD2 | 2 | 4 | 1 | 0 | 0.60342363 | 1.87912708 | 0 | 1 | 1 | 1 | 1 |
| DDX10 | 1 | 1 | 0 | 1 | 0.28410942 | 0 | 7.22981897 | 1 | 1 | 1 | 1 |
| AURKB | 0 | 1 | 0 | 0 | 0.93715545 | 0 | 0 | 1 | 1 | 1 | 1 |
| PER1 | 5 | 5 | 0 | 1 | 0.82443777 | 0 | 4.9476103 | 1 | 1 | 1 | 1 |
| PTPN11 | 2 | 2 | 0 | 0 | 0.82244229 | 0 | 0 | 1 | 1 | 1 | 1 |
| CIITA | 1 | 2 | 0 | 0 | 0.56908912 | 0 | 0 | 1 | 1 | 1 | 1 |
| NRIP1 | 1 | 4 | 0 | 0 | 0.98614666 | 0 | 0 | 1 | 1 | 1 | 1 |
| PRF1 | 4 | 1 | 0 | 0 | 0.31989306 | 0 | 0 | 1 | 1 | 1 | 1 |
| NPM1 | 1 | 1 | 0 | 0 | 0.81878198 | 0 | 0 | 1 | 1 | 1 | 1 |
| PLAG1 | 1 | 1 | 0 | 0 | 0.50369898 | 0 | 0 | 1 | 1 | 1 | 1 |
| P2RY8 | 0 | 1 | 0 | 0 | 0.77272498 | 0 | 0 | 1 | 1 | 1 | 1 |
| EP400 | 7 | 6 | 1 | 0 | 0.47245186 | 1.27188975 | 0 | 1 | 1 | 1 | 1 |
| TMPRSS2 | 1 | 1 | 0 | 1 | 0.50500326 | 0 | 10.7499683 | 1 | 1 | 1 | 1 |
| SS18L1 | 1 | 0 | 0 | 0 | 0 | 0 | 0 | 1 | 1 | 1 | 1 |
| C15orf55 | 1 | 3 | 0 | 0 | 0.87723562 | 0 | 0 | 1 | 1 | 1 | 1 |
| Sep-09 | 1 | 2 | 0 | 1 | 0.87463452 | 0 | 12.3555263 | 1 | 1 | 1 | 1 |
| FAM22A | 0 | 1 | 0 | 0 | 0.46354378 | 0 | 0 | 1 | 1 | 1 | 1 |
| WT1 | 1 | 2 | 0 | 0 | 0.85953984 | 0 | 0 | 1 | 1 | 1 | 1 |
| IKZF1 | 2 | 2 | 0 | 0 | 0.81176875 | 0 | 0 | 1 | 1 | 1 | 1 |
| PTCH1 | 3 | 5 | 0 | 0 | 0.83828 | 0 | 0 | 1 | 1 | 1 | 1 |
| IRS2 | 2 | 1 | 0 | 0 | 0.19609213 | 0 | 0 | 1 | 1 | 1 | 1 |
| TAL2 | 1 | 0 | 0 | 0 | 0 | 0 | 0 | 1 | 1 | 1 | 1 |
| MITF | 0 | 1 | 0 | 0 | 0.53302954 | 0 | 0 | 1 | 1 | 1 | 1 |
| PTPRT | 5 | 8 | 0 | 0 | 0.86852507 | 0 | 0 | 1 | 1 | 1 | 1 |
| SRGAP3 | 4 | 3 | 1 | 0 | 0.51320775 | 3.1637317 | 0 | 1 | 1 | 1 | 1 |
| NCOR2 | 7 | 9 | 0 | 0 | 0.73279451 | 0 | 0 | 1 | 1 | 1 | 1 |

EP 3 452 937 B1

(continued)

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| IGF2R | 6 | 5 | 1 | 0 | 0.45321092 | 1.69385238 | 0 | 1 | 1 | 1 | 1 |
| SRC | 2 | 0 | 0 | 1 | 0 | 0 | 10.1185927 | 1 | 1 | 1 | 1 |
| BLM | 1 | 4 | 0 | 0 | 0.97130112 | 0 | 0 | 1 | 1 | 1 | 1 |
| MAP3K5 | 4 | 3 | 1 | 0 | 0.44723246 | 2.64262834 | 0 | 1 | 1 | 1 | 1 |
| HIST1H4I | 1 | 0 | 0 | 0 | 0 | 0 | 0 | 1 | 1 | 1 | 1 |
| ZNF521 | 3 | 2 | 0 | 0 | 0.31364143 | 0 | 0 | 1 | 1 | 1 | 1 |
| TOP1 | 1 | 2 | 0 | 1 | 0.75853085 | 0 | 7.00833251 | 1 | 1 | 1 | 1 |
| DAXX | 2 | 2 | 0 | 0 | 0.65968819 | 0 | 0 | 1 | 1 | 1 | 1 |
| SDHD | 1 | 0 | 0 | 0 | 0 | 0 | 0 | 1 | 1 | 1 | 1 |
| POU5F1 | 2 | 1 | 0 | 0 | 0.69623669 | 0 | 0 | 1 | 1 | 1 | 1 |
| OLIG2 | 1 | 0 | 0 | 0 | 0 | 0 | 0 | 1 | 1 | 1 | 1 |
| FGFR1OP | 0 | 1 | 0 | 0 | 0.67245987 | 0 | 0 | 1 | 1 | 1 | 1 |
| CHUK | 0 | 2 | 0 | 0 | 0.95827608 | 0 | 0 | 1 | 1 | 1 | 1 |
| CEBPA | 0 | 1 | 0 | 0 | 0.67784151 | 0 | 0 | 1 | 1 | 1 | 1 |
| PIK3R2 | 0 | 2 | 0 | 0 | 0.93515765 | 0 | 0 | 1 | 1 | 1 | 1 |
| KAT6A | 1 | 10 | 0 | 0 | NA | NA | NA | NA | NA | NA | NA |
| SRSF3 | 1 | 1 | 0 | 0 | NA | NA | NA | NA | NA | NA | NA |
| CNTRL | 1 | 9 | 2 | 0 | NA | NA | NA | NA | NA | NA | NA |
| CCDC170 | 3 | 4 | 2 | 0 | NA | NA | NA | NA | NA | NA | NA |
| SPECC1 | 1 | 4 | 0 | 1 | NA | NA | NA | NA | NA | NA | NA |
| KAT6B | 6 | 11 | 0 | 0 | NA | NA | NA | NA | NA | NA | NA |
| SRSF2 | 1 | 4 | 1 | 0 | NA | NA | NA | NA | NA | NA | NA |
| NBPF10 | 3 | 18 | 1 | 0 | NA | NA | NA | NA | NA | NA | NA |
| RAD51B | 1 | 1 | 0 | 0 | NA | NA | NA | NA | NA | NA | NA |
| AMER1 | 6 | 12 | 0 | 0 | NA | NA | NA | NA | NA | NA | NA |
| FAM22B | 1 | 2 | 0 | 0 | NA | NA | NA | NA | NA | NA | NA |

(continued)

| gene_name | n_syn | n_mis | n_non | n_splice | wMIS3 | wNON3 | wSPLICE3 | qMIS3 | qNON3 | qSPLICE3 | qALL3 |
|---|---|---|---|---|---|---|---|---|---|---|---|
| MDS2 | 0 | 1 | 0 | 0 | NA | NA | NA | NA | NA | NA | NA |

Table 4

| gene_name | n_syn | n_mis | n_non | n_splice | num_indels | num_unique | wMIS3 | wNON3 | wSPLICE3 | wIND | qMIS_tiered | qNON_tiered | qSPLICE_tiered | qIND_tiered | gALL_tiered | knownCG |
|---|---|---|---|---|---|---|---|---|---|---|---|---|---|---|---|---|
| TP53 | 4 | 263 | 70 | 32 | 85 | 77 | 138.488624 | 557.103224 | 435.485088 | 491.078996 | 0 | 0 | 0 | 1.86E-81 | 0 | TRUE |
| MAP3K1 | 4 | 19 | 19 | 0 | 90 | 85 | 2.7954968 | 36.434436 | 0 | 146.649598 | 0.0352479 | 0 | 0.93544783 | 2.35E-49 | 0 | TRUE |
| CDH1 | 2 | 12 | 19 | 11 | 45 | 45 | 3.75225448 | 117.8965 | 104.178082 | 130.981342 | 0.02547274 | 0 | 0 | 3.81 E-33 | 0 | TRUE |
| PTEN | 1 | 15 | 9 | 5 | 29 | 26 | 10.3690274 | 81.6152935 | 81.6601712 | 163.271741 | 1.27E-06 | 1.91E-11 | 2.81E-06 | 7.11E-26 | 0 | TRUE |
| MAP2K4 | 0 | 19 | 6 | 4 | 17 | 15 | 10.3220662 | 48.8633638 | 39.2304743 | 93.619997 | 3.27E-07 | 1.82E-06 | 0.00090259 | 3.55E-14 | 0 | TRUE |
| PIK3CA | 3 | 386 | 0 | 0 | 23 | 19 | 76.123425 | 0 | 0 | 45.5963551 | 0 | 0.71299944 | 0.93544783 | 1.01E-11 | 0 | TRUE |
| CBFB | 2 | 16 | 4 | 8 | 7 | 7 | 19.5403867 | 75.6426793 | 178.98812 | 91.7195911 | 1.25E-10 | 2.77E-05 | 8.33E-13 | 5.78E-08 | 0 | TRUE |
| AKT1 | 1 | 36 | 0 | 0 | 0 | 0 | 20.0319312 | 0 | 0 | 0 | 0 | 0.71299944 | 0.93544783 | 1 | 0 | TRUE |
| RB1 | 2 | 8 | 12 | 5 | 11 | 11 | 2.20904394 | 33.770558 | 23.1699851 | 29.4202193 | 0.15297243 | 7.96E-11 | 0.00090259 | 1.37E-06 | 0 | TRUE |
| MLL3 | 10 | 37 | 26 | 3 | 45 | 41 | 1.40386545 | 10.8395917 | 4.3481643 | 21.8727654 | 0.15297243 | 1.12E-11 | 0.93544783 | 6.78E-10 | 0 | TRUE |
| NCOR1 | 3 | 13 | 17 | 3 | 14 | 14 | 1.30913921 | 19.0002426 | 8.46074209 | 14.7261096 | 0.15297243 | 6.61E-11 | 0.4200982 | 9.72E-05 | 0 | TRUE |
| TBX3 | 2 | 10 | 4 | 1 | 21 | 21 | 2.4396338 | 28.0431898 | 13.0038525 | 74.2643056 | 0.15297243 | 0.00125227 | 0.93544783 | 6.98E-16 | 0 | TRUE |
| GATA3 | 6 | 12 | 2 | 1 | 123 | 82 | 3.59067666 | 13.4933071 | 15.7733242 | 484.075094 | 0.02464771 | 0.2756131 | 0.93544783 | 4.05E-84 | 0 | TRUE |
| RUNX1 | 3 | 8 | 1 | 2 | 19 | 17 | 3.26818984 | 7.60309507 | 28.2136847 | 91.2636171 | 0.09658042 | 0.71299944 | 0.21070238 | 2.99E-15 | 0 | TRUE |
| PIK3R1 | 1 | 10 | 0 | 1 | 19 | 18 | 3.37078915 | 0 | 9.77442621 | 63.299198 | 0.05597865 | 0.71299944 | 0.93544783 | 1.87E-13 | 3.76E-14 | TRUE |
| ARID1A | 2 | 11 | 9 | 0 | 16 | 16 | 1.64223196 | 19.2913706 | 0 | 18.5347609 | 0.15297243 | 2.51E-06 | 0.93544783 | 5.39E-06 | 5.64E-11 | TRUE |
| CTCF | 4 | 17 | 5 | 2 | 3 | 3 | 5.22043907 | 24.4751871 | 24.9014379 | 10.7392466 | 0.00024996 | 0.00025515 | 0.23317832 | 0.15050351 | 2.10E-10 | TRUE |
| CDKN1B | 0 | 2 | 4 | 0 | 5 | 5 | 3.01950891 | 87.558331 | 0 | 66.2572642 | 0.15297243 | 2.06E-05 | 0.99556259 | 2.14E-05 | 1.26E-09 | TRUE |
| FOXA1 | 2 | 20 | 1 | 0 | 6 | 6 | 6.74879485 | 9.9288163 | 0 | 34.090951 | 1.46E-05 | 0.71299944 | 1 | 9.72E-05 | 6.32E-09 | TRUE |
| MED23 | 0 | 8 | 6 | 2 | 7 | 7 | 1.63744855 | 15.2661498 | 9.30905397 | 13.0163805 | 0.15297243 | 0.00083263 | 0.93544783 | 0.0056081 | 4.24E-07 | TRUE |
| NF1 | 1 | 12 | 6 | 2 | 12 | 12 | 1.58124769 | 10.3123267 | 6.08953756 | 10.7817315 | 0.15297243 | 0.00505293 | 0.93544783 | 0.00216769 | 1.48E-06 | TRUE |
| SPEN | 7 | 13 | 10 | 0 | 14 | 13 | 0.85697778 | 8.88329556 | 0 | 9.53700344 | 0.15297243 | 0.00016458 | 0.93544783 | 0.00374824 | 1.56E-05 | TRUE |
| ZFP36L1 | 0 | 3 | 1 | 0 | 8 | 8 | 2.78086494 | 22.1661308 | | 63.0753821 | 0.19768575 | 0.92828942 | 1 | 3.81 E-05 | 3.92E-05 | FALSE |
| HIST1H3B | 3 | 7 | 1 | 0 | 2 | 2 | 7.29835567 | 25.3590889 | 0 | 38.8415506 | 0.00517301 | 0.5015476 | 1 | 0.05550329 | 0.00023971 | TRUE |
| MLLT4 | 0 | 9 | 5 | 0 | 7 | 7 | 1.55544763 | 10.7826997 | | 10.8890839 | 0.15297243 | 0.01252194 | 0.93544783 | 0.01236044 | 0.000246 | TRUE |
| TBL1XR1 | 2 | 5 | 1 | 2 | 6 | 4 | 2.22311297 | 6.3812303 | 15.1099108 | 19.4092496 | 0.15297243 | 0.71299944 | 0.4200982 | 0.01236044 | 0.00027949 | TRUE |
| BRCA2 | 0 | 11 | 7 | 1 | 6 | 6 | 1.37155115 | 10.1465189 | 7.35202875 | 4.66453156 | 0.15297243 | 0.0023492 | 0.93544783 | 0.33258197 | 0.00037238 | TRUE |
| BRCA1 | 1 | 10 | 3 | 2 | 5 | 5 | 1.90201914 | 7.09664197 | 15.128767 | 6.94659848 | 0.15297243 | 0.2756131 | 0.44284817 | 0.14895162 | 0.00064594 | TRUE |

65

| gene_name | n_syn | n_mis | n_non | n_splice | num_indels | num_unique | wMIS3 | wNON3 | wSPLICE3 | wIND | qMIS_tiered | qNON_tiered | qSPLICE_tiered | qIND_tiered | gALL_tiered | knownCG |
|---|---|---|---|---|---|---|---|---|---|---|---|---|---|---|---|---|
| XBP1 | 0 | 2 | 0 | 0 | 7 | 7 | 2.12190828 | 0 | 0 | 68.460556 | 0.19768575 | 0.92828942 | 1 | 6.49E-05 | 0.00068641 | FALSE |
| HLA-DRB1 | 2 | 2 | 0 | 2 | 7 | 5 | 1.64101588 | 0 | 44.5569403 | 47.480525 | 0.19768575 | 0.92828942 | 1 | 0.0139135 | 0.00132798 | FALSE |
| SF3B1 | 2 | 27 | 1 | 0 | 0 | 0 | 5.70556891 | 2.64328888 | 0 | 0 | 1.14E-05 | 0.71299944 | 0.93544783 | 1 | 0.00175413 | TRUE |
| FOXP1 | 1 | 3 | 2 | 3 | 2 | 2 | 1.0287299 | 7.71836497 | 19.8854446 | 7.43286037 | 0.15361396 | 0.48663863 | 0.06726665 | 0.54179194 | 0.00257002 | TRUE |
| CASP8 | 0 | 9 | 3 | 0 | 0 | 0 | 5.68481037 | 22.6341116 | 0 | 0 | 0.00826565 | 0.0155157 | 0.93544783 | 1 | 0.00334101 | TRUE |
| MYB | 0 | 6 | 1 | 0 | 5 | 5 | 2.49389655 | 4.97250701 | 0 | 16.7133001 | 0.15297243 | 0.71299944 | 0.93544783 | 0.00797363 | 0.004917 | TRUE |
| ERBB2 | 3 | 23 | 0 | 0 | 1 | 1 | 5.20498261 | 0 | 0 | 2.02480099 | 8.21E-05 | 0.71299944 | 0.93544783 | 1 | 0.01001401 | TRUE |
| FBXW7 | 0 | 8 | 2 | 0 | 2 | 2 | 3.57500988 | 9.23945744 | 0 | 7.31973716 | 0.06994747 | 0.35456599 | 0.93544783 | 0.54516735 | 0.02874291 | TRUE |
| SETD2 | 2 | 11 | 4 | 0 | 6 | 6 | 1.32813823 | 5.20497855 | 0 | 6.20580372 | 0.15297243 | 0.2756131 | 0.93544783 | 0.14895162 | 0.03331321 | TRUE |
| CRIPAK | 2 | 9 | 0 | 0 | 7 | 5 | 4.10671324 | 0 | 0 | 30.2596092 | 0.19768575 | 0.92828942 | 1 | 0.08344053 | 0.04077807 | FALSE |
| MEN1 | 1 | 2 | 0 | 1 | 3 | 3 | 1.03690891 | 0 | 23.4208983 | 12.6565734 | 0.15954276 | 0.71299944 | 0.93544783 | 0.12448104 | 0.08141925 | TRUE |
| SMAD4 | 0 | 3 | 2 | 0 | 2 | 2 | 1.70230648 | 14.5739802 | 0 | 9.243806 | 0.15297243 | 0.23434298 | 0.93544783 | 0.43969985 | 0.08905229 | TRUE |
| ATM | 1 | 17 | 3 | 1 | 5 | 5 | 1.89433576 | 3.68899242 | 2.48365082 | 4.17533776 | 0.15297243 | 0.70927608 | 0.93544783 | 0.47819862 | 0.0902412 | TRUE |
| ERBB3 | 1 | 16 | 0 | 0 | 2 | 2 | 4.17075849 | 0 | 0 | 3.79491595 | 0.00729729 | 0.71299944 | 0.93544783 | 0.99031135 | 0.11587142 | TRUE |
| ESR1 | 1 | 7 | 0 | 0 | 3 | 3 | 2.68617047 | 0 | 0 | 13.1308561 | 0.15297243 | 0.71299944 | 0.93544783 | 0.11758048 | 0.1369056 | TRUE |
| HSP90AB1 | 3 | 2 | 0 | 0 | 4 | 4 | 0.6504521 | 0 | 0 | 14.3127289 | 0.15297243 | 0.71299944 | 0.93544783 | 0.0339739 | 0.15882784 | TRUE |
| NCOA3 | 0 | 10 | 0 | 0 | 22 | 4 | 2.6632142 | 0 | 0 | 7.29199234 | 0.1251193 | 0.71299944 | 0.93544783 | 0.18735613 | 0.17233662 | TRUE |
| STK11 | 0 | 2 | 2 | 0 | 1 | 1 | 1.45134584 | 32.6684624 | 0 | 5.8736684 | 0.15902119 | 0.05877097 | 0.93544783 | 1 | 0.19619531 | TRUE |
| DLG1 | 0 | 8 | 2 | 2 | 3 | 3 | 2.8155853 | 8.31228668 | 11.0356302 | 8.09252366 | 0.19768575 | 0.92828942 | 1 | 1 | 0.20376222 | FALSE |
| ZFHX3 | 5 | 11 | 7 | 0 | 3 | 3 | 0.79654104 | 7.49950559 | 0 | 2.18964821 | 0.15297243 | 0.00761024 | 0.94950488 | 1 | 0.20564876 | TRUE |
| NPAS4 | 2 | 10 | 4 | 0 | 0 | 0 | 3.91834852 | 24.9580965 | 0 | 0 | 0.19768575 | 0.36928655 | 1 | 1 | 0.23982079 | FALSE |
| NLRP2 | 3 | 6 | 3 | 3 | 1 | 1 | 1.23910738 | 9.90973648 | 26.1771189 | 2.47088136 | 0.19768746 | 0.92828942 | 1 | 1 | 0.23982079 | FALSE |
| HSP90AA1 | 1 | 4 | 2 | 0 | 2 | 2 | 1.51357789 | 10.5197255 | 0 | 6.09023941 | 0.15297243 | 0.34253923 | 0.93544783 | 0.64243852 | 0.24055196 | TRUE |
| FIP1L1 | 2 | 4 | 0 | 0 | 4 | 3 | 1.35809011 | 0 | 0 | 12.4826663 | 0.15297243 | 0.71299944 | 0.93544783 | 0.12448104 | 0.26344225 | TRUE |
| PSIP1 | 1 | 4 | 0 | 1 | 2 | 2 | 1.75181935 | 0 | 8.00097928 | 9.3816941 | 0.15297243 | 0.71299944 | 0.93544783 | 0.43969985 | 0.29588875 | TRUE |
| FOXO3 | 0 | 3 | 0 | 0 | 3 | 3 | 1.28170049 | 0 | 0 | 12.0119683 | 0.15740361 | 0.71299944 | 0.93544783 | 1 | 0.35248986 | TRUE |
| EIF4A2 | 1 | 5 | 0 | 1 | 1 | 1 | 3.18697649 | 0 | 11.602533 | 6.12904528 | 0.13360195 | 0.71299944 | 0.93544783 | 1 | 0.35248986 | TRUE |
| C11orf30 | 1 | 13 | 2 | 0 | 0 | 0 | 3.30338437 | 6.09408889 | 0 | 0 | 0.03750657 | 0.61572599 | 0.93544783 | 1 | 0.35390454 | TRUE |

| gene_name | n_syn | n_mis | n_non | n_splice | num_indels | num_unique | wMIS3 | wNON3 | wSPLICE3 | wIND | qMIS_tiered | qNON_tiered | qSPLICE_tiered | qIND_tiered | gALL_tiered | knownCG |
|---|---|---|---|---|---|---|---|---|---|---|---|---|---|---|---|---|
| MLL | 4 | 9 | 3 | 1 | 7 | 7 | 0.7513908 | 3.02314267 | 4.7129281 | 4.66104934 | 0.15297243 | 0.71299944 | 0.93544783 | 0.28285002 | 0.35696303 | TRUE |
| MET | 0 | 7 | 0 | 0 | 4 | 4 | 1.82066829 | 0 | 0 | 7.38752781 | 0.15297243 | 0.71299944 | 0.93544783 | 0.18735613 | 0.36010944 | TRUE |
| PDGFB | 0 | 1 | 0 | 0 | 2 | 2 | 1.19959323 | 0 | 0 | 20.8044438 | 0.15361396 | 0.71869802 | 0.93544783 | 0.14696679 | 0.36153822 | TRUE |
| KCNB2 | 3 | 11 | 2 | 0 | 5 | 5 | 2.06691166 | 6.15905017 | 0 | 14.8333571 | 0.19768575 | 0.92828942 | 1 | 0.92155554 | 0.36445965 | FALSE |
| KRAS | 0 | 6 | 0 | 0 | 0 | 0 | 8.90364274 | 0 | 0 | 0 | 0.00498387 | 0.71299944 | 0.93544783 | 1 | 0.37189496 | TRUE |
| PRKAR1A | 3 | 1 | 3 | 0 | 1 | 1 | 0.46080145 | 15.4342491 | 0 | 6.56191525 | 0.15297243 | 0.03874075 | 0.93544783 | 1 | 0.39425118 | TRUE |
| AFF3 | 5 | 8 | 2 | 0 | 3 | 3 | 1.05214657 | 4.09464869 | 0 | 6.15364757 | 0.15619817 | 0.71299944 | 0.93544783 | 0.43969985 | 0.39425118 | TRUE |
| PRRX1 | 1 | 7 | 1 | 0 | 0 | 0 | 4.77341195 | 11.7874106 | 0 | 0 | 0.03749549 | 0.71299944 | 0.94950488 | 1 | 0.39425118 | TRUE |
| UNC13C | 6 | 16 | 6 | 0 | 2 | 2 | 1.01625745 | 4.05748896 | 0 | 2.34778073 | 0.15297243 | 0.23434298 | 0.93544783 | 1 | 0.39827511 | TRUE |
| HSPA8 | 0 | 2 | 0 | 0 | 3 | 3 | 0.91581443 | 0 | 0 | 12.1367834 | 0.15297243 | 0.71299944 | 0.93544783 | 0.12826555 | 0.40485531 | TRUE |
| AXL | 0 | 11 | 0 | 0 | 3 | 1 | 4.31725672 | 0 | 0 | 2.83401955 | 0.02159905 | 0.71299944 | 0.93544783 | 1 | 0.4176086 | TRUE |
| NF2 | 1 | 3 | 1 | 1 | 1 | 1 | 1.52204324 | 6.85852151 | 10.1653495 | 4.19720041 | 0.15297243 | 0.71299944 | 0.93544783 | 1 | 0.4176086 | TRUE |
| PREX2 | 3 | 27 | 1 | 0 | 0 | 0 | 3.05065981 | 1.67564854 | 0 | 0 | 0.01163325 | 0.71299944 | 0.93544783 | 1 | 0.4176086 | TRUE |
| MYH9 | 6 | 9 | 2 | 1 | 3 | 3 | 1.08318444 | 4.2381983 | 2.96874522 | 3.90393749 | 0.15902119 | 0.71299944 | 0.93544783 | 0.76398267 | 0.4176086 | TRUE |
| ATIC | 1 | 3 | 1 | 0 | 2 | 2 | 1.26343696 | 7.092333 | 0 | 8.43336401 | 0.1531288 | 0.71299944 | 0.93544783 | 0.47819862 | 0.4176086 | TRUE |
| PTPRD | 3 | 21 | 4 | 1 | 0 | 0 | 1.86323136 | 4.76194509 | 2.32266152 | 0 | 0.15297243 | 0.34253923 | 0.93544783 | 1 | 0.4176086 | TRUE |
| KEAP1 | 0 | 2 | 1 | 0 | 2 | 2 | 1.04233042 | 8.77017291 | 0 | 8.49469757 | 0.16419825 | 0.71299944 | 0.99149881 | 0.47819862 | 0.42226248 | TRUE |
| ZMYM2 | 0 | 6 | 1 | 0 | 3 | 3 | 1.5625817 | 2.54275361 | 0 | 5.62063227 | 0.15297243 | 0.71299944 | 0.93544783 | 0.47819862 | 0.42226248 | TRUE |
| NOTCH4 | 4 | 12 | 1 | 0 | 4 | 4 | 1.54879124 | 2.27187039 | 0 | 5.18133486 | 0.15297243 | 0.71299944 | 0.93544783 | 0.42744241 | 0.42226248 | TRUE |
| IL7R | 0 | 5 | 1 | 0 | 1 | 1 | 3.13381403 | 8.18997554 | 0 | 5.60010028 | 0.15297243 | 0.71299944 | 0.93544783 | 1 | 0.42226248 | TRUE |
| CASC5 | 1 | 10 | 2 | 0 | 3 | 3 | 1.68301855 | 4.20514849 | 0 | 3.36513091 | 0.15297243 | 0.71299944 | 0.93544783 | 0.8901381 | 0.42951487 | TRUE |
| GRIN2A | 1 | 15 | 1 | 0 | 2 | 2 | 2.49976259 | 3.16527583 | 0 | 3.62177028 | 0.12812869 | 0.71299944 | 0.93544783 | 0.99850374 | 0.4316877 | TRUE |
| LIFR | 1 | 4 | 3 | 0 | 1 | 1 | 1.01926759 | 7.96112807 | 0 | 2.34676992 | 0.15297243 | 0.23434298 | 0.93544783 | 1 | 0.4316877 | TRUE |
| NTRK1 | 1 | 12 | 0 | 0 | 0 | 0 | 4.79785374 | 0 | 0 | 0 | 0.00818464 | 0.71299944 | 0.93544783 | 1 | 0.44993119 | TRUE |
| MAP3K13 | 2 | 7 | 1 | 1 | 1 | 1 | 1.8490394 | 3.0913418 | 11.1184966 | 2.69750789 | 0.15297243 | 0.71299944 | 0.93544783 | 1 | 0.45781299 | TRUE |
| LZTR1 | 0 | 1 | 1 | 1 | 2 | 2 | 0.43295886 | 9.53618856 | 9.81304636 | 5.98327582 | 0.15297243 | 0.71299944 | 0.93544783 | 0.65057061 | 0.46963592 | TRUE |
| IL6ST | 0 | 8 | 1 | 0 | 1 | 1 | 3.13083735 | 4.37438404 | 0 | 2.81257186 | 0.10324597 | 0.71299944 | 0.93544783 | 1 | 0.46963592 | TRUE |
| EXT1 | 0 | 4 | 1 | 0 | 2 | 2 | 1.51877834 | 5.7533407 | 0 | 6.9554627 | 0.15297243 | 0.71299944 | 0.93544783 | 0.56417325 | 0.47445889 | TRUE |

| gene_name | n_syn | n_mis | n_non | n_splice | num_indels | num_unique | wMIS3 | wNON3 | wSPLICE3 | wIND | qMIS_tiered | qNON_tiered | qSPLICE_tiered | qIND_tiered | gALL_tiered | knownCG |
|---|---|---|---|---|---|---|---|---|---|---|---|---|---|---|---|---|
| ARID1B | 1 | 11 | 2 | 0 | 3 | 3 | 1.57896323 | 4.75967821 | 0 | 3.52927183 | 0.15297243 | 0.71299944 | 0.93544783 | 0.84894876 | 0.48129522 | TRUE |
| SET | 0 | 1 | 1 | 0 | 1 | 1 | 1.06678984 | 14.9433637 | 0 | 8.57937713 | 0.15297243 | 0.64465098 | 0.93544783 | 0.9576052 | 0.48129522 | TRUE |
| JAK1 | 1 | 4 | 2 | 1 | 2 | 2 | 0.94871951 | 7.78868431 | 5.56877378 | 4.41357431 | 0.15361396 | 0.5015476 | 0.93544783 | 0.8901381 | 0.48129522 | TRUE |
| RPL5 | 0 | 1 | 0 | 0 | 2 | 2 | 0.99364994 | 0 | 0 | 16.7888017 | 0.15297243 | 0.71299944 | 0.93544783 | 0.18735613 | 0.48129522 | TRUE |
| EWSR1 | 3 | 7 | 1 | 0 | 1 | 1 | 2.62176317 | 5.27373787 | 0 | 3.7747675 | 0.13367276 | 0.71299944 | 0.93544783 | 1 | 0.48881814 | TRUE |
| SOX10 | 1 | 3 | 0 | 0 | 2 | 2 | 1.87092025 | 0 | 0 | 11.4271788 | 0.15297243 | 0.71299944 | 1 | 0.33258197 | 0.50957773 | TRUE |
| TRA2B | 0 | 5 | 0 | 3 | 0 | 0 | 4.1997784 | 0 | 45.6566649 | 0 | 0.19768575 | 0.92828942 | 0.94091492 | 1 | 0.52328392 | FALSE |
| CACNA1 D | 3 | 16 | 2 | 0 | 2 | 2 | 2.01236829 | 3.91370469 | 0 | 2.32408937 | 0.15297243 | 0.71299944 | 0.93544783 | 1 | 0.53506929 | TRUE |
| COL1A1 | 3 | 8 | 0 | 1 | 3 | 3 | 1.35747354 | 0 | 2.80230466 | 5.00070117 | 0.15297243 | 0.71299944 | 0.93544783 | 0.55433803 | 0.54387325 | TRUE |
| GRB7 | 0 | 9 | 0 | 0 | 0 | 0 | 5.36751998 | 0 | 0 | 0 | 0.01163325 | 0.71299944 | 0.93544783 | 1 | 0.54387325 | TRUE |
| NOTCH2 | 8 | 11 | 4 | 1 | 3 | 3 | 0.80094926 | 4.55991526 | 2.71098769 | 3.1625115 | 0.15297243 | 0.34253923 | 0.93544783 | 0.94559677 | 0.54903356 | TRUE |
| BAG4 | 2 | 1 | 1 | 0 | 2 | 2 | 0.57391824 | 5.69615559 | 0 | 11.4833517 | 0.15297243 | 0.71299944 | 0.93556656 | 0.33258197 | 0.55385901 | TRUE |
| AURKA | 0 | 4 | 1 | 0 | 1 | 1 | 2.25355895 | 5.81593829 | 0 | 6.32828669 | 0.15297243 | 0.71299944 | 0.93544783 | 1 | 0.55385901 | TRUE |
| CREBBP | 1 | 9 | 2 | 0 | 3 | 3 | 1.46380633 | 4.2887656 | 0 | 3.21094898 | 0.15297243 | 0.71299944 | 0.93544783 | 0.93198736 | 0.56667723 | TRUE |
| ARNT | 0 | 5 | 2 | 0 | 0 | 0 | 2.29464241 | 12.4951285 | 0 | 0 | 0.15297243 | 0.2756131 | 0.93544783 | 1 | 0.56937858 | TRUE |
| NIN | 2 | 11 | 2 | 1 | 1 | 1 | 1.67713952 | 3.13635947 | 5.8847693 | 1.24769516 | 0.15297243 | 0.71299944 | 0.93544783 | 1 | 0.62089606 | TRUE |

**Table 5**

| Chr | Start | End | Gene | Alternative_Gene_name | Gene_source/PMID | Paper title |
|---|---|---|---|---|---|---|
| 10 | 27035522 | 27149959 | ABI1 | | Cancer_Gene_Census | |
| 9 | 133589333 | 133763062 | ABL1 | | Cancer_Gene_Census | |
| 1 | 179068462 | 179198819 | ABL2 | | Cancer_Gene_Census | |
| 2 | 223725652 | 223809357 | ACSL3 | | Cancer_Gene_Census | |
| 5 | 131142683 | 131347936 | ACSL6 | FACL6 | Cancer_Gene_Census | |
| 2 | 158592958 | 158732374 | ACVR1 | ACVR1/ALK2 | Cancer_Gene_Census | |
| 2 | 148602086 | 148688393 | ACVR2A | | Cancer_Related_Genes_Pane l | |
| 19 | 36208921 | 36229779 | AD000671.3 | MLL4 | PMID: 24292195 | Mutational landscape of gingivo-buccal oral squamous cell carcinoma reveals new recurrently-mutated genes and molecular subgroups. |
| 4 | 157750819 | 175899331 | ADAM29 | | Cancer_Related_Genes_Pane l | Mutation detection in formalin-fixed prostate cancer biopsies taken at the time of diagnosis using next-generation DNA sequencing. |
| 4 | 87856154 | 88062190 | AFF1 | M LLT2 | Cancer_Gene_Census | |
| 2 | 100163718 | 100759201 | AFF3 | LAF4 | Cancer_Gene_Census | |
| 5 | 132211071 | 132299326 | AFF4 | AF5q31 | Cancer_Gene_Census | |
| 7 | 91570181 | 91739989 | AKAP9 | | Cancer_Gene_Census | |
| 14 | 105235689 | 105262080 | AKT1 | | Cancer_Gene_Census | |
| 19 | 40736224 | 40791302 | AKT2 | | Cancer_Gene_Census | |
| 1 | 243651535 | 244013430 | AKT3 | | Cancer_Related_Genes_Pane l | |
| 12 | 112204346 | 112247782 | ALDH2 | | Cancer_Gene_Census | |
| 2 | 29415640 | 30144432 | ALK | | Cancer_Gene_Census | |
| 5 | 112043195 | 112181936 | APC | | Cancer_Gene_Census | |
| X | 66764465 | 66950461 | AR | | Cancer_Related_Genes_Pane l | |

(continued)

| Chr | Start | End | Gene | Alternative_Gene_name | Gene_source/PMID | Paper title |
|---|---|---|---|---|---|---|
| X | 47420516 | 47431319 | ARAF | | Cancer_Related_Genes_Panel | |
| 20 | 62329996 | 62339355 | ARFRP1 | | Cancer_Related_Genes_Panel | |
| 5 | 142149949 | 142608576 | ARHGAP26 | GRAF | Cancer_Gene_Census | |
| 11 | 120207946 | 120360645 | ARHGEF12 | | Cancer_Gene_Census | |
| 1 | 27022522 | 27108601 | ARID1A | | Cancer_Gene_Census | |
| 6 | 157099063 | 157530401 | ARID1B | | Cancer_Gene_Census | |
| 12 | 46123492 | 46301823 | ARID2 | | Cancer_Gene_Census | |
| 10 | 63661059 | 63856703 | ARID5B | | PMID: 23636398 | Integrated genomic characterization of endometrial carcinoma. |
| 1 | 150782181 | 150849244 | ARNT | | Cancer_Gene_Census | |
| 17 | 79935426 | 79975280 | ASPSCR1 | | Cancer_Gene_Census | |
| 20 | 30946153 | 31027122 | ASXL1 | | Cancer_Gene_Census | |
| 12 | 51157819 | 51214907 | ATF1 | | Cancer_Gene_Census | |
| 2 | 216176540 | 216214487 | ATIC | | Cancer_Gene_Census | |
| 11 | 108093559 | 108239826 | ATM | | Cancer_Gene_Census | |
| 1 | 116915290 | 116952883 | ATP1A1 | | Cancer_Gene_Census | |
| X | 152783134 | 152848397 | ATP2B3 | | Cancer_Gene_Census | |
| 3 | 142168077 | 142297668 | ATR | | Cancer_Gene_Census | |
| X | 76760359 | 77041719 | ATRX | | Cancer_Gene_Census | |
| 20 | 54944445 | 54967393 | AURKA | | Cancer_Related_Genes_Panel | |
| 17 | 8108051 | 8113936 | AURKB | | Cancer_Related_Genes_Panel | |
| 16 | 337440 | 402673 | AXIN1 | | Cancer Gene Census | |
| 17 | 63524685 | 63557765 | AXIN2 | | Cancer Gene Census | |

| Chr | Start | End | Gene | Alternative_Gene_name | Gene_source/PMID | Paper title |
|---|---|---|---|---|---|---|
| 19 | 41725108 | 41767670 | AXL | | Cancer_Related_Genes_Panel | Mutation detection in formalin-fixed prostate cancer biopsies taken at the time of diagnosis using next-qeneration DNA sequencing. |
| 8 | 38034312 | 38070809 | BAG4 | | Cancer_Related_Genes_Panel | |
| 3 | 52435029 | 52444366 | BAP1 | | Cancer_Gene_Census | |
| 1 | 85731464 | 85743771 | BCL10 | | Cancer_Gene_Census | |
| 2 | 60678302 | 60780768 | BCL11A | | Cancer_Gene_Census | |
| 14 | 99635624 | 99737861 | BCL11B | | Cancer_Gene_Census | |
| 18 | 60790579 | 60987361 | BCL2 | | Cancer_Gene_Census | |
| 15 | 80253231 | 80263788 | BCL2A1 | | Cancer_Related_Genes_Panel | |
| 15 | 52401460 | 52404972 | BCL2L1 | | Cancer_Related_Genes_Panel | |
| 14 | 23776026 | 23780968 | BCL2L2 | | Cancer_Related_Genes_Panel | |
| 19 | 45250962 | 45263301 | BCL3 | | Cancer_Gene_Census | |
| 3 | 187439165 | 187463515 | BCL6 | | Cancer_Gene_Census | |
| 12 | 122459861 | 122499950 | BCL7A | | Cancer_Gene_Census | |
| 1 | 147013182 | 147098017 | BCL9 | | Cancer_Gene_Census | |
| X | 39909068 | 40036582 | BCOR | | Cancer_Gene_Census | |
| 22 | 23521891 | 23660224 | BCR | | Cancer_Gene_Census | |
| 11 | 102217966 | 102249394 | BIRC2 | | Cancer_Related_Genes_Panel | |
| 11 | 102188194 | 102208448 | BIRC3 | | Cancer Gene Census | |
| 20 | 61867235 | 61871859 | BIRC7 | | Cancer_Related_Genes_Panel | |
| 15 | 91260558 | 91358692 | BLM | | Cancer_Gene_Census | |

| Chr | Start | End | Gene | Alternative_Gene_name | Gene_source/PMID | Paper title |
|---|---|---|---|---|---|---|
| 10 | 88516396 | 88684945 | BMPR1A | | Cancer_Gene_Census | |
| 17 | 65821640 | 66033571 | BPTF | | Cancer_Related_Genes_Panel | |
| 7 | 140424943 | 140624564 | BRAF | | Cancer_Gene_Census | |
| 17 | 41196312 | 41277500 | BRCA1 | | Cancer_Gene_Census | |
| 13 | 32889611 | 32973347 | BRCA2 | | Cancer_Gene_Census | |
| 9 | 136897963 | 136933657 | BRD3 | | Cancer_Gene_Census | |
| 19 | 15348301 | 15391262 | BRD4 | | Cancer_Gene_Census | |
| 17 | 59759985 | 59940755 | BRIP1 | | Cancer_Gene_Census | |
| 12 | 92534074 | 92539673 | BTG1 | | Cancer_Gene_Census | |
| 15 | 40453210 | 40513378 | BUB1B | | Cancer_Gene_Census | |
| 11 | 76156069 | 76262586 | C11orf30 | | Cancer_Related_Genes_Panel | |
| 15 | 45803334 | 45848928 | C15orf21 | HMGN2P46 | Cancer_Gene_Census | |
| 15 | 34635834 | 34649933 | C15orf55 | NUTM1 | Cancer_Gene_Census | |
| 16 | 11410636 | 11445619 | C16orf75 | RMI2 | Cancer_Gene_Census | |
| 2 | 24252210 | 24272445 | C2orf44 | | Cancer_Gene_Census | |
| 6 | 151815175 | 151942328 | C6orf97 | CCDC170 | PMID: 25099679 | Recurrent ESR1-CCDC170 rearrangements in an aggressive subset of oestrogen receptor-positive breast cancers |
| 3 | 53528683 | 53846490 | CACNA1D | | Cancer_Gene_Census | |
| 19 | 13049414 | 13055304 | CALR | | Cancer_Gene_Census | |
| 1 | 6845384 | 7829764 | CAMTA1 | | Cancer_Gene_Census | |
| 17 | 76987799 | 77005838 | CANT1 | | Cancer_Gene_Census | |
| 7 | 2945775 | 3083579 | CARD11 | | Cancer_Gene_Census | |
| 11 | 3022152 | 3078667 | CARS | | Cancer_Gene_Census | |
| 15 | 40886447 | 40954881 | CASC5 | AF15Q14 | Cancer_Gene_Census | |

EP 3 452 937 B1

(continued)

| Chr | Start | End | Gene | Alternative_Gene_name | Gene_source/PMID | Paper title |
|---|---|---|---|---|---|---|
| 2 | 202098166 | 202152434 | CASP8 | | Cancer_Gene_Census | |
| 16 | 88941266 | 89043401 | CBFA2T3 | | Cancer_Gene_Census | |
| 16 | 67063050 | 67134961 | CBFB | | Cancer_Gene_Census | |
| 11 | 119076990 | 119178856 | CBL | | Cancer_Gene_Census | |
| 3 | 105374305 | 105588396 | CBLB | | Cancer_Gene_Census | |
| 19 | 45281126 | 45303891 | CBLC | | Cancer_Gene_Census | |
| 10 | 61548521 | 61666818 | CCDC6 | | Cancer_Gene_Census | |
| 14 | 20779529 | 20801457 | CCNB1IP1 | | Cancer_Gene_Census | |
| 11 | 69455873 | 69469241 | CCND1 | | Cancer_Gene_Census | |
| 12 | 4382902 | 4414521 | CCND2 | | Cancer_Gene_Census | |
| 6 | 41902671 | 42018095 | CCND3 | | Cancer_Gene_Census | |
| 19 | 30302901 | 30315216 | CCNE1 | | Cancer_Gene_Census | |
| 9 | 5450525 | 5470547 | CD274 | | Cancer_Gene_Census | |
| 5 | 149781206 | 149792332 | CD74 | | Cancer_Gene_Census | |
| 19 | 42381190 | 42385439 | CD79A | | Cancer_Gene_Census | |
| 17 | 62006100 | 62009714 | CD79B | | Cancer_Gene_Census | |
| 17 | 38443885 | 38459171 | CDC6 | | Cancer_Related_Genes_Pane l | |
| 1 | 193090919 | 193223031 | CDC73 | | Cancer_Gene_Census | |
| 16 | 68771128 | 68869444 | CDH1 | | Cancer_Gene_Census | |
| 16 | 64977656 | 65156101 | CDH11 | | Cancer_Gene_Census | |
| 17 | 37617739 | 37690800 | CDK12 | | Cancer_Gene_Census | |
| 12 | 58142005 | 58146164 | CDK4 | | Cancer_Gene_Census | |
| 7 | 92234235 | 92465908 | CDK6 | | Cancer_Gene_Census | |
| 13 | 26828276 | 26979375 | CDK8 | | Cancer_Related_Genes_Pane l | |

| Chr | Start | End | Gene | Alternative_ Gene_name | Gene_source/PMID | Paper title |
|---|---|---|---|---|---|---|
| 6 | 36644305 | 36655116 | CDKN1A | | Cancer_Related_Genes_Panel | Mutation detection in formalin-fixed prostate cancer biopsies taken at the time of diagnosis using next-generation DNA sequencing. |
| 12 | 12867992 | 12875305 | CDKN1B | | Cancer_Gene_Census | |
| 9 | 21967752 | 21995300 | CDKN2A | | Cancer_Gene_Census | |
| 9 | 22002902 | 22009280 | CDKN2B | | Cancer_Related_Genes_Panel | |
| 1 | 51426417 | 51440305 | CDKN2C | | Cancer_Gene_Census | |
| 13 | 28536278 | 28543505 | CDX2 | | Cancer_Gene_Census | |
| 19 | 33790840 | 33793470 | CEBPA | | Cancer_Gene_Census | |
| 9 | 123837141 | 123939888 | CEP110 | CEP1 | Cancer_Gene_Census | |
| 18 | 12672631 | 12702773 | CEP76 | | PMID: 24390350 | Discovery and saturation analysis of cancer genes across 21 tumour types. |
| 8 | 57124315 | 57131174 | CHCHD7 | | Cancer_Gene_Census | |
| 5 | 98190908 | 98262240 | CHD1 | | Cancer_Related_Genes_Panel | |
| 1 | 146714291 | 146767443 | CHD1L | | Cancer_Related_Genes_Panel | |
| 15 | 93443419 | 93571237 | CHD2 | | PMID: 24014293 | Recurrent gene mutations in CLL. |
| 14 | 21853358 | 21905404 | CHD8 | | PMID: 24390350 | Mutation detection in formalin-fixed prostate cancer biopsies taken at the time of diagnosis using next-generation DNA sequencing. |
| 11 | 125495036 | 125546148 | CHEK1 | | Cancer_Related_Genes_Panel | |
| 22 | 29083731 | 29138410 | CHEK2 | | Cancer_Gene_Census | |
| 4 | 54875956 | 54930857 | CHIC2 | | Cancer_Gene_Census | |
| 2 | 175664042 | 175870097 | CHN1 | | Cancer_Gene_Census | |

| Chr | Start | End | Gene | Alternative_Gene_name | Gene_source/PMID | Paper title |
|---|---|---|---|---|---|---|
| 10 | 101948055 | 101989376 | CHUK | | Cancer_Related_Genes_Panel | Mutation detection in formalin-fixed prostate cancer biopsies taken at the time of diagnosis using next-qeneration DNA sequencing. |
| 19 | 42788817 | 42799949 | CIC | | Cancer_Gene_Census | |
| 16 | 10971039 | 11023624 | CIITA | | Cancer_Gene_Census | |
| 1 | 154947129 | 154951725 | CKS1B | | Cancer_Related_Genes_Panel | |
| 11 | 57425216 | 57429334 | CLP1 | HEAB | Cancer_Gene_Census | |
| 17 | 57697219 | 57774317 | CLTC | | Cancer_Gene_Census | |
| 3 | 128886661 | 128902810 | CNBP | ZNF9 | Cancer_Gene_Census | |
| 19 | 54641444 | 54659419 | CNOT3 | | Cancer_Gene_Census | |
| 17 | 48260650 | 48278993 | COL1A1 | | Cancer_Gene_Census | |
| 12 | 48366748 | 48398285 | COL2A1 | | Cancer_Gene_Census | |
| 8 | 100890372 | 100905895 | COX6C | | Cancer_Gene_Census | |
| 2 | 208394461 | 208468155 | CREB1 | | Cancer_Gene_Census | |
| 11 | 46299228 | 46342970 | CREB3L1 | | Cancer_Gene_Census | |
| 7 | 137559725 | 137686813 | CREB3L2 | | Cancer_Gene_Census | |
| 16 | 3775055 | 3930722 | CREBBP | | Cancer_Gene_Census | |
| 22 | 21271714 | 21308037 | CRKL | | Cancer_Related_Genes_Panel | |
| X | 1314890 | 1331616 | CRLF2 | | Cancer_Gene_Census | |
| 19 | 18794425 | 18893142 | CRTC1 | MECT1 | Cancer_Gene_Census | |
| 15 | 91073157 | 91188577 | CRTC3 | | Cancer_Gene_Census | |
| 1 | 36931644 | 36948879 | CSF3R | | Cancer_Gene_Census | |
| 16 | 67596310 | 67673086 | CTCF | | Cancer_Related_Genes_Panel | |
| 3 | 41236328 | 41301587 | CTNNB1 | | Cancer_Gene_Census | |

EP 3 452 937 B1

75

(continued)

| Chr | Start | End | Gene | Alternative_Gene_name | Gene_source/PMID | Paper title |
|---|---|---|---|---|---|---|
| 11 | 57529234 | 57586651 | CTNND1 | | Cancer_Related_Genes_Panel | Mutation detection in formalin-fixed prostate cancer biopsies taken at the time of diagnosis using next-qeneration DNA sequencing. |
| 7 | 101459240 | 101927250 | CUX1 | | Cancer_Gene_Census | |
| 2 | 237476430 | 237491001 | CXCR7 | CMKOR1 | Cancer_Gene_Census | |
| 16 | 50775961 | 50835846 | CYLD | | Cancer_Gene_Census | |
| 17 | 19912614 | 20222339 | CYTSB | HCMOGT-1 | Cancer_Gene_Census | |
| 6 | 33286335 | 33297046 | DAXX | | Cancer_Gene_Census | |
| 3 | 182655862 | 182703741 | DCUN1D1 | | Cancer_Related_Genes_Panel | |
| 11 | 47236493 | 47260767 | DDB2 | | Cancer_Gene_Census | |
| 12 | 57910373 | 57914300 | DDIT3 | | Cancer_Gene_Census | |
| 1 | 162601163 | 162750237 | DDR2 | | Cancer_Related_Genes_Panel | |
| 11 | 108535804 | 108811656 | DDX10 | | Cancer_Gene_Census | |
| 12 | 31226779 | 31257725 | DDX11 | | Cancer_Related_Genes_Panel | |
| X | 41192651 | 41223725 | DDX3X | | Cancer_Related_Genes_Panel | |
| 17 | 62495740 | 62502407 | DDX5 | | Cancer_Gene_Census | |
| 11 | 118618475 | 118661972 | DDX6 | | Cancer_Gene_Census | |
| 6 | 18224099 | 18265054 | DEK | | Cancer_Gene_Census | |
| 14 | 95552566 | 95623759 | DICER1 | | Cancer_Gene_Census | |
| 19 | 10824143 | 10942579 | DNM2 | | Cancer_Gene_Census | |
| 2 | 25455496 | 25565459 | DNMT3A | | Cancer_Gene_Census | |
| 4 | 191005567 | 191006841 | DUX4 | | Cancer_Gene_Census | |
| 19 | 40315993 | 40324841 | DYRK1B | | Cancer_Related_Genes_Panel | |

| Chr | Start | End | Gene | Alternative_Gene_name | Gene_source/PMID | Paper title |
|---|---|---|---|---|---|---|
| 6 | 20402398 | 20493941 | E2F3 | | Cancer_Related_Genes_Panel | |
| 5 | 158122928 | 158526769 | EBF1 | | Cancer_Gene_Census | |
| 6 | 139117063 | 139225207 | ECT2L | | Cancer_Gene_Census | |
| 20 | 62119366 | 62130505 | EEF1A2 | | Cancer_Related_Genes_Panel | |
| 7 | 55086714 | 55324313 | EGFR | | Cancer_Gene_Census | |
| X | 20142636 | 20159962 | EIF1AX | | PMID: 23793026, PMID: 24423917 | Exome sequencing identifies recurrent somatic mutations in EIF1AX and SF3B1 in uveal melanoma with disomy 3. AND Mutation frequencies of GNAQ, GNA11, BAP1, SF3B1, EIF1AX and TERT in uveal melanoma: detection of an activating mutation in the TERT gene promoter in a single case of uveal melanoma. |
| 3 | 186500994 | 186507689 | EIF4A2 | | Cancer_Gene_Census | |
| 3 | 170606204 | 170626482 | EIF5A2 | | Cancer_Related_Genes_Panel | |
| X | 129198849 | 129244691 | ELF4 | | Cancer_Gene_Census | |
| 12 | 96588207 | 96661608 | ELK3 | | Cancer_Related_Genes_Panel | |
| 1 | 205577071 | 205601090 | ELK4 | | Cancer_Gene_Census | |
| 19 | 18553475 | 18632918 | ELL | | Cancer_Gene_Census | |
| 7 | 73442119 | 73484237 | ELN | | Cancer_Gene_Census | |
| 2 | 42396490 | 42559688 | EML4 | | Cancer_Gene_Census | |
| 22 | 41487790 | 41576081 | EP300 | | Cancer_Gene_Census | |
| 12 | 132434508 | 132565005 | EP400 | | Cancer_Related_Genes_Panel | |

| Chr | Start | End | Gene | Alternative_Gene_name | Gene_source/PMID | Paper title |
|---|---|---|---|---|---|---|
| 2 | 46520806 | 46613836 | EPAS1 | HIF2A | PMID: 24466223, PMID: 24741025, PMID: 23533246, PMID: 23418310 | Integrative genetic characterization and phenotype correlations in pheochromocytoma and paraganglioma tumours. AND Frequent EPAS1/HIF2α exons 9 and 12 mutations in non-familial pheochromocytoma. AND In vivo and in vitro oncogenic effects of HIF2A mutations in pheochromocytomas and paragangliomas. AND PMID: 23418310 |
| 3 | 89156674 | 89531284 | EPHA3 | | Cancer_Related_Genes_Panel | |
| 3 | 134316643 | 134979309 | EPHB1 | | Cancer_Related_Genes_Panel | |
| 1 | 51819935 | 51985000 | EPS15 | | Cancer_Gene_Census | |
| 17 | 37844393 | 37884915 | ERBB2 | | Cancer_Gene_Census | |
| 12 | 56473892 | 56497128 | ERBB3 | | Cancer_Related_Genes_Panel | |
| 2 | 212240446 | 213403565 | ERBB4 | | Cancer_Related_Genes_Panel | |
| 12 | 1100404 | 1605099 | ERC1 | ELKS | Cancer_Gene_Census | |
| 19 | 45854246 | 45873876 | ERCC2 | | Cancer_Gene_Census | |
| 2 | 128014866 | 128051752 | ERCC3 | | Cancer_Gene_Census | |
| 16 | 14014014 | 14046205 | ERCC4 | | Cancer_Gene_Census | |
| 13 | 103497194 | 103528345 | ERCC5 | | Cancer_Gene_Census | |
| 21 | 39751949 | 40033704 | ERG | | Cancer_Gene_Census | |
| 6 | 151977826 | 152450754 | ESR1 | | Cancer_Related_Genes_Panel | |
| 7 | 13930853 | 14031050 | ETV1 | | Cancer_Gene_Census | |
| 17 | 41605212 | 41623762 | ETV4 | | Cancer_Gene_Census | |
| 3 | 185764097 | 185828107 | ETV5 | | Cancer_Gene_Census | |
| 12 | 11802788 | 12048321 | ETV6 | | Cancer_Gene_Census | |

| Chr | Start | End | Gene | Alternative_Gene_name | Gene_source/PMID | Paper title |
|---|---|---|---|---|---|---|
| 22 | 29663998 | 29696515 | EWSR1 | | Cancer_Gene_Census | |
| 8 | 118806729 | 119123938 | EXT1 | | Cancer_Gene_Census | |
| 11 | 44117099 | 44266979 | EXT2 | | Cancer_Gene_Census | |
| 7 | 148504475 | 148581413 | EZH2 | | Cancer_Gene_Census | |
| 6 | 159186773 | 159240444 | EZR | | Cancer_Gene_Census | |
| 11 | 70049269 | 70053508 | FADD | | Cancer_Related_Genes_Panel | |
| X | 63404997 | 63425624 | FAM123B | AMER1 | Cancer_Gene_Census | |
| 10 | 88985205 | 88994912 | FAM22A | NUTM2A | Cancer_Gene_Census | |
| 10 | 81462983 | 81474437 | FAM22B | NUTM2B | Cancer_Gene_Census | |
| 1 | 118148556 | 118170994 | FAM46C | | Cancer_Gene_Census | |
| 16 | 89803959 | 89883065 | FANCA | | Cancer_Gene_Census | |
| 9 | 97861336 | 98079991 | FANCC | | Cancer_Gene_Census | |
| 3 | 10068098 | 10143614 | FANCD2 | | Cancer_Gene_Census | |
| 6 | 35420138 | 35434880 | FANCE | | Cancer_Gene_Census | |
| 11 | 22644079 | 22647387 | FANCF | | Cancer_Gene_Census | |
| 9 | 35073832 | 35080013 | FANCG | | Cancer_Gene_Census | |
| 10 | 90749226 | 90775542 | FAS | | Cancer_Gene_Census | |
| 2 | 48016455 | 48132932 | FBXO11 | | Cancer_Gene_Census | |
| 4 | 153242410 | 153456172 | FBXW7 | | Cancer_Gene_Census | |
| 1 | 161551136 | 161648444 | FCGR2B | | Cancer_Gene_Census | |
| 1 | 157543539 | 157567870 | FCRL4 | IRTA1 | Cancer_Gene_Census | |
| 2 | 219845809 | 219850379 | FEV | | Cancer_Gene_Census | |
| 8 | 38268656 | 38326352 | FGFR1 | | Cancer_Gene_Census | |
| 6 | 167412670 | 167455906 | FGFR1OP | | Cancer_Gene_Census | |
| 10 | 123237848 | 123357972 | FGFR2 | | Cancer_Gene_Census | |

| Chr | Start | End | Gene | Alternative_Gene_name | Gene_source/PMID | Paper title |
|---|---|---|---|---|---|---|
| 4 | 1795034 | 1810598 | FGFR3 | | Cancer_Gene_Census | |
| 5 | 176513887 | 176525145 | FGFR4 | | Cancer_Gene_Census | |
| 1 | 241660903 | 241683061 | FH | | Cancer_Gene_Census | |
| 3 | 59735036 | 61237133 | FHIT | | Cancer_Gene_Census | |
| 4 | 54243810 | 55161439 | FIP1L1 | | Cancer_Gene_Census | |
| 17 | 17115526 | 17140502 | FLCN | BHD | Cancer_Gene_Census | |
| 11 | 128563813 | 128683161 | FLI1 | | Cancer_Gene_Census | |
| 13 | 28874489 | 29069232 | FLT1 | | Cancer_Related_Genes_Panel | |
| 13 | 28577411 | 28674729 | FLT3 | | Cancer_Gene_Census | |
| 5 | 180028506 | 180076624 | FLT4 | | Cancer_Related_Genes_Panel | |
| 9 | 132649466 | 132805473 | FNBP1 | | Cancer_Gene_Census | |
| 14 | 38059189 | 38064239 | FOXA1 | | Cancer_Gene_Census | |
| 20 | 22561643 | 22566101 | FOXA2 | | Cancer_Related_Genes_Panel | Mutation detection in formalin-fixed prostate cancer biopsies taken at the time of diagnosis using next-qeneration DNA sequencing. |
| 3 | 138663066 | 138665982 | FOXL2 | | Cancer_Gene_Census | |
| 13 | 41129817 | 41240734 | FOXO1 | FOXO1A | Cancer_Gene_Census | |
| 6 | 108881038 | 109005977 | FOXO3 | FOXO3A | Cancer_Gene_Census | |
| X | 70316047 | 70323385 | FOXO4 | MLLT7 | Cancer_Gene_Census | |
| 3 | 71003844 | 71633140 | FOXP1 | | Cancer_Gene_Census | |
| 19 | 676389 | 683392 | FSTL3 | | Cancer_Gene_Census | |
| 1 | 78400603 | 78444794 | FUBP1 | | Cancer_Gene_Census | |
| 16 | 31191445 | 31203127 | FUS | | Cancer_Gene_Census | |
| 6 | 111981535 | 112194655 | FYN | | PMID: 24413734 | Recurrent mutations in epigenetic regulators, RHOA and FYN kinase in peripheral T cell lymphomas. |

EP 3 452 937 B1

(continued)

| Chr | Start | End | Gene | Alternative_Gene_name | Gene_source/PMID | Paper title |
|---|---|---|---|---|---|---|
| 11 | 77926342 | 78128766 | GAB2 | | Cancer_Related_Genes_Panel | |
| 17 | 9813926 | 10101868 | GAS7 | | Cancer_Gene_Census | |
| X | 48644962 | 48652718 | GATA1 | | Cancer_Gene_Census | |
| 3 | 128198265 | 128212028 | GATA2 | | Cancer_Gene_Census | |
| 10 | 8095567 | 8117161 | GATA3 | | Cancer_Gene_Census | |
| 18 | 19749404 | 19782491 | GATA6 | | Cancer_Related_Genes_Panel | |
| 3 | 155588325 | 155658457 | GMPS | | Cancer_Gene_Census | |
| 19 | 3094408 | 3121452 | GNA11 | | Cancer_Gene_Census | |
| 9 | 80331003 | 80646374 | GNAQ | | Cancer_Gene_Census | |
| 20 | 57414750 | 57486247 | GNAS | | Cancer_Gene_Census | |
| 14 | 93260650 | 93306304 | GOLGA5 | | Cancer_Gene_Census | |
| 6 | 117639374 | 117923691 | GOPC | | Cancer_Gene_Census | |
| X | 132669773 | 133119922 | GPC3 | | Cancer_Gene_Census | |
| 13 | 92050929 | 93519490 | GPC5 | | Cancer_Related_Genes_Panel | |
| 14 | 66974125 | 67648520 | GPHN | | Cancer_Gene_Census | |
| 17 | 73314157 | 73401790 | GRB2 | | Cancer_Related_Genes_Panel | |
| 17 | 37894187 | 37903545 | GRB7 | | Cancer_Related_Genes_Panel | |
| 16 | 9847261 | 10276611 | GRIN2A | | Cancer_Gene_Census | |
| 7 | 86273224 | 86494200 | GRM3 | | PMID: 21946352 | Exon capture analysis of G protein-coupled receptors identifies activating mutations in GRM3 in melanoma. |
| 1 | 226249552 | 226259702 | H3F3A | | Cancer_Gene_Census | |
| 17 | 73772517 | 73775860 | H3F3B | | Cancer_Gene_Census | |

| Chr | Start | End | Gene | Alternative_Gene_name | Gene_source/PMID | Paper title |
|---|---|---|---|---|---|---|
| 16 | 56965748 | 56977793 | HERPUD1 | | Cancer_Gene_Census | |
| 8 | 80676246 | 80680098 | HEY1 | | Cancer_Gene_Census | |
| 7 | 75162621 | 75368280 | HIP1 | | Cancer_Gene_Census | |
| 6 | 26031878 | 26032288 | HIST1H3B | | Cancer_Gene_Census | |
| 6 | 27107076 | 27108418 | HIST1H4I | | Cancer_Gene_Census | |
| 17 | 53342321 | 53402426 | HLF | | Cancer_Gene_Census | |
| 6 | 34204577 | 34214008 | HMGA1 | | Cancer_Gene_Census | |
| 12 | 66218240 | 66360068 | HMGA2 | | Cancer_Gene_Census | |
| 12 | 121416346 | 121440899 | HNF1A | TCF1 | Cancer_Gene_Census | |
| 7 | 26229547 | 26241149 | HNRNPA2B1 | | Cancer_Gene_Census | |
| 8 | 42752033 | 42885671 | HOOK3 | | Cancer_Gene_Census | |
| 7 | 27220776 | 27224835 | HOXA11 | | Cancer_Gene_Census | |
| 7 | 27235022 | 27239725 | HOXA13 | | Cancer_Gene_Census | |
| 7 | 27202057 | 27219880 | HOXA9 | | Cancer_Gene_Census | |
| 12 | 54366912 | 54370203 | HOXC11 | | Cancer_Gene_Census | |
| 12 | 54332549 | 54340328 | HOXC13 | | Cancer_Gene_Census | |
| 2 | 176968944 | 176974482 | HOXD11 | | Cancer_Gene_Census | |
| 2 | 176957619 | 176960666 | HOXD13 | | Cancer_Gene_Census | |
| 11 | 532242 | 537287 | HRAS | | Cancer_Gene_Census | |
| 14 | 102547073 | 102606086 | HSP90AA1 | HSPCA | Cancer_Gene_Census | |
| 6 | 44214824 | 44221620 | HSP90AB1 | HSPCB | Cancer_Gene_Census | |
| 11 | 122928203 | 122932844 | HSPA8 | | Cancer_Related_Genes_Panel | Mutation detection in formalin-fixed prostate cancer biopsies taken at the time of diagnosis using next-generation DNA sequencing. |
| 20 | 30193086 | 30194318 | ID1 | | Cancer_Related_Genes_Panel | |

(continued)

| Chr | Start | End | Gene | Alternative_Gene_name | Gene_source/PMID | Paper title |
|---|---|---|---|---|---|---|
| 2 | 209100951 | 209130798 | IDH1 | | Cancer_Gene_Census | |
| 15 | 90626277 | 90645736 | IDH2 | | Cancer_Gene_Census | |
| 15 | 99192200 | 99507759 | IGF1R | | Cancer_Related_Genes_Panel | |
| 6 | 160390131 | 160534539 | IGF2R | | Cancer_Related_Genes_Panel | |
| 8 | 42128829 | 42189965 | IKBKB | | Cancer_Gene_Census | |
| 1 | 206643791 | 206670223 | IKBKE | | Cancer_Related_Genes_Panel | |
| 7 | 50343720 | 50472799 | IKZF1 | | Cancer_Gene_Census | |
| 17 | 37913968 | 38020441 | IKZF3 | | PMID: 24212482 | ASXL1, TP53 and IKZF3 mutations are present in the chronic phase and blast crisis of chronic myeloid leukemia. |
| 4 | 123372625 | 123377880 | IL2 | | Cancer_Gene_Census | |
| 16 | 27413686 | 27462115 | IL21R | | Cancer_Gene_Census | |
| 5 | 55230923 | 55290772 | IL6ST | | Cancer_Gene_Census | |
| 5 | 35852797 | 35879705 | IL7R | | Cancer_Gene_Census | |
| 7 | 41724712 | 41742706 | INHBA | | Cancer_Related_Genes_Panel | |
| 6 | 391739 | 411447 | IRF4 | | Cancer_Gene_Census | |
| 13 | 110406184 | 110438915 | IRS2 | | Cancer_Related_Genes_Panel | |
| 17 | 45331208 | 45390077 | ITGB3 | | PMID: 17679464 | Mutational analysis of proapoptotic integrin beta 3 cytoplasmic domain in common human cancers. |
| 5 | 156607854 | 156682195 | ITK | | Cancer_Gene_Census | |
| 1 | 65298912 | 65432187 | JAK1 | | Cancer_Gene_Census | |
| 9 | 4985033 | 5128183 | JAK2 | | Cancer_Gene_Census | |
| 19 | 17935595 | 17958870 | JAK3 | | Cancer_Gene_Census | |
| 7 | 27870192 | 28220362 | JAZF1 | | Cancer_Gene_Census | |

(continued)

| Chr | Start | End | Gene | Alternative_Gene_name | Gene_source/PMID | Paper title |
|---|---|---|---|---|---|---|
| 1 | 59246465 | 59249785 | JUN | | Cancer_Gene_Census | |
| 11 | 128761313 | 128787949 | KCNJ5 | | Cancer_Gene_Census | |
| 12 | 389223 | 498620 | KDM5A | | Cancer_Gene_Census | |
| X | 53221334 | 53254604 | KDM5C | | Cancer_Gene_Census | |
| X | 44732423 | 44971847 | KDM6A | | Cancer_Gene_Census | |
| 4 | 55944644 | 55991756 | KDR | | Cancer_Gene_Census | |
| 18 | 60994971 | 61034743 | KDSR | FVT1 | Cancer_Gene_Census | |
| 19 | 10596796 | 10614054 | KEAP1 | | Cancer_Related_Genes_Panel | |
| 7 | 138516126 | 138666064 | KIAA1549 | | Cancer_Gene_Census | |
| 10 | 32297938 | 32345359 | KIF5B | | Cancer_Gene_Census | |
| 4 | 55524085 | 55606881 | KIT | | Cancer_Gene_Census | |
| 9 | 110247133 | 110252763 | KLF4 | | Cancer_Gene_Census | |
| 10 | 3818188 | 3827467 | KLF6 | COPEB | Cancer_Gene_Census | |
| 19 | 51376689 | 51383822 | KLK2 | | Cancer_Gene_Census | |
| 12 | 25358182 | 25403854 | KRAS | | Cancer_Gene_Census | |
| 14 | 56047033 | 56151301 | KTN1 | | Cancer_Gene_Census | |
| 17 | 37026256 | 37078023 | LASP1 | | Cancer_Gene_Census | |
| 1 | 32716840 | 32751766 | LCK | | Cancer_Gene_Census | |
| 13 | 46700061 | 46786006 | LCP1 | | Cancer_Gene_Census | |
| 13 | 39917029 | 40177665 | LHFP | | Cancer_Gene_Census | |
| 5 | 38475065 | 38608456 | LIFR | | Cancer_Gene_Census | |
| 11 | 8245857 | 8290181 | LMO1 | | Cancer_Gene_Census | |
| 11 | 33880122 | 33913836 | LMO2 | | Cancer_Gene_Census | |

(continued)

| Chr | Start | End | Gene | Alternative_Gene_name | Gene_source/PMID | Paper title |
|---|---|---|---|---|---|---|
| 9 | 113635543 | 113800738 | LPAR1 | | PMID: 24147068 | Massively parallel sequencing reveals an accumulation of de novo mutations and an activating mutation of LPAR1 in a patient with metastatic neuroblastoma. |
| 3 | 187871072 | 188608460 | LPP | | Cancer_Gene_Census | |
| 12 | 59265937 | 59314262 | LRIG3 | | Cancer_Gene_Census | |
| 2 | 140988992 | 142889270 | LRP1B | | PMID: 24014293 | Recurrent gene mutations in CLL. |
| 19 | 13209848 | 13213681 | LYL1 | | Cancer_Gene_Census | |
| 22 | 21333751 | 21353327 | LZTR1 | | PMID: 23917401, PMID: 24362817 | The integrated landscape of driver genomic alterations in glioblastoma. AND Germline loss-of-function mutations in LZTR1 predispose to an inherited disorder of multiple schwannomas |
| 16 | 79627735 | 79634611 | MAF | | Cancer_Gene_Census | |
| 8 | 144511515 | 144512576 | MAFA | | Cancer_Related_Genes_Panel | |
| 20 | 39314488 | 39317880 | MAFB | | Cancer_Gene_Census | |
| 18 | 56338618 | 56421130 | MALT1 | | Cancer_Gene_Census | |
| 11 | 95711440 | 96075059 | MAML2 | | Cancer_Gene_Census | |
| 15 | 66679155 | 66783882 | MAP2K1 | | Cancer_Gene_Census | |
| 19 | 4090319 | 4124126 | MAP2K2 | | Cancer_Gene_Census | |
| 17 | 11924141 | 12047140 | MAP2K4 | | Cancer_Gene_Census | |
| 15 | 67835021 | 68099461 | MAP2K5 | | Cancer_Related_Genes_Panel | |
| 17 | 67410838 | 67538451 | MAP2K6 | | Cancer_Related_Genes_Panel | |
| 19 | 7968728 | 7979363 | MAP2K7 | | Cancer_Related_Genes_Panel | |
| 5 | 56111401 | 56191979 | MAP3K1 | | Cancer_Gene_Census | |
| 11 | 65365226 | 65381720 | MAP3K11 | | Cancer_Related_Genes_Panel | |

EP 3 452 937 B1

85

(continued)

| Chr | Start | End | Gene | Alternative_Gene_name | Gene_source/PMID | Paper title |
|---|---|---|---|---|---|---|
| 12 | 53874280 | 53893271 | MAP3K12 | | Cancer_Related_Genes_Panel | |
| 3 | 185000729 | 185206885 | MAP3K13 | | Cancer_Gene_Census | |
| 17 | 43340488 | 43394414 | MAP3K14 | | Cancer_Related_Genes_Panel | |
| 2 | 128056306 | 128146041 | MAP3K2 | | Cancer_Related_Genes_Panel | |
| 17 | 61699801 | 61773667 | MAP3K3 | | Cancer_Related_Genes_Panel | |
| 6 | 161412822 | 161551917 | MAP3K4 | | Cancer_Related_Genes_Panel | |
| 6 | 136878185 | 137113656 | MAP3K5 | | Cancer_Related_Genes_Panel | |
| 1 | 27681670 | 27693383 | MAP3K6 | | Cancer_Related_Genes_Panel | |
| 6 | 91223292 | 91296764 | MAP3K7 | | Cancer_Related_Genes_Panel | |
| 10 | 30722866 | 30750762 | MAP3K8 | | Cancer_Related_Genes_Panel | |
| 14 | 71194852 | 71275888 | MAP3K9 | | Cancer_Related_Genes_Panel | |
| 2 | 39476407 | 39664453 | MAP4K3 | | PMID: 24390350 | |
| 2 | 102313312 | 102508449 | MAP4K4 | | Cancer_Related_Genes_Panel | |
| 22 | 22108789 | 22221970 | MAPK1 | | Cancer_Related_Genes_Panel | |
| 4 | 86936276 | 87515284 | MAPK10 | | Cancer_Related_Genes_Panel | |
| 17 | 19281034 | 19286857 | MAPK7 | | Cancer_Related_Genes_Panel | |

| Chr | Start | End | Gene | Alternative_Gene_name | Gene_source/PMID | Paper title |
|---|---|---|---|---|---|---|
| 10 | 49514698 | 49647403 | MAPK8 | | Cancer_Related_Genes_Panel | |
| 5 | 179660594 | 179719071 | MAPK9 | | Cancer_Related_Genes_Panel | |
| 14 | 65472892 | 65569227 | MAX | | Cancer_Gene_Census | |
| 1 | 150547032 | 150552066 | MCL1 | | Cancer_Related_Genes_Panel | |
| 12 | 69201956 | 69239214 | MDM2 | | Cancer_Gene_Census | |
| 1 | 204485511 | 204596130 | MDM4 | | Cancer_Gene_Census | |
| 1 | 23907985 | 23967058 | MDS2 | | Cancer_Gene_Census | |
| 3 | 168801287 | 169381406 | MECOM | EVI1 | Cancer_Gene_Census | |
| X | 70338406 | 70362303 | MED12 | | Cancer_Gene_Census | |
| 6 | 131895106 | 131949369 | MED23 | MED23/CRSP3 | PMID: 24390350 | Discovery and saturation analysis of cancer genes across 21 tumour types. |
| 19 | 39881963 | 39891201 | MED29 | | Cancer_Related_Genes_Panel | |
| 11 | 64570988 | 64578766 | MEN1 | | Cancer_Gene_Census | |
| 7 | 116312248 | 116438440 | MET | | Cancer_Gene_Census | |
| 3 | 69788586 | 70017487 | MITF | | Cancer_Gene_Census | |
| 22 | 40806285 | 41032706 | MKL1 | | Cancer_Gene_Census | |
| 3 | 158288952 | 158325041 | MLF1 | | Cancer_Gene_Census | |
| 3 | 37034823 | 37092409 | MLH1 | | Cancer_Gene_Census | |
| 11 | 118307205 | 118395936 | MLL | KMT2A | Cancer_Gene_Census | |
| 12 | 49412762 | 49449107 | MLL2 | KMT2D | Cancer_Gene_Census | |
| 7 | 151832007 | 152133628 | MLL3 | KMT2C | Cancer_Gene_Census | |
| 19 | 6212966 | 6279959 | MLLT1 | | Cancer_Gene_Census | |
| 10 | 21823094 | 22032559 | MLLT10 | | Cancer_Gene_Census | |

(continued)

| Chr | Start | End | Gene | Alternative_Gene_name | Gene_source/PMID | Paper title |
|-----|-------|-----|------|----------------------|------------------|-------------|
| 1 | 151030234 | 151040970 | MLLT11 | AF1Q | Cancer_Gene_Census | |
| 9 | 20341663 | 20622542 | MLLT3 | | Cancer_Gene_Census | |
| 6 | 168227602 | 168372703 | MLLT4 | | Cancer_Gene_Census | |
| 17 | 36861795 | 36886056 | MLLT6 | | Cancer_Gene_Census | |
| 22 | 28144265 | 28197486 | MN1 | | Cancer_Gene_Census | |
| 7 | 156786745 | 156803239 | MNX1 | HLXB9 | Cancer_Gene_Census | |
| 1 | 43803475 | 43818443 | MPL | | Cancer_Gene_Census | |
| 3 | 138066539 | 138124375 | MRAS | | Cancer_Related_Genes_Panel | |
| 11 | 94150475 | 94227040 | MRE11A | | Cancer_Related_Genes_Panel | |
| 2 | 47630108 | 47789450 | MSH2 | | Cancer_Gene_Census | |
| 2 | 48010221 | 48034092 | MSH6 | | Cancer_Gene_Census | |
| 17 | 55333931 | 55757299 | MSI2 | | Cancer_Gene_Census | |
| X | 64887537 | 64961792 | MSN | | Cancer_Gene_Census | |
| X | 154289897 | 154376212 | MTCP1 | | Cancer_Gene_Census | |
| 8 | 98656407 | 98742488 | MTDH | | Cancer_Related_Genes_Panel | |
| 1 | 11166592 | 11322564 | MTOR | | Cancer_Related_Genes_Panel | |
| 1 | 155158300 | 155162707 | MUC1 | | Cancer_Gene_Census | |
| 1 | 45794914 | 45806142 | MUTYH | | Cancer_Gene_Census | |
| 6 | 135502453 | 135540311 | MYB | | Cancer_Gene_Census | |
| 8 | 128747680 | 128753674 | MYC | | Cancer_Gene_Census | |
| 1 | 40361098 | 40367925 | MYCL1 | MYCL | Cancer_Gene_Census | |
| 2 | 16080679 | 16087129 | MYCN | | Cancer_Gene_Census | |
| 3 | 38179969 | 38184513 | MYD88 | | Cancer_Gene_Census | |

(continued)

| Chr | Start | End | Gene | Alternative_Gene_name | Gene_source/PMID | Paper title |
|---|---|---|---|---|---|---|
| 16 | 15796992 | 15950890 | MYH11 | | Cancer_Gene_Census | |
| 22 | 36677327 | 36784063 | MYH9 | | Cancer_Gene_Census | |
| 1 | 171604557 | 171621811 | MYOC | | Cancer_Related_Genes_Panel | |
| 11 | 17741110 | 17743675 | MYOD1 | | Cancer_Gene_Census | |
| 8 | 41786997 | 41909508 | MYST3 | RUNXBP2 | Cancer_Gene_Census | |
| 10 | 76585340 | 76792380 | MYST4 | KAT6B | Cancer_Gene_Census | |
| 12 | 57094565 | 57119326 | NACA | | Cancer_Gene_Census | |
| 8 | 90945564 | 90996899 | NBN | NBS1 | Cancer_Gene_Census | |
| 1 | 148250245 | 148356319 | NBPF10 | | Cancer_Related_Genes_Panel | Characterization of the genomic features and expressed fusion genes in micropapillary carcinomas of the breast. AND Mutation detection in formalin-fixed prostate cancer biopsies taken at the time of diagnosis using next-generation DNA sequencing. |
| 3 | 48701364 | 48723797 | NCKIPSD | AF3p21 | Cancer_Gene_Census | |
| 2 | 24714783 | 24993571 | NCOA1 | | Cancer_Gene_Census | |
| 8 | 71015826 | 71316020 | NCOA2 | | Cancer_Gene_Census | |
| 20 | 46130646 | 46285616 | NCOA3 | | Cancer_Related_Genes_Panel | |
| 10 | 51565108 | 51590734 | NCOA4 | | Cancer_Gene Census | |
| 17 | 15934718 | 16119010 | NCOR1 | | Cancer_Gene Census | |
| 12 | 124808961 | 125052010 | NCOR2 | | Cancer_Related_Genes_Panel | Mutation detection in formalin-fixed prostate cancer biopsies taken at the time of diagnosis using next-generation DNA sequencing. |
| 8 | 134249414 | 134309547 | NDRG1 | | Cancer_Gene_Census | |
| 4 | 115748919 | 116035032 | NDST4 | | PMID: 23825612 | NDST4 is a novel candidate tumor suppressor gene at chromosome 4q26 and its genetic loss predicts adverse prognosis in colorectal cancer. |

| Chr | Start | End | Gene | Alternative_ Gene_name | Gene_source/PMID | Paper title |
|---|---|---|---|---|---|---|
| 19 | 19626540 | 19646885 | NDUFA13 | GRIM-19 | PMID: 23851499, PMID: 23386605 | GRIM-19 mutations fail to inhibit v-Src-induced oncogenesis. AND Tumor-derived mutations in the gene associated with retinoid interferon-induced mortality (GRIM-19) disrupt its anti-signal transducer and activator of transcription 3 (STAT3) activity and promote oncogenesis. |
| 17 | 29421945 | 29705949 | NF1 | | Cancer_Gene_Census | |
| 22 | 29999545 | 30094587 | NF2 | | Cancer_Gene_Census | |
| 2 | 178092323 | 178257425 | NFE2L2 | | Cancer_Gene_Census | |
| 9 | 14081847 | 14314581 | NFIB | | Cancer_Gene_Census | |
| 10 | 104154229 | 104162281 | NFKB2 | | Cancer_Gene_Census | |
| 17 | 47572655 | 47592379 | NGFR | | Cancer_Related_Genes_Panel | |
| 14 | 51186481 | 51297848 | NIN | | Cancer_Gene_Census | |
| 14 | 36985603 | 36989416 | NKX2-1 | | Cancer_Gene_Census | |
| X | 70503433 | 70521018 | NONO | | Cancer_Gene_Census | |
| 9 | 139388896 | 139440314 | NOTCH1 | | Cancer_Gene_Census | |
| 1 | 120454176 | 120612274 | NOTCH2 | | Cancer_Gene_Census | |
| 19 | 15270445 | 15311792 | NOTCH3 | | Cancer_Related_Genes_Panel | |
| 6 | 32162620 | 32191844 | NOTCH4 | | Cancer_Related_Genes_Panel | |
| 8 | 120428552 | 120436678 | NOV | | Cancer_Related_Genes_Panel | |
| 5 | 170814708 | 170838141 | NPM1 | | Cancer_Gene_Census | |
| 9 | 102584137 | 102629173 | NR4A3 | | Cancer_Gene_Census | |
| 1 | 115247090 | 115259515 | NRAS | | Cancer_Gene_Census | |
| 21 | 16333556 | 16437321 | NRIP1 | | PMID: 24239470 | The prostate cancer genome: perspectives and potential. |
| 5 | 176560026 | 176727216 | NSD1 | | Cancer_Gene_Census | |

EP 3 452 937 B1

(continued)

| Chr | Start | End | Gene | Alternative_Gene_name | Gene_source/PMID | Paper title |
|---|---|---|---|---|---|---|
| 10 | 104845940 | 104953056 | NT5C2 | | Cancer_Gene_Census | |
| 1 | 156785448 | 156851642 | NTRK1 | | Cancer_Gene_Census | |
| 9 | 87283466 | 87638505 | NTRK2 | | Cancer_Related_Genes_Panel | |
| 15 | 88402982 | 88799978 | NTRK3 | | Cancer_Gene_Census | |
| 11 | 71713911 | 71791573 | NUMA1 | | Cancer_Gene_Census | |
| 9 | 134000976 | 134110057 | NUP214 | | Cancer_Gene_Census | |
| 11 | 3696241 | 3819018 | NUP98 | | Cancer_Gene_Census | |
| 21 | 34398153 | 34401504 | OLIG2 | | Cancer_Gene_Census | |
| 9 | 95176527 | 95186743 | OMD | | Cancer_Gene_Census | |
| X | 1581465 | 1656000 | P2RY8 | | Cancer_Gene_Census | |
| 11 | 117015040 | 117046694 | PAFAH1B2 | | Cancer_Gene_Census | |
| 11 | 77033061 | 77185108 | PAK1 | | Cancer_Related_Genes_Panel | |
| X | 110187513 | 110464159 | PAK3 | | Cancer_Related_Genes_Panel | |
| 16 | 23614483 | 23652678 | PALB2 | | Cancer_Gene_Census | |
| 22 | 31721790 | 31742218 | PATZ1 | ZNF278 | Cancer_Gene_Census | |
| 2 | 223064607 | 223163715 | PAX3 | | Cancer_Gene_Census | |
| 9 | 36833272 | 37034476 | PAX5 | | Cancer_Gene_Census | |
| 1 | 18957500 | 19075360 | PAX7 | | Cancer_Gene_Census | |
| 2 | 113973574 | 114036498 | PAX8 | | Cancer_Gene_Census | |
| 14 | 37126782 | 37147007 | PAX9 | | Cancer_Related_Genes_Panel | |
| 3 | 52579368 | 52719933 | PBRM1 | | Cancer_Gene_Census | |
| 1 | 164524821 | 164821067 | PBX1 | | Cancer_Gene_Census | |
| 8 | 17780366 | 17887453 | PCM1 | | Cancer_Gene_Census | |

91

EP 3 452 937 B1

| Chr | Start | End | Gene | Alternative_Gene_name | Gene_source/PMID | Paper title |
|-----|-------|-----|------|------------------------|-------------------|-------------|
| 11 | 117075810 | 117102811 | PCSK7 | | Cancer_Gene_Census | |
| 9 | 5510545 | 5571282 | PDCD1LG2 | CD273 | Cancer_Gene_Census | |
| 1 | 144833168 | 145076186 | PDE4DIP | | Cancer_Gene_Census | |
| 22 | 39619364 | 39640756 | PDGFB | | Cancer_Gene_Census | |
| 4 | 55095264 | 55164414 | PDGFRA | | Cancer_Gene_Census | |
| 5 | 149493400 | 149535423 | PDGFRB | | Cancer_Gene_Census | |
| 17 | 8043809 | 8059678 | PER1 | | Cancer_Gene_Census | |
| X | 133507283 | 133562820 | PHF6 | | Cancer_Gene_Census | |
| 1 | 120202421 | 120286838 | PHGDH | | Cancer_Related_Genes_Panel | |
| 4 | 41746099 | 41750987 | PHOX2B | | Cancer_Gene_Census | |
| 11 | 85668486 | 85780144 | PICALM | | Cancer_Gene_Census | |
| 11 | 17108129 | 17191354 | PIK3C2A | | Cancer_Related_Genes_Panel | Mutation detection in formalin-fixed prostate cancer biopsies taken at the time of diagnosis using next-generation DNA sequencing. |
| 1 | 204391756 | 204463852 | PIK3C2B | | PMID: 22510280 | Identification of somatic mutations in non-small cell lung carcinomas using whole-exome sequencing. |
| 12 | 18414474 | 18801348 | PIK3C2G | | Cancer_Related_Genes_Panel | Mutation detection in formalin-fixed prostate cancer biopsies taken at the time of diagnosis using next-generation DNA sequencing. |
| 18 | 39535171 | 39667794 | PIK3C3 | | Cancer_Related_Genes_Panel | |
| 3 | 178865902 | 178957881 | PIK3CA | | Cancer_Gene_Census | |
| 3 | 138372860 | 138553780 | PIK3CB | | Cancer_Related_Genes_Panel | Mutation detection in formalin-fixed prostate cancer biopsies taken at the time of diagnosis using next-generation DNA sequencing. |
| 7 | 106505723 | 106547590 | PIK3CG | | Cancer_Related_Genes_Panel | |

(continued)

| Chr | Start | End | Gene | Alternative_Gene_name | Gene_source/PMID | Paper title |
|-----|-------|-----|------|----------------------|------------------|-------------|
| 5 | 67522462 | 67597649 | PIK3R1 | | Cancer_Gene_Census | |
| 19 | 18263928 | 18281343 | PIK3R2 | | Cancer_Related_Genes_Panel | |
| 6 | 37137922 | 37143202 | PIM1 | | Cancer_Gene_Census | |
| 8 | 57073468 | 57123859 | PLAG1 | | Cancer_Gene_Census | |
| 20 | 39765600 | 39804361 | PLCG1 | | Cancer_Gene_Census | |
| 1 | 6526152 | 6580121 | PLEKHG5 | | PMID: 24014293 | Recurrent gene mutations in CLL. |
| 15 | 74287014 | 74340151 | PML | | Cancer_Gene_Census | |
| 2 | 190648811 | 190742355 | PMS1 | | Cancer_Gene_Census | |
| 7 | 6012870 | 6048756 | PMS2 | | Cancer_Gene_Census | |
| 7 | 124462440 | 124570037 | POT1 | | Cancer_Gene_Census | |
| 3 | 87307379 | 87325737 | POU1F1 | | Cancer_Related_Genes_Panel | |
| 11 | 111222980 | 111250157 | POU2AF1 | | Cancer_Gene_Census | |
| 6 | 31132114 | 31138470 | POU5F1 | | Cancer_Gene_Census | |
| 3 | 12328867 | 12475855 | PPARG | | Cancer_Gene_Census | |
| 17 | 58677554 | 58742036 | PPM1D | | Cancer_Related_Genes_Panel | |
| 19 | 52693274 | 52730687 | PPP2R1A | | Cancer_Gene_Census | |
| 9 | 127908852 | 127952218 | PPP6C | | Cancer_Gene_Census | |
| 1 | 156720402 | 156770609 | PRCC | | Cancer_Gene_Census | |
| 6 | 106534195 | 106557814 | PRDM1 | | Cancer_Gene_Census | |
| 1 | 2985732 | 3355185 | PRDM16 | | Cancer_Gene_Census | |
| 8 | 68864353 | 69143897 | PREX2 | | PMID: 22622578 | Melanoma genome sequencing reveals frequent PREX2 mutations. |
| 10 | 72357104 | 72362531 | PRF1 | | Cancer_Gene_Census | |
| 17 | 66508110 | 66528908 | PRKAR1A | | Cancer_Gene_Census | |

| Chr | Start | End | Gene | Alternative_Gene_name | Gene_source/PMID | Paper title |
|---|---|---|---|---|---|---|
| 19 | 54382444 | 54410906 | PRKCG | | Cancer_Related_Genes_Panel | |
| 3 | 169940153 | 170023769 | PRKCI | | Cancer_Related_Genes_Panel | |
| 1 | 170632323 | 170708560 | PRRX1 | PMX1 | Cancer_Gene_Census | |
| 9 | 15464064 | 15511017 | PSIP1 | PSIP2 | Cancer_Gene_Census | |
| 9 | 98205262 | 98279339 | PTCH1 | | Cancer_Gene_Census | |
| 10 | 89622870 | 89731687 | PTEN | | Cancer_Gene_Census | |
| 20 | 62159778 | 62168695 | PTK6 | | Cancer_Related_Genes_Panel | |
| 6 | 64231666 | 64293492 | PTP4A1 | | Cancer_Related_Genes_Panel | |
| 8 | 142432007 | 142441620 | PTP4A3 | | Cancer_Related_Genes_Panel | |
| 12 | 112856713 | 112947717 | PTPN11 | | Cancer_Gene_Census | |
| 4 | 87515468 | 87736324 | PTPN13 | | PMID: 22245727, PMID: 15155950 | The nonreceptor-type tyrosine phosphatase PTPN13 is a tumor suppressor gene in non-small cell lung cancer. AND Mutational analysis of the tyrosine phosphatome in colorectal cancers. |
| 12 | 70910630 | 71031210 | PTPRB | | Cancer_Gene_Census | |
| 1 | 198607801 | 198726545 | PTPRC | | Cancer_Gene_Census | |
| 9 | 8314246 | 10612723 | PTPRD | | PMID: 24395800, PMID: 19074898 | Frequent mutation of receptor protein tyrosine phosphatases provides a mechanism for STAT3 hyperactivation in head and neck cancer. AND Mutational inactivation of PTPRD in glioblastoma multiforme and malignant melanoma. |
| 11 | 48002110 | 48192393 | PTPRJ | | PMID: 12089527 | Ptprj is a candidate for the mouse colon-cancer susceptibility locus Scc1 and is frequently deleted in human cancers. |
| 6 | 128289924 | 128841870 | PTPRK | | Cancer_Gene_Census | |

94

EP 3 452 937 B1

| Chr | Start | End | Gene | Alternative_Gene_name | Gene_source/PMID | Paper title |
|---|---|---|---|---|---|---|
| 18 | 7567817 | 8406859 | PTPRM | | PMID: 24395800 | Frequent mutation of receptor protein tyrosine phosphatases provides a mechanism for STAT3 hyperactivation in head and neck cancer. |
| 19 | 5205519 | 5340814 | PTPRS | | PMID: 19000305 | Frameshift mutations in coding repeats of protein tyrosine phosphatase genes in colorectal tumors with microsatellite instability. |
| 20 | 40701392 | 41818610 | PTPRT | | PMID: 24395800 | Frequent mutation of receptor protein tyrosine phosphatases provides a mechanism for STAT3 hyperactivation in head and neck cancer. |
| 6 | 57053581 | 57087078 | RAB23 | | Cancer_Related_Genes_Panel | |
| 1 | 156030951 | 156040295 | RAB25 | | Cancer_Related_Genes_Panel | |
| 17 | 5185558 | 5289129 | RABEP1 | RAB5EP | Cancer_Gene_Census | |
| 7 | 6414154 | 6443608 | RAC1 | | Cancer_Gene_Census | |
| 8 | 117858174 | 117887105 | RAD21 | | Cancer_Gene_Census | |
| 5 | 131891711 | 131979752 | RAD50 | | Cancer_Related_Genes_Panel | Mutation detection in formalin-fixed prostate cancer biopsies taken at the time of diagnosis using next-generation DNA sequencing. |
| 15 | 40987358 | 41024356 | RAD51 | | Cancer_Related_Genes_Panel | Mutation detection in formalin-fixed prostate cancer biopsies taken at the time of diagnosis using next-qeneration DNA sequencing. |
| 14 | 68286496 | 69196935 | RAD51L1 | RAD51B | Cancer_Gene_Census | |
| 3 | 12625100 | 12705725 | RAF1 | | Cancer_Gene_Census | |
| 9 | 135973107 | 136024721 | RALGDS | | Cancer_Gene_Census | |
| 5 | 170288874 | 170725862 | RANBP17 | | Cancer_Gene_Census | |
| 4 | 99182535 | 99365012 | RAP1GDS1 | | Cancer_Gene_Census | |
| 17 | 38465436 | 38513895 | RARA | | Cancer_Gene_Census | |

EP 3 452 937 B1

| Chr | Start | End | Gene | Alternative_Gene_name | Gene_source/PMID | Paper title |
|---|---|---|---|---|---|---|
| 12 | 113537318 | 113574021 | RASAL1 | | PMID: 24136889 | Identification of RASAL1 as a major tumor suppressor gene in thyroid cancer. |
| 13 | 48877911 | 49056122 | RB1 | | Cancer_Gene_Census | |
| 1 | 110876516 | 110889299 | RBM15 | | Cancer_Gene_Census | |
| 8 | 145736672 | 145743200 | RECQL4 | | Cancer_Gene_Census | |
| 1 | 120336641 | 120354283 | REG4 | | Cancer_Related_Genes_Panel | |
| 2 | 61108656 | 61150645 | REL | | Cancer_Gene_Census | |
| 10 | 43572475 | 43625799 | RET | | Cancer_Gene_Census | |
| 7 | 151163098 | 151217206 | RHEB | | PMID: 24390350 | Mutation detection in formalin-fixed prostate cancer biopsies taken at the time of diagnosis using next-generation DNA sequencing. |
| 3 | 49396578 | 49450431 | RHOA | | Cancer_Gene_Census | |
| 4 | 40192673 | 40246281 | RHOH | ARHH | Cancer_Gene_Census | |
| 5 | 38938021 | 39074510 | RICTOR | | Cancer_Related_Genes_Panel | |
| 17 | 78234665 | 78370085 | RNF213 | ALO17 | Cancer_Gene_Census | |
| 17 | 56429861 | 56494480 | RNF43 | | Cancer_Gene_Census | |
| 3 | 78646390 | 79816965 | ROBO1 | | Cancer_Related_Genes_Panel | |
| 3 | 75955826 | 77699115 | ROBO2 | | Cancer_Related_Genes_Panel | |
| 6 | 117609463 | 117747018 | ROS1 | | Cancer_Gene_Census | |
| X | 153618315 | 153637504 | RPL10 | | Cancer_Gene_Census | |
| 1 | 6241329 | 6269449 | RPL22 | | Cancer_Gene_Census | |
| 1 | 93297597 | 93307481 | RPL5 | | Cancer_Gene_Census | |
| 3 | 128338817 | 128399918 | RPN1 | | Cancer_Gene_Census | |

| Chr | Start | End | Gene | Alternative_Gene_name | Gene_source/PMID | Paper title |
|---|---|---|---|---|---|---|
| 17 | 57970443 | 58027925 | RPS6KB1 | | Cancer_Related_Genes_Panel | |
| 17 | 78518625 | 78940173 | RPTOR | | Cancer_Related_Genes_Panel | |
| 8 | 103216732 | 103251346 | RRM2B | | Cancer_Related_Genes_Panel | |
| 8 | 108911544 | 109095913 | RSPO2 | | Cancer_Gene_Census | |
| 6 | 127439749 | 127518910 | RSPO3 | | Cancer_Gene_Census | |
| 21 | 36160098 | 37357047 | RUNX1 | | Cancer_Gene_Census | |
| 8 | 92971152 | 93107443 | RUNX1T1 | CBFA2T1 | Cancer_Gene_Census | |
| 7 | 66452664 | 66460635 | SBDS | | Cancer_Gene_Census | |
| 20 | 43953928 | 43977064 | SDC4 | | Cancer_Gene_Census | |
| 5 | 218356 | 256815 | SDHA | | PMID: 21505157, PMID: 22955521 | SDHA loss-of-function mutations in KIT-PDGFRA wild-type gastrointestinal stromal tumors identified by massively parallel sequencing. AND Loss of expression of |
| | | | | | | SDHA predicts SDHA mutations in gastrointestinal stromal tumors. |
| 11 | 61197597 | 61214225 | SDHAF2 | SDH5 | Cancer_Gene_Census | |
| 1 | 17345217 | 17380665 | SDHB | | Cancer_Gene_Census | |
| 1 | 161284047 | 161332984 | SDHC | | Cancer_Gene_Census | |
| 11 | 111957622 | 111966517 | SDHD | | Cancer_Gene_Census | |
| 22 | 19701987 | 19712295 | SEPT5 | PNUTL1 | Cancer_Gene_Census | |
| X | 118749687 | 118829533 | SEPT6 | | Cancer_Gene_Census | |
| 17 | 75277492 | 75496674 | SEPT9 | MSF | Cancer_Gene_Census | |
| 9 | 131445703 | 131458679 | SET | | Cancer_Gene_Census | |
| 18 | 42260138 | 42648475 | SETBP1 | | Cancer_Gene_Census | |
| 3 | 47057919 | 47205457 | SETD2 | | Cancer_Gene_Census | |

| Chr | Start | End | Gene | Alternative_Gene_name | Gene_source/PMID | Paper title |
|---|---|---|---|---|---|---|
| 11 | 64532078 | 64546258 | SF1 | | PMID: 24467717 | Chronic myelomonocytic leukaemia: a concise clinical and pathophysiological review. |
| 2 | 198256698 | 198299815 | SF3B1 | | Cancer_Gene_Census | |
| 1 | 35641979 | 35658749 | SFPQ | | Cancer_Gene_Census | |
| 17 | 74730201 | 74733413 | SFRS2 | SRSF2 | Cancer_Gene_Census | |
| 6 | 36562090 | 36571209 | SFRS3 | SRSF3 | Cancer_Gene_Census | |
| 12 | 111843752 | 111889426 | SH2B3 | | Cancer_Gene_Census | |
| 19 | 4360370 | 4400496 | SH3GL1 | | Cancer_Gene_Census | |
| 1 | 154934774 | 154946959 | SHC1 | | Cancer_Related_Genes_Panel | |
| 5 | 36152091 | 36184421 | SKP2 | | Cancer_Related_Genes_Panel | |
| 4 | 25656923 | 25680370 | SLC34A2 | | Cancer_Gene_Census | |
| 1 | 205626979 | 205649587 | SLC45A3 | | Cancer_Gene_Census | |
| 4 | 20254883 | 20622184 | SLIT2 | | Cancer_Related_Genes_Panel | |
| 18 | 45357922 | 45457515 | SMAD2 | | Cancer_Related_Genes_Panel | |
| 15 | 67358183 | 67487533 | SMAD3 | | Cancer_Related_Genes_Panel | |
| 18 | 48556583 | 48611415 | SMAD4 | | Cancer_Gene_Census | |
| 19 | 11071598 | 11172958 | SMARCA4 | | Cancer_Gene_Census | |
| 22 | 24129150 | 24176703 | SMARCB1 | | Cancer_Gene_Census | |
| 12 | 50478983 | 50494495 | SMARCD1 | | Cancer_Gene_Census | |
| 17 | 38781214 | 38821393 | SMARCE1 | | Cancer_Gene_Census | |
| X | 53401070 | 53449677 | SMC1A | | PMID: 24710217 | The landscape of somatic mutations in epigenetic regulators across 1,000 paediatric cancer genomes. |

| Chr | Start | End | Gene | Alternative_Gene_name | Gene_source/PMID | Paper title |
|---|---|---|---|---|---|---|
| 10 | 112327449 | 112364394 | SMC3 | | Cancer_Related_Genes_Panel | Mutation detection in formalin-fixed prostate cancer biopsies taken at the time of diagnosis using next-generation DNA sequencing. |
| 7 | 128828713 | 128853386 | SMO | | Cancer_Gene_Census | |
| 7 | 98625061 | 98741723 | SMURF1 | | Cancer_Related_Genes_Panel | |
| 16 | 12146055 | 12668145 | SNX29 | RUNDC2A | Cancer_Gene_Census | |
| 16 | 11348262 | 11350036 | SOCS1 | | Cancer_Gene_Census | |
| 22 | 38366693 | 38383429 | SOX10 | | Cancer_Related_Genes_Panel | |
| 3 | 181429722 | 181432221 | SOX2 | | Cancer_Gene_Census | |
| 17 | 70117161 | 70122552 | SOX9 | | Cancer_Related_Genes_Panel | |
| 1 | 16174359 | 16266955 | SPEN | | Cancer_Gene_Census | |
| 17 | 47676246 | 47755596 | SPOP | | Cancer_Gene_Census | |
| 15 | 38545052 | 38649450 | SPRED1 | | PMID: 24469042 | SPRED1, a RAS MAPK pathway inhibitor that causes Legius syndrome, is a tumour suppressor downregulated in paediatric acute myeloblastic leukaemia. |
| 20 | 35973088 | 36034453 | SRC | | Cancer_Related_Genes_Panel | |
| 3 | 9022278 | 9439200 | SRGAP3 | | Cancer_Gene_Census | |
| 18 | 23596219 | 23670611 | SS18 | | Cancer_Gene_Census | |
| 20 | 60718822 | 60757540 | SS18L1 | | Cancer_Gene_Census | |
| X | 48114752 | 48126879 | SSX1 | | Cancer_Gene_Census | |
| X | 52673111 | 52736239 | SSX2 | | Cancer_Gene_Census | |
| X | 48242863 | 48252785 | SSX4 | | Cancer_Gene_Census | |
| X | 123094062 | 123556514 | STAG2 | | Cancer_Gene_Census | |
| 17 | 40465342 | 40540513 | STAT3 | | Cancer_Gene_Census | |

| Chr | Start | End | Gene | Alternative_Gene_name | Gene_source/PMID | Paper title |
|---|---|---|---|---|---|---|
| 2 | 191894302 | 192016322 | STAT4 | | Cancer_Related_Genes_Panel | Mutation detection in formalin-fixed prostate cancer biopsies taken at the time of diagnosis using next-generation DNA sequencing. |
| 17 | 40351186 | 40428725 | STAT5B | | Cancer_Gene_Census | |
| 1 | 47715811 | 47779819 | STIL | SIL | Cancer_Gene_Census | |
| 19 | 1205798 | 1228434 | STK11 | | Cancer_Gene_Census | |
| 10 | 104263744 | 104393292 | SUFU | | Cancer_Gene_Census | |
| 17 | 30264056 | 30328064 | SUZ12 | | Cancer_Gene_Census | |
| 9 | 93564069 | 93660831 | SYK | | Cancer_Gene_Census | |
| 17 | 34136488 | 34174237 | TAF15 | | Cancer_Gene_Census | |
| 1 | 47681963 | 47697892 | TAL1 | | Cancer_Gene_Census | |
| 9 | 108424738 | 108425126 | TAL2 | | Cancer_Gene_Census | |
| 3 | 176737143 | 176915261 | TBL1XR1 | | Cancer_Gene_Census | |
| X | 79270255 | 79287268 | TBX22 | | Cancer_Related_Genes_Panel | |
| 12 | 115108059 | 115121969 | TBX3 | | Cancer_Gene_Census | |
| 8 | 54879119 | 54935008 | TCEA1 | | Cancer_Gene_Census | |
| 15 | 57210823 | 57582051 | TCF12 | | Cancer_Gene_Census | |
| 19 | 1609293 | 1652326 | TCF3 | | Cancer_Gene_Census | |
| 10 | 114710009 | 114927437 | TCF7L2 | | Cancer_Gene_Census | |
| 14 | 96176305 | 96180533 | TCL1A | | Cancer_Gene_Census | |
| 14 | 96116835 | 96158965 | TCL6 | | Cancer_Gene_Census | |
| 5 | 1253262 | 1295162 | TERT | | Cancer_Gene_Census | |
| 10 | 70320413 | 70454239 | TET1 | LCX | Cancer_Gene_Census | |
| 4 | 106067032 | 106200973 | TET2 | | Cancer_Gene_Census | |
| X | 48886242 | 48901012 | TFE3 | | Cancer_Gene_Census | |

| Chr | Start | End | Gene | Alternative_ Gene_name | Gene_source/PMID | Paper title |
|-----|-------|-----|------|------------------------|------------------|-------------|
| 6 | 41651716 | 41703997 | TFEB | | Cancer_Gene_Census | |
| 3 | 100428205 | 100467810 | TFG | | Cancer_Gene_Census | |
| 19 | 54610320 | 54619055 | TFPT | | Cancer_Gene_Census | |
| 3 | 195754054 | 195809060 | TFRC | | Cancer_Gene_Census | |
| 3 | 30647994 | 30735634 | TGFBR2 | | Cancer_Related_Genes_Panel | |
| 15 | 43568479 | 43594453 | TGM7 | | PMID: 24014293 | Recurrent gene mutations in CLL. |
| 1 | 36690017 | 36770958 | THRAP3 | | Cancer_Gene_Census | |
| 10 | 102890262 | 102897546 | TLX1 | | Cancer_Gene_Census | |
| 5 | 170736288 | 170739137 | TLX3 | | Cancer_Gene_Census | |
| 21 | 42836478 | 42903043 | TMPRSS2 | | Cancer_Gene_Census | |
| 6 | 138188351 | 138204449 | TNFAIP3 | | Cancer_Gene_Census | |
| 1 | 2487078 | 2497061 | TNFRSF14 | | Cancer_Gene_Census | |
| 16 | 12058964 | 12061925 | TNFRSF17 | | Cancer_Gene_Census | |
| 20 | 39657458 | 39753127 | TOP1 | | Cancer_Gene_Census | |
| 17 | 7565257 | 7590856 | TP53 | | Cancer_Gene_Census | |
| 1 | 3569084 | 3652765 | TP73 | | Cancer_Related_Genes_Panel | |
| 1 | 154127784 | 154167124 | TPM3 | | Cancer_Gene_Census | |
| 19 | 16178317 | 16213813 | TPM4 | | Cancer_Gene_Census | |
| 1 | 186282954 | 186344825 | TPR | | Cancer_Gene_Census | |
| 9 | 139776364 | 139821059 | TRAF2 | | Cancer_Related_Genes_Panel | |
| 16 | 2205766 | 2228130 | TRAF7 | | Cancer_Gene_Census | |
| 7 | 138144953 | 138274738 | TRIM24 | TIF1 | Cancer_Gene_Census | |
| 6 | 28870779 | 28891768 | TRIM27 | | Cancer_Gene_Census | |

(continued)

| Chr | Start | End | Gene | Alternative_Gene_name | Gene_source/PMID | Paper title |
|---|---|---|---|---|---|---|
| 1 | 114935399 | 115053781 | TRIM33 | | Cancer_Gene_Census | |
| 1 | 33611003 | 33647671 | TRIM62 | DEAR1 | PMID: 19536326, PMID: 23838884 | DEAR1 is a dominant regulator of acinar morphogenesis and an independent predictor of local recurrence-free survival in early-onset breast cancer. AND DEAR1 is a chromosome 1p35 tumor suppressor and master regulator of TGF-β-driven epithelial-mesenchymal transition. |
| 14 | 92435462 | 92506817 | TRIP11 | | Cancer_Gene_Census | |
| 9 | 73149949 | 74061820 | TRPM3 | | PMID: 24292195 | Mutational landscape of gingivo-buccal oral squamous cell carcinoma reveals new recurrently-mutated genes and molecular subgroups. |
| 7 | 98475556 | 98610866 | TRRAP | | Cancer_Gene_Census | |
| 9 | 135766735 | 135820020 | TSC1 | | Cancer_Gene_Census | |
| 16 | 2097466 | 2138721 | TSC2 | | Cancer_Gene_Census | |
| 14 | 81421775 | 81612646 | TSHR | | Cancer_Gene_Census | |
| 2 | 113239731 | 113290227 | TTL | | Cancer_Gene_Census | |
| 21 | 44513066 | 44527697 | U2AF1 | | Cancer_Gene_Census | |
| 19 | 56165416 | 56186081 | U2AF2 | | PMID: 24467717 | Chronic myelomonocytic leukaemia: a concise clinical and pathophysiological review. |
| 8 | 103265572 | 103424495 | UBR5 | | Cancer_Gene_Census | |
| 15 | 54305101 | 54920806 | UNC13C | | PMID: 24292195 | Mutational landscape of gingivo-buccal oral squamous cell carcinoma reveals new recurrently-mutated genes and molecular subgroups. |
| 17 | 5019733 | 5078329 | USP6 | | Cancer_Gene_Census | |
| X | 40944888 | 41092185 | USP9X | | Cancer_Related_Genes_Panel | |
| 6 | 43737921 | 43754224 | VEGFA | | Cancer_Related_Genes_Panel | |
| 3 | 10182692 | 10193904 | VHL | | Cancer_Gene_Census | |
| 10 | 114206756 | 114578503 | VTI1A | | Cancer_Gene_Census | |

(continued)

| Chr | Start | End | Gene | Alternative_Gene_name | Gene_source/PMID | Paper title |
|---|---|---|---|---|---|---|
| X | 48534985 | 48549818 | WAS | | Cancer_Gene_Census | |
| 4 | 1873151 | 1983934 | WHSC1 | | Cancer_Gene_Census | |
| 8 | 38132560 | 38239790 | WHSC1L1 | | Cancer_Gene_Census | |
| 12 | 65444407 | 65515116 | WIF1 | | Cancer_Gene_Census | |
| 8 | 30890778 | 31031276 | WRN | | Cancer_Gene_Census | |
| 17 | 25621106 | 25640657 | WSB1 | | Cancer_Related_Genes_Panel | |
| 11 | 32409321 | 32457176 | WT1 | | Cancer_Gene_Census | |
| 3 | 149235022 | 149454501 | WWTR1 | | Cancer_Gene_Census | |
| 22 | 29190543 | 29196585 | XBP1 | | Idenified from this analysis | |
| 9 | 100437191 | 100459639 | XPA | | Cancer_Gene_Census | |
| 3 | 14186647 | 14220283 | XPC | | Cancer_Gene_Census | |
| 2 | 61704984 | 61765761 | XPO1 | | Cancer_Gene_Census | |
| 11 | 101981210 | 102104154 | YAP1 | | Cancer_Related_Genes_Panel | |
| 20 | 43514317 | 43537173 | YWHAB | | Cancer_Related_Genes_Panel | |
| 17 | 1247566 | 1303505 | YWHAE | | Cancer_Gene_Census | |
| 2 | 9724101 | 9771143 | YWHAQ | | Cancer_Related_Genes_Panel | |
| 8 | 101930804 | 101965616 | YWHAZ | | Cancer_Related_Genes_Panel | |
| 8 | 81397902 | 81438500 | ZBTB10 | | Cancer_Related_Genes_Panel | Mutation detection in formalin-fixed prostate cancer biopsies taken at the time of diagnosis using next-generation DNA sequencing. |
| 11 | 113930315 | 114121395 | ZBTB16 | ZNF145 | Cancer_Gene_Census | |

103

EP 3 452 937 B1

| Chr | Start | End | Gene | Alternative_ Gene_name | Gene_source/PMID | Paper title |
|-----|-------|-----|------|------------------------|------------------|-------------|
| 16 | 72816784 | 73082274 | ZFHX3 | | Cancer_Related_Genes_Panel | Mutation detection in formalin-fixed prostate cancer biopsies taken at the time of diagnosis using next-generation DNA sequencing. |
| 14 | 69254375 | 69261453 | ZFP36L1 | | Idenified from this analysis | |
| 13 | 20532810 | 20663253 | ZMYM2 | ZNF198 | Cancer_Gene_Census | |
| X | 70459474 | 70474996 | ZMYM3 | | PMID: 24014293 | Recurrent gene mutations in CLL. |
| 20 | 52183604 | 52226446 | ZNF217 | | Cancer_Related_Genes_Panel | |
| 19 | 54024235 | 54083523 | ZNF331 | | Cancer_Gene_Census | |
| 12 | 6775643 | 6798676 | ZN F384 | | Cancer_Gene_Census | |
| 18 | 22641888 | 22932214 | ZNF521 | | Cancer_Gene_Census | |
| 3 | 179040779 | 179053323 | ZNF639 | | Cancer_Related_Genes_Panel | Mutation detection in formalin-fixed prostate cancer biopsies taken at the time of diagnosis using next-generation DNA sequencing. |
| 8 | 81550769 | 81787016 | ZNF704 | | Cancer_Related_Genes_Panel | Mutation detection in formalin-fixed prostate cancer biopsies taken at the time of diagnosis using next-generation DNA sequencing. |
| X | 15808595 | 15841383 | ZRSR2 | | Cancer_Gene_Census | |

Table 6

| gene | freq | sample_ count | chrID | start | end | density | expect_ sample_ count | pvalue | pvalue_adjusted | Is Breast Cancer gene | property | fragile_site |
|---|---|---|---|---|---|---|---|---|---|---|---|---|
| ABI1 | 8 | 5 | 10 | 27035522 | 27149959 | 78.6458925 | 3 | 0.18428445 | 0.589710244 | No | Dom | N/A |
| ABL1 | 8 | 5 | 9 | 133589333 | 133763062 | 46.0487311 | 6 | 0.71638537 | 0.988302814 | No | Dom | N/A |
| ABL2 | 4 | 4 | 1 | 179068462 | 179198819 | 38.3562064 | 8 | 0.95864081 | 1 | No | Dom | N/A |
| ACSL3 | 8 | 7 | 2 | 223725652 | 223809357 | 119.467176 | 3 | 0.03310304 | 0.183444415 | No | Dom | N/A |
| ACSL6 | 1 | 1 | 5 | 131142683 | 131347936 | 9.74407195 | 3 | 0.95061283 | 1 | No | Dom | Whole |
| ACVR1 | 4 | 3 | 2 | 158592958 | 158732374 | 43.0366672 | 5 | 0.87647828 | 1 | No | Dom | N/A |
| ACVR2A | 1 | 1 | 2 | 148602086 | 148688393 | 11.5865457 | 1 | 0.63244927 | 0.917609012 | No | Unknown | within 2 Mb |
| AD000671.3 | 5 | 3 | 19 | 36208921 | 36229779 | 287.659411 | 3 | 0.57741173 | 0.917609012 | No | Unknown | within 2 Mb |
| ADAM29 | 1 | 1 | 4 | 175750819 | 175899331 | 6.73346262 | 4 | 0.98194539 | 1 | No | Unknown | N/A |
| AFF1 | 12 | 9 | 4 | 87856154 | 88062190 | 58.2422489 | 4 | 0.02093841 | 0.125630467 | No | Dom | within 2 Mb |
| AFF3 | 35 | 31 | 2 | 100163718 | 100759201 | 82.2861442 | 22 | 0.03740913 | 0.205215796 | No | Dom | Whole |
| AFF4 | 1 | 1 | 5 | 132211071 | 132299326 | 11.3308028 | 1.5 | 0.77731844 | 1 | No | Dom | Whole |
| AKAP9 | 4 | 4 | 7 | 91570181 | 91739989 | 29.4450203 | 5 | 0.73623267 | 1 | No | Dom | Whole |
| AKT1 | 2 | 2 | 14 | 105235689 | 105262080 | 75.783411 | 1 | 0.26424102 | 0.67645701 | Yes | Dom | N/A |
| AKT2 | 8 | 5 | 19 | 40736224 | 40791302 | 145.248557 | 4.5 | 0.46827906 | 0.850879303 | Yes | Dom | Whole |
| AKT3 | 12 | 10 | 1 | 243651535 | 244013430 | 38.685254 | 14 | 0.89357833 | 1 | No | Dom | Whole |
| ALK | 20 | 16 | 2 | 29415640 | 30144432 | 41.1640084 | 8 | 0.00778406 | 0.062272449 | Yes | Dom | N/A |
| APC | 4 | 4 | 5 | 112043195 | 112181936 | 36.0383737 | 5 | 0.73623267 | 1 | Yes | Rec | N/A |
| AR | 10 | 7 | 23 | 66764465 | 66950461 | 53.7645971 | 10 | 0.87212233 | 1 | No | Dom? | N/A |
| ARFRP1 | 2 | 2 | 20 | 62329996 | 62339355 | 213.698045 | 0 | 0 | 0 | No | Unknown | N/A |
| ARHGAP26 | 8 | 6 | 5 | 142149949 | 142608576 | 21.8042113 | 14 | 0.99487924 | 1 | No | Dom | N/A |
| ARHGEF12 | 7 | 6 | 11 | 120207946 | 120360645 | 65.4883136 | 5 | 0.38403817 | 0.779273892 | No | Dom | Whole |

| gene | freq | sample_ count | chrID | start | end | density | expect_ sample_ count | pvalue | pvalue_adjusted | Is Breast Cancer gene | property | fragile_site |
|---|---|---|---|---|---|---|---|---|---|---|---|---|
| ARID1A | 7 | 6 | 1 | 27022522 | 27108601 | 104.555118 | 2 | 0.01637035 | 0.114991732 | Yes | Rec | Whole |
| ARID1B | 43 | 28 | 6 | 157099063 | 157530401 | 125.191845 | 14 | 0.00053155 | 0.005669877 | Yes | Rec | N/A |
| ARID2 | 13 | 7 | 12 | 46123492 | 46301823 | 89.7208001 | 11 | 0.92340858 | 1 | Yes | Rec | Whole |
| ARID5B | 21 | 9 | 10 | 63661059 | 63856703 | 117.560467 | 9 | 0.54541588 | 0.917609012 | No | Unknown | Whole |
| ARNT | 6 | 5 | 1 | 150782181 | 150849244 | 89.4681121 | 7.5 | 0.86965349 | 1 | No | Dom | Whole |
| ASPSCR1 | 3 | 2 | 17 | 79935426 | 79975280 | 100.366337 | 2 | 0.59447836 | 0.917609012 | No | Dom | N/A |
| ASXL1 | 7 | 7 | 20 | 30946153 | 31027122 | 86.45284 | 4.5 | 0.16817227 | 0.556708199 | No | Rec | N/A |
| ATF1 | 2 | 2 | 12 | 51157819 | 51214907 | 35.0336323 | 3 | 0.80165331 | 1 | No | Dom | Whole |
| ATIC | 1 | 1 | 2 | 216176540 | 216214487 | 26.3525443 | 1.5 | 0.77731844 | 1 | No | Dom | N/A |
| ATM | 7 | 6 | 11 | 108093559 | 108239826 | 41.0208728 | 3 | 0.08337607 | 0.35456646 | Yes | Rec | N/A |
| ATP1A1 | 2 | 2 | 1 | 116915290 | 116952883 | 53.2013939 | 3.5 | 0.86493865 | 1 | No | Dom | N/A |
| ATR | 7 | 6 | 3 | 142168077 | 142297668 | 69.4492673 | 9 | 0.88626912 | 1 | No | Rec | N/A |
| ATRX | 9 | 7 | 23 | 76760359 | 77041719 | 39.0958203 | 13 | 0.97533701 | 1 | No | Rec | N/A |
| AURKA | 4 | 4 | 20 | 54944445 | 54967393 | 217.883911 | 1.5 | 0.06538985 | 0.315362842 | No | Dom? | N/A |
| AURKB | 1 | 1 | 17 | 8108051 | 8113936 | 169.923534 | 0.5 | 0.39360479 | 0.779273892 | No | Dom? | N/A |
| AXIN1 | 7 | 6 | 16 | 337440 | 402673 | 122.637315 | 3.5 | 0.14176386 | 0.488754016 | No | Rec | N/A |
| AXIN2 | 7 | 5 | 17 | 63524685 | 63557765 | 241.037969 | 5 | 0.56029392 | 0.917609012 | No | Dom/Rec | N/A |
| AXL | 2 | 2 | 19 | 41725108 | 41767670 | 46.990273 | 1 | 0.26424102 | 0.67645701 | No | Dom | Whole |
| BAG4 | 10 | 8 | 8 | 38034312 | 38070809 | 246.595611 | 7 | 0.4012842 | 0.786189453 | No | Dom? | N/A |
| BAGE3 | 21 | 18 | 7 | 151832007 | 152133628 | 92.8317325 | 13 | 0.10695149 | 0.407973887 | Yes | Rec | Whole |
| BAP1 | 1 | 1 | 3 | 52435029 | 52444366 | 107.100782 | 2 | 0.86514834 | 1 | Yes | Rec | N/A |
| BCL10 | 5 | 1 | 1 | 85731464 | 85743771 | 406.272853 | 0.5 | 0.39360479 | 0.779273892 | No | Dom | Whole |
| BCL11A | 1 | 1 | 2 | 60678302 | 60780768 | 9.7593348 | 3 | 0.95061283 | 1 | No | Dom | N/A |
| BCL11B | 5 | 3 | 14 | 99635624 | 99737861 | 48.9059734 | 2 | 0.3233233 | 0.776778728 | No | Dom | N/A |
| BCL2 | 5 | 5 | 18 | 60790579 | 60987361 | 40.6541249 | 19.6782 | 0.9999825 | 1 | No | Dom | Whole |
| BCL6 | 1 | 1 | 3 | 187439165 | 187463515 | 0 | 1 | 0.63244927 | 0.917609012 | No | Dom | Whole |

| | | | | | | | | | | | | |
|---|---|---|---|---|---|---|---|---|---|---|---|---|
| BCL7A | 2 | 2 | 12 | 122459861 | 122499950 | 49.888997 | 1.5 | 0.442399 | 0.819362781 | No | Dom | Whole |
| BCL9 | 3 | 3 | 1 | 147013182 | 147098017 | 47.1503507 | 5 | 0.87647828 | 1 | No | Dom | Whole |
| BCOR | 3 | 3 | 23 | 39909068 | 40036582 | 31.3691046 | 3 | 0.57741173 | 0.917609012 | No | Rec | N/A |
| BCR | 10 | 9 | 22 | 23521891 | 23660224 | 93.9761301 | 4 | 0.02093841 | 0.125630467 | No | Dom | N/A |
| BLM | 9 | 6 | 15 | 91260558 | 91358692 | 81.5211853 | 0 | 0 | 0 | No | Unknown | N/A |
| BMPR1A | 6 | 3 | 10 | 88516396 | 88684945 | 47.4639422 | 6 | 0.93898741 | 1 | No | Rec | within 2 Mb |
| BPTF | 47 | 25 | 17 | 65821640 | 66033571 | 240.644361 | 20 | 0.15270196 | 0.514364498 | No | Dom | N/A |
| BRAF | 10 | 10 | 7 | 140424943 | 140624564 | 60.1139159 | 5 | 0.03117995 | 0.181410595 | Yes | Dom | N/A |
| BRCA1 | 6 | 5 | 17 | 41196312 | 41277500 | 86.2196384 | 5 | 0.56029392 | 0.917609012 | Yes | Rec | N/A |
| BRCA2 | 5 | 5 | 13 | 32889611 | 32973347 | 35.8268845 | 3 | 0.18428445 | 0.589710244 | Yes | Rec | Partial |
| BRD3 | 1 | 1 | 9 | 136897963 | 136933657 | 28.015913 | 1.5 | 0.77731844 | 1 | No | Dom | N/A |
| BRD4 | 7 | 5 | 19 | 15348301 | 15391262 | 162.938479 | 8.5922 | 0.93110416 | 1 | No | Dom | N/A |
| BRIP1 | 53 | 24 | 17 | 59759985 | 59940755 | 298.722133 | 33 | 0.9611682 | 1 | Yes | Rec | N/A |
| BUB1B | 2 | 2 | 15 | 40453210 | 40513378 | 33.2402606 | 1 | 0.26424102 | 0.67645701 | No | Rec | N/A |
| C11orf30 | 15 | 13 | 11 | 76156069 | 76262586 | 168.98711 | 17 | 0.86883646 | 1 | No | Dom | Whole |
| C16orf75 | 2 | 2 | 16 | 11410636 | 11445619 | 28.5853129 | 1 | 0.26424102 | 0.67645701 | No | Dom | N/A |
| C6orf97 | 10 | 7 | 6 | 151015175 | 151942328 | 78.6454114 | 0 | 0 | 0 | No | Unknown | N/A |
| CACNA1D | 9 | 7 | 3 | 53528683 | 53846490 | 37.7587655 | 6 | 0.39369566 | 0.779273892 | No | Dom | N/A |
| CALR | 3 | 3 | 19 | 13049414 | 13055304 | 509.337861 | 1.5 | 0.19098454 | 0.591436001 | No | Dom | N/A |
| CAMTA1 | 20 | 15 | 1 | 6845384 | 7829764 | 18.2856214 | 22 | 0.95543189 | 1 | No | Dom | Whole |
| CARD11 | 5 | 5 | 7 | 2945775 | 3083579 | 43.5401004 | 3 | 0.18428445 | 0.589710244 | No | Dom | Whole |
| CARS | 2 | 1 | 11 | 3022152 | 3078667 | 35.3888348 | 2.5 | 0.91837315 | 1 | No | Dom | N/A |
| CASC5 | 3 | 2 | 15 | 40886447 | 40954881 | 43.8378584 | 0 | 0 | 0 | No | Dom | N/A |
| CASP8 | 5 | 5 | 2 | 202098166 | 202152434 | 128.98946 | 1.5 | 0.01841834 | 0.116925749 | Yes | Rec | Whole |
| CBFA2T3 | 7 | 6 | 16 | 88941266 | 89043401 | 88.1186665 | 1 | 0.00058329 | 0.006018047 | No | Dom | N/A |
| CBFB | 4 | 3 | 16 | 67063050 | 67134961 | 69.5303917 | 3 | 0.57741173 | 0.917609012 | Yes | Dom | Whole |

| | | | | | | | | | | | | |
|---|---|---|---|---|---|---|---|---|---|---|---|---|
| CBL | 5 | 5 | 11 | 119076990 | 119178856 | 49.0840909 | 2 | 0.05233021 | 0.274020034 | No | Dom/Rec | Whole |
| CBLB | 3 | 3 | 3 | 105374305 | 105588396 | 28.0254658 | 5 | 0.87647828 | 1 | No | Rec | N/A |
| CBLC | 1 | 1 | 19 | 45281126 | 45303891 | 43.927081 | 1 | 0.63244927 | 0.917609012 | No | Rec | Whole |
| CCDC6 | 5 | 5 | 10 | 61548521 | 61666818 | 50.7197985 | 7 | 0.82872664 | 1 | No | Dom | Whole |
| CCND1 | 6 | 6 | 11 | 69455873 | 69469241 | 374.027528 | 6.5 | 0.63223308 | 0.917609012 | Yes | Dom | Whole |
| CCND2 | 1 | 1 | 12 | 4382902 | 4414521 | 31.6265537 | 1 | 0.63244927 | 0.917609012 | No | Dom | N/A |
| CCND3 | 3 | 2 | 6 | 41902671 | 42018095 | 25.9911284 | 3 | 0.80165331 | 1 | No | Dom | N/A |
| CD274 | 1 | 1 | 9 | 5450525 | 5470547 | 49.9450604 | 0 | 0 | 0 | No | Dom | N/A |
| CD74 | 2 | 2 | 5 | 149781206 | 149792332 | 179.759123 | 0.5 | 0.09013623 | 0.35456646 | No | Dom | N/A |
| CD79B | 1 | 1 | 17 | 62006100 | 62009714 | 276.701716 | 0 | 0 | 0 | No | Dom | N/A |
| CDC6 | 16 | 9 | 17 | 38443885 | 38459171 | 1046.70941 | 10.7002 | 0.74280719 | 1 | No | Dom | N/A |
| CDC73 | 4 | 4 | 1 | 193090919 | 193223031 | 45.4160107 | 2 | 0.14255325 | 0.488754016 | No | Rec | Whole |
| CDH1 | 8 | 7 | 16 | 68771128 | 68869444 | 101.712844 | 4.5 | 0.16817227 | 0.556708199 | Yes | Rec | Whole |
| CDH11 | 2 | 1 | 16 | 64977656 | 65156101 | 11.2079352 | 1 | 0.63244927 | 0.917609012 | No | Dom | within 2 Mb |
| CDK12 | 47 | 28 | 17 | 37617739 | 37690800 | 684.359645 | 22.5 | 0.14167208 | 0.488754016 | Yes | Rec | N/A |
| CDK6 | 23 | 15 | 7 | 92234235 | 92465908 | 146.758578 | 6 | 0.00130666 | 0.012139304 | No | Dom | Whole |
| CDK8 | 9 | 8 | 13 | 26828276 | 26979375 | 59.5635974 | 5 | 0.13243327 | 0.488754016 | No | Dom | N/A |
| CDKN1A | 2 | 1 | 6 | 36644305 | 36655116 | 184.996763 | 1 | 0.63244927 | 0.917609012 | No | Unknown | N/A |
| CDKN1B | 2 | 1 | 12 | 12867992 | 12875305 | 273.485574 | 1 | 0.63244927 | 0.917609012 | Yes | Rec | N/A |
| CDKN2A | 8 | 7 | 9 | 21967752 | 21995300 | 326.702483 | 0 | 0 | 0 | Yes | Rec | Whole |
| CDKN2B | 1 | 1 | 9 | 22002902 | 22009280 | 156.788962 | 1.5 | 0.77731844 | 1 | No | Rec | Whole |
| CDKN2C | 1 | 1 | 1 | 51426417 | 51440305 | 72.0046083 | 0 | 0 | 0 | No | Rec | Whole |
| CEP110 | 4 | 3 | 9 | 123837141 | 123939888 | 38.930577 | 5 | 0.87647828 | 1 | No | Dom | N/A |
| CEP76 | 1 | 1 | 18 | 12672631 | 12702773 | 33.1762989 | 1 | 0.63244927 | 0.917609012 | Yes | Rec | N/A |
| CHD1 | 2 | 2 | 5 | 98190908 | 98262240 | 56.0758145 | 1 | 0.26424102 | 0.67645701 | No | Unknown | Whole |
| CHD1L | 5 | 3 | 1 | 146714291 | 146767443 | 94.0698374 | 2 | 0.3233233 | 0.776778728 | No | Dom | Whole |

| | | | | | | | | | | | | |
|---|---|---|---|---|---|---|---|---|---|---|---|---|
| CHD2 | 8 | 8 | 15 | 93443419 | 93571237 | 78.2362422 | 10 | 0.78221225 | 1 | No | Rec | N/A |
| CHD8 | 2 | 2 | 14 | 21853358 | 21905404 | 38.4275449 | 3.5 | 0.86493865 | 1 | No | Unknown | N/A |
| CHEK1 | 2 | 2 | 11 | 125495036 | 125546148 | 58.6946314 | 2 | 0.59447836 | 0.917609012 | No | Unknown | N/A |
| CHEK2 | 5 | 5 | 22 | 29083731 | 29138410 | 109.731341 | 39.5 | 1 | 1 | Yes | Rec | Whole |
| CHIC2 | 2 | 2 | 4 | 54875956 | 54930857 | 54.6438134 | 5 | 0.96017324 | 1 | No | Dom | Whole |
| CHN1 | 8 | 6 | 2 | 175664042 | 175870097 | 43.6776589 | 4 | 0.21430775 | 0.636295161 | No | Dom | Whole |
| CHUK | 3 | 2 | 10 | 101948055 | 101989376 | 72.6023088 | 1 | 0.26424102 | 0.67645701 | No | Unknown | N/A |
| CIC | 1 | 1 | 19 | 42788817 | 42799949 | 89.8311175 | 0.5 | 0.39360479 | 0.779273892 | No | Rec | Whole |
| CIITA | 3 | 3 | 16 | 10971039 | 11023624 | 57.0504897 | 1.5 | 0.19098454 | 0.591436001 | No | Dom | N/A |
| CKS1B | 2 | 2 | 1 | 154947129 | 154951725 | 435.16101 | 0 | 0 | 0 | No | Dom | within 2 Mb |
| CLTC | 35 | 19 | 17 | 57697219 | 57774317 | 453.967678 | 17.5 | 0.39031443 | 0.779273892 | No | Dom | N/A |
| CNBP | 1 | 1 | 3 | 128886661 | 128902810 | 61.9233389 | 1 | 0.63244927 | 0.917609012 | No | Dom | N/A |
| COL1A1 | 3 | 2 | 17 | 48260650 | 48278993 | 163.550128 | 4.5 | 0.93960347 | 1 | No | Dom | N/A |
| COL2A1 | 1 | 1 | 12 | 48366748 | 48398285 | 31.7087865 | 1 | 0.63244927 | 0.917609012 | No | Unknown | Whole |
| COX6C | 5 | 4 | 8 | 100890372 | 100905895 | 322.102686 | 2.5 | 0.24218407 | 0.67645701 | No | Dom | within 2 Mb |
| CREB1 | 2 | 2 | 2 | 208394461 | 208468155 | 13.5696257 | 1 | 0.26424102 | 0.67645701 | No | Dom | Whole |
| CREB3L2 | 4 | 4 | 7 | 137559725 | 137686813 | 39.3428176 | 2 | 0.14255325 | 0.488754016 | No | Dom | N/A |
| CREBBP | 26 | 22 | 16 | 3775055 | 3930722 | 224.838919 | 11 | 0.00202096 | 0.017908831 | No | Dom/Rec | N/A |
| CRKL | 2 | 2 | 22 | 21271714 | 21308037 | 55.0615313 | 1.5 | 0.442399 | 0.819362781 | No | Dom | N/A |
| CRTC1 | 6 | 5 | 19 | 18794425 | 18893142 | 60.7798049 | 3.5 | 0.27425794 | 0.698993691 | No | Dom | N/A |
| CRTC3 | 4 | 3 | 15 | 91073157 | 91188577 | 43.3200485 | 3 | 0.57741173 | 0.917609012 | No | Dom | N/A |
| CSF3R | 1 | 1 | 1 | 36931644 | 36948879 | 58.0214679 | 1 | 0.63244927 | 0.917609012 | No | Unknown | N/A |
| CTCF | 5 | 5 | 16 | 67596310 | 67673086 | 78.1494217 | 4 | 0.37116223 | 0.779273892 | Yes | Rec | Whole |
| CTNNB1 | 9 | 7 | 3 | 41236328 | 41301587 | 168.559126 | 1 | 8.10E-05 | 0.00093289 | Yes | Dom | N/A |
| CTNND1 | 1 | 1 | 11 | 57529234 | 57586651 | 17.4164446 | 5 | 0.99341167 | 1 | No | Dom | N/A |
| CUX1 | 16 | 13 | 7 | 101459240 | 101927250 | 40.5974231 | 10 | 0.20672753 | 0.633377956 | No | Rec | Whole |

| | | | | | | | | | | | | |
|---|---|---|---|---|---|---|---|---|---|---|---|---|
| CYLD | 2 | 1 | 16 | 50775961 | 50835846 | 33.3973449 | 0.5 | 0.39360479 | 0.779273892 | No | Rec | N/A |
| CYTSB | 41 | 24 | 17 | 19912614 | 20222339 | 132.375494 | 18 | 0.09753687 | 0.374541574 | No | Dom | N/A |
| DCUN1D1 | 4 | 3 | 3 | 182655862 | 182703741 | 125.3159 | 1 | 0.0801369 | 0.35456646 | No | Dom | within 2 Mb |
| DDB2 | 2 | 2 | 11 | 47236493 | 47260767 | 41.1963418 | 1.5 | 0.442399 | 0.819362781 | No | Rec | N/A |
| DDR2 | 15 | 11 | 1 | 162601163 | 162750237 | 107.329246 | 7 | 0.09718076 | 0.374541574 | No | Dom/Rec | N/A |
| DDX10 | 15 | 13 | 11 | 108535804 | 108811656 | 61.6272494 | 11 | 0.31020891 | 0.763591158 | No | Dom | N/A |
| DDX11 | 2 | 1 | 12 | 31226779 | 31257725 | 64.6287081 | 1.5 | 0.77731844 | 1 | No | Dom | N/A |
| DDX5 | 1 | 1 | 17 | 62495740 | 62502407 | 149.9925 | 1 | 0.63244927 | 0.917609012 | No | Dom | N/A |
| DEK | 3 | 3 | 6 | 18224099 | 18265054 | 97.6681724 | 1.5 | 0.19098454 | 0.591436001 | No | Dom | N/A |
| DICER1 | 4 | 4 | 14 | 95552566 | 95623759 | 56.1852991 | 2 | 0.14255325 | 0.488754016 | No | Rec | N/A |
| DNM2 | 12 | 8 | 19 | 10824143 | 10942579 | 109.763923 | 13 | 0.94793161 | 1 | No | Rec | N/A |
| DNMT3A | 4 | 4 | 2 | 25455496 | 25565459 | 45.4698399 | 5 | 0.73623267 | 1 | No | Rec | N/A |
| E2F3 | 9 | 9 | 6 | 20402398 | 20493941 | 142.009766 | 9.5 | 0.60975924 | 0.917609012 | No | Dom | N/A |
| EBF1 | 10 | 7 | 5 | 158122928 | 158526769 | 27.2384429 | 6 | 0.39369566 | 0.779273892 | No | Dom | N/A |
| ECT2L | 9 | 7 | 6 | 139117063 | 139225207 | 83.2223702 | 8 | 0.68838419 | 0.971836497 | No | Rec | N/A |
| EEF1A2 | 2 | 2 | 20 | 62119366 | 62130505 | 179.549331 | 0 | 0 | 0 | No | Dom | N/A |
| EGFR | 11 | 9 | 7 | 55086714 | 55324313 | 63.1315788 | 10 | 0.66921162 | 0.958070381 | Yes | Dom | Whole |
| EIF4A2 | 1 | 1 | 3 | 186500994 | 186507689 | 149.365198 | 0.5 | 0.39360479 | 0.779273892 | No | Dom | Whole |
| ELK3 | 3 | 1 | 12 | 96588207 | 96661608 | 40.8713778 | 2 | 0.86514834 | 1 | No | Dom | N/A |
| ELK4 | 2 | 2 | 1 | 205577071 | 205601090 | 124.90112 | 2.5 | 0.71339092 | 0.987772049 | No | Dom | N/A |
| ELL | 6 | 5 | 19 | 18553475 | 18632918 | 88.1134902 | 5 | 0.56029392 | 0.917609012 | No | Dom | N/A |
| ELN | 1 | 1 | 7 | 73442119 | 73484237 | 23.7428178 | 0 | 0 | 0 | No | Dom | Whole |
| EML4 | 6 | 6 | 2 | 42396490 | 42559688 | 49.0202086 | 7 | 0.70089872 | 0.982281417 | No | Dom | N/A |
| EP400 | 6 | 4 | 12 | 132434508 | 132565005 | 45.9780685 | 8 | 0.95864081 | 1 | No | Unknown | within 2 Mb |
| EPAS1 | 6 | 6 | 2 | 46520806 | 46613836 | 64.4953241 | 4 | 0.21430775 | 0.636295161 | No | Unknown | N/A |
| EPHA3 | 17 | 10 | 3 | 89156674 | 89531284 | 50.7194149 | 11 | 0.66164556 | 0.952769602 | No | Unknown | N/A |

| | | | | | | | | | | | | |
|---|---|---|---|---|---|---|---|---|---|---|---|---|
| EPHB1 | 25 | 19 | 3 | 134316643 | 134979309 | 57.3441221 | 16 | 0.25522438 | 0.67645701 | No | Unknown | N/A |
| EPS15 | 6 | 5 | 1 | 51819935 | 51985000 | 48.4657559 | 12 | 0.99284829 | 1 | No | Dom | Whole |
| ERBB2 | 36 | 17 | 17 | 37844393 | 37884915 | 888.406298 | 16.2088 | 0.45504388 | 0.8320534 | Yes | Dom | N/A |
| ERBB3 | 2 | 2 | 12 | 56473892 | 56497128 | 86.0733345 | 1 | 0.26424102 | 0.67645701 | No | Dom | N/A |
| ERBB4 | 40 | 29 | 2 | 212240446 | 213403565 | 44.7073773 | 20 | 0.03174103 | 0.182828358 | No | Unknown | N/A |
| ERC1 | 42 | 16 | 12 | 1100404 | 1605099 | 93.1255511 | 24 | 0.96839299 | 1 | No | Dom | N/A |
| ERCC2 | 3 | 2 | 19 | 45854246 | 45873876 | 152.827305 | 1 | 0.26424102 | 0.67645701 | Yes | Rec | Whole |
| ERCC3 | 1 | 1 | 2 | 128014866 | 128051752 | 27.1105569 | 0 | 0 | 0 | No | Rec | N/A |
| ERCC4 | 2 | 2 | 16 | 14014014 | 14046205 | 62.1291665 | 1 | 0.26424102 | 0.67645701 | No | Rec | within 2 Mb |
| ERCC5 | 4 | 2 | 13 | 103497194 | 103528345 | 96.3050945 | 0 | 0 | 0 | No | Rec | N/A |
| ERG | 16 | 13 | 21 | 39751949 | 40033704 | 60.3361076 | 8 | 0.06241519 | 0.315362842 | No | Dom | N/A |
| ESR1 | 55 | 32 | 6 | 151977826 | 152450754 | 126.869206 | 0 | 0 | 0 | Yes | Dom | N/A |
| ETV1 | 2 | 2 | 7 | 13930853 | 14031050 | 19.9606775 | 1 | 0.26424102 | 0.67645701 | No | Dom | N/A |
| ETV5 | 4 | 3 | 3 | 185764097 | 185828107 | 78.1127949 | 1 | 0.0801369 | 0.35456646 | No | Dom | Whole |
| ETV6 | 75 | 39 | 12 | 11802788 | 12048321 | 374.695051 | 17 | 2.17E-06 | 2.72E-05 | No | Dom | N/A |
| EWSR1 | 2 | 2 | 22 | 29663998 | 29696515 | 61.506289 | 2.5 | 0.71339092 | 0.987772049 | No | Dom | Whole |
| EXT1 | 43 | 21 | 8 | 118806729 | 119123938 | 151.319792 | 21 | 0.53068761 | 0.917609012 | No | Rec | Whole |
| EXT2 | 9 | 5 | 11 | 44117099 | 44266979 | 66.7200427 | 11 | 0.98558996 | 1 | No | Rec | N/A |
| EZH2 | 17 | 13 | 7 | 148504475 | 148581413 | 298.942005 | 5 | 0.001913 | 0.017216982 | No | Rec? | Whole |
| EZR | 4 | 3 | 6 | 159186773 | 159240444 | 74.5281437 | 2.5 | 0.45647374 | 0.8320534 | No | Dom | within 2 Mb |
| FADD | 2 | 2 | 11 | 70049269 | 70053508 | 471.809389 | 1.5 | 0.442399 | 0.819362781 | No | Dom | Whole |
| FAM123B | 1 | 1 | 23 | 63404997 | 63425624 | 48.4801474 | 1 | 0.63244927 | 0.917609012 | No | Unknown | N/A |
| FAM22B | 1 | 1 | 10 | 81462983 | 81474437 | 87.3057447 | 1 | 0.63244927 | 0.917609012 | No | Dom | N/A |
| FAM46C | 4 | 4 | 1 | 118148556 | 118170994 | 178.269008 | 0.5 | 0.00173754 | 0.015886075 | No | Rec | N/A |
| FANCA | 7 | 6 | 16 | 89803959 | 89883065 | 101.130129 | 2 | 0.01637035 | 0.114991732 | No | Rec | N/A |
| FANCC | 3 | 2 | 9 | 97861336 | 98079991 | 13.7202442 | 5 | 0.96017324 | 1 | No | Rec | N/A |

| | | | | | | | | | | | | |
|---|---|---|---|---|---|---|---|---|---|---|---|---|
| FANCD2 | 13 | 6 | 3 | 10068098 | 10143614 | 172.148949 | 2.5 | 0.0416477 | 0.224196951 | No | Rec | N/A |
| FANCF | 4 | 1 | 11 | 22644079 | 22647387 | 906.892382 | 1.5 | 0.77731844 | 1 | No | Rec | within 2 Mb |
| FAS | 1 | 1 | 10 | 90749226 | 90775542 | 37.999696 | 0.5 | 0.39360479 | 0.779273892 | No | Unknown | Whole |
| FBXO11 | 20 | 10 | 2 | 48016455 | 48132932 | 223.220035 | 6 | 0.08281174 | 0.35456646 | No | Rec | N/A |
| FBXW7 | 16 | 14 | 4 | 153242410 | 153456172 | 98.2400988 | 5 | 0.00065177 | 0.006586279 | Yes | Rec | N/A |
| FCGR2B | 2 | 2 | 1 | 161551136 | 161648444 | 20.5532947 | 6 | 0.98304553 | 1 | No | Dom | N/A |
| FGFR1 | 30 | 19 | 8 | 38268656 | 38326352 | 571.963394 | 28.848 | 0.98125539 | 1 | No | Dom | N/A |
| FGFR1OP | 1 | 1 | 6 | 167412670 | 167455906 | 23.1288741 | 0.5 | 0.39360479 | 0.779273892 | No | Dom | N/A |
| FGFR2 | 15 | 9 | 10 | 123237848 | 123357972 | 141.520429 | 10 | 0.66921162 | 0.958870381 | Yes | Dom | Whole |
| FGFR3 | 1 | 1 | 4 | 1795034 | 1810598 | 64.2508353 | 1.5 | 0.77731844 | 1 | Yes | Dom | N/A |
| FGFR4 | 1 | 1 | 5 | 176513887 | 176525145 | 88.8257239 | 1 | 0.63244927 | 0.917609012 | No | Unknown | Whole |
| FH | 1 | 1 | 1 | 241660903 | 241683061 | 45.1304269 | 0.5 | 0.39360479 | 0.779273892 | No | Rec | within 2 Mb |
| FHIT | 55 | 34 | 3 | 59735036 | 61237133 | 51.9274055 | 24 | 0.02843525 | 0.168852606 | No | Dom | Whole |
| FIP1L1 | 44 | 27 | 4 | 54243810 | 55161439 | 62.116607 | 20 | 0.07416448 | 0.34450596 | No | Dom | Whole |
| FLCN | 2 | 1 | 17 | 17115526 | 17140502 | 80.0768738 | 0.5 | 0.39360479 | 0.779273892 | No | Rec? | within 2 Mb |
| FLI1 | 2 | 2 | 11 | 128563813 | 128683161 | 16.7577169 | 6 | 0.98304553 | 1 | No | Dom | N/A |
| FLT1 | 11 | 6 | 13 | 28874489 | 29069232 | 61.6196731 | 7 | 0.70089872 | 0.982281417 | No | Unknown | N/A |
| FLT3 | 7 | 3 | 13 | 28577411 | 28674729 | 82.2047309 | 3.5 | 0.68002246 | 0.962390512 | No | Dom | N/A |
| FLT4 | 3 | 2 | 5 | 180028506 | 180076624 | 62.346731 | 1.5 | 0.442399 | 0.819362781 | No | Dom | Whole |
| FNBP1 | 3 | 3 | 9 | 132649466 | 132805473 | 19.2299064 | 4 | 0.76294643 | 1 | No | Dom | N/A |
| FOXO1 | 6 | 6 | 13 | 41129817 | 41240734 | 81.1417546 | 4 | 0.21430775 | 0.636295161 | No | Dom | N/A |
| FOXO3 | 12 | 10 | 6 | 108881038 | 109005977 | 112.054683 | 8 | 0.28247773 | 0.710511667 | No | Dom | Whole |
| FOXP1 | 25 | 21 | 3 | 71003844 | 71633140 | 54.0286288 | 22 | 0.61634869 | 0.917609012 | No | Dom | N/A |
| FSTL3 | 1 | 1 | 19 | 676389 | 683392 | 142.795945 | 0 | 0 | 0 | No | Dom | N/A |
| FUBP1 | 4 | 3 | 1 | 78400603 | 78444794 | 90.5161685 | 1 | 0.0801369 | 0.35456646 | No | Dom | N/A |
| FYN | 6 | 6 | 6 | 111981535 | 112194655 | 28.1531532 | 5 | 0.38403817 | 0.779273892 | No | Rec | Whole |

| Gene | | | | | | | | | | | | |
|---|---|---|---|---|---|---|---|---|---|---|---|---|
| GAB2 | 33 | 21 | 11 | 77926342 | 78128766 | 163.024147 | 25 | 0.82042283 | 1 | No | Dom | within 2 Mb |
| GAS7 | 11 | 9 | 17 | 9813926 | 10101868 | 41.6750596 | 3 | 0.00369514 | 0.031299968 | No | Dom | within 2 Mb |
| GATA2 | 1 | 1 | 3 | 128198265 | 128212028 | 72.6585773 | 0 | 0 | 0 | No | Dom | N/A |
| GATA3 | 4 | 2 | 10 | 8095567 | 8117161 | 231.5458 | 1 | 0.26424102 | 0.67645701 | Yes | Rec | N/A |
| GATA6 | 2 | 2 | 18 | 19749404 | 19782491 | 90.6700517 | 0 | 0 | 0 | No | Dom | N/A |
| GMPS | 4 | 3 | 3 | 155588325 | 155658457 | 71.2941311 | 1 | 0.0801369 | 0.35456646 | No | Dom | Whole |
| GNA11 | 1 | 1 | 19 | 3094408 | 3121452 | 36.9767786 | 2 | 0.86514834 | 1 | No | Dom | N/A |
| GNAQ | 9 | 7 | 9 | 80331003 | 80646374 | 31.708686 | 7 | 0.55122679 | 0.917609012 | No | Dom | N/A |
| GNAS | 12 | 8 | 20 | 57414750 | 57486247 | 181.825811 | 8 | 0.54804434 | 0.917609012 | No | Dom | N/A |
| GOLGA5 | 3 | 3 | 14 | 93260650 | 93306304 | 65.7116572 | 0 | 0 | 0 | No | Dom | N/A |
| GOPC | 42 | 19 | 6 | 117639374 | 117923691 | 151.239637 | 6 | 1.52E-05 | 0.000186315 | No | Dom | N/A |
| GPC3 | 10 | 7 | 23 | 132669773 | 133119922 | 31.1008133 | 12 | 0.95581368 | 1 | No | Dom/Rec | N/A |
| GPC5 | 62 | 25 | 13 | 92050929 | 93519490 | 46.3038308 | 38 | 0.99157956 | 1 | No | Dom | within 2 Mb |
| GPHN | 27 | 21 | 14 | 66974125 | 67648520 | 50.4155576 | 34 | 0.99446687 | 1 | No | Dom | Partial |
| GRB2 | 24 | 11 | 17 | 73314157 | 73401790 | 273.869433 | 6.5 | 0.06570059 | 0.315362842 | No | Dom | N/A |
| GRB7 | 21 | 14 | 17 | 37894187 | 37903545 | 2350.92969 | 9.358 | 0.09153703 | 0.356252239 | No | Dom | N/A |
| GRIN2A | 18 | 12 | 16 | 9047261 | 10276611 | 48.9111448 | 6 | 0.01948959 | 0.119425569 | No | Unknown | N/A |
| GRM3 | 8 | 8 | 7 | 86273224 | 86494200 | 45.2537832 | 7 | 0.4012842 | 0.786189453 | No | Dom/Rec | N/A |
| H3F3A | 1 | 1 | 1 | 226249552 | 226259702 | 98.5221675 | 2 | 0.86514834 | 1 | No | Dom | Whole |
| H3F3B | 1 | 1 | 17 | 73772517 | 73775860 | 299.132516 | 0.5 | 0.39360479 | 0.779273892 | No | Dom | N/A |
| HERPUD1 | 1 | 1 | 16 | 56965748 | 56977793 | 83.0220008 | 0.5 | 0.39360479 | 0.779273892 | No | Dom | N/A |
| HIP1 | 5 | 4 | 7 | 75162621 | 75368280 | 29.1745073 | 6 | 0.85023496 | 1 | No | Dom | Whole |
| HLF | 14 | 12 | 17 | 53342321 | 53402426 | 266.200815 | 10 | 0.30219147 | 0.74704844 | No | Dom | within 2 Mb |
| HMGA2 | 4 | 4 | 12 | 66218240 | 66360068 | 21.1523818 | 4 | 0.56723033 | 0.917609012 | No | Dom | N/A |
| HNRNPA2B1 | 8 | 7 | 7 | 26229547 | 26241149 | 861.920359 | 4.5 | 0.16817227 | 0.556708199 | No | Dom | N/A |
| HOOK3 | 25 | 18 | 8 | 42752033 | 42885671 | 217.004146 | 26 | 0.96228532 | 1 | No | Dom | N/A |

| Gene | | | | | | | | | | | | |
|---|---|---|---|---|---|---|---|---|---|---|---|---|
| HOXA11 | 1 | 1 | 7 | 27220776 | 27224835 | 246.3661 | 0 | 0 | 0 | No | Dom | N/A |
| HOXA9 | 3 | 3 | 7 | 27202057 | 27219880 | 168.321831 | 0 | 0 | 0 | No | Dom | N/A |
| HRAS | 1 | 1 | 11 | 532242 | 537287 | 198.216056 | 1 | 0.63244927 | 0.917609012 | No | Dom | N/A |
| HSP90AA1 | 4 | 4 | 14 | 102547073 | 102606086 | 67.7816752 | 4 | 0.56723033 | 0.917609012 | No | Dom | N/A |
| IDH1 | 1 | 1 | 2 | 209100951 | 209130798 | 33.5042048 | 0.5 | 0.39360479 | 0.779273892 | No | Dom | within 2 Mb |
| IDH2 | 1 | 1 | 15 | 90626277 | 90645736 | 51.3901023 | 0.5 | 0.39360479 | 0.779273892 | No | Dom | N/A |
| IGF1R | 44 | 18 | 15 | 99192200 | 99507759 | 158.448975 | 10 | 0.01348538 | 0.102204983 | No | Dom | N/A |
| IGF2R | 11 | 6 | 6 | 160390131 | 160534539 | 76.1730652 | 5 | 0.38403817 | 0.779273892 | No | Unknown | within 2 Mb |
| IKBKB | 18 | 12 | 8 | 42128829 | 42189965 | 327.139492 | 8.5 | 0.14970274 | 0.510229463 | No | Rec | N/A |
| IKZF1 | 2 | 2 | 7 | 50343720 | 50472799 | 15.4943872 | 3 | 0.80165331 | 1 | No | Dom/Rec | N/A |
| IKZF3 | 97 | 44 | 17 | 37913968 | 38020441 | 957.989349 | 106.473 | 1 | 1 | No | Dom? | N/A |
| IL21R | 2 | 2 | 16 | 27413686 | 27462115 | 41.2975696 | 2.5 | 0.71339092 | 0.987772049 | No | Dom | Whole |
| IL6ST | 1 | 1 | 5 | 55230923 | 55290772 | 0 | 2 | 0.86514834 | 1 | No | Dom | Whole |
| IL7R | 2 | 2 | 5 | 35852797 | 35879705 | 74.3273376 | 0 | 0 | 0 | No | Dom | Whole |
| IRS2 | 3 | 2 | 13 | 110406184 | 110438915 | 91.656228 | 0.5 | 0.09013623 | 0.35456646 | No | Dom | N/A |
| ITGB3 | 3 | 3 | 17 | 45331208 | 45390077 | 50.9606075 | 7 | 0.97106012 | 1 | No | Unknown | N/A |
| ITK | 3 | 2 | 5 | 156607054 | 156682195 | 40.3545823 | 3 | 0.80165331 | 1 | No | Dom | N/A |
| JAK1 | 12 | 10 | 1 | 65298912 | 65432187 | 112.54924 | 9 | 0.41258887 | 0.802875646 | No | Dom | N/A |
| JAK2 | 4 | 3 | 9 | 4985033 | 5128183 | 34.9283968 | 4 | 0.76294643 | 1 | No | Dom | N/A |
| JAK3 | 2 | 2 | 19 | 17935595 | 17958870 | 85.9291085 | 1.5 | 0.442399 | 0.819362781 | No | Dom | N/A |
| JAZF1 | 38 | 20 | 7 | 27870192 | 28220362 | 114.230231 | 14 | 0.07392746 | 0.34450596 | No | Dom | N/A |
| JUN | 2 | 2 | 1 | 59246465 | 59249785 | 301.204819 | 0.5 | 0.09013623 | 0.35456646 | No | Dom | Whole |
| KCNJ5 | 1 | 1 | 11 | 128761313 | 128787949 | 37.5431747 | 1 | 0.63244927 | 0.917609012 | No | Dom | N/A |
| KDM5A | 6 | 3 | 12 | 389223 | 498620 | 63.9871294 | 5 | 0.87647828 | 1 | No | Dom | N/A |
| KDM5C | 1 | 1 | 23 | 53221334 | 53254604 | 30.0571085 | 0.5 | 0.39360479 | 0.779273892 | Yes | Rec | N/A |
| KDM6A | 14 | 12 | 23 | 44732423 | 44971847 | 79.3571238 | 8 | 0.11036424 | 0.410127751 | Yes | Rec | N/A |

| | | | | | | | | | | | | |
|---|---|---|---|---|---|---|---|---|---|---|---|---|
| KDSR | 1 | 1 | 18 | 60994971 | 61034743 | 25.1433169 | 2 | 0.86514834 | 1 | No | Dom | Whole |
| KEAP1 | 4 | 3 | 19 | 10596796 | 10614054 | 231.776567 | 3 | 0.57741173 | 0.917609012 | No | Unknown | N/A |
| KIAA1549 | 10 | 9 | 7 | 138516126 | 138666064 | 66.6942336 | 3 | 0.00369514 | 0.031299968 | No | Dom | N/A |
| KIF5B | 5 | 5 | 10 | 32297938 | 32345359 | 105.438519 | 1.5 | 0.01841834 | 0.116925749 | No | Dom | N/A |
| KIT | 5 | 4 | 4 | 55524085 | 55606881 | 48.3115126 | 3 | 0.35276757 | 0.779273892 | Yes | Dom | Whole |
| KTN1 | 5 | 3 | 14 | 56047033 | 56151301 | 47.953351 | 4 | 0.76294643 | 1 | No | Dom | Whole |
| LASP1 | 17 | 12 | 17 | 37026256 | 37078023 | 386.346514 | 11.5 | 0.48081339 | 0.868177163 | No | Dom | N/A |
| LCK | 2 | 2 | 1 | 32716840 | 32751766 | 28.6319647 | 0.5 | 0.09013623 | 0.35456646 | No | Dom | N/A |
| LCP1 | 6 | 4 | 13 | 46700061 | 46786006 | 69.8120891 | 2.5 | 0.24218407 | 0.67645701 | No | Dom | N/A |
| LHFP | 2 | 2 | 13 | 39917029 | 40177665 | 15.3470741 | 4 | 0.90920738 | 1 | No | Dom | N/A |
| LIFR | 9 | 6 | 5 | 38475065 | 38608456 | 52.4773036 | 4 | 0.21430775 | 0.636295161 | No | Dom | Whole |
| LMO1 | 2 | 2 | 11 | 8245857 | 8290181 | 45.1222814 | 2 | 0.59447836 | 0.917609012 | No | Dom | N/A |
| LMO2 | 8 | 6 | 11 | 33880122 | 33913836 | 326.273951 | 1.5 | 0.00438996 | 0.036086592 | No | Dom | Whole |
| LPP | 62 | 36 | 3 | 187871072 | 188608460 | 107.134914 | 32 | 0.25681623 | 0.67645701 | No | Dom | Whole |
| LRIG3 | 7 | 2 | 12 | 59265937 | 59314262 | 165.545784 | 1.5 | 0.442399 | 0.819362781 | No | Dom | N/A |
| LRP1B | 59 | 47 | 2 | 140988992 | 142889270 | 47.8877301 | 42 | 0.23179154 | 0.67645701 | No | Rec | within 2 Mb |
| MAML2 | 31 | 23 | 11 | 95711440 | 96075059 | 96.2545962 | 15 | 0.03083542 | 0.101236727 | No | Dom | N/A |
| MAP2K1 | 3 | 3 | 15 | 66679155 | 66783882 | 28.6459079 | 3 | 0.57741173 | 0.917609012 | Yes | Dom | within 2 Mb |
| MAP2K2 | 1 | 1 | 19 | 4090319 | 4124126 | 29.5796728 | 2 | 0.86514834 | 1 | No | Dom | N/A |
| MAP2K4 | 19 | 16 | 17 | 11924141 | 12047140 | 162.602948 | 6 | 0.00046725 | 0.005078055 | Yes | Rec | Whole |
| MAP2K5 | 3 | 3 | 15 | 67835021 | 68099461 | 22.689457 | 4 | 0.76294643 | 1 | No | Unknown | N/A |
| MAP2K6 | 38 | 16 | 17 | 67410838 | 67538451 | 313.447689 | 19 | 0.78969451 | 1 | No | Unknown | N/A |
| MAP3K1 | 16 | 13 | 5 | 56111401 | 56191979 | 223.386036 | 1.5 | 6.99E-09 | 8.95E-08 | Yes | Rec | Whole |
| MAP3K11 | 3 | 3 | 11 | 65365226 | 65381720 | 181.884322 | 2 | 0.3233233 | 0.776778728 | No | Unknown | Whole |
| MAP3K12 | 3 | 3 | 12 | 53874280 | 53893271 | 157.969565 | 1.5 | 0.19098454 | 0.591436001 | No | Unknown | within 2 Mb |
| MAP3K13 | 7 | 5 | 3 | 185000729 | 185206885 | 33.9548691 | 8 | 0.90200775 | 1 | Yes | Rec | Whole |

| | | | | | | | | | | | | |
|---|---|---|---|---|---|---|---|---|---|---|---|---|
| MAP3K14 | 1 | 1 | 17 | 43340488 | 43394414 | 18.5439306 | 2 | 0.86514834 | 1 | No | Unknown | N/A |
| MAP3K2 | 1 | 1 | 2 | 128056306 | 128146041 | 11.1439238 | 1 | 0.63244927 | 0.917609012 | No | Unknown | N/A |
| MAP3K3 | 13 | 6 | 17 | 61699801 | 61773667 | 189.532397 | 11 | 0.9637977 | 1 | No | Unknown | N/A |
| MAP3K4 | 3 | 3 | 6 | 161412822 | 161551917 | 28.7573241 | 5 | 0.87647828 | 1 | No | Unknown | Whole |
| MAP3K5 | 22 | 14 | 6 | 136878185 | 137113656 | 135.897839 | 14 | 0.5368966 | 0.917609012 | No | Dom | N/A |
| MAP3K7 | 5 | 5 | 6 | 91223292 | 91296764 | 81.6637631 | 3 | 0.18428445 | 0.589710244 | No | Unknown | Whole |
| MAP3K8 | 1 | 1 | 10 | 30722866 | 30750762 | 35.8474333 | 0.5 | 0.39360479 | 0.779273892 | No | Unknown | N/A |
| MAP3K9 | 5 | 4 | 14 | 71194852 | 71275888 | 74.0411669 | 2 | 0.14255325 | 0.488754016 | No | Unknown | within 2 Mb |
| MAP4K3 | 4 | 3 | 2 | 39476407 | 39664453 | 31.9070866 | 4 | 0.76294643 | 1 | No | Unknown | N/A |
| MAP4K4 | 7 | 4 | 2 | 102313312 | 102508449 | 40.9968381 | 7 | 0.91953964 | 1 | No | Unknown | within 2 Mb |
| MAPK1 | 4 | 4 | 22 | 22108789 | 22221970 | 53.0124314 | 5 | 0.73623267 | 1 | No | Unknown | N/A |
| MAPK10 | 18 | 11 | 4 | 86936276 | 87515284 | 32.8147452 | 8 | 0.18268083 | 0.589710244 | No | Unknown | within 2 Mb |
| MAPK8 | 2 | 2 | 10 | 49514698 | 49647403 | 15.0710222 | 2 | 0.59447836 | 0.917609012 | No | Unknown | Whole |
| MAPK9 | 5 | 3 | 5 | 179660594 | 179719071 | 85.5037023 | 2.5 | 0.45647374 | 0.8320534 | No | Unknown | Whole |
| MAX | 3 | 3 | 14 | 65472892 | 65569227 | 20.7608865 | 3 | 0.57741173 | 0.917609012 | No | Unknown | Whole |
| MCL1 | 5 | 4 | 1 | 150547032 | 150552066 | 993.245928 | 1.5 | 0.06538985 | 0.315362842 | No | Dom | Whole |
| MDM2 | 4 | 4 | 12 | 69201956 | 69239214 | 161.03924 | 2 | 0.14255325 | 0.488754016 | No | Dom | N/A |
| MDM4 | 13 | 9 | 1 | 204485511 | 204596130 | 99.4404216 | 11.0619 | 0.77612987 | 1 | No | Dom | N/A |
| MDS2 | 6 | 5 | 1 | 23907985 | 23967058 | 135.42566 | 1.5 | 0.01841834 | 0.116925749 | No | Dom | Whole |
| MECOM | 18 | 16 | 3 | 168801287 | 169381406 | 44.8183907 | 18 | 0.71720586 | 0.988302814 | No | Dom | N/A |
| MED12 | 1 | 1 | 23 | 70338406 | 70362303 | 41.8462569 | 0.5 | 0.39360479 | 0.779273892 | No | Dom | N/A |
| MED23 | 2 | 2 | 6 | 131895106 | 131949369 | 36.8575272 | 2 | 0.59447836 | 0.917609012 | Yes | Rec | N/A |
| MEN1 | 1 | 1 | 11 | 64570988 | 64578766 | 257.13551 | 0 | 0 | 0 | No | Rec | Whole |
| MET | 4 | 3 | 7 | 116312248 | 116438440 | 31.6977304 | 7 | 0.97106012 | 1 | Yes | Dom | Whole |
| MITF | 4 | 3 | 3 | 69788586 | 70017487 | 17.4748035 | 5 | 0.87647828 | 1 | No | Dom | N/A |
| MKL1 | 7 | 6 | 22 | 40806285 | 41032706 | 44.1655147 | 12 | 0.98060766 | 1 | No | Dom | Whole |

| MLF1 | 1 | 1 | 3 | 158288952 | 158325041 | 27.7092743 | 1 | 0.63244927 | 0.917609012 | No | Dom | Whole |
|---|---|---|---|---|---|---|---|---|---|---|---|---|
| MLH1 | 2 | 2 | 3 | 37034823 | 37092409 | 52.0959956 | 0.5 | 0.09013623 | 0.35456646 | No | Rec | N/A |
| MLL | 4 | 4 | 11 | 118307205 | 118395936 | 67.6201102 | 2 | 0.14255325 | 0.488754016 | Yes | Dom | Whole |
| MLLT10 | 16 | 11 | 10 | 21823094 | 22032559 | 100.255413 | 11 | 0.54129705 | 0.917609012 | No | Dom | N/A |
| MLLT11 | 1 | 1 | 1 | 151030234 | 151040970 | 93.1445604 | 0.5 | 0.39360479 | 0.779273892 | No | Dom | Whole |
| MLLT3 | 11 | 9 | 9 | 20341663 | 20622542 | 42.7230231 | 12 | 0.84780225 | 1 | No | Dom | Whole |
| MLLT4 | 18 | 16 | 6 | 168227602 | 168372703 | 117.159771 | 5 | 6.22E-05 | 0.000746855 | Yes | Rec | N/A |
| MLLT6 | 16 | 12 | 17 | 36861795 | 36886056 | 659.494662 | 6.5 | 0.03301792 | 0.183444415 | No | Dom | N/A |
| MN1 | 1 | 1 | 22 | 28144265 | 28197486 | 18.7895755 | 1.5 | 0.77731844 | 1 | No | Dom | Whole |
| MPL | 1 | 1 | 1 | 43803475 | 43818443 | 0 | 1 | 0.63244927 | 0.917609012 | No | Dom | N/A |
| MRAS | 1 | 1 | 3 | 138066539 | 138124375 | 17.290269 | 3.5 | 0.97013247 | 1 | No | Unknown | N/A |
| MRE11A | 1 | 1 | 11 | 94150475 | 94227040 | 13.060798 | 3.5 | 0.97013247 | 1 | No | Rec | N/A |
| MSH2 | 7 | 7 | 2 | 47630108 | 47789450 | 56.4822834 | 6 | 0.39369566 | 0.779273892 | No | Rec | N/A |
| MSH6 | 2 | 2 | 2 | 48010221 | 48034092 | 125.675506 | 2 | 0.59447836 | 0.917609012 | No | Rec | N/A |
| MSI2 | 73 | 37 | 17 | 55333931 | 55757299 | 174.788836 | 64 | 0.99995539 | 1 | No | Dom | Partial |
| MSN | 1 | 1 | 23 | 64887537 | 64961792 | 13.4671066 | 2 | 0.86514834 | 1 | No | Dom | N/A |
| MTCP1 | 6 | 4 | 23 | 154209897 | 154376212 | 81.0983027 | 0 | 0 | 0 | No | Dom | N/A |
| MTDH | 10 | 8 | 8 | 98656407 | 98742488 | 116.169654 | 3 | 0.0116735 | 0.089652499 | No | Dom | Whole |
| MTOR | 4 | 3 | 1 | 11166592 | 11322564 | 25.6456287 | 5 | 0.87647828 | 1 | No | Unknown | Whole |
| MUC1 | 2 | 2 | 1 | 155158300 | 155162707 | 453.823463 | 0.5 | 0.09013623 | 0.35456646 | No | Dom | within 2 Mb |
| MYB | 6 | 5 | 6 | 135502453 | 135540311 | 184.901474 | 5.5 | 0.64363595 | 0.931493229 | No | Dom | N/A |
| MYC | 5 | 5 | 8 | 128747680 | 128753674 | 834.167501 | 2 | 0.05233021 | 0.274020034 | No | Dom | within 2 Mb |
| MYCL1 | 2 | 2 | 1 | 40361098 | 40367925 | 292.954446 | 0.5 | 0.09013623 | 0.35456646 | No | Dom | N/A |
| MYD88 | 1 | 1 | 3 | 38179969 | 38184513 | 220.070423 | 0 | 0 | 0 | No | Dom | N/A |
| MYH11 | 5 | 3 | 16 | 15796992 | 15950890 | 38.9868614 | 3 | 0.57741173 | 0.917609012 | No | Dom | Whole |
| MYH9 | 14 | 14 | 22 | 36677327 | 36784063 | 149.902563 | 8 | 0.03312191 | 0.183444415 | No | Dom | Whole |

| MYST3 | 25 | 16 | 8 | 41786997 | 41909508 | 212.225841 | 11 | 0.09048832 | 0.35456646 | No | Dom | N/A |
|---|---|---|---|---|---|---|---|---|---|---|---|---|
| MYST4 | 4 | 4 | 10 | 76585340 | 76792380 | 19.3199382 | 6 | 0.85023496 | 1 | No | Dom | within 2 Mb |
| NACA | 2 | 1 | 12 | 57094565 | 57119326 | 80.7721821 | 1 | 0.63244927 | 0.917609012 | No | Dom | N/A |
| NBN | 6 | 2 | 8 | 90945564 | 90996899 | 97.3994351 | 5.5 | 0.97393149 | 1 | No | Unknown | N/A |
| NCKIPSD | 1 | 1 | 3 | 48701364 | 48723797 | 44.5771854 | 0.5 | 0.39360479 | 0.779273892 | No | Dom | N/A |
| NCOA1 | 5 | 5 | 2 | 24714783 | 24993571 | 17.9347748 | 13 | 0.99653512 | 1 | No | Dom | N/A |
| NCOA2 | 18 | 11 | 8 | 71015826 | 71316020 | 66.6235834 | 10 | 0.41695686 | 0.808643617 | No | Dom | Whole |
| NCOA3 | 28 | 10 | 20 | 46130646 | 46285616 | 193.585855 | 16 | 0.95882844 | 1 | No | Dom | N/A |
| NCOA4 | 2 | 2 | 10 | 51565108 | 51590734 | 78.0457348 | 0.5 | 0.09013623 | 0.35456646 | No | Dom | Whole |
| NCOR1 | 29 | 17 | 17 | 15934718 | 16119010 | 173.637488 | 14 | 0.24187497 | 0.67645701 | Yes | Rec | within 2 Mb |
| NCOR2 | 14 | 9 | 12 | 124808961 | 125052010 | 65.830347 | 9 | 0.54541588 | 0.917609012 | No | Unknown | Whole |
| NDRG1 | 12 | 6 | 8 | 134249414 | 134309547 | 199.557647 | 4.5 | 0.29672329 | 0.736692299 | No | Dom | N/A |
| NDST4 | 4 | 4 | 4 | 115748919 | 116035032 | 20.9707353 | 4 | 0.56723033 | 0.917609012 | No | Unknown | N/A |
| NDUFA13 | 3 | 3 | 19 | 19626540 | 19646885 | 147.456377 | 3.5 | 0.68002246 | 0.962390512 | No | Rec | N/A |
| NF1 | 59 | 34 | 17 | 29421945 | 29705949 | 242.954325 | 23 | 0.01664724 | 0.115527816 | Yes | Rec | N/A |
| NF2 | 6 | 6 | 22 | 29999545 | 30094587 | 63.1299846 | 2 | 0.01637035 | 0.114991732 | No | Rec | Whole |
| NFE2L2 | 6 | 5 | 2 | 178092323 | 178257425 | 42.3980327 | 4 | 0.37116223 | 0.779273892 | Yes | Dom | Whole |
| NFIB | 18 | 15 | 9 | 14081847 | 14314581 | 133.199275 | 13 | 0.3236578 | 0.776778728 | No | Dom | N/A |
| NGFR | 2 | 2 | 17 | 47572655 | 47592379 | 101.39931 | 1.5 | 0.442399 | 0.819362781 | No | Dom | N/A |
| NIN | 11 | 7 | 14 | 51186481 | 51297848 | 98.7725269 | 4 | 0.10992616 | 0.410127751 | No | Dom | N/A |
| NONO | 1 | 1 | 23 | 70503433 | 70521018 | 56.8666477 | 0.5 | 0.39360479 | 0.779273892 | No | Dom | N/A |
| NOTCH1 | 7 | 5 | 9 | 139388896 | 139440314 | 136.139095 | 3 | 0.18428445 | 0.589710244 | Yes | Dom | N/A |
| NOTCH2 | 12 | 11 | 1 | 120454176 | 120612274 | 94.8778606 | 12 | 0.65504561 | 0.945629758 | No | Dom | N/A |
| NOTCH3 | 5 | 5 | 19 | 15270445 | 15311792 | 120.927758 | 0 | 0 | 0 | No | Dom | N/A |
| NPM1 | 3 | 2 | 5 | 170814708 | 170838141 | 128.024581 | 1 | 0.26424102 | 0.67645701 | No | Dom | Whole |
| NR4A3 | 1 | 1 | 9 | 102584137 | 102629173 | 44.4089173 | 0.5 | 0.39360479 | 0.779273892 | No | Dom | N/A |

| NRAS | 1 | 1 | 1 | 115247090 | 115259515 | 80.4828974 | 1 | 0.63244927 | 0.917609012 | Yes | Dom | N/A |
|---|---|---|---|---|---|---|---|---|---|---|---|---|
| NRIP1 | 9 | 8 | 21 | 16333556 | 16437321 | 77.0972871 | 2 | 0.00106626 | 0.010068249 | No | Unknown | N/A |
| NSD1 | 9 | 7 | 5 | 176560026 | 176727216 | 53.8309708 | 0 | 0 | 0 | No | Dom | Whole |
| NT5C2 | 6 | 5 | 10 | 104845940 | 104953056 | 56.0140409 | 4 | 0.37116223 | 0.779273892 | No | Unknown | N/A |
| NTRK1 | 3 | 3 | 1 | 156785448 | 156851642 | 45.3213282 | 6.6194 | 0.96140016 | 1 | No | Dom | N/A |
| NTRK2 | 10 | 10 | 9 | 87283466 | 87638505 | 39.4322877 | 4 | 0.00789705 | 0.062310974 | No | Unknown | within 2 Mb |
| NTRK3 | 10 | 7 | 15 | 88402982 | 88799978 | 30.2270048 | 9 | 0.79543057 | 1 | No | Dom | N/A |
| NUMA1 | 26 | 14 | 11 | 71713911 | 71791573 | 334.784064 | 12 | 0.31731062 | 0.776778728 | No | Dom | Whole |
| NUP214 | 2 | 2 | 9 | 134000976 | 134110057 | 27.5024981 | 2 | 0.59447836 | 0.917609012 | No | Dom | N/A |
| NUP98 | 6 | 5 | 11 | 3696241 | 3819018 | 48.8690879 | 1 | 0.00361886 | 0.031299968 | No | Dom | N/A |
| PAK1 | 46 | 22 | 11 | 77033061 | 77185108 | 309.114945 | 26 | 0.81566441 | 1 | No | Dom | within 2 Mb |
| PAK3 | 12 | 4 | 23 | 110187513 | 110464159 | 50.6061899 | 8 | 0.95864081 | 1 | No | Unknown | N/A |
| PATZ1 | 1 | 1 | 22 | 31721790 | 31742218 | 48.9524182 | 1 | 0.63244927 | 0.917609012 | No | Dom | within 2 Mb |
| PAX5 | 5 | 2 | 9 | 36833272 | 37034476 | 29.8204807 | 5 | 0.96017324 | 1 | No | Dom | N/A |
| PAX7 | 5 | 4 | 1 | 18957500 | 19075360 | 42.423214 | 1 | 0.01887864 | 0.116925749 | No | Dom | Whole |
| PAX8 | 1 | 1 | 2 | 113973574 | 114036498 | 15.8921874 | 1 | 0.63244927 | 0.917609012 | No | Dom | within 2 Mb |
| PAX9 | 1 | 1 | 14 | 37126782 | 37147007 | 49.4437577 | 0.5 | 0.39360479 | 0.779273892 | No | Dom | N/A |
| PBRM1 | 14 | 9 | 3 | 52579368 | 52719933 | 120.94049 | 8 | 0.40745025 | 0.795563878 | Yes | Rec | N/A |
| PBX1 | 16 | 15 | 1 | 164524821 | 164821067 | 74.2626061 | 14 | 0.42955763 | 0.819362781 | No | Dom | N/A |
| PCM1 | 2 | 2 | 8 | 17780366 | 17887453 | 18.6764033 | 1 | 0.98304553 | 1 | No | Dom | N/A |
| PCSK7 | 1 | 1 | 11 | 117075810 | 117102811 | 37.0356653 | 0.5 | 0.39360479 | 0.779273892 | No | Dom | Whole |
| PDE4DIP | 25 | 19 | 1 | 144833168 | 145076186 | 111.102881 | 8 | 0.00058509 | 0.006018047 | No | Dom | Whole |
| PDGFB | 1 | 1 | 22 | 39619364 | 39640756 | 46.7464473 | 1 | 0.63244927 | 0.917609012 | No | Dom | Whole |
| PDGFRA | 2 | 2 | 4 | 55095264 | 55164414 | 28.922632 | 2.5 | 0.71339092 | 0.987772049 | No | Dom | Whole |
| PHF6 | 3 | 3 | 23 | 133507283 | 133562820 | 54.018042 | 0.5 | 0.01433689 | 0.104532288 | No | Rec | N/A |
| PHGDH | 12 | 7 | 1 | 120202421 | 120286838 | 201.381238 | 0 | 0 | 0 | No | Dom | N/A |

| PICALM | 9 | 6 | 11 | 85668486 | 85780144 | 89.5591897 | 5 | 0.38403817 | 0.779273892 | No | Dom | Whole |
|---|---|---|---|---|---|---|---|---|---|---|---|---|
| PIGA | 2 | 1 | 23 | 15337578 | 15353660 | 124.362641 | 1 | 0.63244927 | 0.917609012 | No | Unknown | N/A |
| PIK3C2A | 7 | 7 | 11 | 17108129 | 17191354 | 84.1093421 | 2.5 | 0.0139701 | 0.103163824 | No | Unknown | Whole |
| PIK3C2B | 10 | 8 | 1 | 204391756 | 204463852 | 166.44474 | 0 | 0 | 0 | No | Unknown | N/A |
| PIK3C2G | 17 | 11 | 12 | 18414474 | 18801348 | 49.1115971 | 9 | 0.29304457 | 0.73070854 | No | Unknown | N/A |
| PIK3C3 | 4 | 4 | 18 | 39535171 | 39667794 | 37.7008513 | 2 | 0.14255325 | 0.488754016 | No | Unknown | N/A |
| PIK3CA | 2 | 2 | 3 | 178865902 | 178957881 | 32.6161406 | 9.1979 | 0.99902896 | 1 | Yes | Dom | N/A |
| PIK3CB | 8 | 7 | 3 | 138372860 | 138553780 | 49.745744 | 2 | 0.00444817 | 0.036086592 | No | Unknown | N/A |
| PIK3CG | 2 | 2 | 7 | 106505723 | 106547590 | 47.7703203 | 1.5 | 0.442399 | 0.819362781 | No | Unknown | Whole |
| PIK3R1 | 5 | 4 | 5 | 67522462 | 67597649 | 93.101201 | 1 | 0.01887864 | 0.116925749 | Yes | Rec | N/A |
| PIK3R2 | 2 | 2 | 19 | 18263928 | 18281343 | 114.843526 | 1 | 0.26424102 | 0.67645701 | No | Unknown | N/A |
| PIM1 | 1 | 1 | 6 | 37137922 | 37143202 | 189.393939 | 0.5 | 0.39360479 | 0.779273892 | No | Dom | N/A |
| PLAG1 | 4 | 4 | 8 | 57073468 | 57123859 | 79.3792542 | 2.5 | 0.24218407 | 0.67645701 | No | Dom | N/A |
| PLCG1 | 3 | 3 | 20 | 39765600 | 39804361 | 103.196512 | 0 | 0 | 0 | No | Dom? | N/A |
| PLEKHG5 | 2 | 2 | 1 | 6526152 | 6580121 | 55.5874669 | 1.5 | 0.442399 | 0.819362781 | No | Rec | Whole |
| PMS1 | 6 | 6 | 2 | 190648811 | 190742355 | 85.521252 | 2.5 | 0.0416477 | 0.224196951 | No | Rec | within 2 Mb |
| POT1 | 4 | 3 | 7 | 124462440 | 124570037 | 37.1757577 | 3 | 0.57741173 | 0.917609012 | No | Unknown | N/A |
| POU1F1 | 4 | 2 | 3 | 87307379 | 87325737 | 217.888659 | 1 | 0.26424102 | 0.67645701 | No | Dom | N/A |
| POU2AF1 | 1 | 1 | 11 | 111222980 | 111250157 | 36.79582 | 0 | 0 | 0 | No | Dom | N/A |
| PPARG | 7 | 3 | 3 | 12328867 | 12475855 | 54.4262117 | 5 | 0.87647828 | 1 | No | Dom | N/A |
| PPM1D | 33 | 18 | 17 | 58677554 | 58742036 | 511.770727 | 13.5 | 0.13641018 | 0.488754016 | Yes | Dom | N/A |
| PPP2R1A | 1 | 1 | 19 | 52693274 | 52730687 | 26.7286772 | 0.5 | 0.39360479 | 0.779273892 | Yes | Rec? | N/A |
| PPP6C | 3 | 2 | 9 | 127908852 | 127952218 | 69.1786192 | 0 | 0 | 0 | No | Dom/Rec | N/A |
| PRCC | 2 | 2 | 1 | 156720402 | 156770609 | 39.8350828 | 5.0207 | 0.96086049 | 1 | No | Dom? | N/A |
| PRDM16 | 2 | 2 | 1 | 2985732 | 3355185 | 5.41340847 | 6 | 0.98304553 | 1 | No | Dom | Whole |
| PREX2 | 19 | 13 | 8 | 68864353 | 69143897 | 71.5450877 | 10 | 0.20672753 | 0.633377956 | No | Unknown | Whole |

| PRKAR1A | 3 | 3 | 17 | 66508110 | 66528908 | 144.244639 | 1.5 | 0.19098454 | 0.591436001 | No | Dom/Rec | N/A |
|---|---|---|---|---|---|---|---|---|---|---|---|---|
| PRKCG | 3 | 2 | 19 | 54382444 | 54410906 | 105.403696 | 0.5 | 0.09013623 | 0.35456646 | No | Dom | Whole |
| PRKCI | 2 | 2 | 3 | 169940153 | 170023769 | 35.8783008 | 3 | 0.80165331 | 1 | No | Dom | N/A |
| PRRX1 | 3 | 3 | 1 | 170632323 | 170708560 | 65.5849522 | 2.5 | 0.45647374 | 0.8320534 | No | Dom | within 2 Mb |
| PSIP1 | 2 | 2 | 9 | 15464064 | 15511017 | 85.1915746 | 1 | 0.26424102 | 0.67645701 | No | Rec | within 2 Mb |
| PTEN | 50 | 43 | 10 | 89622870 | 89731687 | 689.230543 | 23 | 8.56E-05 | 0.000966521 | Yes | Rec | Whole |
| PTK6 | 2 | 2 | 20 | 62159778 | 62168695 | 224.290681 | 0.5 | 0.09013623 | 0.35456646 | No | Dom | N/A |
| PTP4A1 | 9 | 5 | 6 | 64231666 | 64293492 | 129.3954 | 4 | 0.37116223 | 0.779273892 | No | Dom | N/A |
| PTPN11 | 1 | 1 | 12 | 112856713 | 112947717 | 10.988528 | 2.5 | 0.91837315 | 1 | No | Dom | Whole |
| PTPN13 | 9 | 6 | 4 | 87515468 | 87736324 | 54.3340457 | 4 | 0.21430775 | 0.636295161 | No | Rec | within 2 Mb |
| PTPRB | 25 | 8 | 12 | 70910630 | 71031210 | 190.744734 | 8 | 0.54804434 | 0.917609012 | No | Unknown | N/A |
| PTPRC | 5 | 5 | 1 | 198607801 | 198726545 | 42.1073907 | 7 | 0.82872664 | 1 | No | Unknown | within 2 Mb |
| PTPRD | 74 | 44 | 9 | 8314246 | 10612723 | 44.8122822 | 40 | 0.27761124 | 0.704423246 | No | Unknown | N/A |
| PTPRJ | 4 | 4 | 11 | 48002110 | 48192393 | 31.5319813 | 9 | 0.97949275 | 1 | No | Unknown | N/A |
| PTPRK | 43 | 19 | 6 | 128289924 | 128841870 | 88.7768006 | 24 | 0.87708869 | 1 | No | Rec | N/A |
| PTPRM | 40 | 24 | 18 | 7567817 | 8406859 | 57.2081016 | 24 | 0.52893368 | 0.917609012 | No | Unknown | N/A |
| PTPRS | 9 | 8 | 19 | 5205519 | 5340814 | 81.3038176 | 6 | 0.25527537 | 0.67645701 | No | Unknown | N/A |
| PTPRT | 58 | 35 | 20 | 40701392 | 41818610 | 63.5507126 | 48 | 0.98276703 | 1 | No | Unknown | N/A |
| RAB23 | 5 | 3 | 6 | 57053581 | 57087078 | 149.267099 | 4.5 | 0.82755471 | 1 | No | Dom | N/A |
| RAB25 | 1 | 1 | 1 | 156030951 | 156040295 | 107.020548 | 0 | 0 | 0 | No | Dom | N/A |
| RABEP1 | 4 | 4 | 17 | 5185558 | 5289129 | 38.6208495 | 3 | 0.35276757 | 0.779273892 | No | Dom | N/A |
| RAD21 | 13 | 9 | 8 | 117858174 | 117887105 | 483.909993 | 2 | 0.00022835 | 0.002529438 | Yes | Rec? | Whole |
| RAD50 | 1 | 1 | 5 | 131891711 | 131979752 | 22.7166888 | 1 | 0.63244927 | 0.917609012 | No | Unknown | Whole |
| RAD51 | 1 | 1 | 15 | 40987358 | 41024356 | 27.028488 | 0 | 0 | 0 | No | Dom? | N/A |
| RAD51L1 | 61 | 43 | 14 | 68286496 | 69196935 | 86.7713268 | 32 | 0.0321248 | 0.183206772 | No | Dom | Whole |
| RAF1 | 5 | 4 | 3 | 12625100 | 12705725 | 62.0155039 | 4 | 0.56723033 | 0.917609012 | No | Dom | N/A |

| RALGDS | 4 | 4 | 9 | 135973107 | 136024721 | 77.4983532 | 1.5 | 0.06538985 | 0.315362842 | No | Dom | N/A |
|---|---|---|---|---|---|---|---|---|---|---|---|---|
| RANBP17 | 15 | 12 | 5 | 170288874 | 170725862 | 41.1910625 | 0 | 0 | 0 | No | Dom | Whole |
| RAP1GDS1 | 5 | 5 | 4 | 99182535 | 99365012 | 49.3212843 | 4 | 0.37116223 | 0.779273892 | No | Dom | within 2 Mb |
| RARA | 40 | 17 | 17 | 38465436 | 38513895 | 887.348067 | 33.9213 | 0.9996387 | 1 | No | Dom | N/A |
| RB1 | 34 | 30 | 13 | 48877911 | 49056122 | 286.177621 | 4 | 5.03E-17 | 6.58E-16 | Yes | Rec | N/A |
| REG4 | 3 | 3 | 1 | 120336641 | 120354283 | 170.048747 | 2.5 | 0.45647374 | 0.8320534 | No | Dom | N/A |
| REL | 3 | 3 | 2 | 61108656 | 61150645 | 119.078806 | 1.5 | 0.19098454 | 0.591436001 | No | Dom | N/A |
| RET | 1 | 1 | 10 | 43572475 | 43625799 | 18.7532818 | 3.5 | 0.97013247 | 1 | No | Dom | Whole |
| RHOA | 1 | 1 | 3 | 49396578 | 49450431 | 18.5690676 | 1 | 0.63244927 | 0.917609012 | No | Unknown | N/A |
| RICTOR | 4 | 4 | 5 | 38938021 | 39074510 | 51.2861842 | 7 | 0.91953964 | 1 | No | Unknown | Whole |
| RNF213 | 16 | 6 | 17 | 78234665 | 78370085 | 132.919805 | 11 | 0.9637977 | 1 | No | Unknown | N/A |
| RNF43 | 12 | 10 | 17 | 56429861 | 56494480 | 216.654544 | 7.5 | 0.22243003 | 0.657024101 | No | Rec | within 2 Mb |
| ROBO1 | 37 | 30 | 3 | 78646390 | 79816965 | 37.5883647 | 28 | 0.37548837 | 0.779273892 | No | Unknown | N/A |
| ROBO2 | 58 | 32 | 3 | 75955826 | 77699115 | 40.1539848 | 24 | 0.06349902 | 0.315362842 | No | Unknown | N/A |
| ROS1 | 23 | 10 | 6 | 117609463 | 117747018 | 152.666206 | 6 | 0.08281174 | 0.35456646 | No | Dom | N/A |
| RPL22 | 1 | 1 | 1 | 6241329 | 6269449 | 35.5618777 | 0.5 | 0.39360479 | 0.779273892 | No | Dom | Whole |
| RPN1 | 3 | 3 | 3 | 128338817 | 128399918 | 65.465377 | 2 | 0.3233233 | 0.776778728 | No | Dom | N/A |
| RPS6KB1 | 29 | 16 | 17 | 57970443 | 58027925 | 504.505758 | 13.5 | 0.28046286 | 0.70853775 | No | Dom | N/A |
| RPTOR | 26 | 20 | 17 | 78518625 | 78940173 | 66.4218547 | 18 | 0.34761562 | 0.779273892 | No | Unknown | N/A |
| RRM2B | 6 | 6 | 8 | 103216732 | 103251346 | 173.340267 | 6.9228 | 0.69088502 | 0.97298233 | No | Dom | Whole |
| RSPO2 | 26 | 19 | 8 | 108911544 | 109095913 | 151.869349 | 16 | 0.25522438 | 0.67645701 | No | Unknown | N/A |
| RSPO3 | 1 | 1 | 6 | 127439749 | 127518910 | 12.6324832 | 5 | 0.99341167 | 1 | No | Unknown | N/A |
| RUNX1 | 125 | 53 | 21 | 36160098 | 37357047 | 129.49591 | 62 | 0.90181363 | 1 | Yes | Dom | N/A |
| RUNX1T1 | 25 | 9 | 8 | 92971152 | 93107443 | 190.768283 | 5 | 0.06721594 | 0.319970116 | No | Dom | within 2 Mb |
| SDC4 | 2 | 2 | 20 | 43953928 | 43977064 | 86.4453665 | 2.5 | 0.71339092 | 0.987772049 | No | Dom | N/A |
| SDHB | 4 | 4 | 1 | 17345217 | 17380665 | 141.051681 | 1 | 0.01887864 | 0.116925749 | No | Rec | Whole |

| | | | | | | | | | | | | |
|---|---|---|---|---|---|---|---|---|---|---|---|---|
| SDHC | 4 | 4 | 1 | 161284047 | 161332984 | 102.172181 | 3 | 0.35276757 | 0.779273892 | No | Rec | N/A |
| 05-Sep | 4 | 3 | 22 | 19701987 | 19712295 | 485.060147 | 1 | 0.0801369 | 0.35456646 | No | Dom | N/A |
| 06-Sep | 2 | 2 | 23 | 118749687 | 118829533 | 37.5723267 | 1.5 | 0.442399 | 0.819362781 | No | Dom | N/A |
| 09-Sep | 24 | 16 | 17 | 75277492 | 75496674 | 127.747717 | 11 | 0.09048832 | 0.35456646 | No | Dom | N/A |
| SETBP1 | 15 | 12 | 18 | 42260138 | 42648475 | 56.6518256 | 3 | 6.68E-05 | 0.000784760 | No | | N/A |
| SETD2 | 7 | 6 | 3 | 47057919 | 47205457 | 74.5570633 | 3 | 0.08337607 | 0.35456646 | Yes | Rec | N/A |
| SF3B1 | 1 | 1 | 2 | 198256698 | 198299815 | 23.1927082 | 2.5 | 0.91837315 | 1 | Yes | Dom | Whole |
| SFPQ | 1 | 1 | 1 | 35641979 | 35658749 | 59.6302922 | 3 | 0.95061283 | 1 | No | Dom | N/A |
| SFRS3 | 1 | 1 | 6 | 36562090 | 36571209 | 109.661147 | 0.5 | 0.39360479 | 0.779273892 | No | Dom | N/A |
| SH2B3 | 3 | 2 | 12 | 111843752 | 111889426 | 65.682883 | 0 | 0 | 0 | No | Rec? | Whole |
| SH3GL1 | 3 | 3 | 19 | 4360370 | 4400496 | 74.7644919 | 3.5 | 0.68002246 | 0.962390512 | No | Dom | N/A |
| SHC1 | 2 | 2 | 1 | 154934774 | 154946959 | 164.136233 | 1 | 0.26424102 | 0.67645701 | No | Dom | within 2 Mb |
| SKP2 | 2 | 2 | 5 | 36152091 | 36184421 | 92.7930715 | 1 | 0.26424102 | 0.67645701 | No | Dom | Whole |
| SLC45A3 | 2 | 2 | 1 | 205626979 | 205649587 | 88.4642604 | 2 | 0.59447836 | 0.917609012 | No | Dom | N/A |
| SLIT2 | 6 | 6 | 4 | 20254883 | 20622184 | 21.7805016 | 8 | 0.81073824 | 1 | No | Unknown | Whole |
| SMAD2 | 4 | 3 | 18 | 45357922 | 45457515 | 70.2860643 | 3 | 0.57741173 | 0.917609012 | No | Unknown | N/A |
| SMAD3 | 10 | 8 | 15 | 67358183 | 67487533 | 85.0405876 | 7 | 0.4012842 | 0.786189453 | No | Unknown | N/A |
| SMAD4 | 4 | 4 | 18 | 48556583 | 48611415 | 91.1876277 | 1 | 0.01887864 | 0.116925749 | Yes | Rec | N/A |
| SMARCA4 | 10 | 8 | 19 | 11071598 | 11172958 | 108.524073 | 9 | 0.67800225 | 0.962390512 | Yes | Rec | N/A |
| SMARCB1 | 1 | 1 | 22 | 24129150 | 24176703 | 0 | 1.5 | 0.77731844 | 1 | No | Rec | N/A |
| SMARCD1 | 1 | 1 | 12 | 50478983 | 50494495 | 64.4662197 | 0.5 | 0.39360479 | 0.779273892 | No | Dom | Whole |
| SMARCE1 | 8 | 8 | 17 | 38781214 | 38821393 | 223.997611 | 7.5 | 0.47585475 | 0.861925588 | No | Unknown | N/A |
| SMC1A | 2 | 2 | 23 | 53401070 | 53449677 | 41.1463369 | 3.5 | 0.86493865 | 1 | No | Unknown | N/A |
| SMC3 | 1 | 1 | 10 | 112327449 | 112364394 | 27.0672621 | 0.5 | 0.39360479 | 0.779273892 | No | Unknown | Whole |
| SMO | 1 | 1 | 7 | 128828713 | 128853386 | 40.5301342 | 0 | 0 | 0 | No | Dom | within 2 Mb |
| SMURF1 | 5 | 4 | 7 | 98625061 | 98741723 | 51.4306287 | 11.6662 | 0.99723828 | 1 | No | Dom | Whole |

| | | | | | | | | | | | | |
|---|---|---|---|---|---|---|---|---|---|---|---|---|
| SNX29 | 25 | 17 | 16 | 12146055 | 12668145 | 57.4613572 | 16 | 0.43403063 | 0.819362781 | No | Dom | N/A |
| SOX10 | 1 | 1 | 22 | 38366693 | 38383429 | 59.751434 | 2 | 0.86514834 | 1 | No | Unknown | Whole |
| SOX9 | 1 | 1 | 17 | 70117161 | 70122552 | 0 | 1 | 0.63244927 | 0.917609012 | No | Unknown | N/A |
| SPEN | 14 | 13 | 1 | 16174359 | 16266955 | 172.793641 | 5.5 | 0.00424431 | 0.035430785 | No | Unknown | Whole |
| SPOP | 24 | 13 | 17 | 47676246 | 47755596 | 340.26465 | 12 | 0.42403028 | 0.819362781 | No | Unknown | N/A |
| SPRED1 | 6 | 5 | 15 | 38545052 | 38649450 | 57.4723654 | 2 | 0.05233021 | 0.274020034 | No | Rec | N/A |
| SRC | 7 | 5 | 20 | 35973088 | 36034453 | 114.071539 | 2.5 | 0.10837315 | 0.410127751 | No | Unknown | N/A |
| SRGAP3 | 32 | 14 | 3 | 9022278 | 9439200 | 93.542677 | 16 | 0.7290329 | 1 | No | Dom | N/A |
| SS18 | 5 | 4 | 18 | 23596219 | 23670611 | 67.2115281 | 1 | 0.01887864 | 0.116925749 | No | Dom | N/A |
| SS18L1 | 4 | 3 | 20 | 60718822 | 60757540 | 103.311121 | 3.5 | 0.68002246 | 0.962390512 | No | Dom | N/A |
| SSX2 | 2 | 2 | 23 | 52673111 | 52736239 | 31.6816627 | 1 | 0.26424102 | 0.67645701 | No | Dom | N/A |
| STAG2 | 6 | 5 | 23 | 123094062 | 123556514 | 25.948639 | 4 | 0.37116223 | 0.779273892 | Yes | Rec | N/A |
| STAT3 | 5 | 5 | 17 | 40465342 | 40540513 | 79.8180149 | 7.5171 | 0.8708939 | 1 | No | Dom | N/A |
| STAT4 | 7 | 5 | 2 | 191894302 | 192016322 | 65.5630225 | 5 | 0.56029392 | 0.917609012 | No | Dom | N/A |
| STAT5B | 5 | 5 | 17 | 40351186 | 40428725 | 77.380415 | 7.7539 | 0.88706906 | 1 | No | Dom | N/A |
| STIL | 1 | 1 | 1 | 47715811 | 47779819 | 15.6230471 | 2.5 | 0.91837315 | 1 | No | Dom | N/A |
| STK11 | 5 | 5 | 19 | 1205798 | 1228434 | 265.064499 | 1.5 | 0.01841834 | 0.116925749 | Yes | Rec | N/A |
| SUFU | 3 | 3 | 10 | 104263744 | 104393292 | 23.1574397 | 6 | 0.93898741 | 1 | No | Rec | N/A |
| SUZ12 | 7 | 5 | 17 | 30264056 | 30328064 | 109.36133 | 4 | 0.37116223 | 0.779273892 | No | Dom | N/A |
| SYK | 4 | 3 | 9 | 93564069 | 93660831 | 51.6731775 | 1 | 0.0801369 | 0.35456646 | No | Dom | N/A |
| TAF15 | 4 | 4 | 17 | 34136488 | 34174237 | 79.4723039 | 4 | 0.56723033 | 0.917609012 | No | Dom | N/A |
| TBL1XR1 | 15 | 12 | 3 | 176737143 | 176915261 | 140.356393 | 4 | 0.00086784 | 0.008472447 | Yes | Dom? | N/A |
| TCEA1 | 5 | 3 | 8 | 54879119 | 54935008 | 89.4630428 | 3 | 0.57741173 | 0.917609012 | No | Dom | N/A |
| TCF12 | 17 | 15 | 15 | 57210823 | 57582051 | 56.5690088 | 12 | 0.22600356 | 0.664173713 | No | Dom | Whole |
| TCF3 | 1 | 1 | 19 | 1609293 | 1652326 | 23.2379802 | 2.5 | 0.91837315 | 1 | No | Dom | N/A |
| TCF7L2 | 16 | 14 | 10 | 114710009 | 114927437 | 78.1868021 | 9 | 0.07222104 | 0.340978047 | No | Dom | Partial |

| | | | | | | | | | | | | |
|---|---|---|---|---|---|---|---|---|---|---|---|---|
| TET1 | 7 | 7 | 10 | 70320413 | 70454239 | 67.2515057 | 4 | 0.10992616 | 0.410127751 | No | Dom | Whole |
| TET2 | 5 | 4 | 4 | 106067032 | 106200973 | 52.2618168 | 1 | 0.01887864 | 0.116925749 | No | Rec | N/A |
| TFE3 | 1 | 1 | 23 | 48886242 | 48901012 | 0 | 0 | 0 | 0 | No | Dom | N/A |
| TFEB | 6 | 4 | 6 | 41651716 | 41703997 | 114.764446 | 5.5 | 0.79969742 | 1 | No | Dom | N/A |
| TFRC | 4 | 3 | 3 | 195754054 | 195809060 | 90.8991746 | 3 | 0.57741173 | 0.917609012 | No | Dom | N/A |
| TGFBR2 | 4 | 4 | 3 | 30647994 | 30735634 | 79.8722045 | 1.5 | 0.06538985 | 0.315362842 | No | Rec | N/A |
| THRAP3 | 3 | 3 | 1 | 36690017 | 36770958 | 37.0640343 | 5.5 | 0.91268724 | 1 | No | Dom | N/A |
| TMPRSS2 | 1 | 1 | 21 | 42836478 | 42903043 | 15.0229099 | 3.5 | 0.97013247 | 1 | No | Dom | N/A |
| TNFAIP3 | 1 | 1 | 6 | 138188351 | 138204449 | 62.119518 | 0.5 | 0.39360479 | 0.779273892 | No | Rec | N/A |
| TNFRSF14 | 1 | 1 | 1 | 2487078 | 2497061 | 100.170289 | 0 | 0 | 0 | No | Rec | Whole |
| TOP1 | 8 | 7 | 20 | 39657458 | 39753127 | 94.0743606 | 0 | 0 | 0 | No | Dom | N/A |
| TP53 | 11 | 11 | 17 | 7565257 | 7590856 | 664.088441 | 3.5 | 0.00097336 | 0.009344262 | Yes | Rec | N/A |
| TP73 | 1 | 1 | 1 | 3569084 | 3652765 | 11.950144 | 1 | 0.63244927 | 0.917609012 | No | Dom | Whole |
| TPM3 | 10 | 6 | 1 | 154127784 | 154167124 | 254.194204 | 4 | 0.21430775 | 0.636295161 | No | Dom | within 2 Mb |
| TPR | 3 | 2 | 1 | 186282954 | 186344825 | 48.4879831 | 3 | 0.80165331 | 1 | No | Dom | Whole |
| TRAF2 | 2 | 2 | 9 | 139776364 | 139821059 | 44.7477346 | 5 | 0.96017324 | 1 | No | Dom | N/A |
| TRAF7 | 5 | 4 | 16 | 2205766 | 2220130 | 223.5736 | 2 | 0.14255325 | 0.488754016 | No | Dom | N/A |
| TRIM24 | 1 | 1 | 7 | 138144953 | 138274738 | 15.4101013 | 3 | 0.95061283 | 1 | No | Dom | N/A |
| TRIM27 | 1 | 1 | 6 | 28870779 | 28891768 | 47.644004 | 1.5 | 0.77731844 | 1 | No | Dom | Whole |
| TRIM33 | 6 | 6 | 1 | 114935399 | 115053781 | 50.6833809 | 6 | 0.555186 | 0.917609012 | No | Dom | N/A |
| TRIM62 | 5 | 5 | 1 | 33611003 | 33647671 | 136.358678 | 0 | 0 | 0 | No | Rec | N/A |
| TRIP11 | 1 | 1 | 14 | 92435462 | 92506817 | 28.0288697 | 2 | 0.86514834 | 1 | No | Dom | N/A |
| TRPM3 | 30 | 25 | 9 | 73149949 | 74061820 | 50.4457319 | 20 | 0.15270196 | 0.514364498 | No | Unknown | N/A |
| TRRAP | 3 | 3 | 7 | 98475556 | 98610866 | 36.9521839 | 0 | 0 | 0 | No | Dom | Whole |
| TSC1 | 4 | 4 | 9 | 135766735 | 135820020 | 93.835038 | 1.5 | 0.06538985 | 0.315362842 | Yes | Rec | N/A |
| TSC2 | 1 | 1 | 16 | 2097466 | 2138721 | 48.4789722 | 0.5 | 0.39360479 | 0.779273892 | No | Rec | N/A |
| TSHR | 4 | 3 | 14 | 81421775 | 81612646 | 26.1957029 | 3 | 0.57741173 | 0.917609012 | No | Dom | N/A |
| TTL | 1 | 1 | 2 | 113239731 | 113290227 | 39.6070976 | 1.5 | 0.77731844 | 1 | No | Dom | Whole |
| U2AF1 | 3 | 2 | 21 | 44513066 | 44527697 | 273.392113 | 0.5 | 0.09013623 | 0.35456646 | No | Dom | N/A |
| U2AF2 | 4 | 4 | 19 | 56165416 | 56186081 | 241.954996 | 1.5 | 0.06538985 | 0.315362842 | No | Unknown | Whole |
| UBR5 | 27 | 17 | 8 | 103265572 | 103424495 | 195.063018 | 47.6769 | 0.99999996 | 1 | No | Unknown | Whole |
| UNC13C | 10 | 8 | 15 | 54305101 | 54920806 | 22.7381619 | 8 | 0.54804434 | 0.917609012 | No | Unknown | within 2 Mb |
| USP6 | 1 | 1 | 17 | 5019733 | 5078329 | 17.0660113 | 3 | 0.95061283 | 1 | No | Dom | N/A |
| USP9X | 2 | 2 | 23 | 40944888 | 41092185 | 13.5780091 | 3 | 0.80165331 | 1 | No | Unknown | N/A |
| VEGFA | 1 | 1 | 6 | 43737921 | 43754224 | 61.338404 | 1.5 | 0.77731844 | 1 | No | Dom | N/A |
| VTI1A | 13 | 6 | 10 | 114206756 | 114578503 | 45.7300261 | 0 | 0 | 0 | No | Dom | Whole |
| WHSC1 | 5 | 3 | 4 | 1873151 | 1983934 | 45.1332786 | 5 | 0.87647828 | 1 | No | Dom | N/A |
| WHSC1L1 | 31 | 19 | 8 | 38132560 | 38239790 | 326.401194 | 32.169 | 0.99606053 | 1 | No | Dom | N/A |
| WIF1 | 2 | 2 | 12 | 65444407 | 65515116 | 28.2849425 | 3.5 | 0.86493865 | 1 | No | Dom | N/A |
| WRN | 3 | 3 | 8 | 30890778 | 31031276 | 21.3526171 | 6 | 0.93898741 | 1 | No | Rec | N/A |
| WSB1 | 3 | 3 | 17 | 25621106 | 25640657 | 153.444837 | 4 | 0.76294643 | 1 | No | Dom | N/A |
| WT1 | 6 | 6 | 11 | 32409321 | 32457176 | 146.275206 | 3.5 | 0.14176386 | 0.488754016 | No | Dom | Whole |
| WWTR1 | 27 | 16 | 3 | 149235022 | 149454501 | 127.574848 | 16 | 0.53469623 | 0.917609012 | No | Rec | within 2 Mb |
| XPC | 2 | 1 | 3 | 14186647 | 14220283 | 59.4601023 | 1.5 | 0.77731844 | 1 | No | Rec | N/A |
| XPO1 | 1 | 1 | 2 | 61704984 | 61765761 | 16.4535926 | 2 | 0.86514834 | 1 | No | Dom | N/A |
| YAP1 | 6 | 6 | 11 | 101981210 | 102104154 | 65.0702759 | 6 | 0.555186 | 0.917609012 | No | Dom | N/A |
| YWHAB | 2 | 2 | 20 | 43514317 | 43537173 | 87.5043752 | 3 | 0.80165331 | 1 | No | Dom | N/A |
| YWHAE | 7 | 7 | 17 | 1247566 | 1303505 | 160.88954 | 2.5 | 0.0139701 | 0.103163824 | No | Dom | N/A |
| YWHAQ | 3 | 3 | 2 | 9724101 | 9771143 | 63.7727988 | 4 | 0.76294643 | 1 | No | Dom | N/A |
| YWHAZ | 8 | 7 | 8 | 101930804 | 101965616 | 287.257268 | 5 | 0.23715837 | 0.67645701 | No | Dom | Whole |
| ZBTB10 | 8 | 7 | 8 | 81397902 | 81438500 | 172.422287 | 3.5 | 0.06468397 | 0.315362842 | No | Dom | N/A |
| ZBTB16 | 9 | 6 | 11 | 113930315 | 114121395 | 62.8009211 | 5 | 0.38403817 | 0.779273892 | No | Dom | within 2 Mb |
| ZFHX3 | 19 | 11 | 16 | 72816784 | 73082274 | 82.8656447 | 11 | 0.54129705 | 0.917609012 | No | Unknown | N/A |
| ZMYM2 | 13 | 12 | 13 | 20532810 | 20663253 | 107.326572 | 4 | 0.00086784 | 0.008472447 | No | Dom | N/A |
| ZNF217 | 23 | 15 | 20 | 52183604 | 52226446 | 560.197937 | 7.5 | 0.009772 | 0.076063137 | No | Dom | N/A |
| ZNF331 | 5 | 2 | 19 | 54024235 | 54083523 | 101.200918 | 2 | 0.59447836 | 0.917609012 | No | Dom | Whole |
| ZNF384 | 1 | 1 | 12 | 6775643 | 6798676 | 43.4159684 | 2 | 0.86514834 | 1 | No | Dom | N/A |
| ZNF521 | 13 | 11 | 18 | 22641888 | 22932214 | 48.2216543 | 9 | 0.29304457 | 0.73070854 | No | Dom | N/A |
| ZNF639 | 3 | 2 | 3 | 179040779 | 179053323 | 239.158163 | 0 | 0 | 0 | No | Dom | N/A |
| ZNF704 | 28 | 15 | 8 | 81550769 | 81787016 | 131.218597 | 47.2494 | 1 | 1 | No | Dom | N/A |
| KEY | | | | | | | | | | | | |
| | putative rearrangement driver | | | | | | | | | | | |

**Table 7A**

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD11389a | PD11389b | 1249507582 | 14 | 105246-551 | C | T | AKT1 | CCDS9994.1 | r.603g>a | c.49G>A | p.E17K | Sub | missense | 0.38 | . |
| PD9845a | PD9845b | 1310544443 | 14 | 105246-551 | C | T | AKT1 | CCDS9994.1 | r.603g>a | c.49G>A | p.E17K | Sub | missense | 0.37 | . |
| PD8977a | PD8977b | 1322284772 | 14 | 105246-551 | C | T | AKT1 | CCDS9994.1 | r.603g>a | c.49G>A | p.E17K | Sub | missense | 0.49 | . |
| PD4248a | PD4248b | 1515911980 | 14 | 105246-551 | C | T | AKT1 | CCDS9994.1 | r.603g>a | c.49G>A | p.E17K | Sub | missense | 0.32 | . |
| PD14453a | PD14453b | 1535351464 | 14 | 105246-551 | C | T | AKT1 | CCDS9994.1 | r.603g>a | c.49G>A | p.E17K | Sub | missense | 0.45 | . |
| PD6043a | PD6043b | 2099980055 | 14 | 105246-551 | C | T | AKT1 | CCDS9994.1 | r.603g>a | c.49G>A | p.E17K | Sub | missense | 0.79 | . |
| PD9572a | PD9572b | 2263653225 | 14 | 105246-551 | C | T | AKT1 | CCDS9994.1 | r.603g>a | c.49G>A | p.E17K | Sub | missense | 0.46 | . |
| PD14462a | PD14462b | 2307747269 | 14 | 105246-551 | C | T | AKT1 | CCDS9994.1 | r.603g>a | c.49G>A | p.E17K | Sub | missense | 0.3 | . |
| PD8609a | PD8609b | 2409461482 | 14 | 105246-551 | C | T | AKT1 | CCDS9994.1 | r.603g>a | c.49G>A | p.E17K | Sub | missense | 0.15 | . |
| PD22364a | PD22364b | 2416910234 | 14 | 105246-551 | C | T | AKT1 | CCDS9994.1 | r.603g>a | c.49G>A | p.E17K | Sub | missense | 0.72 | . |
| PD11765a | PD11765b | 2440478383 | 14 | 105246-551 | C | T | AKT1 | CCDS9994.1 | r.603g>a | c.49G>A | p.E17K | Sub | missense | 0.21 | . |
| PD13428a | PD13428b | 2465507105 | 14 | 105246-551 | C | T | AKT1 | CCDS9994.1 | r.603g>a | c.49G>A | p.E17K | Sub | missense | 0.7 | . |
| PD17973a | PD17973b | . | 14 | 105246-551 | C | T | AKT1 | CCDS9994.1 | r.603g>a | c.49G>A | p.E17K | Sub | missense | 0.19 | . |
| PD24216a | PD24216b | . | 14 | 105246-551 | C | T | AKT1 | CCDS9994.1 | r.603g>a | c.49G>A | p.E17K | Sub | mis-sense | 0.56 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD9541a | PD9541b | 1285821217 | 19 | 407629-59 | C | T | AKT2 | CCDS12552.1 | r.348a>a | c.49G>A | p.E17K | Sub | missense | 0.36 | . |
| PD11365a | PD11365b | 1815022002 | 5 | 1121760-63 | C | CA | APC | CCDS4107.1 | r.5152 5153insa | c.4772 4773insA | p.P1594f-s*38 | Ins | frame-shift | . | 0.246376-81 |
| PD9065a | PD9065b | 1527958236 | 1 | 270234-30 | CTGGCCTGGCA | C | ARID1A | CCDS285.1 | r.908_917d-el UGGC-CUGGCA | c.537_546del TGGCCTGGC-A | p.G180f-s*49 | Del | frame-shift | . | 0.5483871 |
| PD14467a | PD14467b | 2039379320 | 1 | 270236-15 | TCCGGCG CGGCG GCGGCTG CCGGCTCC | T | ARID1A | CCDS285.1 | r.1093_111-8del26 | c.722_747del26 | pS241fs*1 | Del | frame-shift | . | 0.230769-23 |
| PD9760a | PD9760b | 1700212597 | 1 | 270240-01 | C | CG | ARID1A | CCDS285.1 | r.1478 1479insg | c.1107_1108in-sG | p.Q372f-s*28 | Ins | frame-shift | . | 0.340909-09 |
| PD9063a | PD9063b | 2544623933 | 1 | 270578-35 | C | T | ARID1A | CCDS285.1 | r.1914c>u | c.1543C>T | p.Q515* | Sub | nonsense | 0.2 | . |
| PD7322a | PD7322b | 2567881031 | 1 | 270578-94 | C | A | ARID1A | CCDS285.1 | r.1973c>a | c.1602C>A | p.Y534* | Sub | nonsense | 0.34 | . |
| PD11375a | PD11375b | 1703601437 | 1 | 270592-06 | GC | G | ARID1A | CCDS285.1 | r.2215delC | c.1844delC | p.S617fs*2 | Del | frame-shift | . | 0.184210-53 |
| PD4613a | PD4613b | 2261697944 | 1 | 270873-60 | C | G | ARID1A | CCDS285.1 | r.2305c>g | c.1934C>G | p.S645* | Sub | nonsense | 0.38 | . |
| PD17981a | PD17981b | 2423660516 | 1 | 270878-92 | CGGCCA CCCAGT GGCCA | C | ARID1A | CCDS285.1 | r.2551 2566del16 | c.2180_2195del16 | p.P728fs*9 | Del | frame-shift | . | 0.352941-18 |
| PD5937a | PD5937b | 1587037155 | 1 | 270886-58 | A | AC | ARID1A | CCDS285.1 | r.2638 2639insc | c.2267 2268insC | p.Q758f-s*59 | Ins | frame-shift | . | 0.134146-34 |
| PD11402a | PD11402b | 1693009608 | 1 | 270944-54 | C | CT | ARID1A | CCDS285.1 | r.3533 3534insu | c.3162 3163insT | p.Y1055f-s*50 | Ins | frame-shift | . | 0.515151-52 |
| PD5937a | PD5937b | 1587037159 | 1 | 270976-21 | CA | C | ARID1A | CCDS285.1 | r.3582delA | c.3211delA | p.K1072f-s*21 | Del | frame-shift | . | 0.232142-86 |

EP 3 452 937 B1

| Sample | Normal | VariantID | Chr om | Position | Reference | Mutant | Gene | Transcript | RNA | CDS | Protein | Type | Effect | Proportion_ Mutant _Reads _Subs | Proportion _Mutant _Reads _Indels |
|---|---|---|---|---|---|---|---|---|---|---|---|---|---|---|---|
| PD11399a | PD11399b | 1595947145 | 1 | 271001-81 | CGCA | C | ARID1A | CCDS285.1 | r.4349 4351delGCA | c.3978 3980delGCA | p.Q1334del-Q | Del | inframe | . | 0.106382-98 |
| PD7250a | PD7250b | 1588907132 | 1 | 271057-38 | AAATGATG AGGAGAT | A | ARID1A | CCDS285.1 | r.5721_573-4del AAU-GAUGAG-GAGAU | c.5350_5363del AATGATGAG-GAGAT | p.N1784f-s*13 | Del | frame-shift | . | 0.266666-67 |
| PD11381a | PD11381b | 1718184086 | 1 | 271059-30 | T | TG | ARID1A | CCDS285.1 | r.5912_591-3insg | c.5541 5542insG | p.D1850fs*4 | Ins | frameshift | . | 0.3 |
| PD11397a | PD11397b | 1289137706 | 1 | 271063-54 | C | T | ARID1A | CCDS285.1 | r.6336c>u | c.5965C>T | p.R1989* | Sub | nonsense | 0.65 | . |
| PD7206a | PD7206b | 2414633885 | 1 | 271065-04 | C | T | ARID1A | CCDS285.1 | r.6486c>u | c.6115C>T | p.Q2039* | Sub | nonsense | 0.11 | . |
| PD14441a | PD14441b | 1812025755 | 1 | 271069-21 | GA | G | ARID1A | CCDS285.1 | r.6904delA | c.6533delA | p.D2178f-s*22 | Del | frame-shift | . | 0.181818-18 |
| PD23579a | PD23579b | 2384856186 | 6 | 157405-953 | C | CG | ARID1B | CCDS5251.1 | r.2134_213-5insg | c.1982 1983insG | p.S665fs*27 | Ins | frame-shift | . | 0.259259-26 |
| PD9063a | PD9063b | 2544819116 | 6 | 157502-130 | G | T | ARID1B | CCDS5251.1 | r.3261g>u | c.3109G>T | p.E1037* | Sub | nonsense | 0.28 | . |
| PD4252a | PD4252b | 1846711753 | 6 | 157505-463 | C | G | ARID1B | CCDS5251.1 | r.3542c>a | c.3390C>G | p.Y1130* | Sub | nonsense | 0.49 | . |
| PD4844a | PD4844b3 | . | 6 | 157527-960 | GC | G | ARID1B | CCDS5251.1 | | c.5632delC | p.Q1879f-s*77 | Del | Frame-shift del | . | 0.233333-33 |
| PD10011a | PD10011b | 2305931420 | 20 | 310223-80 | TGCAG | T | ASXL1 | CCDS13201.1 | r.2290_229-3del GCAG | c.1866_1869del GCAG | p.Q623f-s*79 | Del | frame-shift | . | 0.229166-67 |
| PD5937a | PD5937b | 1250437496 | 11 | 108170-464 | G | T | ATM | CCDS31669.1 | r.5414a>u | c.5029G>T | p.E1677* | Sub | nonsense | 0.28 | . |
| PD4198a | PD4198b | 2544348563 | 11 | 108216-545 | C | T | ATM | CCDS31669.1 | r.8879c>u | c.8494C>T | p.R2832C | Sub | mis-sense | 0.24 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD14457a | PD14457b | 1840406335 | 3 | 142215-210 | C | T | ATR | CCDS3124.1 | r.6013g>a | c.5891G>A | p.W1964* | Sub | nonsense | 0.17 | . |
| PD23564a | PD23564b | 2337596206 | 3 | 142217-556 | CT | C | ATR | CCDS3124.1 | r.5562delA | c.5440delA | p.R1814fs*10 | Del | frame-shift | . | 0.148936-17 |
| PD5956a | PD5956b | 1606383998 | 3 | 142280-229 | AT | A | ATR | CCDS3124.1 | r.1326delA | c.1204delA | p.M402fs*37 | Del | frame-shift | . | 0.507936-51 |
| PD18749a | PD18749b | 2433909743 | X | 767763-58 | G | A | ATRX | CCDS14434.1 | r.7323c>u | c.7108C>T | p.Q2370* | Sub | nonsense | 0.31 | . |
| PD11462a | PD11462b | 2187347238 | X | 767763-95 | C | A | ATRX | CCDS14434.1 | r.7287-1a>u | c.7072-1G>T | p.? | Sub | ess splice | 0.16 | . |
| PD5937a | PD5937b | 1587360400 | 16 | 348179 | C | CG | AXIN1 | CCDS10405.1 | r.1698_1699insc | c.1326_1327insC | p.A443fs*26 | Ins | frame-shift | . | 0.142857-14 |
| PD6412a | PD6412b | 2258604912 | 16 | 396589 | C | T | AXIN1 | CCDS10405.1 | r.809g>a | c.437G>A | p.R146Q | Sub | missense | 0.58 | . |
| PD4958a | PD4958b | 2490564834 | X | 399165-76 | T | A | BCOR | CCDS48093.1 | r.4658-2a>u | c.4429-2A>T | p.? | Sub | ess splice | 0.33 | . |
| PD4109a | PD4109b | 1822358072 | X | 399339-58 | G | T | BCOR | CCDS48093.1 | r.870c>a | c.641C>A | p.S214* | Sub | nonsense | 0.12 | . |
| PD24333a | PD24333b | 2576040284 | 7 | 140481-411 | C | T | BRAF | CCDS5863.1 | r.1458a>a | c.1397G>A | p.G466E | Sub | missense | 0.42 | . |
| PD11742a | PD11742b | 2379187050 | 17 | 412030-96 | G | GAAGGGCCCATAGCAACAGATTTC | BRCA1 | CCDS11453.1 | gaaaucuguugcuaugoacccuu r.5712_5-713ins | GAAATCTGTTGCTATGGGCCCTT c.5315_531-6ins | p.F1772fs*29 | Ins | frameshift | . | 0.12 |
| PD7215a | PD7215b | 2253912607 | 17 | 412091-30 | T | A | BRCA1 | CCDS11453.1 | r.5613a>u | c.5216A>T | p.D1739V | Sub | missense | 0.42 | . |
| PD9585a | PD9585b | 2273490228 | 17 | 412344-51 | G | A | BRCA1 | CCDS11453.1 | r.4724c>u | c.4327C>T | p.R1443* | Sub | nonsense | 0.7 | . |
| PD7067a | PD7067b | 1226589373 | 17 | 412460-05 | C | A | BRCA1 | CCDS11453.1 | r.1940g>u | c.1543G>T | p.E515* | Sub | non-sense | 0.16 | . |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD23561a | PD23561b | 2402740834 | 17 | 412478-64 | C | CT | BRCA1 | CCDS11453.1 | r.1065_106-6insa | c.668 669insA | p.A224fs*4 | Ins | frame-shift | . | 0.073529-41 |
| PD13604a | PD13604b | 2265983816 | 13 | 3291153-0 | C | G | BRCA2 | CCDS9344.1 | r.3271c>g | c.3038C>G | p.S1013* | Sub | nonsense | 0.18 | . |
| PD11372a | PD11372b | 1213195945 | 13 | 329134-64 | C | T | BRCA2 | CCDS9344.1 | r.5205c>u | c.4972C>T | p.Q1658* | Sub | nonsense | 0.23 | . |
| PD24191a | PD24191b | 2389061790 | 13 | 329142-39 | ATTCACATAAGGTTTTTGCTGACATTCAGAGTGAAGAAATTTTACAACATAACCAAAATATGTCTGGATTGGAGAAAGTTTCTAAAATATCACCTTGTGATGTTAGTTTGGAAACTTCAGATATATGTAAATGTAGTATAGGGAAGCTTCATAAGTCAGTCTCATCTGCAAATACTTGTGGGATTTTTAGCACAGCAAGTGGAAAATCTGTCCAGGTATCAGATGCTTCATTACAAAACGCAAGACAAGTGTTTTCTGAAATAGAAGATAGTACCAA | A | BRCA2 | CCDS9344.1 | r.5981 6840del860 | c.5748 6607del860 | p.H1918fs*20 | Del | frame-shift | . | 0.25 |

(continued)

| Sample | Normal | VariantID | Chrom | Position | Reference | Mutant | Gene | Transcript | RNA | CDS | Protein | Type | Effect | Proportion_Mutant_Reads_Subs | Proportion_tion_Mutant_Reads_Indels |
|---|---|---|---|---|---|---|---|---|---|---|---|---|---|---|---|
| | | | | | GCAAGT | | | | | | | | | | |
| | | | | | CTTTTCC | | | | | | | | | | |
| | | | | | AAAGTAT | | | | | | | | | | |
| | | | | | TGTTTAA | | | | | | | | | | |
| | | | | | AAGTAAC | | | | | | | | | | |
| | | | | | GAACATT | | | | | | | | | | |
| | | | | | CAGACC | | | | | | | | | | |
| | | | | | AGCTCAC | | | | | | | | | | |
| | | | | | AAGAGA | | | | | | | | | | |
| | | | | | AGAAAAT | | | | | | | | | | |
| | | | | | ACTGCTA | | | | | | | | | | |
| | | | | | TACGTAC | | | | | | | | | | |
| | | | | | TCCAGAA | | | | | | | | | | |
| | | | | | CATTTAA | | | | | | | | | | |
| | | | | | TATCCC | | | | | | | | | | |
| | | | | | AAAAAGG | | | | | | | | | | |
| | | | | | CTTTTTC | | | | | | | | | | |
| | | | | | ATATAAT | | | | | | | | | | |
| | | | | | GTGGTAA | | | | | | | | | | |
| | | | | | ATTCATC | | | | | | | | | | |
| | | | | | TGCTTTC | | | | | | | | | | |
| | | | | | TCTGGAT | | | | | | | | | | |
| | | | | | TTAGTAC | | | | | | | | | | |
| | | | | | AGCAAGT | | | | | | | | | | |
| | | | | | GGAAAG | | | | | | | | | | |
| | | | | | CAAGTTT | | | | | | | | | | |
| | | | | | CCATTTT | | | | | | | | | | |
| | | | | | AGAAAG | | | | | | | | | | |
| | | | | | TTCCTTAC | | | | | | | | | | |
| | | | | | ACAAAGT | | | | | | | | | | |
| | | | | | TAAGGG | | | | | | | | | | |
| | | | | | AGTGTTA | | | | | | | | | | |
| | | | | | GAGGAA | | | | | | | | | | |
| | | | | | TTTGATT | | | | | | | | | | |
| | | | | | TAATCAG | | | | | | | | | | |
| | | | | | AACTGAG | | | | | | | | | | |
| | | | | | CATAGT | | | | | | | | | | |
| | | | | | CTTCACT | | | | | | | | | | |
| | | | | | ATTCACC | | | | | | | | | | |
| | | | | | TACGTCT | | | | | | | | | | |
| | | | | | AGACAAA | | | | | | | | | | |
| | | | | | ATGTATC | | | | | | | | | | |
| | | | | | AAAAAT | | | | | | | | | | |
| | | | | | ACTTCCTC | | | | | | | | | | |
| | | | | | GTGTT | | | | | | | | | | |
| | | | | | GATAAGA | | | | | | | | | | |
| | | | | | GAAACCC | | | | | | | | | | |
| | | | | | AGAGCAC | | | | | | | | | | |
| | | | | | TGTGTAA | | | | | | | | | | |
| | | | | | ACTCAGA | | | | | | | | | | |
| | | | | | AATGGA | | | | | | | | | | |
| | | | | | AAAAACC | | | | | | | | | | |
| | | | | | TGCAGT | | | | | | | | | | |
| | | | | | AAAGAAT | | | | | | | | | | |
| | | | | | TTAAATT | | | | | | | | | | |
| | | | | | ATCAAAT | | | | | | | | | | |
| | | | | | AACTTAA | | | | | | | | | | |
| | | | | | ATGTTGA | | | | | | | | | | |
| | | | | | AGGTCG | | | | | | | | | | |
| | | | | | TTCTTCAG | | | | | | | | | | |
| | | | | | AAAATA | | | | | | | | | | |
| | | | | | ATCACTC | | | | | | | | | | |
| | | | | | TATTAA | | | | | | | | | | |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD23565a | PD23565b | 2417322465 | 13 | 329144-51 | AGTTTCT CCATAT CTCTCTC AATTTCA ACAAGAC AAACAAC AGTTGGT ATTAG GAACCAA AGTGTC ACTTGTTG AGAAC ATTCATG TTTTGG GAAAAGA ACAGGC TTCACCT AAAAACG TAAAAAT GGAAA TTGGTAA AACTGA AACTTTT TCTGATG | T | BRCA2 | CCDS9344.1 | r.6192c>u | c.5959C>T | p.Q1987* | Sub | nonsense | 0.15 | . |
| PD13604a | PD13604b | 2265983817 | 13 | 329145-62 | C | T | BRCA2 | CCDS9344.1 | r.6303c>u | c.6070C>T | p.Q2024* | Sub | nonsense | 0.14 | . |
| PD6042a | PD6042b | 2256318290 | 13 | 329145-74 | G | T | BRCA2 | CCDS9344.1 | r.6315a>u | c.6082G>T | p.E2028* | Sub | nonsense | 0.26 | . |
| PD24326a | PD24326b | 2536878386 | 13 | 329290-50 | C | T | BRCA2 | CCDS9344.1 | r.7293c>u | c.7060C>T | p.Q2354* | Sub | nonsense | 0.37 | . |
| PD9000a | PD9000b | 1727367135 | 13 | 329291-99 | CAA | C | BRCA2 | CCDS9344.1 | r.7443_744-4del AA | c.7210_7211del AA | p.K2404fs*7 | Del | frame-shift | . | 0.575757-58 |
| PD8978a | PD8978b | 1585454714 | 13 | 329376-14 | GTGGGCT CTCCTGA | G | BRCA2 | CCDS9344.1 | r.8509_852-1 del UGGGCU-CUCCUGA | c.8276_8288del TGGGCTCTC-TGGA | p.G2760f-s*13 | Del | frame-shift | . | 0.733333-33 |
| PD8984a | PD8984b | 1544356612 | 13 | 329540-22 | C | CA | BRCA2 | CCDS9344.1 | r.9322 9323insa | c.9089_9090in-sA | p.T3033f-s*11 | Ins | frame-shift | . | 0.184210-53 |

| Sample | Normal | VariantID | Chrom | Position | Reference | Mutant | Gene | Transcript | RNA | CDS | Protein | Type | Effect | Proportion_Mutant _Reads _Subs | Proportion _Mutant _Reads _Indels |
|---|---|---|---|---|---|---|---|---|---|---|---|---|---|---|---|
| PD23579a | PD23579b | 2384276473 | 15 | 404775-05 | AC | A | BUB1B | CCDS10053.1 | r.1087delC | c.892delC | p.M300fs*31 | Del | frame-shift | . | 0.169811-32 |
| PD4607a | PD4607b | 2249469601 | 2 | 202150-003 | C | T | CASP8 | CCDS42798.1 | r.1640c>u | c.1444C>T | p.Q482* | Sub | nonsense | 0.24 | . |
| PD7209a | PD7209b | 2249301586 | 16 | 670633-12 | T | C | CBFB | CCDS45508.1 | r.263u>c | c.2T>C | p.M1T | Sub | start-lost | 0.5 | . |
| PD7210a | PD7210b | 2260924384 | 16 | 670633-89 | G | C | CBFB | CCDS45508.1 | r.339+1a>c | c.78+1G>C | p.? | Sub | ess splice | 0.39 | . |
| PD13310a | PD13310b | 1290169395 | 16 | 670636-72 | C | T | CBFB | CCDS45508.1 | r.382c>u | c.121C>T | p.Q41* | Sub | nonsense | 0.83 | . |
| PD18046a | PD18046b | 2138029652 | 16 | 670637-09 | C | A | CBFB | CCDS45508.1 | r.419c>a | c.158C>A | p.S53* | Sub | nonsense | 0.57 | . |
| PD9761a | PD9761b | 1311635032 | 16 | 670637-17 | G | T | CBFB | CCDS45508.1 | r.426+1g>u | c.165+1G>T | p.? | Sub | ess splice | 0.38 | . |
| PD13762a | PD13762b | 1508476196 | 16 | 670637-17 | G | T | CBFB | CCDS45508.1 | r.426+1g>u | c.165+1G>T | p.? | Sub | ess splice | 0.38 | . |
| PD17973a | PD17973b | 1821625151 | 16 | 670637-17 | G | A | CBFB | CCDS45508.1 | r.426+1g>a | c165+1 G>A | p.? | Sub | ess splice | 0.36 | . |
| PD18047a | PD18047b | 2111959342 | 16 | 670637-17 | G | GT | CBFB | CCDS45508.1 | r.426+1 426+2insu | c.165+1 165+2insT | p.? | Ins | ess splice | . | 0.4 |
| PD4982a | PD4982b | 2262444318 | 16 | 670637-17 | G | C | CBFB | CCDS45508.1 | r.426+1g>c | c.165+1G>C | p.? | Sub | ess splice | 0.64 | . |
| PD14458a | PD14458b | 2463529210 | 16 | 670637-17 | G | C | CBFB | CCDS45508.1 | r.426+1g>c | c.165+1G>C | p.? | Sub | ess splice | 0.53 | . |
| PD4972a | PD4972b | 1572128739 | 16 | 670705-77 | G | GT | CBFB | CCDS45508.1 | r.462 463insu | c.201_202insT | p. P70fs*13 | Ins | frameshift | . | 0.2962963 |
| PD4976a | PD4976b | 1737035387 | 16 | 670705-99 | G | GGA | CBFB | CCDS45508.1 | r.484_485insga | c.223 224insGA | p.Q77fs*13 | Ins | frame-shift | . | 0.625 |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18269a | PD18269b | 2447398226 | 16 | 670705-99 | G | T | CBFB | CCDS45508.1 | r.484a>u | c.223G>T | p.G75* | Sub | nonsense | 0.39 | . |
| PD7218a | PD7218b | 1628563496 | 16 | 670706-11 | CA | C | CBFB | CCDS45508.1 | r.497delA | c.236delA | p.T80fs*9 | Del | frameshift | . | 0.5483871 |
| PD18756a | PD18756b | 2537373249 | 16 | 6711611-5 | G | A | CBFB | CCDS45508.1 | r.661-1a>a | c.400-1G>A | p.? | Sub | ess splice | 0.3 | . |
| PD6044a | PD6044b | 1741449821 | 3 | 105439-049 | CT | C | CBLB | CCDS2948.1 | r.1570delA | c.1248delA | p.G417fs*5 | Del | frame-shift | . | 0.191780-82 |
| PD18748a | PD18748b | 1799840814 | 16 | 687713-11 | GGCCA GCCA | G | CDH1 | CCDS10869.1 | r.185_192del GCCAGCCA | c.1delA | p.? | Del | start-lost | . | 0.2173913 |
| PD13763a | PD13763b | 1818014934 | 16 | 687713-44 | C | A | CDH1 | CCDS10869.1 | r.217c>a | c.26C>A | p.S9* | Sub | nonsense | 0.4 | . |
| PD5961a | PD5961b | 2251446211 | 16 | 687722-18 | C | T | CDH1 | CCDS10869.1 | r.258c>u | c.67C>T | p.Q23* | Sub | nonsense | 0.23 | . |
| PD14472a | PD14472b | 2042684605 | 16 | 687722-25 | C | CG | CDH1 | CCDS10869.1 | r.265_266insg | c.74_75insG | p.E26fs*8 | Ins | frame-shift | . | 0.482758-62 |
| PD9067a | PD9067b | 2549675437 | 16 | 687722-29 | G | GC | CDH1 | CCDS10869.1 | r.269 270insc | c.78_79insC | p.C28fs*6 | Ins | frame-shift | . | 0.212765-96 |
| PD4194a | PD4194b | 2565854457 | 16 | 687722-36 | CACCC | CGTG | CDH1 | CCDS10869.1 | r.277_280del ACCCins-gug | c.86_89del AC-CCinsGTG | p.H29fs*27 | Complex | frameshift | . | 0.25 |
| PD14460a | PD14460b | 2226396789 | 16 | 687722-59 | CTACA | CTG | CDH1 | CCDS10869.1 | r.300_303del UACAinsua | c.109_112del TACAinsTG | p.Y37fs*21 | Complex | frame-shift | . | 0.368421-05 |
| PD9578a | PD9578b | 2534786745 | 16 | 687723-14 | GGT | G | CDH1 | CCDS10869.1 | r.354+1 354+2delau | c.163+1 163+2delat | p.? | Del | ess splice | . | 0.194444-44 |

EP 3 452 937 B1

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD6016a2 | PD6016b | 1319092030 | 16 | 688356-13 | T | A | CDH1 | CCDS10869.1 | r.395u>a | c.204T>A | p.Y68* | Sub | nonsense | 0.29 | . |
| PD7204a | PD7204b | 2178523092 | 16 | 688357-51 | C | CA | CDH1 | CCDS10869.1 | r.533 534insa | c.342 343insA | p.T115fs*53 | Ins | frame-shift | . | 0.363636-36 |
| PD11376a | PD11376b | 1254857050 | 16 | 688423-26 | G | C | CDH1 | CCDS10869.1 | r.579-1a>c | c.388-1G>C | p.? | Sub | ess splice | 0.15 | . |
| PD13306a | PD13306b | 1560375757 | 16 | 688423-48 | GC | G | CDH1 | CCDS10869.1 | r.601delC | c.410delC | p.A137fs*78 | Del | frame-shift | . | 0.357142-86 |
| PD7238a | PD7238b | 1599550369 | 16 | 688424-32 | G | GA | CDH1 | CCDS10869.1 | r.684 685insa | c.493 494insA | p.N166fs*2 | Ins | frame-shift | . | 0.416666-67 |
| PD9579a | PD9579b | 2272671736 | 16 | 688424-71 | G | A | CDH1 | CCDS10869.1 | r.722+1a>a | c.531+1G>A | p.? | Sub | ess splice | 0.22 | . |
| PD13607a | PD13607b | 2165290946 | 16 | 688426-63 | C | CGT | CDH1 | CCDS10869.1 | r.790_791insgu | c.599 600insGT | p.P201fs*15 | Ins | frame-shift | . | 0.310344-83 |
| PD14465a | PD14465b | 2228364311 | 16 | 688427-14 | CAG | C | CDH1 | CCDS10869.1 | r.842 843delAG | c.651 652delAG | p.E218fs*4 | Del | frame-shift | . | 0.387096-77 |
| PD9597a | PD9597b | 1322709667 | 16 | 688441-75 | C | T | CDH1 | CCDS10869.1 | r.954c>u | c.763C>T | p.Q255* | Sub | nonsense | 0.17 | . |
| PD4985a | PD4985b | 2258951398 | 16 | 688441-75 | C | T | CDH1 | CCDS10869.1 | r.954c>u | c.763C>T | p.Q255* | Sub | nonsense | 0.73 | . |
| PD9063a | PD9063b | 2544704444 | 16 | 688441-75 | C | T | CDH1 | CCDS10869.1 | r.954c>u | c.763C>T | p.Q255* | Sub | nonsense | 0.11 | . |
| PD11740a | PD11740b | 2022557920 | 16 | 688441-93 | G | T | CDH1 | CCDS10869.1 | r.972a>u | c.781G>T | p.E261* | Sub | nonsense | 0.24 | . |
| PD11358a | PD11358b | 2559488943 | 16 | 688442-45 | G | C | CDH1 | CCDS10869.1 | r.1023+1g>-c | c.832+1G>C | p.? | Sub | ess splice | 0.78 | . |
| PD7214a | PD7214b | 1642087850 | 16 | 688456-58 | T | TA | CDH1 | CCDS10869.1 | r.1095_109-6insa | c.904_905insA | p.Y302fs*1 | Ins | frame-shift | . | 0.254901-96 |

EP 3 452 937 B1

125

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18188a | PD18188b | 2179313801 | 16 | 688457-23 | AGGAGTCATC | AGT | CDH1 | CCDS10869.1 | r.1161_1169del GGAGUCAUCinsgu | c.970_978del GGAGTCATCinsGT | p.G324fs*30 | Complex | frameshift | . | 0.22580645 |
| PD11342a | PD11342b | 1551245099 | 16 | 688460-53 | AC | A | CDH1 | CCDS10869.1 | r.1216delC | c.1025delC | p.L343fs*13 | Del | frameshift | . | 0.31818182 |
| PD4977a | PD4977b | 2259169109 | 16 | 688472-76 | G | A | CDH1 | CCDS10869.1 | r.1389a>a | c.1198G>A | p.D400N | Sub | missense | 0.34 | . |
| PD5936a | PD5936b | 1600387674 | 16 | 688472-85 | G | GC | CDH1 | CCDS10869.1 | r.1398_1399insc | c.1207_1208insC | p.N405fs*14 | Ins | frameshift | . | 0.77777778 |
| PD18756a | PD18756b | 2537373265 | 16 | 688496-28 | C | T | CDH1 | CCDS10869.1 | r.1722c>u | c.1531C>T | p.Q511* | Sub | nonsense | 0.29 | . |
| PD9589a | PD9589b | 1601167181 | 16 | 688560-49 | TC | T | CDH1 | CCDS10869.1 | r.2049delC | c.1858delC | p. P620fs*11 | Del | frameshift | . | 0.42857143 |
| PD18049a | PD18049b | 2106698576 | 16 | 688560-77 | G | GA | CDH1 | CCDS10869.1 | r.2076_2077insa | c.1885_1886insA | p.L630fs*33 | Ins | frameshift | . | 0.28787879 |
| PD14442a | PD14442b | 1844020950 | 16 | 688574-60 | C | T | CDH1 | CCDS10869.1 | r.2286c>u | c.2095C>T | p.Q699* | Sub | nonsense | 0.32 | . |
| PD5960a | PD5960b | 1306351543 | 16 | 688575-30 | G | T | CDH1 | CCDS10869.1 | r.2355+1g>u | c.2164+1G>T | p.? | Sub | ess splice | 0.28 | . |
| PD8618a | PD8618b | 1370683273 | 16 | 688622-12 | G | T | CDH1 | CCDS10869.1 | r.2486+5g>u | c.2295+5G>T | p.? | Sub | ess splice | 0.46 | . |
| PD7209a | PD7209b | 1638131033 | 16 | 688672-05 | GCTGATACTGAC | G | CDH1 | CCDS10869.1 | r.2644_2654del CUGAUACUGAC | c.2453_2463del CTGATACTGAC | p.D819fs*6 | Del | frameshift | . | 0.19354839 |
| PD11375a | PD11375b | 1703883542 | 16 | 688672-84 | GTC | G | CDH1 | CCDS10869.1 | r.2723_2724delUC | c.2532_2533delTC | p.L845fs*15 | Del | frameshift | . | 0.40540541 |

126

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD13416a | PD13416b | 1315351920 | 12 | 128710-92 | C | T | CDKN1-B | CCDS8653.1 | r.696c>u | c.319C>T | p.Q107* | Sub | nonsense | 0.38 | . |
| PD14472a | PD14472b | 2056559511 | 12 | 128712-12 | C | T | CDKN1-B | CCDS8653.1 | r.816c>u | c.439C>T | p.Q147* | Sub | nonsense | 0.38 | . |
| PD11341a | PD11341b | 2414367523 | 9 | 219710-36 | C | T | CDKN2-A | CCDS6510.1 | r.593a>a | c.322G>A | p.D108N | Sub | missense | 0.27 | . |
| PD11336a | PD11336b | 2415374729 | 9 | 2197111-1 | G | A | CDKN2-A | CCDS6510.1 | r.518c>u | c.247C>T | p.H83Y | Sub | missense | 0.14 | . |
| PD18733a | PD18733b | 2453038215 | 9 | 2197112-0 | G | A | CDKN2-A | CCDS6510.1 | r.509c>u | c.238C>T | p.R80* | Sub | nonsense | 0.6 | . |
| PD13604a | PD13604b | 2266039912 | 19 | 427930-85 | C | G | CIC | CCDS12601.1 | r.1017c>g | c.977C>G | p.S326* | Sub | nonsense | 0.21 | . |
| PD5937a | PD5937b | 1250507996 | 19 | 546468-87 | G | A | CNOT3 | CCDS12880.1 | r.1661g>a | c.58G>A | p.E20K | Sub | missense | 0.097 | . |
| PD23565a | PD23565b | 2368246951 | 16 | 3807363 | T | TG | CREBBP | CCDS10509.1 | r.4428 4429insc | c.3623 3624insC | p.Q1209f-s*25 | Ins | frame-shift | . | 0.380952-38 |
| PD5937a | PD5937b | 1587361854 | 16 | 3817720 | CT | C | CREBBP | CCDS10509.1 | r.4055delA | c.3250delA | p.11084f-s*15 | Del | frame-shift | . | 0.246153-85 |
| PD9694a | PD9694b | 1210862299 | 16 | 3820624 | G | A | CREBBP | CCDS10509.1 | r.3632c>u | c.2827C>T | p.Q943* | Sub | nonsense | 0.21 | . |
| PD23579a | PD23579b | 2416737661 | 16 | 3823754 | G | A | CREBBP | CCDS10509.1 | r.3266c>u | c.2461C>T | p.Q821* | Sub | nonsense | 0.31 | . |
| PD9009a | PD9009b | 2406837316 | 16 | 676450-84 | C | T | CTCF | CCDS10841.1 | r.793c>u | c.349C>T | p.Q117* | Sub | nonsense | 0.13 | . |
| PD9541a | PD9541b | 1285812760 | 16 | 676459-22 | C | T | CTCF | CCDS10841.1 | r.1294c>u | c.850C>T | p.H284Y | Sub | missense | 0.61 | . |
| PD9467a | PD9467b | 1820972433 | 16 | 676459-24 | C | G | CTCF | CCDS10841.1 | r.1296c>a | c.852C>G | p.H284Q | Sub | mis-sense | 0.35 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD24223a | PD24223b | 2472825195 | 16 | 676507-56 | C | T | CTCF | CCDS10841.1 | r.1505c>u | c.1061C>T | p.S354F | Sub | missense | 0.44 | . |
| PD9570a | PD9570b | 2261182675 | 16 | 676707-55 | G | T | CTCF | CCDS10841.1 | r.2443+1g>-u | c.1999+1G>T | p.? | Sub | ess splice | 0.12 | . |
| PD24326a | PD24326b | 2536956717 | 7 | 101833-132 | G | T | CUX1 | CCDS5721.1 | r.1095g>u | c.1057G>T | p.E353* | Sub | nonsense | 0.5 | . |
| PD4957a | PD4957b | 2246202941 | 7 | 101877-410 | G | A | CUX1 | CCDS5721.1 | r.3550g>a | c.3512G>A | p.W1171* | Sub | nonsense | 0.22 | . |
| PD7207a | PD7207b | 2248152330 | 2 | 254586-49 | G | A | DNMT3-A | CCDS33157.1 | r.2862c>u | c.2524C>T | p.Q842* | Sub | nonsense | 0.31 | . |
| PD18768a | PD18768b | 2478680833 | 2 | 254709-03 | CACCTCGT | C | DNMT3-A | CCDS33157.1 | r.1189_1193+2del ACGAGau | c.851_855+2del ACGAGgt | p.? | Del | ess splice | . | 0.304347-83 |
| PD5937a | PD5937b | 1250608229 | 6 | 139167-714 | C | G | ECT2L | CCDS43508.1 | r.964c>a | c.803C>G | p.S268* | Sub | nonsense | 0.25 | . |
| PD9568a | PD9568b | 1853806315 | 7 | 552330-36 | C | G | EGFR | CCDS5514.1 | r.1963c>a | c.1786C>G | p.P596A | Sub | missense | 0.25 | . |
| PD23564a | PD23564b | 2418491806 | 7 | 552417-22 | G | A | EGFR | CCDS5514.1 | r.2347a>a | c.2170G>A | p.G724S | Sub | missense | 0.26 | . |
| PD13425a | PD13425b | 1319884916 | 17 | 378682-08 | C | A | ERBB2 | CCDS32642.1 | r.1167c>a | c.929C>A | p.S310Y | Sub | missense | 0.15 | . |
| PD11338a | PD11338b | 1208074171 | 17 | 378802-20 | T | C | ERBB2 | CCDS32642.1 | r.2502u>c | c2264T>C | p.L755S | Sub | missense | 0.39 | . |
| PD4072a | PD4072b | 2256066191 | 17 | 378802-57 | C | G | ERBB2 | CCDS32642.1 | r.2539c>g | c.2301C>G | p.I767M | Sub | missense | 0.21 | . |
| PD4072a | PD4072b | 2256066192 | 17 | 378802-61 | G | T | ERBB2 | CCDS32642.1 | r.2543a>u | c.2305G>T | p. D769Y | Sub | missense | 0.22 | . |
| PD23565a | PD23565b | 2417340538 | 17 | 378802-61 | G | T | ERBB2 | CCDS32642.1 | r.2543a>u | c.2305G>T | p.D769Y | Sub | missense | 0.45 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18049a | PD18049b | 2406512772 | 17 | 378810-00 | G | T | ERBB2 | CCDS32642.1 | r.2567a>u | c.2329G>T | p.V777L | Sub | missense | 0.67 | . |
| PD11386a | PD11386b | 1568050463 | 17 | 378810-02 | G | GGGCTCCCCA | ERBB2 | CCDS32642.1 | r.2569_257-0ins aa-cucccca | c.2331_2332ins GGCTCCCCA | p.P780_-Y781 insGSP | Ins | inframe | . | 0.191176-47 |
| PD9575a | PD9575b | 1920705885 | 12 | 564788-54 | G | T | ERBB3 | CCDS31833.1 | r.503g>u | c.310G>T | p.V104L | Sub | missense | 0.19 | . |
| PD11348a | PD11348b | 2175085442 | 12 | 564788-54 | G | T | ERBB3 | CCDS31833.1 | r.503g>u | c.310G>T | p.V104L | Sub | missense | 0.24 | . |
| PD13306a | PD13306b | 2464848618 | 12 | 564788-54 | G | T | ERBB3 | CCDS31833.1 | r.503g>u | c.310G>T | p.V104L | Sub | missense | 0.27 | . |
| PD23564a | PD23564b | 2418342616 | 16 | 140260-58 | C | T | ERCC4 | CCDS32390.1 | r.1027c>u | c.1018C>T | p.R340* | Sub | nonsense | 0.23 | . |
| PD18264a | PD18264b | 2453231924 | 6 | 152419-923 | A | G | ESR1 | CCDS5234.1 | r.1980a>g | c.1610A>G | p.Y537C | Sub | missense | 0.23 | . |
| PD13768a | PD13768b | 2452957675 | 6 | 152419-926 | A | G | ESR1 | CCDS5234.1 | r.1983a>g | c.1613A>G | p.D538G | Sub | missense | 0.2 | . |
| PD24320a | PD24320b | 2575269744 | 6 | 152419-926 | A | G | ESR1 | CCDS5234.1 | r.1983a>g | c.1613A>G | p.D538G | Sub | missense | 0.34 | . |
| PD24208a | PD24208b | 2385306441 | 4 | 153247-289 | G | A | FBXW7 | CCDS3777.1 | r.1662c>u | c.1513C>T | p.R505C | Sub | missense | 0.89 | . |
| PD11379a | PD11379b | 2415469239 | 4 | 153268-084 | G | A | FBXW7 | CCDS3777.1 | r.873c>u | c.724C>T | p.Q242* | Sub | nonsense | 0.44 | . |
| PD9571a | PD9571b | 1856278251 | 4 | 153332-811 | C | A | FBXW7 | CCDS3777.1 | r.294a>u | c.145G>T | p.E49* | Sub | nonsense | 0.76 | . |
| PD11359a | PD11359b | 1310642758 | 10 | 123258-034 | A | T | FGFR2 | CCDS7620.2 | r.2242u>a | c.1650T>A | p.N550K | Sub | missense | 0.39 | . |
| PD9597a | PD9597b | 1322688424 | 14 | 380612-40 | G | A | FOXA1 | CCDS9665.1 | r.811c>u | c.749C>T | p.S250F | Sub | missense | 0.22 | . |

EP 3 452 937 B1

(continued)

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD13623a | PD13623b | 1508838239 | 14 | 380612-40 | G | A | FOXA1 | CCDS9665.1 | r.811c>u | c.749C>T | p.S250F | Sub | missense | 0.41 | . |
| PD11386a | PD11386b | 1323200122 | 14 | 380613-13 | C | T | FOXA1 | CCDS9665.1 | r.738a>a | c.676G>A | p.D226N | Sub | missense | 0.31 | . |
| PD9063a | PD9063b | 2544683024 | 14 | 380613-13 | C | T | FOXA1 | CCDS9665.1 | r.738a>a | c.676G>A | p.D226N | Sub | missense | 0.3 | . |
| PD18247a | PD18247b | . | 14 | 380613-13 | C | T | FOXA1 | CCDS9665.1 | r.738a>a | c.676G>A | p.D226N | Sub | missense | 0.094 33962 | . |
| PD6016a2 | PD6016b | . | 14 | 380613-13 | C | T | FOXA1 | CCDS9665.1 | r.738a>a | c.676G>A | p.D226N | Sub | missense | 0.1111 1111 | . |
| PD14433a | PD14433b | 2493924319 | 14 | 380614-61 | G | C | FOXA1 | CCDS9665.1 | r.590c>g | c.528C>G | p.1176M | Sub | missense | 0.25 | . |
| PD5950a | PD5950b | 1228278001 | 3 | 710217-85 | G | A | FOXP1 | CCDS2914.1 | r.2099c>u | c.1573C>T | p.R525* | Sub | nonsense | 0.15 | . |
| PD9605a | PD9605b | 1742397208 | 3 | 7102611-3 | TC | T | FOXP1 | CCDS2914.1 | r.2034delG | c.1508delG | p. R503fs*24 | Del | frame-shift | . | 0.351351-35 |
| PD13753a | PD13753b | 2451864525 | 3 | 710646-95 | C | T | FOXP1 | CCDS2914.1 | r.1500+5g>-a | c.974+5G>A | p.? | Sub | ess splice | 0.14 | . |
| PD11379a | PD11379b | 2415456840 | 3 | 7110291-4 | G | T | FOXP1 | CCDS2914.1 | r.819c>a | c.293C>A | p.S98* | Sub | nonsense | 0.49 | . |
| PD13623a | PD13623b | 1482874026 | 10 | 8097752 | AGGAGGT GGATG TGCTTTTT | A | GATA3 | CCDS31143.1 | r.703_721del19 | c.135_153del19 | p. E46fs*143 | Del | frame-shift | . | 0.194444-44 |
| PD5937a | PD5937b | 1250416409 | 10 | 8106058 | T | A | GATA3 | CCDS31143.1 | r.1449u>a | c.881T>A | p.M294K | Sub | missense | 0.2 | . |
| PD9063a | PD9063b | 2544645668 | 10 | 8106058 | T | A | GATA3 | CCDS31143.1 | r.1449u>a | c.881T>A | p.M294K | Sub | missense | 0.18 | . |
| PD13752a | PD13752b | 1489656042 | 10 | 8111432 | TCA | T | GATA3 | CCDS31143.1 | r.1493-3_1-493-2delca | c.925-3_925-2-delca | p.? | Del | ess splice | . | 0.3 |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4982a | PD4982b | 1558585200 | 10 | 8111432 | TCA | T | GATA3 | CCDS31143.1 | r.1493-3_1-493-2delca | c.925-3_925-2-delca | p.? | Del | ess splice | . | 0.285714-29 |
| PD4069a | PD4069b | 1578712271 | 10 | 8111432 | TCA | T | GATA3 | CCDS31143.1 | r.1493-3_1-493-2delca | c.925-3_925-2-delca | p.? | Del | ess splice | . | 0.361702-13 |
| PD7218a | PD7218b | 1628359351 | 10 | 8111432 | TCA | T | GATA3 | CCDS31143.1 | r.1493-3_1-493-2delca | c.925-3_925-2-delca | p.? | Del | ess splice | . | 0.555555-56 |
| PD9001a | PD9001b | 1677955208 | 10 | 8111432 | TCA | T | GATA3 | CCDS31143.1 | r.1493-3_1-493-2delca | c.925-3_925-2-delca | p.? | Del | ess splice | . | 0.2972973 |
| PD7210a | PD7210b | 1747391809 | 10 | 8111432 | TCA | T | GATA3 | CCDS31143.1 | r.1493-3_1-493-2delca | c.925-3_925-2-delca | p.? | Del | ess splice | . | 0.173913-04 |
| PD9581a | PD9581b | 1748195211 | 10 | 8111432 | TCA | T | GATA3 | CCDS31143.1 | r.1493-3_1-493-2delca | c.925-3_925-2-delca | p.? | Del | ess splice | . | 0.309859-15 |
| PD13619a | PD13619b | 1832199288 | 10 | 8111432 | TCA | T | GATA3 | CCDS31143.1 | r.1493-3_1-493-2delca | c.925-3_925-2-delca | p.? | Del | ess splice | . | 0.341463-41 |
| PD4085a | PD4085b | 2172392702 | 10 | 8111432 | TCA | T | GATA3 | CCDS31143.1 | r.1493-3_1-493-2delca | c.925-3_925-2-delca | p.? | Del | ess splice | . | 0.44 |
| PD23570a | PD23570b | 2400736400 | 10 | 8111432 | TCA | T | GATA3 | CCDS31143.1 | r.1493-3_1-493-2delca | c.925-3_925-2-delca | p.? | Del | ess splice | . | 0.243243-24 |
| PD18269a | PD18269b | 2439664541 | 10 | 8111432 | TCA | T | GATA3 | CCDS31143.1 | r.1493-3_1-493-2delca | c.925-3_925-2-delca | p.? | Del | ess splice | . | 0.170212-77 |
| PD7322a | PD7322b | 2488385972 | 10 | 8111432 | TCA | T | GATA3 | CCDS31143.1 | r.1493-3_1-493-2delca | c.925-3_925-2-delca | p.? | Del | ess splice | . | 0.34 |
| PD9539a | PD9539b | 2531735365 | 10 | 8111432 | TCA | T | GATA3 | CCDS31143.1 | r.1493-3_1-493-2delca | c.925-3_925-2-delca | p.? | Del | ess splice | . | 0.277777-78 |
| PD13756a | PD13756b | 2552598735 | 10 | 8111432 | TCA | T | GATA3 | CCDS31143.1 | r.1493-3_1-493-2delca | c.925-3_925-2-delca | p.? | Del | ess splice | . | 0.295454-55 |
| PD6422a | PD6422b | 1641080197 | 10 | 8111484 | A | ACAACCACACT | GATA3 | CCDS31143.1 | r.1541_154-2ins caac-cacacu | c.973_974ins CAACCACACT | p.W329f-s*27 | Ins | frame-shift | . | 0.125 |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD13625a | PD13625b | 1813207266 | 10 | 8111493 | C | CT | GATA3 | CCDS31143.1 | r.1550_155-1insu | c.982_983insT | p.W329fs*24 | Ins | frame-shift | . | 0.378787-88 |
| PD18734a | PD18734b | 2410368888 | 10 | 8111496 | T | TG | GATA3 | CCDS31143.1 | r.1553_155-4insg | c.985_986insG | p.R330fs*23 | Ins | frameshift | . | 0.32 |
| PD14456a | PD14456b | 1483930818 | 10 | 8111497 | G | GGA GGA | GATA3 | CCDS31143.1 | r.1554_155-5ins aaaaa | c.986_987ins GAGGA | p.N332fs*26 | Ins | frameshift | . | 0.25 |
| PD11336a | PD11336b | 1762439312 | 10 | 8111499 | AGG | A | GATA3 | CCDS31143.1 | r.1557_155-8delGG | c.989_990delG-G | p.R330fs*22 | Del | frame-shift | . | 0.238095-24 |
| PD11388a | PD11388b | 2553848069 | 10 | 8111500 | G | GA | GATA3 | CCDS31143.1 | r.1557_155-8insa | c.989_990insA | p.R331fs*22 | Ins | frame-shift | . | 0.210526-32 |
| PD11384a | PD11384b | 1715682690 | 10 | 8111513 | T | TGG | GATA3 | CCDS31143.1 | r.1570_157-1 insgg | c.1002_1003in-sGG | p.D336fs*21 | Ins | frame-shift | . | 0.218181-82 |
| PD7209a | PD7209b | 1637928061 | 10 | 8111537 | T | TG | GATA3 | CCDS31143.1 | r.1594_159-5insg | c.1026_1027in-sG | p.L344fs"9 | Ins | frame-shift | . | 0.239130-43 |
| PD9761a | PD9761b | 1311607618 | 10 | 8111549 | C | A | GATA3 | CCDS31143.1 | r.1606c>a | c.1038C>A | p.Y346* | Sub | nonsense | 0.39 | . |
| PD11389a | PD11389b | 1675277083 | 10 | 8111554 | T | TTC | GATA3 | CCDS31143.1 | r.1611_161-2insuc | c.1043_1044in-sTC | p.H349fs*8 | Ins | frameshift | . | 0.3125 |
| PD14450a | PD14450b | 2227138959 | 10 | 8115704 | TAACA | T | GATA3 | CCDS31143.1 | r.1622_162-5del AACA | c.1054_1057del AACA | p.N352fs*3 | Del | frame-shift | . | 0.275862-07 |
| PD14458a | PD14458b | 2461533029 | 10 | 8115705 | AAC | A | GATA3 | CCDS31143.1 | r.1623_162-4delAC | c.1055_1056d-elAC | p.N352fs*19 | Del | frame-shift | . | 0.327272-73 |
| PD13310a | PD13310b | 1561046913 | 10 | 8115710 | AC | A | GATA3 | CCDS31143.1 | r.1628delC | c.1060delC | p. L355fs*1 | Del | frame-shift | . | 0.521739-13 |
| PD22362a | PD22362b | 2314367145 | 10 | 8115741 | ACCAGAAACCG AAAAATGTCTAG CAAATCCAAAA AGTGCAAAAAA GTGCATGACTC ACTGGAGGACT TCCCCAAGAAC AGCTCGTTTAA CCCGGCCGCC CTCTCCAGACA CATGTCCTCCC TGAGCCACATC | A | GATA3 | CCDS31143.1 | r.1659_182-5del167 | c.1091_1257d-el167 | p.R365fs*87 | Del | frame-shift | . | 0.194444-44 |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| | | | | | TCGCCCTTCAGCCACTCCAGCCACATGCTGACCACG | | | | | | | | | | |
| PD18264a | PD18264b | 2434901164 | 10 | 8115770 | CAA | C | GATA3 | CCDS31143.1 | r.1688_1689delAA | c.1120_1121delAA | p.K375fs*6 | Del | frameshift | . | 0.14 |
| PD24214a | PD24214b | 2479221831 | 10 | 8115779 | C | A | GATA3 | CCDS31143.1 | r.1696c>a | c.1128C>A | p.C376* | Sub | nonsense | 0.35 | . |
| PD11395a | PD11395b | 1803239107 | 10 | 8115814 | AGAACAGCTCGTTTAACCCGGCCG | A | GATA3 | CCDS31143.1 | r.1732_1754del23 | c.1164_1186del23 | p.K388fs*112 | Del | frameshift | . | 0.11904762 |
| PD7305a | PD7305b | 2114075544 | 10 | 8115850 | A | ACATGTCCTCCCTGAGC | GATA3 | CCDS31143.1 | r.1767_1768ins cauauc-cucccuaaac | CATGTCCTCCCTGAGC c.1199_1200ins | p.1407fs*106 | Ins | frameshift | . | 0.22 |
| PD9847a | PD9847b | 1686582986 | 10 | 8115851 | C | CA | GATA3 | CCDS31143.1 | r.1768_1769insa | c.1200_1201insA | p.M401fs*107 | Ins | frameshift | . | 0.35897436 |
| PD11348a | PD11348b | 2152289242 | 10 | 8115853 | T | TG | GATA3 | CCDS31143.1 | r.1770_1771insg | c.1202_1203insG | p.S402fs*106 | Ins | frameshift | . | 0.29787234 |
| PD11818a | PD11818b | 1660202148 | 10 | 8115873 | T | TC | GATA3 | CCDS31143.1 | r.1790_1791insc | c.1222_1223insC | p.P409fs*99 | Ins | frameshift | . | 0.21052632 |
| PD11381a | PD11381b | 1718253498 | 10 | 8115874 | C | CG | GATA3 | CCDS31143.1 | r.1791_1792insg | c.1223_1224insG | p.P409fs*99 | Ins | frameshift | . | 0.27083333 |
| PD6048a | PD6048b | 2131239466 | 10 | 8115874 | C | CT | GATA3 | CCDS31143.1 | r.1791_1792insu | c.1223_1224insT | p.P409fs*99 | Ins | frameshift | . | 0.24561404 |
| PD11761a | PD11761b | 2377830902 | 10 | 8115874 | C | CT | GATA3 | CCDS31143.1 | r.1791_1792insu | c.1223_1224insT | p.P409fs*99 | Ins | frameshift | . | 0.15625 |
| PD24320a | PD24320b | 2560421339 | 10 | 8115874 | C | CA | GATA3 | CCDS31143.1 | r.1791_1792insa | c.1223_1224insA | p.P409fs*99 | Ins | frameshift | . | 0.30434783 |
| PD9577a | PD9577b | 1681948570 | 10 | 8115892 | G | GC | GATA3 | CCDS31143.1 | r.1809_1810insc | c.1241_1242insC | p.H415fs*93 | Ins | frame-shift | . | 0.22058824 |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD9760a | PD9760b | 1700278180 | 10 | 8115911 | C | CA | GATA3 | CCDS31143.1 | r.1828_182-9insa | c.1260_1261in-sA | p.T421fs*87 | Ins | frame-shift | . | 0.288888-89 |
| PD13764a | PD13764b | 2515235648 | 10 | 8115919 | T | TG | GATA3 | CCDS31143.1 | r.1836_183-7insg | c.1268_1269in-sG | p.H424fs*84 | Ins | frame-shift | . | 0.222222-22 |
| PD9000a | PD9000b | 1727259349 | 10 | 8115928 | C | CA | GATA3 | CCDS31143.1 | r.1845_184-6insa | c.1277_1278in-sA | p.S427fs*81 | Ins | frame-shift | . | 0.315789-47 |
| PD4103a | PD4103b | . | 10 | 8115944 | T | TG | GATA3 | CCDS31143.1 | c.1295_12-96insG | p.P433fs*75 | Ins | Frameshift ins . | | | |
| PD9759a | PD9759b | 1739163172 | 10 | 8115952 | A | AC | GATA3 | CCDS31143.1 | r.1869_187-0insc | c.1301_1302in-sC | p.H435fs*73 | Ins | frame-shift | . | 0.151515-15 |
| PD14467a | PD14467b | 2039451005 | 10 | 8115952 | A | ACC | GATA3 | CCDS31143.1 | r.1869_187-0inscc | c.1301_1302in-sCC | p.H435fs*42 | Ins | frame-shift | . | 0.244444-44 |
| PD7306a | PD7306b | 2160956120 | 10 | 8115955 | A | AC | GATA3 | CCDS31143.1 | r.1872_187-3insc | c.1304_1305in-sC | p.S437fs*71 | Ins | frame-shift | . | 0.192307-69 |
| PD6711a2 | PD6711b | 2503502278 | 10 | 8115955 | A | ACC | GATA3 | CCDS31143.1 | r.1872_187-3inscc | c.1304_1305in-sCC | p.S437fs*40 | Ins | frame-shift | . | 0.416666-67 |
| PD5964a | PD5964b | 1580512876 | 10 | 8115962 | C | CAG CAT | GATA3 | CCDS31143.1 | r.1879_188-0ins aacau | c.1311_1312ins AGCAT | p.M439f-s*39 | Ins | frame-shift | . | 0.256410-26 |
| PD6417a | PD6417b | 1638709198 | 10 | 8115982 | GT | G | GATA3 | CCDS31143.1 | r.1900delU | c.1332delT | p.*445fs*31 | Del | frameshift | . | 0.2962963 |
| PD9193a | PD9193b | 2203330086 | 10 | 8115982 | G | GT | GATA3 | CCDS31143.1 | r.1899_190-0insu | c.1331_1332in-sT | p.*445fs*63 | Ins | frameshift | . | 0.14 |
| PD9584a | PD9584b | 2408553682 | 11 | 533874 | T | A | HRAS | CCDS7698.1 | r.370a>u | c.182A>T | p.Q61L | Sub | missense | 0.25 | . |
| PD14472a | PD14472b | 2043340041 | X | 448942-28 | AAAGT | A | KDM6A | CCDS14265.1 | r.659_660+-2del AAau | c.618_619+2del AAgt | p.? | Del | ess splice | . | 0.2173913 |
| PD7321a | PD7321b | 2244889631 | X | 449183-38 | G | A | KDM6A | CCDS14265.1 | r.1004a>a | c.963G>A | p.W321* | Sub | non-sense | 0.33 | . |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD14458a | PD14458b | 2462409174 | X | 44922769 | C | CA | KDM6A | CCDS14265.1 | r.1671_1672insa | c.1630_1631insA | p.L545fs*8 | Ins | frameshift | . | 0.30769231 |
| PD9001a | PD9001b | 2264385750 | X | 44929206 | C | A | KDM6A | CCDS14265.1 | r.2347c>a | c.2306C>A | p.S769* | Sub | nonsense | 0.35 | . |
| PD9568a | PD9568b | 1853838523 | X | 44949176 | G | C | KDM6A | CCDS14265.1 | r.3777+1g>c | c.3736+1G>C | p.? | Sub | ess splice | 0.57 | . |
| PD24333a | PD24333b | 2575951852 | 12 | 25398284 | C | A | KRAS | CCDS8703.1 | r.216a>u | c.35G>T | p.G12V | Sub | missense | 0.3 | . |
| PD7220a | PD7220b | 2272325989 | 17 | 11958295 | C | T | MAP2K4 | CCDS11162.1 | r.268c>u | c.205C>T | p.Q69* | Sub | nonsense | 0.43 | . |
| PD11395a | PD11395b | 1803507772 | 17 | 11984670 | CAGAG | C | MAP2K4 | CCDS11162.1 | r.282-2_28-3delaaAG | c.219-2_220delagAG | p.? | Del | ess splice | . | 0.46428571 |
| PD4085a | PD4085b | 2172575159 | 17 | 11984670 | CAG | C | MAP2K4 | CCDS11162.1 | r.282-2_28-2-1delag | c.219-2_219-1delaa | p.? | Del | ess splice | . | 0.45714286 |
| PD11369a | PD11369b | 1210679700 | 17 | 11984698 | G | T | MAP2K4 | CCDS11162.1 | r.307a>u | c.244G>T | p.E82* | Sub | nonsense | 0.78 | . |
| PD11344a | PD11344b | 2184229327 | 17 | 12011144 | C | T | MAP2K4 | CCDS11162.1 | r.614c>u | c.551C>T | p.S184L | Sub | missense | 0.31 | . |
| PD9067a | PD9067b | 2549687689 | 17 | 12016583 | GT | G | MAP2K4 | CCDS11162.1 | r.783delU | c.720delT | p.S240fs*36 | Del | frameshift | . | 0.148936 |
| PD11339a | PD11339b | 1211868321 | 17 | 12032605 | G | T | MAP2K4 | CCDS11162.1 | r.1103+1g>u | c.1040+1G>T | p.? | Sub | ess splice | 0.9 | . |
| PD8982a | PD8982b | 1517874520 | 5 | 56111421 | T | TC | MAP3K1 | CCDS43318.1 | r.21_22insc | c.21_22insC | p.R8fs*16 | Ins | frameshift | . | 0.19565217 |
| PD7202a | PD7202b | 2135097917 | 5 | 56111785 | GCGGCGCCCGACAGCGGCGCC | G | MAP3K1 | CCDS43318.1 | r.386_404del19 | c.386_404del19 | p.A130fs*49 | Del | frameshift | . | 0.17948718 |
| PD5956a | PD5956b | 1606467257 | 5 | 56155714 | TAA | T | MAP3K1 | CCDS43318.1 | r.807_808delAA | c.807_808delAA | p.R270fs*30 | Del | frameshift | . | 0.47727273 |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4605a | PD4605b | 1655034490 | 5 | 561557-20 | GAA | G | MAP3-K1 | CCDS43318.1 | r.813_814delAA | c.813_814delAA | p.R273fs*27 | Del | frame-shift | . | 0.263157-89 |
| PD18247a | PD18247b | 2452585970 | 5 | 561606-16 | CA | C | MAP3-K1 | CCDS43318.1 | r.891delA | c.891delA | p.P298fs*14 | Del | frame-shift | . | 0.148936-17 |
| PD13427a | PD13427b | 1677613489 | 5 | 561606-36 | A | ACAAACCGC | MAP3-K1 | CCDS43318.1 | r.910_911ins caaaccac | c.910_911ins CAAACCGC | p.R307fs*8 | Ins | frameshift | . | 0.2 |
| PD11740a | PD11740b | 2022184858 | 5 | 561606-63 | C | CA | MAP3-K1 | CCDS43318.1 | r.937_938insa | c.937_938insA | p.R313fs*13 | Ins | frame-shift | . | 0.365853-66 |
| PD6016a2 | PD6016b | 1634239916 | 5 | 561606-74 | GTACT | G | MAP3-K1 | CCDS43318.1 | r.949_952del UACU | c.949_952del TACT | p.L318fs*4 | Del | frame-shift | . | 0.181818-18 |
| PD11394a | PD11394b | 1611471410 | 5 | 561606-95 | G | GT | MAP3-K1 | CCDS43318.1 | r.969_970insu | c.969_970insT | p.P324fs*2 | Ins | frame-shift | . | 0.326530-61 |
| PD24302a | PD24302b | 2484931733 | 5 | 561607-48 | T | TG | MAP3-K1 | CCDS43318.1 | r.1022_102-3insg | c.1022_1023in-sG | p.F341fs*44 | Ins | frameshift | . | 0.15 |
| PD11394a | PD11394b | 1611471411 | 5 | 5616119-5 | GTATT | G | MAP3-K1 | CCDS43318.1 | r.1065_106-8del UAUU | c.1065_1068del TATT | p.H357fs*4 | Del | frame-shift | . | 0.340909-09 |
| PD3851a | PD3851b | 2068615756 | 5 | 561612-30 | C | T | MAP3-K1 | CCDS43318.1 | r.1099c>u | c.1099C>T | p.Q367* | Sub | nonsense | 0.51 | . |
| PD5961a | PD5961b | 1530637228 | 5 | 561678-03 | T | TA | MAP3-K1 | CCDS43318.1 | r.1368_136-9insa | c.1368_1369in-sA | p.T457fs*4 | Ins | frame-shift | . | 0.191489-36 |
| PD18776a | PD18776b | 2517339991 | 5 | 561678-03 | T | TA | MAP3-K1 | CCDS43318.1 | r.1368_136-9insa | c.1368_1369in-sA | p.T457fs*4 | Ins | frame-shift | . | 0.176470-59 |
| PD7201a | PD7201b | 1697092721 | 5 | 561684-71 | CAGAAGAGTGT | C | MAP3-K1 | CCDS43318.1 | r.1428_143-7de IAGAA-GAGUGU | c.1428_1437del AGAAGAGTGT | p.C479fs*5 | Del | frameshift | . | 0.2 |
| PD9570a | PD9570b | 1598169622 | 5 | 561709-36 | C | CCATGATGT | MAP3-K1 | CCDS43318.1 | r.1764_176-5ins cauaauau | c.1764_1765ins CATGATGT | p.S592fs*67 | Ins | frame-shift | . | 0.166666-67 |

| Sample | Normal | VariantID | Chr om | Position | Reference | Mutant | Gene | Transcript | RNA | CDS | Protein | Type | Effect | Proportion_ Mutant _Reads _Subs | Proportion _Mutant _Reads _Indels |
|---|---|---|---|---|---|---|---|---|---|---|---|---|---|---|---|
| PD9606a | PD9606b | 1649876339 | 5 | 561710-35 | GTCTTCC-CA | G | MAP3-K1 | CCDS43318.1 | r.1864_187-1del UCUUCC-CA | c.1864_1871del TCTTCCCA | p.S622fs*21 | Del | frame-shift | . | 0.324324-32 |
| PD13419a | PD13419b | 1690351759 | 5 | 561710-39 | TC | T | MAP3-K1 | CCDS43318.1 | r.1868delC | c.1868delC | p.Q624f-s*32 | Del | frame-shift | . | 0.314285-71 |
| PD13307a | PD13307b | 1624857351 | 5 | 561710-66 | GTGGAG GCATGCT GCAGCGT TCT | GCAT GCC | MAP3-K1 | CCDS43318.1 | r.1895_191-6del22 in-scauacc | c.1895_1916d-el22 in-sCATGCC | p.V632fs*19 | Complex | frame-shift | . | 0.181818-18 |
| PD11756a | PD11756b | 1507145260 | 5 | 561710-69 | G | T | MAP3-K1 | CCDS43318.1 | r.1897a>u | c.1897G>T | p.E633* | Sub | nonsense | 0.21 | . |
| PD4225a | PD4225b | 2253030224 | 5 | 561710-91 | C | A | MAP3-K1 | CCDS43318.1 | r.1919c>a | c.1919C>A | p.S640* | Sub | nonsense | 0.24 | . |
| PD11359a | PD11359b | 1709539727 | 5 | 5617112-3 | T | TA | MAP3-K1 | CCDS43318.1 | r.1951_195-2insa | c.1951_1952in-sA | pY651fs*1 | Ins | frame-shift | . | 0.689655-17 |
| PD13755a | PD13755b | 1839326759 | 5 | 561749-27 | A | AG | MAP3-K1 | CCDS43318.1 | r.2086_208-7insg | c.2086_2087in-sG | p.S696fs*40 | Ins | frame-shift | . | 0.285714-29 |
| PD13619a | PD13619b | 1832870557 | 5 | 561769-75 | AAC | A | MAP3-K1 | CCDS43318.1 | r.2246 2247delAC | c.2246 2247delAC | p.N749fs*14 | Del | frame-shift | . | 0.292682-93 |
| PD9842a | PD9842b | 1311919668 | 5 | 561770-17 | A | T | MAP3-K1 | CCDS43318.1 | r.2287a>u | c.2287A>T | p.R763* | Sub | nonsense | 0.33 | . |
| PD8828a | PD8828b | 1672733083 | 5 | 561770-53 | CAT | C | MAP3-K1 | CCDS43318.1 | r.2324_232-5delAU | c.2324_2325d-elAT | p.I776fs*4 | Del | frame-shift | . | 0.128205-13 |
| PD13755a | PD13755b | 1839326761 | 5 | 561775-63 | TC | T | MAP3-K1 | CCDS43318.1 | r.2537delC | c.2537delC | p.T847fs*10 | Del | frameshift | . | 0.1372549 |
| PD5961a | PD5961b | 1530637230 | 5 | 561776-54 | CT | C | MAP3-K1 | CCDS43318.1 | r.2628delU | c.2628delT | p.L877fs*32 | Del | frame-shift | . | 0.234042-55 |

| Sample | Normal | VariantID | Chr om | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD13768a | PD13768b | 2445129566 | 5 | 561776-85 | GGCATCT-GTTC | G | MAP3-K1 | CCDS43318.1 | r.2659_266-8del GCAU-CUGUUC | c.2659_2668del GCATCTGTTC | pA887fs*19 | Del | frame-shift | . | 0.169811-32 |
| PD6046a | PD6046b | 1639971704 | 5 | 561777-62 | GA | G | MAP3-K1 | CCDS43318.1 | r.2736delA | c.2736delA | p.G914fs*7 | Del | frame-shift | . | 0.313432-84 |
| PD9574a | PD9574b | 1591041282 | 5 | 561777-84 | A | AT | MAP3-K1 | CCDS43318.1 | r.2757_275-8insu | c.2757 2758insT | p.L920fs*10 | Ins | frame-shift | . | 0.346938-78 |
| PD9606a | PD9606b | 1649876343 | 5 | 561782-57 | C | CA | MAP3-K1 | CCDS43318.1 | r.3230_323-1insa | c.3230 3231insA | p.N1079fs*2 | Ins | frame-shift | . | 0.304347-83 |
| PD6418a | PD6418b | 1761200038 | 5 | 561784-33 | CT | C | MAP3-K1 | CCDS43318.1 | r.3407delU | c.3407delT | p.E1137f-s*11 | Del | frame-shift | . | 0.357142-86 |
| PD18247a | PD18247b | 2452585975 | 5 | 561784-74 | TA | T | MAP3-K1 | CCDS43318.1 | r.3448delA | c.3448delA | p.K1150f-s*22 | Del | frame-shift | . | 0.125 |
| PD4266a | PD4266b | 1731822311 | 5 | 561785-28 | CAAAGATG ATGTGA | C | MAP3-K1 | CCDS43318.1 | r.3502_351-4del AAA-GAUGAU-GUGA | c.3502_3514del AAAGAT-GATGTGA | p.K1168f-s*18 | Del | frame-shift | . | 0.1369863 |
| PD11756a | PD11756b | 1491391835 | 5 | 561786-72 | TATCATCATT ATTCAACA | T | MAP3-K1 | CCDS43318.1 | r.3646_366-2del17 | c.3646_3662d-el17 | p.I1216f-s*17 | Del | frame-shift | . | 0.153846-15 |
| PD13623a | PD13623b | 1483544710 | 5 | 561794-38 | C | CT | MAP3-K1 | CCDS43318.1 | r.3751_375-2insu | c.3751_3752in-sT | p.G1252f-s*21 | Ins | frame-shift | . | 0.267857-14 |
| PD11343a | PD11343b | 2556047957 | 5 | 561795-00 | T | TA | MAP3-K1 | CCDS43318.1 | r.3813_381-4insa | c.3813_3814in-sA | p.Q1273f-s*11 | Ins | frame-shift | . | 0.222222-22 |
| PD13768a | PD13768b | 2445129568 | 5 | 561805-50 | AGAGAT | A | MAP3-K1 | CCDS43318.1 | r.3880_388-4del GA-GAU | c.3880_3884del GAGAT | p.E1294f-s*13 | Del | frame-shift | . | 0.18 |
| PD18776a | PD18776b | 2525053934 | 5 | 561806-14 | G | T | MAP3-K1 | CCDS43318.1 | r.3943a>u | c.3943G>T | pE1315" | Sub | non-sense | 0.23 | . |

(continued)

| Sample | Normal | VariantID | Chr om | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD9574a | PD9574b | 1591041285 | 5 | 561818-44 | C | CCT TTC GTA TCT | MAP3-K1 | CCDS43318.1 | r.4068_406-9ins cuuu-cauaucu | c.4068_4069ins CTTTCGTATCT | p.H1361f-s*20 | Ins | frame-shift | . | 0.25 |
| PD17994a | PD17994b | 1823240809 | 5 | 561832-34 | C | T | MAP3-K1 | CCDS43318.1 | r.4144c>u | c.4144C>T | p.Q1382* | Sub | nonsense | 0.19 | . |
| PD13619a | PD13619b | 1832870560 | 5 | 561832-41 | TA | T | MAP3-K1 | CCDS43318.1 | r.4152delA | c.4152delA | p.R1385f-s*37 | Del | frame-shift | . | 0.292307-69 |
| PD13427a | PD13427b | 1677613496 | 5 | 561833-30 | GC | G | MAP3-K1 | CCDS43318.1 | r.4241delC | c.4241delC | p.A1414fs*8 | Del | frame-shift | . | 0.173913-04 |
| PD13618a | PD13618b | 1829787035 | 5 | 561833-46 | A | AG | MAP3-K1 | CCDS43318.1 | r.4256_425-7insg | c.4256_4257in-sG | p.V1420f-s*12 | Ins | frame-shift | . | 0.193548-39 |
| PD13618a | PD13618b | 1829787040 | 5 | 561894-15 | G | GA | MAP3-K1 | CCDS43318.1 | r.4447_444-8insa | c.4447_4448in-sA | p.D1483f-s*40 | Ins | frame-shift | . | 0.346153-85 |
| PD4977a | PD4977b | 1937107384 | 5 | 561894-34 | TAGAACTTCAA CCTCAGGACAG ACCTCCATCA | T | MAP3-K1 | CCDS43318.1 | r.4467_449-7del31 | c.4467_4497d-el31 | p.L1491fs*3 | Del | frame-shift | . | 0.157894-74 |
| PD24307a | PD24307b | 2485794960 | 6 | 131908-954 | AG | A | MED23 | CCDS5147.1 | r.4151delC | c.3971delC | p.A1324fs*2 | Del | frame-shift | . | 0.313725-49 |
| PD18116a | PD18116b | 2525238089 | 6 | 131917-741 | G | A | MED23 | CCDS5147.1 | r.2875c>u | c.2695C>T | p.R899* | Sub | nonsense | 0.41 | . |
| PD7238a | PD7238b | 1599949028 | 6 | 131919-439 | TA | T | MED23 | CCDS5147.1 | r.2774delU | c.2594delT | p.L865fs*1 | Del | frameshift | . | 0.5 |
| PD23564a | PD23564b | 2337826710 | 6 | 131919-845 | AT | A | MED23 | CCDS5147.1 | r.2456delA | c.2276delA | p.N759fs*12 | Del | frame-shift | . | 0.222222-22 |
| PD24216a | PD24216b | 2484222814 | 6 | 131923-421 | C | CTT | MED23 | CCDS5147.1 | r.2211_221-2insaa | c.2031_2032in-sAA | p.A678fs*7 | Ins | frame-shift | . | 0.529411-76 |
| PD11762a | PD11762b | 1333921256 | 6 | 131927-766 | T | C | MED23 | CCDS5147.1 | r.1402-2a>a | c.1222-2A>G | p.? | Sub | ess splice | 0.3 | . |
| PD9578a | PD9578b | 2537098423 | 6 | 131931-387 | C | A | MED23 | CCDS5147.1 | r.1057-1a>u | c.877-1G>T | p.? | Sub | ess splice | 0.24 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD7322a | PD7322b | 2488445550 | 11 | 645737-30 | CCAGGCC-TG | C | MEN1 | CCDS8083.1 | r.1534_154-1del CAGGC-CUG | c.1030_1037d-e1 CAGGCCTG | p.Q344f-s*25 | Del | frame-shift | . | 0.476191-76 |
| PD24193a | PD24193b | 2398176351 | 3 | 370903-94 | G | C | MLH1 | CCDS2663.1 | r.2206-1a>c | c.1990-1G>C | p.? | Sub | ess splice | 0.3 | . |
| PD6412a | PD6412b | 1777477609 | 3 | 370908-96 | AAAACAAGGCTC TATAACTCCTCTT ACAGGTCAGCT TGACACAAAAA GGGTGCCTGG ATTCCTAATGT TTCATTGTCAC TTTTCCCAGTC AGATGATAATG | A | MLH1 | CCDS2663.1 | r.2319+389-_2409 del1170 | c.2103+389_2-193 del1170 | p.? | Del | ess splice | . | 0.142857-14 |

(continued)

| Sample | Normal | VariantID | Chrom | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| | | | | | CTTTTCAAATC AACATATATTT TGGGGGAGGT TGGAAGGGAG AGTTGAAATAT TCTAAGAATCA AAGAGTAGCCC ACTTAATCAGA GTATGACCCCT GATTGCTCACA GTCATCTCCTG AGCAGTGTGAG CGAGTTTCAGA TGAGGAGGCTG AAGGCCAGTCA GGCATGCTCGA GGATTCCAAG TCTGTAGGTG GGAGGGCAG AGATTTAGTC CTGTTGGCCA AAGCCTCTAG GGAATTTCTC ACTCCAGTGG AGAAGGCAAC ACACTTACCAA ACTGTGTGGAA ACTATCTCATT TGATTAGAAAT TTTACCTCCAG AAGAGGAAGG ACAGTTGAGA AAGAACATTTT CTTACACATGA GACAGCTAAGG CTTACAAGAAG GAGAGGAATAA TGAGGCAAAAT AATCCTCATTAA TATTTTCATTCC TCCCCTGGGGA TTAGAACTACTT TCAGACCCGAT TTTAATGGTAAG TTAGGTACTTCC TACAGTTGCCAT CCAAATATCAGT CAGGATCAGACA TGATGTTAGCTC CTGCTACAATAA AACCATTTTCTC CCTGAATGAAAA CAAAGGTTCCAC AGGAGACAGTC CCACAGAGCAG TGGCTTCTTTTC CTCCCTTTAAA ACCTCATGTTG GCTGGCACAG TGGCTCACACC TGTAATCCCAG CATTTTAGGAG GCTGAGGTGG | | | | | | | | | | |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| | | | | | GAAGATGGCTT AAGCCCAGGAG TTTGAGGCTGTA GAGCTATGATCA CACCACTGCCCT TCAGCCTGGGTG ACAGAGCAAGAC CTTGTCTCTAAAT AAACAAACAAAC AAAAAATCCTCT TGTGTTCAGGC CTGTGGGATCC CCTGAGAGGCT AGCCCACAAGA TCCACTTCAAAA GCCCTAGATAAC ACCAAGTCTTTC CAGACCCAGTG CACATCCCATCA GCCAGGACACC AGTGTATGTTGG GATGCAAACAGG GAGGCTTATGAC ATCTAATGTGTTT TCCAGAGTGAAG TGCCTGGCTCCA TTCCAAACTCCT GGAAGTGGACT GTGGAACACAT TGTCTATAAAGC CTTGCGCTCACA CATTCTGCCTCCT | | | | | | | | | | |
| PD4109a | PD4109b | 1822262560 | 12 | 494158-46 | G | A | MLL2 | CCDS44873.1 | r.16501c>u | c.16501C>T | p.R5501* | Sub | nonsense | 0.44 | . |
| PD6412a | PD6412b | 1777056254 | 12 | 494226-31 | CTG | C | MLL2 | CCDS44873.1 | r.14360_14-361del CA | c.14360_1436-1del CA | p.T4787f-s*29 | Del | frame-shift | . | 0.272727-27 |
| PD4006a | PD4006b | 2119618377 | 12 | 494226-56 | TCCTTTGC | T | MLL2 | CCDS44873.1 | r.14330_14-336del GCAAAGG | c.14330_1433-6del GCAAAGG | p.G4777f-s*18 | Del | frame-shift | . | 0.318181-82 |
| PD6412a | PD6412b | 1777056256 | 12 | 494262-21 | CAG | C | MLL2 | CCDS44873.1 | r.12265_12-266del CU | c.12265_1226-6del CT | p.L4089f-s*17 | Del | frame-shift | . | 0.246376-81 |
| PD13754a | PD13754b | 2447661474 | 12 | 494365-23 | C | A | MLL2 | CCDS44873.1 | r.5782+1g>-u | c.5782+1G>T | p.? | Sub | ess splice | 0.19 | . |
| PD11389a | PD11389b | 1675990483 | 7 | 151845-466 | ATTCTTG CTCATG AATTCCC | A | MLL3 | CCDS5931.1 | r.13746_13-764del 19 | c.13527_1354-5del 19 | p.4509fs*2 | Del | frame-shift | . | 0.288135-59 |
| PD13771a | PD13771b | 1837612036 | 7 | 151845-485 | CTTTGGT TTGTGCA TGGGGC AAAGCATA GT | C | MLL3 | CCDS5931.1 | r.13717_13-745del 29 | c.13498_1352-6de1 29 | p.T4500fs*4 | Del | frame-shift | . | 0.258064-52 |

142

EP 3 452 937 B1

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18733a | PD18733b | 2446255919 | 7 | 151845958 | AT | A | MLL3 | CCDS5931.1 | r.13272delA | c.13053delA | p.K4351fs*5 | Del | frameshift | . | 0.21428571 |
| PD7249a | PD7249b | 1254027997 | 7 | 151846177 | G | A | MLL3 | CCDS5931.1 | r.13054c>u | c.12835C>T | p.Q4279* | Sub | nonsense | 0.64 | . |
| PD7217a | PD7217b | 2258889061 | 7 | 151856024 | G | C | MLL3 | CCDS5931.1 | r.11813c>a | c.11594C>G | p.S3865* | Sub | nonsense | 0.19 | . |
| PD7210a | PD7210b | 2260972487 | 7 | 151860212 | G | A | MLL3 | CCDS5931.1 | r.10669c>u | c.10450C>T | p.Q3484* | Sub | nonsense | 0.5 | . |
| PD5937a | PD5937b | 1250625858 | 7 | 151860728 | G | A | MLL3 | CCDS5931.1 | r.10153c>u | c.9934C>T | p.Q3312* | Sub | nonsense | 0.33 | . |
| PD24333a | PD24333b | 2548399734 | 7 | 151860863 | T | TC | MLL3 | CCDS5931.1 | r.10017_10-018insg | c.9798_9799in-sG | p.13267f-s*59 | Ins | frame-shift | . | 0.51351351 |
| PD13753a | PD13753b | 2441473908 | 7 | 151864252 | CATGCTTTGCTGTTCAG | C | MLL3 | CCDS5931.1 | r.9932_994-7del16 | c.9713_9728d-el16 | p.T3238fs*6 | Del | frameshift | . | 0.10344828 |
| PD13758a | PD13758b | 2433670250 | 7 | 151873725 | GGAGACCCCGATGGCCT | G | MLL3 | CCDS5931.1 | r.9016_903-1del16 | c.8797_8812d-el16 | p.R2933f-s*21 | Del | frame-shift | . | 0.23809524 |
| PD13425a | PD13425b | 1319995214 | 7 | 151873749 | G | C | MLL3 | CCDS5931.1 | r.9008c>a | c.8789C>G | p.S2930* | Sub | nonsense | 0.15 | . |
| PD18756a | PD18756b | 2537429743 | 7 | 151873888 | G | A | MLL3 | CCDS5931.1 | r.8869c>u | c.8650C>T | pR2884" | Sub | nonsense | 0.18 | . |
| PD6414a | PD6414b | 1753177726 | 7 | 151874147 | CT | C | MLL3 | CCDS5931.1 | r.8609delA | c.8390delA | p.K2797f-s*26 | Del | frame-shift | . | 0.24193548 |
| PD6412a | PD6412b | 1777798198 | 7 | 151874147 | CT | C | MLL3 | CCDS5931.1 | r.8609delA | c.8390delA | p.K2797f-s*26 | Del | frame-shift | . | 0.16326531 |
| PD23564a | PD23564b | 2337913466 | 7 | 151874147 | CT | C | MLL3 | CCDS5931.1 | r.8609delA | c.8390delA | p.K2797f-s*26 | Del | frame-shift | . | 0.31147541 |
| PD13762a | PD13762b | 1494848206 | 7 | 151875059 | CG | C | MLL3 | CCDS5931.1 | r.7697delC | c.7478delC | p.P2493f-s*22 | Del | frame-shift | . | 0.26190476 |

(continued)

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD4103a | PD4103b | 2057044308 | 7 | 151876-918 | C | T | MLL3 | CCDS5931.1 | r.7661+1g>-a | c.7442+1G>A | p.? | Sub | ess splice | 0.2 | . |
| PD11344a | PD11344b | 2184328784 | 7 | 151878-287 | G | A | MLL3 | CCDS5931.1 | r.6877c>u | c.6658C>T | p.Q2220* | Sub | nonsense | 0.35 | . |
| PD24204a | PD24204b | 2385784160 | 7 | 151878-863 | G | A | MLL3 | CCDS5931.1 | r.6301c>u | c.6082C>T | p.R2028* | Sub | nonsense | 0.41 | . |
| PD9541a | PD9541b | 1717260176 | 7 | 151879-253 | ATGGAT CCATT | A | MLL3 | CCDS5931.1 | r.5901_591-0del AAUG-GAUCCA | c.5682_5691 del AATG-GATCCA | p.D1896f-s*40 | Del | frame-shift | . | 0.145833-33 |
| PD13304a | PD13304b | 1567586554 | 7 | 151879-521 | ACT | A | MLL3 | CCDS5931.1 | r.5641_564-2del AG | c.5422_5423del AG | p.P1809fs*8 | Del | frameshift | . | 0.25 |
| PD11372a | PD11372b | 1213257244 | 7 | 151884-502 | G | C | MLL3 | CCDS5931.1 | r.5072c>a | c.4853C>G | pS1618* | Sub | nonsense | 0.5 | . |
| PD24204a | PD24204b | 2383123323 | 7 | 151891-329 | A | AT | MLL3 | CCDS5931.1 | r.4743_474-4insa | c.4524_4525in-sA | p.L1509fs*3 | Ins | frameshift | . | 0.35 |
| PD10011a | PD10011b | 2509394558 | 7 | 151932-901 | C | A | MLL3 | CCDS5931.1 | r.2988+1g>-u | c.2769+1G>T | p.? | Sub | ess splice | 0.25 | . |
| PD11402a | PD11402b | 1693813298 | 7 | 151932-992 | CT | C | MLL3 | CCDS5931.1 | r.2897delA | c.2678delA | p.K893fs*20 | Del | frameshift | . | 0.1971831 |
| PD4981a | PD4981b | 2248721442 | 7 | 151945-226 | C | A | MLL3 | CCDS5931.1 | r.2512a>u | c.2293G>T | p.E765* | Sub | nonsense | 0.2 | . |
| PD11352a | PD11352b | 1692883999 | 7 | 151945-631 | TTTTCAG GTCTTCA CTATCAAC TTCA | T | MLL3 | CCDS5931.1 | r.2082_210-6del 25 | c.1863_1887del 25 | p. N621 fs*6 | Del | frameshift | . | 0.4 |
| PD22364a | PD22364b | 2316515928 | 7 | 151946-974 | ACTAT | A | MLL3 | CCDS5931.1 | r.2015_201-8del AUAG | c.1796_1799del ATAG | p.D599fs*12 | Del | frameshift | . | 0.66 |
| PD11336a | PD11336b | 2415367073 | 7 | 151949-719 | G | A | MLL3 | CCDS5931.1 | r.1600c>u | c.1381C>T | p.Q461* | Sub | non-sense | 0.19 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD9589a | PD9589b | 1601577255 | 7 | 151949-738 | C | CA | MLL3 | CCDS5931.1 | r.1580_158-1insu | c.1361_1362in-sT | p.I455fs*3 | Ins | frameshift | . | 0.16 |
| PD23561a | PD23561b | 2403470640 | 7 | 151949-758 | G | A | MLL3 | CCDS5931.1 | r.1561c>u | c.1342C>T | p.Q448* | Sub | nonsense | 0.27 | . |
| PD18046a | PD18046b | 2119392391 | 7 | 151960-177 | G | GT | MLL3 | CCDS5931.1 | r.1441_144-2insa | c.1222_1223in-sA | p.T408fs*3 | Ins | frame-shift | . | 0.363636-36 |
| PD9064a | PD9064b | 2304507523 | 7 | 151960-206 | TC | T | MLL3 | CCDS5931.1 | r.1412delG | c.1193delG | p.G398fs*7 | Del | frame-shift | . | 0.773809-52 |
| PD13298a | PD13298b | 1552558893 | 7 | 152007-138 | AC | A | MLL3 | CCDS5931.1 | r.980delG | c.761delG | p.R254fs*7 | Del | frameshift | . | 0.1627907 |
| PD5944a | PD5944b | 1553363374 | 7 | 152008-968 | AT | A | MLL3 | CCDS5931.1 | r.872delA | c.653delA | p.D218fs*43 | Del | frame-shift | . | 0.358974-36 |
| PD6412a | PD6412b | 1777798374 | 7 | 152012-385 | CT | C | MLL3 | CCDS5931.1 | r.646delA | c.427delA | p.S143fs*3 | Del | frame-shift | . | 0.133333-33 |
| PD4967a | PD4967b | 1605759060 | 6 | 1682811-25 | CT | C | MLLT4 | CCDS47516.1 | r.823delU | c.823delT | p.F275fs*7 | Del | frame-shift | . | 0.103448-28 |
| PD4005a | PD4005b | 2068839597 | 6 | 168297-599 | C | T | MLLT4 | CCDS47516.1 | r.1261c>u | c.1261C>T | p.Q421* | Sub | nonsense | 0.12 | . |
| PD9575a | PD9575b | 1854497042 | 6 | 168303-032 | ATATTC | A | MLLT4 | CCDS47516.1 | r.1614_161-8del UAUUC | c.1614_1618del TATTC | p.D538fs*2 | Del | frame-shift | . | 0.212765-96 |
| PD22251a | PD22251b | 2309455075 | 6 | 168307-939 | AGCAGCC AGATTATC | A | MLLT4 | CCDS47516.1 | r.1737_175-0del GCAGCCA-GAUUAUC | c1737 _1750del GCAGCCA-GATTATC | p.P581fs*12 | Del | frame-shift | . | 0.533333-33 |
| PD14457a | PD14457b | 1834759707 | 6 | 168315-877 | A | AT | MLLT4 | CCDS47516.1 | r.2305_230-6insu | c.2305_2306in-sT | p.M769f-s*32 | Ins | frame-shift | . | 0.461538-46 |
| PD13771a | PD13771b | 1840529124 | 6 | 168315-898 | A | T | MLLT4 | CCDS47516.1 | r.2326a>u | c.2326A>T | p.R776* | Sub | non-sense | 0.57 | . |

EP 3 452 937 B1

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD24189a | PD24189b | 2489981099 | 6 | 168325-728 | TA | T | MLLT4 | CCDS47516.1 | r.3033delA | c.3033delA | p.K1013fs*20 | Del | frame-shift | . | 0.232558-14 |
| PD6043a | PD6043b | 1738927689 | 6 | 168347-439 | GCTCTATAATAAT | GC | MLLT4 | CCDS47516.1 | r.3388_3399del CU-CUAUAAU-AAUinsc | c.3388_3399del CTCTATAATAA-TinsC | p.Y1131fs*7 | Complex | frame-shift | . | 0.629032-26 |
| PD5937a | PD5937b | 1250512507 | 2 | 476435-01 | C | T | MSH2 | CCDS1834.1 | r.1232c>u | c.1009C>T | p.Q337* | Sub | nonsense | 0.2 | . |
| PD5937a | PD5937b | 1250512511 | 2 | 476902-01 | C | A | MSH2 | CCDS1834.1 | r.1641c>a | c.1418C>A | p.S473* | Sub | nonsense | 0.33 | . |
| PD22251a | PD22251b | 2309865276 | 17 | 159612-68 | G | A | NCOR1 | CCDS11175.1 | r.6379c>u | c.6121C>T | p.Q2041* | Sub | nonsense | 0.34 | . |
| PD11740a | PD11740b | 2022559666 | 17 | 159747-91 | G | A | NCOR1 | CCDS11175.1 | r.4342c>u | c.4084C>T | p.Q1362* | Sub | nonsense | 0.38 | . |
| PD4072a | PD4072b | 2256065600 | 17 | 159832-80 | G | A | NCOR1 | CCDS11175.1 | r.3757c>u | c.3499C>T | p.Q1167* | Sub | nonsense | 0.25 | . |
| PD5961a | PD5961b | 2251448110 | 17 | 159839-36 | C | A | NCOR1 | CCDS11175.1 | r.3541a>u | c.3283G>T | p E1095* | Sub | nonsense | 0.21 | . |
| PD24217a | PD24217b | 2503187790 | 17 | 160045-95 | C | A | NCOR1 | CCDS11175.1 | r.2917a>u | c.2659G>T | p.E887* | Sub | nonsense | 0.18 | . |
| PD11745a | PD11745b | 2455019441 | 17 | 160227-72 | CG | C | NCOR1 | CCDS11175.1 | r.2137delC | c.1879delC | p.R627fs*14 | Del | frame-shift | . | 0.208333-33 |
| PD3945a | PD3945b | 2153219479 | 17 | 160243-80 | GGTGGCAGAGGTGGTGGGGGCTCTTCAGTAGCCGCTGCGGCTGCAGCA | G | NCOR1 | CCDS11175.1 | r.2049_209-5del47 | c.1791_1837d-el47 | p.A599fs*23 | Del | frame-shift | . | 0.416666-67 |
| PD18047a | PD18047b | 2128605790 | 17 | 160294-27 | C | A | NCOR1 | CCDS11175.1 | r.1861a>u | c.1603G>T | p.E535* | Sub | nonsense | 0.45 | . |
| PD13625a | PD13625b | 1813496697 | 17 | 160294-45 | C | CT | NCOR1 | CCDS11175.1 | r.1842_184-3insa | c.1584_1585in-sA | p.E529fs*7 | Ins | frame-shift | . | 0.46 |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD24320a | PD24320b | 2560606548 | 17 | 160294-45 | CT | C | NCOR1 | CCDS11175.1 | r.1842delA | c.1584delA | p.E529fs*26 | Del | frameshift | . | 0.4516129 |
| PD24215a | PD24215b | 2495487000 | 17 | 160294-52 | TTC | T | NCOR1 | CCDS11175.1 | r.1834_183-5delGA | c.1576_1577d-elGA | p.E526fs*9 | Del | frameshift | . | 0.4375 |
| PD4605a | PD4605b | 1654757951 | 17 | 160294-56 | G | GT | NCOR1 | CCDS11175.1 | r.1831_183-2insa | c.1573_1574in-sA | p.T525fs*11 | Ins | frame-shift | . | 0.382352-94 |
| PD23564a | PD23564b | 2337299249 | 17 | 294860-49 | GA | G | NF1 | CCDS42292.1 | r.610delA | c.227delA | p.N78fs*7 | Del | frame-shift | . | 0.483870-97 |
| PD13163a | PD13163b | 2184415564 | 17 | 295274-39 | G | C | NF1 | CCDS42292.1 | r.1272-1a>c | c.889-1G>C | p.? | Sub | ess splice | 0.56 | . |
| PD24202a | PD24202b | 2397394526 | 17 | 295505-05 | C | T | NF1 | CCDS42292.1 | r.2148c>u | c.1765C>T | p.Q589* | Sub | nonsense | 0.15 | . |
| PD4976a | PD4976b | 2254485687 | 17 | 295572-77 | G | T | NF1 | CCDS42292.1 | r.3374-1a>u | c.2991-1G>T | p.? | Sub | ess splice | 0.86 | . |
| PD7426a | PD7426b | 1852765322 | 17 | 295761-34 | C | A | NF1 | CCDS42292.1 | r.4490c>a | c.4107C>A | pY1369* | Sub | nonsense | 0.67 | . |
| PD22036a | PD22036b | 2417067498 | 17 | 295860-92 | G | T | NF1 | CCDS42292.1 | r.4758a>u | c.4375G>T | p.E1459* | Sub | nonsense | 0.24 | . |
| PD6719a | PD6719b | 1772476469 | 17 | 296529-22 | AATTGT | A | NF1 | CCDS42292.1 | r.5304_530-8del AUU-GU | c.4921_4925del ATTGT | p.I641fs*9 | Del | frame-shift | . | 0.333333-33 |
| PD4605a | PD4605b | 1654760964 | 17 | 296619-16 | G | GT | NF1 | CCDS42292.1 | r.6256_625-7insu | c.5873_5874in-sT | p.C1960fs*4 | Ins | frame-shift | . | 0.322580-65 |
| PD7240a | PD7240b | 2509081572 | 17 | 296772-27 | C | T | NF1 | CCDS42292.1 | r.7731c>u | c.7348C>T | p.R2450* | Sub | nonsense | 0.4 | . |
| PD5934a | PD5934b | 2253379616 | 22 | 300352-02 | G | T | NF2 | CCDS13861.1 | r.804+1g>u | c.363+1G>T | p.? | Sub | ess splice | 0.21 | . |
| PD9568a | PD9568b | 1853598783 | 9 | 139390-935 | AG | A | NOTC-H1 | CCDS43905.1 | r.7331delC | c.7255delC | p.G2420f-s*2 | Del | frame-shift | . | 0.714285-71 |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4315a | PD4315b | 17301740 60 | 9 | 139397- 631 | T | TA | NOTC- H1 | CCDS43905.1 | r.5243+2_4- 3+3insu | c.5167+2_516- 7+3insT | p.? | Ins | ess splice | . | 0.46875 |
| PD23564a | PD23564b | 2336922284 | 1 | 120458- 084 | ACT | A | NOTC- H2 | CCDS908.1 | r.7479_748- 0del AG | c.7259_7260del AG | p.E2420fs*3 | Del | frame- shift | . | 0.347826- 09 |
| PD5950a | PD5950b | 1228091733 | 1 | 120458- 396 | G | A | NOTC- H2 | CCDS908.1 | r.7169c>u | c.6949C>T | p.Q2317* | Sub | nonsense | 0.2 | . |
| PD8619a | PD8619b | 1535602193 | 1 | 120458- 477 | C | A | NOTC- H2 | CCDS908.1 | r.7088a>u | c.6868G>T | p.E2290* | Sub | nonsense | 0.61 | . |
| PD13298a | PD13298b | 1216388063 | 1 | 120458- 549 | C | A | NOTC- H2 | CCDS908.1 | r.7016a>u | c.6796G>T | p.E2266* | Sub | nonsense | 0.9 | . |
| PD8618a | PD8618b | 1370648743 | 1 | 120466- 609 | T | G | NOTC- H2 | CCDS908.1 | r.4732-2a>c | c.4512-2A>C | p.? | Sub | ess splice | 0.22 | . |
| PD11345a | PD11345b | 2184677984 | 1 | 120547- 967 | G | A | NOTC- H2 | CCDS908.1 | r.620c>u | c.400C>T | p.Q134* | Sub | nonsense | 0.32 | . |
| PD24212a | PD24212b | 2470487211 | 1 | 1152587- 48 | C | A | NRAS | CCDS877.1 | r.288a>u | c.34G>T | p.G12C | Sub | missense | 0.21 | . |
| PD11340a | PD11340b | 1661363457 | 16 | 236253- 22 | TACC | T | PALB2 | CCDS32406.1 | r.3401_340- 1+2del Gau | c.3201_3201+- 2del Ggt | p.? | Del | ess splice | . | 0.107692- 31 |
| PD24205a | PD24205b | 2397900727 | 16 | 236412- 18 | G | A | PALB2 | CCDS32406.1 | r.2457c>u | c.2257C>T | p.R753* | Sub | nonsense | 0.38 | . |
| PD24212a | PD24212b | 2468541299 | 16 | 236466- 54 | GAA | G | PALB2 | CCDS32406.1 | r.1411_141- 2delUU | c.1211_1212d- elTT | p.F404fs*7 | Del | frame- shift | . | 0.326086- 96 |
| PD8980a | PD8980b | 1721439714 | 3 | 526857- 91 | AG | A | PBRM1 | CCDS2859.1 | r.683delC | c.680delC | p.P227fs*2 | Del | frameshift | . | 0.275 |
| PD9591a | PD9591b | 2377601952 | X | 133512- 118 | TA | T | PHF6 | CCDS14639.1 | r.425delA | c.223delA | p.R76fs*5 | Del | frame- shift | . | 0.179487- 18 |
| PD5930a | PD5930b | 2262004442 | X | 133559- 304 | G | T | PHF6 | CCDS14639.1 | r.1244a>u | c.1042G>T | p.G348* | Sub | non- sense | 0.11 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4970a | PD4970b | 1597314182 | 3 | 178916-919 | TGAACCA | T | PIK3CA | CCDS43171.1 | r.464_469del GAACCA | c307_312del GAACCA | p.E103_-P104 delEP | Del | inframe | . | 0.181818-18 |
| PD11342a | PD11342b | 1551483982 | 3 | 178916-923 | CCAGTAG | C | PIK3CA | CCDS43171.1 | r.468_473del CAGUAG | c.311_316del CAGTAG | p.P104_-G106 delinsR | Del | complex sub | . | 0.425 |
| PD13619a | PD13619b | 1840282349 | 3 | 178916-924 | C | T | PIK3CA | CCDS43171.1 | r.468c>u | c.311C>T | p.P104L | Sub | missense | 0.45 | . |
| PD4967a | PD4967b | 2246447769 | 3 | 178916-924 | C | T | PIK3CA | CCDS43171.1 | r.468c>u | c311C>T | p.P104L | Sub | missense | 0.18 | . |
| PD24320a | PD24320b | 2575255639 | 3 | 178916-924 | C | T | PIK3CA | CCDS43171.1 | r.468c>u | c.311C>T | p.P104L | Sub | missense | 0.43 | . |
| PD4605a | PD4605b | 1654963026 | 3 | 178916-928 | AGG-CAACCGT | A | PIK3CA | CCDS43171.1 | r.473_481del GGCAACCGU | c.316_324del GGCAACCGT | p.G106_-R108 delGNR | Del | inframe | . | 0.3442623 |
| PD13758a | PD13758b | 2441793431 | 3 | 178916-929 | G | C | PIK3CA | CCDS43171.1 | r.473a>c | c.316G>C | p.G106R | Sub | missense | 0.19 | . |
| PD17994a | PD17994b | 1807469530 | 3 | 178916-943 | AAAGATC | A | PIK3CA | CCDS43171.1 | r.488_493del AAGAUC | c.331_336del AAGATC | p.K111_I112 delKI | Del | inframe | . | 0.1 6666667 |
| PD9595a | PD9595b | 1622426192 | 3 | 178916-945 | AGATCCT | A | PIK3CA | CCDS43171.1 | r.490_495del GAUCCU | c.333_338del GATCCT | p.K111_-L113 delinsN | Del | complex sub | . | 0.123076-92 |
| PD4844a | PD4844b3 | 1833620942 | 3 | 178916-949 | CCTCAAT | C | PIK3CA | CCDS43171.1 | r.494_499del CUCAAU | c.337_342del CTCAAT | p.L113_-N114 delLN | Del | inframe | . | 0.237288-14 |
| PD6720a | PD6720b | 2250191994 | 3 | 178917-478 | G | A | PIK3CA | CCDS43171.1 | r.510a>a | c.353G>A | p.G118D | Sub | missense | 0.21 | . |
| PD24329a | PD24329b | 2576137463 | 3 | 178921-548 | G | A | PIK3CA | CCDS43171.1 | r.1187a>a | c.1030G>A | p.V344M | Sub | mis-sense | 0.17 | . |

EP 3 452 937 B1

149

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD11380a | PD11380b | 1222824001 | 3 | 178921-553 | T | A | PIK3CA | CCDS43171.1 | r.1192u>a | c.1035T>A | p.N345K | Sub | missense | 0.35 | . |
| PD11816a | PD11816b | 1315784182 | 3 | 178921-553 | T | A | PIK3CA | CCDS43171.1 | r.1192u>a | c.1035T>A | p. N345K | Sub | missense | 0.36 | . |
| PD13631a | PD13631b | 1507823173 | 3 | 178921-553 | T | A | PIK3CA | CCDS43171.1 | r.1192u>a | c.1035T>A | p. N345K | Sub | missense | 0.4 | . |
| PD11366a | PD11366b | 1508207630 | 3 | 178921-553 | T | A | PIK3CA | CCDS43171.1 | r.1192u>a | c.1035T>A | p. N345K | Sub | missense | 0.47 | . |
| PD14473a | PD14473b | 2055987408 | 3 | 178921-553 | T | A | PIK3CA | CCDS43171.1 | r.1192u>a | c.1035T>A | p.N345K | Sub | missense | 0.3 | . |
| PD18050a | PD18050b | 2187589361 | 3 | 178921-553 | T | A | PIK3CA | CCDS43171.1 | r.1192u>a | c.1035T>A | p. N345K | Sub | missense | 0.44 | . |
| \PD7219a | PD7219b | 2248909400 | 3 | 178921-553 | T | A | PIK3CA | CCDS43171.1 | r.1192u>a | c.1035T>A | p. N345K | Sub | missense | 0.13 | . |
| PD4261a | PD4261b | 2256205229 | 3 | 178921-553 | T | A | PIK3CA | CCDS43171.1 | r.1192u>a | c.1035T>A | p.N345K | Sub | missense | 0.28 | . |
| PD4969a | PD4969b | . | 3 | 178921-553 | T | A | PIK3CA | CCDS43171.1 | r.1192u>a | c.1035T>A | p. N345K | Sub | missense | 0.15 | . |
| PD18247a | PD18247b | 2456887847 | 3 | 178922-324 | G | A | PIK3CA | CCDS43171.1 | r.1250a>a | c.1093G>A | p.E365K | Sub | missense | 0.15 | . |
| PD18047a | PD18047b | 2128653573 | 3 | 178922-328 | C | G | PIK3CA | CCDS43171.1 | r.1254c>a | c.1097C>G | p.P366R | Sub | missense | 0.25 | . |
| PD11372a | PD11372b | 1213234656 | 3 | 178927-980 | T | C | PIK3CA | CCDS43171.1 | r.1415u>c | c.1258T>C | p.C420R | Sub | missense | 0.43 | . |
| PD13425a | PD13425b | 1319940705 | 3 | 178927-980 | T | C | PIK3CA | CCDS43171.1 | r.1415u>c | c.1258T>C | p.C420R | Sub | missense | 0.76 | . |
| PD11465a | PD11465b | 2186512817 | 3 | 178927-980 | T | C | PIK3CA | CCDS43171.1 | r.1415u>c | c.1258T>C | p.C420R | Sub | mis-sense | 0.32 | . |

EP 3 452 937 B1

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD22361a | PD22361b | 2421151247 | 3 | 178927-980 | T | C | PIK3CA | CCDS43171.1 | r.1415u>c | c.1258T>C | p.C420R | Sub | missense | 0.23 | . |
| PD11767a | PD11767b | . | 3 | 178927-980 | T | C | PIK3CA | CCDS43171.1 | r.1415u>c | c.1258T>C | p.C420R | Sub | missense | 0.09 | . |
| PD9844a | PD9844b | . | 3 | 178927-980 | T | C | PIK3CA | CCDS43171.1 | r.1415u>c | c.1258T>C | p.C420R | Sub | missense | 0.44 | . |
| PD13753a | PD13753b | 2441228314 | 3 | 178928-070 | CATGGATTAGAAGATT | C | PIK3CA | CCDS43171.1 | AUGGAUUAGAAGAUU r.1506_1-520del | ATGGATTAGAAGATT c.1349_136-3del | p.H450_-D454 delHGLED | Del | inframe | . | 0.18 |
| PD13626a | PD13626b | 1493477366 | 3 | 178928-073 | GGATTAGAAGATTTGC | G | PIK3CA | CCDS43171.1 | GAUUAGAAGAUUUGC r.1509_1-523del | GATTAGAAGATTTGC c.1352_136-6del | p.G451_-L456 de-linsV | Del | complex sub | . | 0.285714-29 |
| PD4977a | PD4977b | 2259214811 | 3 | 178928-079 | G | A | PIK3CA | CCDS43171.1 | r.1514a>a | c.1357G>A | p.E453K | Sub | missense | 0.41 | . |
| PD24193a | PD24193b | 2398182208 | 3 | 178928-079 | G | A | PIK3CA | CCDS43171.1 | r.1514g>a | c.1357G>A | p.E453K | Sub | missense | 0.12 | . |
| PD9063a | PD9063b | 2544778157 | 3 | 178928-079 | G | A | PIK3CA | CCDS43171.1 | r.1514g>a | c.1357G>A | p.E453K | Sub | missense | 0.19 | . |
| PD23558a | PD23558b | 2342400295 | 3 | 178928-080 | A | T | PIK3CA | CCDS43171.1 | r.1515a>u | c.1358A>T | p.E453V | Sub | missense | 0.12 | . |
| PD13299a | PD13299b | 1212896598 | 3 | 178936-082 | G | A | PIK3CA | CCDS43171.1 | r.1781g>a | c.1624G>A | p.E542K | Sub | missense | 0.38 | . |
| PD11352a | PD11352b | 1239266764 | 3 | 178936-082 | G | A | PIK3CA | CCDS43171.1 | r.1781g>a | c.1624G>A | p.E542K | Sub | missense | 0.55 | . |
| PD11359a | PD11359b | 1310693681 | 3 | 178936-082 | G | A | PIK3CA | CCDS43171.1 | r.1781g>a | c.1624G>A | p.E542K | Sub | missense | 0.42 | . |
| PD13623a | PD13623b | 1508888790 | 3 | 178936-082 | G | A | PIK3CA | CCDS43171.1 | r.1781g>a | c.1624G>A | p.E542K | Sub | mis-sense | 0.28 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD13760a | PD13760b | 1509179039 | 3 | 178936-082 | G | A | PIK3CA | CCDS43171.1 | r.1781g>a | c.1624G>A | p.E542K | Sub | missense | 0.27 | . |
| PD18100a | PD18100b | 1840708988 | 3 | 178936-082 | G | A | PIK3CA | CCDS43171.1 | r.1781g>a | c.1624G>A | p.E542K | Sub | missense | 0.23 | . |
| PD4267a | PD4267b | 2246625034 | 3 | 178936-082 | G | A | PIK3CA | CCDS43171.1 | r.1781g>a | c.1624G>A | p.E542K | Sub | missense | 0.32 | . |
| PD5951a | PD5951b | 2259764509 | 3 | 178936-082 | G | A | PIK3CA | CCDS43171.1 | r.1781g>a | c.1624G>A | p.E542K | Sub | missense | 0.56 | . |
| PD9579a | PD9579b | 2272728899 | 3 | 178936-082 | G | A | PIK3CA | CCDS43171.1 | r.1781g>a | c.1624G>A | p.E542K | Sub | missense | 0.16 | . |
| PD6416a | PD6416b | 2273016967 | 3 | 178936-082 | G | A | PIK3CA | CCDS43171.1 | r.1781g>a | c.1624G>A | p. E542K | Sub | missense | 0.11 | |
| PD13629a | PD13629b | 2376577524 | 3 | 178936-082 | G | A | PIK3CA | CCDS43171.1 | r.1781g>a | c.1624G>A | p.E542K | Sub | missense | 0.42 | . |
| PD23561a | PD23561b | 2403416368 | 3 | 178936-082 | G | A | PIK3CA | CCDS43171.1 | r.1781a>a | c.1624G>A | p.E542K | Sub | missense | 0.35 | . |
| PD9755a | PD9755b | 2407768122 | 3 | 178936-082 | G | A | PIK3CA | CCDS43171.1 | r.1781g>a | c.1624G>A | p.E542K | Sub | missense | 0.38 | . |
| PD17981a | PD17981b | 2427059109 | 3 | 178936-082 | G | A | PIK3CA | CCDS43171.1 | r.1781g>a | c.1624G>A | p.E542K | Sub | missense | 0.67 | . |
| PD11745a | PD11745b | 2460167469 | 3 | 178936-082 | G | A | PIK3CA | CCDS43171.1 | r.1781g>a | c.1624G>A | p.E542K | Sub | missense | 0.38 | . |
| PD9002a | PD9002b | 2464253276 | 3 | 178936-082 | G | A | PIK3CA | CCDS43171.1 | r.1781g>a | c.1624G>A | p.E542K | Sub | missense | 0.44 | . |
| PD24225a | PD24225b | 2498067700 | 3 | 178936-082 | G | A | PIK3CA | CCDS43171.1 | r.1781g>a | c.1624G>A | p.E542K | Sub | missense | 0.19 | . |
| PD3905a | PD3905b | 2544561747 | 3 | 178936-082 | G | A | PIK3CA | CCDS43171.1 | r.1781g>a | c.1624G>A | p.E542K | Sub | mis-sense | 0.15 | . |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD9067a | PD9067b | 2551525319 | 3 | 178936-082 | G | A | PIK3CA | CCDS43171.1 | r.1781g>a | c.1624G>A | p.E542K | Sub | missense | 0.14 | . |
| PD11343a | PD11343b | 2559090543 | 3 | 178936-082 | G | A | PIK3CA | CCDS43171.1 | r.1781g>a | c.1624G>A | p.E542K | Sub | missense | 0.41 | . |
| PD24333a | PD24333b | 2576007561 | 3 | 178936-082 | G | A | PIK3CA | CCDS43171.1 | r.1781g>a | c.1624G>A | p.E542K | Sub | missense | 0.38 | . |
| PD13757a | PD13757b | | 3 | 178936-082 | G | A | PIK3CA | CCDS43171.1 | r.1781g>a | c.1624G>A | p.E542K | Sub | missense | 0.11 | . |
| PD5937a | PD5937b | 1250564720 | 3 | 178936-091 | G | A | PIK3CA | CCDS43171.1 | r.1790a>a | c.1633G>A | p.E545K | Sub | missense | 0.2 | . |
| PD9606a | PD9606b | 1315617182 | 3 | 178936-091 | G | A | PIK3CA | CCDS43171.1 | r.1790a>a | c.1633G>A | p.E545K | Sub | missense | 0.48 | . |
| PD11402a | PD11402b | 1316356449 | 3 | 178936-091 | G | A | PIK3CA | CCDS43171.1 | r.1790a>a | c.1633G>A | p.E545K | Sub | missense | 0.39 | . |
| PD13762a | PD13762b | 1508517584 | 3 | 178936-091 | G | A | PIK3CA | CCDS43171.1 | r.1790a>a | c.1633G>A | p.E545K | Sub | missense | 0.26 | . |
| PD9467a | PD9467b | 1821012809 | 3 | 178936-091 | G | A | PIK3CA | CCDS43171.1 | r.1790a>a | c.1633G>A | p.E545K | Sub | missense | 0.23 | . |
| PD11395a | PD11395b | 1821530182 | 3 | 178936-091 | G | A | PIK3CA | CCDS43171.1 | r.1790a>a | c.1633G>A | p.E545K | Sub | missense | 0.37 | . |
| PD13770a | PD13770b | 1821805384 | 3 | 178936-091 | G | A | PIK3CA | CCDS43171.1 | r.1790a>a | c.1633G>A | p.E545K | Sub | missense | 0.54 | . |
| PD18748a | PD18748b | 1823643529 | 3 | 178936-091 | G | A | PIK3CA | CCDS43171.1 | r.1790a>a | c.1633G>A | p.E545K | Sub | missense | 0.27 | . |
| PD14472a | PD14472b | 2056589925 | 3 | 178936-091 | G | A | PIK3CA | CCDS43171.1 | r.1790a>a | c.1633G>A | p.E545K | Sub | missense | 0.3 | . |
| PD13162a | PD13162b | 2121198182 | 3 | 178936-091 | G | A | PIK3CA | CCDS43171.1 | r.1790a>a | c.1633G>A | p.E545K | Sub | mis-sense | 0.39 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18046a | PD18046b | 2138073486 | 3 | 178936-091 | G | A | PIK3CA | CCDS43171.1 | r.1790a>a | c.1633G>A | p.E545K | Sub | missense | 0.77 | . |
| PD7209a | PD7209b | 2249328331 | 3 | 178936-091 | G | A | PIK3CA | CCDS43171.1 | r.1790a>a | c.1633G>A | p.E545K | Sub | missense | 0.4 | . |
| PD6417a | PD6417b | 2250882404 | 3 | 178936-091 | G | A | PIK3CA | CCDS43171.1 | r.1790a>a | c.1633G>A | p.E545K | Sub | missense | 0.68 | . |
| PD4225a | PD4225b | 2253017619 | 3 | 178936-091 | G | A | PIK3CA | CCDS43171.1 | r.1790a>a | c.1633G>A | p.E545K | Sub | missense | 0.21 | . |
| PD4315a | PD4315b | 2253186318 | 3 | 178936-091 | G | A | PIK3CA | CCDS43171.1 | r.1790a>a | c.1633G>A | p.E545K | Sub | missense | 0.29 | . |
| PD4976a | PD4976b | 2254544652 | 3 | 178936-091 | G | A | PIK3CA | CCDS43171.1 | r.1790a>a | c.1633G>A | p.E545K | Sub | missense | 0.61 | . |
| PD4965a | PD4965b | 2255533065 | 3 | 178936-091 | G | A | PIK3CA | CCDS43171.1 | r.1790a>a | c.1633G>A | p.E545K | Sub | missense | 0.3 | . |
| PD3989a | PD3989b | 2255868587 | 3 | 178936-091 | G | A | PIK3CA | CCDS43171.1 | r.1790a>a | c.1633G>A | p.E545K | Sub | missense | 0.39 | . |
| PD4072a | PD4072b | 2256089816 | 3 | 178936-091 | G | A | PIK3CA | CCDS43171.1 | r.1790a>a | c.1633G>A | p.E545K | Sub | missense | 0.13 | . |
| PD4982a | PD4982b | 2262462700 | 3 | 178936-091 | G | A | PIK3CA | CCDS43171.1 | r.1790a>a | c.1633G>A | p.E545K | Sub | missense | 0.59 | . |
| PD11379a | PD11379b | 2415462179 | 3 | 178936-091 | G | A | PIK3CA | CCDS43171.1 | r.1790a>a | c.1633G>A | p.E545K | Sub | missense | 0.44 | . |
| PD18749a | PD18749b | 2433871386 | 3 | 178936-091 | G | A | PIK3CA | CCDS43171.1 | r.1790a>a | c.1633G>A | p.E545K | Sub | missense | 0.38 | . |
| PD4978a | PD4978b | 2439540277 | 3 | 178936-091 | G | A | PIK3CA | CCDS43171.1 | r.1790a>a | c.1633G>A | p.E545K | Sub | missense | 0.44 | . |
| PD13767a | PD13767b | 2455821416 | 3 | 178936-091 | G | A | PIK3CA | CCDS43171.1 | r.1790a>a | c.1633G>A | p.E545K | Sub | mis-sense | 0.39 | . |

EP 3 452 937 B1

154

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD24307a | PD24307b | 2488055485 | 3 | 178936-091 | G | A | PIK3CA | CCDS43171.1 | r.1790a>a | c.1633G>A | p.E545K | Sub | missense | 0.2 | . |
| PD11327a | PD11327b | 2511385923 | 3 | 178936-091 | G | A | PIK3CA | CCDS43171.1 | r.1790a>a | c.1633G>A | p.E545K | Sub | missense | 0.31 | . |
| PD18116a | PD18116b | 2525208414 | 3 | 178936-091 | G | A | PIK3CA | CCDS43171.1 | r.1790a>a | c.1633G>A | p.E545K | Sub | missense | 0.4 | . |
| PD9578a | PD9578b | 2537075568 | 3 | 178936-091 | G | A | PIK3CA | CCDS43171.1 | r.1790a>a | c.1633G>A | p.E545K | Sub | missense | 0.21 | . |
| PD24327a | PD24327b | 2575763466 | 3 | 178936-091 | G | A | PIK3CA | CCDS43171.1 | r.1790a>a | c.1633G>A | p.E545K | Sub | missense | 0.44 | . |
| PD24329a | PD24329b | 2576137465 | 3 | 178936-091 | G | A | PIK3CA | CCDS43171.1 | r.1790a>a | c.1633G>A | p.E545K | Sub | missense | 0.63 | . |
| PD9541a | PD9541b | . | 3 | 178936-091 | G | A | PIK3CA | CCDS43171.1 | r.1790a>a | c.1633G>A | p.E545K | Sub | missense | 0.1 | . |
| PD13165a | PD13165b | 2145993202 | 3 | 178936-092 | A | C | PIK3CA | CCDS43171.1 | r.1791a>c | c.1634A>C | p.E545A | Sub | missense | 0.41 | . |
| PD4986a | PD4986b | 2257775257 | 3 | 178936-092 | A | C | PIK3CA | CCDS43171.1 | r.1791a>c | c.1634A>C | p.E545A | Sub | missense | 0.41 | . |
| PD9574a | PD9574b | 2260852390 | 3 | 178936-092 | A | C | PIK3CA | CCDS43171.1 | r.1791a>c | c.1634A>C | p.E545A | Sub | missense | 0.24 | . |
| PD24207a | PD24207b | 2398443236 | 3 | 178936-092 | A | C | PIK3CA | CCDS43171.1 | r.1791a>c | c.1634A>C | p.E545A | Sub | missense | 0.44 | . |
| PD11345a | PD11345b | 2184783778 | 3 | 178936-093 | G | C | PIK3CA | CCDS43171.1 | r.1792a>c | c.1635G>C | p.E545D | Sub | missense | 0.35 | . |
| PD24199a | PD24199b | 2389612730 | 3 | 178936-093 | G | C | PIK3CA | CCDS43171.1 | r.1792a>c | c.1635G>C | p.E545D | Sub | missense | 0.3 | . |
| PD11345a | PD11345b | 2184783779 | 3 | 178936-094 | C | A | PIK3CA | CCDS43171.1 | r.1793c>a | c.1636C>A | p.Q546K | Sub | mis-sense | 0.35 | . |

EP 3 452 937 B1

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4069a | PD4069b | 2251202150 | 3 | 178936-094 | C | A | PIK3CA | CCDS43171.1 | r.1793c>a | c.1636C>A | p.Q546K | Sub | missense | 0.41 | . |
| PD11381a | PD11381b | 1211341214 | 3 | 178936-095 | A | C | PIK3CA | CCDS43171.1 | r.1794a>c | c.1637A>C | p.Q546P | Sub | missense | 0.3 | . |
| PD13618a | PD13618b | 1837912748 | 3 | 178937-422 | T | C | PIK3CA | CCDS43171.1 | r.1967u>c | c.1810T>C | p.C604R | Sub | missense | 0.11 | . |
| PD5937a | PD5937b | 1250564721 | 3 | 178938-934 | G | A | PIK3CA | CCDS43171.1 | r.2333a>a | c.2176G>A | p.E726K | Sub | missense | 0.38 | . |
| PD24333a | PD24333b | 2576007562 | 3 | 178938-934 | G | A | PIK3CA | CCDS43171.1 | r.2333a>a | c.2176G>A | p.E726K | Sub | missense | 0.21 | . |
| PD9063a | PD9063b | . | 3 | 178938-934 | G | A | PIK3CA | CCDS43171.1 | r.2333a>a | c.2176G>A | p.E726K | Sub | missense | 0.07 | . |
| PD24189a | PD24189b | 2490371719 | 3 | 178941-923 | C | A | PIK3CA | CCDS43171.1 | r.2399c>a | c.2242C>A | p.L748I | Sub | missense | 0.33 | . |
| PD18776a | PD18776b | 2525042995 | 3 | 178951-957 | G | A | PIK3CA | CCDS43171.1 | r.3169a>a | c.3012G>A | p.M1004I | Sub | missense | 0.2 | . |
| PD4844a | PD4844b3 | 1839770378 | 3 | 178952-064 | T | C | PIK3CA | CCDS43171.1 | r.3276u>c | c.3119T>C | p.M1040T | Sub | missense | 0.42 | . |
| PD4607a | PD4607b | 2249502370 | 3 | 178952-074 | G | A | PIK3CA | CCDS43171.1 | r.3286g>a | c.3129G>A | p.M1043I | Sub | missense | 0.091 | . |
| PD13770a | PD13770b | 1821805385 | 3 | 178952-084 | C | T | PIK3CA | CCDS43171.1 | r.3296c>u | c.3139C>T | p.H1047Y | Sub | missense | 0.17 | . |
| PD24193a | PD24193b | 2398182209 | 3 | 178952-084 | C | T | PIK3CA | CCDS43171.1 | r.3296c>u | c.3139C>T | p.H1047Y | Sub | missense | 0.14 | . |
| PD11338a | PD11338b | 1208113414 | 3 | 178952-085 | A | G | PIK3CA | CCDS43171.1 | r.3297a>g | c.3140A>G | p.H1047R | Sub | missense | 0.23 | . |
| PD9756a | PD9756b | 1210320374 | 3 | 178952-085 | A | G | PIK3CA | CCDS43171.1 | r.3297a>g | c.3140A>G | p.H1047R | Sub | mis-sense | 0.36 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD9544a | PD9544b | 1217056013 | 3 | 178952-085 | A | G | PIK3CA | CCDS43171.1 | r.3297a>g | c.3140A>G | p.H1047R | Sub | missense | 0.28 | . |
| PD8828a | PD8828b | 1228540168 | 3 | 178952-085 | A | G | PIK3CA | CCDS43171.1 | r.3297a>g | c.3140A>G | p.H1047R | Sub | missense | 0.18 | . |
| PD13426a | PD13426b | 1306869936 | 3 | 178952-085 | A | G | PIK3CA | CCDS43171.1 | r.3297a>g | c.3140A>G | p.H1047R | Sub | missense | 0.27 | . |
| PD6016a2 | PD6016b | 1319135371 | 3 | 178952-085 | A | G | PIK3CA | CCDS43171.1 | r.3297a>g | c.3140A>G | p.H1047R | Sub | missense | 0.33 | . |
| PD11386a | PD11386b | 1323240820 | 3 | 178952-085 | A | T | PIK3CA | CCDS43171.1 | r.3297a>u | c.3140A>T | p.H1047L | Sub | missense | 0.24 | . |
| PD11762a | PD11762b | 1333889645 | 3 | 178952-085 | A | G | PIK3CA | CCDS43171.1 | r.3297a>g | c.3140A>G | p.H1047R | Sub | missense | 0.4 | . |
| PD8623a | PD8623b | 1383494481 | 3 | 178952-085 | A | T | PIK3CA | CCDS43171.1 | r.3297a>u | c.3140A>T | p.H1047L | Sub | missense | 0.27 | . |
| PD8622a | PD8622b | 1392993179 | 3 | 178952-085 | A | G | PIK3CA | CCDS43171.1 | r.3297a>g | c.3140A>G | p.H1047R | Sub | missense | 0.28 | . |
| PD8617a | PD8617b | 1534997913 | 3 | 178952-085 | A | G | PIK3CA | CCDS43171.1 | r.3297a>g | c.3140A>G | p.H1047R | Sub | missense | 0.19 | . |
| PD18751a | PD18751b | 1821901679 | 3 | 178952-085 | A | T | PIK3CA | CCDS43171.1 | r.3297a>u | c.3140A>T | p.H1047L | Sub | missense | 0.24 | . |
| PD13618a | PD13618b | 1837912749 | 3 | 178952-085 | A | G | PIK3CA | CCDS43171.1 | r.3297a>g | c.3140A>G | p.H1047R | Sub | missense | 0.44 | . |
| PD11364a | PD11364b | 1838454742 | 3 | 178952-085 | A | G | PIK3CA | CCDS43171.1 | r.3297a>g | c.3140A>G | p.H1047R | Sub | missense | 0.42 | . |
| PD13619a | PD13619b | 1840282352 | 3 | 178952-085 | A | G | PIK3CA | CCDS43171.1 | r.3297a>g | c.3140A>G | p.H1047R | Sub | missense | 0.43 | . |
| PD4076a | PD4076b | 1850841177 | 3 | 178952-085 | A | G | PIK3CA | CCDS43171.1 | r.3297a>g | c.3140A>G | p.H1047R | Sub | mis-sense | 0.29 | . |

| Sample | Normal | VariantID | Chr om | Position | Reference | Mutant | Gene | Transcript | RNA | CDS | Protein | Type | Effect | Proportion_ Mutant _Reads _Subs | Proportion _Mutant _Reads _Indels |
|---|---|---|---|---|---|---|---|---|---|---|---|---|---|---|---|
| PD17991a | PD17991b | 1860415977 | 3 | 178952-085 | A | G | PIK3CA | CCDS43171.1 | r.3297a>g | c.3140A>G | p.H1047R | Sub | missense | 0.31 | . |
| PD4085a | PD4085b | 2068381093 | 3 | 178952-085 | A | G | PIK3CA | CCDS43171.1 | r.3297a>g | c.3140A>G | p.H1047R | Sub | missense | 0.62 | . |
| PD11347a | PD11347b | 2185945721 | 3 | 178952-085 | A | G | PIK3CA | CCDS43171.1 | r.3297a>g | c.3140A>G | p.H1047R | Sub | missense | 0.26 | . |
| PD4967a | PD4967b | 2246447774 | 3 | 178952-085 | A | G | PIK3CA | CCDS43171.1 | r.3297a>g | c.3140A>G | p.H1047R | Sub | missense | 0.14 | . |
| PD7199a | PD7199b | 2246808785 | 3 | 178952-085 | A | G | PIK3CA | CCDS43171.1 | r.3297a>g | c.3140A>G | p.H1047R | Sub | missense | 0.37 | . |
| PD5928a | PD5928b | 2248043145 | 3 | 178952-085 | A | G | PIK3CA | CCDS43171.1 | r.3297a>g | c.3140A>G | p.H1047R | Sub | missense | 0.37 | . |
| PD4985a | PD4985b | 2258978872 | 3 | 178952-085 | A | G | PIK3CA | CCDS43171.1 | r.3297a>g | c.3140A>G | p.H1047R | Sub | missense | 0.61 | . |
| PD4977a | PD4977b | 2259214813 | 3 | 178952-085 | A | G | PIK3CA | CCDS43171.1 | r.3297a>g | c.3140A>G | p.H1047R | Sub | missense | 0.36 | . |
| PD9570a | PD9570b | 2261246495 | 3 | 178952-085 | A | G | PIK3CA | CCDS43171.1 | r.3297a>g | c.3140A>G | p.H1047R | Sub | missense | 0.16 | . |
| PD7201a | PD7201b | 2261374637 | 3 | 178952-085 | A | G | PIK3CA | CCDS43171.1 | r.3297a>g | c.3140A>G | p.H1047R | Sub | missense | 0.41 | . |
| PD4266a | PD4266b | 2262947513 | 3 | 178952-085 | A | G | PIK3CA | CCDS43171.1 | r.3297a>g | c.3140A>G | p.H1047R | Sub | missense | 0.28 | . |
| PD13602a | PD13602b | 2267902863 | 3 | 178952-085 | A | G | PIK3CA | CCDS43171.1 | r.3297a>g | c.3140A>G | p.H1047R | Sub | missense | 0.51 | . |
| PD14465a | PD14465b | 2269103822 | 3 | 178952-085 | A | G | PIK3CA | CCDS43171.1 | r.3297a>g | c.3140A>G | p.H1047R | Sub | missense | 0.2 | . |
| PD24204a | PD24204b | 2385749057 | 3 | 178952-085 | A | G | PIK3CA | CCDS43171.1 | r.3297a>g | c.3140A>G | p.H1047R | Sub | mis-sense | 0.59 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD24199a | PD24199b | 2389612732 | 3 | 178952-085 | A | G | PIK3CA | CCDS43171.1 | r.3297a>g | c.3140A>G | p.H1047R | Sub | missense | 0.49 | . |
| PD9009a | PD9009b | 2406999266 | 3 | 178952-085 | A | G | PIK3CA | CCDS43171.1 | r.3297a>g | c.3140A>G | p.H1047R | Sub | missense | 0.18 | . |
| PD8965a | PD8965b | 2410059275 | 3 | 178952-085 | A | G | PIK3CA | CCDS43171.1 | r.3297a>g | c.3140A>G | p.H1047R | Sub | missense | 0.26 | . |
| PD18734a | PD18734b | 2411878511 | 3 | 178952-085 | A | T | PIK3CA | CCDS43171.1 | r.3297a>u | c.3140A>T | p.H1047L | Sub | missense | 0.15 | . |
| PD6041a | PD6041b | 2414540970 | 3 | 178952-085 | A | G | PIK3CA | CCDS43171.1 | r.3297a>g | c.3140A>G | p.H1047R | Sub | missense | 0.21 | . |
| PD23579a | PD23579b | 2416799810 | 3 | 178952-085 | A | G | PIK3CA | CCDS43171.1 | r.3297a>g | c.3140A>G | p.H1047R | Sub | missense | 0.22 | . |
| PD22359a | PD22359b | 2417501179 | 3 | 178952-085 | A | G | PIK3CA | CCDS43171.1 | r.3297a>g | c.3140A>G | p.H1047R | Sub | missense | 0.14 | . |
| PD18269a | PD18269b | 2447428359 | 3 | 178952-085 | A | G | PIK3CA | CCDS43171.1 | r.3297a>g | c.3140A>G | p.H1047R | Sub | missense | 0.3 | . |
| PD13768a | PD13768b | 2452933032 | 3 | 178952-085 | A | T | PIK3CA | CCDS43171.1 | r.3297a>u | c.3140A>T | p.H1047L | Sub | missense | 0.26 | . |
| PD18258a | PD18258b | 2455910751 | 3 | 178952-085 | A | G | PIK3CA | CCDS43171.1 | r.3297a>g | c.3140A>G | p.H1047R | Sub | missense | 0.38 | . |
| PD18247a | PD18247b | 2456887851 | 3 | 178952-085 | A | T | PIK3CA | CCDS43171.1 | r.3297a>u | c.3140A>T | p.H1047L | Sub | missense | 0.18 | . |
| PD11744a | PD11744b | 2462496434 | 3 | 178952-085 | A | G | PIK3CA | CCDS43171.1 | r.3297a>g | c.3140A>G | p.H1047R | Sub | missense | 0.36 | . |
| PD13304a | PD13304b | 2465394260 | 3 | 178952-085 | A | G | PIK3CA | CCDS43171.1 | r.3297a>g | c.3140A>G | p.H1047R | Sub | missense | 0.32 | . |
| PD18768a | PD18768b | 2479361932 | 3 | 178952-085 | A | G | PIK3CA | CCDS43171.1 | r.3297a>g | c.3140A>G | p.H1047R | Sub | mis-sense | 0.51 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD14459a | PD14459b | 2487591955 | 3 | 178952-085 | A | G | PIK3CA | CCDS43171.1 | r.3297a>g | c.3140A>G | p.H1047R | Sub | missense | 0.4 | . |
| PD24189a | PD24189b | 2490371721 | 3 | 178952-085 | A | G | PIK3CA | CCDS43171.1 | r.3297a>g | c.3140A>G | p.H1047R | Sub | missense | 0.38 | . |
| PD24215a | PD24215b | 2496259547 | 3 | 178952-085 | A | G | PIK3CA | CCDS43171.1 | r.3297a>g | c.3140A>G | p.H1047R | Sub | missense | 0.29 | . |
| PD24217a | PD24217b | 2503248058 | 3 | 178952-085 | A | G | PIK3CA | CCDS43171.1 | r.3297a>g | c.3140A>G | p.H1047R | Sub | missense | 0.32 | . |
| PD24224a | PD24224b | 2503395751 | 3 | 178952-085 | A | G | PIK3CA | CCDS43171.1 | r.3297a>g | c.3140A>G | p.H1047R | Sub | missense | 0.54 | . |
| PD6466b | PD6466a | 2508051001 | 3 | 178952-085 | A | G | PIK3CA | CCDS43171.1 | r.3297a>a | c.3140A>G | p.H1047R | Sub | missense | 0.15 | . |
| PD18754a | PD18754b | 2523354074 | 3 | 178952-085 | A | G | PIK3CA | CCDS43171.1 | r.3297a>a | c.3140A>G | p.H1047R | Sub | missense | 0.53 | . |
| PD24326a | PD24326b | 2536927666 | 3 | 178952-085 | A | G | PIK3CA | CCDS43171.1 | r.3297a>a | c.3140A>G | p.H1047R | Sub | missense | 0.29 | . |
| PD11766a | PD11766b | 2537261397 | 3 | 178952-085 | A | G | PIK3CA | CCDS43171.1 | r.3297a>a | c.3140A>G | p.H1047R | Sub | missense | 0.12 | . |
| PD18756a | PD18756b | 2537405703 | 3 | 178952-085 | A | G | PIK3CA | CCDS43171.1 | r.3297a>a | c.3140A>G | p.H1047R | Sub | missense | 0.23 | . |
| PD4192a | PD4192b | 2559223372 | 3 | 178952-085 | A | G | PIK3CA | CCDS43171.1 | r.3297a>a | c.3140A>G | p.H1047R | Sub | missense | 0.31 | . |
| PD24336a | PD24336b | 2575126232 | 3 | 178952-085 | A | G | PIK3CA | CCDS43171.1 | r.3297a>a | c.3140A>G | p.H1047R | Sub | missense | 0.12 | . |
| PD24302a | PD24302b | 2575608888 | 3 | 178952-085 | A | G | PIK3CA | CCDS43171.1 | r.3297a>a | c.3140A>G | p.H1047R | Sub | missense | 0.43 | . |
| PD24194a | PD24194b | . . | 3 | 178952-085 | A | G | PIK3CA | CCDS43171.1 | r.3297a>a | c.3140A>G | p.H1047R | Sub | mis-sense | 0.0612 2449 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18022a | PD18022b | . . | 3 | 178952-085 | A | G | PIK3CA | CCDS43171.1 | r.3297a>g | c.3140A>G | p.H1047R | Sub | missense | 0.0625 | . |
| PD9584a | PD9584b | . . | 3 | 178952-085 | A | G | PIK3CA | CCDS43171.1 | r.3297a>g | c.3140A>G | p.H1047R | Sub | missense | 0.16 | . |
| PD9591a | PD9591b | . . | 3 | 178952-085 | A | G | PIK3CA | CCDS43171.1 | r.3297a>g | c.3140A>G | p.H1047R | Sub | missense | 0.16 | . |
| PD8615a | PD8615b | . . | 3 | 178952-085 | A | G | PIK3CA | CCDS43171.1 | r.3297a>g | c.3140A>G | p.H1047R | Sub | missense | 0.16 | . |
| PD14441a | PD14441b | . . | 3 | 178952-085 | A | G | PIK3CA | CCDS43171.1 | r.3297a>g | c.3140A>G | p.H1047R | Sub | missense | 0.29 | . |
| PD14439a | PD14439b | . | 3 | 178952-085 | A | G | PIK3CA | CCDS43171.1 | r.3297a>g | c.3140A>G | p.H1047R | Sub | missense | 0.35 | . |
| PD18728a | PD18728b | 2463803387 | 3 | 178952-086 | T | A | PIK3CA | CCDS43171.1 | r.3298u>a | c.3141T>A | p.H1047Q | Sub | missense | 0.22 | . |
| PD4847a | PD4847b2 | 1945706781 | 5 | 675226-99 | A | AG | PIK3R1 | CCDS3993.1 | r.238_239insg | c.196_197insG | p.D68fs*38 | Ins | frame-shift | . | 0.164383-56 |
| PD6412a | PD6412b | 1777631276 | 5 | 675227-40 | GA | G | PIK3R1 | CCDS3993.1 | r.280delA | c.238delA | p.I82fs*32 | Del | frame-shift | . | 0.333333-33 |
| PD5925a | PD5925b | 1646623863 | 5 | 675881-54 | AC | A | PIK3R1 | CCDS3993.1 | r.1027delC | c.985delC | p.Q329f-s*15 | Del | frame-shift | . | 0.314285-71 |
| PD7217a | PD7217b | 1941822104 | 5 | 675894-94 | TGATTAAATACCTTATCCATTGAATTTATTTTAATCTTTCTAGGATCAAGTTGTCAAAGAAGATAATATTGAAGCTGTAGGGAAAAAATTACATGAATATAACACTCAGTTTCAAGAAAAAAGTCGAGAATATGATAGATTATATGAAGAATATACCCGCACATCCCA | T | PIK3R1 | CCDS3993.1 | r.1342-42_-1467+52del220 | c.1300-42_142-5+52 del220 | p.? | Del | ess splice | . | 0.157894-74 |

EP 3 452 937 B1

161

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| | | | | | GGTGAGTTTTCTATGAAAATCAGATTAAAAAATAAGAGTTCTAAACTTTTAAA | | | | | | | | | | |
| PD6042a | PD6042b | 1702505346 | 5 | 67589568 | CTGTAGGGAAAAAATACA | C | PIK3R1 | CCDS3993.1 | r.1374_1391del18 | c.1332_1349del18 | p.V445_H450delVGKKLH | Del | inframe | .. | 0.231884-06 |
| PD9599a | PD9599b | 1745954530 | 5 | 67589609 | GAAA | G | PIK3R1 | CCDS3993.1 | r.1415_1417deIAAA | c.1373_1375delAAA | p.K459delK | Del | inframe | .. | 0.218181-82 |
| PD4952a | PD4952b | 1626485722 | 5 | 67589622 | AATATGATAGATT | A | PIK3R1 | CCDS3993.1 | r.1428_1439del AUAUGAUAGAUU | c.1386_1397del ATATGATAGATT | p.D464_Y467delIDRLY | Del | inframe | . | 0.326923-08 |
| PD23567a | PD23567b | 2331110214 | 5 | 67589664 | T | A | PIK3R1 | CCDS3993.1 | r.1467+2u>a | c.1425+2T>A | p.? | Sub | ess splice | 0.33 | . |
| PD9575a | PD9575b | 1854422025 | 5 | 67591116 | TTATCCAGCTGAG | T | PIK3R1 | CCDS3993.1 | r.1752_1763del UAUCCAGCUGAG | c.1710_1721del TATCCAGCTGAG | p.I571_R574delIQLR | Del | inframe | .. | 0.173076-92 |
| PD24195a | PD24195b | 2397163484 | 5 | 67591144 | ATACTTGATGT | A | PIK3R1 | CCDS3993.1 | r.1780_1787+2del UACUUGAUau | c.1738_1745+2del TACTTGATgt | p.? | Del | ess splice | . | 0.173913-04 |
| PD22361a | PD22361b | 2421172705 | 7 | 6035259 | G | C | PMS2 | CCDS5343.1 | r.915c>a | c.809C>G | p.S270* | Sub | nonsense | 0.27 | . |
| PD13296a | PD13296b | 1334107947 | 6 | 106555250 | C | A | PRDM1 | CCDS5054.2 | r.2601c>a | c.2367C>A | p.C789* | Sub | nonsense | 0.15 | . |
| PD6406a | PD6406b | 2412234801 | 8 | 68939479 | G | A | PREX2 | CCDS6201.1 | r.741a>a | c.464G>A | p.R155Q | Sub | missense | 0.077 | . |
| PD8652a2 | PD8652b | 1969491817 | 10 | 89653862 | GTAAGGTAAGAATGCTTTGATTTTC | G | PTEN | ENST00000371953 | r.1518_1521+20del24 | c.161_164+20del24 | p.? | Del | ess splice | . | 0.156862-75 |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4872a | PD4872b | 2422121563 | 10 | 896853-07 | T | A | PTEN | ENST 00000371953 | r.1559u>a | c202T>A | p.Y68N | Sub | missense | 0.14 | . |
| PD11376a | PD11376b | 1577910765 | 10 | 896853-14 | TGTAA | T | PTEN | ENST 00000371953 | r.1566+1_1-566+4del auaa | c.209+1_209+-4del ataa | p.? | Del | ess splice | .. | 0.425 |
| PD9589a | PD9589b | 1600988549 | 10 | 896927-54 |  | TATCTTTTTACCACAGTTGCACA | PTEN | ENST 00000371953 | r.1611-15_1617del 22 | c.254-15_260del 22 | p.? | Del | frameshift | . | 0.3030303 |
| PD6422a | PD6422b | 2385092704 | 10 | 896929-04 | C | T | PTEN | ENST 00000371953 | r.1745c>u | c.388C>T | p.R130* | Sub | nonsense | 0.37 | . |
| PD23564a | PD23564b | 2418292683 | 10 | 896929-04 | C | T | PTEN | ENST 00000371953 | r.1745c>u | c.388C>T | p.R130* | Sub | nonsense | 0.21 | . |
| PD10014a | PD10014b | 1839908604 | 10 | 896929-05 | G | A | PTEN | ENST 00000371953 | r.1746g>a | c.389G>A | p.R130Q | Sub | missense | 0.21 | . |
| PD9694a | PD9694b | 1210832507 | 10 | 896929-11 | G | A | PTEN | ENST 00000371953 | r.1752g>a | c.395G>A | p.G132D | Sub | missense | 0.26 | . |
| PD4958a | PD4958b | 1610156041 | 10 | 896929-84 | GGAAGTAAGGA | G | PTEN | ENST 00000371953 | r.1826_183-5del GAA-GUAAGGA | c.469_478del GAAGTAAGGA | p.E157fs"7 | Del | frame-shift | . | 0.259259-26 |
| PD5961a | PD5961b | 2251402093 | 10 | 8971187-3 | A | G | PTEN | ENST 00000371953 | r.1850-2a>g | c.493-2A>G | p.? | Sub | ess splice | 0.36 | . |
| PD18257a | PD18257b | 2428295789 | 10 | 8971189-1 | G | T | PTEN | ENST 00000371953 | r.1866a>u | c.509G>T | p.S170I | Sub | missense | 0.29 | . |
| PD11742a | PD11742b | 2378924371 | 10 | 8971195-2 | AGTGGCACTGTTGTTCACAAGATGATGTTTGAAACTATTCCAATGTTCAGT | A | PTEN | ENST 00000371953 | r.1928 1978del51 | c.571 621del51 | p.V191_S207del-VALLFHKMMFE-TIPMFS | Del | inframe | . | 0.526315-79 |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD5944a | PD5944b | 2410149918 | 10 | 897176-09 | G | A | PTEN | ENST 00000371953 | r.1992-1g>a | c.635-1G>A | p.? | Sub | ess splice | 0.63 | . |
| PD11368a | PD11368b | 1279781087 | 10 | 897176-72 | C | T | PTEN | ENST 00000371953 | r.2054c>u | c.697C>T | p.R233* | Sub | nonsense | 0.59 | . |
| PD23564a | PD23564b | 2418292687 | 10 | 897176-72 | C | T | PTEN | ENST 00000371953 | r.2054c>u | c.697C>T | p.R233* | Sub | nonsense | 0.41 | . |
| PD8965a | PD8965b | 2409964937 | 10 | 897177-08 | C | T | PTEN | ENST 00000371953 | r.2090c>u | c.733C>T | p.Q245* | Sub | nonsense | 0.39 | . |
| PD11355a | PD11355b | 1705404491 | 10 | 897177-39 | TAGAGTTC | T | PTEN | ENST 00000371953 | r.2122_212-8del AGA-GUUC | c.765_771del AGAGTTC | p.E256fs*8 | Del | frameshift | . . | 0.3255814 |
| PD24320a | PD24320b | 2560441091 | 10 | 897177-52 | C | CA | PTEN | ENST 00000371953 | r.2134_213-5insa | c.777_778insA | p.Q261f-s*37 | Ins | frame-shift | . | 0.529411-76 |
| PD13422a | PD13422b | 2494417693 | 10 | 897177-77 | G | T | PTEN | ENST 00000371953 | r.2158+1g>-u | c.801+1G>T | p.? | Sub | ess splice | 0.33 | . |
| PD10011a | PD10011b | 2509235928 | 10 | 897206-50 | G | C | PTEN | ENST 00000371953 | r.2159-1a>c | c.802-1G>C | p.? | Sub | ess splice | 0.49 | . |
| PD5953a | PD5953b | 1656854950 | 10 | 897207-25 | T | TG | PTEN | ENST 00000371953 | r.2233_223-4insg | c.876_877insG | p.S294fs*4 | Ins | frameshift | . | 0.6875 |
| PD4980a | PD4980b | 2254780358 | 10 | 897207-41 | C | T | PTEN | ENST 00000371953 | r.2249c>u | c.892C>T | p.Q298* | Sub | nonsense | 0.25 | . |
| PD23567a | PD23567b | 2330429769 | 10 | 897207-78 | ATAATGACA AGGAATATC TAG | A | PTEN | ENST 00000371953 | r.2287_230-6del 20 | c.930_949del 20 | p.N311fs*7 | Del | frame-shift | . | 0.193548-39 |
| PD3989a | PD3989b | 1563550098 | 10 | 897207-98 | GTACT | G | PTEN | ENST 00000371953 | r.2307_231-0del UACU | c.950_953del TACT | p.T319fs*1 | Del | frame-shift | . | 0.384615-38 |
| PD6418a | PD6418b | 1760716595 | 10 | 897207-98 | GTACT | G | PTEN | ENST 00000371953 | r.2307_231-0del UACU | c.950_953del TACT | p.T319fs*1 | Del | frame-shift | . | 0.479166-67 |
| PD7240a | PD7240b | 2508227360 | 10 | 897207-98 | GTACT | G | PTEN | ENST 00000371953 | r.2307_231-0del UACU | c.950_953del TACT | p.T319fs*1 | Del | frame-shift | . | 0.394736-84 |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD11372a | PD11372b | 1574232085 | 10 | 897208-04 | ACTTT | A | PTEN | ENST 00000371953 | r.2313_231-6del CUUU | c.956_959del CTTT | p.T319fs*24 | Del | frame-shift | . | 0.517241-38 |
| PD9575a | PD9575b | 1853931806 | 10 | 897208-32 | CAAAT | C | PTEN | ENST 00000371953 | r.2341_234-4del AAAU | c.984_987del AAAT | p.N329fs"14 | Del | frame-shift | . | 0.241379-31 |
| PD6711a2 | PD6711b | 2503523435 | 10 | 897208-49 | AACCGATACTTTTCTCCAAATTTTAAGGTCAGTTAAATTAAACATTTTGT | A | PTEN | ENST 00000371953 | r.2358_238-3+23 del49 | c.1001_1026+-23 del49 | p.? | Del | ess splice | . | 0.866666-67 |
| PD24191a | PD24191b | 2388992167 | 10 | 897250-36 | TTCTCTAGGTGAAGCTGTACTTCACAAAAACAGTAGAGGAGCCG | T | PTEN | ENST 00000371953 | r.2384-7_2-419 del43 | c.1027-7_1062 del43 | p.? | Del | ess splice | . | 0.333333-33 |
| PD6412a | PD6412b | 1777105910 | 13 | 488814-88 | CAG | C | RB1 | CCDS31973.1 | r.349 350delAG | c.211 212delAG | p.R73fs"36 | Del | frameshift | . | 0.3442623 |
| PD13428a | PD13428b | 2465503821 | 13 | 488815-13 | G | T | RB1 | CCDS31973.1 | r.373a>u | c.235G>T | pE79* | Sub | nonsense | 0.61 | . |
| PD13425a | PD13425b | 1319864028 | 13 | 489167-59 | G | T | RB1 | CCDS31973.1 | r.427a>u | c.289G>T | p.E97* | Sub | nonsense | 0.43 | . |
| PD4975a | PD4975b | 2411204264 | 13 | 489342-34 | C | A | RB1 | CCDS31973.1 | r.827c>a | c.689C>A | p.S230* | Sub | nonsense | 0.51 | . |
| PD6414a | PD6414b | 1752659722 | 13 | 489342-37 | CTCCCATGTTGCTCA | C | RB1 | CCDS31973.1 | UCCCAUGUUGCUCA r.831_84-4del | TCCCATGTTGCTCA c.693_706d-el | p.P232fs*4 | Del | frame-shift | . | 0.142857-14 |
| PD9702a | PD9702b | 1634659631 | 13 | 489342-62 | TAGTA | T | RB1 | CCDS31973.1 | r.856_856+-3 delAaua | c.718_718+3 delAata | p.? | Del | ess splice | . | 0.444444-44 |

EP 3 452 937 B1

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD24218a | PD24218b | 2466476435 | 13 | 489367-86 | TTTGTAGTAGATATGGATGAAATTGTTATCCTTCTAATGAAACCTAATAAGTAAAAGTAGTAGAATGTTACCAAGATTATTTTTGACCTAAGTTATAGTTAGAATACTTCATTATTTTATATGATGGATGTACAATTGTTCTTATCTAATTTACCACTTTTACAGAAACAGCTGTTATACCCATTAATGGTTCACCTCGAACACCCAGGCGAGGTCAGAACAGGAGTGCACGGATAGCAAAACAACTAGAAAATGATACAAGAATTATTGAAGTTCTCTGTAAAGAACATGAATGTAATATAGATGAGGTAATTTAACTTCATGATTTCTTTA | T | RB1 | CCDS31973.1 | r.857-164_-999+25del332 | c.719-164_861+25del332 | p.? | Del | ess splice | . | 0.447368-42 |
| PD22357a | PD22357b | 2315176187 | 13 | 489416-39 | CTT | C | RB1 | CCDS31973.1 | r.1088_108-9delUU | c.950_951delT-T | p.S318fs*1 | Del | frame-shift | . | 0.461538-46 |
| PD4844a | PD4844b3 | 1833345384 | 13 | 489417-20 | CAGACTGATTCTAT | C | RB1 | CCDS31973.1 | AGACUGAUUCUAU r.1169_1-181 del | AGACTGATTCTAT c.1031_104-3del | p.D346fs"17 | Del | frame-shift | . | 0.615384-62 |
| PD11766a | PD11766b | 2537175451 | 13 | 489475-40 | G | A | RB1 | CCDS31973.1 | r.1266-1g>a | c.1128-1G>A | p.? | Sub | ess splice | 0.4 | . |

| Sample | Normal | VariantID | Chr om | Position | Reference | Mutant | Gene | Transcript | RNA | CDS | Protein | Type | Effect | Proportion_Mutant _Reads _Subs | Proportion _Mutant _Reads _Indels |
|---|---|---|---|---|---|---|---|---|---|---|---|---|---|---|---|
| PD7220a | PD7220b | 2272304271 | 13 | 489475-96 | C | T | RB1 | CCDS31973.1 | r.1321c>u | c.1183C>T | p.Q395* | Sub | nonsense | 0.58 | . |
| PD9568a | PD9568b | 1853051334 | 13 | 489510-83 | ACTGAAAA GAGTGAA GGATATAG GATACAT CTTTAAAG AGAAATTT GCTAAAG | A | RB1 | CCDS31973.1 | r.1384_143-5del52 | c.1246_1297d-el52 | p.K417fs*23 | Del | frame-shift | . | 0.577777-78 |
| PD24194a | PD24194b | 2420099978 | 13 | 489543-20 | ATT | A | RB1 | CCDS31973.1 | r.1580_158-1delUU | c.1442_1443d-elTT | p.F482fs*10 | Del | frame-shift | . | 0.272727-27 |
| PD6722a | PD6722b | 2257142265 | 13 | 489555-50 | C | T | RB1 | CCDS31973.1 | r.1804c>u | c.1666C>T | p.R556* | Sub | nonsense | 0.21 | . |
| PD4956a | PD4956b | 1520906174 | 13 | 490304-90 | G | C | RB1 | CCDS31973.1 | r.2098+5g>-c | c.1960+5G>C | p.? | Sub | ess splice | 0.61 | . |
| PD6404a | PD6404b | 2093302889 | 13 | 490338-44 | C | T | RB1 | CCDS31973.1 | r.2119c>u | c.1981C>T | p.R661W | Sub | missense | 0.5 | . |
| PD4980a | PD4980b | 2254799759 | 13 | 490339-55 | A | T | RB1 | CCDS31973.1 | r.2230a>u | c.2092A>T | p.R698W | Sub | missense | 0.21 | . |
| PD5942a | PD5942b | 2256788036 | 13 | 490378-66 | G | C | RB1 | CCDS31973.1 | r.2245-1a>c | c.2107-1G>C | p.? | Sub | ess splice | 0.62 | . |
| PD8965a | PD8965b | 2409984541 | 13 | 490378-66 | G | C | RB1 | CCDS31973.1 | r.2245-1a>c | c.2107-1G>C | p.? | Sub | ess splice | 0.52 | . |
| PD7307a | PD7307b | 2091337658 | 13 | 490395-05 | G | C | RB1 | CCDS31973.1 | r.2627+1g>-c | c.2489+1G>C | p.? | Sub | ess splice | 0.18 | . |

(continued)

| Sample | Normal | VariantID | Chrom | Position | Reference | Mutant | Gene | Transcript | RNA | CDS | Protein | Type | Effect | Proportion_Mutant_Reads_Subs | Proportion_tion_Mutant_Reads_Indels |
|---|---|---|---|---|---|---|---|---|---|---|---|---|---|---|---|
| PD18031a | PD18031b | 2033371018 | 13 | 490500-03 | TATGGGAC CTTATAAG GACAGTTG GAACTGGC TAGGTATC ACATAGTG GTCTTCAA ACATTTTG CTTGCCAT AACCTCTAA AATAATTGG GAAAAAG TTGAATGTA CTTCCATA TCTTAAAGC TGATAATT TAAAATATT ATACATTTA ATAGCAGC ACGGGATT TAGTTTTG TTAAATTGT ATATGTGCT CCAAATAG ATTTACCAT CAAAACCT GTTTTGAAT TTAATATTG GGAGAATT CGCTAGTTT AATTTTTGG AAAATAAAG TATAATTGG CAAAGCTA ATCCTCACT | T | RB1 | CCDS31973.1 | r.2659-833-_2801 +17del993 | c.2521-833_26-63 +17del993 | p.? | Del | ess splice | . | 0.254901-96 |

168

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| | | | | | GTTGAATC | | | | | | | | | | |
| | | | | | TATCCGTC | | | | | | | | | | |
| | | | | | AAATCAGAT | | | | | | | | | | |
| | | | | | ATAATTTCT | | | | | | | | | | |
| | | | | | ATCAGAAA | | | | | | | | | | |
| | | | | | GTCTATATG | | | | | | | | | | |
| | | | | | ACTTGTCA | | | | | | | | | | |
| | | | | | ACATAATAC | | | | | | | | | | |
| | | | | | CCATAAAG | | | | | | | | | | |
| | | | | | TGAATCAAA | | | | | | | | | | |
| | | | | | AATTATTAT | | | | | | | | | | |
| | | | | | TCATTGAAC | | | | | | | | | | |
| | | | | | ACATCATC | | | | | | | | | | |
| | | | | | TCTTATCAA | | | | | | | | | | |
| | | | | | ATTCTTGT | | | | | | | | | | |
| | | | | | GACCTTCC | | | | | | | | | | |
| | | | | | TTCTGGTT | | | | | | | | | | |
| | | | | | GTATAATA | | | | | | | | | | |
| | | | | | GCCTAAAAA | | | | | | | | | | |
| | | | | | ACAAAAAA | | | | | | | | | | |
| | | | | | AGGACAAAA | | | | | | | | | | |
| | | | | | GCAAGTTT | | | | | | | | | | |
| | | | | | CCAGAAAGC | | | | | | | | | | |
| | | | | | TGTTCTGA | | | | | | | | | | |
| | | | | | CTTGCCTAC | | | | | | | | | | |
| | | | | | TTCTGAAAA | | | | | | | | | | |
| | | | | | GTAGTCCT | | | | | | | | | | |
| | | | | | GTATGGTG | | | | | | | | | | |
| | | | | | GGTTCTGAA | | | | | | | | | | |
| | | | | | AATGAGGA | | | | | | | | | | |
| | | | | | ACCAGGACT | | | | | | | | | | |
| | | | | | TGCAGAGTA | | | | | | | | | | |
| | | | | | GGCAGTTG | | | | | | | | | | |
| | | | | | CTGGAGGA | | | | | | | | | | |
| | | | | | AGAATGTGA | | | | | | | | | | |
| | | | | | GCTGCATG | | | | | | | | | | |
| | | | | | GGAAAAGAC | | | | | | | | | | |
| | | | | | AGGAGGAT | | | | | | | | | | |
| | | | | | TTACAAGA | | | | | | | | | | |
| | | | | | GTGGGTGT | | | | | | | | | | |
| | | | | | TTAATTGGG | | | | | | | | | | |
| | | | | | GATGGAAT | | | | | | | | | | |
| | | | | | TAGGTAGTT | | | | | | | | | | |
| | | | | | ATTCTGATT | | | | | | | | | | |
| | | | | | TTTAGATT | | | | | | | | | | |
| | | | | | TTCATATCT | | | | | | | | | | |
| | | | | | TTTATTTGG | | | | | | | | | | |
| | | | | | TCCAATGAA | | | | | | | | | | |
| | | | | | GCAGAAAA | | | | | | | | | | |
| | | | | | TTTAAATGA | | | | | | | | | | |
| | | | | | AGTTATTAC | | | | | | | | | | |
| | | | | | CTTTGCCTG | | | | | | | | | | |
| | | | | | ATTTTTGAC | | | | | | | | | | |
| | | | | | ACACCTCAA | | | | | | | | | | |
| | | | | | ACTATAACT | | | | | | | | | | |
| | | | | | TGAGGTTGC | | | | | | | | | | |
| | | | | | TAACTATG | | | | | | | | | | |
| | | | | | AAACACTGG | | | | | | | | | | |
| | | | | | CATTTAATG | | | | | | | | | | |
| | | | | | ATTTAAAGT | | | | | | | | | | |
| | | | | | AAAGAATTC | | | | | | | | | | |
| | | | | | TGTAATTTG | | | | | | | | | | |
| | | | | | TAGACTTCT | | | | | | | | | | |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| | | | | | GAGAAGTTC CAGAAAATA AATCAGATG GTATGTAAC AGCGACCGT GTGCTCAAA AGAAGTGCT GAAGGAAGC AACCCTCCT AAACCACTG AAAAAACTAC GCTTTGATA TTGAAGGAT CAGATGAAG CAGATGGAA GGTAGGAAC CAGTTTTGA | | | | | | | | | | |
| PD9578a | PD9578b | 2537070365 | 3 | 494128-98 | T | C | RHOA | CCDS2795.1 | r.510a>a | c.125A>G | p.Y42C | Sub | missense | 0.34 | . |
| PD11375a | PD11375b | 1253100357 | 3 | 494129-73 | C | T | RHOA | CCDS2795.1 | r.435a>a | c.50G>A | p.G17E | Sub | missense | 0.13 | . |
| PD13604a | PD13604b | 2266085534 | 3 | 494129-73 | C | T | RHOA | CCDS2795.1 | r.435a>a | c.50G>A | p.G17E | Sub | missense | 0.29 | . |
| PD9575a | PD9575b | 1854279166 | 21 | 362068-99 | CTATTGTG-G | C | RUNX1 | CCDS13639.1 | r.1059-9_1-059-2 delc-cacaaua | c.614-9_614-2 delcacaaata | p.? | Del | ess splice | .. | 0.145454-55 |
| PD11389a | PD11389b | 1675695697 | 21 | 362529-84 | A | AT | RUNX1 | CCDS13639.1 | r.822_823insa | c.377_378insA | p.D126fs"12 | Ins | frame-shift | . | 0.333333-33 |
| PD11402a | PD11402b | 1693514622 | 21 | 362529-94 | T | TCC | RUNX1 | CCDS13639.1 | r.812_813i-nsgg | c.367_368insG-G | p.D123fs"11 | Ins | frame-shift | . | 0.236363-64 |
| PD18775a | PD18775b | 2536433330 | 21 | 362530-12 | T | C | RUNX1 | CCDS13639.1 | r.797-2a>a | c.352-2A>G | p.? | Sub | ess splice | 0.21 | . |
| PD9541a | PD9541b | 1285838696 | 3 | 471258-24 | C | A | SETD2 | CCDS2749.2 | r.5489g>u | c.5446G>T | p.E1816* | Sub | nonsense | 0.3 | . |
| PD24320a | PD24320b | 2560763499 | 3 | 471296-89 | CAG | C | SETD2 | CCDS2749.2 | r.5232_523-3del CU | c.5189_5190del CT | p.S1730fs*1 | Del | frameshift | . | 0.32 |
| PD18748a | PD18748b | 1823640599 | 3 | 471582-25 | G | A | SETD2 | CCDS2749.2 | r.4517c>u | c.4474C>T | p.R1492* | Sub | non-sense | 0.22 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|--------|--------|-----------|-------|----------|-----------|--------|------|-----------|-----|-----|---------|------|--------|-------------------------------|---------------------------------|
| PD4072a | PD4072b | 2256085057 | 3 | 471619-57 | G | C | SETD2 | CCDS2749.2 | r.4212c>a | c.4169C>G | p.S1390* | Sub | nonsense | 0.16 | . |
| PD11357a | PD11357b | 1532379924 | 3 | 471625-06 | GAAGA | G | SETD2 | CCDS2749.2 | r.3659_366-2del UCUU | c.3616_3619del TCTT | p.S1206f-s*29 | Del | frame-shift | . | 0.121951-22 |
| PD9694a | PD9694b | 1210898710 | 2 | 198266-834 | T | C | SF3B1 | CCDS33356.1 | r.2190a>a | c.2098A>G | p. K700E | Sub | missense | 0.15 | . |
| PD13312a | PD13312b | 1306495548 | 2 | 198266-834 | T | C | SF3B1 | CCDS33356.1 | r.2190a>a | c.2098A>G | p. K700E | Sub | missense | 0.5 | . |
| PD9847a | PD9847b | 1317828373 | 2 | 198266-834 | T | C | SF3B1 | CCDS33356.1 | r.2190a>a | c.2098A>G | p. K700E | Sub | missense | 0.53 | . |
| PD13623a | PD13623b | 1508868256 | 2 | 198266-834 | T | C | SF3B1 | CCDS33356.1 | r.2190a>a | c.2098A>G | p.K700E | Sub | missense | 0.19 | . |
| PD13770a | PD13770b | 1821776813 | 2 | 198266-834 | T | C | SF3B1 | CCDS33356.1 | r.2190a>a | c.2098A>G | p. K700E | Sub | missense | 0.13 | . |
| PD4076a | PD4076b | 1850829269 | 2 | 198266-834 | T | C | SF3B1 | CCDS33356.1 | r.2190a>a | c.2098A>G | p. K700E | Sub | missense | 0.16 | . |
| PD18149a | PD18149b | 2056126755 | 2 | 198266-834 | T | C | SF3B1 | CCDS33356.1 | r.2190a>a | c.2098A>G | p.K700E | Sub | missense | 0.27 | . |
| PD4965a | PD4965b | 2255517382 | 2 | 198266-834 | T | C | SF3B1 | CCDS33356.1 | r.2190a>g | c.2098A>G | p. K700E | Sub | missense | 0.26 | . |
| PD3989a | PD3989b | 2255843725 | 2 | 198266-834 | T | C | SF3B1 | CCDS33356.1 | r.2190a>g | c.2098A>G | p. K700E | Sub | missense | 0.22 | . |
| PD9572a | PD9572b | 2263694553 | 2 | 198266-834 | T | C | SF3B1 | CCDS33356.1 | r.2190a>g | c.2098A>G | p.K700E | Sub | missense | 0.26 | . |
| PD9599a | PD9599b | 2466045345 | 2 | 198266-834 | T | C | SF3B1 | CCDS33356.1 | r.2190a>g | c.2098A>G | p. K700E | Sub | missense | 0.24 | . |
| PD6711a2 | PD6711b | 2512449899 | 2 | 198266-834 | T | C | SF3B1 | CCDS33356.1 | r.2190a>g | c.2098A>G | p. K700E | Sub | mis-sense | 0.45 | . |

EP 3 452 937 B1

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD11384a | PD11384b | 1206914976 | 2 | 198267484 | G | A | SF3B1 | CCDS33356.1 | r.1965c>u | c.1873C>T | p.R625C | Sub | missense | 0.16 | . |
| PD11337a | PD11337b | 1720364593 | 18 | 48584611 | C | CAT | SMAD4 | CCDS11950.1 | r.1322_132-3insau | c.784_785insAT | p.N263fs*74 | Ins | frameshift | . | 0.216216-22 |
| PD13625a | PD13625b | 1823509454 | 18 | 48603116 | G | T | SMAD4 | CCDS11950.1 | r.1955a>u | c.1417G>T | p.G473* | Sub | nonsense | 0.68 | . |
| PD4605a | PD4605b | 2256994681 | 19 | 11118599 | C | T | SMARCA4 | CCDS45971.1 | r.2307c>u | c.2023C>T | p.Q675* | Sub | nonsense | 0.23 | . |
| PD9595a | PD9595b | 1622269316 | 19 | 11169548 | AACCTGGAGGGCTCCCTGGTGAGGGC | A | SMARCA4 | CCDS45971.1 | r.4999_501-5+8 del25 | c.4715_4731+8 del25 | p.? | Del | ess splice | . | 0.125 |
| PD23564a | PD23564b | 2418257973 | 1 | 16202812 | C | T | SPEN | CCDS164.1 | r.724c>u | c.520C>T | p.R174* | Sub | nonsense | 0.36 | . |
| PD11762a | PD11762b | 1333756018 | 1 | 16255256 | G | T | SPEN | CCDS164.1 | r.2725g>u | c.2521G>T | p.E841* | Sub | nonsense | 0.13 | . |
| PD11365a | PD11365b | 1814193004 | 1 | 16255882 | T | TA | SPEN | CCDS164.1 | r.3351_335-2insa | c.3147_3148in-sA | p.I1052fs*7 | Ins | frameshift | . | 0.333333-33 |
| PD11380a | PD11380b | 1222739018 | 1 | 16256282 | G | T | SPEN | CCDS164.1 | r.3751g>u | c.3547G>T | p.E1183* | Sub | nonsense | 0.095 | . |
| PD14460a | PD14460b | 2226070847 | 1 | 16256320 | T | TA | SPEN | CCDS164.1 | r.3789_379-0insa | c.3585_3586in-sA | p.D1198fs*4 | Ins | frameshift | . | 0.4516129 |
| PD24320a | PD24320b | 2560364827 | 1 | 16256320 | T | TA | SPEN | CCDS164.1 | r.3789_379-0insa | c.3585 3586insA | p.D1198fs*4 | Ins | frameshift | . | 0.166666-67 |
| PD4255a | PD4255b | 1943569959 | 1 | 16256666 | C | CCTTATGATTAAATCT | SPEN | CCDS164.1 | r.4135_413-6ins cuuauaauu-aaaucu | c.3931_3932ins CTTATGAT-TAAATCT | p.D1313_S1314 ins* | Ins | nonsense | . | 0.1111111-1 |
| PD13620a | PD13620b | 2516032507 | 1 | 16256965 | CAG | C | SPEN | CCDS164.1 | r.4435_443-6delAG | c.4231_4232d-elAG | p.E1412fs*5 | Del | frameshift | . | 0.65 |

172

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD9847a | PD9847b | 1317762446 | 1 | 162573-59 | C | T | SPEN | CCDS164.1 | r.4828c>u | c.4624C>T | p.R1542* | Sub | nonsense | 0.75 | . |
| PD9063a | PD9063b | 2544622365 | 1 | 162577-43 | G | T | SPEN | CCDS164.1 | r.5212g>u | c.5008G>T | p.E1670* | Sub | nonsense | 0.19 | . |
| PD13756a | PD13756b | 2553711805 | 1 | 162594-52 | T | G | SPEN | CCDS164.1 | r.6921u>g | c.6717T>G | p.Y2239* | Sub | nonsense | 0.36 | . |
| PD9004a | PD9004b | 2277204768 | 1 | 162599-35 | TGACCTAAGCAA | T | SPEN | CCDS164.1 | r.7405_7415del GACCUAAGCAA | c.7201_7211del GACCTAAGCAA | p.L2402fs"13 | Del | frame-shift | . | 0.189189-19 |
| PD11389a | PD11389b | 1675195780 | 1 | 162626-79 | A | AC | SPEN | CCDS164.1 | r.10148_10-149insc | c.9944_9945in-sC | p.A3318fs*30 | Ins | frame-shift | . | 0.256410-26 |
| PD5950a | PD5950b | 1676912525 | X | 123164-816 | T | TA | STAG2 | CCDS43990.1 | r.639_640insa | c.129_130insA | p.G46fs*41 | Ins | frame-shift | . | 0.107142-86 |
| PD8973a | PD8973b | 2262405694 | X | 123197-716 | C | T | STAG2 | CCDS43990.1 | r.2350c>u | c.1840C>T | p.R614* | Sub | nonsense | 0.22 | . |
| PD11741a | PD11741b | 2495057080 | 19 | 1219401 | C | A | STK11 | CCDS45896.1 | r.1568c>a | c.453C>A | p.C151* | Sub | nonsense | 0.15 | . |
| PD7201a | PD7201b | 2261340180 | 12 | 1151119-72 | G | A | TBX3 | CCDS9176.1 | r.2732c>u | c.1768C>T | p.Q590* | Sub | nonsense | 0.35 | . |
| PD18776a | PD18776b | 2517001126 | 12 | 1151122-47 | A | AGG | TBX3 | CCDS9176.1 | r.2456_245-7inscc | c.1492_1493in-sCC | p.L498fs"135 | Ins | frame-shift | . | 0.155555-56 |
| PD13427a | PD13427b | 1315445372 | 12 | 1151124-82 | C | A | TBX3 | CCDS9176.1 | r.2222g>u | c.1258G>T | p.E420* | Sub | nonsense | 0.29 | . |
| PD18188a | PD18188b | 2179216122 | 12 | 1151124-89 | G | GA | TBX3 | CCDS9176.1 | r.2214_221-5insu | c.1250_1251in-sT | p.A418fs*3 | Ins | frame-shift | . | 0.195652-17 |
| PD9584a | PD9584b | 2276164403 | 12 | 1151141-15 | T | TAC | TBX3 | CCDS9176.1 | r.2063+2_2-063+3 insau | c.1099+2_109-9+3 insGT | p.? | Ins | ess splice | . | 0.261904-76 |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18049a | PD18049b | 2106601535 | 12 | 1151141-48 | C | CG | TBX3 | CCDS9176.1 | r.2032_203-3insc | c.1068_1069ins-sC | p.A357fs*18 | Ins | frame-shift | . | 0.338709-68 |
| PD9541a | PD9541b | 1285797034 | 12 | 1151141-66 | G | A | TBX3 | CCDS9176.1 | r.2015c>u | c.1051C>T | p.Q351* | Sub | nonsense | 0.24 | . |
| PD11368a | PD11368b | 1555389214 | 12 | 1151142-15 | C | CCCAT | TBX3 | CCDS9176.1 | r.1965_196-6ins auaa | c.1001_1002ins ATGG | p.T335fs*4 | Ins | frame-shift | . | 0.555555-56 |
| PD7210a | PD7210b | 1747492090 | 12 | 1151142-32 | G | GTCTT | TBX3 | CCDS9176.1 | r.1948_194-9insaaga | c.984_985in-sAAGA | p.H329fs*10 | Ins | frame-shift | . | 0.222222-22 |
| PD18756a | PD18756b | 2533800141 | 12 | 1151154-04 | T | TTC | TBX3 | CCDS9176.1 | r.1885_188-6insga | c.921_922in-sGA | p.N308fs"16 | Ins | frame-shift | . | 0.173076-92 |
| PD5937a | PD5937b | 1587253174 | 12 | 1151173-07 | TA | T | TBX3 | CCDS9176.1 | r.1828+2delu | c.864+2delt | p.? | Del | ess splice | . | 0.239436-62 |
| PD11816a | PD11816b | 1664583951 | 12 | 1151187-33 | G | GT | TBX3 | CCDS9176.1 | r.1571_157-2insa | c.607_608insA | p.T203fs*24 | Ins | frame-shift | . | 0.192982-46 |
| PD9009a | PD9009b | 2148478247 | 12 | 1151188-88 | TAAAATG | T | TBX3 | CCDS9176.1 | r.1411_141-6del CAUUUU | c.447_452del CATTTT | p.Y149_-L151 delins* | Del | non-sense | . | 0.120689-66 |
| PD11766a | PD11766b | 2537272276 | 4 | 106190-819 | G | A | TET2 | CCDS47120.1 | r.4483a>a | c.4097G>A | p.R1366H | Sub | missense | 0.23 | . |
| PD7249a | PD7249b | 1253959948 | 17 | 7573996 | A | C | TP53 | CCDS11118.1 | r.1221u>a | c.1031T>G | p.L344R | Sub | missense | 0.73 | . |
| PD3905a | PD3905b | 2543711902 | 17 | 7573997 | GCT | G | TP53 | CCDS11118.1 | r.1218_121-9delAG | c.1028_1029d-elAG | p.E343fs*3 | Del | frame-shift | . | 0.166666-67 |
| PD8982a | PD8982b | 1517528762 | 17 | 7574002 | CG | C | TP53 | CCDS11118.1 | r.1214delC | c.1024delC | p.R342fs*3 | Del | frameshift | . | 0.5 |
| PD11379a | PD11379b | 2415428980 | 17 | 7574002 | C | G | TP53 | CCDS11118.1 | r.1215a>c | c.1025G>C | p.R342P | Sub | missense | 0.65 | . |
| PD18022a | PD18022b | 2512547392 | 17 | 7574002 | C | G | TP53 | CCDS11118.1 | r.1215a>c | c.1025G>C | p.R342P | Sub | mis-sense | 0.23 | . |

EP 3 452 937 B1

174

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD22359a | PD22359b | 2417462675 | 17 | 7574003 | G | A | TP53 | CCDS11118.1 | r.1214c>u | c.1024C>T | p.R342* | Sub | nonsense | 0.26 | . |
| PD24206a | PD24206b | 2400100644 | 17 | 7574006 | ACATCTCGAAGCGCTCACGCCC | A | TP53 | CCDS11118.1 | r.1190_121-0del21 | c.1000_1020del21 | p.G334_-M340 del-GRERFEM | Del | inframe | . | 0.22727273 |
| PD18017a | PD18017b | 2510512182 | 17 | 7574017 | CGCTCACG | C | TP53 | CCDS11118.1 | r.1193_119-9del CGU-GAGC | c.1003_1009del CGTGAGC | p.R335fs"8 | Del | frameshift | . | 0.3 |
| PD5947a | PD5947b | 2390580769 | 17 | 7574018 | G | A | TP53 | CCDS11118.1 | r.1199c>u | c.1009C>T | p.R337C | Sub | missense | 0.32 | . |
| PD24224a | PD24224b | 2503363536 | 17 | 7574018 | G | A | TP53 | CCDS11118.1 | r.1199c>u | c.1009C>T | p.R337C | Sub | missense | 0.32 | . |
| PD5948a | PD5948b | 1934523985 | 17 | 7574026 | C | CCA | TP53 | CCDS11118.1 | r.1190_119-1insug | c.1000 1001insTG | p.G334f-s*12 | Ins | frame-shift | . | 0.61764706 |
| PD5942a | PD5942b | 2256811525 | 17 | 7574034 | C | G | TP53 | CCDS11118.1 | r.1184-1a>c | c.994-1G>C | p.? | Sub | ess splice | 0.78 | . |
| PD7206a | PD7206b | 1775562698 | 17 | 7576827 | TGATAAGAGGTCCCAAGACTTAGTAC | T | TP53 | CCDS11118.1 | r.1183+1_1-183 +25del25 | c.993+1_993 +25del25 | p.? | Del | ess splice | . | 0.23809524 |
| PD8652a2 | PD8652b | 2273625752 | 17 | 7576851 | A | T | TP53 | CCDS11118.1 | r.1183+2u>-a | c.993+2T>A | p.? | Sub | ess splice | 0.33 | . |
| PD22363a | PD22363b | 2319670515 | 17 | 7576852 | C | G | TP53 | CCDS11118.1 | r.1183+1g>c | c993+1G>C | p.? | Sub | ess splice | 0.52 | . |
| PD23578a | PD23578b | 2416581267 | 17 | 7576852 | C | G | TP53 | CCDS11118.1 | r.1183+1g>c | c993+1G>C | p.? | Sub | ess splice | 0.35 | . |
| PD4199a | PD4199b | 2539990019 | 17 | 7576852 | C | T | TP53 | CCDS11118.1 | r.1183+1g>-a | c.993+1G>A | p.? | Sub | ess splice | 0.43 | . |
| PD4967a | PD4967b | 2246389907 | 17 | 7576855 | G | A | TP53 | CCDS11118.1 | r.1181c>u | c.991C>T | p.Q331* | Sub | non-sense | 0.18 | . |

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD7069a | PD7069b | 2511655012 | 17 | 7576855 | G | A | TP53 | CCDS11118.1 | r.1181c>u | c.991C>T | p.Q331* | Sub | nonsense | 0.089 | . |
| PD4836a | PD4836b | 1473663973 | 17 | 7576862 | GA | G | TP53 | CCDS11118.1 | r.1173delU | c.983delT | p.F328fs*17 | Del | frameshift | . | 0.36 |
| PD9604a | PD9604b | 2245824381 | 17 | 7576865 | A | T | TP53 | CCDS11118.1 | r.1171u>a | c.981T>A | p.Y327* | Sub | nonsense | 0.64 | . |
| PD7426a | PD7426b | 1852764028 | 17 | 7576873 | C | A | TP53 | CCDS11118.1 | r.1163g>u | c.973G>T | p.G325* | Sub | nonsense | 0.33 | . |
| PD9760a | PD9760b | 1292097859 | 17 | 7576885 | T | A | TP53 | CCDS11118.1 | r.1151a>u | c961A>T | p.K321* | Sub | nonsense | 0.61 | . |
| PD24329a | PD24329b | 2576106260 | 17 | 7576897 | G | A | TP53 | CCDS11118.1 | r.1139c>u | c.949C>T | p.Q317* | Sub | nonsense | 0.63 | . |
| PD7250a | PD7250b | 1589180597 | 17 | 7577005 | TCCTGCTTGCTTA | T | TP53 | CCDS11118.1 | r.1109+2_1-109 +13de-luaagcaagcagg | c919+2_919 +13deltaagcaagcagg | p.? | Del | ess splice | . | 0.333333-33 |
| PD11751a | PD11751b | 1337595770 | 17 | 7577018 | C | G | TP53 | CCDS11118.1 | r.1109+1g>c | c.919+1G>C | p.? | Sub | ess splice | 0.72 | . |
| PD11398a | PD11398b | 1657932366 | 17 | 7577049 | G | GGTGA | TP53 | CCDS11118.1 | r.1078_107-9ins ucac | c.888_889insTCAC | p.H297fs*10 | Ins | frame-shift | . | 0.323529-41 |
| PD24216a | PD24216b | 2483877659 | 17 | 7577060 | CCT | C | TP53 | CCDS11118.1 | r.1066_106-7delAG | c.876 877delAG | p.E294fs"11 | Del | frame-shift | . | 0.285714-29 |
| PD9579a | PD9579b | 2272673262 | 17 | 7577085 | C | T | TP53 | CCDS11118.1 | r.1043g>a | c.853G>A | p.E285K | Sub | missense | 0.17 | . |
| PD18251a | PD18251b | 2458814773 | 17 | 7577085 | C | T | TP53 | CCDS11118.1 | r.1043g>a | c.853G>A | p.E285K | Sub | missense | 0.62 | . |
| PD5944a | PD5944b | 2410184836 | 17 | 7577094 | G | A | TP53 | CCDS11118.1 | r.1034c>u | c.844C>T | p.R282W | Sub | mis-sense | 0.34 | . |

EP 3 452 937 B1

176

(continued)

| Sample | Normal | VariantID | Chr om | Position | Reference | Mutant | Gene | Transcript | RNA | CDS | Protein | Type | Effect | Proportion_Mutant _Reads _Subs | Proportion_Mutant _Reads _Indels |
|---|---|---|---|---|---|---|---|---|---|---|---|---|---|---|---|
| PD6410a | PD6410b | 2411423186 | 17 | 7577094 | G | A | TP53 | CCDS11118.1 | r.1034c>u | c.844C>T | p.R282W | Sub | missense | 0.28 | . |
| PD7067a | PD7067b | 1778224942 | 17 | 7577095 | GTCTCTC | G | TP53 | CCDS11118.1 | r.1027_103-2del GAGA-GA | c.837_842del GAGAGA | p.D281_-R282 delDR | Del | inframe | . | 0.358974-36 |
| PD7304a | PD7304b | 2174762912 | 17 | 7577096 | T | C | TP53 | CCDS11118.1 | r.1032a>a | c.842A>G | p.D281G | Sub | missense | 0.16 | . |
| PD13163a | PD13163b | 2184412669 | 17 | 7577097 | C | G | TP53 | CCDS11118.1 | r.1031g>c | c.841G>C | p.D281H | Sub | missense | 0.69 | . |
| PD8609a | PD8609b | 2409474071 | 17 | 7577099 | C | T | TP53 | CCDS11118.1 | r.1029g>a | c.839G>A | p. R280K | Sub | missense | 0.33 | . |
| PD24337a | PD24337b | 2550430644 | 17 | 7577099 | C | T | TP53 | CCDS11118.1 | r.1029g>a | c.839G>A | p. R280K | Sub | missense | 0.75 | . |
| PD13166a | PD13166b | 2407228871 | 17 | 7577105 | G | A | TP53 | CCDS11118.1 | r.1023c>u | c.833C>T | p.P278L | Sub | missense | 0.56 | . |
| PD8832a | PD8832b | 1586407525 | 17 | 7577111 | GCAC | G | TP53 | CCDS11118.1 | r.1014_101-6del GUG | c.824_826del GTG | p.C275_-A276 delinsS | Del | complex sub | . . | 0.424242-42 |
| PD4847a | PD4847b2 | 1945409374 | 17 | 7577113 | A | ACA AAC ACG CAC CTC AAA G | TP53 | CCDS11118.1 | cuuugaggugcgu guuu r.1014_1-015ins | CTTTGAGGT GCGTGTTTG c.824_825i-ns | p.C275_-A276 in-sFEVRVC | Ins | inframe | . | 0.072727-27 |
| PD18031a | PD18031b | 2300800247 | 17 | 7577117 | A | C | TP53 | CCDS11118.1 | r.1011u>g | c.821T>G | p.V274G | Sub | missense | 0.19 | . |
| PD6727b | PD6727a | 1227587250 | 17 | 7577120 | C | T | TP53 | CCDS11118.1 | r.1008g>a | c.818G>A | p.R273H | Sub | missense | 0.47 | . |
| PD8619a | PD8619b | 1535642293 | 17 | 7577120 | C | T | TP53 | CCDS11118.1 | r.1008g>a | c.818G>A | p.R273H | Sub | mis-sense | 0.46 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|--------|--------|-----------|--------|----------|-----------|--------|------|------------|-----|-----|---------|------|--------|------|------|
| PD5934a | PD5934b | 2253330240 | 17 | 7577120 | C | T | TP53 | CCDS11118.1 | r.1008g>a | c.818G>A | p.R273H | Sub | missense | 0.62 | . |
| PD13603a | PD13603b | 2268092002 | 17 | 7577120 | C | T | TP53 | CCDS11118.1 | r.1008g>a | c.818G>A | p.R273H | Sub | missense | 0.59 | . |
| PD22366a | PD22366b | 2319443612 | 17 | 7577120 | C | T | TP53 | CCDS11118.1 | r.1008g>a | c.818G>A | p.R273H | Sub | missense | 0.39 | . |
| PD24196a | PD24196b | 2385366340 | 17 | 7577120 | C | A | TP53 | CCDS11118.1 | r.1008g>u | c.818G>T | p.R273L | Sub | missense | 0.39 | . |
| PD8979a | PD8979b | 1212077867 | 17 | 7577121 | G | A | TP53 | CCDS11118.1 | r.1007c>u | c.817C>T | p.R273C | Sub | missense | 0.14 | . |
| PD9752a | PD9752b | 1222394733 | 17 | 7577121 | G | A | TP53 | CCDS11118.1 | r.1007c>u | c.817C>T | p.R273C | Sub | missense | 0.47 | . |
| PD18050a | PD18050b | 2187550665 | 17 | 7577121 | G | T | TP53 | CCDS11118.1 | r.1007c>a | c.817C>A | p.R273S | Sub | missense | 0.38 | . |
| PD8621a | PD8621b | 2245984165 | 17 | 7577121 | G | C | TP53 | CCDS11118.1 | r.1007c>a | c.817C>G | p.R273G | Sub | missense | 0.66 | . |
| PD18020a | PD18020b | 2510166985 | 17 | 7577121 | G | A | TP53 | CCDS11118.1 | r.1007c>u | c.817C>T | p.R273C | Sub | missense | 0.5 | . |
| PD22355a | PD22355b | 2309648018 | 17 | 7577124 | C | T | TP53 | CCDS11118.1 | r.1004a>a | c.814G>A | p.V272M | Sub | missense | 0.78 | . |
| PD13418a | PD13418b | 1517087384 | 17 | 7577127 | C | T | TP53 | CCDS11118.1 | r.1001g>a | c.811G>A | p.E271K | Sub | missense | 0.89 | . |
| PD8830a | PD8830b | 2262554954 | 17 | 7577141 | C | T | TP53 | CCDS11118.1 | r.987g>a | c.797G>A | p.G266E | Sub | missense | 0.5 | . |
| PD11393a | PD11393b | 2449969560 | 17 | 7577151 | TACC | T | TP53 | CCDS11118.1 | r.974_976del GGU | c.784_786del GGT | p.G262delG | Del | inframe | . | 0.4 |
| PD22357a | PD22357b | 2319549354 | 17 | 7577156 | C | T | TP53 | CCDS11118.1 | r.973-1g>a | c.783-1G>A | p.? | Sub | ess splice | 0.72 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD5951a | PD5951b | 2259719426 | 17 | 7577498 | C | A | TP53 | CCDS11118.1 | r.972+1g>u | c.782+1G>T | p.? | Sub | ess splice | 0.44 | . |
| PD22360a | PD22360b | 2322698493 | 17 | 7577498 | C | G | TP53 | CCDS11118.1 | r.972+1g>c | c.782+1G>C | p.? | Sub | ess splice | 0.28 | . |
| PD24201a | PD24201b | 2398725893 | 17 | 7577501 | GGAGTCTTCC AGTGTGATGA TGGT | G | TP53 | CCDS11118.1 | r.947 969del23 | c.757 779del23 | p.T253fs*3 | Del | frameshift | . | 0.45 |
| PD24326a | PD24326b | 2536893812 | 17 | 7577505 | T | A | TP53 | CCDS11118.1 | r.966a>u | c.776A>T | p.D259V | Sub | missense | 0.67 | . |
| PD8965a | PD8965b | 2410005573 | 17 | 7577507 | T | G | TP53 | CCDS11118.1 | r.964a>c | c.774A>C | p.E258D | Sub | missense | 0.57 | . |
| PD13299a | PD13299b | 1579781138 | 17 | 7577514 | GTGA | G | TP53 | CCDS11118.1 | r.954_956del UCA | c.764_766del TCA | p.I255delI | Del | inframe | . | 0.555555- 56 |
| PD10014a | PD10014b | 1839956703 | 17 | 7577517 | A | G | TP53 | CCDS11118.1 | r.954u>c | c.764T>C | p.I255T | Sub | missense | 0.68 | . |
| PD6729a2 | PD6729a | 1968312604 | 17 | 7577526 | A | G | TP53 | CCDS11118.1 | r.945u>c | c.755T>C | p.L252P | Sub | missense | 0.69 | . |
| PD4962a | PD4962b | 2496023034 | 17 | 7577538 | C | T | TP53 | CCDS11118.1 | r.933g>a | c.743G>A | p.R248Q | Sub | missense | 0.21 | . |
| PD24314a | PD24314b | 2505871093 | 17 | 7577538 | C | T | TP53 | CCDS11118.1 | r.933g>a | c.743G>A | p.R248Q | Sub | missense | 0.47 | . |
| PD14435a | PD14435b | 2567661196 | 17 | 7577538 | C | T | TP53 | CCDS11118.1 | r.933g>a | c.743G>A | p.R248Q | Sub | missense | 0.76 | . |
| PD6413a | PD6413b | . | 17 | 7577538 | C | T | TP53 | CCDS11118.1 | r.933g>a | c.743G>A | p.R248Q | Sub | missense | 0.2 | . |
| PD3890a | PD3890b | 2068115666 | 17 | 7577539 | G | A | TP53 | CCDS11118.1 | r.932c>u | c.742C>T | p.R248W | Sub | missense | 0.57 | . |
| PD13167a | PD13167b | 2151863955 | 17 | 7577539 | G | A | TP53 | CCDS11118.1 | r.932c>u | c.742C>T | p.R248W | Sub | mis- sense | 0.5 | . |

EP 3 452 937 B1

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD18049a | PD18049b | 2406509627 | 17 | 7577539 | G | A | TP53 | CCDS11118.1 | r.932c>u | c.742C>T | p.R248W | Sub | missense | 0.17 | . |
| PD13622a | PD13622b | 1697657316 | 17 | 7577544 | ATGCCGCCCATGCAGG | A | TP53 | CCDS11118.1 | CCUGCAUGGGCGGCA r.912_92-6del | CCTGCATGGGCGGCA c.722_736d-el | p.S241_-M246 de-linsL | Del | complex sub | . | 0.448275-86 |
| PD22361a | PD22361b | 2421119494 | 17 | 7577547 | C | A | TP53 | CCDS11118.1 | r.924a>u | c.734G>T | p.G245V | Sub | missense | 0.39 | . |
| PD4980a | PD4980b | 2254816221 | 17 | 7577548 | C | A | TP53 | CCDS11118.1 | r.923g>u | c.733G>T | p.G245C | Sub | missense | 0.52 | . |
| PD11742a | PD11742b | 2380187734 | 17 | 7577548 | C | T | TP53 | CCDS11118.1 | r.923g>a | c.733G>A | p.G245S | Sub | missense | 0.6 | . |
| PD8969a | PD8969b | 2409648540 | 17 | 7577548 | C | T | TP53 | CCDS11118.1 | r.923g>a | c.733G>A | p.G245S | Sub | missense | 0.32 | . |
| PD18771a | PD18771b | 2479176762 | 17 | 7577550 | C | T | TP53 | CCDS11118.1 | r.921g>a | c.731G>A | p.G244D | Sub | missense | 0.31 | . |
| PD23577a | PD23577b | 2386334790 | 17 | 7577557 | AG | A | TP53 | CCDS11118.1 | r.913delC | c.723delC | p.C242fs*5 | Del | frame-shift | . | 0.684210-53 |
| PD11345a | PD11345b | 2184733461 | 17 | 7577559 | G | A | TP53 | CCDS11118.1 | r.912c>u | c.722C>T | p.S241F | Sub | missense | 0.77 | . |
| PD11341a | PD11341b | 2414167657 | 17 | 7577559 | G | A | TP53 | CCDS11118.1 | r.912c>u | c.722C>T | p.S241F | Sub | missense | 0.11 | . |
| PD6046a | PD6046b | 2249173589 | 17 | 7577560 | A | C | TP53 | CCDS11118.1 | r.911u>a | c.721T>G | p.S241A | Sub | missense | 0.55 | . |
| PD14454a | PD14454b | 2493786496 | 17 | 7577560 | A | T | TP53 | CCDS11118.1 | r.911u>a | c.721T>A | p.S241T | Sub | missense | 0.27 | . |
| PD9568a | PD9568b | 1853706344 | 17 | 7577567 | A | T | TP53 | CCDS11118.1 | r.904u>a | c.714T>A | p.C238* | Sub | non-sense | 0.67 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4847a | PD4847b2 | 2411638755 | 17 | 7577568 | C | T | TP53 | CCDS11118.1 | r.903g>a | c.713G>A | p.C238Y | Sub | missense | 0.21 | . |
| PD22364a | PD22364b | 2416920919 | 17 | 7577568 | C | A | TP53 | CCDS11118.1 | r.903g>u | c.713G>T | p.C238F | Sub | missense | 0.7 | . |
| PD9592a | PD9592b | 1398972192 | 17 | 7577570 | C | A | TP53 | CCDS11118.1 | r.901g>u | c.711G>T | p.M237I | Sub | missense | 0.42 | . |
| PD11346a | PD11346b | 2162589007 | 17 | 7577570 | C | T | TP53 | CCDS11118.1 | r.901g>a | c.711G>A | p.M237I | Sub | missense | 0.75 | . |
| PD9599a | PD9599b | 1745598719 | 17 | 7577572 | TGTA | T | TP53 | CCDS11118.1 | r.896_898del UAC | c.706_708del TAC | p.Y236delY | Del | inframe | . | 0.647058-82 |
| PD7316a | PD7316b | 1403020350 | 17 | 7577579 | G | T | TP53 | CCDS11118.1 | r.892c>a | c.702C>A | p.Y234* | Sub | nonsense | 0.32 | . |
| PD4976a | PD4976b | 2254484393 | 17 | 7577580 | T | C | TP53 | CCDS11118.1 | r.891a>g | c.701A>G | p.Y234C | Sub | missense | 0.85 | . |
| PD6732b | PD6732a | 1227771704 | 17 | 7577581 | A | T | TP53 | CCDS11118.1 | r.890u>a | c.700T>A | p.Y234N | Sub | missense | 0.39 | . |
| PD18048a | PD18048b | 2133730644 | 17 | 7577609 | C | A | TP53 | CCDS11118.1 | r.863-1g>u | c.673-1G>T | p.? | Sub | ess splice | 0.56 | . |
| PD8981a | PD8981b | 1204887437 | 17 | 7578175 | A | T | TP53 | CCDS11118.1 | r.862+2u>a | c.672+2T>A | p.? | Sub | ess splice | 0.58 | . |
| PD8964a | PD8964b | 2261030290 | 17 | 7578176 | C | A | TP53 | CCDS11118.1 | r.862+1g>u | c.672+1G>T | p.? | Sub | ess splice | 0.54 | . |
| PD11344a | PD11344b | 2184229028 | 17 | 7578189 | A | C | TP53 | CCDS11118.1 | r.850u>a | c.660T>G | p.Y220* | Sub | nonsense | 0.33 | . |
| PD8980a | PD8980b | 1204303771 | 17 | 7578190 | T | C | TP53 | CCDS11118.1 | r.849a>a | c.659A>G | p.Y220C | Sub | missense | 0.61 | . |
| PD13630a | PD13630b | 1818129390 | 17 | 7578190 | T | C | TP53 | CCDS11118.1 | r.849a>a | c.659A>G | p.Y220C | Sub | mis-sense | 0.3 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4109a | PD4109b | 1822277093 | 17 | 7578190 | T | C | TP53 | CCDS11118.1 | r.849a>a | c.659A>G | p.Y220C | Sub | missense | 0.41 | . |
| PD24192a | PD24192b | 2388124017 | 17 | 7578190 | T | C | TP53 | CCDS11118.1 | r.849a>a | c.659A>G | p.Y220C | Sub | missense | 0.93 | . |
| PD24327a | PD24327b | 2575724294 | 17 | 7578190 | T | C | TP53 | CCDS11118.1 | r.849a>a | c.659A>G | p.Y220C | Sub | missense | 0.37 | . |
| PD24308a | PD24308b | 2505969607 | 17 | 7578191 | A | T | TP53 | CCDS11118.1 | r.848u>a | c.658T>A | p.Y220N | Sub | missense | 0.64 | . |
| PD11326a | PD11326b | 1931258472 | 17 | 7578198 | CACCACACTA | C | TP53 | CCDS11118.1 | r.832_840del UAGUGUGGU | c.642_650del TAGTGTGGT | p.H214_-V217 delinsQ | Del | complex sub | . | 0.5 |
| PD11349a | PD11349b | 2169010015 | 17 | 7578203 | C | T | TP53 | CCDS11118.1 | r.836g>a | c.646G>A | p.V216M | Sub | missense | 0.45 | . |
| PD24223a | PD24223b | 2472825740 | 17 | 7578203 | C | T | TP53 | CCDS11118.1 | r.836g>a | c.646G>A | p.V216M | Sub | missense | 0.5 | . |
| PD9595a | PD9595b | 2244652865 | 17 | 7578211 | C | A | TP53 | CCDS11118.1 | r.828g>u | c.638G>T | p.R213L | Sub | missense | 0.69 | . |
| PD5950a | PD5950b | 1228192510 | 17 | 7578212 | G | A | TP53 | CCDS11118.1 | r.827c>u | c.637C>T | p.R213* | Sub | nonsense | 0.22 | . |
| PD4005a | PD4005b | 2068790858 | 17 | 7578212 | G | A | TP53 | CCDS11118.1 | r.827c>u | c.637C>T | p.R213* | Sub | nonsense | 0.48 | . |
| PD6730b | PD6730b2 | 2271992479 | 17 | 7578212 | G | A | TP53 | CCDS11118.1 | r.827c>u | c.637C>T | p.R213* | Sub | nonsense | 0.25 | . |
| PD7428a | PD7428b | 2292293260 | 17 | 7578212 | G | A | TP53 | CCDS11118.1 | r.827c>u | c.637C>T | p.R213* | Sub | nonsense | 0.76 | . |
| PD7066a | PD7066b | 2302187235 | 17 | 7578212 | G | A | TP53 | CCDS11118.1 | r.827c>u | c.637C>T | p.R213* | Sub | nonsense | 0.14 | . |
| PD23563a | PD23563b | 2353151930 | 17 | 7578212 | G | A | TP53 | CCDS11118.1 | r.827c>u | c.637C>T | p.R213* | Sub | non-sense | 0.26 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD8660a2 | PD8660b | 2408902605 | 17 | 7578212 | G | A | TP53 | CCDS11118.1 | r.827c>u | c.637C>T | p.R213* | Sub | nonsense | 0.73 | . |
| PD4975a | PD4975b | 2411225612 | 17 | 7578212 | G | A | TP53 | CCDS11118.1 | r.827c>u | c.637C>T | p.R213* | Sub | nonsense | 0.27 | . |
| PD4874a | PD4874b | 2412354020 | 17 | 7578212 | G | A | TP53 | CCDS11118.1 | r.827c>u | c.637C>T | p.R213* | Sub | nonsense | 0.51 | . |
| PD7248a | PD7248b | 1757726770 | 17 | 7578221 | TTC | T | TP53 | CCDS11118.1 | r.816_817del GA | c.626_627del GA | p. R209fs*6 | Del | frame-shift | . . | 0.441176-47 |
| PD9575a | PD9575b | 1854124090 | 17 | 7578221 | TTC | T | TP53 | CCDS11118.1 | r.816_817del GA | c.626_627del GA | p.R209fs*6 | Del | frame-shift | . | 0.238095-24 |
| PD7344a | PD7344b | 2296604825 | 17 | 7578224 | T | A | TP53 | CCDS11118.1 | r.815a>u | c.625A>T | p.R209* | Sub | nonsense | 0.097 | . |
| PD8997a | PD8997b | 2091620382 | 17 | 7578236 | A | G | TP53 | CCDS11118.1 | r.803u>c | c.613T>C | p.Y205H | Sub | missense | 0.36 | . |
| PD23554a | PD23554b | 2342130322 | 17 | 7578236 | A | T | TP53 | CCDS11118.1 | r.803u>a | c.613T>A | p.Y205N | Sub | missense | 0.54 | . |
| PD9585a | PD9585b | 1966155039 | 17 | 7578249 | A | AT | TP53 | CCDS11118.1 | r.789_790insa | c.599_600insA | p.N200fs*9 | Ins | frame-shift | . | 0.464285-71 |
| PD4107a | PD4107b | 1756932597 | 17 | 7578262 | CG | C | TP53 | CCDS11118.1 | r.776delC | c.586delC | p. R196fs*51 | Del | frame-shift | . | 0.529411-76 |
| PD5925a | PD5925b | 1208711553 | 17 | 7578263 | G | A | TP53 | CCDS11118.1 | r.776c>u | c.586C>T | p.R196* | Sub | nonsense | 0.25 | . |
| PD6047a | PD6047b | 1226403934 | 17 | 7578263 | G | A | TP53 | CCDS11118.1 | r.776c>u | c.586C>T | p.R196* | Sub | nonsense | 0.84 | . |
| PD13297a | PD13297b | 1520069071 | 17 | 7578263 | G | A | TP53 | CCDS11118.1 | r.776c>u | c.586C>T | p.R196* | Sub | nonsense | 0.89 | . |
| PD6412a | PD6412b | 2258611747 | 17 | 7578263 | G | A | TP53 | CCDS11118.1 | r.776c>u | c.586C>T | p.R196* | Sub | non-sense | 0.48 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD24208a | PD24208b | 2385277030 | 17 | 7578263 | G | A | TP53 | CCDS11118.1 | r.776c>u | c.586C>T | p.R196* | Sub | nonsense | 0.72 | . |
| PD7211a | PD7211b | 2409855477 | 17 | 7578263 | G | A | TP53 | CCDS11118.1 | r.776c>u | c.586C>T | p.R196* | Sub | nonsense | 0.47 | . |
| PD24318a | PD24318b | 2566344757 | 17 | 7578263 | G | A | TP53 | CCDS11118.1 | r.776c>u | c.586C>T | p.R196* | Sub | nonsense | 0.75 | . |
| PD7217a | PD7217b | 2258787226 | 17 | 7578265 | A | G | TP53 | CCDS11118.1 | r.774u>c | c.584T>C | p.I195T | Sub | missense | 0.14 | . |
| PD13425a | PD13425b | 1319882454 | 17 | 7578268 | A | C | TP53 | CCDS11118.1 | r.771u>a | c.581T>G | p.L194R | Sub | missense | 0.5 | . |
| PD11327a | PD11327b | 2511287788 | 17 | 7578268 | A | C | TP53 | CCDS11118.1 | r.771u>g | c.581T>G | p.L194R | Sub | missense | 0.12 | . |
| PD9696a | PD9696b | 1226130073 | 17 | 7578271 | T | C | TP53 | CCDS11118.1 | r.768a>g | c.578A>G | p.H193R | Sub | missense | 0.14 | . |
| PD7321a | PD7321b | 2244809150 | 17 | 7578271 | T | C | TP53 | CCDS11118.1 | r.768a>g | c.578A>G | p.H193R | Sub | missense | 0.31 | . |
| PD6722a | PD6722b | 2257181026 | 17 | 7578271 | T | C | TP53 | CCDS11118.1 | r.768a>g | c.578A>G | p.H193R | Sub | missense | 0.13 | . |
| PD11743a | PD11743b | 1822830637 | 17 | 7578275 | G | A | TP53 | CCDS11118.1 | r.764c>u | c.574C>T | p.Q192* | Sub | nonsense | 0.41 | . |
| PD4826a | PD4826b | 2259893103 | 17 | 7578275 | G | A | TP53 | CCDS11118.1 | r.764c>u | c.574C>T | p.Q192* | Sub | nonsense | 0.32 | . |
| PD7240a | PD7240b | 2509079916 | 17 | 7578290 | C | T | TP53 | CCDS11118.1 | r.750-1g>a | c560-1 G>A | p.? | Sub | ess splice | 0.42 | . |
| PD10011a | PD10011b | 2509280078 | 17 | 7578370 | C | T | TP53 | CCDS11118.1 | r.749+1g>a | c.559+1G>A | p.? | Sub | ess splice | 0.43 | . |
| PD24202a | PD24202b | 2391624902 | 17 | 7578375 | GCTAT | G | TP53 | CCDS11118.1 | r.741_744del AUAG | c.551_554del ATAG | p.D184fs*62 | Del | frame-shift | . | 0.375 |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD4604a | PD4604b | 2257880104 | 17 | 7578382 | G | C | TP53 | CCDS11118.1 | r.738c>g | c.548C>G | p.S183* | Sub | nonsense | 0.6 | . |
| PD9759a | PD9759b | 1316897443 | 17 | 7578384 | G | C | TP53 | CCDS11118.1 | r.736c>g | c.546C>G | p.C182W | Sub | missense | 0.31 | . |
| PD4875a | PD4875b | 2245658673 | 17 | 7578388 | C | G | TP53 | CCDS11118.1 | r.732g>c | c.542G>C | p.R181P | Sub | missense | 0.35 | . |
| PD8978a | PD8978b | 1245128967 | 17 | 7578394 | T | C | TP53 | CCDS11118.1 | r.726a>g | c.536A>G | p.H179R | Sub | missense | 0.66 | . |
| PD9571a | PD9571b | 1856217761 | 17 | 7578394 | T | C | TP53 | CCDS11118.1 | r.726a>g | c.536A>G | p.H179R | Sub | missense | 0.88 | . |
| PD4255a | PD4255b | 2255363349 | 17 | 7578394 | T | C | TP53 | CCDS11118.1 | r.726a>g | c.536A>G | p.H179R | Sub | missense | 0.56 | . |
| PD4968a | PD4968b | 2258225638 | 17 | 7578394 | T | C | TP53 | CCDS11118.1 | r.726a>g | c.536A>G | p.H179R | Sub | missense | 0.36 | . |
| PD13311a | PD13311b | 2512181886 | 17 | 7578394 | T | C | TP53 | CCDS11118.1 | r.726a>g | c.536A>G | p.H179R | Sub | missense | 0.19 | . |
| PD24225a | PD24225b | . | 17 | 7578394 | T | C | TP53 | CCDS11118.1 | r.726a>g | c.536A>G | p.H179R | Sub | missense | 0.25 | . |
| PD9009a | PD9009b | 2406843420 | 17 | 7578398 | G | C | TP53 | CCDS11118.1 | r.722c>g | c.532C>G | p.H178D | Sub | missense | 0.21 | . |
| PD24194a | PD24194b | 2537519866 | 17 | 7578402 | G | C | TP53 | CCDS11118.1 | r.718c>g | c.528C>G | p.C176W | Sub | missense | 0.19 | . |
| PD6731a2 | PD6731a | 1969271932 | 17 | 7578406 | C | T | TP53 | CCDS11118.1 | r.714g>a | c.524G>A | p.R175H | Sub | missense | 0.21 | . |
| PD18024a | PD18024b | 2056732810 | 17 | 7578406 | C | T | TP53 | CCDS11118.1 | r.714g>a | c.524G>A | p.R175H | Sub | missense | 0.24 | . |
| PD6404a | PD6404b | 2093329831 | 17 | 7578406 | C | T | TP53 | CCDS11118.1 | r.714g>a | c.524G>A | p.R175H | Sub | mis-sense | 0.39 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD9576a | PD9576b | 2094487059 | 17 | 7578406 | C | T | TP53 | CCDS11118.1 | r.714g>a | c.524G>A | p.R175H | Sub | missense | 0.41 | . |
| PD13609a | PD13609b | 2163055289 | 17 | 7578406 | C | T | TP53 | CCDS11118.1 | r.714g>a | c.524G>A | p.R175H | Sub | missense | 0.45 | . |
| PD18037a | PD18037b | 2306349256 | 17 | 7578406 | C | T | TP53 | CCDS11118.1 | r.714g>a | c.524G>A | p.R175H | Sub | missense | 0.24 | . |
| PD23566a | PD23566b | 2348373374 | 17 | 7578406 | C | T | TP53 | CCDS11118.1 | r.714g>a | c.524G>A | p.R175H | Sub | missense | 0.47 | . |
| PD6728b | PD6728a | 2409342084 | 17 | 7578406 | C | T | TP53 | CCDS11118.1 | r.714g>a | c.524G>A | p.R175H | Sub | missense | 0.56 | . |
| PD23574a | PD23574b | 2420761347 | 17 | 7578406 | C | T | TP53 | CCDS11118.1 | r.714g>a | c.524G>A | p.R175H | Sub | missense | 0.38 | . |
| PD11750a | PD11750b | 2494223506 | 17 | 7578406 | C | T | TP53 | CCDS11118.1 | r.714g>a | c.524G>A | p.R175H | Sub | missense | 0.27 | . |
| PD24182a | PD24182b | 2505756053 | 17 | 7578406 | C | T | TP53 | CCDS11118.1 | r.714g>a | c.524G>A | p.R175H | Sub | missense | 0.27 | . |
| PD6409a | PD6409b | . | 17 | 7578406 | C | T | TP53 | CCDS11118.1 | r.714g>a | c.524G>A | p.R175H | Sub | missense | 0.11 | . |
| PD24221a | PD24221b | . . | 17 | 7578406 | C | T | TP53 | CCDS11118.1 | r.714g>a | c.524G>A | p.R175H | Sub | missense | 0.23 | . |
| PD6405a | PD6405b | . . | 17 | 7578406 | C | T | TP53 | CCDS11118.1 | r.714g>a | c.524G>A | p.R175H | Sub | missense | 0.74 | . |
| PD24191a | PD24191b | . | 17 | 7578406 | C | T | TP53 | CCDS11118.1 | r.714g>a | c.524G>A | p.R175H | Sub | missense | 0.85 | . |
| PD9702a | PD9702b | 1227965555 | 17 | 7578407 | G | C | TP53 | CCDS11118.1 | r.713c>a | c.523C>G | p.R175G | Sub | missense | 0.53 | . |
| PD4605a | PD4605b | 2256988490 | 17 | 7578416 | C | A | TP53 | CCDS11118.1 | r.704a>u | c.514G>T | p.V172F | Sub | mis-sense | 0.4 | . |

EP 3 452 937 B1

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD8984a | PD8984b | 1544463690 | 17 | 7578420 | CG | C | TP53 | CCDS11118.1 | r.699delC | c.509delC | p.T170fs*4 | Del | frame-shift | . | 0.444444-44 |
| PD24215a | PD24215b | 2495484740 | 17 | 7578434 | AC | A | TP53 | CCDS11118.1 | r.685delG | c.495delG | p.Q165fs*5 | Del | frameshift | . | 0.5625 |
| PD11745a | PD11745b | 2460099537 | 17 | 7578437 | G | A | TP53 | CCDS11118.1 | r.683c>u | c.493C>T | p.Q165* | Sub | nonsense | 0.35 | . |
| PD18046a | PD18046b | 2138030829 | 17 | 7578440 | T | A | TP53 | CCDS11118.1 | r.680a>u | c.490A>T | p.K164* | Sub | nonsense | 0.73 | . |
| PD6406a | PD6406b | 1707441109 | 17 | 7578441 | GTAGATGGCCATGGCG | GGCCCC | TP53 | CCDS11118.1 | CGCCAUGGCCAUCUA insaaaac r.664_67-8del | CGCCATGGCCATCTA insGGGGC c.474 488del | p.A159fs*8 | Complex | frame-shift | . | 0.230769-23 |
| PD6411a | PD6411b | 2246959717 | 17 | 7578442 | T | C | TP53 | CCDS11118.1 | r.678a>a | c.488A>G | p.Y163C | Sub | missense | 0.22 | . |
| PD9002a | PD9002b | 2464183187 | 17 | 7578442 | T | C | TP53 | CCDS11118.1 | r.678a>a | c.488A>G | p.Y163C | Sub | missense | 0.77 | . |
| PD9539a | PD9539b | 2536705007 | 17 | 7578442 | T | C | TP53 | CCDS11118.1 | r.678a>a | c.488A>G | p.Y163C | Sub | missense | 0.74 | . |
| PD24220a | PD24220b | 2575420859 | 17 | 7578442 | T | C | TP53 | CCDS11118.1 | r.678a>a | c.488A>G | p.Y163C | Sub | missense | 0.82 | . |
| PD14471a | PD14471b | 2047432076 | 17 | 7578454 | G | A | TP53 | CCDS11118.1 | r.666c>u | c.476C>T | p.A159V | Sub | missense | 0.76 | . |
| PD4841a | PD4841b | 2257630334 | 17 | 7578463 | C | G | TP53 | CCDS11118.1 | r.657g>c | c.467G>C | p.R156P | Sub | missense | 0.83 | . |
| PD13627a | PD13627b | 1512414345 | 17 | 7578470 | CGGGCGGGGGTGTG | C | TP53 | CCDS11118.1 | CACACCCCCGCCC r.637_64-9del | CACACCCCCGCCC c.447_459d-el | p.T150fs*16 | Del | frame-shift | . | 0.380952-38 |

187

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD4833a | PD4833b | 1938400760 | 17 | 7578474 | C | CG | TP53 | CCDS11118.1 | r.645_646insc | c.455_456insC | p.P153fs*28 | Ins | frame-shift | . | 0.295454-55 |
| PD4971a | PD4971b | 2260231437 | 17 | 7578479 | G | A | TP53 | CCDS11118.1 | r.641c>u | c.451C>T | p.P151S | Sub | missense | 0.57 | . |
| PD23559a | PD23559b | 2538286233 | 17 | 7578479 | G | A | TP53 | CCDS11118.1 | r.641c>u | c.451C>T | p.P151S | Sub | missense | 0.36 | . |
| PD5945a | PD5945b | 2252057034 | 17 | 7578492 | C | T | TP53 | CCDS11118.1 | r.628a>a | c.438G>A | p.W146* | Sub | nonsense | 0.86 | . |
| PD8612a | PD8612b | 1576288285 | 17 | 7578496 | AGC | A | TP53 | CCDS11118.1 | r.622_623del GC | c.432_433del GC | p.Q144fs*4 | Del | frame-shift | . | 0.348837-21 |
| PD24193a | PD24193b | 2398137732 | 17 | 7578505 | G | T | TP53 | CCDS11118.1 | r.615c>a | c.425C>A | p.P142H | Sub | missense | 0.21 | . |
| PD13428a | PD13428b | 1513322036 | 17 | 7578506 | G | GC | TP53 | CCDS11118.1 | r.613 614insg | c.423 424insG | p.P142fs*7 | Ins | frame-shift | . | 0.6111111-1 |
| PD13298a | PD13298b | 1216451427 | 17 | 7578508 | C | T | TP53 | CCDS11118.1 | r.612a>a | c.422G>A | p.C141Y | Sub | missense | 0.7 | . |
| PD4252a | PD4252b | . | 17 | 7578515 | TG | T | TP53 | CCDS11118.1 | c.414delC | p.K139fs*31 | Del | Frameshift del | . | . | . |
| PD8615a | PD8615b | 1838010423 | 17 | 7578526 | C | T | TP53 | CCDS11118.1 | r.594a>a | c.404G>A | p.C135Y | Sub | missense | 0.25 | . |
| PD24219a | PD24219b | 2576231336 | 17 | 7578532 | A | C | TP53 | CCDS11118.1 | r.588u>a | c.398T>G | p.M133R | Sub | missense | 0.75 | . |
| PD7307a | PD7307b | 2091365184 | 17 | 7578535 | T | C | TP53 | CCDS11118.1 | r.585a>a | c.395A>G | p.K132R | Sub | missense | 0.22 | . |
| PD7220a | PD7220b | 2272325837 | 17 | 7578535 | T | C | TP53 | CCDS11118.1 | r.585a>a | c.395A>G | p.K132R | Sub | missense | 0.57 | . |
| PD24197a | PD24197b | 2417229886 | 17 | 7578535 | T | C | TP53 | CCDS11118.1 | r.585a>a | c.395A>G | p.K132R | Sub | mis-sense | 0.33 | . |

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD23560a | PD23560b | 2342518992 | 17 | 7578555 | C | G | TP53 | CCDS11118.1 | r.566-1a>c | c.376-1G>C | p.? | Sub | ess splice | 0.16 | . |
| PD13608a | PD13608b | 2407839957 | 17 | 7578556 | T | C | TP53 | CCDS11118.1 | r.566-2a>a | c.376-2A>G | p.? | Sub | ess splice | 0.39 | . |
| PD11752a | PD11752b | 1749495884 | 17 | 7579310 | AC | A | TP53 | CCDS11118.1 | r.565+1delg | c.375+1 delg | p.? | Del | ess splice | . | 0.40425532 |
| PD5935a | PD5935b | 1405472204 | 17 | 7579313 | G | C | TP53 | CCDS11118.1 | r.564c>a | c.374C>G | p.T125R | Sub | missense | 0.55 | . |
| PD9004a | PD9004b | 2277469774 | 17 | 7579320 | TCA | T | TP53 | CCDS11118.1 | r.555_556delUG | c.365_366delTG | p.V122fs"26 | Del | frameshift | . | 0.4 |
| PD8611a | PD8611b | 1770848368 | 17 | 7579335 | TC | T | TP53 | CCDS11118.1 | r.541delG | c.351 delG | p.T118fs*5 | Del | frameshift | . | 0.11627907 |
| PD4192a | PD4192b | 2556540440 | 17 | 7579344 | GCAAGAAGCCCAGA | G | TP53 | CCDS11118.1 | UCUGGGCUUCUUG r.520_53-2del | TCTGGGCTTCTTG c.330_342del | p.L111fs*8 | Del | frameshift | . | 0.27586207 |
| PD4956a | PD4956b | 1623736521 | 17 | 7579358 | CG | C | TP53 | CCDS11118.1 | r.518delC | c.328delC | p.R110fs*13 | Del | frameshift | . | 0.5 |
| PD4844a | PD4844b3 | 1833425927 | 17 | 7579358 | CG | C | TP53 | CCDS11118.1 | r.518delC | c.328delC | p.R110fs*13 | Del | frameshift | . | 0.74285714 |
| PD9064a | PD9064b | 2304124707 | 17 | 7579359 | GGAAACC | G | TP53 | CCDS11118.1 | r.512_517del GGUUUC | c.322_327del GGTTTC | p.G108_-F109 delGF | Del | inframe | . | 0.31428571 |
| PD14437a | PD14437b | 1844212716 | 17 | 7579377 | G | A | TP53 | CCDS11118.1 | r.500c>u | c.310C>T | p.Q104* | Sub | nonsense | 0.32 | . |
| PD9464a | PD9464b | 1800722614 | 17 | 7579382 | GT | G | TP53 | CCDS11118.1 | r.494delA | c.304delA | p.T102fs*21 | Del | frameshift | . | 0.53846154 |
| PD6733b | PD6733a | 1228717176 | 17 | 7579389 | G | A | TP53 | CCDS11118.1 | r.488c>u | c.298C>T | p.Q100* | Sub | non-sense | 0.21 | . |

EP 3 452 937 B1

(continued)

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD6043a | PD6043b | 1738551249 | 17 | 7579391 | GAAGGGACAGAAGATGACAGGGGCCAGGAGGGGGCTGGTGCAGGGGCCGCCGGTA | G | TP53 | CCDS11118.1 | r.430_485del56 | c.240_295del56 | p.T81fs*49 | Del | frameshift | . | 0.352941-18 |
| PD7238a | PD7238b | 1599558980 | 17 | 7579395 | GGACA | G | TP53 | CCDS11118.1 | r.478_481del UGUC | c.288_291del TGTC | p.V97fs*25 | Del | frameshift | . | 0.6 |
| PD11748a | PD11748b | 1653083632 | 17 | 7579397 | ACAGAAGATGACAGGGGC | A | TP53 | CCDS11118.1 | r.463_479del17 | c.273_289del17 | p.W91fs*52 | Del | frameshift | . | 0.170731-71 |
| PD6415a | PD6415b | 1856629336 | 17 | 7579405 | T | TG | TP53 | CCDS11118.1 | r.471_472insc | c.281_282insC | p.S95fs*54 | Ins | frameshift | . | 0.285714-29 |
| PD23565a | PD23565b | 2417338376 | 17 | 7579415 | C | T | TP53 | CCDS11118.1 | r.462a>a | c.272G>A | p.W91* | Sub | nonsense | 0.38 | . |
| PD6719a | PD6719b | 1772470964 | 17 | 7579470 | CG | C | TP53 | CCDS11118.1 | r.406delC | c.216delC | p.V73fs*50 | Del | frameshift | . | 0.15625 |
| PD11462a | PD11462b | 2171152117 | 17 | 7579470 | CG | C | TP53 | CCDS11118.1 | r.406delC | c.216delC | p.V73fs*50 | Del | frameshift | . | 0.526315-79 |
| PD24333a | PD24333b | 2548020384 | 17 | 7579470 | C | CG | TP53 | CCDS11118.1 | r.406 407insc | c.216 217insC | p.V73fs*76 | Ins | frameshift | . | 0.571428-57 |
| PD23562a | PD23562b | 2335015342 | 17 | 7579486 | T | TG | TP53 | CCDS11118.1 | r.390 391insc | c.200_201insC | p.E68fs*81 | Ins | frameshift | . | 0.692307-69 |
| PD10010a | PD10010b | 1257633997 | 17 | 7579494 | T | A | TP53 | CCDS11118.1 | r.383a>u | c.193A>T | p.R65* | Sub | nonsense | 0.8 | . |
| PD14453a | PD14453b | 1529376150 | 17 | 7579494 | TG | T | TP53 | CCDS11118.1 | r.382delC | c.192delC | p.R65fs*58 | Del | frameshift | . | 0.315789-47 |
| PD23567a | PD23567b | 2330590459 | 17 | 7579525 | GA | G | TP53 | CCDS11118.1 | r.351delU | c.161delT | p.F54fs*69 | Del | frameshift | . | 0.12 |
| PD4086a | PD4086b | 1515036285 | 17 | 7579528 | C | T | TP53 | CCDS11118.1 | r.349g>a | c.159G>A | p.W53* | Sub | nonsense | 0.57 | . |

EP 3 452 937 B1

| Sample | Normal | VariantID | Chrom | Position | Reference | Mutant | Gene | Transcript | RNA | CDS | Protein | Type | Effect | Proportion_Mutant_Reads_Subs | Proportion_Mutant_Reads_Indels |
|---|---|---|---|---|---|---|---|---|---|---|---|---|---|---|---|
| PD7215a | PD7215b | 2253910115 | 17 | 7579529 | C | T | TP53 | CCDS11118.1 | r.348g>a | c.158G>A | p.W53* | Sub | nonsense | 0.27 | . |
| PD5932a | PD5932b | 1936022652 | 17 | 7579534 | TTCAA | T | TP53 | CCDS11118.1 | r.339_342del UUGA | c.149_152del TTGA | p.I50fs*72 | Del | frameshift | . | 0.3030303 |
| PD8617a | PD8617b | 1523116285 | X | 409940-73 | TG | T | USP9X | CCDS43930.1 | r.1052delG | c.419delG | p.W140fs*1 | Del | frame-shift | . | 0.266666-67 |
| PD6412a | PD6412b | 1777921119 | X | 410436-47 | CAGGT | C | USP9X | CCDS43930.1 | r.3913-2_3-914 delaa-GU | c.3280-2_3281 delaaGT | p.? | Del | ess splice | . | 0.3 |
| PD11367a | PD11367b | 2551397719 | X | 410579-47 | TCAAA | T | USP9X | CCDS43930.1 | r.5181_518-4del CAAA | c.4548_4551del CAAA | p.Q1518f-s*1 | Del | frame-shift | . | 0.541666-67 |
| PD23564a | PD23564b | 2338038178 | X | 410756-71 | C | CT | USP9X | CCDS43930.1 | r.6484_648-5insu | c.5851_5852in-sT | p.Y1953fs*2 | Ins | frame-shift | . | 0.247191-01 |
| PD4972a | PD4972b | 1572292101 | 22 | 291916-00 | AG | A | XBP1 | CCDS13847.1 | r.792delC | c.719delC | p.P240fs*20 | Del | frame-shift | . | 0.447368-42 |
| PD4606a | PD4606b | 1743217089 | 22 | 291916-58 | G | GGA AGG GCA TTT | XBP1 | CCDS13847.1 | r.734_735i-ns aaauacc-cuuc | c.661_662ins AAATGCCCTT-C | p.P221fs*43 | Ins | frameshift | . | 0.125 |
| PD13604a | PD13604b | 2265568695 | 22 | 291920-37 | CTG | C | XBP1 | CCDS13847.1 | r.668_669d-elCA | c.595_596del-CA | p.Q199f-s*187 | Del | frame-shift | . | 0.160714-29 |
| PD11394a | PD11394b | 1611326058 | 22 | 291920-46 | AAG | A | XBP1 | CCDS13847.1 | r.659_660d-elCU | c.586_587delC-T | p.L196f-s*190 | Del | frame-shift | . | 0.391304-35 |
| PD11396a | PD11396b | 1598627592 | 14 | 692567-97 | A | AG | ZFP36-L1 | CCDS9791.1 | r.2267_226-8insc | c.469_470insC | p.L157fs*24 | Ins | frame-shift | . | 0.444444-44 |
| PD13605a | PD13605b | 2132188826 | 14 | 692570-86 | TG | T | ZFP36-L1 | CCDS9791.1 | r.1978delC | c.180delC | p.K61fs*19 | Del | frame-shift | . | 0.444444-44 |
| PD23570a | PD23570b | 2417888213 | 10 | 896242-74 | T | A | PTEN | ENS-T00000371953 | r.1405u>a | c.48T>A | p.Y16* | Sub | non-sense | 0.4 | . |

| Sample | Normal | VariantID | Chr om | Position | Reference | Mutant | Gene | Transcript | RNA | CDS | Protein | Type | Effect | Propor tion_ Mutant _Reads _Subs | Propor tion _Mutant _Reads _Indels |
|---|---|---|---|---|---|---|---|---|---|---|---|---|---|---|---|
| PD8615a | PD8615b | 1830200930 | 10 | 896242-94 | T | TA | PTEN | ENS-T00000371953 | r.1425_142-6insa | c.68_69insA | p.D24fs"20 | Ins | frame-shift | . | 0.310344-83 |
| PD24190a | PD24190b | 2393701237 | 10 | 896242-65 | AAGGAGAT | A | PTEN | ENS-T00000371953 | r.1397_140-3del AGGA-GAU | c.40_46del AG-GAGAT | p.R14fs*8 | Del | frameshift | . | 0.4 |
| PD11361a | PD11361b | 1696257319 | 5 | 561678-38 | A | AC | MAP3-K1 | CCDS43318.1 | r.1403_140-4insc | c.1403_1404in-sC | p.H469fs"12 | Ins | frame-shift | . | 0.193548-39 |
| PD11361a | PD11361b | 1696257321 | 5 | 561779-98 | CCATCTG TACCAGCTGG | C | MAP3-K1 | CCDS43318.1 | r.2972_298-7del16 | c.2972_2987d-el16 | p.S992fs*85 | Del | frame-shift | . | 0.181818-18 |
| PD7221a | PD7221b | 2251120259 | 20 | 574844-20 | C | T | GNAS | CCDS46622.1 | r.2533c>u | c.2530C>T | p.R844C | Sub | missense | 0.42 | . |
| PD11366a | PD11366b | 1508195051 | 20 | 574844-20 | C | T | GNAS | CCDS46622.1 | r.2533c>u | c.2530C>T | p.R844C | Sub | missense | 0.35 | . |

Table 7B

| Sample | Tumour name | Gene | Effect |
|--------|-------------|------|--------|
| PD11338 | PD11338a | DNMT3A | HD |
| PD14467 | PD14467a | DNMT3A | HD |
| PD13296 | PD13296a | PIK3CA | amp |
| PD22355 | PD22355a | PIK3CA | amp |
| PD7428 | PD7428a | PIK3CA | amp |
| PD8611 | PD8611a | PIK3CA | amp |
| PD13428 | PD13428a | PIK3CA | amp |
| PD5945 | PD5945a | PIK3CA | amp |
| PD6411 | PD6411a | PIK3CA | amp |
| PD24224 | PD24224a | PIK3CA | amp |
| PD11327 | PD11327a | PIK3CA | amp |
| PD8980 | PD8980a | PIK3CA | amp |
| PD5950 | PD5950a | PIK3CA | amp |
| PD23562 | PD23562a | PIK3CA | amp |
| PD24202 | PD24202a | PIK3CA | amp |
| PD22366 | PD22366a | PIK3CA | amp |
| PD23558 | PD23558a | PIK3CA | amp |
| PD11349 | PD11349a | PDGFRA | amp |
| PD23560 | PD23560a | PDGFRA | amp |
| PD7428 | PD7428a | PDGFRA | amp |
| PD6410 | PD6410a | PDGFRA | amp |
| PD6684 | PD6684a | MAP3K1 | HD |
| PD18020 | PD18020a | MAP3K1 | HD |
| PD22036 | PD22036a | CCND3 | amp |
| PD7428 | PD7428a | CCND3 | amp |
| PD4874 | PD4874a | CCND3 | amp |
| PD11327 | PD11327a | CCND3 | amp |
| PD5950 | PD5950a | CCND3 | amp |
| PD3905 | PD3905a | CCND3 | amp |
| PD6732 | PD6732b | CCND3 | amp |
| PD18037 | PD18037a | CCND3 | amp |
| PD23554 | PD23554a | CCND3 | amp |
| PD5942 | PD5942a | CCND3 | amp |
| PD24318 | PD24318a | CCND3 | amp |
| PD24308 | PD24308a | CCND3 | amp |
| PD7067 | PD7067a | CCND3 | amp |
| PD4264 | PD4264a | CCND3 | amp |
| PD22251 | PD22251a | CCND3 | amp |
| PD5932 | PD5932a | CCND3 | amp |

(continued)

| Sample | Tumour name | Gene | Effect |
|---|---|---|---|
| PD5942 | PD5942a | ESR1 | amp |
| PD13425 | PD13425a | ESR1 | amp |
| PD24199 | PD24199a | ESR1 | amp |
| PD24216 | PD24216a | ESR1 | amp |
| PD6416 | PD6416a | EGFR | amp |
| PD24314 | PD24314a | EGFR | amp |
| PD24308 | PD24308a | EGFR | amp |
| PD24326 | PD24326a | EGFR | amp |
| PD11766 | PD11766a | EGFR | amp |
| PD22361 | PD22361a | EGFR | amp |
| PD24201 | PD24201a | CDK6 | amp |
| PD6043 | PD6043a | CDK6 | amp |
| PD14453 | PD14453a | CDK6 | amp |
| PD4605 | PD4605a | CDK6 | amp |
| PD9464 | PD9464a | CDK6 | amp |
| PD11751 | PD11751a | CDK6 | amp |
| PD9467 | PD9467a | Chr8:(ZNF703/FGFR1) | amp |
| PD11379 | PD11379a | Chr8:(ZNF703/FGFR1) | amp |
| PD18188 | PD18188a | Chr8:(ZNF703/FGFR1) | amp |
| PD9760 | PD9760a | Chr8:(ZNF703/FGFR1) | amp |
| PD13608 | PD13608a | Chr8:(ZNF703/FGFR1) | amp |
| PD22365 | PD22365a | Chr8:(ZNF703/FGFR1) | amp |
| PD18257 | PD18257a | Chr8:(ZNF703/FGFR1) | amp |
| PD8979 | PD8979a | Chr8:(ZNF703/FGFR1) | amp |
| PD13425 | PD13425a | Chr8:(ZNF703/FGFR1) | amp |
| PD4264 | PD4264a | Chr8:(ZNF703/FGFR1) | amp |
| PD4965 | PD4965a | Chr8:(ZNF703/FGFR1) | amp |
| PD9464 | PD9464a | Chr8:(ZNF703/FGFR1) | amp |
| PD11368 | PD11368a | Chr8:(ZNF703/FGFR1) | amp |
| PD6719 | PD6719a | Chr8:(ZNF703/FGFR1) | amp |
| PD9599 | PD9599a | Chr8:(ZNF703/FGFR1) | amp |
| PD13630 | PD13630a | Chr8:(ZNF703/FGFR1) | amp |
| PD13165 | PD13165a | Chr8:(ZNF703/FGFR1) | amp |
| PD9002 | PD9002a | Chr8:(ZNF703/FGFR1) | amp |
| PD9759 | PD9759a | Chr8:(ZNF703/FGFR1) | amp |
| PD9694 | PD9694a | Chr8:(ZNF703/FGFR1) | amp |
| PD4978 | PD4978a | Chr8:(ZNF703/FGFR1) | amp |
| PD9009 | PD9009a | Chr8:(ZNF703/FGFR1) | amp |
| PD14453 | PD14453a | Chr8:(ZNF703/FGFR1) | amp |

(continued)

| Sample | Tumour name | Gene | Effect |
|---|---|---|---|
| PD11760 | PD11760a | Chr8:(ZNF703/FGFR1) | amp |
| PD7240 | PD7240a | Chr8:(ZNF703/FGFR1) | amp |
| PD13168 | PD13168a | Chr8:(ZNF703/FGFR1) | amp |
| PD5951 | PD5951a | Chr8:(ZNF703/FGFR1) | amp |
| PD7202 | PD7202a | Chr8:(ZNF703/FGFR1) | amp |
| PD18189 | PD18189a | Chr8:(ZNF703/FGFR1) | amp |
| PD11339 | PD11339a | Chr8:(ZNF703/FGFR1) | amp |
| PD11394 | PD11394a | Chr8:(ZNF703/FGFR1) | amp |
| PD7215 | PD7215a | Chr8:(ZNF703/FGFR1) | amp |
| PD4315 | PD4315a | Chr8:(ZNF703/FGFR1) | amp |
| PD11399 | PD11399a | Chr8:(ZNF703/FGFR1) | amp |
| PD8980 | PD8980a | Chr8:(ZNF703/FGFR1) | amp |
| PD5959 | PD5959a | Chr8:(ZNF703/FGFR1) | amp |
| PD3904 | PD3904a | Chr8:(ZNF703/FGFR1) | amp |
| PD8660 | PD8660a2 | Chr8:(ZNF703/FGFR1) | amp |
| PD14457 | PD14457a | Chr8:(ZNF703/FGFR1) | amp |
| PD4605 | PD4605a | Chr8:(ZNF703/FGFR1) | amp |
| PD6048 | PD6048a | Chr8:(ZNF703/FGFR1) | amp |
| PD9604 | PD9604a | Chr8:(ZNF703/FGFR1) | amp |
| PD11340 | PD11340a | Chr8:(ZNF703/FGFR1) | amp |
| PD4845 | PD4845a | Chr8:(ZNF703/FGFR1) | amp |
| PD11741 | PD11741a | Chr8:(ZNF703/FGFR1) | amp |
| PD18748 | PD18748a | Chr8:(ZNF703/FGFR1) | amp |
| PD14461 | PD14461a | Chr8:(ZNF703/FGFR1) | amp |
| PD9567 | PD9567a | Chr8:(ZNF703/FGFR1) | amp |
| PD6044 | PD6044a | Chr8:(ZNF703/FGFR1) | amp |
| PD14471 | PD14471a | Chr8:(ZNF703/FGFR1) | amp |
| PD8981 | PD8981a | Chr8:(ZNF703/FGFR1) | amp |
| PD6732 | PD6732b | Chr8:(ZNF703/FGFR1) | amp |
| PD11369 | PD11369a | Chr8:(ZNF703/FGFR1) | amp |
| PD9752 | PD9752a | Chr8:(ZNF703/FGFR1) | amp |
| PD4976 | PD4976a | Chr8:(ZNF703/FGFR1) | amp |
| PD9539 | PD9539a | Chr8:(ZNF703/FGFR1) | amp |
| PD18045 | PD18045a | Chr8:(ZNF703/FGFR1) | amp |
| PD18031 | PD18031a | Chr8:(ZNF703/FGFR1) | amp |
| PD11374 | PD11374a | Chr8:(ZNF703/FGFR1) | amp |
| PD18733 | PD18733a | Chr8:(ZNF703/FGFR1) | amp |
| PD4255 | PD4255a | Chr8:(ZNF703/FGFR1) | amp |
| PD24182 | PD24182a | Chr8:(ZNF703/FGFR1) | amp |

(continued)

| Sample | Tumour name | Gene | Effect |
|---|---|---|---|
| PD18047 | PD18047a | Chr8:(ZNF703/FGFR1) | amp |
| PD7344 | PD7344a | Chr8:(ZNF703/FGFR1) | amp |
| PD6044 | PD6044a | MYC | amp |
| PD18046 | PD18046a | MYC | amp |
| PD11393 | PD11393a | MYC | amp |
| PD11385 | PD11385a | MYC | amp |
| PD13162 | PD13162a | MYC | amp |
| PD17981 | PD17981a | MYC | amp |
| PD4826 | PD4826a | MYC | amp |
| PD9754 | PD9754a | MYC | amp |
| PD11398 | PD11398a | MYC | amp |
| PD6727 | PD6727b | MYC | amp |
| PD6733 | PD6733b | MYC | amp |
| PD24325 | PD24325a | MYC | amp |
| PD9002 | PD9002a | MYC | amp |
| PD24322 | PD24322a | MYC | amp |
| PD3945 | PD3945a | MYC | amp |
| PD6409 | PD6409a | MYC | amp |
| PD11339 | PD11339a | MYC | amp |
| PD14437 | PD14437a | MYC | amp |
| PD24218 | PD24218a | MYC | amp |
| PD9760 | PD9760a | MYC | amp |
| PD22361 | PD22361a | MYC | amp |
| PD18251 | PD18251a | MYC | amp |
| PD7069 | PD7069a | MYC | amp |
| PD24192 | PD24192a | MYC | amp |
| PD4955 | PD4955a | MYC | amp |
| PD4954 | PD4954a | MYC | amp |
| PD17994 | PD17994a | MYC | amp |
| PD11464 | PD11464a | MYC | amp |
| PD9567 | PD9567a | MYC | amp |
| PD9569 | PD9569a | MYC | amp |
| PD18047 | PD18047a | MYC | amp |
| PD7215 | PD7215a | MYC | amp |
| PD5942 | PD5942a | MYC | amp |
| PD23558 | PD23558a | MYC | amp |
| PD18149 | PD18149a | MYC | amp |
| PD14435 | PD14435a | MYC | amp |
| PD18022 | PD18022a | MYC | amp |

(continued)

| Sample | Tumour name | Gene | Effect |
|--------|-------------|------|--------|
| PD9599 | PD9599a | MYC | amp |
| PD11756 | PD11756a | MYC | amp |
| PD5951 | PD5951a | MYC | amp |
| PD6042 | PD6042a | MYC | amp |
| PD13602 | PD13602a | MYC | amp |
| PD4115 | PD4115a | MYC | amp |
| PD24201 | PD24201a | MYC | amp |
| PD7316 | PD7316a | MYC | amp |
| PD4005 | PD4005a | MYC | amp |
| PD4876 | PD4876a | MYC | amp |
| PD6413 | PD6413a | MYC | amp |
| PD9576 | PD9576a | MYC | amp |
| PD11394 | PD11394a | MYC | amp |
| PD13418 | PD13418a | MYC | amp |
| PD24196 | PD24196a | MYC | amp |
| PD5950 | PD5950a | MYC | amp |
| PD8611 | PD8611a | MYC | amp |
| PD24337 | PD24337a | MYC | amp |
| PD4255 | PD4255a | MYC | amp |
| PD24220 | PD24220a | MYC | amp |
| PD8980 | PD8980a | MYC | amp |
| PD13167 | PD13167a | MYC | amp |
| PD18020 | PD18020a | MYC | amp |
| PD4116 | PD4116a | MYC | amp |
| PD11375 | PD11375a | MYC | amp |
| PD7428 | PD7428a | MYC | amp |
| PD11345 | PD11345a | MYC | amp |
| PD9000 | PD9000a | MYC | amp |
| PD7250 | PD7250a | MYC | amp |
| PD23563 | PD23563a | MYC | amp |
| PD6047 | PD6047a | MYC | amp |
| PD9696 | PD9696a | MYC | amp |
| PD11750 | PD11750a | MYC | amp |
| PD9591 | PD9591a | MYC | amp |
| PD7243 | PD7243a | MYC | amp |
| PD10014 | PD10014a | MYC | amp |
| PD13299 | PD13299a | MYC | amp |
| PD11368 | PD11368a | MYC | amp |
| PD23577 | PD23577a | MYC | amp |

(continued)

| Sample | Tumour name | Gene | Effect |
|--------|-------------|------|--------|
| PD13298 | PD13298a | MYC | amp |
| PD4953 | PD4953a | MYC | amp |
| PD24214 | PD24214a | MYC | amp |
| PD24308 | PD24308a | MYC | amp |
| PD11346 | PD11346a | MYC | amp |
| PD18031 | PD18031a | MYC | amp |
| PD8969 | PD8969a | MYC | amp |
| PD11367 | PD11367a | MYC | amp |
| PD13607 | PD13607a | MYC | amp |
| PD4957 | PD4957a | MYC | amp |
| PD9604 | PD9604a | MYC | amp |
| PD5948 | PD5948a | MYC | amp |
| PD4978 | PD4978a | MYC | amp |
| PD4956 | PD4956a | MYC | amp |
| PD8984 | PD8984a | MYC | amp |
| PD4103 | PD4103a | MYC | amp |
| PD13164 | PD13164a | MYC | amp |
| PD22357 | PD22357a | MYC | amp |
| PD7066 | PD7066a | MYC | amp |
| PD13609 | PD13609a | MYC | amp |
| PD24215 | PD24215a | MYC | amp |
| PD6728 | PD6728b | MYC | amp |
| PD9702 | PD9702a | MYC | amp |
| PD9539 | PD9539a | MYC | amp |
| PD18045 | PD18045a | MYC | amp |
| PD4841 | PD4841a | MYC | amp |
| PD18734 | PD18734a | MYC | amp |
| PD24191 | PD24191a | MYC | amp |
| PD4315 | PD4315a | MYC | amp |
| PD3890 | PD3890a | MYC | amp |
| PD8982 | PD8982a | MYC | amp |
| PD8996 | PD8996a | MYC | amp |
| PD13166 | PD13166a | MYC | amp |
| PD24212 | PD24212a | CDKN2A | HD |
| PD24212 | PD24212a | CDKN2B | HD |
| PD7067 | PD7067a | CDKN2A | HD |
| PD7067 | PD7067a | CDKN2B | HD |
| PD5956 | PD5956a | CDKN2A | HD |
| PD5956 | PD5956a | CDKN2B | HD |

(continued)

| Sample | Tumour name | Gene | Effect |
|--------|-------------|------|--------|
| PD8980 | PD8980a | CDKN2A | HD |
| PD8980 | PD8980a | CDKN2B | HD |
| PD23560 | PD23560a | CDKN2A | HD |
| PD23560 | PD23560a | CDKN2B | HD |
| PD11748 | PD11748a | CDKN2A | HD |
| PD11748 | PD11748a | CDKN2B | HD |
| PD4876 | PD4876a | CDKN2A | HD |
| PD4876 | PD4876a | CDKN2B | HD |
| PD24209 | PD24209a | CDKN2A | HD |
| PD24209 | PD24209a | CDKN2B | HD |
| PD14467 | PD14467a | CDKN2A | HD |
| PD14467 | PD14467a | CDKN2B | HD |
| PD8830 | PD8830a | CDKN2A | HD |
| PD8830 | PD8830a | CDKN2B | HD |
| PD13627 | PD13627a | CDKN2A | HD |
| PD6410 | PD6410a | CDKN2A | HD |
| PD13627 | PD13627a | CDKN2B | HD |
| PD6410 | PD6410a | CDKN2B | HD |
| PD11364 | PD11364a | CDKN2A | HD |
| PD11364 | PD11364a | CDKN2B | HD |
| PD4836 | PD4836a | CDKN2A | HD |
| PD8832 | PD8832a | CDKN2A | HD |
| PD8832 | PD8832a | CDKN2B | HD |
| PD5935 | PD5935a | PTEN | HD |
| PD13311 | PD13311a | PTEN | HD |
| PD4844 | PD4844a | PTEN | HD |
| PD13763 | PD13763a | PTEN | HD |
| PD23562 | PD23562a | PTEN | HD |
| PD24325 | PD24325a | PTEN | HD |
| PD14460 | PD14460a | PTEN | HD |
| PD5945 | PD5945a | PTEN | HD |
| PD7220 | PD7220a | PTEN | HD |
| PD4607 | PD4607a | PTEN | HD |
| PD23558 | PD23558a | PTEN | HD |
| PD23577 | PD23577a | PTEN | HD |
| PD13627 | PD13627a | PTEN | HD |
| PD9752 | PD9752a | PTEN | HD |
| PD18024 | PD18024a | PTEN | HD |
| PD4874 | PD4874a | PTEN | HD |

(continued)

| Sample | Tumour name | Gene | Effect |
|--------|-------------|------|--------|
| PD9571 | PD9571a | FGFR2 | amp |
| PD5948 | PD5948a | FGFR2 | amp |
| PD14462 | PD14462a | CCND1 | amp |
| PD7341 | PD7341a | CCND1 | amp |
| PD11399 | PD11399a | CCND1 | amp |
| PD4965 | PD4965a | CCND1 | amp |
| PD9599 | PD9599a | CCND1 | amp |
| PD11760 | PD11760a | CCND1 | amp |
| PD9754 | PD9754a | CCND1 | amp |
| PD14467 | PD14467a | CCND1 | amp |
| PD8999 | PD8999a | CCND1 | amp |
| PD11337 | PD11337a | CCND1 | amp |
| PD7322 | PD7322a | CCND1 | amp |
| PD24332 | PD24332a | CCND1 | amp |
| PD14461 | PD14461a | CCND1 | amp |
| PD24199 | PD24199a | CCND1 | amp |
| PD5956 | PD5956a | CCND1 | amp |
| PD7202 | PD7202a | CCND1 | amp |
| PD9752 | PD9752a | CCND1 | amp |
| PD13168 | PD13168a | CCND1 | amp |
| PD13766 | PD13766a | CCND1 | amp |
| PD13424 | PD13424a | CCND1 | amp |
| PD22362 | PD22362a | CCND1 | amp |
| PD11348 | PD11348a | CCND1 | amp |
| PD13771 | PD13771a | CCND1 | amp |
| PD13607 | PD13607a | CCND1 | amp |
| PD4959 | PD4959a | CCND1 | amp |
| PD11374 | PD11374a | CCND1 | amp |
| PD18257 | PD18257a | CCND1 | amp |
| PD9591 | PD9591a | CCND1 | amp |
| PD13307 | PD13307a | CCND1 | amp |
| PD9539 | PD9539a | CCND1 | amp |
| PD5946 | PD5946a | CCND1 | amp |
| PD13620 | PD13620a | CCND1 | amp |
| PD18751 | PD18751a | CCND1 | amp |
| PD6045 | PD6045a | CCND1 | amp |
| PD7069 | PD7069a | CCND1 | amp |
| PD14473 | PD14473a | CCND1 | amp |
| PD5964 | PD5964a | CCND1 | amp |

(continued)

| Sample | Tumour name | Gene | Effect |
|---|---|---|---|
| PD8609 | PD8609a | CCND1 | amp |
| PD7204 | PD7204a | CCND1 | amp |
| PD14435 | PD14435a | CCND1 | amp |
| PD4116 | PD4116a | CCND1 | amp |
| PD6044 | PD6044a | CCND1 | amp |
| PD14433 | PD14433a | CCND1 | amp |
| PD11394 | PD11394a | CCND1 | amp |
| PD5951 | PD5951a | CCND1 | amp |
| PD4315 | PD4315a | CCND1 | amp |
| PD13302 | PD13302a | CCND1 | amp |
| PD9541 | PD9541a | CCND1 | amp |
| PD11396 | PD11396a | CCND1 | amp |
| PD4969 | PD4969a | CCND1 | amp |
| PD7306 | PD7306a | CCND1 | amp |
| PD8995 | PD8995a | CCND1 | amp |
| PD24219 | PD24219a | CCND1 | amp |
| PD17981 | PD17981a | CCND1 | amp |
| PD13163 | PD13163a | CCND1 | amp |
| PD4971 | PD4971a | CCND1 | amp |
| PD6048 | PD6048a | CCND1 | amp |
| PD11343 | PD11343a | CCND1 | amp |
| PD11393 | PD11393a | CCND1 | amp |
| PD7305 | PD7305a | CCND1 | amp |
| PD4103 | PD4103a | CCND1 | amp |
| PD9582 | PD9582a | CCND1 | amp |
| PD8977 | PD8977a | CCND1 | amp |
| PD9567 | PD9567a | CCND1 | amp |
| PD23550 | PD23550a | CCND1 | amp |
| PD11385 | PD11385a | CCND1 | amp |
| PD18251 | PD18251a | CCND1 | amp |
| PD11346 | PD11346a | CCND1 | amp |
| PD11761 | PD11761a | CCND1 | amp |
| PD9755 | PD9755a | CCND1 | amp |
| PD13630 | PD13630a | CCND1 | amp |
| PD6046 | PD6046a | CCND1 | amp |
| PD11340 | PD11340a | CCND1 | amp |
| PD6047 | PD6047a | CCND1 | amp |
| PD8980 | PD8980a | CCND1 | amp |
| PD9759 | PD9759a | CCND1 | amp |

(continued)

| Sample | Tumour name | Gene | Effect |
|--------|-------------|------|--------|
| PD11741 | PD11741a | CCND1 | amp |
| PD13606 | PD13606a | CCND1 | amp |
| PD14459 | PD14459a | CCND1 | amp |
| PD4198 | PD4198a | CCND1 | amp |
| PD13608 | PD13608a | CCND1 | amp |
| PD14460 | PD14460a | CCND1 | amp |
| PD4976 | PD4976a | CCND1 | amp |
| PD11360 | PD11360a | CCND1 | amp |
| PD24327 | PD24327a | CCND1 | amp |
| PD11372 | PD11372a | CCND1 | amp |
| PD9605 | PD9605a | CCND1 | amp |
| PD14465 | PD14465a | CCND1 | amp |
| PD5945 | PD5945a | KRAS | amp |
| PD7316 | PD7316a | KRAS | amp |
| PD11327 | PD11327a | KRAS | amp |
| PD4975 | PD4975a | KRAS | amp |
| PD6733 | PD6733b | KRAS | amp |
| PD9582 | PD9582a | KRAS | amp |
| PD11752 | PD11752a | KRAS | amp |
| PD7205 | PD7205a | MDM2 | amp |
| PD6422 | PD6422a | MDM2 | amp |
| PD4978 | PD4978a | MDM2 | amp |
| PD9000 | PD9000a | MDM2 | amp |
| PD18149 | PD18149a | MDM2 | amp |
| PD13605 | PD13605a | MDM2 | amp |
| PD22362 | PD22362a | MDM2 | amp |
| PD11369 | PD11369a | MDM2 | amp |
| PD4315 | PD4315a | MDM2 | amp |
| PD11360 | PD11360a | MDM2 | amp |
| PD11368 | PD11368a | MDM2 | amp |
| PD7307 | PD7307a | MDM2 | amp |
| PD5946 | PD5946a | MDM2 | amp |
| PD13766 | PD13766a | MDM2 | amp |
| PD6733 | PD6733b | MDM2 | amp |
| PD5960 | PD5960a | BRCA2 | HD |
| PD13608 | PD13608a | RB1 | HD |
| PD18020 | PD18020a | RB1 | HD |
| PD22355 | PD22355a | RB1 | HD |
| PD4952 | PD4952a | RB1 | HD |

(continued)

| Sample | Tumour name | Gene | Effect |
|--------|-------------|------|--------|
| PD24192 | PD24192a | RB1 | HD |
| PD8997 | PD8997a | AKT1 | amp |
| PD9001 | PD9001a | AKT1 | amp |
| PD24201 | PD24201a | AKT1 | amp |
| PD11369 | PD11369a | IGF1R | amp |
| PD7428 | PD7428a | IGF1R | amp |
| PD5928 | PD5928a | IGF1R | amp |
| PD6415 | PD6415a | IGF1R | amp |
| PD4968 | PD4968a | IGF1R | amp |
| PD7307 | PD7307a | IGF1R | amp |
| PD9760 | PD9760a | IGF1R | amp |
| PD13764 | PD13764a | IGF1R | amp |
| PD9582 | PD9582a | IGF1R | amp |
| PD18020 | PD18020a | IGF1R | amp |
| PD9464 | PD9464a | IGF1R | amp |
| PD4967 | PD4967a | CDH1 | HD |
| PD4606 | PD4606a | TP53 | HD |
| PD11367 | PD11367a | MAP2K4 | HD |
| PD14471 | PD14471a | MAP2K4 | HD |
| PD7206 | PD7206a | MAP2K4 | HD |
| PD11347 | PD11347a | MAP2K4 | HD |
| PD13760 | PD13760a | MAP2K4 | HD |
| PD18050 | PD18050a | MAP2K4 | HD |
| PD8609 | PD8609a | MAP2K4 | HD |
| PD4088 | PD4088a | MAP2K4 | HD |
| PD4604 | PD4604a | MAP2K4 | HD |
| PD4606 | PD4606a | MAP2K4 | HD |
| PD13608 | PD13608a | MAP2K4 | HD |
| PD13753 | PD13753a | MAP2K4 | HD |
| PD6719 | PD6719a | MAP2K4 | HD |
| PD13625 | PD13625a | MAP2K4 | HD |
| PD11462 | PD11462a | MAP2K4 | HD |
| PD11367 | PD11367a | NCOR1 | HD |
| PD24192 | PD24192a | NF1 | HD |
| PD8995 | PD8995a | ERBB2 | amp |
| PD13603 | PD13603a | ERBB2 | amp |
| PD7307 | PD7307a | ERBB2 | amp |
| PD24194 | PD24194a | ERBB2 | amp |
| PD18189 | PD18189a | ERBB2 | amp |

(continued)

| Sample | Tumour name | Gene | Effect |
|--------|-------------|------|--------|
| PD24332 | PD24332a | ERBB2 | amp |
| PD7341 | PD7341a | ERBB2 | amp |
| PD7069 | PD7069a | ERBB2 | amp |
| PD4613 | PD4613a | ERBB2 | amp |
| PD4826 | PD4826a | ERBB2 | amp |
| PD13604 | PD13604a | ERBB2 | amp |
| PD5950 | PD5950a | ERBB2 | amp |
| PD22362 | PD22362a | ERBB2 | amp |
| PD24207 | PD24207a | ERBB2 | amp |
| PD18188 | PD18188a | ERBB2 | amp |
| PD24335 | PD24335a | ERBB2 | amp |
| PD4199 | PD4199a | ERBB2 | amp |
| PD13163 | PD13163a | ERBB2 | amp |
| PD7204 | PD7204a | ERBB2 | amp |
| PD8998 | PD8998a | ERBB2 | amp |
| PD9576 | PD9576a | ERBB2 | amp |
| PD18047 | PD18047a | ERBB2 | amp |
| PD18050 | PD18050a | ERBB2 | amp |
| PD13605 | PD13605a | ERBB2 | amp |
| PD24220 | PD24220a | ERBB2 | amp |
| PD22359 | PD22359a | ERBB2 | amp |
| PD9009 | PD9009a | ERBB2 | amp |
| PD13168 | PD13168a | ERBB2 | amp |
| PD7205 | PD7205a | ERBB2 | amp |
| PD6048 | PD6048a | ERBB2 | amp |
| PD7203 | PD7203a | ERBB2 | amp |
| PD22366 | PD22366a | ERBB2 | amp |
| PD18048 | PD18048a | ERBB2 | amp |
| PD18045 | PD18045a | ERBB2 | amp |
| PD13166 | PD13166a | ERBB2 | amp |
| PD13164 | PD13164a | ERBB2 | amp |
| PD4198 | PD4198a | ERBB2 | amp |
| PD23560 | PD23560a | ERBB2 | amp |
| PD22357 | PD22357a | ERBB2 | amp |
| PD7202 | PD7202a | ERBB2 | amp |
| PD18049 | PD18049a | ERBB2 | amp |
| PD13602 | PD13602a | ERBB2 | amp |
| PD4192 | PD4192a | ERBB2 | amp |
| PD11345 | PD11345a | ERBB2 | amp |

(continued)

| Sample | Tumour name | Gene | Effect |
|---|---|---|---|
| PD11349 | PD11349a | ERBB2 | amp |
| PD11465 | PD11465a | ERBB2 | amp |
| PD7306 | PD7306a | ERBB2 | amp |
| PD13609 | PD13609a | ERBB2 | amp |
| PD4194 | PD4194a | ERBB2 | amp |
| PD11346 | PD11346a | ERBB2 | amp |
| PD24217 | PD24217a | ERBB2 | amp |
| PD7305 | PD7305a | ERBB2 | amp |
| PD8997 | PD8997a | ERBB2 | amp |
| PD13608 | PD13608a | ERBB2 | amp |
| PD4962 | PD4962a | ERBB2 | amp |
| PD11344 | PD11344a | ERBB2 | amp |
| PD23561 | PD23561a | ERBB2 | amp |
| PD8996 | PD8996a | ERBB2 | amp |
| PD13165 | PD13165a | ERBB2 | amp |
| PD13167 | PD13167a | ERBB2 | amp |
| PD6405 | PD6405a | ERBB2 | amp |
| PD24225 | PD24225a | ERBB2 | amp |
| PD4978 | PD4978a | ERBB2 | amp |
| PD11464 | PD11464a | ERBB2 | amp |
| PD11462 | PD11462a | ERBB2 | amp |
| PD24322 | PD24322a | ERBB2 | amp |
| PD7249 | PD7249a | ERBB2 | amp |
| PD9755 | PD9755a | ERBB2 | amp |
| PD24209 | PD24209a | ERBB2 | amp |
| PD7304 | PD7304a | ERBB2 | amp |
| PD4876 | PD4876a | SMAD4 | HD |
| PD24308 | PD24308a | CCNE1 | amp |
| PD4841 | PD4841a | CCNE1 | amp |
| PD24196 | PD24196a | CCNE1 | amp |
| PD24325 | PD24325a | CCNE1 | amp |
| PD7428 | PD7428a | CCNE1 | amp |
| PD8660 | PD8660a2 | CCNE1 | amp |
| PD18020 | PD18020a | CCNE1 | amp |
| PD4192 | PD4192a | CCNE1 | amp |
| PD13622 | PD13622a | CCNE1 | amp |
| PD6732 | PD6732b | CCNE1 | amp |
| PD14457 | PD14457a | CCNE1 | amp |
| PD24190 | PD24190a | CCNE1 | amp |

(continued)

| Sample | Tumour name | Gene | Effect |
|--------|-------------|------|--------|
| PD4086 | PD4086a | CCNE1 | amp |
| PD13296 | PD13296a | CCNE1 | amp |
| PD8660 | PD8660a2 | AKT2 | amp |
| PD13424 | PD13424a | AKT2 | amp |
| PD24190 | PD24190a | AKT2 | amp |
| PD14457 | PD14457a | AKT2 | amp |
| PD9002 | PD9002a | ZNF217 | amp |
| PD9567 | PD9567a | ZNF217 | amp |
| PD6727 | PD6727b | ZNF217 | amp |
| PD18264 | PD18264a | ZNF217 | amp |
| PD11337 | PD11337a | ZNF217 | amp |
| PD7243 | PD7243a | ZNF217 | amp |
| PD6417 | PD6417a | ZNF217 | amp |
| PD7069 | PD7069a | ZNF217 | amp |
| PD13609 | PD13609a | ZNF217 | amp |
| PD11743 | PD11743a | ZNF217 | amp |
| PD13606 | PD13606a | ZNF217 | amp |
| PD23550 | PD23550a | ZNF217 | amp |
| PD11345 | PD11345a | ZNF217 | amp |
| PD14435 | PD14435a | ZNF217 | amp |
| PD14453 | PD14453a | ZNF217 | amp |
| PD13165 | PD13165a | ZNF217 | amp |
| PD4978 | PD4978a | ZNF217 | amp |
| PD24206 | PD24206a | ZNF217 | amp |
| PD18734 | PD18734a | ZNF217 | amp |
| PD8973 | PD8973a | ZNF217 | amp |
| PD5964 | PD5964a | ZNF217 | amp |
| PD11818 | PD11818a | ZNF217 | amp |
| PD13608 | PD13608a | ZNF217 | amp |
| PD18045 | PD18045a | ZNF217 | amp |
| PD8981 | PD8981a | ZNF217 | amp |
| PD11742 | PD11742a | ZNF217 | amp |
| PD13602 | PD13602a | ZNF217 | amp |
| PD13622 | PD13622a | ZNF217 | amp |
| PD11464 | PD11464a | ZNF217 | amp |
| PD11368 | PD11368a | ZNF217 | amp |
| PD3851 | PD3851a | ZNF217 | amp |
| PD8980 | PD8980a | ZNF217 | amp |
| PD18020 | PD18020a | ZNF217 | amp |

(continued)

| Sample | Tumour name | Gene | Effect |
|--------|-------------|------|--------|
| PD4103 | PD4103a | ZNF217 | amp |
| PD4315 | PD4315a | ZNF217 | amp |
| PD7305 | PD7305a | ZNF217 | amp |

**Table 7C**

| Sample | Gene | Rearrangements |
|--------|------|----------------|
| PD8982a | ARID1B | 1\|Tandem duplication |
| PD13299a | ARID1B | 2\|Translocation\|Translocation |
| PD7215a | ARID1B | 1\|Translocation |
| PD13165a | ARID1B | 1\|Tandem duplication |
| PD5932a | ARID1B | 1\|Deletion |
| PD9760a | ARID1B | 1\|Deletion |
| PD4956a | ARID1B | 1\|Tandem duplication |
| PD4116a | ARID1B | 1\|Translocation |
| PD4315a | ARID1B | 2\|Translocation\|Deletion |
| PD24212a | ARID1B | 1\|Inversion |
| PD7341a | ARID1B | 4\|Inversion\|Translocation\|Deletion\|Inversion |
| PD24303a | ARID1B | 1\|Translocation |
| PD14437a | ARID1B | 4\|Inversion\|Deletion\|Deletion\|Tandem duplication |
| PD9592a | ARID1B | 1\|Tandem duplication |
| PD5942a | ARID1B | 1\|Deletion |
| PD5948a | ARID1B | 1\|Tandem duplication |
| PD5930a | ARID1B | 1\|Translocation |
| PD4248a | ARID1B | 2\|Tandem duplication\|Deletion |
| PD5956a | ARID1B | 1\|Tandem duplication |
| PD23577a | ARID1B | 2\|Translocation\|Translocation |
| PD11396a | ARID1B | 2\|Deletion\|Translocation |
| PD11818a | ARID1B | 3\|Tandem duplication\|Deletion\|Inversion |
| PD23567a | ARID1B | 1\|Translocation |
| PD23566a | ARID1B | 1\|Tandem duplication |
| PD24208a | CDKN2A | 1\|Tandem duplication |
| PD5925a | CDKN2A | 1\|Deletion |
| PD7316a | CDKN2A | 1\|Deletion |
| PD13602a | CDKN2A | 1\|Deletion |
| PD24337a | CDKN2A | 1\|Deletion |
| PD13752a | CDKN2A | 2\|Translocation\|Translocation |
| PD4005a | FBXW7 | 1 \|Translocation |
| PD24201a | FBXW7 | 1\|Tandem duplication |
| PD7248a | FBXW7 | 1\|Inversion |

(continued)

| Sample | Gene | Rearrangements |
|---|---|---|
| PD9702a | FBXW7 | 1|Deletion |
| PD4844a | FBXW7 | 2|Translocation|Translocation |
| PD4315a | FBXW7 | 1|Tandem duplication |
| PD6404a | FBXW7 | 1|Deletion |
| PD7211a | FBXW7 | 1|Tandem duplication |
| PD5948a | FBXW7 | 1|Deletion |
| PD10011a | FBXW7 | 1|Tandem duplication |
| PD4255a | FBXW7 | 1|Tandem duplication |
| PD11762a | FBXW7 | 2|Deletion|Inversion |
| PD4845a | FBXW7 | 1|Tandem duplication |
| PD23566a | FBXW7 | 1|Tandem duplication |
| PD13425a | MAP2K4 | 1|Tandem duplication |
| PD9597a | MAP2K4 | 1 |Translocation |
| PD9572a | MAP2K4 | 1|Inversion |
| PD7305a | MAP2K4 | 2|Inversion|Translocation |
| PD14437a | MAP2K4 | 1|Deletion |
| PD22355a | MAP2K4 | 2|Translocation|Translocation |
| PD14441a | MAP2K4 | 2|Inversion|Translocation |
| PD11761a | MAP2K4 | 1|Deletion |
| PD14454a | MAP2K4 | 1|Deletion |
| PD11380a | MAP2K4 | 1|Deletion |
| PD6417a | MAP2K4 | 1|Translocation |
| PD11385a | MAP2K4 | 1|Deletion |
| PD9754a | MAP2K4 | 1|Tandem duplication |
| PD13626a | MAP2K4 | 1|Inversion |
| PD22361a | MAP3K1 | 1|Tandem duplication |
| PD13307a | MAP3K1 | 1|Translocation |
| PD4956a | MAP3K1 | 1|Translocation |
| PD22360a | MAP3K1 | 1|Translocation |
| PD4847a | MAP3K1 | 1|Tandem duplication |
| PD4836a | MAP3K1 | 1|Deletion |
| PD13602a | MAP3K1 | 1|Tandem duplication |
| PD7201a | MAP3K1 | 2|Deletion|Tandem duplication |
| PD4225a | MAP3K1 | 3|Inversion|Inversion|Deletion |
| PD8618a | MAP3K1 | 1|Deletion |
| PD11740a | MAP3K1 | 1|Deletion |
| PD6722a | PTEN | 1|Deletion |
| PD22357a | PTEN | 1|Inversion |
| PD7426a | PTEN | 2|Inversion|Inversion |

(continued)

| Sample | Gene | Rearrangements |
|--------|------|----------------|
| PD7217a | PTEN | 1|Deletion |
| PD7248a | PTEN | 1|Tandem duplication |
| PD9004a | PTEN | 1|Deletion |
| PD6733b | PTEN | 2|Deletion|Deletion |
| PD4116a | PTEN | 1|Deletion |
| PD9585a | PTEN | 1|Tandem duplication |
| PD9702a | PTEN | 2|Tandem duplication|Tandem duplication |
| PD8984a | PTEN | 1|Deletion |
| PD7321a | PTEN | 1|Tandem duplication |
| PD24202a | PTEN | 1|Tandem duplication |
| PD8611a | PTEN | 2|Tandem duplication|Tandem duplication |
| PD9575a | PTEN | 1|Inversion |
| PD6413a | PTEN | 2|Inversion|Translocation |
| PD8964a | PTEN | 1|Deletion |
| PD6410a | PTEN | 2|Inversion|Inversion |
| PD9696a | PTEN | 1|Deletion |
| PD11327a | PTEN | 1|Deletion |
| PD9752a | PTEN | 1|Inversion |
| PD8621a | PTEN | 1|Tandem duplication |
| PD24303a | PTEN | 1|Tandem duplication |
| PD7211a | PTEN | 1|Tandem duplication |
| PD11347a | PTEN | 1|Deletion |
| PD11757a | PTEN | 1|Deletion |
| PD5948a | PTEN | 1|Tandem duplication |
| PD9595a | PTEN | 1|Deletion |
| PD7316a | PTEN | 1|Deletion |
| PD5934a | PTEN | 1|Tandem duplication |
| PD24186a | PTEN | 1|Deletion |
| PD5960a | PTEN | 1|Deletion |
| PD6406a | PTEN | 1|Tandem duplication |
| PD23563a | PTEN | 1|Deletion |
| PD9571a | PTEN | 1|Deletion |
| PD10010a | PTEN | 1|Deletion |
| PD11755a | PTEN | 1|Tandem duplication |
| PD24306a | PTEN | 1|Tandem duplication |
| PD9579a | PTEN | 1|Deletion |
| PD13622a | PTEN | 1|Deletion |
| PD24200a | PTEN | 1|Deletion |
| PD6731a2 | RB1 | 2|Inversion|Inversion |

(continued)

| Sample | Gene | Rearrangements |
|---|---|---|
| PD7426a | RB1 | 1\|Deletion |
| PD6684a | RB1 | 1\|Tandem duplication |
| PD18769a | RB1 | 1\|Deletion |
| PD6415a | RB1 | 1\|Translocation |
| PD4005a | RB1 | 1\|Tandem duplication |
| PD13296a | RB1 | 1\|Tandem duplication |
| PD22366a | RB1 | 1\|Tandem duplication |
| PD9585a | RB1 | 1\|Deletion |
| PD9702a | RB1 | 1\|Tandem duplication |
| PD7321a | RB1 | 1\|Deletion |
| PD11326a | RB1 | 1\|Tandem duplication |
| PD9064a | RB1 | 1\|Tandem duplication |
| PD3905a | RB1 | 1\|Tandem duplication |
| PD8621a | RB1 | 1\|Tandem duplication |
| PD23562a | RB1 | 1\|Deletion |
| PD5928a | RB1 | 2\|Translocation\|Translocation |
| PD5945a | RB1 | 1\|Tandem duplication |
| PD9592a | RB1 | 1\|Deletion |
| PD5930a | RB1 | 1 \|Translocation |
| PD7250a | RB1 | 1\|Tandem duplication |
| PD23578a | RB1 | 1\|Deletion |
| PD13428a | RB1 | 1\|Deletion |
| PD24304a | RB1 | 2\|Inversion\|Inversion |
| PD5944a | RB1 | 1\|Deletion |
| PD23566a | RB1 | 1\|Tandem duplication |
| PD11344a | TP53 | 1\|Translocation |
| PD13771a | TP53 | 1\|Deletion |
| PD4952a | TP53 | 1\|Deletion |
| PD4116a | TP53 | 1\|Deletion |
| PD24190a | TP53 | 1\|Deletion |
| PD11340a | TP53 | 1\|Deletion |
| PD13602a | TP53 | 1\|Tandem duplication |
| PD5961a | TP53 | 1\|Translocation |
| PD24218a | TP53 | 1\|Deletion |
| PD6414a | TP53 | 1\|Tandem duplication |
| PD4845a | TP53 | 1\|Tandem duplication |
| PD8660a2 | MLLT4 | 1\|Tandem duplication |
| PD11750a | MLLT4 | 1\|Translocation |
| PD4005a | MLLT4 | 1\|Translocation |

(continued)

| Sample | Gene | Rearrangements |
|--------|------|----------------|
| PD11751a | MLLT4 | 1\|Translocation |
| PD9585a | MLLT4 | 3\|Translocation\|Translocation\|Tandem duplication |
| PD9702a | MLLT4 | 1\|Tandem duplication |
| PD9595a | MLLT4 | 1\|Deletion |
| PD8997a | MLLT4 | 1\|Deletion |
| PD23563a | MLLT4 | 1\|Inversion |
| PD11380a | MLLT4 | 1\|Deletion |
| PD4264a | MLLT4 | 1\|Inversion |
| PD11752a | MLLT4 | 1\|Tandem duplication |
| PD8996a | MLLT4 | 1\|Deletion |
| PD5956a | MLLT4 | 1\|Deletion |
| PD24329a | MLLT4 | 1 \|Translocation |
| PD23577a | MLLT4 | 1\|Tandem duplication |

**Table 7D**

| Sample | Tumour_name | Gene | Mutation_Type | Effect |
|--------|-------------|------|---------------|--------|
| PD11389 | PD11389a | AKT1 | Sub | missense |
| PD9845 | PD9845a | AKT1 | Sub | missense |
| PD8977 | PD8977a | AKT1 | Sub | missense |
| PD4248 | PD4248a | AKT1 | Sub | missense |
| PD14453 | PD14453a | AKT1 | Sub | missense |
| PD6043 | PD6043a | AKT1 | Sub | missense |
| PD9572 | PD9572a | AKT1 | Sub | missense |
| PD14462 | PD14462a | AKT1 | Sub | missense |
| PD8609 | PD8609a | AKT1 | Sub | missense |
| PD22364 | PD22364a | AKT1 | Sub | missense |
| PD11765 | PD11765a | AKT1 | Sub | missense |
| PD13428 | PD13428a | AKT1 | Sub | missense |
| PD17973 | PD17973a | AKT1 | Sub | missense |
| PD24216 | PD24216a | AKT1 | Sub | missense |
| PD24201 | PD24201a | AKT1 | CopyNumber | amp |
| PD8997 | PD8997a | AKT1 | CopyNumber | amp |
| PD9001 | PD9001a | AKT1 | CopyNumber | amp |
| PD9541 | PD9541a | AKT2 | Sub | missense |
| PD13424 | PD13424a | AKT2 | CopyNumber | amp |
| PD14457 | PD14457a | AKT2 | CopyNumber | amp |
| PD24190 | PD24190a | AKT2 | CopyNumber | amp |
| PD8660 | PD8660a2 | AKT2 | CopyNumber | amp |
| PD11365 | PD11365a | APC | Ins | frameshift |

(continued)

| Sample | Tumour_name | Gene | Mutation_Type | Effect |
|---|---|---|---|---|
| PD9065 | PD9065a | ARID1A | Del | frameshift |
| PD14467 | PD14467a | ARID1A | Del | frameshift |
| PD9760 | PD9760a | ARID1A | Ins | frameshift |
| PD9063 | PD9063a | ARID1A | Sub | nonsense |
| PD7322 | PD7322a | ARID1A | Sub | nonsense |
| PD11375 | PD11375a | ARID1A | Del | frameshift |
| PD4613 | PD4613a | ARID1A | Sub | nonsense |
| PD17981 | PD17981a | ARID1A | Del | frameshift |
| PD5937 | PD5937a | ARID1A | Ins | frameshift |
| PD11402 | PD11402a | ARID1A | Ins | frameshift |
| PD5937 | PD5937a | ARID1A | Del | frameshift |
| PD11399 | PD11399a | ARID1A | Del | inframe |
| PD7250 | PD7250a | ARID1A | Del | frameshift |
| PD11381 | PD11381a | ARID1A | Ins | frameshift |
| PD11397 | PD11397a | ARID1A | Sub | nonsense |
| PD7206 | PD7206a | ARID1A | Sub | nonsense |
| PD14441 | PD14441a | ARID1A | Del | frameshift |
| PD23579 | PD23579a | ARID1B | Ins | frameshift |
| PD9063 | PD9063a | ARID1B | Sub | nonsense |
| PD4252 | PD4252a | ARID1B | Sub | nonsense |
| PD4844 | PD4844a | ARID1B | Del | frameshift |
| PD8982 | PD8982a | ARID1B | Rearrangement | 1|Tandem duplication |
| PD13299 | PD13299a | ARID1B | Rearrangement | 2|Translocation| Translocation |
| PD7215 | PD7215a | ARID1B | Rearrangement | 1|Translocation |
| PD13165 | PD13165a | ARID1B | Rearrangement | 1|Tandem duplication |
| PD5932 | PD5932a | ARID1B | Rearrangement | 1|Deletion |
| PD9760 | PD9760a | ARID1B | Rearrangement | 1|Deletion |
| PD4956 | PD4956a | ARID1B | Rearrangement | 1|Tandem duplication |
| PD4116 | PD4116a | ARID1B | Rearrangement | 1|Translocation |
| PD4315 | PD4315a | ARID1B | Rearrangement | 2|Translocation| Deletion |
| PD24212 | PD24212a | ARID1B | Rearrangement | 1|Inversion |
| PD7341 | PD7341a | ARID1B | Rearrangement | 4|Inversion| Translocation| Deletion| Inversion |
| PD24303 | PD24303a | ARID1B | Rearrangement | 1|Translocation |
| PD14437 | PD14437a | ARID1B | Rearrangement | 4|Inversion|Deletion| Deletion|Tandem duplication |
| PD9592 | PD9592a | ARID1B | Rearrangement | 1|Tandem duplication |
| PD5942 | PD5942a | ARID1B | Rearrangement | 1|Deletion |
| PD5948 | PD5948a | ARID1B | Rearrangement | 1|Tandem duplication |
| PD5930 | PD5930a | ARID1B | Rearrangement | 1|Translocation |

(continued)

| Sample | Tumour_name | Gene | Mutation_Type | Effect |
|--------|-------------|------|---------------|--------|
| PD4248 | PD4248a | ARID1B | Rearrangement | 2\|Tandem duplication\| Deletion |
| PD5956 | PD5956a | ARID1B | Rearrangement | 1\|Tandem duplication |
| PD23577 | PD23577a | ARID1B | Rearrangement | 2\|Translocation\| Translocation |
| PD11396 | PD11396a | ARID1B | Rearrangement | 2\|Deletion\|Translocation |
| PD11818 | PD11818a | ARID1B | Rearrangement | 3\|Tandem duplication\| Deletion\| Inversion |
| PD23567 | PD23567a | ARID1B | Rearrangement | 1\|Translocation |
| PD23566 | PD23566a | ARID1B | Rearrangement | 1\|Tandem duplication |
| PD10011 | PD10011a | ASXL1 | Del | frameshift |
| PD5937 | PD5937a | ATM | Sub | nonsense |
| PD4198 | PD4198a | ATM | Sub | missense |
| PD14457 | PD14457a | ATR | Sub | nonsense |
| PD23564 | PD23564a | ATR | Del | frameshift |
| PD5956 | PD5956a | ATR | Del | frameshift |
| PD18749 | PD18749a | ATRX | Sub | nonsense |
| PD11462 | PD11462a | ATRX | Sub | ess splice |
| PD5937 | PD5937a | AXIN1 | Ins | frameshift |
| PD6412 | PD6412a | AXIN1 | Sub | missense |
| PD4958 | PD4958a | BCOR | Sub | ess splice |
| PD4109 | PD4109a | BCOR | Sub | nonsense |
| PD24333 | PD24333a | BRAF | Sub | missense |
| PD11742 | PD11742a | BRCA1 | Ins | frameshift |
| PD7215 | PD7215a | BRCA1 | Sub | missense |
| PD9585 | PD9585a | BRCA1 | Sub | nonsense |
| PD7067 | PD7067a | BRCA1 | Sub | nonsense |
| PD23561 | PD23561a | BRCA1 | Ins | frameshift |
| PD13604 | PD13604a | BRCA2 | Sub | nonsense |
| PD11372 | PD11372a | BRCA2 | Sub | nonsense |
| PD24191 | PD24191a | BRCA2 | Del | frameshift |
| PD23565 | PD23565a | BRCA2 | Sub | nonsense |
| PD13604 | PD13604a | BRCA2 | Sub | nonsense |
| PD6042 | PD6042a | BRCA2 | Sub | nonsense |
| PD24326 | PD24326a | BRCA2 | Sub | nonsense |
| PD9000 | PD9000a | BRCA2 | Del | frameshift |
| PD8978 | PD8978a | BRCA2 | Del | frameshift |
| PD8984 | PD8984a | BRCA2 | Ins | frameshift |
| PD5960 | PD5960a | BRCA2 | CopyNumber | HD |
| PD23579 | PD23579a | BUB1B | Del | frameshift |
| PD4607 | PD4607a | CASP8 | Sub | nonsense |
| PD7209 | PD7209a | CBFB | Sub | start-lost |

(continued)

| Sample | Tumour_name | Gene | Mutation_Type | Effect |
|---|---|---|---|---|
| PD7210 | PD7210a | CBFB | Sub | ess splice |
| PD13310 | PD13310a | CBFB | Sub | nonsense |
| PD18046 | PD18046a | CBFB | Sub | nonsense |
| PD9761 | PD9761a | CBFB | Sub | ess splice |
| PD13762 | PD13762a | CBFB | Sub | ess splice |
| PD17973 | PD17973a | CBFB | Sub | ess splice |
| PD18047 | PD18047a | CBFB | Ins | ess splice |
| PD4982 | PD4982a | CBFB | Sub | ess splice |
| PD14458 | PD14458a | CBFB | Sub | ess splice |
| PD4972 | PD4972a | CBFB | Ins | frameshift |
| PD4976 | PD4976a | CBFB | Ins | frameshift |
| PD18269 | PD18269a | CBFB | Sub | nonsense |
| PD7218 | PD7218a | CBFB | Del | frameshift |
| PD18756 | PD18756a | CBFB | Sub | ess splice |
| PD6044 | PD6044a | CBLB | Del | frameshift |
| PD11337 | PD11337a | CCND1 | CopyNumber | amp |
| PD11340 | PD11340a | CCND1 | CopyNumber | amp |
| PD11343 | PD11343a | CCND1 | CopyNumber | amp |
| PD11346 | PD11346a | CCND1 | CopyNumber | amp |
| PD11348 | PD11348a | CCND1 | CopyNumber | amp |
| PD11360 | PD11360a | CCND1 | CopyNumber | amp |
| PD11372 | PD11372a | CCND1 | CopyNumber | amp |
| PD11374 | PD11374a | CCND1 | CopyNumber | amp |
| PD11385 | PD11385a | CCND1 | CopyNumber | amp |
| PD11393 | PD11393a | CCND1 | CopyNumber | amp |
| PD11394 | PD11394a | CCND1 | CopyNumber | amp |
| PD11396 | PD11396a | CCND1 | CopyNumber | amp |
| PD11399 | PD11399a | CCND1 | CopyNumber | amp |
| PD11741 | PD11741a | CCND1 | CopyNumber | amp |
| PD11760 | PD11760a | CCND1 | CopyNumber | amp |
| PD11761 | PD11761a | CCND1 | CopyNumber | amp |
| PD13163 | PD13163a | CCND1 | CopyNumber | amp |
| PD13168 | PD13168a | CCND1 | CopyNumber | amp |
| PD13302 | PD13302a | CCND1 | CopyNumber | amp |
| PD13307 | PD13307a | CCND1 | CopyNumber | amp |
| PD13424 | PD13424a | CCND1 | CopyNumber | amp |
| PD13606 | PD13606a | CCND1 | CopyNumber | amp |
| PD13607 | PD13607a | CCND1 | CopyNumber | amp |
| PD13608 | PD13608a | CCND1 | CopyNumber | amp |

(continued)

| Sample | Tumour_name | Gene | Mutation_Type | Effect |
|--------|-------------|------|---------------|--------|
| PD13620 | PD13620a | CCND1 | CopyNumber | amp |
| PD13630 | PD13630a | CCND1 | CopyNumber | amp |
| PD13766 | PD13766a | CCND1 | CopyNumber | amp |
| PD13771 | PD13771a | CCND1 | CopyNumber | amp |
| PD14433 | PD14433a | CCND1 | CopyNumber | amp |
| PD14435 | PD14435a | CCND1 | CopyNumber | amp |
| PD14459 | PD14459a | CCND1 | CopyNumber | amp |
| PD14460 | PD14460a | CCND1 | CopyNumber | amp |
| PD14461 | PD14461a | CCND1 | CopyNumber | amp |
| PD14462 | PD14462a | CCND1 | CopyNumber | amp |
| PD14465 | PD14465a | CCND1 | CopyNumber | amp |
| PD14467 | PD14467a | CCND1 | CopyNumber | amp |
| PD14473 | PD14473a | CCND1 | CopyNumber | amp |
| PD17981 | PD17981a | CCND1 | CopyNumber | amp |
| PD18251 | PD18251a | CCND1 | CopyNumber | amp |
| PD18257 | PD18257a | CCND1 | CopyNumber | amp |
| PD18751 | PD18751a | CCND1 | CopyNumber | amp |
| PD22362 | PD22362a | CCND1 | CopyNumber | amp |
| PD23550 | PD23550a | CCND1 | CopyNumber | amp |
| PD24199 | PD24199a | CCND1 | CopyNumber | amp |
| PD24219 | PD24219a | CCND1 | CopyNumber | amp |
| PD24327 | PD24327a | CCND1 | CopyNumber | amp |
| PD24332 | PD24332a | CCND1 | CopyNumber | amp |
| PD4103 | PD4103a | CCND1 | CopyNumber | amp |
| PD4116 | PD4116a | CCND1 | CopyNumber | amp |
| PD4198 | PD4198a | CCND1 | CopyNumber | amp |
| PD4315 | PD4315a | CCND1 | CopyNumber | amp |
| PD4959 | PD4959a | CCND1 | CopyNumber | amp |
| PD4965 | PD4965a | CCND1 | CopyNumber | amp |
| PD4969 | PD4969a | CCND1 | CopyNumber | amp |
| PD4971 | PD4971a | CCND1 | CopyNumber | amp |
| PD4976 | PD4976a | CCND1 | CopyNumber | amp |
| PD5946 | PD5946a | CCND1 | CopyNumber | amp |
| PD5951 | PD5951a | CCND1 | CopyNumber | amp |
| PD5956 | PD5956a | CCND1 | CopyNumber | amp |
| PD5964 | PD5964a | CCND1 | CopyNumber | amp |
| PD6044 | PD6044a | CCND1 | CopyNumber | amp |
| PD6045 | PD6045a | CCND1 | CopyNumber | amp |
| PD6046 | PD6046a | CCND1 | CopyNumber | amp |

(continued)

| Sample | Tumour_name | Gene | Mutation_Type | Effect |
|--------|-------------|------|---------------|--------|
| PD6047 | PD6047a | CCND1 | CopyNumber | amp |
| PD6048 | PD6048a | CCND1 | CopyNumber | amp |
| PD7069 | PD7069a | CCND1 | CopyNumber | amp |
| PD7202 | PD7202a | CCND1 | CopyNumber | amp |
| PD7204 | PD7204a | CCND1 | CopyNumber | amp |
| PD7305 | PD7305a | CCND1 | CopyNumber | amp |
| PD7306 | PD7306a | CCND1 | CopyNumber | amp |
| PD7322 | PD7322a | CCND1 | CopyNumber | amp |
| PD7341 | PD7341a | CCND1 | CopyNumber | amp |
| PD8609 | PD8609a | CCND1 | CopyNumber | amp |
| PD8977 | PD8977a | CCND1 | CopyNumber | amp |
| PD8980 | PD8980a | CCND1 | CopyNumber | amp |
| PD8995 | PD8995a | CCND1 | CopyNumber | amp |
| PD8999 | PD8999a | CCND1 | CopyNumber | amp |
| PD9539 | PD9539a | CCND1 | CopyNumber | amp |
| PD9541 | PD9541a | CCND1 | CopyNumber | amp |
| PD9567 | PD9567a | CCND1 | CopyNumber | amp |
| PD9582 | PD9582a | CCND1 | CopyNumber | amp |
| PD9591 | PD9591a | CCND1 | CopyNumber | amp |
| PD9599 | PD9599a | CCND1 | CopyNumber | amp |
| PD9605 | PD9605a | CCND1 | CopyNumber | amp |
| PD9752 | PD9752a | CCND1 | CopyNumber | amp |
| PD9754 | PD9754a | CCND1 | CopyNumber | amp |
| PD9755 | PD9755a | CCND1 | CopyNumber | amp |
| PD9759 | PD9759a | CCND1 | CopyNumber | amp |
| PD11327 | PD11327a | CCND3 | CopyNumber | amp |
| PD18037 | PD18037a | CCND3 | CopyNumber | amp |
| PD22036 | PD22036a | CCND3 | CopyNumber | amp |
| PD22251 | PD22251a | CCND3 | CopyNumber | amp |
| PD23554 | PD23554a | CCND3 | CopyNumber | amp |
| PD24308 | PD24308a | CCND3 | CopyNumber | amp |
| PD24318 | PD24318a | CCND3 | CopyNumber | amp |
| PD3905 | PD3905a | CCND3 | CopyNumber | amp |
| PD4264 | PD4264a | CCND3 | CopyNumber | amp |
| PD4874 | PD4874a | CCND3 | CopyNumber | amp |
| PD5932 | PD5932a | CCND3 | CopyNumber | amp |
| PD5942 | PD5942a | CCND3 | CopyNumber | amp |
| PD5950 | PD5950a | CCND3 | CopyNumber | amp |
| PD6732 | PD6732b | CCND3 | CopyNumber | amp |

(continued)

| Sample | Tumour_name | Gene | Mutation_Type | Effect |
|--------|-------------|------|---------------|--------|
| PD7067 | PD7067a | CCND3 | CopyNumber | amp |
| PD7428 | PD7428a | CCND3 | CopyNumber | amp |
| PD13296 | PD13296a | CCNE1 | CopyNumber | amp |
| PD13622 | PD13622a | CCNE1 | CopyNumber | amp |
| PD14457 | PD14457a | CCNE1 | CopyNumber | amp |
| PD18020 | PD18020a | CCNE1 | CopyNumber | amp |
| PD24190 | PD24190a | CCNE1 | CopyNumber | amp |
| PD24196 | PD24196a | CCNE1 | CopyNumber | amp |
| PD24308 | PD24308a | CCNE1 | CopyNumber | amp |
| PD24325 | PD24325a | CCNE1 | CopyNumber | amp |
| PD4086 | PD4086a | CCNE1 | CopyNumber | amp |
| PD4192 | PD4192a | CCNE1 | CopyNumber | amp |
| PD4841 | PD4841a | CCNE1 | CopyNumber | amp |
| PD6732 | PD6732b | CCNE1 | CopyNumber | amp |
| PD7428 | PD7428a | CCNE1 | CopyNumber | amp |
| PD8660 | PD8660a2 | CCNE1 | CopyNumber | amp |
| PD18748 | PD18748a | CDH1 | Del | start-lost |
| PD13763 | PD13763a | CDH1 | Sub | nonsense |
| PD5961 | PD5961a | CDH1 | Sub | nonsense |
| PD14472 | PD14472a | CDH1 | Ins | frameshift |
| PD9067 | PD9067a | CDH1 | Ins | frameshift |
| PD4194 | PD4194a | CDH1 | Complex | frameshift |
| PD14460 | PD14460a | CDH1 | Complex | frameshift |
| PD9578 | PD9578a | CDH1 | Del | ess splice |
| PD6016 | PD6016a2 | CDH1 | Sub | nonsense |
| PD7204 | PD7204a | CDH1 | Ins | frameshift |
| PD11376 | PD11376a | CDH1 | Sub | ess splice |
| PD13306 | PD13306a | CDH1 | Del | frameshift |
| PD7238 | PD7238a | CDH1 | Ins | frameshift |
| PD9579 | PD9579a | CDH1 | Sub | ess splice |
| PD13607 | PD13607a | CDH1 | Ins | frameshift |
| PD14465 | PD14465a | CDH1 | Del | frameshift |
| PD9597 | PD9597a | CDH1 | Sub | nonsense |
| PD4985 | PD4985a | CDH1 | Sub | nonsense |
| PD9063 | PD9063a | CDH1 | Sub | nonsense |
| PD11740 | PD11740a | CDH1 | Sub | nonsense |
| PD11358 | PD11358a | CDH1 | Sub | ess splice |
| PD7214 | PD7214a | CDH1 | Ins | frameshift |
| PD18188 | PD18188a | CDH1 | Complex | frameshift |

(continued)

| Sample | Tumour_name | Gene | Mutation_Type | Effect |
|---|---|---|---|---|
| PD11342 | PD11342a | CDH1 | Del | frameshift |
| PD4977 | PD4977a | CDH1 | Sub | missense |
| PD5936 | PD5936a | CDH1 | Ins | frameshift |
| PD18756 | PD18756a | CDH1 | Sub | nonsense |
| PD9589 | PD9589a | CDH1 | Del | frameshift |
| PD18049 | PD18049a | CDH1 | Ins | frameshift |
| PD14442 | PD14442a | CDH1 | Sub | nonsense |
| PD5960 | PD5960a | CDH1 | Sub | ess splice |
| PD8618 | PD8618a | CDH1 | Sub | ess splice |
| PD7209 | PD7209a | CDH1 | Del | frameshift |
| PD11375 | PD11375a | CDH1 | Del | frameshift |
| PD4967 | PD4967a | CDH1 | CopyNumber | HD |
| PD11751 | PD11751a | CDK6 | CopyNumber | amp |
| PD14453 | PD14453a | CDK6 | CopyNumber | amp |
| PD24201 | PD24201a | CDK6 | CopyNumber | amp |
| PD4605 | PD4605a | CDK6 | CopyNumber | amp |
| PD6043 | PD6043a | CDK6 | CopyNumber | amp |
| PD9464 | PD9464a | CDK6 | CopyNumber | amp |
| PD13416 | PD13416a | CDKN1B | Sub | nonsense |
| PD14472 | PD14472a | CDKN1B | Sub | nonsense |
| PD11341 | PD11341a | CDKN2A | Sub | missense |
| PD11336 | PD11336a | CDKN2A | Sub | missense |
| PD18733 | PD18733a | CDKN2A | Sub | nonsense |
| PD11364 | PD11364a | CDKN2A | CopyNumber | HD |
| PD11748 | PD11748a | CDKN2A | CopyNumber | HD |
| PD13627 | PD13627a | CDKN2A | CopyNumber | HD |
| PD14467 | PD14467a | CDKN2A | CopyNumber | HD |
| PD23560 | PD23560a | CDKN2A | CopyNumber | HD |
| PD24209 | PD24209a | CDKN2A | CopyNumber | HD |
| PD24212 | PD24212a | CDKN2A | CopyNumber | HD |
| PD4836 | PD4836a | CDKN2A | CopyNumber | HD |
| PD4876 | PD4876a | CDKN2A | CopyNumber | HD |
| PD5956 | PD5956a | CDKN2A | CopyNumber | HD |
| PD6410 | PD6410a | CDKN2A | CopyNumber | HD |
| PD7067 | PD7067a | CDKN2A | CopyNumber | HD |
| PD8830 | PD8830a | CDKN2A | CopyNumber | HD |
| PD8832 | PD8832a | CDKN2A | CopyNumber | HD |
| PD8980 | PD8980a | CDKN2A | CopyNumber | HD |
| PD24208 | PD24208a | CDKN2A | Rearrangement | 1|Tandem duplication |

(continued)

| Sample | Tumour_name | Gene | Mutation_Type | Effect |
|---|---|---|---|---|
| PD5925 | PD5925a | CDKN2A | Rearrangement | 1|Deletion |
| PD7316 | PD7316a | CDKN2A | Rearrangement | 1|Deletion |
| PD13602 | PD13602a | CDKN2A | Rearrangement | 1|Deletion |
| PD24337 | PD24337a | CDKN2A | Rearrangement | 1|Deletion |
| PD13752 | PD13752a | CDKN2A | Rearrangement | 2|Translocation| Translocation |
| PD11364 | PD11364a | CDKN2B | CopyNumber | HD |
| PD11748 | PD11748a | CDKN2B | CopyNumber | HD |
| PD13627 | PD13627a | CDKN2B | CopyNumber | HD |
| PD14467 | PD14467a | CDKN2B | CopyNumber | HD |
| PD23560 | PD23560a | CDKN2B | CopyNumber | HD |
| PD24209 | PD24209a | CDKN2B | CopyNumber | HD |
| PD24212 | PD24212a | CDKN2B | CopyNumber | HD |
| PD4876 | PD4876a | CDKN2B | CopyNumber | HD |
| PD5956 | PD5956a | CDKN2B | CopyNumber | HD |
| PD6410 | PD6410a | CDKN2B | CopyNumber | HD |
| PD7067 | PD7067a | CDKN2B | CopyNumber | HD |
| PD8830 | PD8830a | CDKN2B | CopyNumber | HD |
| PD8832 | PD8832a | CDKN2B | CopyNumber | HD |
| PD8980 | PD8980a | CDKN2B | CopyNumber | HD |
| PD18047 | PD18047a | Chr8:(ZNF703/FGFR1) | CopyNumber | amp |
| PD24182 | PD24182a | Chr8:(ZNF703/FGFR1) | CopyNumber | amp |
| PD4255 | PD4255a | Chr8:(ZNF703/FGFR1) | CopyNumber | amp |
| PD7344 | PD7344a | Chr8:(ZNF703/FGFR1) | CopyNumber | amp |
| PD11339 | PD11339a | Chr8:(ZNF703/FGFR1) | CopyNumber | amp |
| PD11340 | PD11340a | Chr8:(ZNF703/FGFR1) | CopyNumber | amp |
| PD11368 | PD11368a | Chr8:(ZNF703/FGFR1) | CopyNumber | amp |
| PD11369 | PD11369a | Chr8:(ZNF703/FGFR1) | CopyNumber | amp |
| PD11374 | PD11374a | Chr8:(ZNF703/FGFR1) | CopyNumber | amp |
| PD11379 | PD11379a | Chr8:(ZNF703/FGFR1) | CopyNumber | amp |
| PD11394 | PD11394a | Chr8:(ZNF703/FGFR1) | CopyNumber | amp |
| PD11399 | PD11399a | Chr8:(ZNF703/FGFR1) | CopyNumber | amp |
| PD11741 | PD11741a | Chr8:(ZNF703/FGFR1) | CopyNumber | amp |
| PD11760 | PD11760a | Chr8:(ZNF703/FGFR1) | CopyNumber | amp |
| PD13165 | PD13165a | Chr8:(ZNF703/FGFR1) | CopyNumber | amp |
| PD13168 | PD13168a | Chr8:(ZNF703/FGFR1) | CopyNumber | amp |
| PD13425 | PD13425a | Chr8:(ZNF703/FGFR1) | CopyNumber | amp |
| PD13608 | PD13608a | Chr8:(ZNF703/FGFR1) | CopyNumber | amp |
| PD13630 | PD13630a | Chr8:(ZNF703/FGFR1) | CopyNumber | amp |
| PD14453 | PD14453a | Chr8:(ZNF703/FGFR1) | CopyNumber | amp |

(continued)

| Sample | Tumour_name | Gene | Mutation_Type | Effect |
|--------|-------------|------|---------------|--------|
| PD14457 | PD14457a | Chr8:(ZNF703/FGFR1) | CopyNumber | amp |
| PD14461 | PD14461a | Chr8:(ZNF703/FGFR1) | CopyNumber | amp |
| PD14471 | PD14471a | Chr8:(ZNF703/FGFR1) | CopyNumber | amp |
| PD18031 | PD18031a | Chr8:(ZNF703/FGFR1) | CopyNumber | amp |
| PD18045 | PD18045a | Chr8:(ZNF703/FGFR1) | CopyNumber | amp |
| PD18188 | PD18188a | Chr8:(ZNF703/FGFR1) | CopyNumber | amp |
| PD18189 | PD18189a | Chr8:(ZNF703/FGFR1) | CopyNumber | amp |
| PD18257 | PD18257a | Chr8:(ZNF703/FGFR1) | CopyNumber | amp |
| PD18733 | PD18733a | Chr8:(ZNF703/FGFR1) | CopyNumber | amp |
| PD18748 | PD18748a | Chr8:(ZNF703/FGFR1) | CopyNumber | amp |
| PD22365 | PD22365a | Chr8:(ZNF703/FGFR1) | CopyNumber | amp |
| PD3904 | PD3904a | Chr8:(ZNF703/FGFR1) | CopyNumber | amp |
| PD4264 | PD4264a | Chr8:(ZNF703/FGFR1) | CopyNumber | amp |
| PD4315 | PD4315a | Chr8:(ZNF703/FGFR1) | CopyNumber | amp |
| PD4605 | PD4605a | Chr8:(ZNF703/FGFR1) | CopyNumber | amp |
| PD4845 | PD4845a | Chr8:(ZNF703/FGFR1) | CopyNumber | amp |
| PD4965 | PD4965a | Chr8:(ZNF703/FGFR1) | CopyNumber | amp |
| PD4976 | PD4976a | Chr8:(ZNF703/FGFR1) | CopyNumber | amp |
| PD4978 | PD4978a | Chr8:(ZNF703/FGFR1) | CopyNumber | amp |
| PD5951 | PD5951a | Chr8:(ZNF703/FGFR1) | CopyNumber | amp |
| PD5959 | PD5959a | Chr8:(ZNF703/FGFR1) | CopyNumber | amp |
| PD6044 | PD6044a | Chr8:(ZNF703/FGFR1) | CopyNumber | amp |
| PD6048 | PD6048a | Chr8:(ZNF703/FGFR1) | CopyNumber | amp |
| PD6719 | PD6719a | Chr8:(ZNF703/FGFR1) | CopyNumber | amp |
| PD6732 | PD6732b | Chr8:(ZNF703/FGFR1) | CopyNumber | amp |
| PD7202 | PD7202a | Chr8:(ZNF703/FGFR1) | CopyNumber | amp |
| PD7215 | PD7215a | Chr8:(ZNF703/FGFR1) | CopyNumber | amp |
| PD7240 | PD7240a | Chr8:(ZNF703/FGFR1) | CopyNumber | amp |
| PD8660 | PD8660a2 | Chr8:(ZNF703/FGFR1) | CopyNumber | amp |
| PD8979 | PD8979a | Chr8:(ZNF703/FGFR1) | CopyNumber | amp |
| PD8980 | PD8980a | Chr8:(ZNF703/FGFR1) | CopyNumber | amp |
| PD8981 | PD8981a | Chr8:(ZNF703/FGFR1) | CopyNumber | amp |
| PD9002 | PD9002a | Chr8:(ZNF703/FGFR1) | CopyNumber | amp |
| PD9009 | PD9009a | Chr8:(ZNF703/FGFR1) | CopyNumber | amp |
| PD9464 | PD9464a | Chr8:(ZNF703/FGFR1) | CopyNumber | amp |
| PD9467 | PD9467a | Chr8:(ZNF703/FGFR1) | CopyNumber | amp |
| PD9539 | PD9539a | Chr8:(ZNF703/FGFR1) | CopyNumber | amp |
| PD9567 | PD9567a | Chr8:(ZNF703/FGFR1) | CopyNumber | amp |
| PD9599 | PD9599a | Chr8:(ZNF703/FGFR1) | CopyNumber | amp |

(continued)

| Sample | Tumour_name | Gene | Mutation_Type | Effect |
|--------|-------------|------|---------------|--------|
| PD9604 | PD9604a | Chr8:(ZNF703/FGFR1) | CopyNumber | amp |
| PD9694 | PD9694a | Chr8:(ZNF703/FGFR1) | CopyNumber | amp |
| PD9752 | PD9752a | Chr8:(ZNF703/FGFR1) | CopyNumber | amp |
| PD9759 | PD9759a | Chr8:(ZNF703/FGFR1) | CopyNumber | amp |
| PD9760 | PD9760a | Chr8:(ZNF703/FGFR1) | CopyNumber | amp |
| PD13604 | PD13604a | CIC | Sub | nonsense |
| PD5937 | PD5937a | CNOT3 | Sub | missense |
| PD23565 | PD23565a | CREBBP | Ins | frameshift |
| PD5937 | PD5937a | CREBBP | Del | frameshift |
| PD9694 | PD9694a | CREBBP | Sub | nonsense |
| PD23579 | PD23579a | CREBBP | Sub | nonsense |
| PD9009 | PD9009a | CTCF | Sub | nonsense |
| PD9541 | PD9541a | CTCF | Sub | missense |
| PD9467 | PD9467a | CTCF | Sub | missense |
| PD24223 | PD24223a | CTCF | Sub | missense |
| PD9570 | PD9570a | CTCF | Sub | ess splice |
| PD24326 | PD24326a | CUX1 | Sub | nonsense |
| PD4957 | PD4957a | CUX1 | Sub | nonsense |
| PD7207 | PD7207a | DNMT3A | Sub | nonsense |
| PD18768 | PD18768a | DNMT3A | Del | ess splice |
| PD11338 | PD11338a | DNMT3A | CopyNumber | HD |
| PD14467 | PD14467a | DNMT3A | CopyNumber | HD |
| PD5937 | PD5937a | ECT2L | Sub | nonsense |
| PD9568 | PD9568a | EGFR | Sub | missense |
| PD23564 | PD23564a | EGFR | Sub | missense |
| PD11766 | PD11766a | EGFR | CopyNumber | amp |
| PD22361 | PD22361a | EGFR | CopyNumber | amp |
| PD24308 | PD24308a | EGFR | CopyNumber | amp |
| PD24314 | PD24314a | EGFR | CopyNumber | amp |
| PD24326 | PD24326a | EGFR | CopyNumber | amp |
| PD6416 | PD6416a | EGFR | CopyNumber | amp |
| PD13425 | PD13425a | ERBB2 | Sub | missense |
| PD11338 | PD11338a | ERBB2 | Sub | missense |
| PD4072 | PD4072a | ERBB2 | Sub | missense |
| PD4072 | PD4072a | ERBB2 | Sub | missense |
| PD23565 | PD23565a | ERBB2 | Sub | missense |
| PD18049 | PD18049a | ERBB2 | Sub | missense |
| PD11386 | PD11386a | ERBB2 | Ins | inframe |
| PD11344 | PD11344a | ERBB2 | CopyNumber | amp |

(continued)

| Sample | Tumour_name | Gene | Mutation_Type | Effect |
|--------|-------------|------|---------------|--------|
| PD11345 | PD11345a | ERBB2 | CopyNumber | amp |
| PD11346 | PD11346a | ERBB2 | CopyNumber | amp |
| PD11349 | PD11349a | ERBB2 | CopyNumber | amp |
| PD11462 | PD11462a | ERBB2 | CopyNumber | amp |
| PD11464 | PD11464a | ERBB2 | CopyNumber | amp |
| PD11465 | PD11465a | ERBB2 | CopyNumber | amp |
| PD13163 | PD13163a | ERBB2 | CopyNumber | amp |
| PD13164 | PD13164a | ERBB2 | CopyNumber | amp |
| PD13165 | PD13165a | ERBB2 | CopyNumber | amp |
| PD13166 | PD13166a | ERBB2 | CopyNumber | amp |
| PD13167 | PD13167a | ERBB2 | CopyNumber | amp |
| PD13168 | PD13168a | ERBB2 | CopyNumber | amp |
| PD13602 | PD13602a | ERBB2 | CopyNumber | amp |
| PD13603 | PD13603a | ERBB2 | CopyNumber | amp |
| PD13604 | PD13604a | ERBB2 | CopyNumber | amp |
| PD13605 | PD13605a | ERBB2 | CopyNumber | amp |
| PD13608 | PD13608a | ERBB2 | CopyNumber | amp |
| PD13609 | PD13609a | ERBB2 | CopyNumber | amp |
| PD18045 | PD18045a | ERBB2 | CopyNumber | amp |
| PD18047 | PD18047a | ERBB2 | CopyNumber | amp |
| PD18048 | PD18048a | ERBB2 | CopyNumber | amp |
| PD18049 | PD18049a | ERBB2 | CopyNumber | amp |
| PD18050 | PD18050a | ERBB2 | CopyNumber | amp |
| PD18188 | PD18188a | ERBB2 | CopyNumber | amp |
| PD18189 | PD18189a | ERBB2 | CopyNumber | amp |
| PD22357 | PD22357a | ERBB2 | CopyNumber | amp |
| PD22359 | PD22359a | ERBB2 | CopyNumber | amp |
| PD22362 | PD22362a | ERBB2 | CopyNumber | amp |
| PD22366 | PD22366a | ERBB2 | CopyNumber | amp |
| PD23560 | PD23560a | ERBB2 | CopyNumber | amp |
| PD23561 | PD23561a | ERBB2 | CopyNumber | amp |
| PD24194 | PD24194a | ERBB2 | CopyNumber | amp |
| PD24207 | PD24207a | ERBB2 | CopyNumber | amp |
| PD24209 | PD24209a | ERBB2 | CopyNumber | amp |
| PD24217 | PD24217a | ERBB2 | CopyNumber | amp |
| PD24220 | PD24220a | ERBB2 | CopyNumber | amp |
| PD24225 | PD24225a | ERBB2 | CopyNumber | amp |
| PD24322 | PD24322a | ERBB2 | CopyNumber | amp |
| PD24332 | PD24332a | ERBB2 | CopyNumber | amp |

(continued)

| Sample | Tumour_name | Gene | Mutation_Type | Effect |
|--------|-------------|------|---------------|--------|
| PD24335 | PD24335a | ERBB2 | CopyNumber | amp |
| PD4192 | PD4192a | ERBB2 | CopyNumber | amp |
| PD4194 | PD4194a | ERBB2 | CopyNumber | amp |
| PD4198 | PD4198a | ERBB2 | CopyNumber | amp |
| PD4199 | PD4199a | ERBB2 | CopyNumber | amp |
| PD4613 | PD4613a | ERBB2 | CopyNumber | amp |
| PD4826 | PD4826a | ERBB2 | CopyNumber | amp |
| PD4962 | PD4962a | ERBB2 | CopyNumber | amp |
| PD4978 | PD4978a | ERBB2 | CopyNumber | amp |
| PD5950 | PD5950a | ERBB2 | CopyNumber | amp |
| PD6048 | PD6048a | ERBB2 | CopyNumber | amp |
| PD6405 | PD6405a | ERBB2 | CopyNumber | amp |
| PD7069 | PD7069a | ERBB2 | CopyNumber | amp |
| PD7202 | PD7202a | ERBB2 | CopyNumber | amp |
| PD7203 | PD7203a | ERBB2 | CopyNumber | amp |
| PD7204 | PD7204a | ERBB2 | CopyNumber | amp |
| PD7205 | PD7205a | ERBB2 | CopyNumber | amp |
| PD7249 | PD7249a | ERBB2 | CopyNumber | amp |
| PD7304 | PD7304a | ERBB2 | CopyNumber | amp |
| PD7305 | PD7305a | ERBB2 | CopyNumber | amp |
| PD7306 | PD7306a | ERBB2 | CopyNumber | amp |
| PD7307 | PD7307a | ERBB2 | CopyNumber | amp |
| PD7341 | PD7341a | ERBB2 | CopyNumber | amp |
| PD8995 | PD8995a | ERBB2 | CopyNumber | amp |
| PD8996 | PD8996a | ERBB2 | CopyNumber | amp |
| PD8997 | PD8997a | ERBB2 | CopyNumber | amp |
| PD8998 | PD8998a | ERBB2 | CopyNumber | amp |
| PD9009 | PD9009a | ERBB2 | CopyNumber | amp |
| PD9576 | PD9576a | ERBB2 | CopyNumber | amp |
| PD9755 | PD9755a | ERBB2 | CopyNumber | amp |
| PD9575 | PD9575a | ERBB3 | Sub | missense |
| PD11348 | PD11348a | ERBB3 | Sub | missense |
| PD13306 | PD13306a | ERBB3 | Sub | missense |
| PD23564 | PD23564a | ERCC4 | Sub | nonsense |
| PD18264 | PD18264a | ESR1 | Sub | missense |
| PD13768 | PD13768a | ESR1 | Sub | missense |
| PD24320 | PD24320a | ESR1 | Sub | missense |
| PD13425 | PD13425a | ESR1 | CopyNumber | amp |
| PD24199 | PD24199a | ESR1 | CopyNumber | amp |

(continued)

| Sample | Tumour_name | Gene | Mutation_Type | Effect |
|---|---|---|---|---|
| PD24216 | PD24216a | ESR1 | CopyNumber | amp |
| PD5942 | PD5942a | ESR1 | CopyNumber | amp |
| PD24208 | PD24208a | FBXW7 | Sub | missense |
| PD11379 | PD11379a | FBXW7 | Sub | nonsense |
| PD9571 | PD9571a | FBXW7 | Sub | nonsense |
| PD4005 | PD4005a | FBXW7 | Rearrangement | 1|Translocation |
| PD24201 | PD24201a | FBXW7 | Rearrangement | 1|Tandem duplication |
| PD7248 | PD7248a | FBXW7 | Rearrangement | 1|Inversion |
| PD9702 | PD9702a | FBXW7 | Rearrangement | 1|Deletion |
| PD4844 | PD4844a | FBXW7 | Rearrangement | 2|Translocation| Translocation |
| PD4315 | PD4315a | FBXW7 | Rearrangement | 1|Tandem duplication |
| PD6404 | PD6404a | FBXW7 | Rearrangement | 1|Deletion |
| PD7211 | PD7211a | FBXW7 | Rearrangement | 1|Tandem duplication |
| PD5948 | PD5948a | FBXW7 | Rearrangement | 1|Deletion |
| PD10011 | PD10011a | FBXW7 | Rearrangement | 1|Tandem duplication |
| PD4255 | PD4255a | FBXW7 | Rearrangement | 1|Tandem duplication |
| PD11762 | PD11762a | FBXW7 | Rearrangement | 2|Deletion|Inversion |
| PD4845 | PD4845a | FBXW7 | Rearrangement | 1|Tandem duplication |
| PD23566 | PD23566a | FBXW7 | Rearrangement | 1|Tandem duplication |
| PD11359 | PD11359a | FGFR2 | Sub | missense |
| PD5948 | PD5948a | FGFR2 | CopyNumber | amp |
| PD9571 | PD9571a | FGFR2 | CopyNumber | amp |
| PD9597 | PD9597a | FOXA1 | Sub | missense |
| PD13623 | PD13623a | FOXA1 | Sub | missense |
| PD11386 | PD11386a | FOXA1 | Sub | missense |
| PD9063 | PD9063a | FOXA1 | Sub | missense |
| PD18247 | PD18247a | FOXA1 | Sub | missense |
| PD6016 | PD6016a2 | FOXA1 | Sub | missense |
| PD14433 | PD14433a | FOXA1 | Sub | missense |
| PD5950 | PD5950a | FOXP1 | Sub | nonsense |
| PD9605 | PD9605a | FOXP1 | Del | frameshift |
| PD13753 | PD13753a | FOXP1 | Sub | ess splice |
| PD11379 | PD11379a | FOXP1 | Sub | nonsense |
| PD13623 | PD13623a | GATA3 | Del | frameshift |
| PD5937 | PD5937a | GATA3 | Sub | missense |
| PD9063 | PD9063a | GATA3 | Sub | missense |
| PD13752 | PD13752a | GATA3 | Del | ess splice |
| PD4982 | PD4982a | GATA3 | Del | ess splice |
| PD4069 | PD4069a | GATA3 | Del | ess splice |

(continued)

| Sample | Tumour_name | Gene | Mutation_Type | Effect |
|--------|-------------|------|---------------|--------|
| PD7218 | PD7218a | GATA3 | Del | ess splice |
| PD9001 | PD9001a | GATA3 | Del | ess splice |
| PD7210 | PD7210a | GATA3 | Del | ess splice |
| PD9581 | PD9581a | GATA3 | Del | ess splice |
| PD13619 | PD13619a | GATA3 | Del | ess splice |
| PD4085 | PD4085a | GATA3 | Del | ess splice |
| PD23570 | PD23570a | GATA3 | Del | ess splice |
| PD18269 | PD18269a | GATA3 | Del | ess splice |
| PD7322 | PD7322a | GATA3 | Del | ess splice |
| PD9539 | PD9539a | GATA3 | Del | ess splice |
| PD13756 | PD13756a | GATA3 | Del | ess splice |
| PD6422 | PD6422a | GATA3 | Ins | frameshift |
| PD13625 | PD13625a | GATA3 | Ins | frameshift |
| PD18734 | PD18734a | GATA3 | Ins | frameshift |
| PD14456 | PD14456a | GATA3 | Ins | frameshift |
| PD11336 | PD11336a | GATA3 | Del | frameshift |
| PD11388 | PD11388a | GATA3 | Ins | frameshift |
| PD11384 | PD11384a | GATA3 | Ins | frameshift |
| PD7209 | PD7209a | GATA3 | Ins | frameshift |
| PD9761 | PD9761a | GATA3 | Sub | nonsense |
| PD11389 | PD11389a | GATA3 | Ins | frameshift |
| PD14450 | PD14450a | GATA3 | Del | frameshift |
| PD14458 | PD14458a | GATA3 | Del | frameshift |
| PD13310 | PD13310a | GATA3 | Del | frameshift |
| PD22362 | PD22362a | GATA3 | Del | frameshift |
| PD18264 | PD18264a | GATA3 | Del | frameshift |
| PD24214 | PD24214a | GATA3 | Sub | nonsense |
| PD11395 | PD11395a | GATA3 | Del | frameshift |
| PD7305 | PD7305a | GATA3 | Ins | frameshift |
| PD9847 | PD9847a | GATA3 | Ins | frameshift |
| PD11348 | PD11348a | GATA3 | Ins | frameshift |
| PD11818 | PD11818a | GATA3 | Ins | frameshift |
| PD11381 | PD11381a | GATA3 | Ins | frameshift |
| PD6048 | PD6048a | GATA3 | Ins | frameshift |
| PD11761 | PD11761a | GATA3 | Ins | frameshift |
| PD24320 | PD24320a | GATA3 | Ins | frameshift |
| PD9577 | PD9577a | GATA3 | Ins | frameshift |
| PD9760 | PD9760a | GATA3 | Ins | frameshift |
| PD13764 | PD13764a | GATA3 | Ins | frameshift |

(continued)

| Sample | Tumour_name | Gene | Mutation_Type | Effect |
|---|---|---|---|---|
| PD9000 | PD9000a | GATA3 | Ins | frameshift |
| PD4103 | PD4103a | GATA3 | Ins | frameshift |
| PD9759 | PD9759a | GATA3 | Ins | frameshift |
| PD14467 | PD14467a | GATA3 | Ins | frameshift |
| PD7306 | PD7306a | GATA3 | Ins | frameshift |
| PD6711 | PD6711a2 | GATA3 | Ins | frameshift |
| PD5964 | PD5964a | GATA3 | Ins | frameshift |
| PD6417 | PD6417a | GATA3 | Del | frameshift |
| PD9193 | PD9193a | GATA3 | Ins | frameshift |
| PD7221 | PD7221a | GNAS | Sub | missense |
| PD11366 | PD11366a | GNAS | Sub | missense |
| PD9584 | PD9584a | HRAS | Sub | missense |
| PD11369 | PD11369a | IGF1R | CopyNumber | amp |
| PD13764 | PD13764a | IGF1R | CopyNumber | amp |
| PD18020 | PD18020a | IGF1R | CopyNumber | amp |
| PD4968 | PD4968a | IGF1R | CopyNumber | amp |
| PD5928 | PD5928a | IGF1R | CopyNumber | amp |
| PD6415 | PD6415a | IGF1R | CopyNumber | amp |
| PD7307 | PD7307a | IGF1R | CopyNumber | amp |
| PD7428 | PD7428a | IGF1R | CopyNumber | amp |
| PD9464 | PD9464a | IGF1R | CopyNumber | amp |
| PD9582 | PD9582a | IGF1R | CopyNumber | amp |
| PD9760 | PD9760a | IGF1R | CopyNumber | amp |
| PD14472 | PD14472a | KDM6A | Del | ess splice |
| PD7321 | PD7321a | KDM6A | Sub | nonsense |
| PD14458 | PD14458a | KDM6A | Ins | frameshift |
| PD9001 | PD9001a | KDM6A | Sub | nonsense |
| PD9568 | PD9568a | KDM6A | Sub | ess splice |
| PD24333 | PD24333a | KRAS | Sub | missense |
| PD11327 | PD11327a | KRAS | CopyNumber | amp |
| PD11752 | PD11752a | KRAS | CopyNumber | amp |
| PD4975 | PD4975a | KRAS | CopyNumber | amp |
| PD5945 | PD5945a | KRAS | CopyNumber | amp |
| PD6733 | PD6733b | KRAS | CopyNumber | amp |
| PD7316 | PD7316a | KRAS | CopyNumber | amp |
| PD9582 | PD9582a | KRAS | CopyNumber | amp |
| PD7220 | PD7220a | MAP2K4 | Sub | nonsense |
| PD11395 | PD11395a | MAP2K4 | Del | ess splice |
| PD4085 | PD4085a | MAP2K4 | Del | ess splice |

(continued)

| Sample | Tumour_name | Gene | Mutation_Type | Effect |
|---|---|---|---|---|
| PD11369 | PD11369a | MAP2K4 | Sub | nonsense |
| PD11344 | PD11344a | MAP2K4 | Sub | missense |
| PD9067 | PD9067a | MAP2K4 | Del | frameshift |
| PD11339 | PD11339a | MAP2K4 | Sub | ess splice |
| PD11347 | PD11347a | MAP2K4 | CopyNumber | HD |
| PD11367 | PD11367a | MAP2K4 | CopyNumber | HD |
| PD11462 | PD11462a | MAP2K4 | CopyNumber | HD |
| PD13608 | PD13608a | MAP2K4 | CopyNumber | HD |
| PD13625 | PD13625a | MAP2K4 | CopyNumber | HD |
| PD13753 | PD13753a | MAP2K4 | CopyNumber | HD |
| PD13760 | PD13760a | MAP2K4 | CopyNumber | HD |
| PD14471 | PD14471a | MAP2K4 | CopyNumber | HD |
| PD18050 | PD18050a | MAP2K4 | CopyNumber | HD |
| PD4088 | PD4088a | MAP2K4 | CopyNumber | HD |
| PD4604 | PD4604a | MAP2K4 | CopyNumber | HD |
| PD4606 | PD4606a | MAP2K4 | CopyNumber | HD |
| PD6719 | PD6719a | MAP2K4 | CopyNumber | HD |
| PD7206 | PD7206a | MAP2K4 | CopyNumber | HD |
| PD8609 | PD8609a | MAP2K4 | CopyNumber | HD |
| PD13425 | PD13425a | MAP2K4 | Rearrangement | 1|Tandem duplication |
| PD9597 | PD9597a | MAP2K4 | Rearrangement | 1 |Translocation |
| PD9572 | PD9572a | MAP2K4 | Rearrangement | 1|Inversion |
| PD7305 | PD7305a | MAP2K4 | Rearrangement | 2|Inversion| Translocation |
| PD14437 | PD14437a | MAP2K4 | Rearrangement | 1|Deletion |
| PD22355 | PD22355a | MAP2K4 | Rearrangement | 2|Translocation| Translocation |
| PD14441 | PD14441a | MAP2K4 | Rearrangement | 2|Inversion| Translocation |
| PD11761 | PD11761a | MAP2K4 | Rearrangement | 1|Deletion |
| PD14454 | PD14454a | MAP2K4 | Rearrangement | 1|Deletion |
| PD11380 | PD11380a | MAP2K4 | Rearrangement | 1|Deletion |
| PD6417 | PD6417a | MAP2K4 | Rearrangement | 1|Translocation |
| PD11385 | PD11385a | MAP2K4 | Rearrangement | 1|Deletion |
| PD9754 | PD9754a | MAP2K4 | Rearrangement | 1|Tandem duplication |
| PD13626 | PD13626a | MAP2K4 | Rearrangement | 1|Inversion |
| PD8982 | PD8982a | MAP3K1 | Ins | frameshift |
| PD7202 | PD7202a | MAP3K1 | Del | frameshift |
| PD5956 | PD5956a | MAP3K1 | Del | frameshift |
| PD4605 | PD4605a | MAP3K1 | Del | frameshift |
| PD18247 | PD18247a | MAP3K1 | Del | frameshift |
| PD13427 | PD13427a | MAP3K1 | Ins | frameshift |

(continued)

| Sample | Tumour_name | Gene | Mutation_Type | Effect |
|--------|-------------|------|---------------|--------|
| PD11740 | PD11740a | MAP3K1 | Ins | frameshift |
| PD6016 | PD6016a2 | MAP3K1 | Del | frameshift |
| PD11394 | PD11394a | MAP3K1 | Ins | frameshift |
| PD24302 | PD24302a | MAP3K1 | Ins | frameshift |
| PD11394 | PD11394a | MAP3K1 | Del | frameshift |
| PD3851 | PD3851a | MAP3K1 | Sub | nonsense |
| PD5961 | PD5961a | MAP3K1 | Ins | frameshift |
| PD18776 | PD18776a | MAP3K1 | Ins | frameshift |
| PD7201 | PD7201a | MAP3K1 | Del | frameshift |
| PD9570 | PD9570a | MAP3K1 | Ins | frameshift |
| PD9606 | PD9606a | MAP3K1 | Del | frameshift |
| PD13419 | PD13419a | MAP3K1 | Del | frameshift |
| PD13307 | PD13307a | MAP3K1 | Complex | frameshift |
| PD11756 | PD11756a | MAP3K1 | Sub | nonsense |
| PD4225 | PD4225a | MAP3K1 | Sub | nonsense |
| PD11359 | PD11359a | MAP3K1 | Ins | frameshift |
| PD13755 | PD13755a | MAP3K1 | Ins | frameshift |
| PD13619 | PD13619a | MAP3K1 | Del | frameshift |
| PD9842 | PD9842a | MAP3K1 | Sub | nonsense |
| PD8828 | PD8828a | MAP3K1 | Del | frameshift |
| PD13755 | PD13755a | MAP3K1 | Del | frameshift |
| PD5961 | PD5961a | MAP3K1 | Del | frameshift |
| PD13768 | PD13768a | MAP3K1 | Del | frameshift |
| PD6046 | PD6046a | MAP3K1 | Del | frameshift |
| PD9574 | PD9574a | MAP3K1 | Ins | frameshift |
| PD9606 | PD9606a | MAP3K1 | Ins | frameshift |
| PD6418 | PD6418a | MAP3K1 | Del | frameshift |
| PD18247 | PD18247a | MAP3K1 | Del | frameshift |
| PD4266 | PD4266a | MAP3K1 | Del | frameshift |
| PD11756 | PD11756a | MAP3K1 | Del | frameshift |
| PD13623 | PD13623a | MAP3K1 | Ins | frameshift |
| PD11343 | PD11343a | MAP3K1 | Ins | frameshift |
| PD13768 | PD13768a | MAP3K1 | Del | frameshift |
| PD18776 | PD18776a | MAP3K1 | Sub | nonsense |
| PD9574 | PD9574a | MAP3K1 | Ins | frameshift |
| PD17994 | PD17994a | MAP3K1 | Sub | nonsense |
| PD13619 | PD13619a | MAP3K1 | Del | frameshift |
| PD13427 | PD13427a | MAP3K1 | Del | frameshift |
| PD13618 | PD13618a | MAP3K1 | Ins | frameshift |

(continued)

| Sample | Tumour_name | Gene | Mutation_Type | Effect |
|---|---|---|---|---|
| PD13618 | PD13618a | MAP3K1 | Ins | frameshift |
| PD4977 | PD4977a | MAP3K1 | Del | frameshift |
| PD11361 | PD11361a | MAP3K1 | Ins | frameshift |
| PD11361 | PD11361a | MAP3K1 | Del | frameshift |
| PD18020 | PD18020a | MAP3K1 | CopyNumber | HD |
| PD6684 | PD6684a | MAP3K1 | CopyNumber | HD |
| PD22361 | PD22361a | MAP3K1 | Rearrangement | 1|Tandem duplication |
| PD13307 | PD13307a | MAP3K1 | Rearrangement | 1|Translocation |
| PD4956 | PD4956a | MAP3K1 | Rearrangement | 1|Translocation |
| PD22360 | PD22360a | MAP3K1 | Rearrangement | 1|Translocation |
| PD4847 | PD4847a | MAP3K1 | Rearrangement | 1|Tandem duplication |
| PD4836 | PD4836a | MAP3K1 | Rearrangement | 1|Deletion |
| PD13602 | PD13602a | MAP3K1 | Rearrangement | 1|Tandem duplication |
| PD7201 | PD7201a | MAP3K1 | Rearrangement | 2|Deletion| Tandem duplication |
| PD4225 | PD4225a | MAP3K1 | Rearrangement | 3|Inversion| Inversion|Deletion |
| PD8618 | PD8618a | MAP3K1 | Rearrangement | 1|Deletion |
| PD11740 | PD11740a | MAP3K1 | Rearrangement | 1|Deletion |
| PD11360 | PD11360a | MDM2 | CopyNumber | amp |
| PD11368 | PD11368a | MDM2 | CopyNumber | amp |
| PD11369 | PD11369a | MDM2 | CopyNumber | amp |
| PD13605 | PD13605a | MDM2 | CopyNumber | amp |
| PD13766 | PD13766a | MDM2 | CopyNumber | amp |
| PD18149 | PD18149a | MDM2 | CopyNumber | amp |
| PD22362 | PD22362a | MDM2 | CopyNumber | amp |
| PD4315 | PD4315a | MDM2 | CopyNumber | amp |
| PD4978 | PD4978a | MDM2 | CopyNumber | amp |
| PD5946 | PD5946a | MDM2 | CopyNumber | amp |
| PD6422 | PD6422a | MDM2 | CopyNumber | amp |
| PD6733 | PD6733b | MDM2 | CopyNumber | amp |
| PD7205 | PD7205a | MDM2 | CopyNumber | amp |
| PD7307 | PD7307a | MDM2 | CopyNumber | amp |
| PD9000 | PD9000a | MDM2 | CopyNumber | amp |
| PD24307 | PD24307a | MED23 | Del | frameshift |
| PD18116 | PD18116a | MED23 | Sub | nonsense |
| PD7238 | PD7238a | MED23 | Del | frameshift |
| PD23564 | PD23564a | MED23 | Del | frameshift |
| PD24216 | PD24216a | MED23 | Ins | frameshift |
| PD11762 | PD11762a | MED23 | Sub | ess splice |

(continued)

| Sample | Tumour_name | Gene | Mutation_Type | Effect |
|---|---|---|---|---|
| PD9578 | PD9578a | MED23 | Sub | ess splice |
| PD7322 | PD7322a | MEN1 | Del | frameshift |
| PD24193 | PD24193a | MLH1 | Sub | ess splice |
| PD6412 | PD6412a | MLH1 | Del | ess splice |
| PD4109 | PD4109a | MLL2 | Sub | nonsense |
| PD6412 | PD6412a | MLL2 | Del | frameshift |
| PD4006 | PD4006a | MLL2 | Del | frameshift |
| PD6412 | PD6412a | MLL2 | Del | frameshift |
| PD13754 | PD13754a | MLL2 | Sub | ess splice |
| PD11389 | PD11389a | MLL3 | Del | frameshift |
| PD13771 | PD13771a | MLL3 | Del | frameshift |
| PD18733 | PD18733a | MLL3 | Del | frameshift |
| PD7249 | PD7249a | MLL3 | Sub | nonsense |
| PD7217 | PD7217a | MLL3 | Sub | nonsense |
| PD7210 | PD7210a | MLL3 | Sub | nonsense |
| PD5937 | PD5937a | MLL3 | Sub | nonsense |
| PD24333 | PD24333a | MLL3 | Ins | frameshift |
| PD13753 | PD13753a | MLL3 | Del | frameshift |
| PD13758 | PD13758a | MLL3 | Del | frameshift |
| PD13425 | PD13425a | MLL3 | Sub | nonsense |
| PD18756 | PD18756a | MLL3 | Sub | nonsense |
| PD6414 | PD6414a | MLL3 | Del | frameshift |
| PD6412 | PD6412a | MLL3 | Del | frameshift |
| PD23564 | PD23564a | MLL3 | Del | frameshift |
| PD13762 | PD13762a | MLL3 | Del | frameshift |
| PD4103 | PD4103a | MLL3 | Sub | ess splice |
| PD11344 | PD11344a | MLL3 | Sub | nonsense |
| PD24204 | PD24204a | MLL3 | Sub | nonsense |
| PD9541 | PD9541a | MLL3 | Del | frameshift |
| PD13304 | PD13304a | MLL3 | Del | frameshift |
| PD11372 | PD11372a | MLL3 | Sub | nonsense |
| PD24204 | PD24204a | MLL3 | Ins | frameshift |
| PD10011 | PD10011a | MLL3 | Sub | ess splice |
| PD11402 | PD11402a | MLL3 | Del | frameshift |
| PD4981 | PD4981a | MLL3 | Sub | nonsense |
| PD11352 | PD11352a | MLL3 | Del | frameshift |
| PD22364 | PD22364a | MLL3 | Del | frameshift |
| PD11336 | PD11336a | MLL3 | Sub | nonsense |
| PD9589 | PD9589a | MLL3 | Ins | frameshift |

(continued)

| Sample | Tumour_name | Gene | Mutation_Type | Effect |
|---|---|---|---|---|
| PD23561 | PD23561a | MLL3 | Sub | nonsense |
| PD18046 | PD18046a | MLL3 | Ins | frameshift |
| PD9064 | PD9064a | MLL3 | Del | frameshift |
| PD13298 | PD13298a | MLL3 | Del | frameshift |
| PD5944 | PD5944a | MLL3 | Del | frameshift |
| PD6412 | PD6412a | MLL3 | Del | frameshift |
| PD4967 | PD4967a | MLLT4 | Del | frameshift |
| PD4005 | PD4005a | MLLT4 | Sub | nonsense |
| PD9575 | PD9575a | MLLT4 | Del | frameshift |
| PD22251 | PD22251a | MLLT4 | Del | frameshift |
| PD14457 | PD14457a | MLLT4 | Ins | frameshift |
| PD13771 | PD13771a | MLLT4 | Sub | nonsense |
| PD24189 | PD24189a | MLLT4 | Del | frameshift |
| PD6043 | PD6043a | MLLT4 | Complex | frameshift |
| PD8660 | PD8660a2 | MLLT4 | Rearrangement | 1|Tandem duplication |
| PD11750 | PD11750a | MLLT4 | Rearrangement | 1|Translocation |
| PD4005 | PD4005a | MLLT4 | Rearrangement | 1|Translocation |
| PD11751 | PD11751a | MLLT4 | Rearrangement | 1|Translocation |
| PD9585 | PD9585a | MLLT4 | Rearrangement | 3|Translocation| Translocation| Tandem duplication |
| PD9702 | PD9702a | MLLT4 | Rearrangement | 1|Tandem duplication |
| PD9595 | PD9595a | MLLT4 | Rearrangement | 1|Deletion |
| PD8997 | PD8997a | MLLT4 | Rearrangement | 1|Deletion |
| PD23563 | PD23563a | MLLT4 | Rearrangement | 1|Inversion |
| PD11380 | PD11380a | MLLT4 | Rearrangement | 1|Deletion |
| PD4264 | PD4264a | MLLT4 | Rearrangement | 1|Inversion |
| PD11752 | PD11752a | MLLT4 | Rearrangement | 1|Tandem duplication |
| PD8996 | PD8996a | MLLT4 | Rearrangement | 1|Deletion |
| PD5956 | PD5956a | MLLT4 | Rearrangement | 1|Deletion |
| PD24329 | PD24329a | MLLT4 | Rearrangement | 1|Translocation |
| PD23577 | PD23577a | MLLT4 | Rearrangement | 1|Tandem duplication |
| PD5937 | PD5937a | MSH2 | Sub | nonsense |
| PD5937 | PD5937a | MSH2 | Sub | nonsense |
| PD10014 | PD10014a | MYC | CopyNumber | amp |
| PD11339 | PD11339a | MYC | CopyNumber | amp |
| PD11345 | PD11345a | MYC | CopyNumber | amp |
| PD11346 | PD11346a | MYC | CopyNumber | amp |
| PD11367 | PD11367a | MYC | CopyNumber | amp |
| PD11368 | PD11368a | MYC | CopyNumber | amp |
| PD11375 | PD11375a | MYC | CopyNumber | amp |

(continued)

| Sample | Tumour_name | Gene | Mutation_Type | Effect |
|--------|-------------|------|---------------|--------|
| PD11385 | PD11385a | MYC | CopyNumber | amp |
| PD11393 | PD11393a | MYC | CopyNumber | amp |
| PD11394 | PD11394a | MYC | CopyNumber | amp |
| PD11398 | PD11398a | MYC | CopyNumber | amp |
| PD11464 | PD11464a | MYC | CopyNumber | amp |
| PD11750 | PD11750a | MYC | CopyNumber | amp |
| PD11756 | PD11756a | MYC | CopyNumber | amp |
| PD13162 | PD13162a | MYC | CopyNumber | amp |
| PD13164 | PD13164a | MYC | CopyNumber | amp |
| PD13166 | PD13166a | MYC | CopyNumber | amp |
| PD13167 | PD13167a | MYC | CopyNumber | amp |
| PD13298 | PD13298a | MYC | CopyNumber | amp |
| PD13299 | PD13299a | MYC | CopyNumber | amp |
| PD13418 | PD13418a | MYC | CopyNumber | amp |
| PD13602 | PD13602a | MYC | CopyNumber | amp |
| PD13607 | PD13607a | MYC | CopyNumber | amp |
| PD13609 | PD13609a | MYC | CopyNumber | amp |
| PD14435 | PD14435a | MYC | CopyNumber | amp |
| PD14437 | PD14437a | MYC | CopyNumber | amp |
| PD17981 | PD17981a | MYC | CopyNumber | amp |
| PD17994 | PD17994a | MYC | CopyNumber | amp |
| PD18020 | PD18020a | MYC | CopyNumber | amp |
| PD18022 | PD18022a | MYC | CopyNumber | amp |
| PD18031 | PD18031a | MYC | CopyNumber | amp |
| PD18045 | PD18045a | MYC | CopyNumber | amp |
| PD18046 | PD18046a | MYC | CopyNumber | amp |
| PD18047 | PD18047a | MYC | CopyNumber | amp |
| PD18149 | PD18149a | MYC | CopyNumber | amp |
| PD18251 | PD18251a | MYC | CopyNumber | amp |
| PD18734 | PD18734a | MYC | CopyNumber | amp |
| PD22357 | PD22357a | MYC | CopyNumber | amp |
| PD22361 | PD22361a | MYC | CopyNumber | amp |
| PD23558 | PD23558a | MYC | CopyNumber | amp |
| PD23563 | PD23563a | MYC | CopyNumber | amp |
| PD23577 | PD23577a | MYC | CopyNumber | amp |
| PD24191 | PD24191a | MYC | CopyNumber | amp |
| PD24192 | PD24192a | MYC | CopyNumber | amp |
| PD24196 | PD24196a | MYC | CopyNumber | amp |
| PD24201 | PD24201a | MYC | CopyNumber | amp |

(continued)

| Sample | Tumour_name | Gene | Mutation_Type | Effect |
|---|---|---|---|---|
| PD24214 | PD24214a | MYC | CopyNumber | amp |
| PD24215 | PD24215a | MYC | CopyNumber | amp |
| PD24218 | PD24218a | MYC | CopyNumber | amp |
| PD24220 | PD24220a | MYC | CopyNumber | amp |
| PD24308 | PD24308a | MYC | CopyNumber | amp |
| PD24322 | PD24322a | MYC | CopyNumber | amp |
| PD24325 | PD24325a | MYC | CopyNumber | amp |
| PD24337 | PD24337a | MYC | CopyNumber | amp |
| PD3890 | PD3890a | MYC | CopyNumber | amp |
| PD3945 | PD3945a | MYC | CopyNumber | amp |
| PD4005 | PD4005a | MYC | CopyNumber | amp |
| PD4103 | PD4103a | MYC | CopyNumber | amp |
| PD4115 | PD4115a | MYC | CopyNumber | amp |
| PD4116 | PD4116a | MYC | CopyNumber | amp |
| PD4255 | PD4255a | MYC | CopyNumber | amp |
| PD4315 | PD4315a | MYC | CopyNumber | amp |
| PD4826 | PD4826a | MYC | CopyNumber | amp |
| PD4841 | PD4841a | MYC | CopyNumber | amp |
| PD4876 | PD4876a | MYC | CopyNumber | amp |
| PD4953 | PD4953a | MYC | CopyNumber | amp |
| PD4954 | PD4954a | MYC | CopyNumber | amp |
| PD4955 | PD4955a | MYC | CopyNumber | amp |
| PD4956 | PD4956a | MYC | CopyNumber | amp |
| PD4957 | PD4957a | MYC | CopyNumber | amp |
| PD4978 | PD4978a | MYC | CopyNumber | amp |
| PD5942 | PD5942a | MYC | CopyNumber | amp |
| PD5948 | PD5948a | MYC | CopyNumber | amp |
| PD5950 | PD5950a | MYC | CopyNumber | amp |
| PD5951 | PD5951a | MYC | CopyNumber | amp |
| PD6042 | PD6042a | MYC | CopyNumber | amp |
| PD6044 | PD6044a | MYC | CopyNumber | amp |
| PD6047 | PD6047a | MYC | CopyNumber | amp |
| PD6409 | PD6409a | MYC | CopyNumber | amp |
| PD6413 | PD6413a | MYC | CopyNumber | amp |
| PD6727 | PD6727b | MYC | CopyNumber | amp |
| PD6728 | PD6728b | MYC | CopyNumber | amp |
| PD6733 | PD6733b | MYC | CopyNumber | amp |
| PD7066 | PD7066a | MYC | CopyNumber | amp |
| PD7069 | PD7069a | MYC | CopyNumber | amp |

(continued)

| Sample | Tumour_name | Gene | Mutation_Type | Effect |
|---|---|---|---|---|
| PD7215 | PD7215a | MYC | CopyNumber | amp |
| PD7243 | PD7243a | MYC | CopyNumber | amp |
| PD7250 | PD7250a | MYC | CopyNumber | amp |
| PD7316 | PD7316a | MYC | CopyNumber | amp |
| PD7428 | PD7428a | MYC | CopyNumber | amp |
| PD8611 | PD8611a | MYC | CopyNumber | amp |
| PD8969 | PD8969a | MYC | CopyNumber | amp |
| PD8980 | PD8980a | MYC | CopyNumber | amp |
| PD8982 | PD8982a | MYC | CopyNumber | amp |
| PD8984 | PD8984a | MYC | CopyNumber | amp |
| PD8996 | PD8996a | MYC | CopyNumber | amp |
| PD9000 | PD9000a | MYC | CopyNumber | amp |
| PD9002 | PD9002a | MYC | CopyNumber | amp |
| PD9539 | PD9539a | MYC | CopyNumber | amp |
| PD9567 | PD9567a | MYC | CopyNumber | amp |
| PD9569 | PD9569a | MYC | CopyNumber | amp |
| PD9576 | PD9576a | MYC | CopyNumber | amp |
| PD9591 | PD9591a | MYC | CopyNumber | amp |
| PD9599 | PD9599a | MYC | CopyNumber | amp |
| PD9604 | PD9604a | MYC | CopyNumber | amp |
| PD9696 | PD9696a | MYC | CopyNumber | amp |
| PD9702 | PD9702a | MYC | CopyNumber | amp |
| PD9754 | PD9754a | MYC | CopyNumber | amp |
| PD9760 | PD9760a | MYC | CopyNumber | amp |
| PD22251 | PD22251a | NCOR1 | Sub | nonsense |
| PD11740 | PD11740a | NCOR1 | Sub | nonsense |
| PD4072 | PD4072a | NCOR1 | Sub | nonsense |
| PD5961 | PD5961a | NCOR1 | Sub | nonsense |
| PD24217 | PD24217a | NCOR1 | Sub | nonsense |
| PD11745 | PD11745a | NCOR1 | Del | frameshift |
| PD3945 | PD3945a | NCOR1 | Del | frameshift |
| PD18047 | PD18047a | NCOR1 | Sub | nonsense |
| PD13625 | PD13625a | NCOR1 | Ins | frameshift |
| PD24320 | PD24320a | NCOR1 | Del | frameshift |
| PD24215 | PD24215a | NCOR1 | Del | frameshift |
| PD4605 | PD4605a | NCOR1 | Ins | frameshift |
| PD11367 | PD11367a | NCOR1 | CopyNumber | HD |
| PD23564 | PD23564a | NF1 | Del | frameshift |
| PD13163 | PD13163a | NF1 | Sub | ess splice |

(continued)

| Sample | Tumour_name | Gene | Mutation_Type | Effect |
|--------|-------------|------|---------------|--------|
| PD24202 | PD24202a | NF1 | Sub | nonsense |
| PD4976 | PD4976a | NF1 | Sub | ess splice |
| PD7426 | PD7426a | NF1 | Sub | nonsense |
| PD22036 | PD22036a | NF1 | Sub | nonsense |
| PD6719 | PD6719a | NF1 | Del | frameshift |
| PD4605 | PD4605a | NF1 | Ins | frameshift |
| PD7240 | PD7240a | NF1 | Sub | nonsense |
| PD24192 | PD24192a | NF1 | CopyNumber | HD |
| PD5934 | PD5934a | NF2 | Sub | ess splice |
| PD9568 | PD9568a | NOTCH1 | Del | frameshift |
| PD4315 | PD4315a | NOTCH1 | Ins | ess splice |
| PD23564 | PD23564a | NOTCH2 | Del | frameshift |
| PD5950 | PD5950a | NOTCH2 | Sub | nonsense |
| PD8619 | PD8619a | NOTCH2 | Sub | nonsense |
| PD13298 | PD13298a | NOTCH2 | Sub | nonsense |
| PD8618 | PD8618a | NOTCH2 | Sub | ess splice |
| PD11345 | PD11345a | NOTCH2 | Sub | nonsense |
| PD24212 | PD24212a | NRAS | Sub | missense |
| PD11340 | PD11340a | PALB2 | Del | ess splice |
| PD24205 | PD24205a | PALB2 | Sub | nonsense |
| PD24212 | PD24212a | PALB2 | Del | frameshift |
| PD8980 | PD8980a | PBRM1 | Del | frameshift |
| PD11349 | PD11349a | PDGFRA | CopyNumber | amp |
| PD23560 | PD23560a | PDGFRA | CopyNumber | amp |
| PD6410 | PD6410a | PDGFRA | CopyNumber | amp |
| PD7428 | PD7428a | PDGFRA | CopyNumber | amp |
| PD9591 | PD9591a | PHF6 | Del | frameshift |
| PD5930 | PD5930a | PHF6 | Sub | nonsense |
| PD4970 | PD4970a | PIK3CA | Del | inframe |
| PD11342 | PD11342a | PIK3CA | Del | complex sub |
| PD13619 | PD13619a | PIK3CA | Sub | missense |
| PD4967 | PD4967a | PIK3CA | Sub | missense |
| PD24320 | PD24320a | PIK3CA | Sub | missense |
| PD4605 | PD4605a | PIK3CA | Del | inframe |
| PD13758 | PD13758a | PIK3CA | Sub | missense |
| PD17994 | PD17994a | PIK3CA | Del | inframe |
| PD9595 | PD9595a | PIK3CA | Del | complex sub |
| PD4844 | PD4844a | PIK3CA | Del | inframe |
| PD6720 | PD6720a | PIK3CA | Sub | missense |

(continued)

| Sample | Tumour_name | Gene | Mutation_Type | Effect |
|--------|-------------|------|---------------|--------|
| PD24329 | PD24329a | PIK3CA | Sub | missense |
| PD11380 | PD11380a | PIK3CA | Sub | missense |
| PD11816 | PD11816a | PIK3CA | Sub | missense |
| PD13631 | PD13631a | PIK3CA | Sub | missense |
| PD11366 | PD11366a | PIK3CA | Sub | missense |
| PD14473 | PD14473a | PIK3CA | Sub | missense |
| PD18050 | PD18050a | PIK3CA | Sub | missense |
| PD7219 | PD7219a | PIK3CA | Sub | missense |
| PD4261 | PD4261a | PIK3CA | Sub | missense |
| PD4969 | PD4969a | PIK3CA | Sub | missense |
| PD18247 | PD18247a | PIK3CA | Sub | missense |
| PD18047 | PD18047a | PIK3CA | Sub | missense |
| PD11372 | PD11372a | PIK3CA | Sub | missense |
| PD13425 | PD13425a | PIK3CA | Sub | missense |
| PD11465 | PD11465a | PIK3CA | Sub | missense |
| PD22361 | PD22361a | PIK3CA | Sub | missense |
| PD11767 | PD11767a | PIK3CA | Sub | missense |
| PD9844 | PD9844a | PIK3CA | Sub | missense |
| PD13753 | PD13753a | PIK3CA | Del | inframe |
| PD13626 | PD13626a | PIK3CA | Del | complex sub |
| PD4977 | PD4977a | PIK3CA | Sub | missense |
| PD24193 | PD24193a | PIK3CA | Sub | missense |
| PD9063 | PD9063a | PIK3CA | Sub | missense |
| PD23558 | PD23558a | PIK3CA | Sub | missense |
| PD13299 | PD13299a | PIK3CA | Sub | missense |
| PD11352 | PD11352a | PIK3CA | Sub | missense |
| PD11359 | PD11359a | PIK3CA | Sub | missense |
| PD13623 | PD13623a | PIK3CA | Sub | missense |
| PD13760 | PD13760a | PIK3CA | Sub | missense |
| PD18100 | PD18100a | PIK3CA | Sub | missense |
| PD4267 | PD4267a | PIK3CA | Sub | missense |
| PD5951 | PD5951a | PIK3CA | Sub | missense |
| PD9579 | PD9579a | PIK3CA | Sub | missense |
| PD6416 | PD6416a | PIK3CA | Sub | missense |
| PD13629 | PD13629a | PIK3CA | Sub | missense |
| PD23561 | PD23561a | PIK3CA | Sub | missense |
| PD9755 | PD9755a | PIK3CA | Sub | missense |
| PD17981 | PD17981a | PIK3CA | Sub | missense |
| PD11745 | PD11745a | PIK3CA | Sub | missense |

(continued)

| Sample | Tumour_name | Gene | Mutation_Type | Effect |
|---|---|---|---|---|
| PD9002 | PD9002a | PIK3CA | Sub | missense |
| PD24225 | PD24225a | PIK3CA | Sub | missense |
| PD3905 | PD3905a | PIK3CA | Sub | missense |
| PD9067 | PD9067a | PIK3CA | Sub | missense |
| PD11343 | PD11343a | PIK3CA | Sub | missense |
| PD24333 | PD24333a | PIK3CA | Sub | missense |
| PD13757 | PD13757a | PIK3CA | Sub | missense |
| PD5937 | PD5937a | PIK3CA | Sub | missense |
| PD9606 | PD9606a | PIK3CA | Sub | missense |
| PD11402 | PD11402a | PIK3CA | Sub | missense |
| PD13762 | PD13762a | PIK3CA | Sub | missense |
| PD9467 | PD9467a | PIK3CA | Sub | missense |
| PD11395 | PD11395a | PIK3CA | Sub | missense |
| PD13770 | PD13770a | PIK3CA | Sub | missense |
| PD18748 | PD18748a | PIK3CA | Sub | missense |
| PD14472 | PD14472a | PIK3CA | Sub | missense |
| PD13162 | PD13162a | PIK3CA | Sub | missense |
| PD18046 | PD18046a | PIK3CA | Sub | missense |
| PD7209 | PD7209a | PIK3CA | Sub | missense |
| PD6417 | PD6417a | PIK3CA | Sub | missense |
| PD4225 | PD4225a | PIK3CA | Sub | missense |
| PD4315 | PD4315a | PIK3CA | Sub | missense |
| PD4976 | PD4976a | PIK3CA | Sub | missense |
| PD4965 | PD4965a | PIK3CA | Sub | missense |
| PD3989 | PD3989a | PIK3CA | Sub | missense |
| PD4072 | PD4072a | PIK3CA | Sub | missense |
| PD4982 | PD4982a | PIK3CA | Sub | missense |
| PD11379 | PD11379a | PIK3CA | Sub | missense |
| PD18749 | PD18749a | PIK3CA | Sub | missense |
| PD4978 | PD4978a | PIK3CA | Sub | missense |
| PD13767 | PD13767a | PIK3CA | Sub | missense |
| PD24307 | PD24307a | PIK3CA | Sub | missense |
| PD11327 | PD11327a | PIK3CA | Sub | missense |
| PD18116 | PD18116a | PIK3CA | Sub | missense |
| PD9578 | PD9578a | PIK3CA | Sub | missense |
| PD24327 | PD24327a | PIK3CA | Sub | missense |
| PD24329 | PD24329a | PIK3CA | Sub | missense |
| PD9541 | PD9541a | PIK3CA | Sub | missense |
| PD13165 | PD13165a | PIK3CA | Sub | missense |

(continued)

| Sample | Tumour_name | Gene | Mutation_Type | Effect |
|---|---|---|---|---|
| PD4986 | PD4986a | PIK3CA | Sub | missense |
| PD9574 | PD9574a | PIK3CA | Sub | missense |
| PD24207 | PD24207a | PIK3CA | Sub | missense |
| PD11345 | PD11345a | PIK3CA | Sub | missense |
| PD24199 | PD24199a | PIK3CA | Sub | missense |
| PD11345 | PD11345a | PIK3CA | Sub | missense |
| PD4069 | PD4069a | PIK3CA | Sub | missense |
| PD11381 | PD11381a | PIK3CA | Sub | missense |
| PD13618 | PD13618a | PIK3CA | Sub | missense |
| PD5937 | PD5937a | PIK3CA | Sub | missense |
| PD24333 | PD24333a | PIK3CA | Sub | missense |
| PD9063 | PD9063a | PIK3CA | Sub | missense |
| PD24189 | PD24189a | PIK3CA | Sub | missense |
| PD18776 | PD18776a | PIK3CA | Sub | missense |
| PD4844 | PD4844a | PIK3CA | Sub | missense |
| PD4607 | PD4607a | PIK3CA | Sub | missense |
| PD13770 | PD13770a | PIK3CA | Sub | missense |
| PD24193 | PD24193a | PIK3CA | Sub | missense |
| PD11338 | PD11338a | PIK3CA | Sub | missense |
| PD9756 | PD9756a | PIK3CA | Sub | missense |
| PD9544 | PD9544a | PIK3CA | Sub | missense |
| PD8828 | PD8828a | PIK3CA | Sub | missense |
| PD13426 | PD13426a | PIK3CA | Sub | missense |
| PD6016 | PD6016a2 | PIK3CA | Sub | missense |
| PD11386 | PD11386a | PIK3CA | Sub | missense |
| PD11762 | PD11762a | PIK3CA | Sub | missense |
| PD8623 | PD8623a | PIK3CA | Sub | missense |
| PD8622 | PD8622a | PIK3CA | Sub | missense |
| PD8617 | PD8617a | PIK3CA | Sub | missense |
| PD18751 | PD18751a | PIK3CA | Sub | missense |
| PD13618 | PD13618a | PIK3CA | Sub | missense |
| PD11364 | PD11364a | PIK3CA | Sub | missense |
| PD13619 | PD13619a | PIK3CA | Sub | missense |
| PD4076 | PD4076a | PIK3CA | Sub | missense |
| PD17991 | PD17991a | PIK3CA | Sub | missense |
| PD4085 | PD4085a | PIK3CA | Sub | missense |
| PD11347 | PD11347a | PIK3CA | Sub | missense |
| PD4967 | PD4967a | PIK3CA | Sub | missense |
| PD7199 | PD7199a | PIK3CA | Sub | missense |

(continued)

| Sample | Tumour_name | Gene | Mutation_Type | Effect |
|---|---|---|---|---|
| PD5928 | PD5928a | PIK3CA | Sub | missense |
| PD4985 | PD4985a | PIK3CA | Sub | missense |
| PD4977 | PD4977a | PIK3CA | Sub | missense |
| PD9570 | PD9570a | PIK3CA | Sub | missense |
| PD7201 | PD7201a | PIK3CA | Sub | missense |
| PD4266 | PD4266a | PIK3CA | Sub | missense |
| PD13602 | PD13602a | PIK3CA | Sub | missense |
| PD14465 | PD14465a | PIK3CA | Sub | missense |
| PD24204 | PD24204a | PIK3CA | Sub | missense |
| PD24199 | PD24199a | PIK3CA | Sub | missense |
| PD9009 | PD9009a | PIK3CA | Sub | missense |
| PD8965 | PD8965a | PIK3CA | Sub | missense |
| PD18734 | PD18734a | PIK3CA | Sub | missense |
| PD6041 | PD6041a | PIK3CA | Sub | missense |
| PD23579 | PD23579a | PIK3CA | Sub | missense |
| PD22359 | PD22359a | PIK3CA | Sub | missense |
| PD18269 | PD18269a | PIK3CA | Sub | missense |
| PD13768 | PD13768a | PIK3CA | Sub | missense |
| PD18258 | PD18258a | PIK3CA | Sub | missense |
| PD18247 | PD18247a | PIK3CA | Sub | missense |
| PD11744 | PD11744a | PIK3CA | Sub | missense |
| PD13304 | PD13304a | PIK3CA | Sub | missense |
| PD18768 | PD18768a | PIK3CA | Sub | missense |
| PD14459 | PD14459a | PIK3CA | Sub | missense |
| PD24189 | PD24189a | PIK3CA | Sub | missense |
| PD24215 | PD24215a | PIK3CA | Sub | missense |
| PD24217 | PD24217a | PIK3CA | Sub | missense |
| PD24224 | PD24224a | PIK3CA | Sub | missense |
| PD6466 | PD6466b | PIK3CA | Sub | missense |
| PD18754 | PD18754a | PIK3CA | Sub | missense |
| PD24326 | PD24326a | PIK3CA | Sub | missense |
| PD11766 | PD11766a | PIK3CA | Sub | missense |
| PD18756 | PD18756a | PIK3CA | Sub | missense |
| PD4192 | PD4192a | PIK3CA | Sub | missense |
| PD24336 | PD24336a | PIK3CA | Sub | missense |
| PD24302 | PD24302a | PIK3CA | Sub | missense |
| PD24194 | PD24194a | PIK3CA | Sub | missense |
| PD18022 | PD18022a | PIK3CA | Sub | missense |
| PD9584 | PD9584a | PIK3CA | Sub | missense |

(continued)

| Sample | Tumour_name | Gene | Mutation_Type | Effect |
|--------|-------------|------|---------------|--------|
| PD9591 | PD9591a | PIK3CA | Sub | missense |
| PD8615 | PD8615a | PIK3CA | Sub | missense |
| PD14441 | PD14441a | PIK3CA | Sub | missense |
| PD14439 | PD14439a | PIK3CA | Sub | missense |
| PD18728 | PD18728a | PIK3CA | Sub | missense |
| PD11327 | PD11327a | PIK3CA | CopyNumber | amp |
| PD13296 | PD13296a | PIK3CA | CopyNumber | amp |
| PD13428 | PD13428a | PIK3CA | CopyNumber | amp |
| PD22355 | PD22355a | PIK3CA | CopyNumber | amp |
| PD22366 | PD22366a | PIK3CA | CopyNumber | amp |
| PD23558 | PD23558a | PIK3CA | CopyNumber | amp |
| PD23562 | PD23562a | PIK3CA | CopyNumber | amp |
| PD24202 | PD24202a | PIK3CA | CopyNumber | amp |
| PD24224 | PD24224a | PIK3CA | CopyNumber | amp |
| PD5945 | PD5945a | PIK3CA | CopyNumber | amp |
| PD5950 | PD5950a | PIK3CA | CopyNumber | amp |
| PD6411 | PD6411a | PIK3CA | CopyNumber | amp |
| PD7428 | PD7428a | PIK3CA | CopyNumber | amp |
| PD8611 | PD8611a | PIK3CA | CopyNumber | amp |
| PD8980 | PD8980a | PIK3CA | CopyNumber | amp |
| PD4847 | PD4847a | PIK3R1 | Ins | frameshift |
| PD6412 | PD6412a | PIK3R1 | Del | frameshift |
| PD5925 | PD5925a | PIK3R1 | Del | frameshift |
| PD7217 | PD7217a | PIK3R1 | Del | ess splice |
| PD6042 | PD6042a | PIK3R1 | Del | inframe |
| PD9599 | PD9599a | PIK3R1 | Del | inframe |
| PD4952 | PD4952a | PIK3R1 | Del | inframe |
| PD23567 | PD23567a | PIK3R1 | Sub | ess splice |
| PD9575 | PD9575a | PIK3R1 | Del | inframe |
| PD24195 | PD24195a | PIK3R1 | Del | ess splice |
| PD22361 | PD22361a | PMS2 | Sub | nonsense |
| PD13296 | PD13296a | PRDM1 | Sub | nonsense |
| PD6406 | PD6406a | PREX2 | Sub | missense |
| PD8652 | PD8652a2 | PTEN | Del | ess splice |
| PD4872 | PD4872a | PTEN | Sub | missense |
| PD11376 | PD11376a | PTEN | Del | ess splice |
| PD9589 | PD9589a | PTEN | Del | frameshift |
| PD6422 | PD6422a | PTEN | Sub | nonsense |
| PD23564 | PD23564a | PTEN | Sub | nonsense |

(continued)

| Sample | Tumour_name | Gene | Mutation_Type | Effect |
|--------|-------------|------|---------------|--------|
| PD10014 | PD10014a | PTEN | Sub | missense |
| PD9694 | PD9694a | PTEN | Sub | missense |
| PD4958 | PD4958a | PTEN | Del | frameshift |
| PD5961 | PD5961a | PTEN | Sub | ess splice |
| PD18257 | PD18257a | PTEN | Sub | missense |
| PD11742 | PD11742a | PTEN | Del | inframe |
| PD5944 | PD5944a | PTEN | Sub | ess splice |
| PD11368 | PD11368a | PTEN | Sub | nonsense |
| PD23564 | PD23564a | PTEN | Sub | nonsense |
| PD8965 | PD8965a | PTEN | Sub | nonsense |
| PD11355 | PD11355a | PTEN | Del | frameshift |
| PD24320 | PD24320a | PTEN | Ins | frameshift |
| PD13422 | PD13422a | PTEN | Sub | ess splice |
| PD10011 | PD10011a | PTEN | Sub | ess splice |
| PD5953 | PD5953a | PTEN | Ins | frameshift |
| PD4980 | PD4980a | PTEN | Sub | nonsense |
| PD23567 | PD23567a | PTEN | Del | frameshift |
| PD3989 | PD3989a | PTEN | Del | frameshift |
| PD6418 | PD6418a | PTEN | Del | frameshift |
| PD7240 | PD7240a | PTEN | Del | frameshift |
| PD11372 | PD11372a | PTEN | Del | frameshift |
| PD9575 | PD9575a | PTEN | Del | frameshift |
| PD6711 | PD6711a2 | PTEN | Del | ess splice |
| PD24191 | PD24191a | PTEN | Del | ess splice |
| PD23570 | PD23570a | PTEN | Sub | nonsense |
| PD8615 | PD8615a | PTEN | Ins | frameshift |
| PD24190 | PD24190a | PTEN | Del | frameshift |
| PD13311 | PD13311a | PTEN | CopyNumber | HD |
| PD13627 | PD13627a | PTEN | CopyNumber | HD |
| PD13763 | PD13763a | PTEN | CopyNumber | HD |
| PD14460 | PD14460a | PTEN | CopyNumber | HD |
| PD18024 | PD18024a | PTEN | CopyNumber | HD |
| PD23558 | PD23558a | PTEN | CopyNumber | HD |
| PD23562 | PD23562a | PTEN | CopyNumber | HD |
| PD23577 | PD23577a | PTEN | CopyNumber | HD |
| PD24325 | PD24325a | PTEN | CopyNumber | HD |
| PD4607 | PD4607a | PTEN | CopyNumber | HD |
| PD4844 | PD4844a | PTEN | CopyNumber | HD |
| PD4874 | PD4874a | PTEN | CopyNumber | HD |

(continued)

| Sample | Tumour_name | Gene | Mutation_Type | Effect |
|---|---|---|---|---|
| PD5935 | PD5935a | PTEN | CopyNumber | HD |
| PD5945 | PD5945a | PTEN | CopyNumber | HD |
| PD7220 | PD7220a | PTEN | CopyNumber | HD |
| PD9752 | PD9752a | PTEN | CopyNumber | HD |
| PD6722 | PD6722a | PTEN | Rearrangement | 1\|Deletion |
| PD22357 | PD22357a | PTEN | Rearrangement | 1\|Inversion |
| PD7426 | PD7426a | PTEN | Rearrangement | 2\|Inversion\|Inversion |
| PD7217 | PD7217a | PTEN | Rearrangement | 1\|Deletion |
| PD7248 | PD7248a | PTEN | Rearrangement | 1\|Tandem duplication |
| PD9004 | PD9004a | PTEN | Rearrangement | 1\|Deletion |
| PD6733 | PD6733b | PTEN | Rearrangement | 2\|Deletion\|Deletion |
| PD4116 | PD4116a | PTEN | Rearrangement | 1\|Deletion |
| PD9585 | PD9585a | PTEN | Rearrangement | 1\|Tandem duplication |
| PD9702 | PD9702a | PTEN | Rearrangement | 2\|Tandem duplication\|Tandem duplication |
| PD8984 | PD8984a | PTEN | Rearrangement | 1\|Deletion |
| PD7321 | PD7321a | PTEN | Rearrangement | 1\|Tandem duplication |
| PD24202 | PD24202a | PTEN | Rearrangement | 1\|Tandem duplication |
| PD8611 | PD8611a | PTEN | Rearrangement | 2\|Tandem duplication\|Tandem duplication |
| PD9575 | PD9575a | PTEN | Rearrangement | 1\|Inversion |
| PD6413 | PD6413a | PTEN | Rearrangement | 2\|Inversion\|Translocation |
| PD8964 | PD8964a | PTEN | Rearrangement | 1\|Deletion |
| PD6410 | PD6410a | PTEN | Rearrangement | 2\|Inversion\|Inversion |
| PD9696 | PD9696a | PTEN | Rearrangement | 1\|Deletion |
| PD11327 | PD11327a | PTEN | Rearrangement | 1\|Deletion |
| PD9752 | PD9752a | PTEN | Rearrangement | 1\|Inversion |
| PD8621 | PD8621a | PTEN | Rearrangement | 1\|Tandem duplication |
| PD24303 | PD24303a | PTEN | Rearrangement | 1\|Tandem duplication |
| PD7211 | PD7211a | PTEN | Rearrangement | 1\|Tandem duplication |
| PD11347 | PD11347a | PTEN | Rearrangement | 1\|Deletion |
| PD11757 | PD11757a | PTEN | Rearrangement | 1\|Deletion |
| PD5948 | PD5948a | PTEN | Rearrangement | 1\|Tandem duplication |
| PD9595 | PD9595a | PTEN | Rearrangement | 1\|Deletion |
| PD7316 | PD7316a | PTEN | Rearrangement | 1\|Deletion |
| PD5934 | PD5934a | PTEN | Rearrangement | 1\|Tandem duplication |
| PD24186 | PD24186a | PTEN | Rearrangement | 1\|Deletion |
| PD5960 | PD5960a | PTEN | Rearrangement | 1\|Deletion |
| PD6406 | PD6406a | PTEN | Rearrangement | 1\|Tandem duplication |
| PD23563 | PD23563a | PTEN | Rearrangement | 1\|Deletion |

(continued)

| Sample | Tumour_name | Gene | Mutation_Type | Effect |
|--------|-------------|------|---------------|--------|
| PD9571 | PD9571a | PTEN | Rearrangement | 1\|Deletion |
| PD10010 | PD10010a | PTEN | Rearrangement | 1\|Deletion |
| PD11755 | PD11755a | PTEN | Rearrangement | 1\|Tandem duplication |
| PD24306 | PD24306a | PTEN | Rearrangement | 1\|Tandem duplication |
| PD9579 | PD9579a | PTEN | Rearrangement | 1\|Deletion |
| PD13622 | PD13622a | PTEN | Rearrangement | 1\|Deletion |
| PD24200 | PD24200a | PTEN | Rearrangement | 1\|Deletion |
| PD6412 | PD6412a | RB1 | Del | frameshift |
| PD13428 | PD13428a | RB1 | Sub | nonsense |
| PD13425 | PD13425a | RB1 | Sub | nonsense |
| PD4975 | PD4975a | RB1 | Sub | nonsense |
| PD6414 | PD6414a | RB1 | Del | frameshift |
| PD9702 | PD9702a | RB1 | Del | ess splice |
| PD24218 | PD24218a | RB1 | Del | ess splice |
| PD22357 | PD22357a | RB1 | Del | frameshift |
| PD4844 | PD4844a | RB1 | Del | frameshift |
| PD11766 | PD11766a | RB1 | Sub | ess splice |
| PD7220 | PD7220a | RB1 | Sub | nonsense |
| PD9568 | PD9568a | RB1 | Del | frameshift |
| PD24194 | PD24194a | RB1 | Del | frameshift |
| PD6722 | PD6722a | RB1 | Sub | nonsense |
| PD4956 | PD4956a | RB1 | Sub | ess splice |
| PD6404 | PD6404a | RB1 | Sub | missense |
| PD4980 | PD4980a | RB1 | Sub | missense |
| PD5942 | PD5942a | RB1 | Sub | ess splice |
| PD8965 | PD8965a | RB1 | Sub | ess splice |
| PD7307 | PD7307a | RB1 | Sub | ess splice |
| PD18031 | PD18031a | RB1 | Del | ess splice |
| PD13608 | PD13608a | RB1 | CopyNumber | HD |
| PD18020 | PD18020a | RB1 | CopyNumber | HD |
| PD22355 | PD22355a | RB1 | CopyNumber | HD |
| PD24192 | PD24192a | RB1 | CopyNumber | HD |
| PD4952 | PD4952a | RB1 | CopyNumber | HD |
| PD6731 | PD6731a2 | RB1 | Rearrangement | 2\|Inversion\|Inversion |
| PD7426 | PD7426a | RB1 | Rearrangement | 1\|Deletion |
| PD6684 | PD6684a | RB1 | Rearrangement | 1\|Tandem duplication |
| PD18769 | PD18769a | RB1 | Rearrangement | 1\|Deletion |
| PD6415 | PD6415a | RB1 | Rearrangement | 1\|Translocation |
| PD4005 | PD4005a | RB1 | Rearrangement | 1\|Tandem duplication |

(continued)

| Sample | Tumour_name | Gene | Mutation_Type | Effect |
|---|---|---|---|---|
| PD13296 | PD13296a | RB1 | Rearrangement | 1\|Tandem duplication |
| PD22366 | PD22366a | RB1 | Rearrangement | 1\|Tandem duplication |
| PD9585 | PD9585a | RB1 | Rearrangement | 1\|Deletion |
| PD9702 | PD9702a | RB1 | Rearrangement | 1\|Tandem duplication |
| PD7321 | PD7321a | RB1 | Rearrangement | 1\|Deletion |
| PD11326 | PD11326a | RB1 | Rearrangement | 1\|Tandem duplication |
| PD9064 | PD9064a | RB1 | Rearrangement | 1\|Tandem duplication |
| PD3905 | PD3905a | RB1 | Rearrangement | 1\|Tandem duplication |
| PD8621 | PD8621a | RB1 | Rearrangement | 1\|Tandem duplication |
| PD23562 | PD23562a | RB1 | Rearrangement | 1\|Deletion |
| PD5928 | PD5928a | RB1 | Rearrangement | 2\|Translocation\| Translocation |
| PD5945 | PD5945a | RB1 | Rearrangement | 1\|Tandem duplication |
| PD9592 | PD9592a | RB1 | Rearrangement | 1\|Deletion |
| PD5930 | PD5930a | RB1 | Rearrangement | 1\|Translocation |
| PD7250 | PD7250a | RB1 | Rearrangement | 1\|Tandem duplication |
| PD23578 | PD23578a | RB1 | Rearrangement | 1\|Deletion |
| PD13428 | PD13428a | RB1 | Rearrangement | 1\|Deletion |
| PD24304 | PD24304a | RB1 | Rearrangement | 2\|Inversion\|Inversion |
| PD5944 | PD5944a | RB1 | Rearrangement | 1\|Deletion |
| PD23566 | PD23566a | RB1 | Rearrangement | 1\|Tandem duplication |
| PD9578 | PD9578a | RHOA | Sub | missense |
| PD11375 | PD11375a | RHOA | Sub | missense |
| PD13604 | PD13604a | RHOA | Sub | missense |
| PD9575 | PD9575a | RUNX1 | Del | ess splice |
| PD11389 | PD11389a | RUNX1 | Ins | frameshift |
| PD11402 | PD11402a | RUNX1 | Ins | frameshift |
| PD18775 | PD18775a | RUNX1 | Sub | ess splice |
| PD9541 | PD9541a | SETD2 | Sub | nonsense |
| PD24320 | PD24320a | SETD2 | Del | frameshift |
| PD18748 | PD18748a | SETD2 | Sub | nonsense |
| PD4072 | PD4072a | SETD2 | Sub | nonsense |
| PD11357 | PD11357a | SETD2 | Del | frameshift |
| PD9694 | PD9694a | SF3B1 | Sub | missense |
| PD13312 | PD13312a | SF3B1 | Sub | missense |
| PD9847 | PD9847a | SF3B1 | Sub | missense |
| PD13623 | PD13623a | SF3B1 | Sub | missense |
| PD13770 | PD13770a | SF3B1 | Sub | missense |
| PD4076 | PD4076a | SF3B1 | Sub | missense |
| PD18149 | PD18149a | SF3B1 | Sub | missense |

(continued)

| Sample | Tumour_name | Gene | Mutation_Type | Effect |
|---|---|---|---|---|
| PD4965 | PD4965a | SF3B1 | Sub | missense |
| PD3989 | PD3989a | SF3B1 | Sub | missense |
| PD9572 | PD9572a | SF3B1 | Sub | missense |
| PD9599 | PD9599a | SF3B1 | Sub | missense |
| PD6711 | PD6711a2 | SF3B1 | Sub | missense |
| PD11384 | PD11384a | SF3B1 | Sub | missense |
| PD11337 | PD11337a | SMAD4 | Ins | frameshift |
| PD13625 | PD13625a | SMAD4 | Sub | nonsense |
| PD4876 | PD4876a | SMAD4 | CopyNumber | HD |
| PD4605 | PD4605a | SMARCA4 | Sub | nonsense |
| PD9595 | PD9595a | SMARCA4 | Del | ess splice |
| PD23564 | PD23564a | SPEN | Sub | nonsense |
| PD11762 | PD11762a | SPEN | Sub | nonsense |
| PD11365 | PD11365a | SPEN | Ins | frameshift |
| PD11380 | PD11380a | SPEN | Sub | nonsense |
| PD14460 | PD14460a | SPEN | Ins | frameshift |
| PD24320 | PD24320a | SPEN | Ins | frameshift |
| PD4255 | PD4255a | SPEN | Ins | nonsense |
| PD13620 | PD13620a | SPEN | Del | frameshift |
| PD9847 | PD9847a | SPEN | Sub | nonsense |
| PD9063 | PD9063a | SPEN | Sub | nonsense |
| PD13756 | PD13756a | SPEN | Sub | nonsense |
| PD9004 | PD9004a | SPEN | Del | frameshift |
| PD11389 | PD11389a | SPEN | Ins | frameshift |
| PD5950 | PD5950a | STAG2 | Ins | frameshift |
| PD8973 | PD8973a | STAG2 | Sub | nonsense |
| PD11741 | PD11741a | STK11 | Sub | nonsense |
| PD7201 | PD7201a | TBX3 | Sub | nonsense |
| PD18776 | PD18776a | TBX3 | Ins | frameshift |
| PD13427 | PD13427a | TBX3 | Sub | nonsense |
| PD18188 | PD18188a | TBX3 | Ins | frameshift |
| PD9584 | PD9584a | TBX3 | Ins | ess splice |
| PD18049 | PD18049a | TBX3 | Ins | frameshift |
| PD9541 | PD9541a | TBX3 | Sub | nonsense |
| PD11368 | PD11368a | TBX3 | Ins | frameshift |
| PD7210 | PD7210a | TBX3 | Ins | frameshift |
| PD18756 | PD18756a | TBX3 | Ins | frameshift |
| PD5937 | PD5937a | TBX3 | Del | ess splice |
| PD11816 | PD11816a | TBX3 | Ins | frameshift |

(continued)

| Sample | Tumour_name | Gene | Mutation_Type | Effect |
|--------|-------------|------|---------------|--------|
| PD9009 | PD9009a | TBX3 | Del | nonsense |
| PD11766 | PD11766a | TET2 | Sub | missense |
| PD7249 | PD7249a | TP53 | Sub | missense |
| PD3905 | PD3905a | TP53 | Del | frameshift |
| PD8982 | PD8982a | TP53 | Del | frameshift |
| PD11379 | PD11379a | TP53 | Sub | missense |
| PD18022 | PD18022a | TP53 | Sub | missense |
| PD22359 | PD22359a | TP53 | Sub | nonsense |
| PD24206 | PD24206a | TP53 | Del | inframe |
| PD18017 | PD18017a | TP53 | Del | frameshift |
| PD5947 | PD5947a | TP53 | Sub | missense |
| PD24224 | PD24224a | TP53 | Sub | missense |
| PD5948 | PD5948a | TP53 | Ins | frameshift |
| PD5942 | PD5942a | TP53 | Sub | ess splice |
| PD7206 | PD7206a | TP53 | Del | ess splice |
| PD8652 | PD8652a2 | TP53 | Sub | ess splice |
| PD22363 | PD22363a | TP53 | Sub | ess splice |
| PD23578 | PD23578a | TP53 | Sub | ess splice |
| PD4199 | PD4199a | TP53 | Sub | ess splice |
| PD4967 | PD4967a | TP53 | Sub | nonsense |
| PD7069 | PD7069a | TP53 | Sub | nonsense |
| PD4836 | PD4836a | TP53 | Del | frameshift |
| PD9604 | PD9604a | TP53 | Sub | nonsense |
| PD7426 | PD7426a | TP53 | Sub | nonsense |
| PD9760 | PD9760a | TP53 | Sub | nonsense |
| PD24329 | PD24329a | TP53 | Sub | nonsense |
| PD7250 | PD7250a | TP53 | Del | ess splice |
| PD11751 | PD11751a | TP53 | Sub | ess splice |
| PD11398 | PD11398a | TP53 | Ins | frameshift |
| PD24216 | PD24216a | TP53 | Del | frameshift |
| PD9579 | PD9579a | TP53 | Sub | missense |
| PD18251 | PD18251a | TP53 | Sub | missense |
| PD5944 | PD5944a | TP53 | Sub | missense |
| PD6410 | PD6410a | TP53 | Sub | missense |
| PD7067 | PD7067a | TP53 | Del | inframe |
| PD7304 | PD7304a | TP53 | Sub | missense |
| PD13163 | PD13163a | TP53 | Sub | missense |
| PD8609 | PD8609a | TP53 | Sub | missense |
| PD24337 | PD24337a | TP53 | Sub | missense |

(continued)

| Sample | Tumour_name | Gene | Mutation_Type | Effect |
|---|---|---|---|---|
| PD13166 | PD13166a | TP53 | Sub | missense |
| PD8832 | PD8832a | TP53 | Del | complex sub |
| PD4847 | PD4847a | TP53 | Ins | inframe |
| PD18031 | PD18031a | TP53 | Sub | missense |
| PD6727 | PD6727b | TP53 | Sub | missense |
| PD8619 | PD8619a | TP53 | Sub | missense |
| PD5934 | PD5934a | TP53 | Sub | missense |
| PD13603 | PD13603a | TP53 | Sub | missense |
| PD22366 | PD22366a | TP53 | Sub | missense |
| PD24196 | PD24196a | TP53 | Sub | missense |
| PD8979 | PD8979a | TP53 | Sub | missense |
| PD9752 | PD9752a | TP53 | Sub | missense |
| PD18050 | PD18050a | TP53 | Sub | missense |
| PD8621 | PD8621a | TP53 | Sub | missense |
| PD18020 | PD18020a | TP53 | Sub | missense |
| PD22355 | PD22355a | TP53 | Sub | missense |
| PD13418 | PD13418a | TP53 | Sub | missense |
| PD8830 | PD8830a | TP53 | Sub | missense |
| PD11393 | PD11393a | TP53 | Del | inframe |
| PD22357 | PD22357a | TP53 | Sub | ess splice |
| PD5951 | PD5951a | TP53 | Sub | ess splice |
| PD22360 | PD22360a | TP53 | Sub | ess splice |
| PD24201 | PD24201a | TP53 | Del | frameshift |
| PD24326 | PD24326a | TP53 | Sub | missense |
| PD8965 | PD8965a | TP53 | Sub | missense |
| PD13299 | PD13299a | TP53 | Del | inframe |
| PD10014 | PD10014a | TP53 | Sub | missense |
| PD6729 | PD6729a2 | TP53 | Sub | missense |
| PD4962 | PD4962a | TP53 | Sub | missense |
| PD24314 | PD24314a | TP53 | Sub | missense |
| PD14435 | PD14435a | TP53 | Sub | missense |
| PD6413 | PD6413a | TP53 | Sub | missense |
| PD3890 | PD3890a | TP53 | Sub | missense |
| PD13167 | PD13167a | TP53 | Sub | missense |
| PD18049 | PD18049a | TP53 | Sub | missense |
| PD13622 | PD13622a | TP53 | Del | complex sub |
| PD22361 | PD22361a | TP53 | Sub | missense |
| PD4980 | PD4980a | TP53 | Sub | missense |
| PD11742 | PD11742a | TP53 | Sub | missense |

(continued)

| Sample | Tumour_name | Gene | Mutation_Type | Effect |
|--------|-------------|------|---------------|--------|
| PD8969 | PD8969a | TP53 | Sub | missense |
| PD18771 | PD18771a | TP53 | Sub | missense |
| PD23577 | PD23577a | TP53 | Del | frameshift |
| PD11345 | PD11345a | TP53 | Sub | missense |
| PD11341 | PD11341a | TP53 | Sub | missense |
| PD6046 | PD6046a | TP53 | Sub | missense |
| PD14454 | PD14454a | TP53 | Sub | missense |
| PD9568 | PD9568a | TP53 | Sub | nonsense |
| PD4847 | PD4847a | TP53 | Sub | missense |
| PD22364 | PD22364a | TP53 | Sub | missense |
| PD9592 | PD9592a | TP53 | Sub | missense |
| PD11346 | PD11346a | TP53 | Sub | missense |
| PD9599 | PD9599a | TP53 | Del | inframe |
| PD7316 | PD7316a | TP53 | Sub | nonsense |
| PD4976 | PD4976a | TP53 | Sub | missense |
| PD6732 | PD6732b | TP53 | Sub | missense |
| PD18048 | PD18048a | TP53 | Sub | ess splice |
| PD8981 | PD8981a | TP53 | Sub | ess splice |
| PD8964 | PD8964a | TP53 | Sub | ess splice |
| PD11344 | PD11344a | TP53 | Sub | nonsense |
| PD8980 | PD8980a | TP53 | Sub | missense |
| PD13630 | PD13630a | TP53 | Sub | missense |
| PD4109 | PD4109a | TP53 | Sub | missense |
| PD24192 | PD24192a | TP53 | Sub | missense |
| PD24327 | PD24327a | TP53 | Sub | missense |
| PD24308 | PD24308a | TP53 | Sub | missense |
| PD11326 | PD11326a | TP53 | Del | complex sub |
| PD11349 | PD11349a | TP53 | Sub | missense |
| PD24223 | PD24223a | TP53 | Sub | missense |
| PD9595 | PD9595a | TP53 | Sub | missense |
| PD5950 | PD5950a | TP53 | Sub | nonsense |
| PD4005 | PD4005a | TP53 | Sub | nonsense |
| PD6730 | PD6730b | TP53 | Sub | nonsense |
| PD7428 | PD7428a | TP53 | Sub | nonsense |
| PD7066 | PD7066a | TP53 | Sub | nonsense |
| PD23563 | PD23563a | TP53 | Sub | nonsense |
| PD8660 | PD8660a2 | TP53 | Sub | nonsense |
| PD4975 | PD4975a | TP53 | Sub | nonsense |
| PD4874 | PD4874a | TP53 | Sub | nonsense |

(continued)

| Sample | Tumour_name | Gene | Mutation_Type | Effect |
|--------|-------------|------|---------------|--------|
| PD7248 | PD7248a | TP53 | Del | frameshift |
| PD9575 | PD9575a | TP53 | Del | frameshift |
| PD7344 | PD7344a | TP53 | Sub | nonsense |
| PD8997 | PD8997a | TP53 | Sub | missense |
| PD23554 | PD23554a | TP53 | Sub | missense |
| PD9585 | PD9585a | TP53 | Ins | frameshift |
| PD4107 | PD4107a | TP53 | Del | frameshift |
| PD5925 | PD5925a | TP53 | Sub | nonsense |
| PD6047 | PD6047a | TP53 | Sub | nonsense |
| PD13297 | PD13297a | TP53 | Sub | nonsense |
| PD6412 | PD6412a | TP53 | Sub | nonsense |
| PD24208 | PD24208a | TP53 | Sub | nonsense |
| PD7211 | PD7211a | TP53 | Sub | nonsense |
| PD24318 | PD24318a | TP53 | Sub | nonsense |
| PD7217 | PD7217a | TP53 | Sub | missense |
| PD13425 | PD13425a | TP53 | Sub | missense |
| PD11327 | PD11327a | TP53 | Sub | missense |
| PD9696 | PD9696a | TP53 | Sub | missense |
| PD7321 | PD7321a | TP53 | Sub | missense |
| PD6722 | PD6722a | TP53 | Sub | missense |
| PD11743 | PD11743a | TP53 | Sub | nonsense |
| PD4826 | PD4826a | TP53 | Sub | nonsense |
| PD7240 | PD7240a | TP53 | Sub | ess splice |
| PD10011 | PD10011a | TP53 | Sub | ess splice |
| PD24202 | PD24202a | TP53 | Del | frameshift |
| PD4604 | PD4604a | TP53 | Sub | nonsense |
| PD9759 | PD9759a | TP53 | Sub | missense |
| PD4875 | PD4875a | TP53 | Sub | missense |
| PD8978 | PD8978a | TP53 | Sub | missense |
| PD9571 | PD9571a | TP53 | Sub | missense |
| PD4255 | PD4255a | TP53 | Sub | missense |
| PD4968 | PD4968a | TP53 | Sub | missense |
| PD13311 | PD13311a | TP53 | Sub | missense |
| PD24225 | PD24225a | TP53 | Sub | missense |
| PD9009 | PD9009a | TP53 | Sub | missense |
| PD24194 | PD24194a | TP53 | Sub | missense |
| PD6731 | PD6731a2 | TP53 | Sub | missense |
| PD18024 | PD18024a | TP53 | Sub | missense |
| PD6404 | PD6404a | TP53 | Sub | missense |

(continued)

| Sample | Tumour_name | Gene | Mutation_Type | Effect |
|---|---|---|---|---|
| PD9576 | PD9576a | TP53 | Sub | missense |
| PD13609 | PD13609a | TP53 | Sub | missense |
| PD18037 | PD18037a | TP53 | Sub | missense |
| PD23566 | PD23566a | TP53 | Sub | missense |
| PD6728 | PD6728b | TP53 | Sub | missense |
| PD23574 | PD23574a | TP53 | Sub | missense |
| PD11750 | PD11750a | TP53 | Sub | missense |
| PD24182 | PD24182a | TP53 | Sub | missense |
| PD6409 | PD6409a | TP53 | Sub | missense |
| PD24221 | PD24221a | TP53 | Sub | missense |
| PD6405 | PD6405a | TP53 | Sub | missense |
| PD24191 | PD24191a | TP53 | Sub | missense |
| PD9702 | PD9702a | TP53 | Sub | missense |
| PD4605 | PD4605a | TP53 | Sub | missense |
| PD8984 | PD8984a | TP53 | Del | frameshift |
| PD24215 | PD24215a | TP53 | Del | frameshift |
| PD11745 | PD11745a | TP53 | Sub | nonsense |
| PD18046 | PD18046a | TP53 | Sub | nonsense |
| PD6406 | PD6406a | TP53 | Complex | frameshift |
| PD6411 | PD6411a | TP53 | Sub | missense |
| PD9002 | PD9002a | TP53 | Sub | missense |
| PD9539 | PD9539a | TP53 | Sub | missense |
| PD24220 | PD24220a | TP53 | Sub | missense |
| PD14471 | PD14471a | TP53 | Sub | missense |
| PD4841 | PD4841a | TP53 | Sub | missense |
| PD13627 | PD13627a | TP53 | Del | frameshift |
| PD4833 | PD4833a | TP53 | Ins | frameshift |
| PD4971 | PD4971a | TP53 | Sub | missense |
| PD23559 | PD23559a | TP53 | Sub | missense |
| PD5945 | PD5945a | TP53 | Sub | nonsense |
| PD8612 | PD8612a | TP53 | Del | frameshift |
| PD24193 | PD24193a | TP53 | Sub | missense |
| PD13428 | PD13428a | TP53 | Ins | frameshift |
| PD13298 | PD13298a | TP53 | Sub | missense |
| PD4252 | PD4252a | TP53 | Del | frameshift |
| PD8615 | PD8615a | TP53 | Sub | missense |
| PD24219 | PD24219a | TP53 | Sub | missense |
| PD7307 | PD7307a | TP53 | Sub | missense |
| PD7220 | PD7220a | TP53 | Sub | missense |

(continued)

| Sample | Tumour_name | Gene | Mutation_Type | Effect |
|---|---|---|---|---|
| PD24197 | PD24197a | TP53 | Sub | missense |
| PD23560 | PD23560a | TP53 | Sub | ess splice |
| PD13608 | PD13608a | TP53 | Sub | ess splice |
| PD11752 | PD11752a | TP53 | Del | ess splice |
| PD5935 | PD5935a | TP53 | Sub | missense |
| PD9004 | PD9004a | TP53 | Del | frameshift |
| PD8611 | PD8611a | TP53 | Del | frameshift |
| PD4192 | PD4192a | TP53 | Del | frameshift |
| PD4956 | PD4956a | TP53 | Del | frameshift |
| PD4844 | PD4844a | TP53 | Del | frameshift |
| PD9064 | PD9064a | TP53 | Del | inframe |
| PD14437 | PD14437a | TP53 | Sub | nonsense |
| PD9464 | PD9464a | TP53 | Del | frameshift |
| PD6733 | PD6733b | TP53 | Sub | nonsense |
| PD6043 | PD6043a | TP53 | Del | frameshift |
| PD7238 | PD7238a | TP53 | Del | frameshift |
| PD11748 | PD11748a | TP53 | Del | frameshift |
| PD6415 | PD6415a | TP53 | Ins | frameshift |
| PD23565 | PD23565a | TP53 | Sub | nonsense |
| PD6719 | PD6719a | TP53 | Del | frameshift |
| PD11462 | PD11462a | TP53 | Del | frameshift |
| PD24333 | PD24333a | TP53 | Ins | frameshift |
| PD23562 | PD23562a | TP53 | Ins | frameshift |
| PD10010 | PD10010a | TP53 | Sub | nonsense |
| PD14453 | PD14453a | TP53 | Del | frameshift |
| PD23567 | PD23567a | TP53 | Del | frameshift |
| PD4086 | PD4086a | TP53 | Sub | nonsense |
| PD7215 | PD7215a | TP53 | Sub | nonsense |
| PD5932 | PD5932a | TP53 | Del | frameshift |
| PD4606 | PD4606a | TP53 | CopyNumber | HD |
| PD11344 | PD11344a | TP53 | Rearrangement | 1|Translocation |
| PD13771 | PD13771a | TP53 | Rearrangement | 1|Deletion |
| PD4952 | PD4952a | TP53 | Rearrangement | 1|Deletion |
| PD4116 | PD4116a | TP53 | Rearrangement | 1|Deletion |
| PD24190 | PD24190a | TP53 | Rearrangement | 1|Deletion |
| PD11340 | PD11340a | TP53 | Rearrangement | 1|Deletion |
| PD13602 | PD13602a | TP53 | Rearrangement | 1|Tandem duplication |
| PD5961 | PD5961a | TP53 | Rearrangement | 1|Translocation |
| PD24218 | PD24218a | TP53 | Rearrangement | 1|Deletion |

(continued)

| Sample | Tumour_name | Gene | Mutation_Type | Effect |
|---|---|---|---|---|
| PD6414 | PD6414a | TP53 | Rearrangement | 1|Tandem duplication |
| PD4845 | PD4845a | TP53 | Rearrangement | 1|Tandem duplication |
| PD8617 | PD8617a | USP9X | Del | frameshift |
| PD6412 | PD6412a | USP9X | Del | ess splice |
| PD11367 | PD11367a | USP9X | Del | frameshift |
| PD23564 | PD23564a | USP9X | Ins | frameshift |
| PD4972 | PD4972a | XBP1 | Del | frameshift |
| PD4606 | PD4606a | XBP1 | Ins | frameshift |
| PD13604 | PD13604a | XBP1 | Del | frameshift |
| PD11394 | PD11394a | XBP1 | Del | frameshift |
| PD11396 | PD11396a | ZFP36L1 | Ins | frameshift |
| PD13605 | PD13605a | ZFP36L1 | Del | frameshift |
| PD11337 | PD11337a | ZNF217 | CopyNumber | amp |
| PD11345 | PD11345a | ZNF217 | CopyNumber | amp |
| PD11368 | PD11368a | ZNF217 | CopyNumber | amp |
| PD11464 | PD11464a | ZNF217 | CopyNumber | amp |
| PD11742 | PD11742a | ZNF217 | CopyNumber | amp |
| PD11743 | PD11743a | ZNF217 | CopyNumber | amp |
| PD11818 | PD11818a | ZNF217 | CopyNumber | amp |
| PD13165 | PD13165a | ZNF217 | CopyNumber | amp |
| PD13602 | PD13602a | ZNF217 | CopyNumber | amp |
| PD13606 | PD13606a | ZNF217 | CopyNumber | amp |
| PD13608 | PD13608a | ZNF217 | CopyNumber | amp |
| PD13609 | PD13609a | ZNF217 | CopyNumber | amp |
| PD13622 | PD13622a | ZNF217 | CopyNumber | amp |
| PD14435 | PD14435a | ZNF217 | CopyNumber | amp |
| PD14453 | PD14453a | ZNF217 | CopyNumber | amp |
| PD18020 | PD18020a | ZNF217 | CopyNumber | amp |
| PD18045 | PD18045a | ZNF217 | CopyNumber | amp |
| PD18264 | PD18264a | ZNF217 | CopyNumber | amp |
| PD18734 | PD18734a | ZNF217 | CopyNumber | amp |
| PD23550 | PD23550a | ZNF217 | CopyNumber | amp |
| PD24206 | PD24206a | ZNF217 | CopyNumber | amp |
| PD3851 | PD3851a | ZNF217 | CopyNumber | amp |
| PD4103 | PD4103a | ZNF217 | CopyNumber | amp |
| PD4315 | PD4315a | ZNF217 | CopyNumber | amp |
| PD4978 | PD4978a | ZNF217 | CopyNumber | amp |
| PD5964 | PD5964a | ZNF217 | CopyNumber | amp |
| PD6417 | PD6417a | ZNF217 | CopyNumber | amp |

(continued)

| Sample | Tumour_name | Gene | Mutation_Type | Effect |
|---|---|---|---|---|
| PD6727 | PD6727b | ZNF217 | CopyNumber | amp |
| PD7069 | PD7069a | ZNF217 | CopyNumber | amp |
| PD7243 | PD7243a | ZNF217 | CopyNumber | amp |
| PD7305 | PD7305a | ZNF217 | CopyNumber | amp |
| PD8973 | PD8973a | ZNF217 | CopyNumber | amp |
| PD8980 | PD8980a | ZNF217 | CopyNumber | amp |
| PD8981 | PD8981a | ZNF217 | CopyNumber | amp |
| PD9002 | PD9002a | ZNF217 | CopyNumber | amp |
| PD9567 | PD9567a | ZNF217 | CopyNumber | amp |

**Table 8A**

| predicted.inframe.gene.fusion | count |
|---|---|
| ACCN1_RASL10B | 2 |
| ADAMTS3_GUCY1B3 | 2 |
| ARHGAP21_GPR158 | 3 |
| ASXL2_SOX5 | 2 |
| BCAS3_BRIP1 | 2 |
| C10orf11_IQCH | 2 |
| CAMK4_DENND1A | 2 |
| CDH13_ENSG00000223871 | 2 |
| CLTC_BCAS3 | 2 |
| CTNNA3_RSF1 | 2 |
| ERC1_ETV6 | 2 |
| FADS1_SHANK2 | 2 |
| GALNT12_MEGF9 | 2 |
| GFM1_RARRES1 | 2 |
| GMDS_TMEM132B | 2 |
| GOLPH3L_HORMAD1 | 2 |
| GPHN_RAD51L1 | 2 |
| GRIK2_SOBP | 2 |
| IKZF3_SYDE1 | 2 |
| JARID2_RNGTT | 2 |
| LMBRD1_REPS1 | 2 |
| MAP7_AKAP12 | 2 |
| MDGA2_NCOA6 | 2 |
| NLGN1_NAALADL2 | 2 |
| NR3C2_ENSG00000157837 | 2 |
| PARD3B_PIP5K1B | 2 |
| PHF3_EYS | 2 |

(continued)

| predicted.inframe.gene.fusion | count |
|---|---|
| RNF220_MAST2 | 2 |
| ROBO2_PTPRB | 2 |
| SHANK2_ACER3 | 2 |
| SHANK2_FCHSD2 | 2 |
| SHANK2_ODZ4 | 3 |
| STAMBPL1_PANK1 | 2 |
| STK31_ACCN1 | 2 |
| SULF2_ZNF217 | 2 |
| TMEM195_POU6F2 | 2 |
| VAT1L_WWOX | 2 |
| WNK1_ERC1 | 2 |
| WWOX_TGIF2 | 2 |

**Table 8B**

| gene | count | type |
|---|---|---|
| ACCN1 | 14 | acceptor |
| SHANK2 | 14 | acceptor |
| EYS | 9 | acceptor |
| BCAS3 | 7 | acceptor |
| CA10 | 7 | acceptor |
| FBXL20 | 6 | acceptor |
| CSMD1 | 5 | acceptor |
| ENSG00000250898 | 5 | acceptor |
| KCNMA1 | 4 | acceptor |
| CCDC46 | 4 | acceptor |
| DLG2 | 4 | acceptor |
| NF1 | 4 | acceptor |
| SKAP1 | 4 | acceptor |
| SSH2 | 4 | acceptor |
| TANC2 | 4 | acceptor |
| CTNNA3 | 3 | acceptor |
| ERBB4 | 3 | acceptor |
| MAML2 | 3 | acceptor |
| RARRES1 | 3 | acceptor |
| GMDS | 3 | acceptor |
| IKZF3 | 3 | acceptor |
| RNGTT | 3 | acceptor |
| TRDN | 3 | acceptor |
| AGBL4 | 3 | acceptor |

(continued)

| gene | count | type |
|------|-------|------|
| ASCC3 | 3 | acceptor |
| CALN1 | 3 | acceptor |
| CSMD3 | 3 | acceptor |
| DCLK1 | 3 | acceptor |
| DNAJC1 | 3 | acceptor |
| ENSG00000228451 | 3 | acceptor |
| ENSG00000234857 | 3 | acceptor |
| ENSG00000249350 | 3 | acceptor |
| FRM D4A | 3 | acceptor |
| GDPD1 | 3 | acceptor |
| KCNIP4 | 3 | acceptor |
| KHDRBS2 | 3 | acceptor |
| LUC7L | 3 | acceptor |
| MACROD1 | 3 | acceptor |
| MACROD2 | 3 | acceptor |
| MYO1D | 3 | acceptor |
| RGS7 | 3 | acceptor |
| RORA | 3 | acceptor |
| SLC39A11 | 3 | acceptor |
| TTC28 | 3 | acceptor |
| WWOX | 3 | acceptor |
| ZMAT4 | 3 | acceptor |
| MDGA2 | 2 | acceptor |
| ADAMTS3 | 2 | acceptor |
| ARHGAP21 | 2 | acceptor |
| ASXL2 | 2 | acceptor |
| BCAS1 | 2 | acceptor |
| FAM189A1 | 2 | acceptor |
| KPNA3 | 2 | acceptor |
| LMBRD1 | 2 | acceptor |
| NR3C2 | 2 | acceptor |
| PCLO | 2 | acceptor |
| PTPRB | 2 | acceptor |
| SDK2 | 2 | acceptor |
| SULF2 | 2 | acceptor |
| ABCA6 | 2 | acceptor |
| ABCC4 | 2 | acceptor |
| ACER3 | 2 | acceptor |
| ACTN1 | 2 | acceptor |

(continued)

| gene | count | type |
|---|---|---|
| ANK1 | 2 | acceptor |
| ANKS1B | 2 | acceptor |
| AP2B1 | 2 | acceptor |
| ASH1L | 2 | acceptor |
| ATRX | 2 | acceptor |
| BNC2 | 2 | acceptor |
| BRIP1 | 2 | acceptor |
| C10orf11 | 2 | acceptor |
| C18orf45 | 2 | acceptor |
| CACNA1A | 2 | acceptor |
| CACNB1 | 2 | acceptor |
| CADPS2 | 2 | acceptor |
| CAPZB | 2 | acceptor |
| CDH4 | 2 | acceptor |
| CPNE8 | 2 | acceptor |
| CPVL | 2 | acceptor |
| CTNND2 | 2 | acceptor |
| DGKB | 2 | acceptor |
| DIP2C | 2 | acceptor |
| DLG1 | 2 | acceptor |
| DLGAP5 | 2 | acceptor |
| DMD | 2 | acceptor |
| DNER | 2 | acceptor |
| DOK5 | 2 | acceptor |
| DST | 2 | acceptor |
| ENSG00000160172 | 2 | acceptor |
| ENSG00000204987 | 2 | acceptor |
| ENSG00000223871 | 2 | acceptor |
| ENSG00000226623 | 2 | acceptor |
| EPRS | 2 | acceptor |
| ERN1 | 2 | acceptor |
| EXOC6B | 2 | acceptor |
| FADS1 | 2 | acceptor |
| FAM107B | 2 | acceptor |
| FBXO47 | 2 | acceptor |
| FUT10 | 2 | acceptor |
| GAOL1 | 2 | acceptor |
| GOLPH3L | 2 | acceptor |
| GPATCH2 | 2 | acceptor |

(continued)

| gene | count | type |
|------|-------|------|
| GRID1 | 2 | acceptor |
| HACE1 | 2 | acceptor |
| HUNK | 2 | acceptor |
| IARS | 2 | acceptor |
| ISPD | 2 | acceptor |
| JAZF1 | 2 | acceptor |
| KCNH1 | 2 | acceptor |
| KCTD2 | 2 | acceptor |
| KIF6 | 2 | acceptor |
| LRP12 | 2 | acceptor |
| MAP7 | 2 | acceptor |
| MARVELD2 | 2 | acceptor |
| MCM3 | 2 | acceptor |
| MSI2 | 2 | acceptor |
| MYO18B | 2 | acceptor |
| NAALADL2 | 2 | acceptor |
| NARS2 | 2 | acceptor |
| NBEA | 2 | acceptor |
| NCKAP5 | 2 | acceptor |
| NFIX | 2 | acceptor |
| NRG1 | 2 | acceptor |
| PAK1 | 2 | acceptor |
| PALM2-AKAP2 | 2 | acceptor |
| PARD3 | 2 | acceptor |
| PARK2 | 2 | acceptor |
| PDE4DIP | 2 | acceptor |
| PDZRN4 | 2 | acceptor |
| PHLPP1 | 2 | acceptor |
| PITPNC1 | 2 | acceptor |
| PKHD1 | 2 | acceptor |
| PRKDC | 2 | acceptor |
| PROM1 | 2 | acceptor |
| PSMA1 | 2 | acceptor |
| PXDNL | 2 | acceptor |
| RAB28 | 2 | acceptor |
| RAD51L1 | 2 | acceptor |
| RGS22 | 2 | acceptor |
| RNLS | 2 | acceptor |
| RSF1 | 2 | acceptor |

(continued)

| gene | count | type |
|------|-------|------|
| SAE1 | 2 | acceptor |
| SAMD12 | 2 | acceptor |
| SKA2 | 2 | acceptor |
| SLC7A7 | 2 | acceptor |
| SPIRE1 | 2 | acceptor |
| SPOCK1 | 2 | acceptor |
| SPTBN2 | 2 | acceptor |
| TDRD5 | 2 | acceptor |
| TECPR2 | 2 | acceptor |
| TNPO2 | 2 | acceptor |
| TNR | 2 | acceptor |
| TPCN2 | 2 | acceptor |
| TRIM37 | 2 | acceptor |
| TTLL6 | 2 | acceptor |
| ULK4 | 2 | acceptor |
| USH2A | 2 | acceptor |
| USP32 | 2 | acceptor |
| UST | 2 | acceptor |
| UVRAG | 2 | acceptor |
| WDR45L | 2 | acceptor |
| ZMYND8 | 2 | acceptor |
| ZSWIM5 | 2 | acceptor |
| BCAS3 | 8 | donor |
| RABGAP1L | 8 | donor |
| TANC2 | 6 | donor |
| NF1 | 5 | donor |
| ACER3 | 5 | donor |
| ENSG00000234857 | 5 | donor |
| MACROD2 | 5 | donor |
| NKAIN2 | 5 | donor |
| SHANK2 | 5 | donor |
| UNC5D | 5 | donor |
| NRG1 | 4 | donor |
| VPS13B | 4 | donor |
| ADAM9 | 4 | donor |
| CLTC | 4 | donor |
| PITPNC1 | 4 | donor |
| PSMD11 | 4 | donor |
| TSHZ2 | 4 | donor |

(continued)

| gene | count | type |
|---|---|---|
| TTC28 | 4 | donor |
| C10orf11 | 3 | donor |
| ODZ4 | 3 | donor |
| FAT3 | 3 | donor |
| IKZF3 | 3 | donor |
| PARD3B | 3 | donor |
| ROBO2 | 3 | donor |
| AP2B1 | 3 | donor |
| AUTS2 | 3 | donor |
| CCDC46 | 3 | donor |
| CDH4 | 3 | donor |
| CNTN5 | 3 | donor |
| COL19A1 | 3 | donor |
| DLG2 | 3 | donor |
| ENSG00000249384 | 3 | donor |
| FER1L6 | 3 | donor |
| KCNU1 | 3 | donor |
| LAMA2 | 3 | donor |
| LPP | 3 | donor |
| NME7 | 3 | donor |
| PHACTR1 | 3 | donor |
| PRKCA | 3 | donor |
| TACC1 | 3 | donor |
| TAOK1 | 3 | donor |
| THSD4 | 3 | donor |
| POU6F2 | 2 | donor |
| GPR158 | 2 | donor |
| AKAP12 | 2 | donor |
| CDKAL1 | 2 | donor |
| HDAC9 | 2 | donor |
| IDO2 | 2 | donor |
| JARID2 | 2 | donor |
| ADAM18 | 2 | donor |
| ADAM32 | 2 | donor |
| ADCY2 | 2 | donor |
| ADIPOR1 | 2 | donor |
| AKAP1 | 2 | donor |
| ALDH5A1 | 2 | donor |
| ARHGEF17 | 2 | donor |

(continued)

| gene | count | type |
|------|-------|------|
| BAALC | 2 | donor |
| BPTF | 2 | donor |
| C1orf125 | 2 | donor |
| C21orf29 | 2 | donor |
| C6orf142 | 2 | donor |
| CACNA1A | 2 | donor |
| CADM2 | 2 | donor |
| CCNY | 2 | donor |
| CDH13 | 2 | donor |
| CDK12 | 2 | donor |
| COL4A2 | 2 | donor |
| CPD | 2 | donor |
| CTNNA3 | 2 | donor |
| CTNNBL1 | 2 | donor |
| DHX32 | 2 | donor |
| DIAPH3 | 2 | donor |
| DNAJC24 | 2 | donor |
| ENSG00000172421 | 2 | donor |
| ENSG00000189337 | 2 | donor |
| ENSG00000232495 | 2 | donor |
| ERBB2 | 2 | donor |
| ETV6 | 2 | donor |
| FANCD2 | 2 | donor |
| FRY | 2 | donor |
| GAB2 | 2 | donor |
| GALNTL6 | 2 | donor |
| GPHN | 2 | donor |
| GPI | 2 | donor |
| GRM7 | 2 | donor |
| HLCS | 2 | donor |
| HORMAD1 | 2 | donor |
| IGF2BP1 | 2 | donor |
| INADL | 2 | donor |
| KALRN | 2 | donor |
| KCNK2 | 2 | donor |
| KCNMA1 | 2 | donor |
| KCNQ5 | 2 | donor |
| KDM2A | 2 | donor |
| KHDRBS2 | 2 | donor |

(continued)

| gene | count | type |
|------|-------|------|
| KLHDC10 | 2 | donor |
| KMO | 2 | donor |
| LARGE | 2 | donor |
| MACF1 | 2 | donor |
| MAN2B2 | 2 | donor |
| MCF2L | 2 | donor |
| MDGA2 | 2 | donor |
| MGAM | 2 | donor |
| MMP28 | 2 | donor |
| MSI2 | 2 | donor |
| MTA3 | 2 | donor |
| MTBP | 2 | donor |
| NARS2 | 2 | donor |
| NFIA | 2 | donor |
| NLGN1 | 2 | donor |
| NOS1AP | 2 | donor |
| NPAS3 | 2 | donor |
| NUDT13 | 2 | donor |
| PARD3 | 2 | donor |
| PCDHGA12 | 2 | donor |
| PDE3B | 2 | donor |
| PDSS2 | 2 | donor |
| PHF3 | 2 | donor |
| PLA2G4A | 2 | donor |
| PLCB4 | 2 | donor |
| PLEKHM2 | 2 | donor |
| PPFIA1 | 2 | donor |
| PPM1D | 2 | donor |
| PTK2 | 2 | donor |
| PTPRG | 2 | donor |
| PTPRQ | 2 | donor |
| QKI | 2 | donor |
| RALGAPA2 | 2 | donor |
| RASL10B | 2 | donor |
| RNF135 | 2 | donor |
| ROS1 | 2 | donor |
| RYR3 | 2 | donor |
| SAPS3 | 2 | donor |
| SEC24A | 2 | donor |

(continued)

| gene | count | type |
|------|-------|------|
| SETBP1 | 2 | donor |
| SKA2 | 2 | donor |
| SLC35F3 | 2 | donor |
| SLC4A4 | 2 | donor |
| SP2 | 2 | donor |
| SPOCK3 | 2 | donor |
| STARD3 | 2 | donor |
| SYN2 | 2 | donor |
| SYT1 | 2 | donor |
| TAF1 | 2 | donor |
| TCF7L1 | 2 | donor |
| TMEM49 | 2 | donor |
| TOX2 | 2 | donor |
| TRAPPC9 | 2 | donor |
| TRIO | 2 | donor |
| UHRF1BP1 | 2 | donor |
| VAT1L | 2 | donor |
| WAPAL | 2 | donor |
| ZNF208 | 2 | donor |
| ZNF410 | 2 | donor |

**Table 8C Table 9**

| length or palindromic sequence | chr | pos | location | palindromic seq 5 prime | core motif | palindromic seq 3 prime | reference base | number of times mutated | reference strand |
|---|---|---|---|---|---|---|---|---|---|
| 4+5 | 18 | 42769999 | intergenic | ACCTGGGTCC | TGAACA | GGACACAGGT | G | 5 | 1 |
| 4+5 | 18 | 42770002 | intergenic | ACCTGGGTCC | TGAACA | GGACACAGGT | C | 4 | 1 |
| 7 | 11 | 10331381 | intron ADM | CCTTGGC | TGAACA | GCCAAGG | G | 2 | 1 |
| 7 | 11 | 10331384 | intron ADM | CCTTGGC | TGAACA | GCCAAGG | C | 2 | 1 |
| 7 | 7 | 17658895 | intergenic | TCCTCTT | TGAACA | AAGAGGA | G | 4 | -1 |
| 9 | 6 | 142706206 | intron GPR126 | CTCTTTGTA | TGAACA | TACAAAGAG | G | 10 | 1 |
| 9 | 6 | 142706209 | intron GPR126 | CTCTTTGTA | TGAACA | TACAAAGAG | C | 5 | 1 |
| 9 | 10 | 115511590 | promoter PLEKHS1 | TTTTGCAAT | TGAACA | ATTGCAAAA | G | 11 | 1 |
| 9 | 10 | 115511593 | promoter PLEKHS1 | TTTTGCAAT | TGAACA | ATTGCAAAA | C | 9 | 1 |
| 9 | 11 | 74510805 | intron RNF169 | TTCTGCTTA | TGAACA | TAAGCAGAA | G | 3 | 1 |
| 10 | 10 | 82156892 | intergenic | TCAGATTCCC | TGAACA | GGGAATCTGA | C | 4 | 1 |
| 10 | 7 | 11273340 | intergenic | TTATAAACTG | TGAACA | CAGTTTATAA | G | 4 | -1 |
| 10 | 7 | 11273343 | intergenic | TTATAAACTG | TGAACA | CAGTTTATAA | C | 7 | -1 |
| 10 | 15 | 96590693 | intergenic | ACAATTGATG | TGAACA | CATCAATTGT | G | 2 | -1 |
| 10 | 15 | 96590696 | intergenic | ACAATTGATG | TGAACA | CATCAATTGT | C | 2 | -1 |
| 11 | 3 | 82807069 | intergenic | TAAAATTGTAA | TGAACA | TTACAATTTTA | G | 2 | -1 |

(continued)

| length or palindromic sequence | chr | pos | location | palindromic seq 5 prime | core motif | palindromic seq 3 prime | reference base | number of times mutated | reference strand |
|---|---|---|---|---|---|---|---|---|---|
| | | | | | | | | | |

**Highly mutable non-coding sites at inverted repeats.** Table of ten loci of inverted repeats demonstrating recurrent mutagenesis within the TGAACA/TGTTCA core motif in this cohort of 560 breast cancers. All inverted repeats placed in identical orientation (TGAACA) for ease of reading. True orientation on the reference genome is provided in the last column (reference strand: 1 = plus strand, -1 = minus strand).

Mutated nucleotide in core motif is highlighted red. Palindromic sequence on chromosome 18 has a 1 bp mismatch within the palindrome. This is underscored in the sequence and reported in the length of palindromic sequence as 4+5.

**Table 10A**

| Substitution | Sequence Context | Signature 1A | Signature 2 | Signature 3 | Signature 5 | Signature 6 | Signature 8 | Signature 13 | Signature 17 | Signature 18 | Signature 20 | Signature 26 | Signature 30 |
|---|---|---|---|---|---|---|---|---|---|---|---|---|---|
| C>A | ACA | 1.1098% | 0.0683% | 2.2172% | 1.4942% | 0.1700% | 3.6718% | 0.0335% | 0.1832% | 5.0536% | 0.1180% | 0.2040% | 0.0000% |
| C>A | ACC | 0.9149% | 0.0619% | 1.7872% | 0.8961% | 0.2800% | 3.3246% | 0.0649% | 0.0342% | 1.0940% | 0.2212% | 0.1487% | 0.0000% |
| C>A | ACG | 0.1490% | 0.0099% | 0.2138% | 0.2208% | 0.0500% | 0.2525% | 0.0038% | 0.0002% | 0.2288% | 0.0000% | 0.0284% | 0.1967% |
| C>A | ACT | 0.6234% | 0.0324% | 1.6265% | 0.9207% | 0.1900% | 3.3599% | 0.0847% | 0.3180% | 1.9424% | 0.3008% | 0.0598% | 0.0000% |
| C>A | CCA | 0.6596% | 0.0677% | 1.8782% | 0.9675% | 1.0100% | 3.1724% | 0.1710% | 0.1032% | 8.8768% | 1.7377% | 0.3706% | 0.0000% |
| C>A | CCC | 0.7342% | 0.0214% | 1.5760% | 0.4952% | 2.4100% | 2.5505% | 0.1159% | 0.0422% | 2.0641% | 3.6502% | 0.3981% | 0.0000% |
| C>A | CCG | 0.0893% | 0.0007% | 0.1963% | 0.2801% | 0.9100% | 0.1160% | 0.0244% | 0.0297% | 1.7178% | 1.2483% | 0.0812% | 0.2262% |
| C>A | CCT | 0.7187% | 0.0416% | 1.4723% | 1.1013% | 5.7100% | 2.8791% | 0.1257% | 0.0031% | 3.7677% | 10.3401% | 1.9038% | 0.0000% |
| C>A | GCA | 0.8233% | 0.0352% | 0.9697% | 1.1892% | 0.2400% | 2.3682% | 0.0132% | 0.6535% | 12.8724% | 0.1116% | 0.1375% | 0.8853% |
| C>A | GCC | 0.5758% | 0.0134% | 1.0843% | 0.9248% | 0.5800% | 1.5822% | 0.0754% | 0.1294% | 1.6093% | 0.3271% | 0.1962% | 0.9345% |
| C>A | GCG | 0.0616% | 0.0178% | 0.0929% | 0.2809% | 0.2100% | 0.0851% | 0.0097% | 0.1284% | 0.9271% | 0.3752% | 0.0013% | 0.0885% |
| C>A | GCT | 0.4459% | 0.0123% | 1.2215% | 1.0301% | 0.8700% | 2.1061% | 0.0551% | 0.3104% | 7.2017% | 1.2937% | 0.1935% | 0.8165% |
| C>A | TCA | 1.2250% | 1.5127% | 1.1653% | 1.4774% | 0.1700% | 2.7032% | 4.8166% | 0.1825% | 6.7081% | 0.0688% | 0.2680% | 0.0000% |
| C>A | TCC | 1.1162% | 0.6532% | 1.6607% | 1.2043% | 0.2900% | 1.8090% | 1.7330% | 0.1287% | 4.1869% | 0.4058% | 0.2032% | 0.0000% |
| C>A | TCG | 0.2275% | 0.1656% | 0.1357% | 0.3902% | 0.1100% | 0.1695% | 0.2293% | 0.2173% | 1.5162% | 0.1001% | 0.0265% | 0.1672% |
| C>A | TCT | 1.5259% | 1.2395% | 1.6328% | 1.8243% | 0.5800% | 3.8141% | 1.8635% | 0.5012% | 11.7878% | 1.2229% | 0.3017% | 0.0000% |
| C>G | ACA | 0.1801% | 0.0263% | 2.4003% | 1.1671% | 0.1300% | 0.8357% | 0.3775% | 0.1662% | 0.1517% | 0.0697% | 0.1273% | 0.0000% |
| C>G | ACC | 0.2581% | 0.0270% | 1.2160% | 0.7292% | 0.1200% | 0.4306% | 0.0921% | 0.1627% | 0.2499% | 0.2059% | 0.1528% | 0.0000% |
| C>G | ACG | 0.0593% | 0.0219% | 0.5275% | 0.2304% | 0.0000% | 0.0584% | 0.0020% | 0.0026% | 0.2615% | 0.0013% | 0.0307% | 0.4820% |
| C>G | ACT | 0.2964% | 0.0611% | 2.3278% | 1.1696% | 0.1800% | 0.8635% | 0.3861% | 0.1329% | 0.3983% | 0.0849% | 0.2498% | 0.0000% |
| C>G | CCA | 0.1285% | 0.0028% | 1.6833% | 0.7538% | 0.0000% | 0.6619% | 0.5334% | 0.1925% | 0.0458% | 0.0013% | 0.1279% | 0.0000% |
| C>G | CCC | 0.0702% | 0.0280% | 1.3531% | 0.7633% | 0.0000% | 0.6078% | 0.0912% | 0.0709% | 0.3654% | 0.0548% | 0.1215% | 0.0000% |
| C>G | CCG | 0.0506% | 0.0019% | 0.4176% | 0.2614% | 0.0000% | 0.0656% | 0.0285% | 0.0004% | 0.5406% | 0.0000% | 0.0208% | 0.3246% |
| C>G | CCT | 0.1382% | 0.0313% | 2.4046% | 0.9417% | 0.0200% | 0.7805% | 0.6383% | 0.0954% | 0.5520% | 0.0121% | 0.2297% | 0.0000% |

| Substitution | Sequence Context | Signature 1A | Signature 2 | Signature 3 | Signature 5 | Signature 6 | Signature 8 | Signature 13 | Signature 17 | Signature 18 | Signature 20 | Signature 26 | Signature 30 |
|---|---|---|---|---|---|---|---|---|---|---|---|---|---|
| C>G | GCA | 0.0602% | 0.0045% | 1.1917% | 0.5559% | 0.0000% | 0.3725% | 0.1349% | 0.1296% | 0.0924% | 0.1340% | 0.1321% | 0.7378% |
| C>G | GCC | 0.2393% | 0.0015% | 0.9824% | 0.5389% | 0.3000% | 0.3047% | 0.0789% | 0.0261% | 0.0649% | 0.1811% | 0.1846% | 0.6591% |
| C>G | GCG | 0.0000% | 0.0041% | 0.1671% | 0.1101% | 0.0000% | 0.0321% | 0.0082% | 0.0004% | 0.0543% | 0.0001% | 0.0205% | 0.1574% |
| C>G | GCT | 0.0890% | 0.0268% | 1.7914% | 0.6041% | 0.1700% | 0.5776% | 0.2680% | 0.1820% | 0.1377% | 0.1238% | 0.1226% | 0.0000% |
| C>G | TCA | 0.1875% | 3.7242% | 1.6041% | 0.2681% | 0.0000% | 0.4118% | 28.0235% | 0.0393% | 0.1803% | 0.0024% | 0.4202% | 0.0000% |
| C>G | TCC | 0.2067% | 0.0019% | 2.0150% | 0.7924% | 0.0200% | 0.3800% | 6.3885% | 0.0656% | 0.7863% | 0.0152% | 0.2808% | 0.0000% |
| C>G | TCG | 0.0305% | 0.1625% | 0.2528% | 0.1319% | 0.0000% | 0.0026% | 0.9528% | 0.0014% | 0.1513% | 0.0000% | 0.0000% | 0.1967% |
| C>G | TCT | 0.3152% | 6.6880% | 3.2674% | 0.6645% | 0.0100% | 0.6248% | 33.0225% | 0.2852% | 0.3567% | 0.0683% | 0.8019% | 0.0000% |
| C>T | ACA | 2.9515% | 0.7442% | 1.7872% | 2.1839% | 3.1200% | 1.8067% | 0.1480% | 0.9350% | 0.3826% | 3.2858% | 0.5907% | 6.5119% |
| C>T | ACC | 1.4323% | 0.2726% | 0.8896% | 1.2756% | 1.6300% | 0.5650% | 0.0041% | 0.4224% | 0.2465% | 2.2196% | 1.0626% | 5.4397% |
| C>T | ACG | 17.1647% | 0.3322% | 0.3573% | 1.6760% | 9.0800% | 1.9265% | 0.0317% | 0.9229% | 0.8464% | 3.4572% | 1.9930% | 2.0460% |
| C>T | ACT | 1.2624% | 0.3327% | 1.4798% | 1.6478% | 1.4900% | 2.0806% | 0.0000% | 0.5736% | 0.5606% | 1.8820% | 1.1335% | 2.1936% |
| C>T | CCA | 2.0896% | 1.5020% | 1.4395% | 2.2769% | 0.8500% | 0.4866% | 0.3647% | 2.1265% | 0.9768% | 1.1145% | 0.6594% | 6.9447% |
| C>T | CCC | 1.8502% | 0.3517% | 0.8545% | 1.7509% | 0.9900% | 0.3980% | 0.1004% | 0.3847% | 0.5655% | 1.8153% | 0.6511% | 6.3840% |
| C>T | CCG | 9.5577% | 0.4979% | 0.3518% | 1.2862% | 9.0100% | 0.8339% | 0.0129% | 1.3176% | 1.6183% | 4.4385% | 1.1905% | 1.7313% |
| C>T | CCT | 1.7113% | 0.8957% | 1.6076% | 2.0430% | 0.8700% | 1.8844% | 0.0612% | 0.9648% | 0.5153% | 1.3847% | 0.6239% | 3.4232% |
| C>T | GCA | 2.4944% | 0.6391% | 1.6127% | 2.0038% | 6.5300% | 0.2261% | 0.2934% | 0.2069% | 0.2703% | 5.6794% | 0.9607% | 4.8593% |
| C>T | GCC | 2.7161% | 0.1996% | 0.8209% | 1.8022% | 7.7300% | 0.1617% | 0.2515% | 0.0325% | 0.4199% | 5.4430% | 1.9507% | 4.9479% |
| C>T | GCG | 10.3571% | 0.0303% | 0.1213% | 1.3194% | 13.3900% | 1.1607% | 0.3205% | 0.7511% | 1.1897% | 0.4593% | 2.2503% | 1.5739% |
| C>T | GCT | 1.7690% | 0.3266% | 1.0612% | 1.9503% | 5.2400% | 0.6285% | 0.1688% | 0.0926% | 0.1913% | 4.9581% | 1.7307% | 1.8887% |
| C>T | TCA | 1.4492% | 41.9941% | 0.8880% | 1.0998% | 0.7400% | 0.7181% | 11.3842% | 1.6101% | 1.4923% | 1.0710% | 1.1303% | 8.4989% |
| C>T | TCC | 1.7681% | 8.1972% | 1.3530% | 2.0645% | 0.6700% | 0.4061% | 1.5025% | 1.6780% | 0.9963% | 0.8176% | 1.0808% | 9.0301% |
| C>T | TCG | 7.6002% | 4.7720% | 0.1705% | 0.7534% | 3.9100% | 0.5535% | 0.6102% | 1.6272% | 1.1550% | 2.3164% | 1.0364% | 1.5149% |
| C>T | TCT | 1.3762% | 22.8675% | 1.0304% | 1.1787% | 0.4700% | 1.2209% | 2.8141% | 1.9827% | 0.5398% | 0.9540% | 0.7249% | 4.5544% |

266

EP 3 452 937 B1

| Substi tution | Sequ ence Cont ext | Signature 1A | Signature 2 | Signature 3 | Signature 5 | Signature 6 | Signature 8 | Signature 13 | Signature 17 | Signature 18 | Signature 20 | Signature 26 | Signature 30 |
|---|---|---|---|---|---|---|---|---|---|---|---|---|---|
| T>A | ATA | 0.4022% | 0.0000% | 0.8429% | 0.8903% | 0.0600% | 1.3365% | 0.1244% | 0.0613% | 0.3037% | 0.0574% | 0.4459% | 0.7574% |
| T>A | ATC | 0.2371% | 0.0113% | 0.7373% | 0.7399% | 0.3300% | 1.2431% | 0.1060% | 0.1879% | 0.0811% | 0.1971% | 1.2822% | 0.3738% |
| T>A | ATG | 0.2811% | 0.0533% | 0.7357% | 1.1508% | 0.0000% | 1.4037% | 0.0103% | 0.1087% | 0.4503% | 0.0191% | 0.1172% | 0.6591% |
| T>A | ATT | 0.8361% | 0.0149% | 0.8754% | 1.1194% | 0.5300% | 2.4110% | 0.0972% | 0.2045% | 0.2737% | 0.7421% | 0.3993% | 0.9345% |
| T>A | CTA | 0.1183% | 0.0155% | 0.7571% | 0.5043% | 0.0100% | 1.2024% | 0.0285% | 0.0030% | 0.0412% | 0.0045% | 0.3561% | 0.5312% |
| T>A | CTC | 0.1903% | 0.0464% | 1.2725% | 0.6209% | 0.2600% | 1.7881% | 0.0222% | 1.0224% | 0.2491% | 0.4389% | 0.3902% | 0.6787% |
| T>A | CTG | 0.1488% | 0.0230% | 1.1509% | 1.0508% | 0.0800% | 1.6357% | 0.0287% | 0.4467% | 0.5523% | 0.1128% | 0.2390% | 0.8263% |
| T>A | CTT | 0.2179% | 0.0575% | 1.6456% | 0.9785% | 0.1100% | 2.6248% | 0.0001% | 3.1245% | 0.3314% | 0.2386% | 0.1636% | 0.0000% |
| T>A | GTA | 0.0689% | 0.0115% | 0.4435% | 0.6740% | 0.0000% | 0.8139% | 0.0138% | 0.0009% | 0.1875% | 0.0163% | 0.2243% | 0.3738% |
| T>A | GTC | 0.0552% | 0.0294% | 0.5615% | 0.4022% | 0.2800% | 0.7910% | 0.0253% | 0.2573% | 0.1510% | 0.3799% | 0.5207% | 0.3345% |
| T>A | GTG | 0.1200% | 0.0089% | 0.8070% | 0.6814% | 0.0600% | 0.9057% | 0.0154% | 0.3539% | 0.1505% | 0.0828% | 0.1358% | 0.5017% |
| T>A | GTT | 0.2107% | 0.0216% | 0.8679% | 0.5101% | 0.2100% | 1.7812% | 0.0123% | 0.3879% | 0.3611% | 0.1901% | 0.2513% | 0.6394% |
| T>A | TTA | 0.5600% | 0.0081% | 0.7133% | 0.9206% | 0.0200% | 1.5140% | 0.0403% | 0.0699% | 0.4663% | 0.0925% | 0.0628% | 0.7673% |
| T>A | TTC | 0.1999% | 0.0005% | 0.9103% | 0.6835% | 0.0800% | 1.2094% | 0.0772% | 0.2579% | 0.2578% | 0.0708% | 0.5074% | 0.6492% |
| T>A | TTG | 0.1090% | 0.0067% | 0.6566% | 0.7144% | 0.0000% | 0.8363% | 0.0245% | 0.0021% | 0.2017% | 0.0097% | 0.0020% | 0.3640% |
| T>A | TTT | 0.3981% | 0.0276% | 1.4712% | 1.0241% | 0.0700% | 2.7653% | 0.0160% | 0.3883% | 0.2819% | 0.2313% | 0.1236% | 0.0000% |
| T>C | ATA | 1.3916% | 0.1304% | 1.3036% | 3.5367% | 0.7500% | 1.6207% | 0.0549% | 0.2697% | 0.2933% | 1.6395% | 5.5029% | 0.0000% |
| T>C | ATC | 0.6275% | 0.0426% | 0.9186% | 1.3771% | 0.5600% | 0.7788% | 0.0146% | 0.8078% | 0.1513% | 1.9252% | 2.7595% | 0.8755% |
| T>C | ATG | 1.0138% | 0.0575% | 1.1717% | 2.8449% | 2.1700% | 0.9853% | 0.0279% | 0.4433% | 0.3330% | 5.4785% | 5.1791% | 0.9050% |
| T>C | ATT | 0.9256% | 0.1488% | 1.6979% | 2.7303% | 0.2300% | 2.1829% | 0.0267% | 0.3140% | 0.5491% | 0.7341% | 3.9072% | 0.0000% |
| T>C | CTA | 0.4177% | 0.0547% | 0.7895% | 1.4239% | 0.6200% | 0.5174% | 0.0043% | 0.7427% | 0.0192% | 1.4868% | 3.7889% | 0.0000% |
| T>C | CTC | 0.5253% | 0.0392% | 1.4431% | 1.2490% | 0.4000% | 1.0937% | 0.0043% | 3.9657% | 0.3050% | 1.8356% | 2.1741% | 0.0000% |
| T>C | CTG | 0.7013% | 0.0362% | 0.8423% | 1.8742% | 2.7000% | 0.5658% | 0.0054% | 2.8610% | 0.2596% | 7.4151% | 4.7240% | 0.0000% |
| T>C | CTT | 0.6714% | 0.0561% | 1.1932% | 1.9054% | 0.3300% | 1.1120% | 0.0001% | 10.1870% | 0.1310% | 1.1001% | 2.0741% | 0.0000% |

EP 3 452 937 B1

(continued)

| Substitution | Sequence Context | Signature 1A | Signature 2 | Signature 3 | Signature 5 | Signature 6 | Signature 8 | Signature 13 | Signature 17 | Signature 18 | Signature 20 | Signature 26 | Signature 30 |
|---|---|---|---|---|---|---|---|---|---|---|---|---|---|
| T>C | GTA | 1.1248% | 0.0005% | 0.6850% | 1.6295% | 1.2200% | 0.5721% | 0.1054% | 0.0011% | 0.0529% | 0.2469% | 9.8053% | 0.0000% |
| T>C | GTC | 0.7000% | 0.0186% | 0.6261% | 0.9575% | 0.5900% | 0.5181% | 0.0530% | 0.9658% | 0.2279% | 0.1566% | 4.0226% | 0.6591% |
| T>C | GTG | 0.4978% | 0.0000% | 0.6099% | 1.4104% | 1.1500% | 0.5041% | 0.0630% | 0.4654% | 0.5871% | 2.7342% | 4.4621% | 0.7869% |
| T>C | GTT | 1.0667% | 0.0579% | 0.7509% | 1.5667% | 0.4200% | 0.8488% | 0.0676% | 0.8149% | 0.4772% | 0.1498% | 5.5528% | 0.8460% |
| T>C | TTA | 0.8074% | 0.0102% | 0.9115% | 1.7060% | 0.5900% | 0.4901% | 0.0784% | 0.1709% | 0.0302% | 0.5734% | 2.8790% | 0.0000% |
| T>C | TTC | 0.4857% | 0.0470% | 1.0954% | 1.4196% | 0.3500% | 0.6081% | 0.0133% | 1.1074% | 0.6261% | 1.1234% | 3.6639% | 0.9148% |
| T>C | TTG | 0.8325% | 0.0192% | 0.6113% | 1.2597% | 1.0600% | 0.1712% | 0.0037% | 0.5932% | 0.1541% | 2.3768% | 1.9144% | 0.6000% |
| T>C | TTT | 0.6257% | 0.0585% | 1.0774% | 1.7375% | 0.2900% | 1.0003% | 0.0180% | 0.7208% | 0.1358% | 0.5395% | 2.3072% | 0.0000% |
| T>G | ATA | 0.1588% | 0.0034% | 0.2351% | 0.3462% | 0.0000% | 0.4220% | 0.0386% | 0.0053% | 0.0386% | 0.0002% | 0.0081% | 0.5312% |
| T>G | ATC | 0.1784% | 0.0025% | 0.1464% | 0.2247% | 0.1700% | 0.0794% | 0.0112% | 0.6469% | 0.1255% | 0.0381% | 0.0163% | 0.2459% |
| T>G | ATG | 0.1386% | 0.0273% | 0.9054% | 0.5490% | 0.0700% | 0.6866% | 0.0036% | 0.3482% | 0.2369% | 0.0659% | 0.1255% | 0.6296% |
| T>G | ATT | 0.3159% | 0.0218% | 0.7031% | 0.3821% | 0.2900% | 0.2940% | 0.0192% | 3.0926% | 0.0339% | 0.0532% | 0.1850% | 0.9542% |
| T>G | CTA | 0.0303% | 0.0114% | 0.1974% | 0.2042% | 0.0100% | 0.2424% | 0.0015% | 0.0091% | 0.0061% | 0.0023% | 0.0095% | 0.4623% |
| T>G | CTC | 0.2099% | 0.0022% | 0.5824% | 0.3479% | 0.4000% | 0.2096% | 0.0148% | 1.9814% | 0.2016% | 0.8476% | 0.3919% | 0.6000% |
| T>G | CTG | 0.1600% | 0.0228% | 1.0465% | 0.7147% | 0.5000% | 0.6604% | 0.0046% | 1.3450% | 0.5312% | 1.5384% | 0.5497% | 0.7378% |
| T>G | CTT | 0.2759% | 0.0067% | 0.8724% | 1.1487% | 0.8600% | 0.4867% | 0.0464% | 26.1457% | 0.2342% | 0.2185% | 0.6789% | 0.9148% |
| T>G | GTA | 0.0099% | 0.0096% | 0.4144% | 0.1628% | 0.0000% | 0.0975% | 0.0018% | 0.0039% | 0.1342% | 0.0004% | 0.0037% | 0.3246% |
| T>G | GTC | 0.0202% | 0.0047% | 0.4502% | 0.0328% | 0.1600% | 0.0525% | 0.0093% | 0.9078% | 0.0001% | 0.2831% | 0.2461% | 0.1869% |
| T>G | GTG | 0.1188% | 0.0110% | 1.6391% | 0.5949% | 0.1000% | 0.6088% | 0.0011% | 0.4783% | 0.9695% | 0.2733% | 0.0817% | 0.3345% |
| T>G | GTT | 0.0801% | 0.0086% | 0.7067% | 0.3307% | 0.3500% | 0.5427% | 0.0054% | 6.3498% | 0.2891% | 0.3559% | 0.7834% | 0.5607% |
| T>G | TTA | 0.1398% | 0.0072% | 0.5427% | 0.5203% | 0.0900% | 0.1743% | 0.0547% | 0.0133% | 0.0036% | 0.0000% | 0.0009% | 0.8656% |
| T>G | TTC | 0.1292% | 0.0014% | 0.6160% | 0.5132% | 0.1900% | 0.2550% | 0.0235% | 0.9613% | 0.3234% | 0.0377% | 0.2719% | 0.4328% |
| T>G | TTG | 0.2031% | 0.0207% | 1.1077% | 0.6055% | 0.1100% | 0.6030% | 0.0000% | 0.4522% | 0.0755% | 0.0515% | 0.1369% | 0.8263% |
| T>G | TTT | 0.4030% | 0.0024% | 1.3001% | 1.3370% | 0.7200% | 0.7224% | 0.0671% | 5.8040% | 0.2126% | 0.0616% | 0.2568% | 0.0000% |

268

**Table 10B**

| Sample Name | Sign ature 1A | Sign ature 2 | Sign ature 3 | Sign ature 5 | Sign ature 6 | Sign ature 8 | Sign ature 13 | Sign ature 17 | Sign ature 18 | Sign ature 20 | Sign ature 26 | Sign ature 30 | Accuracy | Cluster ID |
|---|---|---|---|---|---|---|---|---|---|---|---|---|---|---|
| PD10010 | 544 | 102 | 530 | 820 | 0 | 273 | 161 | 0 | 0 | 0 | 0 | 0 | 0.94 | 4 |
| PD10011 | 814 | 0 | 1482 | 3734 | 0 | 905 | 434 | 903 | 0 | 0 | 0 | 0 | 0.75 | 4 |
| PD10014 | 399 | 0 | 3467 | 3410 | 0 | 4324 | 525 | 0 | 0 | 0 | 0 | 0 | 0.96 | 6 |
| PD11326 | 389 | 250 | 3446 | 1547 | 0 | 3105 | 891 | 0 | 0 | 0 | 0 | 0 | 0.97 | 6 |
| PD11327 | 265 | 0 | 5400 | 938 | 0 | 5244 | 868 | 0 | 0 | 0 | 0 | 0 | 0.96 | 6 |
| PD11336 | 1335 | 633 | 1542 | 1158 | 0 | 0 | 0 | 422 | 495 | 0 | 0 | 0 | 0.95 | 4 |
| PD11337 | 747 | 265 | 0 | 768 | 0 | 792 | 299 | 0 | 202 | 0 | 0 | 0 | 0.96 | 3 |
| PD11338 | 754 | 60 | 0 | 1144 | 0 | 342 | 0 | 0 | 0 | 0 | 0 | 0 | 0.95 | 3 |
| PD11339 | 1114 | 68 | 0 | 1277 | 0 | 0 | 70 | 0 | 0 | 0 | 0 | 0 | 0.97 | 3 |
| PD11340 | 979 | 520 | 0 | 2211 | 0 | 694 | 267 | 0 | 393 | 0 | 0 | 0 | 0.93 | 3 |
| PD11341 | 518 | 266 | 0 | 385 | 0 | 391 | 92 | 0 | 0 | 0 | 0 | 0 | 0.97 | 3 |
| PD11342 | 701 | 853 | 0 | 1422 | 0 | 0 | 329 | 0 | 0 | 0 | 0 | 0 | 0.99 | 3 |
| PD11343 | 793 | 157 | 0 | 1094 | 0 | 757 | 143 | 0 | 0 | 0 | 0 | 0 | 0.96 | 3 |
| PD11344 | 452 | 4740 | 0 | 0 | 0 | 0 | 5836 | 0 | 0 | 0 | 0 | 0 | 1.00 | 11 |
| PD11345 | 794 | 4121 | 0 | 1522 | 0 | 996 | 2258 | 0 | 660 | 0 | 0 | 0 | 1.00 | 8 |
| PD11346 | 610 | 474 | 0 | 957 | 0 | 0 | 251 | 0 | 0 | 0 | 0 | 0 | 0.97 | 3 |
| PD11347 | 517 | 386 | 0 | 994 | 0 | 0 | 134 | 0 | 0 | 0 | 0 | 0 | 0.98 | 3 |
| PD11348 | 1744 | 176 | 0 | 1487 | 0 | 997 | 172 | 0 | 434 | 0 | 0 | 0 | 0.97 | 3 |
| PD11349 | 1069 | 1106 | 0 | 2741 | 0 | 1594 | 939 | 0 | 0 | 0 | 0 | 0 | 0.98 | 3 |
| PD11352 | 835 | 473 | 0 | 1278 | 0 | 0 | 0 | 0 | 0 | 0 | 0 | 0 | 0.98 | 3 |
| PD11355 | 430 | 66 | 0 | 679 | 0 | 382 | 0 | 0 | 281 | 0 | 0 | 0 | 0.96 | 3 |
| PD11357 | 1256 | 94 | 0 | 1530 | 0 | 499 | 0 | 0 | 0 | 0 | 0 | 0 | 0.96 | 3 |
| PD11358 | 929 | 1630 | 0 | 1768 | 0 | 0 | 0 | 0 | 0 | 0 | 0 | 0 | 0.99 | 3 |
| PD11359 | 797 | 158 | 0 | 1155 | 0 | 474 | 82 | 0 | 0 | 0 | 0 | 0 | 0.97 | 3 |
| PD11360 | 1055 | 146 | 0 | 1284 | 0 | 631 | 0 | 0 | 0 | 0 | 0 | 0 | 0.96 | 3 |
| PD11361 | 766 | 113 | 0 | 467 | 0 | 377 | 0 | 0 | 0 | 0 | 0 | 0 | 0.97 | 3 |
| PD11364 | 738 | 85 | 0 | 1012 | 0 | 384 | 120 | 0 | 0 | 0 | 0 | 0 | 0.96 | 3 |
| PD11365 | 0 | 0 | 0 | 1131 | 0 | 0 | 0 | 0 | 0 | 12521 | 0 | 0 | 0.86 | 10 |
| PD11366 | 823 | 228 | 0 | 1763 | 0 | 0 | 0 | 0 | 191 | 0 | 0 | 0 | 0.94 | 3 |
| PD11367 | 1136 | 167 | 2518 | 2135 | 0 | 1972 | 236 | 0 | 0 | 0 | 0 | 0 | 0.96 | 6 |
| PD11368 | 1416 | 1798 | 0 | 1740 | 0 | 1395 | 479 | 0 | 0 | 0 | 0 | 0 | 0.99 | 3 |
| PD11369 | 1868 | 887 | 0 | 1651 | 0 | 1182 | 546 | 0 | 0 | 0 | 0 | 0 | 0.98 | 3 |
| PD11370 | 302 | 0 | 0 | 828 | 0 | 122 | 27 | 0 | 0 | 0 | 0 | 0 | 0.94 | 3 |
| PD11372 | 2011 | 0 | 0 | 0 | 0 | 0 | 11782 | 0 | 0 | 0 | 0 | 0 | 0.89 | 11 |

| | | | | | | | | | | | | | | |
|---|---|---|---|---|---|---|---|---|---|---|---|---|---|---|
| PD11374 | 720 | 72 | 0 | 1212 | 0 | 288 | 91 | 0 | 0 | 0 | 0 | 0 | 0.94 | 3 |
| PD11375 | 811 | 68 | 0 | 565 | 0 | 490 | 0 | 0 | 0 | 0 | 0 | 0 | 0.94 | 3 |
| PD11376 | 1124 | 196 | 0 | 1528 | 0 | 0 | 0 | 0 | 304 | 0 | 0 | 0 | 0.95 | 3 |
| PD11379 | 0 | 6710 | 0 | 1660 | 0 | 2118 | 8894 | 0 | 0 | 0 | 0 | 0 | 1.00 | 11 |
| PD11380 | 837 | 289 | 0 | 1594 | 0 | 0 | 0 | 0 | 0 | 0 | 0 | 0 | 0.95 | 3 |
| PD11381 | 456 | 129 | 0 | 1512 | 0 | 0 | 0 | 0 | 0 | 0 | 0 | 0 | 0.94 | 3 |
| PD11383 | 605 | 64 | 0 | 1277 | 0 | 572 | 61 | 0 | 0 | 0 | 0 | 0 | 0.94 | 3 |
| PD11384 | 526 | 159 | 0 | 930 | 0 | 0 | 110 | 0 | 0 | 0 | 0 | 0 | 0.96 | 3 |
| PD11385 | 548 | 97 | 0 | 913 | 0 | 329 | 0 | 0 | 0 | 0 | 0 | 0 | 0.94 | 3 |
| PD11386 | 947 | 653 | 0 | 1604 | 0 | 0 | 178 | 0 | 0 | 0 | 0 | 0 | 0.97 | 3 |
| PD11388 | 1495 | 267 | 0 | 1623 | 0 | 1339 | 265 | 0 | 0 | 0 | 0 | 0 | 0.95 | 3 |
| PD11389 | 402 | 30 | 0 | 1000 | 0 | 245 | 0 | 0 | 0 | 0 | 0 | 0 | 0.93 | 3 |
| PD11391 | 219 | 41 | 0 | 865 | 0 | 149 | 0 | 0 | 0 | 0 | 0 | 0 | 0.92 | 3 |
| PD11393 | 1536 | 114 | 0 | 987 | 0 | 1001 | 124 | 0 | 907 | 0 | 0 | 0 | 0.97 | 3 |
| PD11394 | 1641 | 84 | 0 | 0 | 0 | 484 | 0 | 0 | 0 | 0 | 0 | 0 | 0.98 | 9 |
| PD11395 | 963 | 176 | 0 | 1398 | 0 | 757 | 0 | 264 | 234 | 0 | 0 | 0 | 0.95 | 3 |
| PD11396 | 648 | 101 | 0 | 1088 | 0 | 543 | 55 | 0 | 0 | 0 | 0 | 0 | 0.96 | 3 |
| PD11397 | 1269 | 305 | 0 | 1409 | 0 | 985 | 316 | 0 | 543 | 0 | 0 | 0 | 0.98 | 3 |

| | | | | | | | | | | | | | | |
|---|---|---|---|---|---|---|---|---|---|---|---|---|---|---|
| PD11398 | 748 | 56 | 0 | 767 | 0 | 684 | 92 | 0 | 0 | 0 | 0 | 0 | 0.94 | 3 |
| PD11399 | 1313 | 311 | 0 | 1439 | 0 | 0 | 95 | 0 | 338 | 0 | 0 | 0 | 0.97 | 3 |
| PD11402 | 836 | 166 | 0 | 1634 | 0 | 0 | 0 | 0 | 0 | 0 | 0 | 0 | 0.96 | 3 |
| PD11462 | 980 | 99 | 0 | 1006 | 0 | 635 | 119 | 0 | 0 | 0 | 0 | 0 | 0.95 | 3 |
| PD11464 | 882 | 1432 | 0 | 0 | 0 | 0 | 2183 | 0 | 0 | 0 | 0 | 0 | 0.99 | 11 |
| PD11465 | 2163 | 5334 | 0 | 3605 | 0 | 3327 | 8122 | 0 | 0 | 0 | 0 | 0 | 1.00 | 11 |
| PD11740 | 670 | 88 | 0 | 1166 | 0 | 410 | 0 | 0 | 0 | 0 | 0 | 0 | 0.95 | 3 |
| PD11741 | 583 | 142 | 0 | 0 | 0 | 807 | 140 | 0 | 304 | 0 | 0 | 0 | 0.94 | 2 |
| PD11742 | 475 | 478 | 2159 | 1287 | 0 | 1650 | 956 | 0 | 0 | 0 | 0 | 0 | 0.98 | 6 |
| PD11743 | 698 | 147 | 928 | 1057 | 0 | 479 | 0 | 0 | 0 | 0 | 0 | 0 | 0.94 | 4 |
| PD11744 | 545 | 0 | 0 | 1017 | 0 | 320 | 0 | 0 | 0 | 0 | 0 | 0 | 0.95 | 3 |
| PD11745 | 518 | 257 | 0 | 828 | 0 | 953 | 205 | 0 | 257 | 0 | 0 | 0 | 0.94 | 3 |
| PD11748 | 912 | 0 | 5805 | 1494 | 0 | 2934 | 616 | 0 | 0 | 0 | 0 | 0 | 0.96 | 6 |
| PD11750 | 416 | 0 | 5650 | 962 | 0 | 0 | 3050 | 0 | 0 | 0 | 0 | 0 | 0.98 | 6 |
| PD11751 | 1121 | 0 | 9079 | 2886 | 0 | 1178 | 2203 | 181 | 0 | 0 | 0 | 0 | 0.96 | 6 |
| PD11752 | 2511 | 0 | 0 | 2784 | 0 | 1329 | 260 | 0 | 752 | 0 | 0 | 0 | 0.97 | 3 |
| PD11753 | 1154 | 355 | 2182 | 2733 | 0 | 0 | 0 | 434 | 0 | 0 | 0 | 0 | 0.94 | 4 |
| PD11755 | 292 | 0 | 1292 | 743 | 0 | 265 | 184 | 0 | 0 | 0 | 0 | 0 | 0.93 | 6 |

| | | | | | | | | | | | | | | |
|---|---|---|---|---|---|---|---|---|---|---|---|---|---|---|
| PD11756 | 619 | 254 | 0 | 627 | 0 | 271 | 0 | 0 | 0 | 0 | 0 | 0 | 0.97 | 3 |
| PD11757 | 151 | 0 | 1001 | 0 | 0 | 283 | 517 | 0 | 0 | 0 | 0 | 0 | 0.98 | 6 |
| PD11760 | 1523 | 151 | 0 | 1340 | 0 | 0 | 0 | 0 | 1077 | 0 | 0 | 0 | 0.97 | 3 |
| PD11761 | 356 | 282 | 0 | 1585 | 0 | 414 | 70 | 0 | 0 | 0 | 0 | 0 | 0.91 | 3 |
| PD11762 | 423 | 186 | 0 | 784 | 0 | 300 | 41 | 0 | 0 | 0 | 0 | 0 | 0.96 | 3 |
| PD11765 | 375 | 95 | 0 | 1219 | 0 | 243 | 70 | 0 | 0 | 0 | 0 | 0 | 0.92 | 3 |
| PD11766 | 318 | 136 | 0 | 914 | 0 | 185 | 142 | 0 | 0 | 0 | 0 | 0 | 0.94 | 3 |
| PD11767 | 210 | 18 | 0 | 439 | 0 | 202 | 0 | 0 | 0 | 0 | 0 | 0 | 0.85 | 3 |
| PD11769 | 673 | 109 | 0 | 650 | 0 | 244 | 46 | 0 | 0 | 0 | 0 | 0 | 0.96 | 3 |
| PD11816 | 535 | 535 | 0 | 1639 | 0 | 605 | 697 | 0 | 0 | 0 | 0 | 0 | 0.98 | 3 |
| PD11818 | 427 | 49 | 0 | 848 | 0 | 220 | 61 | 0 | 0 | 0 | 0 | 0 | 0.94 | 3 |
| PD11819 | 475 | 0 | 0 | 903 | 0 | 264 | 44 | 0 | 0 | 0 | 0 | 0 | 0.92 | 3 |
| PD13162 | 1160 | 820 | 0 | 0 | 0 | 0 | 3424 | 0 | 0 | 0 | 0 | 0 | 0.99 | 11 |
| PD13163 | 900 | 776 | 0 | 1995 | 0 | 863 | 819 | 0 | 0 | 0 | 0 | 0 | 0.98 | 3 |
| PD13164 | 673 | 158 | 0 | 1130 | 0 | 436 | 154 | 0 | 171 | 0 | 0 | 0 | 0.94 | 3 |
| PD13165 | 1162 | 1560 | 0 | 0 | 0 | 1905 | 656 | 0 | 1058 | 0 | 0 | 0 | 0.98 | 2 |
| PD13166 | 591 | 186 | 0 | 1005 | 0 | 0 | 318 | 0 | 231 | 0 | 0 | 0 | 0.96 | 3 |
| PD13167 | 857 | 148 | 587 | 656 | 0 | 0 | 157 | 0 | 0 | 0 | 0 | 0 | 0.97 | 4 |

| | | | | | | | | | | | | | | |
|---|---|---|---|---|---|---|---|---|---|---|---|---|---|---|
| PD13168 | 930 | 435 | 0 | 788 | 0 | 0 | 135 | 0 | 240 | 0 | 0 | 0 | 0.99 | 3 |
| PD13296 | 1384 | 0 | 9417 | 1752 | 0 | 3315 | 2205 | 509 | 0 | 0 | 0 | 0 | 0.97 | 6 |
| PD13297 | 886 | 0 | 5916 | 0 | 0 | 0 | 725 | 0 | 0 | 0 | 0 | 7455 | 0.97 | Singleton 1 |
| PD13298 | 1416 | 244 | 1815 | 2394 | 0 | 1290 | 725 | 0 | 483 | 0 | 0 | 0 | 0.96 | 4 |
| PD13299 | 425 | 1034 | 3460 | 1087 | 0 | 740 | 3989 | 0 | 0 | 0 | 0 | 0 | 1.00 | Singleton 2 |
| PD13302 | 1404 | 317 | 0 | 2102 | 0 | 1073 | 0 | 0 | 0 | 0 | 0 | 0 | 0.96 | 3 |
| PD13304 | 701 | 55 | 0 | 429 | 0 | 332 | 0 | 0 | 0 | 0 | 0 | 0 | 0.95 | 3 |
| PD13306 | 557 | 371 | 0 | 765 | 0 | 333 | 0 | 0 | 0 | 0 | 0 | 0 | 0.97 | 3 |
| PD13307 | 1426 | 2726 | 0 | 0 | 0 | 0 | 1201 | 0 | 0 | 0 | 0 | 0 | 0.99 | 8 |
| PD13310 | 718 | 61 | 0 | 826 | 0 | 226 | 0 | 0 | 0 | 0 | 0 | 0 | 0.96 | 3 |
| PD13311 | 256 | 0 | 2257 | 917 | 0 | 857 | 223 | 0 | 0 | 0 | 0 | 0 | 0.91 | 6 |
| PD13312 | 475 | 37 | 0 | 1256 | 0 | 217 | 49 | 0 | 0 | 0 | 0 | 0 | 0.94 | 3 |
| PD13416 | 611 | 81 | 0 | 930 | 0 | 317 | 0 | 0 | 0 | 0 | 0 | 0 | 0.95 | 3 |
| PD13418 | 2040 | 1715 | 0 | 0 | 0 | 0 | 3053 | 0 | 0 | 0 | 0 | 0 | 0.99 | 11 |
| PD13419 | 327 | 85 | 0 | 571 | 0 | 109 | 0 | 0 | 0 | 0 | 0 | 0 | 0.92 | 3 |
| PD13420 | 590 | 37 | 0 | 954 | 0 | 235 | 46 | 0 | 0 | 0 | 0 | 0 | 0.95 | 3 |
| PD13422 | 586 | 109 | 0 | 642 | 0 | 297 | 0 | 0 | 171 | 0 | 0 | 0 | 0.96 | 3 |
| PD13424 | 1394 | 165 | 0 | 945 | 0 | 1223 | 227 | 0 | 0 | 0 | 0 | 0 | 0.97 | 3 |

| PD13425 | 1238 | 5637 | 3946 | 2767 | 0 | 1455 | 17864 | 0 | 1595 | 0 | 0 | 0 | 1.00 | 11 |
| PD13426 | 875 | 191 | 0 | 1633 | 0 | 0 | 0 | 0 | 0 | 0 | 0 | 0 | 0.95 | 3 |
| PD13427 | 324 | 0 | 0 | 610 | 0 | 170 | 30 | 0 | 0 | 0 | 0 | 0 | 0.94 | 3 |
| PD13428 | 785 | 146 | 0 | 2369 | 0 | 0 | 122 | 0 | 0 | 0 | 0 | 0 | 0.94 | 3 |
| PD13602 | 1204 | 156 | 1958 | 1814 | 0 | 0 | 246 | 0 | 536 | 0 | 0 | 0 | 0.94 | 4 |
| PD13603 | 799 | 310 | 0 | 1292 | 0 | 0 | 347 | 0 | 316 | 0 | 0 | 0 | 0.96 | 3 |
| PD13604 | 0 | 42713 | 0 | 0 | 0 | 2316 | 44752 | 0 | 3321 | 0 | 0 | 0 | 1.00 | 8 |
| PD13605 | 311 | 19 | 0 | 533 | 0 | 309 | 66 | 0 | 0 | 0 | 0 | 0 | 0.92 | 3 |
| PD13606 | 1557 | 268 | 0 | 1681 | 0 | 1540 | 201 | 0 | 646 | 0 | 0 | 0 | 0.95 | 3 |
| PD13607 | 864 | 266 | 0 | 1177 | 0 | 684 | 297 | 0 | 0 | 0 | 0 | 0 | 0.97 | 3 |
| PD13608 | 1339 | 1096 | 0 | 0 | 0 | 0 | 1672 | 0 | 0 | 0 | 0 | 0 | 0.98 | 11 |
| PD13609 | 0 | 1813 | 0 | 0 | 0 | 0 | 2893 | 0 | 0 | 0 | 0 | 0 | 0.99 | 11 |
| PD13618 | 503 | 242 | 0 | 1016 | 0 | 0 | 72 | 0 | 0 | 0 | 0 | 0 | 0.96 | 3 |
| PD13619 | 684 | 102 | 0 | 1326 | 0 | 0 | 0 | 0 | 0 | 0 | 0 | 0 | 0.94 | 3 |
| PD13620 | 1455 | 166 | 0 | 1488 | 0 | 1269 | 220 | 0 | 0 | 0 | 0 | 0 | 0.94 | 3 |
| PD13622 | 1315 | 113 | 0 | 1087 | 0 | 1129 | 0 | 0 | 711 | 0 | 0 | 0 | 0.96 | 3 |
| PD13623 | 997 | 2303 | 0 | 0 | 0 | 0 | 500 | 0 | 0 | 0 | 0 | 0 | 0.99 | 8 |
| PD13625 | 1785 | 517 | 0 | 1404 | 0 | 4407 | 379 | 2747 | 0 | 0 | 0 | 0 | 0.96 | Singleton 3 |

| PD13626 | 361 | 25 | 0 | 494 | 0 | 156 | 40 | 0 | 0 | 0 | 0 | 0 | 0.91 | 3 |
| PD13627 | 581 | 0 | 5802 | 1378 | 0 | 0 | 681 | 0 | 0 | 0 | 0 | 0 | 0.93 | 6 |
| PD13629 | 633 | 441 | 0 | 955 | 0 | 0 | 0 | 0 | 0 | 0 | 0 | 0 | 0.97 | 3 |
| PD13630 | 724 | 664 | 0 | 1024 | 0 | 898 | 797 | 0 | 0 | 0 | 0 | 0 | 0.99 | 3 |
| PD13631 | 479 | 189 | 0 | 1246 | 0 | 391 | 105 | 0 | 193 | 0 | 0 | 0 | 0.94 | 3 |
| PD13752 | 608 | 152 | 0 | 1310 | 0 | 469 | 0 | 119 | 151 | 0 | 0 | 0 | 0.93 | 3 |
| PD13753 | 0 | 3150 | 0 | 0 | 0 | 0 | 4845 | 0 | 0 | 0 | 0 | 0 | 0.99 | 11 |
| PD13754 | 650 | 0 | 0 | 1361 | 0 | 382 | 113 | 0 | 0 | 0 | 0 | 0 | 0.91 | 3 |
| PD13755 | 550 | 90 | 0 | 1296 | 0 | 0 | 0 | 0 | 0 | 0 | 0 | 0 | 0.93 | 3 |
| PD13756 | 502 | 41 | 0 | 1099 | 0 | 398 | 43 | 0 | 0 | 0 | 0 | 0 | 0.95 | 3 |
| PD13757 | 408 | 404 | 0 | 484 | 0 | 0 | 191 | 0 | 0 | 0 | 0 | 0 | 0.98 | 3 |
| PD13758 | 428 | 86 | 0 | 1603 | 0 | 182 | 0 | 0 | 0 | 0 | 0 | 0 | 0.91 | 3 |
| PD13760 | 494 | 187 | 1179 | 788 | 0 | 1357 | 629 | 0 | 0 | 0 | 0 | 0 | 0.98 | 6 |
| PD13761 | 299 | 89 | 0 | 1097 | 0 | 203 | 0 | 0 | 0 | 0 | 0 | 0 | 0.91 | 3 |
| PD13762 | 486 | 62 | 0 | 816 | 0 | 289 | 0 | 0 | 0 | 0 | 0 | 0 | 0.93 | 3 |
| PD13763 | 872 | 165 | 0 | 855 | 0 | 671 | 0 | 0 | 0 | 0 | 0 | 0 | 0.97 | 3 |
| PD13764 | 775 | 279 | 0 | 1896 | 0 | 1409 | 471 | 0 | 0 | 0 | 0 | 0 | 0.95 | 3 |
| PD13765 | 1303 | 234 | 0 | 1616 | 0 | 0 | 127 | 0 | 2199 | 0 | 0 | 0 | 0.97 | Singleton 4 |

| PD13766 | 636 | 2215 | 0 | 0 | 0 | 0 | 986 | 0 | 0 | 0 | 0 | 0 | 1.00 | 8 |
| PD13767 | 655 | 341 | 0 | 1368 | 0 | 0 | 61 | 0 | 213 | 0 | 0 | 0 | 0.97 | 3 |
| PD13768 | 512 | 45 | 0 | 795 | 0 | 0 | 0 | 0 | 0 | 0 | 0 | 0 | 0.95 | 3 |
| PD13770 | 608 | 136 | 0 | 708 | 0 | 380 | 0 | 0 | 247 | 0 | 0 | 0 | 0.96 | 3 |
| PD13771 | 478 | 723 | 5582 | 2468 | 0 | 2570 | 1268 | 0 | 0 | 0 | 0 | 0 | 0.96 | 6 |
| PD14432 | 469 | 91 | 0 | 1072 | 0 | 238 | 0 | 0 | 0 | 0 | 0 | 0 | 0.92 | 3 |
| PD14433 | 1227 | 1906 | 0 | 0 | 0 | 0 | 449 | 0 | 0 | 0 | 0 | 0 | 0.99 | 8 |
| PD14435 | 1154 | 245 | 0 | 1373 | 0 | 696 | 248 | 0 | 0 | 0 | 0 | 0 | 0.98 | 3 |
| PD14437 | 953 | 163 | 0 | 1540 | 0 | 1353 | 354 | 0 | 0 | 0 | 0 | 0 | 0.93 | 3 |
| PD14439 | 443 | 86 | 0 | 637 | 0 | 0 | 0 | 0 | 150 | 0 | 0 | 0 | 0.92 | 3 |
| PD14441 | 466 | 146 | 1227 | 1292 | 0 | 1316 | 215 | 0 | 0 | 0 | 0 | 0 | 0.95 | 6 |
| PD14442 | 410 | 70 | 0 | 878 | 0 | 388 | 0 | 0 | 0 | 0 | 0 | 0 | 0.93 | 3 |
| PD14450 | 278 | 50 | 0 | 783 | 0 | 152 | 35 | 0 | 0 | 0 | 0 | 0 | 0.91 | 3 |
| PD14453 | 1030 | 2619 | 0 | 3249 | 0 | 0 | 3018 | 0 | 0 | 0 | 0 | 0 | 0.99 | 7 |
| PD14454 | 588 | 146 | 0 | 843 | 0 | 734 | 199 | 0 | 0 | 0 | 0 | 0 | 0.96 | 3 |
| PD14456 | 797 | 0 | 0 | 768 | 0 | 267 | 0 | 0 | 145 | 0 | 0 | 0 | 0.95 | 3 |
| PD14457 | 1091 | 394 | 0 | 2129 | 0 | 0 | 0 | 0 | 953 | 0 | 0 | 0 | 0.96 | 3 |
| PD14458 | 564 | 70 | 0 | 635 | 0 | 244 | 0 | 0 | 0 | 0 | 0 | 0 | 0.95 | 3 |

| PD14459 | 673 | 137 | 0 | 844 | 0 | 0 | 0 | 0 | 432 | 0 | 0 | 0 | 0.97 | 3 |
| PD14460 | 1112 | 2334 | 0 | 0 | 0 | 0 | 1078 | 0 | 0 | 0 | 0 | 0 | 1.00 | 8 |
| PD14461 | 490 | 0 | 0 | 728 | 0 | 178 | 40 | 0 | 0 | 0 | 0 | 0 | 0.95 | 3 |
| PD14462 | 600 | 404 | 0 | 1371 | 0 | 0 | 253 | 0 | 0 | 0 | 0 | 0 | 0.96 | 3 |
| PD14465 | 1006 | 131 | 0 | 1088 | 0 | 749 | 123 | 0 | 0 | 0 | 0 | 0 | 0.97 | 3 |
| PD14467 | 656 | 96 | 0 | 560 | 0 | 230 | 82 | 0 | 0 | 0 | 0 | 0 | 0.96 | 3 |
| PD14468 | 565 | 74 | 0 | 725 | 0 | 185 | 0 | 0 | 0 | 0 | 0 | 0 | 0.96 | 3 |
| PD14471 | 1388 | 99 | 0 | 805 | 0 | 866 | 112 | 0 | 0 | 0 | 0 | 0 | 0.97 | 3 |
| PD14472 | 563 | 204 | 0 | 1092 | 0 | 321 | 118 | 0 | 0 | 0 | 0 | 0 | 0.97 | 3 |
| PD14473 | 628 | 60 | 0 | 542 | 0 | 266 | 47 | 0 | 0 | 0 | 0 | 0 | 0.96 | 3 |
| PD17973 | 546 | 65 | 0 | 837 | 0 | 0 | 0 | 0 | 577 | 0 | 0 | 0 | 0.96 | 3 |
| PD17981 | 2460 | 1330 | 0 | 1730 | 0 | 0 | 262 | 0 | 1182 | 0 | 0 | 0 | 0.99 | 3 |
| PD17991 | 468 | 65 | 0 | 689 | 0 | 0 | 0 | 0 | 0 | 0 | 0 | 0 | 0.94 | 3 |
| PD17994 | 486 | 235 | 0 | 520 | 0 | 0 | 65 | 0 | 0 | 0 | 0 | 0 | 0.96 | 3 |
| PD18017 | 440 | 114 | 0 | 1165 | 0 | 338 | 51 | 0 | 0 | 0 | 0 | 0 | 0.93 | 3 |
| PD18020 | 747 | 0 | 6873 | 1952 | 0 | 3857 | 4343 | 0 | 0 | 0 | 0 | 0 | 0.98 | 6 |
| PD18022 | 531 | 128 | 0 | 481 | 0 | 336 | 126 | 0 | 0 | 0 | 0 | 0 | 0.96 | 3 |
| PD18024 | 547 | 0 | 5622 | 1298 | 0 | 2858 | 684 | 95 | 0 | 0 | 0 | 0 | 0.96 | 6 |

| | | | | | | | | | | | | | | |
|---|---|---|---|---|---|---|---|---|---|---|---|---|---|---|
| PD18031 | 719 | 168 | 0 | 1089 | 0 | 546 | 247 | 0 | 0 | 0 | 0 | 0 | 0.94 | 3 |
| PD18037 | 2943 | 369 | 0 | 2132 | 0 | 0 | 0 | 0 | 527 | 0 | 0 | 0 | 0.98 | 3 |
| PD18045 | 2544 | 3999 | 0 | 1916 | 0 | 1314 | 9695 | 0 | 0 | 0 | 0 | 0 | 1.00 | 11 |
| PD18046 | 939 | 128 | 0 | 1125 | 0 | 607 | 102 | 0 | 0 | 0 | 0 | 0 | 0.96 | 3 |
| PD18047 | 831 | 109 | 0 | 933 | 0 | 389 | 82 | 0 | 0 | 0 | 0 | 0 | 0.96 | 3 |
| PD18048 | 1199 | 128 | 0 | 2691 | 0 | 498 | 270 | 0 | 0 | 0 | 0 | 0 | 0.96 | 3 |
| PD18049 | 950 | 511 | 0 | 1675 | 0 | 0 | 256 | 0 | 0 | 0 | 0 | 0 | 0.97 | 3 |
| PD18050 | 1956 | 506 | 0 | 0 | 0 | 760 | 344 | 0 | 0 | 0 | 0 | 0 | 0.97 | 9 |
| PD18100 | 417 | 114 | 0 | 577 | 0 | 261 | 37 | 0 | 125 | 0 | 0 | 0 | 0.95 | 3 |
| PD18101 | 311 | 0 | 0 | 333 | 0 | 180 | 39 | 0 | 0 | 0 | 0 | 0 | 0.91 | 3 |
| PD18116 | 482 | 108 | 0 | 645 | 0 | 245 | 64 | 0 | 0 | 0 | 0 | 0 | 0.95 | 3 |
| PD18149 | 561 | 74 | 0 | 1533 | 0 | 311 | 91 | 0 | 0 | 0 | 0 | 0 | 0.94 | 3 |
| PD18188 | 616 | 111 | 0 | 1199 | 0 | 257 | 53 | 0 | 0 | 0 | 0 | 0 | 0.95 | 3 |
| PD18189 | 720 | 527 | 0 | 1587 | 0 | 690 | 279 | 0 | 469 | 0 | 0 | 0 | 0.97 | 3 |
| PD18247 | 0 | 3398 | 0 | 0 | 0 | 0 | 5738 | 0 | 0 | 0 | 0 | 0 | 1.00 | 11 |
| PD18251 | 618 | 160 | 0 | 1858 | 0 | 659 | 192 | 0 | 0 | 0 | 0 | 0 | 0.92 | 3 |
| PD18257 | 568 | 296 | 0 | 965 | 0 | 478 | 395 | 0 | 271 | 0 | 0 | 0 | 0.98 | 3 |
| PD18258 | 422 | 34 | 0 | 426 | 0 | 206 | 0 | 0 | 54 | 0 | 0 | 0 | 0.92 | 3 |

| | | | | | | | | | | | | | | |
|---|---|---|---|---|---|---|---|---|---|---|---|---|---|---|
| PD18259 | 992 | 510 | 2445 | 0 | 0 | 2274 | 418 | 0 | 0 | 0 | 0 | 0 | 0.97 | 6 |
| PD18264 | 0 | 4554 | 0 | 0 | 0 | 0 | 2126 | 0 | 0 | 0 | 0 | 0 | 0.99 | 8 |
| PD18269 | 334 | 26 | 0 | 856 | 0 | 171 | 0 | 0 | 0 | 0 | 0 | 0 | 0.93 | 3 |
| PD18728 | 352 | 224 | 0 | 965 | 0 | 228 | 0 | 0 | 0 | 0 | 0 | 0 | 0.96 | 3 |
| PD18730 | 501 | 670 | 0 | 0 | 0 | 0 | 730 | 0 | 0 | 0 | 0 | 0 | 0.99 | 11 |
| PD18733 | 539 | 211 | 0 | 1476 | 0 | 720 | 73 | 0 | 0 | 0 | 0 | 0 | 0.94 | 3 |
| PD18734 | 978 | 150 | 0 | 1215 | 0 | 1029 | 107 | 0 | 349 | 0 | 0 | 0 | 0.95 | 3 |
| PD18748 | 969 | 688 | 0 | 1143 | 0 | 0 | 158 | 0 | 0 | 0 | 0 | 0 | 0.98 | 3 |
| PD18749 | 0 | 1401 | 0 | 0 | 0 | 0 | 3260 | 0 | 0 | 0 | 0 | 0 | 0.99 | 11 |
| PD18751 | 772 | 82 | 0 | 536 | 0 | 471 | 46 | 0 | 0 | 0 | 0 | 0 | 0.96 | 3 |
| PD18754 | 654 | 784 | 0 | 884 | 0 | 0 | 271 | 0 | 0 | 0 | 0 | 0 | 0.98 | 3 |
| PD18756 | 726 | 99 | 0 | 663 | 0 | 0 | 0 | 0 | 147 | 0 | 0 | 0 | 0.96 | 3 |
| PD18768 | 577 | 92 | 0 | 1376 | 0 | 456 | 55 | 0 | 0 | 0 | 0 | 0 | 0.95 | 3 |
| PD18769 | 466 | 659 | 0 | 0 | 0 | 0 | 1857 | 0 | 0 | 0 | 0 | 0 | 0.99 | 11 |
| PD18771 | 461 | 35 | 0 | 1227 | 0 | 0 | 53 | 0 | 0 | 0 | 0 | 0 | 0.96 | 3 |
| PD18775 | 457 | 91 | 0 | 607 | 0 | 175 | 0 | 0 | 0 | 0 | 0 | 0 | 0.93 | 3 |
| PD18776 | 488 | 0 | 0 | 866 | 0 | 235 | 40 | 0 | 0 | 0 | 0 | 0 | 0.94 | 3 |
| PD22036 | 255 | 17 | 0 | 587 | 0 | 188 | 0 | 0 | 0 | 0 | 0 | 0 | 0.94 | 3 |

| | | | | | | | | | | | | | | |
|---|---|---|---|---|---|---|---|---|---|---|---|---|---|---|
| PD22251 | 645 | 76 | 0 | 784 | 0 | 0 | 0 | 0 | 0 | 0 | 0 | 0 | 0.94 | 3 |
| PD22355 | 1014 | 0 | 3740 | 1206 | 0 | 3376 | 425 | 0 | 0 | 0 | 0 | 0 | 0.95 | 6 |
| PD22357 | 1347 | 3073 | 0 | 0 | 0 | 0 | 3299 | 0 | 0 | 0 | 0 | 0 | 0.99 | 11 |
| PD22358 | 284 | 0 | 3055 | 899 | 0 | 937 | 983 | 0 | 0 | 0 | 0 | 0 | 0.98 | 6 |
| PD22359 | 521 | 269 | 0 | 942 | 0 | 0 | 0 | 0 | 85 | 0 | 0 | 0 | 0.96 | 3 |
| PD22360 | 312 | 211 | 4705 | 0 | 0 | 2489 | 793 | 0 | 0 | 0 | 0 | 0 | 0.96 | 6 |
| PD22361 | 1231 | 1876 | 0 | 0 | 0 | 0 | 2792 | 0 | 0 | 0 | 0 | 0 | 1.00 | 11 |
| PD22362 | 282 | 354 | 0 | 718 | 0 | 520 | 75 | 0 | 0 | 0 | 0 | 0 | 0.94 | 3 |
| PD22363 | 436 | 165 | 4024 | 1126 | 0 | 2872 | 282 | 0 | 0 | 0 | 0 | 0 | 0.96 | 6 |
| PD22364 | 911 | 549 | 0 | 617 | 0 | 0 | 325 | 0 | 0 | 0 | 0 | 0 | 0.98 | 3 |
| PD22365 | 294 | 42 | 0 | 771 | 0 | 730 | 69 | 0 | 0 | 0 | 0 | 0 | 0.89 | 3 |
| PD22366 | 666 | 487 | 5634 | 0 | 0 | 1597 | 1425 | 0 | 0 | 0 | 0 | 0 | 0.97 | 6 |
| PD23550 | 651 | 492 | 0 | 1615 | 0 | 0 | 108 | 0 | 0 | 0 | 0 | 0 | 0.95 | 3 |
| PD23554 | 243 | 0 | 3948 | 0 | 0 | 2251 | 457 | 0 | 0 | 0 | 0 | 0 | 0.95 | 6 |
| PD23558 | 709 | 0 | 3535 | 2282 | 0 | 2699 | 909 | 0 | 0 | 0 | 0 | 0 | 0.92 | 6 |
| PD23559 | 537 | 211 | 0 | 2434 | 0 | 1591 | 609 | 0 | 0 | 0 | 0 | 0 | 0.95 | 3 |
| PD23560 | 504 | 271 | 0 | 1174 | 0 | 624 | 433 | 0 | 0 | 0 | 0 | 0 | 0.96 | 3 |
| PD23561 | 1325 | 18114 | 0 | 0 | 3953 | 0 | 30615 | 0 | 0 | 0 | 4021 | 0 | 1.00 | 11 |

| | | | | | | | | | | | | | | |
|---|---|---|---|---|---|---|---|---|---|---|---|---|---|---|
| PD23562 | 707 | 0 | 4433 | 1603 | 0 | 0 | 605 | 0 | 0 | 0 | 0 | 0 | 0.97 | 6 |
| PD23563 | 264 | 0 | 3351 | 867 | 0 | 3418 | 323 | 0 | 0 | 0 | 0 | 0 | 0.96 | 6 |
| PD23564 | 768 | 0 | 0 | 2361 | 61435 | 0 | 0 | 0 | 0 | 0 | 18883 | 0 | 0.94 | Singleton 5 |
| PD23565 | 2074 | 3451 | 0 | 0 | 0 | 0 | 3327 | 0 | 0 | 0 | 0 | 0 | 1.00 | 11 |
| PD23566 | 599 | 0 | 3762 | 454 | 0 | 1852 | 0 | 0 | 0 | 0 | 0 | 0 | 0.95 | 6 |
| PD23567 | 650 | 80 | 3004 | 1127 | 0 | 2337 | 0 | 0 | 0 | 0 | 0 | 0 | 0.97 | 6 |
| PD23569 | 72 | 41 | 0 | 548 | 0 | 320 | 74 | 0 | 0 | 0 | 0 | 0 | 0.80 | 3 |
| PD23570 | 430 | 691 | 0 | 833 | 0 | 0 | 291 | 0 | 0 | 0 | 0 | 0 | 0.99 | 3 |
| PD23574 | 671 | 0 | 6833 | 1436 | 0 | 1769 | 2385 | 0 | 0 | 0 | 0 | 0 | 0.97 | 6 |
| PD23577 | 354 | 0 | 1454 | 1212 | 0 | 673 | 181 | 96 | 0 | 0 | 0 | 0 | 0.95 | 6 |
| PD23578 | 191 | 0 | 2528 | 1361 | 0 | 2437 | 323 | 0 | 0 | 0 | 0 | 0 | 0.96 | 6 |
| PD23579 | 1423 | 0 | 0 | 1456 | 23145 | 0 | 0 | 0 | 0 | 24170 | 37117 | 0 | 0.86 | 5 |
| PD24182 | 574 | 348 | 4922 | 1820 | 0 | 0 | 1873 | 0 | 0 | 0 | 0 | 0 | 0.98 | 6 |
| PD24186 | 0 | 0 | 2710 | 2615 | 0 | 1596 | 358 | 209 | 0 | 0 | 0 | 0 | 0.93 | 6 |
| PD24189 | 934 | 0 | 0 | 1367 | 12148 | 0 | 0 | 0 | 0 | 5404 | 9737 | 0 | 0.94 | 5 |
| PD24190 | 0 | 144 | 0 | 3619 | 0 | 3234 | 673 | 2138 | 0 | 0 | 0 | 0 | 0.97 | 1 |
| PD24191 | 310 | 0 | 2966 | 1808 | 0 | 825 | 211 | 0 | 0 | 0 | 0 | 0 | 0.92 | 6 |
| PD24192 | 432 | 0 | 8853 | 4763 | 0 | 1757 | 2022 | 0 | 0 | 0 | 0 | 0 | 0.97 | 6 |

| | | | | | | | | | | | | | | |
|---|---|---|---|---|---|---|---|---|---|---|---|---|---|---|
| PD24193 | 1012 | 593 | 0 | 0 | 3406 | 0 | 400 | 0 | 0 | 1441 | 4385 | 0 | 0.92 | 5 |
| PD24194 | 0 | 5344 | 0 | 1913 | 0 | 774 | 5535 | 129 | 0 | 0 | 0 | 0 | 1.00 | 11 |
| PD24195 | 429 | 121 | 0 | 1356 | 0 | 334 | 0 | 0 | 0 | 0 | 0 | 0 | 0.94 | 3 |
| PD24196 | 314 | 140 | 632 | 651 | 0 | 565 | 190 | 0 | 128 | 0 | 0 | 0 | 0.95 | 6 |
| PD24197 | 0 | 314 | 7558 | 1219 | 0 | 10397 | 513 | 214 | 0 | 0 | 0 | 0 | 0.96 | 6 |
| PD24199 | 540 | 168 | 0 | 1316 | 0 | 557 | 273 | 0 | 0 | 0 | 0 | 0 | 0.96 | 3 |
| PD24200 | 41 | 0 | 450 | 164 | 0 | 295 | 24 | 0 | 0 | 0 | 0 | 0 | 0.82 | 6 |
| PD24201 | 1110 | 734 | 3180 | 4398 | 0 | 9769 | 1287 | 0 | 0 | 0 | 0 | 0 | 0.93 | 6 |
| PD24202 | 585 | 190 | 3224 | 1242 | 0 | 1626 | 912 | 0 | 0 | 0 | 0 | 0 | 0.98 | 6 |
| PD24204 | 1554 | 0 | 0 | 903 | 0 | 0 | 0 | 0 | 338 | 0 | 0 | 0 | 0.98 | 3 |
| PD24205 | 615 | 239 | 3588 | 1903 | 0 | 2278 | 305 | 0 | 0 | 0 | 0 | 0 | 0.96 | 6 |
| PD24206 | 333 | 355 | 0 | 1564 | 0 | 1152 | 206 | 0 | 0 | 0 | 0 | 0 | 0.93 | 3 |
| PD24207 | 1630 | 1694 | 0 | 0 | 0 | 0 | 1421 | 0 | 0 | 0 | 0 | 0 | 0.99 | 8 |
| PD24208 | 612 | 15048 | 0 | 0 | 0 | 0 | 9964 | 0 | 0 | 0 | 0 | 0 | 1.00 | 8 |
| PD24209 | 297 | 2243 | 949 | 0 | 0 | 1223 | 5457 | 0 | 0 | 0 | 0 | 0 | 1.00 | 11 |
| PD24212 | 588 | 519 | 2990 | 2030 | 0 | 3495 | 433 | 0 | 0 | 0 | 0 | 0 | 0.94 | 6 |
| PD24214 | 1270 | 0 | 0 | 803 | 0 | 594 | 0 | 0 | 412 | 0 | 0 | 0 | 0.98 | 3 |
| PD24215 | 0 | 184 | 0 | 2678 | 0 | 2432 | 642 | 0 | 241 | 0 | 0 | 0 | 0.86 | 1 |

| | | | | | | | | | | | | | | |
|---|---|---|---|---|---|---|---|---|---|---|---|---|---|---|
| PD24216 | 0 | 2660 | 0 | 4234 | 0 | 0 | 2445 | 0 | 0 | 0 | 0 | 0 | 0.99 | 7 |
| PD24217 | 0 | 4080 | 0 | 284 | 0 | 329 | 10612 | 0 | 0 | 0 | 0 | 0 | 1.00 | 11 |
| PD24218 | 463 | 163 | 0 | 2567 | 0 | 0 | 95 | 0 | 0 | 0 | 0 | 0 | 0.91 | 3 |
| PD24219 | 1635 | 0 | 0 | 1042 | 0 | 536 | 106 | 0 | 0 | 0 | 0 | 0 | 0.97 | 3 |
| PD24220 | 1520 | 272 | 0 | 0 | 0 | 896 | 923 | 0 | 0 | 0 | 0 | 0 | 0.98 | 9 |
| PD24221 | 131 | 0 | 0 | 1299 | 0 | 575 | 141 | 0 | 147 | 0 | 0 | 0 | 0.85 | 3 |
| PD24223 | 972 | 1041 | 0 | 1415 | 0 | 0 | 0 | 0 | 0 | 0 | 0 | 0 | 0.98 | 3 |
| PD24224 | 473 | 132 | 0 | 965 | 0 | 523 | 119 | 0 | 0 | 0 | 0 | 0 | 0.95 | 3 |
| PD24225 | 285 | 372 | 0 | 566 | 0 | 390 | 208 | 0 | 0 | 0 | 0 | 0 | 0.98 | 3 |
| PD24302 | 382 | 100 | 0 | 786 | 0 | 0 | 0 | 0 | 0 | 0 | 0 | 0 | 0.93 | 3 |
| PD24303 | 199 | 0 | 2321 | 754 | 0 | 613 | 613 | 0 | 0 | 0 | 0 | 0 | 0.96 | 6 |
| PD24304 | 232 | 0 | 2948 | 0 | 0 | 3452 | 221 | 0 | 0 | 0 | 0 | 0 | 0.96 | 6 |
| PD24306 | 155 | 0 | 1469 | 451 | 0 | 1079 | 182 | 0 | 0 | 0 | 0 | 0 | 0.93 | 6 |
| PD24307 | 315 | 77 | 0 | 880 | 0 | 283 | 39 | 0 | 0 | 0 | 0 | 0 | 0.94 | 3 |
| PD24308 | 625 | 0 | 724 | 2534 | 0 | 516 | 353 | 0 | 0 | 0 | 0 | 0 | 0.88 | 4 |
| PD24314 | 782 | 85 | 0 | 1795 | 0 | 746 | 215 | 0 | 247 | 0 | 0 | 0 | 0.94 | 3 |
| PD24318 | 669 | 54 | 0 | 1723 | 0 | 0 | 100 | 0 | 0 | 0 | 0 | 0 | 0.95 | 3 |
| PD24320 | 0 | 0 | 0 | 0 | 1010 | 0 | 0 | 0 | 0 | 0 | 14685 | 0 | 0.92 | 10 |

| | | | | | | | | | | | | | | |
|---|---|---|---|---|---|---|---|---|---|---|---|---|---|---|
| PD24322 | 919 | 753 | 0 | 2206 | 0 | 0 | 990 | 0 | 0 | 0 | 0 | 0 | 0.98 | 7 |
| PD24325 | 1167 | 0 | 3766 | 3150 | 0 | 940 | 1294 | 70 | 0 | 0 | 0 | 0 | 0.95 | 6 |
| PD24326 | 338 | 10724 | 0 | 1641 | 0 | 0 | 9665 | 0 | 1252 | 0 | 0 | 0 | 1.00 | 8 |
| PD24327 | 779 | 6158 | 0 | 1152 | 0 | 695 | 7168 | 233 | 0 | 0 | 0 | 0 | 1.00 | 11 |
| PD24329 | 908 | 140 | 0 | 519 | 0 | 469 | 75 | 0 | 0 | 0 | 0 | 0 | 0.98 | 3 |
| PD24332 | 808 | 181 | 0 | 394 | 0 | 537 | 0 | 0 | 0 | 0 | 0 | 0 | 0.95 | 3 |
| PD24333 | 878 | 1726 | 0 | 581 | 0 | 0 | 1249 | 18450 | 0 | 0 | 0 | 0 | 0.94 | Singleton 6 |
| PD24335 | 1553 | 240 | 0 | 1115 | 0 | 712 | 328 | 0 | 0 | 0 | 0 | 0 | 0.98 | 3 |
| PD24336 | 239 | 368 | 0 | 1863 | 0 | 716 | 153 | 0 | 0 | 0 | 0 | 0 | 0.95 | 3 |
| PD24337 | 416 | 0 | 2689 | 1125 | 0 | 1141 | 336 | 0 | 0 | 0 | 0 | 0 | 0.95 | 6 |
| PD3851 | 394 | 123 | 0 | 665 | 0 | 605 | 32 | 0 | 0 | 0 | 0 | 0 | 0.95 | 3 |
| PD3890 | 375 | 194 | 3418 | 798 | 0 | 0 | 648 | 151 | 0 | 0 | 0 | 0 | 0.98 | 6 |
| PD3904 | 286 | 440 | 2168 | 1285 | 0 | 1135 | 354 | 0 | 0 | 0 | 0 | 0 | 0.95 | 6 |
| PD3905 | 299 | 227 | 1878 | 740 | 0 | 937 | 522 | 0 | 0 | 0 | 0 | 0 | 0.97 | 6 |
| PD3945 | 0 | 344 | 2586 | 3293 | 0 | 3689 | 639 | 0 | 0 | 0 | 0 | 0 | 0.96 | 6 |
| PD3989 | 754 | 344 | 0 | 769 | 0 | 371 | 0 | 0 | 0 | 0 | 0 | 0 | 0.97 | 3 |
| PD4005 | 517 | 776 | 3238 | 0 | 0 | 0 | 1510 | 0 | 0 | 0 | 0 | 0 | 0.99 | 6 |
| PD4006 | 676 | 0 | 5772 | 0 | 0 | 2780 | 347 | 0 | 0 | 0 | 0 | 0 | 0.94 | 6 |

| | | | | | | | | | | | | | | |
|---|---|---|---|---|---|---|---|---|---|---|---|---|---|---|
| PD4069 | 812 | 110 | 0 | 838 | 0 | 0 | 0 | 0 | 325 | 0 | 0 | 0 | 0.94 | 3 |
| PD4072 | 0 | 13732 | 0 | 0 | 0 | 305 | 13894 | 0 | 0 | 0 | 0 | 0 | 1.00 | 8 |
| PD4076 | 786 | 294 | 0 | 1011 | 0 | 0 | 0 | 0 | 0 | 0 | 0 | 0 | 0.98 | 3 |
| PD4085 | 602 | 122 | 0 | 1091 | 0 | 822 | 67 | 0 | 0 | 0 | 0 | 0 | 0.95 | 3 |
| PD4086 | 279 | 219 | 503 | 544 | 0 | 0 | 406 | 0 | 0 | 0 | 0 | 0 | 0.98 | 4 |
| PD4088 | 547 | 99 | 461 | 253 | 0 | 0 | 69 | 0 | 262 | 0 | 0 | 0 | 0.96 | 4 |
| PD4103 | 1140 | 721 | 0 | 1808 | 0 | 753 | 514 | 0 | 282 | 0 | 0 | 0 | 0.97 | 3 |
| PD4107 | 0 | 877 | 2922 | 2407 | 0 | 3464 | 204 | 0 | 0 | 0 | 0 | 0 | 0.97 | 6 |
| PD4109 | 1764 | 0 | 1143 | 3675 | 0 | 1828 | 1903 | 0 | 0 | 0 | 0 | 0 | 0.93 | 3 |
| PD4115 | 459 | 192 | 3565 | 2238 | 0 | 2664 | 344 | 290 | 0 | 0 | 0 | 0 | 0.97 | 6 |
| PD4116 | 0 | 335 | 2787 | 1688 | 0 | 2436 | 924 | 0 | 0 | 0 | 0 | 0 | 0.97 | 6 |
| PD4192 | 910 | 341 | 2294 | 0 | 0 | 0 | 747 | 0 | 0 | 0 | 0 | 0 | 0.92 | 6 |
| PD4194 | 468 | 143 | 0 | 353 | 0 | 254 | 210 | 0 | 0 | 0 | 0 | 0 | 0.96 | 3 |
| PD4198 | 1055 | 657 | 0 | 1494 | 0 | 1012 | 299 | 0 | 0 | 0 | 0 | 0 | 0.97 | 3 |
| PD4199 | 0 | 3599 | 0 | 0 | 0 | 0 | 3349 | 0 | 0 | 0 | 0 | 0 | 1.00 | 8 |
| PD4225 | 1031 | 131 | 0 | 533 | 0 | 260 | 0 | 0 | 0 | 0 | 0 | 0 | 0.98 | 3 |
| PD4248 | 340 | 154 | 0 | 1140 | 0 | 414 | 148 | 0 | 0 | 0 | 0 | 0 | 0.94 | 3 |
| PD4252 | 1076 | 0 | 0 | 1592 | 0 | 492 | 200 | 0 | 413 | 0 | 0 | 0 | 0.95 | 3 |

| | | | | | | | | | | | | | | |
|---|---|---|---|---|---|---|---|---|---|---|---|---|---|---|
| PD4255 | 749 | 0 | 0 | 1973 | 0 | 837 | 241 | 0 | 754 | 0 | 0 | 0 | 0.91 | 3 |
| PD4261 | 410 | 37 | 0 | 1123 | 0 | 186 | 40 | 0 | 0 | 0 | 0 | 0 | 0.95 | 3 |
| PD4264 | 1026 | 279 | 0 | 813 | 0 | 0 | 0 | 0 | 0 | 0 | 0 | 0 | 0.97 | 3 |
| PD4266 | 626 | 1224 | 0 | 0 | 0 | 0 | 538 | 0 | 0 | 0 | 0 | 0 | 0.99 | 8 |
| PD4267 | 745 | 148 | 0 | 716 | 0 | 0 | 510 | 0 | 0 | 0 | 0 | 0 | 0.98 | 3 |
| PD4315 | 614 | 515 | 0 | 978 | 0 | 812 | 469 | 0 | 0 | 0 | 0 | 0 | 0.98 | 3 |
| PD4604 | 526 | 301 | 3301 | 2056 | 0 | 2561 | 578 | 0 | 0 | 0 | 0 | 0 | 0.91 | 6 |
| PD4605 | 1046 | 78 | 0 | 1325 | 0 | 0 | 201 | 0 | 0 | 0 | 0 | 0 | 0.96 | 3 |
| PD4606 | 401 | 43 | 0 | 685 | 0 | 0 | 0 | 0 | 0 | 0 | 0 | 0 | 0.94 | 3 |
| PD4607 | 0 | 16095 | 0 | 0 | 0 | 0 | 9858 | 0 | 0 | 0 | 0 | 0 | 1.00 | 8 |
| PD4613 | 575 | 948 | 0 | 0 | 0 | 0 | 672 | 0 | 0 | 0 | 0 | 0 | 0.99 | 8 |
| PD4826 | 681 | 553 | 0 | 1259 | 0 | 740 | 585 | 0 | 0 | 0 | 0 | 0 | 0.97 | 3 |
| PD4833 | 423 | 129 | 3714 | 982 | 0 | 718 | 435 | 0 | 0 | 0 | 0 | 0 | 0.97 | 6 |
| PD4836 | 690 | 138 | 1044 | 1531 | 0 | 604 | 296 | 0 | 0 | 0 | 0 | 0 | 0.95 | 4 |
| PD4841 | 656 | 159 | 1798 | 2138 | 0 | 1903 | 366 | 0 | 0 | 0 | 0 | 0 | 0.95 | 6 |
| PD4844 | 568 | 0 | 6149 | 3480 | 0 | 1452 | 1161 | 0 | 0 | 0 | 0 | 0 | 0.96 | 6 |
| PD4845 | 525 | 0 | 1058 | 1432 | 0 | 1821 | 100 | 799 | 0 | 0 | 0 | 0 | 0.94 | 6 |
| PD4847 | 970 | 257 | 0 | 5343 | 0 | 3022 | 215 | 525 | 710 | 0 | 0 | 0 | 0.89 | 3 |

| | | | | | | | | | | | | | | |
|---|---|---|---|---|---|---|---|---|---|---|---|---|---|---|
| PD4872 | 0 | 560 | 0 | 1477 | 0 | 1880 | 893 | 0 | 0 | 0 | 0 | 0 | 0.98 | 1 |
| PD4874 | 0 | 900 | 0 | 2677 | 0 | 3353 | 2779 | 0 | 0 | 0 | 0 | 0 | 0.99 | 1 |
| PD4875 | 638 | 163 | 1721 | 2268 | 0 | 1691 | 324 | 0 | 0 | 0 | 0 | 0 | 0.97 | 6 |
| PD4876 | 272 | 240 | 3105 | 931 | 0 | 0 | 360 | 0 | 0 | 0 | 0 | 0 | 0.96 | 6 |
| PD4951 | 277 | 85 | 945 | 741 | 0 | 515 | 216 | 0 | 0 | 0 | 0 | 0 | 0.95 | 6 |
| PD4952 | 1109 | 650 | 4080 | 2586 | 0 | 2771 | 837 | 0 | 0 | 0 | 0 | 0 | 0.98 | 6 |
| PD4953 | 329 | 220 | 2559 | 1900 | 0 | 1601 | 337 | 0 | 0 | 0 | 0 | 0 | 0.95 | 6 |
| PD4954 | 498 | 215 | 2647 | 1213 | 0 | 747 | 444 | 0 | 0 | 0 | 0 | 0 | 0.96 | 6 |
| PD4955 | 353 | 283 | 1714 | 1503 | 0 | 1883 | 334 | 0 | 0 | 0 | 0 | 0 | 0.96 | 6 |
| PD4956 | 766 | 0 | 7852 | 1627 | 0 | 0 | 1337 | 0 | 0 | 0 | 0 | 0 | 0.97 | 6 |
| PD4957 | 298 | 213 | 740 | 939 | 0 | 818 | 271 | 0 | 0 | 0 | 0 | 0 | 0.97 | 6 |
| PD4958 | 0 | 665 | 1511 | 3275 | 0 | 3088 | 799 | 0 | 0 | 0 | 0 | 0 | 0.96 | 1 |
| PD4959 | 488 | 123 | 1358 | 1104 | 0 | 1420 | 118 | 0 | 0 | 0 | 0 | 0 | 0.94 | 6 |
| PD4962 | 0 | 1689 | 0 | 0 | 0 | 2240 | 2914 | 0 | 0 | 0 | 0 | 0 | 1.00 | 11 |
| PD4965 | 799 | 116 | 0 | 1203 | 0 | 535 | 53 | 0 | 0 | 0 | 0 | 0 | 0.96 | 3 |
| PD4967 | 533 | 609 | 0 | 1158 | 0 | 0 | 927 | 0 | 0 | 0 | 0 | 0 | 0.99 | 7 |
| PD4968 | 1283 | 1115 | 0 | 1616 | 0 | 766 | 0 | 0 | 0 | 0 | 0 | 0 | 0.98 | 3 |
| PD4969 | 240 | 51 | 0 | 514 | 0 | 227 | 0 | 0 | 0 | 0 | 0 | 0 | 0.91 | 3 |

| | | | | | | | | | | | | | | |
|---|---|---|---|---|---|---|---|---|---|---|---|---|---|---|
| PD4970 | 668 | 118 | 0 | 1252 | 0 | 277 | 95 | 0 | 0 | 0 | 0 | 0 | 0.95 | 3 |
| PD4971 | 970 | 124 | 0 | 745 | 0 | 797 | 129 | 0 | 0 | 0 | 0 | 0 | 0.97 | 3 |
| PD4972 | 355 | 33 | 0 | 542 | 0 | 189 | 26 | 0 | 0 | 0 | 0 | 0 | 0.92 | 3 |
| PD4975 | 698 | 109 | 3959 | 1412 | 0 | 987 | 341 | 0 | 0 | 0 | 0 | 0 | 0.95 | 6 |
| PD4976 | 2097 | 175 | 0 | 2804 | 0 | 0 | 499 | 0 | 700 | 0 | 0 | 0 | 0.95 | 3 |
| PD4977 | 0 | 21981 | 0 | 2315 | 0 | 0 | 17259 | 0 | 703 | 0 | 0 | 0 | 1.00 | 8 |
| PD4978 | 610 | 628 | 0 | 1243 | 0 | 671 | 584 | 0 | 0 | 0 | 0 | 0 | 0.97 | 3 |
| PD4980 | 675 | 0 | 0 | 1374 | 0 | 1035 | 210 | 0 | 502 | 0 | 0 | 0 | 0.95 | 3 |
| PD4981 | 626 | 56 | 0 | 605 | 0 | 376 | 0 | 0 | 0 | 0 | 0 | 0 | 0.96 | 3 |
| PD4982 | 534 | 47 | 0 | 685 | 0 | 208 | 0 | 0 | 0 | 0 | 0 | 0 | 0.96 | 3 |
| PD4983 | 427 | 70 | 0 | 1266 | 0 | 0 | 0 | 0 | 0 | 0 | 0 | 0 | 0.94 | 3 |
| PD4985 | 531 | 79 | 0 | 1129 | 0 | 210 | 55 | 0 | 0 | 0 | 0 | 0 | 0.96 | 3 |
| PD4986 | 402 | 49 | 298 | 1016 | 0 | 211 | 0 | 0 | 0 | 0 | 0 | 0 | 0.93 | 4 |
| PD5925 | 0 | 280 | 2877 | 2655 | 0 | 1608 | 1360 | 0 | 0 | 0 | 0 | 0 | 0.99 | 6 |
| PD5928 | 584 | 281 | 2567 | 1012 | 0 | 2419 | 251 | 0 | 0 | 0 | 0 | 0 | 0.97 | 6 |
| PD5930 | 356 | 0 | 1910 | 616 | 0 | 573 | 491 | 0 | 0 | 0 | 0 | 0 | 0.95 | 6 |
| PD5932 | 536 | 0 | 5501 | 2251 | 0 | 0 | 1772 | 0 | 0 | 0 | 0 | 0 | 0.97 | 6 |
| PD5934 | 696 | 0 | 6408 | 521 | 0 | 3548 | 406 | 0 | 0 | 0 | 0 | 0 | 0.96 | 6 |

| | | | | | | | | | | | | | | |
|---|---|---|---|---|---|---|---|---|---|---|---|---|---|---|
| PD5935 | 877 | 0 | 5508 | 1971 | 0 | 0 | 1315 | 0 | 0 | 0 | 0 | 0 | 0.97 | 6 |
| PD5936 | 752 | 258 | 0 | 1316 | 0 | 456 | 0 | 0 | 0 | 0 | 0 | 0 | 0.97 | 3 |
| PD5937 | 1099 | 29416 | 0 | 0 | 17791 | 0 | 19561 | 0 | 0 | 0 | 8230 | 0 | 0.99 | 8 |
| PD5942 | 1290 | 231 | 2198 | 1763 | 0 | 0 | 283 | 0 | 225 | 0 | 0 | 0 | 0.95 | 4 |
| PD5944 | 744 | 99 | 0 | 1136 | 0 | 546 | 84 | 0 | 0 | 0 | 0 | 0 | 0.94 | 3 |
| PD5945 | 344 | 0 | 4532 | 1104 | 0 | 2331 | 522 | 0 | 0 | 0 | 0 | 0 | 0.95 | 6 |
| PD5946 | 1424 | 195 | 0 | 1640 | 0 | 0 | 198 | 0 | 455 | 0 | 0 | 0 | 0.97 | 3 |
| PD5947 | 513 | 210 | 0 | 532 | 0 | 319 | 205 | 0 | 0 | 0 | 0 | 0 | 0.96 | 3 |
| PD5948 | 442 | 0 | 8707 | 5513 | 0 | 0 | 510 | 0 | 0 | 0 | 0 | 0 | 0.84 | 6 |
| PD5950 | 953 | 0 | 0 | 712 | 0 | 304 | 0 | 0 | 221 | 0 | 0 | 0 | 0.96 | 3 |
| PD5951 | 1514 | 217 | 0 | 887 | 0 | 471 | 104 | 0 | 0 | 0 | 0 | 0 | 0.98 | 3 |
| PD5953 | 775 | 49 | 0 | 1001 | 0 | 311 | 0 | 0 | 0 | 0 | 0 | 0 | 0.96 | 3 |
| PD5956 | 657 | 223 | 0 | 1072 | 0 | 1761 | 0 | 0 | 372 | 0 | 0 | 0 | 0.94 | 3 |
| PD5959 | 601 | 311 | 0 | 1518 | 0 | 0 | 74 | 0 | 0 | 0 | 0 | 0 | 0.95 | 3 |
| PD5960 | 449 | 151 | 838 | 1457 | 0 | 966 | 184 | 0 | 0 | 0 | 0 | 0 | 0.92 | 6 |
| PD5961 | 826 | 338 | 0 | 906 | 0 | 0 | 0 | 0 | 244 | 0 | 0 | 0 | 0.95 | 3 |
| PD5964 | 337 | 99 | 0 | 522 | 0 | 428 | 74 | 0 | 130 | 0 | 0 | 0 | 0.91 | 3 |
| PD6016 | 487 | 433 | 0 | 756 | 0 | 0 | 235 | 0 | 0 | 0 | 0 | 0 | 0.99 | 3 |

| | | | | | | | | | | | | | | |
|---|---|---|---|---|---|---|---|---|---|---|---|---|---|---|
| PD6041 | 505 | 68 | 0 | 976 | 0 | 222 | 0 | 0 | 0 | 0 | 0 | 0 | 0.94 | 3 |
| PD6042 | 0 | 577 | 0 | 2805 | 0 | 2398 | 1339 | 0 | 0 | 0 | 0 | 0 | 0.99 | 1 |
| PD6043 | 0 | 6348 | 3522 | 992 | 0 | 4100 | 7151 | 165 | 486 | 0 | 0 | 0 | 1.00 | 11 |
| PD6044 | 725 | 92 | 0 | 1623 | 0 | 0 | 133 | 0 | 0 | 0 | 0 | 0 | 0.95 | 3 |
| PD6045 | 345 | 534 | 0 | 871 | 0 | 402 | 181 | 0 | 0 | 0 | 0 | 0 | 0.99 | 3 |
| PD6046 | 831 | 156 | 0 | 1057 | 0 | 583 | 87 | 0 | 262 | 0 | 0 | 0 | 0.96 | 3 |
| PD6047 | 1985 | 427 | 1763 | 4068 | 0 | 0 | 262 | 0 | 937 | 0 | 0 | 0 | 0.96 | 4 |
| PD6048 | 1017 | 215 | 0 | 737 | 0 | 673 | 181 | 0 | 279 | 0 | 0 | 0 | 0.94 | 3 |
| PD6404 | 780 | 321 | 2807 | 1829 | 0 | 0 | 533 | 283 | 0 | 0 | 0 | 0 | 0.95 | 6 |
| PD6405 | 2315 | 0 | 0 | 0 | 0 | 0 | 33002 | 0 | 0 | 0 | 0 | 0 | 0.98 | 11 |
| PD6406 | 410 | 0 | 3435 | 0 | 0 | 850 | 331 | 0 | 0 | 0 | 0 | 0 | 0.93 | 6 |
| PD6409 | 329 | 0 | 4220 | 0 | 0 | 3064 | 1197 | 0 | 0 | 0 | 0 | 0 | 0.98 | 6 |
| PD6410 | 462 | 0 | 3661 | 1280 | 0 | 1031 | 879 | 0 | 0 | 0 | 0 | 0 | 0.96 | 6 |
| PD6411 | 445 | 103 | 4568 | 0 | 0 | 954 | 605 | 0 | 0 | 0 | 0 | 0 | 0.95 | 6 |
| PD6412 | 2011 | 0 | 0 | 2619 | 9832 | 0 | 691 | 0 | 0 | 0 | 10034 | 0 | 0.90 | 5 |
| PD6413 | 394 | 312 | 2250 | 0 | 0 | 1022 | 610 | 0 | 0 | 0 | 0 | 0 | 0.99 | 6 |
| PD6414 | 371 | 158 | 0 | 1142 | 0 | 0 | 46 | 44 | 0 | 0 | 0 | 0 | 0.95 | 3 |
| PD6415 | 773 | 697 | 4753 | 0 | 0 | 1605 | 1717 | 0 | 0 | 0 | 0 | 0 | 0.98 | 6 |

| | | | | | | | | | | | | | | |
|---|---|---|---|---|---|---|---|---|---|---|---|---|---|---|
| PD6416 | 0 | 142 | 746 | 592 | 0 | 1115 | 477 | 259 | 0 | 0 | 0 | 0 | 0.96 | 6 |
| PD6417 | 849 | 54 | 0 | 573 | 0 | 0 | 70 | 0 | 0 | 0 | 0 | 0 | 0.97 | 3 |
| PD6418 | 541 | 86 | 0 | 1169 | 0 | 224 | 0 | 0 | 0 | 0 | 0 | 0 | 0.94 | 3 |
| PD6422 | 758 | 448 | 0 | 2499 | 0 | 930 | 179 | 0 | 367 | 0 | 0 | 0 | 0.93 | 3 |
| PD6466 | 516 | 166 | 0 | 1127 | 0 | 0 | 0 | 0 | 0 | 0 | 0 | 0 | 0.86 | 3 |
| PD6684 | 713 | 0 | 7891 | 2159 | 0 | 0 | 2713 | 201 | 0 | 0 | 0 | 0 | 0.98 | 6 |
| PD6711 | 630 | 169 | 0 | 1115 | 0 | 0 | 0 | 0 | 0 | 0 | 0 | 0 | 0.96 | 3 |
| PD6719 | 610 | 0 | 2222 | 988 | 0 | 984 | 167 | 0 | 0 | 0 | 0 | 0 | 0.95 | 6 |
| PD6720 | 607 | 118 | 0 | 1195 | 0 | 0 | 98 | 0 | 0 | 0 | 0 | 0 | 0.95 | 3 |
| PD6721 | 767 | 170 | 0 | 850 | 0 | 377 | 67 | 0 | 0 | 0 | 0 | 0 | 0.96 | 3 |
| PD6722 | 0 | 1943 | 978 | 1204 | 0 | 1279 | 4706 | 0 | 0 | 0 | 0 | 0 | 1.00 | 11 |
| PD6727 | 1625 | 354 | 2533 | 1319 | 0 | 0 | 848 | 0 | 0 | 0 | 0 | 0 | 0.97 | 4 |
| PD6728 | 489 | 90 | 731 | 434 | 0 | 672 | 162 | 0 | 0 | 0 | 0 | 0 | 0.94 | 6 |
| PD6729 | 1073 | 90 | 1466 | 1562 | 0 | 506 | 175 | 0 | 0 | 0 | 0 | 0 | 0.95 | 4 |
| PD6730 | 671 | 0 | 5120 | 1194 | 0 | 2529 | 575 | 0 | 0 | 0 | 0 | 0 | 0.95 | 6 |
| PD6731 | 0 | 1523 | 0 | 0 | 0 | 2903 | 4123 | 0 | 0 | 0 | 0 | 0 | 0.99 | 11 |
| PD6732 | 472 | 127 | 730 | 1199 | 0 | 0 | 387 | 0 | 0 | 0 | 0 | 0 | 0.96 | 4 |
| PD6733 | 1225 | 576 | 0 | 1746 | 0 | 0 | 792 | 0 | 337 | 0 | 0 | 0 | 0.97 | 3 |

| | | | | | | | | | | | | | | |
|---|---|---|---|---|---|---|---|---|---|---|---|---|---|---|
| PD7066 | 688 | 0 | 1745 | 562 | 0 | 338 | 305 | 0 | 0 | 0 | 0 | 0 | 0.95 | 6 |
| PD7067 | 832 | 102 | 0 | 2663 | 0 | 1494 | 692 | 197 | 0 | 0 | 0 | 0 | 0.89 | 3 |
| PD7069 | 898 | 1145 | 0 | 1553 | 0 | 0 | 1535 | 0 | 0 | 0 | 0 | 0 | 0.99 | 7 |
| PD7199 | 485 | 45 | 0 | 382 | 0 | 218 | 0 | 0 | 0 | 0 | 0 | 0 | 0.95 | 3 |
| PD7201 | 588 | 135 | 0 | 861 | 0 | 0 | 0 | 0 | 0 | 0 | 0 | 0 | 0.97 | 3 |
| PD7202 | 1836 | 482 | 0 | 1488 | 0 | 0 | 271 | 0 | 1064 | 0 | 0 | 0 | 0.97 | 3 |
| PD7203 | 253 | 251 | 0 | 473 | 0 | 0 | 333 | 0 | 254 | 0 | 0 | 0 | 0.97 | 3 |
| PD7204 | 729 | 226 | 0 | 920 | 0 | 301 | 0 | 0 | 0 | 0 | 0 | 0 | 0.97 | 3 |
| PD7205 | 991 | 1224 | 0 | 3048 | 0 | 0 | 2595 | 0 | 0 | 0 | 0 | 0 | 0.99 | 7 |
| PD7206 | 799 | 150 | 0 | 2219 | 0 | 1369 | 203 | 0 | 0 | 0 | 0 | 0 | 0.93 | 3 |
| PD7207 | 988 | 416 | 0 | 1450 | 0 | 0 | 0 | 0 | 0 | 0 | 0 | 0 | 0.95 | 3 |
| PD7209 | 701 | 139 | 0 | 641 | 0 | 0 | 0 | 0 | 184 | 0 | 0 | 0 | 0.97 | 3 |
| PD7210 | 461 | 38 | 0 | 572 | 0 | 248 | 0 | 0 | 0 | 0 | 0 | 0 | 0.96 | 3 |
| PD7211 | 701 | 122 | 3860 | 1122 | 0 | 2062 | 419 | 0 | 0 | 0 | 0 | 0 | 0.97 | 6 |
| PD7214 | 344 | 120 | 0 | 1029 | 0 | 0 | 0 | 0 | 0 | 0 | 0 | 0 | 0.92 | 3 |
| PD7215 | 414 | 1066 | 0 | 1809 | 0 | 1677 | 1418 | 0 | 0 | 0 | 0 | 0 | 0.99 | 1 |
| PD7217 | 583 | 757 | 2868 | 2148 | 0 | 1453 | 1360 | 0 | 0 | 0 | 0 | 0 | 0.98 | 6 |
| PD7218 | 579 | 56 | 0 | 612 | 0 | 0 | 0 | 0 | 227 | 0 | 0 | 0 | 0.97 | 3 |

| | | | | | | | | | | | | | | |
|---|---|---|---|---|---|---|---|---|---|---|---|---|---|---|
| PD7219 | 510 | 4455 | 0 | 0 | 0 | 0 | 6656 | 0 | 0 | 0 | 0 | 0 | 1.00 | 11 |
| PD7220 | 910 | 469 | 0 | 1113 | 0 | 0 | 217 | 0 | 0 | 0 | 0 | 0 | 0.97 | 3 |
| PD7221 | 758 | 405 | 0 | 675 | 0 | 0 | 202 | 0 | 0 | 0 | 0 | 0 | 0.98 | 3 |
| PD7238 | 775 | 411 | 0 | 1031 | 0 | 0 | 0 | 0 | 0 | 0 | 0 | 0 | 0.98 | 3 |
| PD7240 | 1432 | 134 | 1143 | 2201 | 0 | 0 | 278 | 0 | 0 | 0 | 0 | 0 | 0.94 | 4 |
| PD7243 | 1066 | 607 | 0 | 2473 | 0 | 1565 | 323 | 0 | 740 | 0 | 0 | 0 | 0.97 | 3 |
| PD7248 | 658 | 0 | 6213 | 2634 | 0 | 1893 | 1644 | 0 | 0 | 0 | 0 | 0 | 0.97 | 6 |
| PD7249 | 873 | 1122 | 0 | 0 | 0 | 1465 | 1124 | 0 | 0 | 0 | 0 | 0 | 0.99 | 2 |
| PD7250 | 645 | 0 | 5670 | 1235 | 0 | 0 | 340 | 0 | 0 | 0 | 0 | 0 | 0.94 | 6 |
| PD7304 | 513 | 191 | 0 | 683 | 0 | 510 | 106 | 0 | 0 | 0 | 0 | 0 | 0.95 | 3 |
| PD7305 | 1579 | 420 | 0 | 1521 | 0 | 0 | 449 | 0 | 787 | 0 | 0 | 0 | 0.96 | 3 |
| PD7306 | 1569 | 833 | 0 | 2422 | 0 | 1010 | 532 | 0 | 497 | 0 | 0 | 0 | 0.94 | 3 |
| PD7307 | 0 | 3282 | 0 | 0 | 0 | 0 | 5142 | 0 | 0 | 0 | 0 | 0 | 0.99 | 11 |
| PD7316 | 493 | 67 | 3598 | 0 | 0 | 1145 | 344 | 0 | 0 | 0 | 0 | 0 | 0.95 | 6 |
| PD7321 | 586 | 124 | 3131 | 1188 | 0 | 0 | 1026 | 0 | 0 | 0 | 0 | 0 | 0.98 | 6 |
| PD7322 | 731 | 48 | 0 | 773 | 0 | 0 | 0 | 0 | 463 | 0 | 0 | 0 | 0.95 | 3 |
| PD7341 | 885 | 300 | 0 | 946 | 0 | 0 | 634 | 0 | 325 | 0 | 0 | 0 | 0.98 | 3 |
| PD7344 | 171 | 0 | 0 | 339 | 0 | 125 | 57 | 0 | 30 | 0 | 0 | 0 | 0.86 | 3 |

| | | | | | | | | | | | | | | |
|---|---|---|---|---|---|---|---|---|---|---|---|---|---|---|
| PD7426 | 576 | 0 | 10544 | 2364 | 0 | 0 | 5013 | 0 | 0 | 0 | 0 | 0 | 0.98 | 6 |
| PD7428 | 573 | 0 | 8516 | 1174 | 0 | 4068 | 1619 | 0 | 0 | 0 | 0 | 0 | 0.93 | 6 |
| PD8609 | 998 | 2838 | 0 | 0 | 0 | 0 | 1251 | 0 | 0 | 0 | 0 | 0 | 1.00 | 8 |
| PD8610 | 206 | 259 | 1704 | 1160 | 0 | 1248 | 521 | 0 | 0 | 0 | 0 | 0 | 0.97 | 6 |
| PD8611 | 398 | 0 | 3562 | 1838 | 0 | 0 | 1030 | 0 | 0 | 0 | 0 | 0 | 0.97 | 6 |
| PD8612 | 968 | 266 | 0 | 0 | 0 | 644 | 90 | 0 | 0 | 0 | 0 | 0 | 0.96 | 9 |
| PD8614 | 349 | 100 | 0 | 775 | 0 | 215 | 0 | 0 | 0 | 0 | 0 | 0 | 0.92 | 3 |
| PD8615 | 611 | 589 | 0 | 1367 | 0 | 0 | 1703 | 0 | 0 | 0 | 0 | 0 | 0.99 | 7 |
| PD8617 | 499 | 85 | 0 | 1315 | 0 | 0 | 0 | 0 | 0 | 0 | 0 | 0 | 0.94 | 3 |
| PD8618 | 401 | 53 | 0 | 852 | 0 | 157 | 43 | 0 | 0 | 0 | 0 | 0 | 0.95 | 3 |
| PD8619 | 927 | 129 | 0 | 1385 | 0 | 759 | 371 | 0 | 0 | 0 | 0 | 0 | 0.95 | 3 |
| PD8620 | 536 | 904 | 0 | 0 | 0 | 0 | 1217 | 0 | 0 | 0 | 0 | 0 | 0.99 | 11 |
| PD8621 | 598 | 0 | 4130 | 1827 | 0 | 890 | 617 | 0 | 0 | 0 | 0 | 0 | 0.95 | 6 |
| PD8622 | 377 | 96 | 0 | 973 | 0 | 294 | 69 | 0 | 0 | 0 | 0 | 0 | 0.93 | 3 |
| PD8623 | 423 | 287 | 0 | 1032 | 0 | 278 | 0 | 0 | 0 | 0 | 0 | 0 | 0.95 | 3 |
| PD8652 | 1082 | 287 | 7935 | 2828 | 0 | 2062 | 1662 | 0 | 0 | 0 | 0 | 0 | 0.97 | 6 |
| PD8660 | 2417 | 2626 | 9140 | 7201 | 0 | 0 | 3421 | 1323 | 0 | 0 | 0 | 0 | 0.96 | 6 |
| PD8828 | 520 | 375 | 0 | 839 | 0 | 0 | 327 | 0 | 0 | 0 | 0 | 0 | 0.99 | 3 |

| | | | | | | | | | | | | | | |
|---|---|---|---|---|---|---|---|---|---|---|---|---|---|---|
| PD8830 | 1567 | 0 | 3302 | 1292 | 0 | 1781 | 304 | 0 | 0 | 0 | 0 | 0 | 0.95 | 6 |
| PD8832 | 931 | 0 | 14022 | 3194 | 0 | 6620 | 1296 | 346 | 0 | 0 | 0 | 0 | 0.95 | 6 |
| PD8964 | 666 | 0 | 5128 | 2496 | 0 | 1960 | 901 | 0 | 0 | 0 | 0 | 0 | 0.96 | 6 |
| PD8965 | 443 | 524 | 0 | 1848 | 0 | 1177 | 459 | 0 | 0 | 0 | 0 | 0 | 0.97 | 3 |
| PD8969 | 569 | 410 | 3010 | 1729 | 0 | 0 | 780 | 0 | 322 | 0 | 0 | 0 | 0.97 | 6 |
| PD8973 | 1213 | 1774 | 0 | 0 | 0 | 0 | 2921 | 0 | 0 | 0 | 0 | 0 | 1.00 | 11 |
| PD8977 | 2155 | 186 | 0 | 1171 | 0 | 534 | 0 | 0 | 0 | 0 | 0 | 0 | 0.96 | 3 |
| PD8978 | 984 | 135 | 3381 | 1681 | 0 | 0 | 489 | 0 | 0 | 0 | 0 | 0 | 0.92 | 6 |
| PD8979 | 951 | 346 | 0 | 2085 | 0 | 440 | 215 | 0 | 0 | 0 | 0 | 0 | 0.94 | 3 |
| PD8980 | 749 | 0 | 3704 | 3866 | 0 | 5337 | 1803 | 0 | 0 | 0 | 0 | 0 | 0.91 | 6 |
| PD8981 | 504 | 143 | 0 | 620 | 0 | 386 | 95 | 0 | 0 | 0 | 0 | 0 | 0.94 | 3 |
| PD8982 | 1341 | 0 | 11357 | 3246 | 0 | 0 | 5613 | 0 | 0 | 0 | 0 | 0 | 0.96 | 6 |
| PD8984 | 1038 | 200 | 6942 | 0 | 0 | 0 | 972 | 0 | 0 | 0 | 0 | 0 | 0.96 | 6 |
| PD8995 | 2654 | 2830 | 0 | 0 | 0 | 0 | 2102 | 0 | 0 | 0 | 0 | 0 | 0.99 | 8 |
| PD8996 | 1229 | 146 | 0 | 937 | 0 | 807 | 111 | 0 | 0 | 0 | 0 | 0 | 0.95 | 3 |
| PD8997 | 961 | 149 | 0 | 1303 | 0 | 0 | 226 | 0 | 192 | 0 | 0 | 0 | 0.97 | 3 |
| PD8998 | 0 | 1237 | 0 | 0 | 0 | 1214 | 2318 | 0 | 0 | 0 | 0 | 0 | 1.00 | 11 |
| PD8999 | 442 | 70 | 0 | 614 | 0 | 221 | 0 | 0 | 0 | 0 | 0 | 0 | 0.95 | 3 |

| | | | | | | | | | | | | | | |
|---|---|---|---|---|---|---|---|---|---|---|---|---|---|---|
| PD9000 | 400 | 440 | 3809 | 1650 | 0 | 2488 | 723 | 0 | 0 | 0 | 0 | 0 | 0.97 | 6 |
| PD9001 | 705 | 115 | 0 | 1885 | 0 | 0 | 0 | 0 | 0 | 0 | 0 | 0 | 0.93 | 3 |
| PD9002 | 476 | 331 | 0 | 4460 | 0 | 2076 | 330 | 302 | 501 | 0 | 0 | 0 | 0.94 | 3 |
| PD9004 | 470 | 0 | 4480 | 2833 | 0 | 1276 | 1566 | 0 | 0 | 0 | 0 | 0 | 0.94 | 6 |
| PD9009 | 920 | 2948 | 0 | 914 | 0 | 1037 | 8967 | 424 | 0 | 0 | 0 | 0 | 1.00 | 11 |
| PD9063 | 0 | 30448 | 0 | 0 | 0 | 0 | 19597 | 0 | 0 | 0 | 0 | 0 | 1.00 | 8 |
| PD9064 | 735 | 0 | 4548 | 2357 | 0 | 3731 | 980 | 0 | 0 | 0 | 0 | 0 | 0.94 | 6 |
| PD9065 | 531 | 297 | 0 | 761 | 0 | 0 | 0 | 0 | 621 | 0 | 0 | 0 | 0.98 | 3 |
| PD9067 | 533 | 226 | 0 | 468 | 0 | 216 | 111 | 0 | 0 | 0 | 0 | 0 | 0.96 | 3 |
| PD9193 | 435 | 50 | 438 | 400 | 0 | 0 | 136 | 0 | 223 | 0 | 0 | 0 | 0.94 | 4 |
| PD9464 | 661 | 494 | 0 | 2238 | 0 | 1152 | 396 | 0 | 310 | 0 | 0 | 0 | 0.96 | 3 |
| PD9467 | 1035 | 218 | 0 | 739 | 0 | 471 | 0 | 0 | 0 | 0 | 0 | 0 | 0.96 | 3 |
| PD9539 | 2349 | 121 | 0 | 0 | 0 | 930 | 180 | 0 | 0 | 0 | 0 | 0 | 0.98 | 9 |
| PD9541 | 1688 | 369 | 0 | 0 | 0 | 649 | 0 | 0 | 0 | 0 | 0 | 0 | 0.97 | 9 |
| PD9544 | 437 | 101 | 0 | 568 | 0 | 283 | 0 | 0 | 0 | 0 | 0 | 0 | 0.94 | 3 |
| PD9567 | 943 | 244 | 0 | 1113 | 0 | 951 | 261 | 0 | 0 | 0 | 0 | 0 | 0.97 | 3 |
| PD9568 | 1513 | 0 | 13307 | 5318 | 0 | 5527 | 4420 | 310 | 0 | 0 | 0 | 0 | 0.95 | 6 |
| PD9569 | 632 | 0 | 0 | 1157 | 0 | 253 | 99 | 0 | 0 | 0 | 0 | 0 | 0.95 | 3 |

| | | | | | | | | | | | | | | |
|---|---|---|---|---|---|---|---|---|---|---|---|---|---|---|
| PD9570 | 498 | 301 | 0 | 358 | 0 | 249 | 0 | 0 | 0 | 0 | 0 | 0 | 0.98 | 3 |
| PD9571 | 700 | 97 | 1603 | 2083 | 0 | 0 | 217 | 0 | 0 | 0 | 0 | 0 | 0.91 | 4 |
| PD9572 | 584 | 181 | 0 | 2134 | 0 | 0 | 1184 | 0 | 0 | 0 | 0 | 0 | 0.97 | 7 |
| PD9573 | 821 | 0 | 0 | 565 | 0 | 230 | 0 | 0 | 0 | 0 | 0 | 0 | 0.95 | 3 |
| PD9574 | 594 | 61 | 0 | 621 | 0 | 358 | 0 | 0 | 0 | 0 | 0 | 0 | 0.95 | 3 |
| PD9575 | 506 | 179 | 0 | 4136 | 0 | 669 | 771 | 239 | 0 | 0 | 0 | 0 | 0.90 | 3 |
| PD9576 | 713 | 0 | 8169 | 3868 | 0 | 4293 | 795 | 0 | 0 | 0 | 0 | 0 | 0.95 | 6 |
| PD9577 | 392 | 67 | 0 | 842 | 0 | 323 | 0 | 0 | 0 | 0 | 0 | 0 | 0.93 | 3 |
| PD9578 | 284 | 40 | 0 | 615 | 0 | 149 | 0 | 0 | 0 | 0 | 0 | 0 | 0.90 | 3 |
| PD9579 | 397 | 73 | 0 | 803 | 0 | 279 | 82 | 0 | 0 | 0 | 0 | 0 | 0.94 | 3 |
| PD9581 | 423 | 52 | 0 | 930 | 0 | 211 | 0 | 0 | 0 | 0 | 0 | 0 | 0.93 | 3 |
| PD9582 | 1072 | 171 | 830 | 0 | 0 | 525 | 142 | 0 | 0 | 0 | 0 | 0 | 0.96 | 9 |
| PD9584 | 746 | 132 | 0 | 1466 | 0 | 341 | 68 | 0 | 0 | 0 | 0 | 0 | 0.97 | 3 |
| PD9585 | 806 | 0 | 3856 | 1740 | 0 | 2682 | 1200 | 0 | 0 | 0 | 0 | 0 | 0.97 | 6 |
| PD9589 | 425 | 177 | 0 | 568 | 0 | 243 | 0 | 0 | 0 | 0 | 0 | 0 | 0.97 | 3 |
| PD9591 | 460 | 81 | 0 | 596 | 0 | 159 | 0 | 0 | 0 | 0 | 0 | 0 | 0.96 | 3 |
| PD9592 | 762 | 0 | 6015 | 0 | 0 | 1204 | 515 | 0 | 0 | 0 | 0 | 0 | 0.96 | 6 |
| PD9593 | 424 | 49 | 0 | 992 | 0 | 0 | 39 | 0 | 113 | 0 | 0 | 0 | 0.94 | 3 |

| | | | | | | | | | | | | | | |
|---|---|---|---|---|---|---|---|---|---|---|---|---|---|---|
| PD9595 | 943 | 148 | 4089 | 1698 | 0 | 2492 | 352 | 0 | 0 | 0 | 0 | 0 | 0.95 | 6 |
| PD9597 | 557 | 1438 | 0 | 0 | 0 | 0 | 916 | 0 | 0 | 0 | 0 | 0 | 1.00 | 8 |
| PD9599 | 1151 | 216 | 0 | 1632 | 0 | 0 | 296 | 0 | 0 | 0 | 0 | 0 | 0.97 | 3 |
| PD9600 | 609 | 45 | 0 | 869 | 0 | 439 | 57 | 0 | 0 | 0 | 0 | 0 | 0.90 | 3 |
| PD9604 | 0 | 0 | 0 | 0 | 1530 | 0 | 221 | 0 | 0 | 0 | 8918 | 0 | 0.95 | 10 |
| PD9605 | 886 | 158 | 0 | 1329 | 0 | 611 | 88 | 0 | 301 | 0 | 0 | 0 | 0.95 | 3 |
| PD9606 | 462 | 39 | 0 | 551 | 0 | 170 | 0 | 0 | 0 | 0 | 0 | 0 | 0.95 | 3 |
| PD9694 | 941 | 0 | 0 | 550 | 0 | 0 | 65 | 0 | 156 | 0 | 0 | 0 | 0.97 | 3 |
| PD9696 | 463 | 93 | 3026 | 0 | 0 | 1499 | 784 | 0 | 0 | 0 | 0 | 0 | 0.98 | 6 |
| PD9702 | 700 | 0 | 5968 | 0 | 0 | 0 | 1193 | 0 | 0 | 0 | 0 | 0 | 0.97 | 6 |
| PD9752 | 1147 | 331 | 0 | 972 | 0 | 680 | 346 | 0 | 0 | 0 | 0 | 0 | 0.97 | 3 |
| PD9754 | 735 | 78 | 0 | 649 | 0 | 435 | 0 | 0 | 0 | 0 | 0 | 0 | 0.95 | 3 |
| PD9755 | 527 | 137 | 0 | 1046 | 0 | 0 | 0 | 0 | 0 | 0 | 0 | 0 | 0.94 | 3 |
| PD9756 | 855 | 1916 | 0 | 0 | 0 | 0 | 1441 | 0 | 0 | 0 | 0 | 0 | 0.99 | 8 |
| PD9759 | 676 | 176 | 0 | 1078 | 0 | 262 | 0 | 0 | 0 | 0 | 0 | 0 | 0.95 | 3 |
| PD9760 | 1178 | 633 | 0 | 1559 | 0 | 932 | 704 | 0 | 0 | 0 | 0 | 0 | 0.99 | 3 |
| PD9761 | 746 | 141 | 0 | 1037 | 0 | 0 | 0 | 0 | 179 | 0 | 0 | 0 | 0.95 | 3 |
| PD9842 | 705 | 110 | 0 | 2242 | 0 | 0 | 144 | 0 | 0 | 0 | 0 | 0 | 0.94 | 3 |

| | | | | | | | | | | | | | | |
|---|---|---|---|---|---|---|---|---|---|---|---|---|---|---|
| PD9843 | 400 | 36 | 0 | 1211 | 0 | 279 | 0 | 0 | 84 | 0 | 0 | 0 | 0.92 | 3 |
| PD9844 | 95 | 20 | 0 | 285 | 0 | 61 | 0 | 0 | 46 | 0 | 0 | 0 | 0.87 | 3 |
| PD9845 | 456 | 326 | 0 | 2208 | 0 | 0 | 157 | 0 | 0 | 0 | 0 | 0 | 0.95 | 3 |
| PD9847 | 471 | 49 | 0 | 1369 | 0 | 340 | 0 | 0 | 0 | 0 | 0 | 0 | 0.93 | 3 |

EP 3 452 937 B1

Table 10C

| sample | chr | start.bp | end.bp | length.bp | number.bps | number.bps.clustered | avgDist.bp | alternative.kataegis |
|---|---|---|---|---|---|---|---|---|
| PD4072a | 18 | 43349588 | 43350533 | 945 | 6 | 0 | 237.5 | |
| PD4072a | 2 | 100755082 | 100762600 | 7518 | 19 | 0 | 412.842105 | |
| PD4072a | 2 | 100771228 | 100776154 | 4926 | 14 | 0 | 368.285714 | |
| PD4072a | 22 | 48430430 | 48431300 | 870 | 6 | 0 | 162.333333 | |
| PD4225a | 8 | 18542550 | 18550128 | 7578 | 12 | 0 | 707.833333 | |
| PD4225a | 8 | 26800058 | 26803672 | 3614 | 18 | 0 | 208.555556 | |
| PD4267a | 21 | 37284933 | 37289262 | 4329 | 6 | 0 | 735.833333 | |
| PD4315a | 1 | 168586753 | 168588546 | 1793 | 13 | 0 | 156 | |
| PD4315a | 16 | 86088169 | 86089331 | 1162 | 11 | 0 | 107.909091 | |
| PD4315a | 17 | 51294462 | 51298344 | 3882 | 16 | 0 | 261.875 | |
| PD4315a | 20 | 52957602 | 52958338 | 736 | 9 | 0 | 135 | |
| PD4315a | 6 | 145760793 | 145764106 | 3313 | 8 | 0 | 469.5 | |
| PD4315a | 6 | 162598392 | 162605465 | 7073 | 18 | 0 | 442 | |
| PD4604a | 17 | 11661583 | 11661814 | 231 | 8 | 0 | 31.375 | |
| PD4604a | 17 | 22106497 | 22106956 | 459 | 7 | 0 | 74.2857143 | |
| PD4604a | 4 | 61800897 | 61802159 | 1262 | 7 | 0 | 204.857143 | |
| PD4604a | 4 | 81641549 | 81642100 | 551 | 6 | 0 | 113 | |
| PD4604a | 7 | 109420982 | 109421906 | 924 | 10 | 0 | 92.7 | |
| PD4605a | 11 | 95640304 | 95644320 | 4016 | 18 | 0 | 230.888889 | |
| PD4605a | 18 | 4977333 | 4980907 | 3574 | 21 | 0 | 177.666667 | |
| PD4605a | 7 | 116317977 | 116323931 | 5954 | 10 | 0 | 636.3 | |
| PD4607a | 14 | 34870751 | 34875166 | 4415 | 7 | 0 | 631.428571 | |
| PD4607a | 14 | 38081500 | 38083410 | 1910 | 6 | 0 | 334.166667 | |
| PD4607a | 19 | 46303307 | 46307819 | 4512 | 6 | 0 | 931.166667 | |
| PD4607a | 8 | 128897164 | 128901876 | 4712 | 10 | 0 | 685.4 | |
| PD4607a | 9 | 73066853 | 73074793 | 7940 | 21 | 0 | 401.047619 | |

(continued)

| sample | chr | start.bp | end.bp | length.bp | number.bps | number.bps.clustered | avgDist.bp | alternative.kataegis |
|---|---|---|---|---|---|---|---|---|
| PD4872a | 13 | 60381176 | 60383318 | 2142 | 31 | 0 | 70.516129 | |
| PD4951a | 11 | 69893428 | 69895141 | 1713 | 8 | 0 | 385 | |
| PD4952a | 11 | 35445711 | 35448563 | 2852 | 16 | 0 | 243.5625 | |
| PD4952a | 13 | 58094099 | 58094893 | 794 | 7 | 0 | 132.142857 | |
| PD4952a | 14 | 47327376 | 47328414 | 1038 | 8 | 0 | 138.25 | |
| PD4952a | 18 | 66248743 | 66254682 | 5939 | 9 | 0 | 665.333333 | |
| PD4952a | 8 | 105949942 | 105959913 | 9971 | 15 | 0 | 670.466667 | |
| PD4957a | 11 | 38882781 | 38883361 | 580 | 8 | 0 | 72.875 | |
| PD4957a | 11 | 65997884 | 65998552 | 668 | 14 | 0 | 53.8571429 | |
| PD4959a | 11 | 15557951 | 15559277 | 1326 | 13 | 0 | 133.923077 | |
| PD4965a | 1 | 38152429 | 38154910 | 2481 | 7 | 0 | 384.714286 | |
| PD4967a | 1 | 20093630 | 20096843 | 3213 | 7 | 0 | 471.285714 | |
| PD4967a | 19 | 9900857 | 9901894 | 1037 | 6 | 0 | 181.333333 | |
| PD4968a | 10 | 79958727 | 79959348 | 621 | 6 | 0 | 155.333333 | |
| PD4968a | 17 | 51819548 | 51822158 | 2610 | 6 | 0 | 820.5 | |
| PD4968a | 20 | 20690202 | 20690512 | 310 | 7 | 0 | 60.5714286 | |
| PD4970a | 1 | 198939383 | 198939756 | 373 | 7 | 0 | 67.7142857 | |
| PD4970a | 19 | 5674781 | 5676856 | 2075 | 8 | 0 | 290.375 | |
| PD4970a | 19 | 12504910 | 12509978 | 5068 | 19 | 0 | 289.210526 | |
| PD4970a | 4 | 169917595 | 169918213 | 618 | 6 | 0 | 106.333333 | |
| PD4971a | 15 | 60207154 | 60211015 | 3861 | 18 | 0 | 230.555556 | |
| PD4971a | 8 | 57846436 | 57848400 | 1964 | 6 | 0 | 350.333333 | |
| PD4971a | 9 | 87963711 | 87964617 | 906 | 7 | 0 | 138.142857 | |
| PD4976a | 11 | 79618021 | 79618513 | 492 | 7 | 0 | 185 | |
| PD4976a | 3 | 35861083 | 35863033 | 1950 | 11 | 0 | 177.363636 | |
| PD5937a | 1 | 1262932 | 1263678 | 746 | 6 | 0 | 225.333333 | |

(continued)

| sample | chr | start.bp | end.bp | length.bp | number.bps | number.bps.clustered | avgDist.bp | alternative.kataegis |
|---|---|---|---|---|---|---|---|---|
| PD5937a | 1 | 26846158 | 26846723 | 565 | 8 | 0 | 83.375 | |
| PD5937a | 1 | 27607491 | 27609264 | 1773 | 8 | 0 | 229.25 | |
| PD5937a | 1 | 45600276 | 45601551 | 1275 | 8 | 0 | 159.75 | |
| PD5937a | 10 | 9194145 | 9197525 | 3380 | 8 | 0 | 496.75 | |
| PD5937a | 14 | 91426228 | 91428259 | 2031 | 7 | 0 | 317.285714 | |
| PD5937a | 17 | 29723619 | 29725813 | 2194 | 6 | 0 | 370.333333 | |
| PD5937a | 19 | 40578957 | 40580370 | 1413 | 6 | 0 | 248 | |
| PD5937a | 2 | 181545233 | 181548021 | 2788 | 8 | 0 | 412 | |
| PD5937a | 22 | 46534752 | 46538018 | 3266 | 9 | 0 | 383 | |
| PD5937a | 4 | 106936023 | 106938579 | 2556 | 6 | 0 | 469.5 | |
| PD5937a | 8 | 67367782 | 67369700 | 1918 | 8 | 0 | 252.125 | |
| PD5937a | 9 | 130262328 | 130264953 | 2625 | 7 | 0 | 422.285714 | |
| PD5946a | 11 | 2075077 | 2075922 | 845 | 6 | 0 | 152.833333 | yes |
| PD5946a | 12 | 47842464 | 47844367 | 1903 | 6 | 0 | 373.166667 | |
| PD5946a | 15 | 35186338 | 35187044 | 706 | 6 | 0 | 218.833333 | |
| PD5946a | 17 | 70801752 | 70803186 | 1434 | 17 | 0 | 86.2941176 | |
| PD5947a | 14 | 30981316 | 30982281 | 965 | 11 | 0 | 117.090909 | |
| PD5947a | 15 | 66530866 | 66534292 | 3426 | 18 | 0 | 194.944444 | |
| PD5947a | 15 | 95492886 | 95498316 | 5430 | 6 | 0 | 928.166667 | |
| PD5947a | 15 | 97596785 | 97602242 | 5457 | 6 | 0 | 911.333333 | |
| PD5947a | 15 | 98070185 | 98072320 | 2135 | 8 | 0 | 276 | |
| PD5947a | 15 | 99039659 | 99040130 | 471 | 7 | 0 | 121.857143 | |
| PD5947a | 17 | 31349834 | 31352488 | 2654 | 9 | 0 | 540.111111 | |
| PD5947a | 17 | 31957758 | 31958054 | 296 | 7 | 0 | 48.5714286 | |
| PD5947a | 17 | 47258193 | 47265099 | 6906 | 11 | 0 | 630.727273 | |
| PD5951a | 11 | 58790673 | 58793223 | 2550 | 7 | 0 | 418.142857 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD5951a | 16 | 71758658 | 71759300 | 642 | 6 | 0 | 117.166667 | |
| PD5951a | 8 | 83091192 | 83091449 | 257 | 6 | 0 | 50.6666667 | |
| PD5956a | 18 | 77756746 | 77770050 | 13304 | 15 | 0 | 897.666667 | |
| PD5959a | 1 | 33813433 | 33831708 | 18275 | 26 | 0 | 734.615385 | |
| PD5959a | 1 | 34097615 | 34100058 | 2443 | 7 | 0 | 432.714286 | |
| PD5959a | 1 | 36094464 | 36095849 | 1385 | 6 | 0 | 235.833333 | |
| PD5959a | 1 | 39877402 | 39878586 | 1184 | 7 | 0 | 253.714286 | |
| PD5959a | 1 | 42259799 | 42260150 | 351 | 9 | 0 | 40.2222222 | |
| PD5959a | 1 | 42432022 | 42432421 | 399 | 6 | 0 | 68.3333333 | |
| PD5959a | 1 | 50802923 | 50803878 | 955 | 8 | 0 | 141.5 | |
| PD5959a | 1 | 55429188 | 55432057 | 2869 | 11 | 0 | 266.545455 | |
| PD5959a | 12 | 74477276 | 74477670 | 394 | 11 | 0 | 42.0909091 | |
| PD5959a | 12 | 99517172 | 99518961 | 1789 | 13 | 0 | 148.769231 | |
| PD5959a | 12 | 115515308 | 115515956 | 648 | 6 | 0 | 186.333333 | |
| PD5959a | 12 | 132169506 | 132170562 | 1056 | 19 | 0 | 57.1578947 | |
| PD5959a | 17 | 76033255 | 76039154 | 5899 | 12 | 0 | 498.5 | |
| PD5959a | 6 | 24403024 | 24404767 | 1743 | 13 | 0 | 135.692308 | |
| PD5959a | 8 | 10810197 | 10810593 | 396 | 6 | 0 | 110.333333 | |
| PD5959a | 8 | 23465649 | 23470004 | 4355 | 9 | 0 | 603.333333 | |
| PD5959a | X | 3230045 | 3230725 | 680 | 7 | 0 | 110.142857 | |
| PD5961a | 6 | 99840147 | 99840452 | 305 | 8 | 0 | 72.75 | yes |
| PD5964a | 20 | 52130737 | 52131930 | 1193 | 6 | 0 | 200.166667 | |
| PD6016a2 | 3 | 70675957 | 70683706 | 7749 | 19 | 0 | 412.052632 | |
| PD6042a | 10 | 59368439 | 59369507 | 1068 | 9 | 0 | 129.888889 | |
| PD6042a | 10 | 69846469 | 69847124 | 655 | 8 | 0 | 91.5 | |
| PD6042a | 10 | 71745647 | 71747868 | 2221 | 6 | 0 | 397.333333 | |

(continued)

| sample | chr | start.bp | end.bp | length.bp | number.bps | number.bps.clustered | avgDist.bp | alternative.kataegis |
|---|---|---|---|---|---|---|---|---|
| PD6042a | 8 | 118272247 | 118273507 | 1260 | 6 | 0 | 213.666667 | |
| PD6043a | 12 | 101999701 | 102002431 | 2730 | 8 | 0 | 345.625 | |
| PD6043a | 13 | 21112503 | 21116869 | 4366 | 13 | 0 | 338 | |
| PD6043a | 13 | 21144904 | 21149432 | 4528 | 15 | 0 | 303.4 | |
| PD6043a | 13 | 21196785 | 21216366 | 19581 | 65 | 0 | 302.153846 | |
| PD6043a | 14 | 95753420 | 95754632 | 1212 | 6 | 0 | 263.833333 | |
| PD6043a | 6 | 139500841 | 139503531 | 2690 | 6 | 0 | 502.166667 | |
| PD6044a | 1 | 120480193 | 120484348 | 4155 | 6 | 0 | 740.166667 | |
| PD6044a | 11 | 38898058 | 38901130 | 3072 | 10 | 0 | 311.8 | |
| PD6044a | 11 | 76287274 | 76287844 | 570 | 6 | 0 | 138.333333 | |
| PD6044a | 11 | 77065278 | 77065936 | 658 | 10 | 0 | 134.9 | |
| PD6045a | 11 | 70796681 | 70800148 | 3467 | 7 | 0 | 522.285714 | |
| PD6422a | 10 | 81071879 | 81076836 | 4957 | 8 | 0 | 622.375 | |
| PD6422a | 10 | 96618615 | 96621508 | 2893 | 9 | 0 | 427.333333 | |
| PD6422a | 12 | 20184162 | 20186040 | 1878 | 13 | 0 | 145.769231 | |
| PD6422a | 12 | 68290245 | 68292462 | 2217 | 12 | 0 | 221.75 | |
| PD6422a | 20 | 55912235 | 55913476 | 1241 | 7 | 0 | 227.285714 | |
| PD6422a | 21 | 25677230 | 25677654 | 424 | 6 | 0 | 74.5 | |
| PD6422a | 21 | 25755745 | 25756298 | 553 | 6 | 0 | 93 | |
| PD6422a | 22 | 19813003 | 19813376 | 373 | 7 | 0 | 65.2857143 | |
| PD6422a | 7 | 149252889 | 149254627 | 1738 | 15 | 0 | 122.6 | |
| PD6422a | 8 | 10600171 | 10601443 | 1272 | 8 | 0 | 170.875 | |
| PD6422a | 8 | 22027778 | 22034639 | 6861 | 12 | 0 | 610.583333 | |
| PD6422a | 9 | 17470456 | 17470995 | 539 | 8 | 0 | 96.5 | |
| PD6422a | 9 | 33375671 | 33377199 | 1528 | 8 | 0 | 219.625 | |
| PD6719a | 17 | 27307429 | 27308396 | 967 | 8 | 0 | 131.125 | |

| sample | chr | start.bp | end.bp | length.bp | number.bps | number.bps.clustered | avgDist.bp | alternative.kataegis |
|---|---|---|---|---|---|---|---|---|
| PD6721a | 11 | 58385857 | 58392887 | 7030 | 12 | 0 | 593.916667 | |
| PD6721a | 11 | 79353465 | 79360208 | 6743 | 8 | 0 | 858.125 | |
| PD6721a | 14 | 28223081 | 28224091 | 1010 | 7 | 0 | 183.714286 | |
| PD6721a | 14 | 38641421 | 38642842 | 1421 | 7 | 0 | 224.714286 | |
| PD6721a | 14 | 47392479 | 47393387 | 908 | 7 | 0 | 200.714286 | |
| PD7206a | 18 | 23060232 | 23060880 | 648 | 6 | 0 | 132 | |
| PD7206a | 6 | 39168217 | 39179275 | 11058 | 23 | 0 | 481.608696 | |
| PD7206a | 6 | 42230525 | 42231908 | 1383 | 8 | 0 | 181 | |
| PD7206a | 6 | 48141031 | 48141508 | 477 | 8 | 0 | 62.25 | |
| PD7207a | 6 | 19237884 | 19238237 | 353 | 9 | 0 | 50.2222222 | |
| PD7209a | 1 | 56372939 | 56384067 | 11128 | 32 | 0 | 391.21875 | |
| PD7219a | 1 | 28033570 | 28038489 | 4919 | 12 | 0 | 414.166667 | |
| PD7219a | 1 | 154164212 | 154165781 | 1569 | 9 | 0 | 395.333333 | |
| PD7219a | 17 | 61922800 | 61927088 | 4288 | 7 | 0 | 757.714286 | |
| PD7219a | 22 | 32737654 | 32742043 | 4389 | 6 | 0 | 788 | |
| PD7219a | 3 | 54480453 | 54483102 | 2649 | 6 | 0 | 805.166667 | |
| PD7238a | 4 | 54575713 | 54579366 | 3653 | 18 | 0 | 230.666667 | |
| PD7240a | 1 | 197160182 | 197163241 | 3059 | 13 | 0 | 280.692308 | |
| PD7240a | 19 | 43693906 | 43694101 | 195 | 6 | 0 | 64 | |
| PD7240a | X | 53632798 | 53635368 | 2570 | 11 | 0 | 238.272727 | |
| PD7243a | 11 | 90416854 | 90425147 | 8293 | 18 | 0 | 460.833333 | |
| PD7243a | 11 | 91034978 | 91035568 | 590 | 8 | 0 | 75.5 | |
| PD7243a | 11 | 95333352 | 95341930 | 8578 | 13 | 0 | 662.615385 | |
| PD8609a | 11 | 17035538 | 17044242 | 8704 | 11 | 0 | 819.272727 | |
| PD8609a | 17 | 27937790 | 27938590 | 800 | 6 | 0 | 260 | |
| PD8610a | 14 | 54545002 | 54545226 | 224 | 7 | 0 | 34 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD8610a | 6 | 168169680 | 168171653 | 1973 | 9 | 0 | 229.444444 | |
| PD8614a | 18 | 49122588 | 49123173 | 585 | 7 | 0 | 90.5714286 | |
| PD8620a | 12 | 62515146 | 62516210 | 1064 | 7 | 0 | 168.428571 | |
| PD8620a | 16 | 391203 | 394338 | 3135 | 6 | 0 | 862.666667 | |
| PD8620a | 2 | 136498731 | 136501472 | 2741 | 6 | 0 | 529 | |
| PD8620a | 20 | 25303967 | 25310301 | 6334 | 8 | 0 | 803.5 | |
| PD8973a | 2 | 213156923 | 213161285 | 4362 | 6 | 0 | 785.166667 | |
| PD8977a | 1 | 22112698 | 22116450 | 3752 | 6 | 0 | 720.5 | |
| PD8977a | 11 | 70315051 | 70319138 | 4087 | 8 | 0 | 515.375 | |
| PD8977a | 14 | 64243725 | 64249390 | 5665 | 13 | 0 | 578.769231 | |
| PD8978a | 10 | 119997854 | 120000971 | 3117 | 21 | 0 | 176.380952 | |
| PD8979a | 14 | 40265762 | 40266446 | 684 | 10 | 0 | 125.1 | |
| PD8979a | 17 | 52699285 | 52699915 | 630 | 8 | 0 | 127.375 | |
| PD8979a | 17 | 61479490 | 61481950 | 2460 | 14 | 0 | 179.928571 | |
| PD8979a | 18 | 8223958 | 8226908 | 2950 | 7 | 0 | 449 | |
| PD8979a | 18 | 9990255 | 9992251 | 1996 | 8 | 0 | 251 | |
| PD8979a | 3 | 8831221 | 8832025 | 804 | 10 | 0 | 96.2 | |
| PD8979a | 4 | 107738532 | 107739710 | 1178 | 6 | 0 | 212 | |
| PD8979a | 6 | 52319119 | 52320879 | 1760 | 7 | 0 | 543 | |
| PD8979a | 6 | 54000300 | 54001784 | 1484 | 8 | 0 | 349.5 | |
| PD8979a | 6 | 79425945 | 79429371 | 3426 | 11 | 0 | 422.636364 | |
| PD8979a | 8 | 85749040 | 85754692 | 5652 | 6 | 0 | 948.833333 | |
| PD8980a | 14 | 30265994 | 30266391 | 397 | 8 | 0 | 277.625 | |
| PD8980a | 14 | 40988989 | 40999620 | 10631 | 21 | 0 | 527.47619 | |
| PD8980a | 20 | 34877701 | 34880344 | 2643 | 6 | 0 | 453.5 | |
| PD8980a | 20 | 51198172 | 51200805 | 2633 | 12 | 0 | 225.833333 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD8980a | 20 | 53235894 | 53242372 | 6478 | 14 | 0 | 664.714286 | |
| PD8980a | 21 | 42122710 | 42125230 | 2520 | 16 | 0 | 165.375 | |
| PD8980a | 21 | 42137184 | 42141221 | 4037 | 24 | 0 | 181.833333 | |
| PD8980a | 5 | 3872219 | 3872609 | 390 | 7 | 0 | 67.7142857 | |
| PD8980a | 5 | 5188080 | 5189100 | 1020 | 6 | 0 | 174.333333 | |
| PD8980a | 5 | 7389259 | 7395726 | 6467 | 22 | 0 | 296.045455 | |
| PD8980a | 5 | 7448686 | 7458014 | 9328 | 37 | 0 | 258.189189 | |
| PD8980a | 5 | 7593099 | 7593540 | 441 | 9 | 0 | 60 | |
| PD8980a | 5 | 7634387 | 7636176 | 1789 | 8 | 0 | 235 | |
| PD8980a | 5 | 8883233 | 8885722 | 2489 | 18 | 0 | 139 | |
| PD8980a | 5 | 10704261 | 10705818 | 1557 | 12 | 0 | 163 | |
| PD8980a | 5 | 10781281 | 10787482 | 6201 | 18 | 0 | 347.944444 | |
| PD8980a | 5 | 10853768 | 10855951 | 2183 | 15 | 0 | 146.4 | |
| PD8980a | 5 | 17759519 | 17769220 | 9701 | 34 | 0 | 290.382353 | |
| PD8980a | 5 | 30896355 | 30897911 | 1556 | 9 | 0 | 303.888889 | |
| PD8980a | 5 | 35826036 | 35830267 | 4231 | 19 | 0 | 224.263158 | |
| PD8980a | 5 | 36763538 | 36768046 | 4508 | 19 | 0 | 241.105263 | |
| PD8980a | 5 | 37710283 | 37713787 | 3504 | 8 | 0 | 460.625 | |
| PD8980a | 5 | 37881068 | 37892554 | 11486 | 47 | 0 | 248.553191 | |
| PD8980a | 5 | 38066559 | 38068143 | 1584 | 7 | 0 | 261 | |
| PD8980a | 5 | 38843232 | 38846462 | 3230 | 7 | 0 | 538.714286 | |
| PD8980a | 5 | 40122693 | 40128153 | 5460 | 20 | 0 | 276.9 | |
| PD8980a | 5 | 87138172 | 87143849 | 5677 | 27 | 0 | 262.37037 | |
| PD8980a | 5 | 87150390 | 87150921 | 531 | 14 | 0 | 43.5 | |
| PD8980a | 5 | 94948525 | 94949709 | 1184 | 13 | 0 | 93.3846154 | |
| PD8980a | 5 | 95510057 | 95511638 | 1581 | 11 | 0 | 200.818182 | |

(continued)

| sample | chr | start.bp | end.bp | length.bp | number.bps | number.bps.clustered | avgDist.bp | alternative.kataegis |
|---|---|---|---|---|---|---|---|---|
| PD8980a | 5 | 95687435 | 95688674 | 1239 | 14 | 0 | 98.2142857 | |
| PD8980a | 5 | 96156187 | 96158355 | 2168 | 16 | 0 | 144.6875 | |
| PD8980a | 5 | 96995482 | 96997997 | 2515 | 6 | 0 | 728.666667 | |
| PD8980a | 6 | 65627061 | 65628547 | 1486 | 7 | 0 | 283.142857 | |
| PD8980a | 6 | 65637620 | 65643929 | 6309 | 45 | 0 | 140.688889 | |
| PD8980a | 6 | 67663852 | 67668988 | 5136 | 16 | 0 | 344.875 | |
| PD8980a | 6 | 71257763 | 71258596 | 833 | 7 | 0 | 124.285714 | |
| PD8980a | 6 | 71289506 | 71295210 | 5704 | 10 | 0 | 580.1 | |
| PD8980a | 6 | 71980074 | 71980816 | 742 | 9 | 0 | 86.3333333 | |
| PD8980a | 6 | 71992471 | 71993419 | 948 | 14 | 0 | 77.2857143 | |
| PD8980a | 6 | 73411079 | 73414812 | 3733 | 19 | 0 | 212.421053 | |
| PD8980a | 6 | 73803242 | 73806469 | 3227 | 9 | 0 | 368.222222 | |
| PD8980a | 6 | 75093906 | 75099775 | 5869 | 15 | 0 | 584.6 | |
| PD8980a | 6 | 77604677 | 77609713 | 5036 | 13 | 0 | 387.461538 | |
| PD8980a | 8 | 86313433 | 86314402 | 969 | 15 | 0 | 65.9333333 | |
| PD8980a | X | 91182015 | 91184072 | 2057 | 10 | 0 | 222.2 | |
| PD8981a | 20 | 46864920 | 46867886 | 2966 | 22 | 0 | 142.681818 | |
| PD8981a | 20 | 56324074 | 56325571 | 1497 | 12 | 0 | 145.083333 | |
| PD8981a | 8 | 34337827 | 34346069 | 8242 | 26 | 0 | 320.692308 | |
| PD9000a | 4 | 12237973 | 12238831 | 858 | 8 | 0 | 113.125 | |
| PD9001a | 1 | 166128143 | 166129360 | 1217 | 16 | 0 | 78.5625 | |
| PD9002a | 12 | 90324004 | 90325342 | 1338 | 8 | 0 | 172.625 | |
| PD9002a | 14 | 26776850 | 26777668 | 818 | 10 | 0 | 87.9 | |
| PD9002a | 19 | 4823263 | 4823986 | 723 | 8 | 0 | 93.625 | |
| PD9002a | 19 | 5285125 | 5286631 | 1506 | 9 | 0 | 187.666667 | |
| PD9002a | 20 | 7117427 | 7117928 | 501 | 10 | 0 | 50.6 | |

293

(continued)

| sample | chr | start.bp | end.bp | length.bp | number.bps | number.bps.clustered | avgDist.bp | alternative.kataegis |
|---|---|---|---|---|---|---|---|---|
| PD9063a | 1 | 26677491 | 26681302 | 3811 | 6 | 0 | 779.666667 | |
| PD9063a | 1 | 68049226 | 68049975 | 749 | 8 | 0 | 93.75 | |
| PD9063a | 1 | 153938530 | 153940807 | 2277 | 6 | 0 | 443.5 | |
| PD9063a | 1 | 167990085 | 167991382 | 1297 | 6 | 0 | 427.333333 | |
| PD9063a | 1 | 175549777 | 175550741 | 964 | 9 | 0 | 107.666667 | |
| PD9063a | 1 | 208454456 | 208455602 | 1146 | 10 | 0 | 115.6 | |
| PD9063a | 1 | 231603327 | 231603938 | 611 | 6 | 0 | 152.666667 | |
| PD9063a | 10 | 37597871 | 37608516 | 10645 | 30 | 0 | 373.333333 | |
| PD9063a | 11 | 58540022 | 58541671 | 1649 | 8 | 0 | 227.875 | |
| PD9063a | 11 | 58545287 | 58552161 | 6874 | 13 | 0 | 559.153846 | |
| PD9063a | 11 | 63994543 | 63998887 | 4344 | 6 | 0 | 934 | |
| PD9063a | 11 | 73809822 | 73816815 | 6993 | 16 | 0 | 464.5625 | |
| PD9063a | 11 | 130435264 | 130437383 | 2119 | 7 | 0 | 313.428571 | |
| PD9063a | 14 | 37981917 | 37987632 | 5715 | 6 | 0 | 970.833333 | |
| PD9063a | 14 | 74472799 | 74474882 | 2083 | 6 | 0 | 459.833333 | |
| PD9063a | 16 | 10694766 | 10696976 | 2210 | 8 | 0 | 436.125 | |
| PD9063a | 16 | 75499026 | 75500337 | 1311 | 6 | 0 | 308.833333 | |
| PD9063a | 17 | 54028634 | 54037002 | 8368 | 11 | 0 | 778.181818 | |
| PD9063a | 2 | 100326230 | 100326798 | 568 | 7 | 0 | 113.714286 | |
| PD9063a | 2 | 100532408 | 100536369 | 3961 | 13 | 0 | 305.153846 | |
| PD9063a | 2 | 100542733 | 100546480 | 3747 | 6 | 0 | 745.166667 | |
| PD9063a | 20 | 42114512 | 42116505 | 1993 | 7 | 0 | 294.285714 | |
| PD9063a | 3 | 48653500 | 48657767 | 4267 | 12 | 0 | 374.416667 | |
| PD9063a | 3 | 53420857 | 53423741 | 2884 | 10 | 0 | 300.9 | |
| PD9063a | 3 | 174299510 | 174303369 | 3859 | 7 | 0 | 684.285714 | |
| PD9063a | 6 | 8826739 | 8827103 | 364 | 6 | 0 | 66.3333333 | |

EP 3 452 937 B1

(continued)

| sample | chr | start.bp | end.bp | length.bp | number.bps | number.bps.clustered | avgDist.bp | alternative.kataegis |
|---|---|---|---|---|---|---|---|---|
| PD9063a | 7 | 154517229 | 154522665 | 5436 | 11 | 0 | 494.363636 | |
| PD9063a | 8 | 116801601 | 116804998 | 3397 | 8 | 0 | 500.125 | |
| PD9193a | 1 | 158859163 | 158860396 | 1233 | 7 | 0 | 186.428571 | |
| PD9193a | 1 | 158910630 | 158914497 | 3867 | 21 | 0 | 188.857143 | |
| PD9193a | 1 | 177313261 | 177316593 | 3332 | 6 | 0 | 604.5 | |
| PD9193a | 1 | 202124179 | 202128974 | 4795 | 11 | 0 | 491.727273 | |
| PD9193a | 17 | 57034097 | 57035499 | 1402 | 10 | 0 | 155.6 | |
| PD9464a | 1 | 173271962 | 173274337 | 2375 | 6 | 0 | 402.5 | |
| PD9464a | 1 | 243443243 | 243447665 | 4422 | 14 | 0 | 321.428571 | |
| PD9464a | 2 | 228221388 | 228222702 | 1314 | 11 | 0 | 122.636364 | |
| PD9464a | 8 | 36388561 | 36391035 | 2474 | 8 | 0 | 386.625 | |
| PD9464a | 8 | 39028562 | 39030250 | 1688 | 10 | 0 | 251.8 | |
| PD9539a | 14 | 62648216 | 62652561 | 4345 | 11 | 0 | 460.636364 | |
| PD9539a | 8 | 38494321 | 38495453 | 1132 | 7 | 0 | 229.571429 | |
| PD9541a | 11 | 72592311 | 72592538 | 227 | 7 | 0 | 51.1428571 | |
| PD9567a | 11 | 57622627 | 57629724 | 7097 | 23 | 0 | 402.956522 | |
| PD9567a | 11 | 69215836 | 69219491 | 3655 | 6 | 0 | 632.333333 | |
| PD9567a | 11 | 81293497 | 81294289 | 792 | 6 | 0 | 294.166667 | |
| PD9567a | 11 | 81344192 | 81344485 | 293 | 7 | 0 | 45.1428571 | |
| PD9569a | 15 | 99707588 | 99708214 | 626 | 8 | 0 | 107.5 | |
| PD9572a | 5 | 133805245 | 133810771 | 5526 | 6 | 0 | 975.5 | |
| PD9574a | 8 | 37266152 | 37266799 | 647 | 7 | 0 | 118.857143 | |
| PD9582a | 12 | 27030329 | 27032423 | 2094 | 7 | 0 | 388.571429 | |
| PD9582a | 12 | 30358349 | 30360132 | 1783 | 9 | 0 | 209.555556 | |
| PD9582a | 12 | 70499858 | 70500246 | 388 | 6 | 0 | 96.5 | |
| PD9582a | 15 | 39358755 | 39359589 | 834 | 10 | 0 | 106.2 | |

295

(continued)

| sample | chr | start.bp | end.bp | length.bp | number.bps | number.bps.clustered | avgDist.bp | alternative.kataegis |
|--------|-----|----------|--------|-----------|-----------|---------------------|------------|---------------------|
| PD9582a | 15 | 75543217 | 75546717 | 3500 | 9 | 0 | 393.333333 | |
| PD9582a | 15 | 98042078 | 98042443 | 365 | 8 | 0 | 50.375 | |
| PD9597a | 5 | 134503299 | 134510910 | 7611 | 11 | 0 | 753.909091 | |
| PD9599a | 12 | 91500594 | 91507932 | 7338 | 8 | 0 | 919.5 | |
| PD9599a | 2 | 40135541 | 40146569 | 11028 | 18 | 0 | 631.5 | |
| PD9599a | 2 | 41107747 | 41111378 | 3631 | 12 | 0 | 335.25 | |
| PD9599a | 20 | 1652803 | 1655460 | 2657 | 10 | 0 | 278.5 | |
| PD9599a | 20 | 1861661 | 1862033 | 372 | 6 | 0 | 63.5 | |
| PD9599a | 6 | 3658987 | 3659704 | 717 | 8 | 0 | 94.25 | |
| PD9599a | 8 | 98347724 | 98353207 | 5483 | 10 | 0 | 573.2 | |
| PD9605a | 11 | 66482505 | 66483499 | 994 | 7 | 0 | 156.714286 | |
| PD9605a | 11 | 73226756 | 73229619 | 2863 | 7 | 0 | 467.857143 | |
| PD9605a | 11 | 75502525 | 75504286 | 1761 | 15 | 0 | 117.866667 | |
| PD9605a | 19 | 22273347 | 22274413 | 1066 | 10 | 0 | 108.8 | |
| PD9694a | 3 | 102170700 | 102173822 | 3122 | 6 | 0 | 594 | |
| PD9694a | 3 | 175635494 | 175635707 | 213 | 6 | 0 | 57.8333333 | |
| PD9752a | 1 | 241443051 | 241444251 | 1200 | 7 | 0 | 395.142857 | |
| PD9752a | 12 | 48984949 | 48987363 | 2414 | 15 | 0 | 186.2 | |
| PD9752a | 12 | 70633186 | 70634320 | 1134 | 9 | 0 | 138.777778 | |
| PD9752a | 12 | 70643485 | 70644770 | 1285 | 9 | 0 | 144.333333 | |
| PD9752a | 12 | 74974347 | 74976543 | 2196 | 7 | 0 | 320.571429 | |
| PD9752a | 12 | 90637357 | 90639482 | 2125 | 9 | 0 | 239.555556 | |
| PD9752a | 5 | 52775787 | 52777628 | 1841 | 8 | 0 | 269.125 | |
| PD9752a | 6 | 50659992 | 50661116 | 1124 | 6 | 0 | 213.166667 | |
| PD9752a | 6 | 100431092 | 100437127 | 6035 | 7 | 0 | 883.285714 | |
| PD9752a | 6 | 100922127 | 100924348 | 2221 | 11 | 0 | 203.909091 | |

(continued)

| sample | chr | start.bp | end.bp | length.bp | number.bps | number.bps.clustered | avgDist.bp | alternative.kataegis |
|---|---|---|---|---|---|---|---|---|
| PD9752a | 6 | 104801518 | 104802281 | 763 | 7 | 0 | 219.142857 | |
| PD9752a | 6 | 134343540 | 134352456 | 8916 | 13 | 0 | 707.538462 | |
| PD9752a | 6 | 147116173 | 147116780 | 607 | 8 | 0 | 102.875 | |
| PD9752a | 6 | 148367428 | 148369733 | 2305 | 17 | 0 | 162.117647 | |
| PD9752a | 7 | 84639696 | 84641686 | 1990 | 7 | 0 | 308.571429 | |
| PD9754a | 11 | 66461615 | 66462035 | 420 | 10 | 0 | 50.2 | |
| PD9759a | 17 | 31276514 | 31281886 | 5372 | 9 | 0 | 634.666667 | |
| PD9760a | 15 | 98060548 | 98061322 | 774 | 7 | 0 | 113.571429 | |
| PD9760a | 15 | 98999535 | 99002863 | 3328 | 6 | 0 | 566.666667 | |
| PD9760a | 15 | 99965755 | 99967138 | 1383 | 12 | 0 | 130.833333 | |
| PD9760a | 2 | 120524784 | 120529144 | 4360 | 7 | 0 | 752.428571 | |
| PD9760a | 2 | 180516022 | 180517458 | 1436 | 7 | 0 | 232.857143 | |
| PD9761a | 1 | 181570387 | 181576608 | 6221 | 11 | 0 | 567.545455 | |
| PD9761a | 1 | 181858956 | 181862235 | 3279 | 21 | 0 | 169.047619 | |
| PD11336a | 1 | 1159550 | 1160418 | 868 | 7 | 0 | 124.142857 | |
| PD11336a | 17 | 51417012 | 51419727 | 2715 | 8 | 0 | 366 | |
| PD11336a | 5 | 157055155 | 157055479 | 324 | 7 | 0 | 68.2857143 | |
| PD11337a | 13 | 35070628 | 35072138 | 1510 | 14 | 0 | 114.285714 | |
| PD11337a | 20 | 53339378 | 53340247 | 869 | 7 | 0 | 150.857143 | |
| PD11340a | 10 | 101249 | 104729 | 3480 | 44 | 0 | 79.9318182 | |
| PD11340a | 12 | 29891580 | 29892853 | 1273 | 14 | 0 | 92.0714286 | |
| PD11340a | 12 | 86113483 | 86117663 | 4180 | 7 | 0 | 600 | |
| PD11340a | 17 | 22215518 | 22218350 | 2832 | 18 | 0 | 157.444444 | |
| PD11340a | 18 | 32904531 | 32906222 | 1691 | 17 | 0 | 102.294118 | |
| PD11340a | 18 | 32931987 | 32932718 | 731 | 12 | 0 | 63.5 | |
| PD11340a | 2 | 85009986 | 85012464 | 2478 | 22 | 0 | 244.954545 | |

(continued)

| sample | chr | start.bp | end.bp | length.bp | number.bps | number.bps.clustered | avgDist.bp | alternative.kataegis |
|---|---|---|---|---|---|---|---|---|
| PD11340a | 2 | 85029874 | 85032873 | 2999 | 7 | 0 | 433 | |
| PD11340a | 4 | 59057000 | 59062379 | 5379 | 12 | 0 | 487.416667 | |
| PD11340a | 7 | 147548016 | 147548908 | 892 | 6 | 0 | 169.5 | |
| PD11340a | 9 | 12360004 | 12366030 | 6026 | 35 | 0 | 178.142857 | |
| PD11343a | 11 | 88166017 | 88168005 | 1988 | 7 | 0 | 328.428571 | yes |
| PD11343a | 20 | 18151964 | 18153896 | 1932 | 7 | 0 | 344 | |
| PD11343a | X | 91782822 | 91786957 | 4135 | 11 | 0 | 424.454545 | |
| PD11343a | X | 95794239 | 95794619 | 380 | 7 | 0 | 74.2857143 | |
| PD11358a | 19 | 4773713 | 4777623 | 3910 | 7 | 0 | 857.571429 | |
| PD11367a | 20 | 16812393 | 16812967 | 574 | 7 | 0 | 103.285714 | yes |
| PD11368a | 1 | 74238510 | 74238984 | 474 | 7 | 0 | 83.2857143 | |
| PD11368a | 9 | 17414457 | 17416546 | 2089 | 9 | 0 | 236.222222 | |
| PD11369a | 1 | 246130209 | 246134373 | 4164 | 17 | 0 | 247.529412 | |
| PD11369a | 12 | 46120323 | 46123889 | 3566 | 10 | 0 | 360.5 | |
| PD11369a | 12 | 73316291 | 73318173 | 1882 | 8 | 0 | 256.5 | |
| PD11369a | 12 | 84597159 | 84600598 | 3439 | 16 | 0 | 215.3125 | |
| PD11369a | 12 | 85298501 | 85304651 | 6150 | 37 | 0 | 167.351351 | |
| PD11369a | 12 | 85471143 | 85472931 | 1788 | 15 | 0 | 121.066667 | |
| PD11369a | 14 | 98326594 | 98336011 | 9417 | 16 | 0 | 603.125 | |
| PD11369a | 17 | 31869875 | 31872172 | 2297 | 13 | 0 | 224.846154 | |
| PD11369a | 17 | 53392769 | 53395572 | 2803 | 13 | 0 | 221.692308 | |
| PD11369a | 17 | 62856301 | 62862462 | 6161 | 53 | 0 | 119.056604 | |
| PD11369a | 17 | 69467484 | 69470031 | 2547 | 8 | 0 | 323.5 | |
| PD11369a | 2 | 4444365 | 4444808 | 443 | 7 | 0 | 77.5714286 | |
| PD11369a | 3 | 84284381 | 84285511 | 1130 | 9 | 0 | 192.444444 | |
| PD11369a | 8 | 26649606 | 26653363 | 3757 | 14 | 0 | 340.571429 | |

(continued)

| sample | chr | start.bp | end.bp | length.bp | number.bps | number.bps.clustered | avgDist.bp | alternative.kataegis |
|--------|-----|----------|--------|-----------|------------|---------------------|-----------|---------------------|
| PD11369a | 8 | 60500439 | 60501668 | 1229 | 9 | 0 | 169 | |
| PD11372a | 12 | 99876572 | 99880574 | 4002 | 7 | 0 | 573.428571 | |
| PD11372a | 22 | 28382224 | 28385768 | 3544 | 8 | 0 | 444.625 | |
| PD11372a | 5 | 179654487 | 179659351 | 4864 | 16 | 0 | 307.5625 | |
| PD11372a | 5 | 179667001 | 179670519 | 3518 | 12 | 0 | 297.416667 | |
| PD11372a | 7 | 47293549 | 47306973 | 13424 | 19 | 0 | 715.631579 | |
| PD11372a | 7 | 64270073 | 64279267 | 9194 | 10 | 0 | 980.4 | |
| PD11374a | 8 | 53350712 | 53353382 | 2670 | 7 | 0 | 705.428571 | |
| PD11379a | 10 | 88623247 | 88626575 | 3328 | 6 | 0 | 636.666667 | |
| PD11379a | 13 | 100974088 | 100975688 | 1600 | 7 | 0 | 293.857143 | |
| PD11379a | 14 | 50953721 | 50956483 | 2762 | 7 | 0 | 468 | |
| PD11379a | 17 | 16091610 | 16094558 | 2948 | 7 | 0 | 431.285714 | |
| PD11379a | 19 | 9326747 | 9328151 | 1404 | 10 | 0 | 171.8 | |
| PD11379a | 19 | 16884082 | 16884388 | 306 | 6 | 0 | 92.1666667 | |
| PD11379a | 20 | 19034282 | 19036609 | 2327 | 6 | 0 | 456.666667 | |
| PD11379a | 3 | 71088585 | 71094869 | 6284 | 7 | 0 | 898.714286 | |
| PD11379a | 3 | 71102914 | 71111381 | 8467 | 10 | 0 | 957.2 | |
| PD11379a | 6 | 31740007 | 31748954 | 8947 | 11 | 0 | 818.727273 | |
| PD11379a | 6 | 31754228 | 31764155 | 9927 | 14 | 0 | 725.642857 | |
| PD11379a | 7 | 104691602 | 104693982 | 2380 | 6 | 0 | 769 | |
| PD11379a | 9 | 21524762 | 21525207 | 445 | 8 | 0 | 90.5 | |
| PD11393a | 1 | 147116339 | 147117739 | 1400 | 6 | 0 | 255.333333 | |
| PD11393a | 11 | 77108502 | 77114621 | 6119 | 7 | 0 | 911.142857 | |
| PD11395a | 12 | 41935324 | 41935602 | 278 | 8 | 0 | 44.875 | |
| PD11395a | 6 | 127241475 | 127242931 | 1456 | 15 | 0 | 129.133333 | |
| PD11397a | 11 | 39261865 | 39263918 | 2053 | 7 | 0 | 293.428571 | |

| sample | chr | start.bp | end.bp | length.bp | number.bps | number.bps.clustered | avgDist.bp | alternative.kataegis |
|---|---|---|---|---|---|---|---|---|
| PD11397a | 16 | 49515212 | 49520046 | 4834 | 34 | 0 | 146.735294 | |
| PD11397a | 8 | 65770044 | 65773078 | 3034 | 6 | 0 | 506.333333 | |
| PD11397a | 8 | 81270077 | 81272762 | 2685 | 10 | 0 | 268.6 | |
| PD11397a | 8 | 93124791 | 93126479 | 1688 | 8 | 0 | 227.625 | |
| PD11397a | 8 | 136662181 | 136665227 | 3046 | 14 | 0 | 280.357143 | |
| PD11397a | 8 | 139965380 | 139973913 | 8533 | 21 | 0 | 419 | |
| PD11398a | 14 | 39248823 | 39250286 | 1463 | 6 | 0 | 252.666667 | |
| PD11399a | 11 | 68418390 | 68419953 | 1563 | 9 | 0 | 204.222222 | |
| PD11399a | 11 | 93052642 | 93053385 | 743 | 7 | 0 | 129.428571 | |
| PD11399a | 8 | 13399007 | 13399652 | 645 | 6 | 0 | 277.833333 | |
| PD11399a | 8 | 40083607 | 40088683 | 5076 | 32 | 0 | 197.5 | |
| PD11399a | 8 | 41497900 | 41501814 | 3914 | 32 | 0 | 156.125 | |
| PD11399a | 8 | 41846048 | 41849111 | 3063 | 9 | 0 | 388.888889 | |
| PD11399a | 8 | 42648758 | 42651711 | 2953 | 12 | 0 | 248.083333 | |
| PD11399a | 8 | 47555179 | 47563627 | 8448 | 40 | 0 | 230.175 | |
| PD11402a | 11 | 87213386 | 87214401 | 1015 | 25 | 0 | 40.68 | |
| PD11741a | 8 | 39366217 | 39370470 | 4253 | 6 | 0 | 746.333333 | |
| PD11742a | 2 | 113369848 | 113370857 | 1009 | 6 | 0 | 284 | |
| PD11742a | 3 | 42276963 | 42279490 | 2527 | 6 | 0 | 666.166667 | |
| PD11745a | 11 | 31435954 | 31438694 | 2740 | 12 | 0 | 258.083333 | |
| PD11745a | 11 | 32809340 | 32813662 | 4322 | 9 | 0 | 498 | |
| PD11745a | 11 | 68344918 | 68345783 | 865 | 8 | 0 | 155.5 | |
| PD11745a | 8 | 771280 | 771441 | 161 | 6 | 0 | 81.3333333 | |
| PD11745a | 8 | 76383705 | 76391416 | 7711 | 9 | 0 | 864.777778 | |
| PD11745a | 8 | 80787063 | 80788260 | 1197 | 10 | 0 | 137.5 | |
| PD11745a | 8 | 103087544 | 103090892 | 3348 | 7 | 0 | 518.857143 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD11745a | 8 | 131023588 | 131024492 | 904 | 6 | 0 | 171.666667 | |
| PD11750a | 12 | 77587350 | 77593994 | 6644 | 10 | 0 | 748.2 | |
| PD11750a | 9 | 4259776 | 4260415 | 639 | 7 | 0 | 103.285714 | |
| PD11756a | 8 | 39445990 | 39446489 | 499 | 8 | 0 | 74.375 | |
| PD11761a | 11 | 71722031 | 71727763 | 5732 | 8 | 0 | 734.25 | |
| PD11765a | 17 | 80972387 | 80974644 | 2257 | 8 | 0 | 332 | |
| PD11766a | 3 | 81634593 | 81637461 | 2868 | 9 | 0 | 325.444444 | |
| PD11769a | 11 | 58923931 | 58925007 | 1076 | 6 | 0 | 179.5 | |
| PD13307a | 11 | 80697892 | 80699586 | 1694 | 8 | 0 | 212.25 | |
| PD13419a | 6 | 127614485 | 127617713 | 3228 | 11 | 0 | 293.727273 | |
| PD13424a | 17 | 55192330 | 55193987 | 1657 | 11 | 0 | 230.818182 | |
| PD13424a | 17 | 57284823 | 57285061 | 238 | 6 | 0 | 211.5 | |
| PD13424a | 17 | 75178712 | 75180093 | 1381 | 9 | 0 | 159.111111 | |
| PD13424a | 19 | 45339503 | 45340818 | 1315 | 6 | 0 | 322.5 | |
| PD13424a | 19 | 45399820 | 45400639 | 819 | 7 | 0 | 147.857143 | |
| PD13425a | 12 | 29986441 | 29988588 | 2147 | 6 | 0 | 573.666667 | |
| PD13425a | 2 | 53354367 | 53355798 | 1431 | 7 | 0 | 248.285714 | |
| PD13425a | 3 | 1810202 | 1811860 | 1658 | 6 | 0 | 280.833333 | |
| PD13425a | 3 | 9216275 | 9219950 | 3675 | 10 | 0 | 367.8 | |
| PD13425a | 3 | 19115850 | 19117084 | 1234 | 6 | 0 | 274 | |
| PD13425a | 3 | 45320509 | 45322114 | 1605 | 6 | 0 | 359.333333 | |
| PD13425a | 6 | 65653633 | 65655728 | 2095 | 6 | 0 | 492.333333 | |
| PD13425a | 6 | 65669573 | 65671765 | 2192 | 9 | 0 | 264.111111 | |
| PD13425a | 6 | 104951558 | 104953315 | 1757 | 8 | 0 | 247.875 | |
| PD13425a | 6 | 131844428 | 131851191 | 6763 | 16 | 0 | 423 | |
| PD13425a | X | 86585014 | 86585156 | 142 | 7 | 0 | 27.2857143 | |

(continued)

| sample | chr | start.bp | end.bp | length.bp | number.bps | number.bps.clustered | avgDist.bp | alternative.kataegis |
|---|---|---|---|---|---|---|---|---|
| PD13620a | 17 | 70011305 | 70013140 | 1835 | 10 | 0 | 184.6 | |
| PD13620a | 7 | 156731298 | 156732636 | 1338 | 6 | 0 | 305.333333 | |
| PD13620a | 8 | 65197204 | 65199843 | 2639 | 11 | 0 | 240.545455 | |
| PD13620a | 9 | 30146693 | 30147355 | 662 | 8 | 0 | 97.375 | |
| PD13625a | 10 | 124945403 | 124948635 | 3232 | 7 | 0 | 462.142857 | |
| PD13625a | 12 | 105821691 | 105830222 | 8531 | 16 | 0 | 571.6875 | |
| PD13625a | 15 | 38987184 | 38987912 | 728 | 8 | 0 | 111.75 | |
| PD13625a | 15 | 53688734 | 53692936 | 4202 | 14 | 0 | 314.5 | |
| PD13625a | 15 | 77461912 | 77465217 | 3305 | 8 | 0 | 512 | |
| PD13625a | 18 | 18835102 | 18838636 | 3534 | 36 | 0 | 98.25 | |
| PD13626a | 1 | 31906780 | 31910619 | 3839 | 7 | 0 | 562.571429 | |
| PD13631a | 12 | 16888624 | 16890942 | 2318 | 6 | 0 | 412.166667 | |
| PD13631a | 16 | 32446286 | 32448966 | 2680 | 7 | 0 | 429.285714 | |
| PD13764a | 1 | 109081475 | 109082066 | 591 | 6 | 0 | 302.666667 | |
| PD13764a | 1 | 160365561 | 160367971 | 2410 | 14 | 0 | 174.571429 | |
| PD13764a | 1 | 161446702 | 161447325 | 623 | 6 | 0 | 115.333333 | |
| PD13764a | 14 | 56608106 | 56608770 | 664 | 10 | 0 | 75.3 | |
| PD13764a | 4 | 22240779 | 22248188 | 7409 | 36 | 0 | 213.055556 | |
| PD13764a | 8 | 64433834 | 64435310 | 1476 | 11 | 0 | 205.909091 | |
| PD13764a | 8 | 72598918 | 72604387 | 5469 | 10 | 0 | 547.2 | |
| PD13764a | 8 | 82974471 | 82975167 | 696 | 7 | 0 | 206.285714 | |
| PD13764a | 8 | 88506611 | 88506973 | 362 | 6 | 0 | 110.5 | |
| PD13764a | 8 | 90395553 | 90396141 | 588 | 6 | 0 | 616 | |
| PD13764a | 8 | 90931724 | 90933855 | 2131 | 10 | 0 | 227.4 | |
| PD13765a | 11 | 37521726 | 37523324 | 1598 | 7 | 0 | 438.571429 | |
| PD13765a | 11 | 37865737 | 37866624 | 887 | 9 | 0 | 116.888889 | |

(continued)

| sample | chr | start.bp | end.bp | length.bp | number.bps | number.bps.clustered | avgDist.bp | alternative.kataegis |
|--------|-----|----------|--------|-----------|------------|---------------------|------------|---------------------|
| PD13765a | 11 | 48637299 | 48638497 | 1198 | 12 | 0 | 101.583333 | |
| PD13765a | 11 | 55354999 | 55357126 | 2127 | 7 | 0 | 333.142857 | |
| PD13765a | 13 | 19653566 | 19655759 | 2193 | 11 | 0 | 213.454545 | |
| PD13765a | 17 | 48758424 | 48765080 | 6656 | 8 | 0 | 956.25 | |
| PD13765a | 17 | 53964909 | 53965737 | 828 | 7 | 0 | 121.285714 | |
| PD13765a | 17 | 56087008 | 56091697 | 4689 | 17 | 0 | 371.823529 | |
| PD13765a | 17 | 67909450 | 67914237 | 4787 | 13 | 0 | 369.538462 | |
| PD13765a | 6 | 104925735 | 104926635 | 900 | 10 | 0 | 103.6 | |
| PD13765a | X | 141378712 | 141379065 | 353 | 8 | 0 | 53.375 | |
| PD13766a | 3 | 129091035 | 129094549 | 3514 | 9 | 0 | 460.888889 | |
| PD13767a | 19 | 4355723 | 4356608 | 885 | 9 | 0 | 142.222222 | |
| PD13771a | 6 | 63950107 | 63950995 | 888 | 10 | 0 | 95.3 | |
| PD13771a | 7 | 2138597 | 2139572 | 975 | 6 | 0 | 291.166667 | |
| PD13771a | 7 | 104325385 | 104326527 | 1142 | 9 | 0 | 154.222222 | |
| PD14433a | 11 | 21674031 | 21680513 | 6482 | 17 | 0 | 401.176471 | |
| PD14435a | 12 | 47305822 | 47309744 | 3922 | 9 | 0 | 440.888889 | |
| PD14435a | 19 | 13381080 | 13384228 | 3148 | 20 | 0 | 160.5 | |
| PD14435a | 19 | 23449866 | 23456044 | 6178 | 12 | 0 | 516.75 | |
| PD14435a | 19 | 46329643 | 46334249 | 4606 | 10 | 0 | 464.4 | |
| PD14435a | 6 | 160816706 | 160817988 | 1282 | 7 | 0 | 242.428571 | |
| PD14435a | 8 | 64243758 | 64247461 | 3703 | 15 | 0 | 268.133333 | |
| PD14437a | 1 | 14695948 | 14698833 | 2885 | 14 | 0 | 222.142857 | |
| PD14437a | 13 | 55288118 | 55291904 | 3786 | 9 | 0 | 504.777778 | |
| PD14441a | 6 | 20957837 | 20959054 | 1217 | 6 | 0 | 207.833333 | |
| PD14453a | 16 | 15735640 | 15737747 | 2107 | 6 | 0 | 394 | |
| PD14453a | 18 | 75571731 | 75573105 | 1374 | 10 | 0 | 192.8 | |

303

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD14453a | 2 | 120859308 | 120859652 | 344 | 6 | 0 | 78 | |
| PD14453a | 20 | 53453241 | 53455209 | 1968 | 24 | 0 | 83.9583333 | |
| PD14453a | 21 | 17701481 | 17707198 | 5717 | 11 | 0 | 526.363636 | |
| PD14453a | 21 | 19296712 | 19299988 | 3276 | 8 | 0 | 501.5 | |
| PD14453a | 3 | 118604904 | 118610519 | 5615 | 23 | 0 | 244.652174 | |
| PD14453a | 3 | 121418878 | 121428522 | 9644 | 23 | 0 | 441.347826 | |
| PD14453a | 3 | 121531256 | 121541978 | 10722 | 44 | 0 | 244.454545 | |
| PD14453a | 3 | 192174325 | 192181788 | 7463 | 18 | 0 | 418.888889 | |
| PD14453a | 3 | 194895980 | 194900468 | 4488 | 11 | 0 | 415.363636 | |
| PD14453a | 6 | 58289113 | 58293452 | 4339 | 14 | 0 | 401.285714 | |
| PD14454a | 11 | 81943371 | 81947262 | 3891 | 8 | 0 | 502.875 | |
| PD14457a | 19 | 33809575 | 33813992 | 4417 | 22 | 0 | 238.227273 | |
| PD14457a | 19 | 36212835 | 36215162 | 2327 | 8 | 0 | 563.5 | |
| PD14457a | 2 | 237161863 | 237162904 | 1041 | 12 | 0 | 89.6666667 | |
| PD14460a | 1 | 28174497 | 28178064 | 3567 | 14 | 0 | 256.428571 | |
| PD14460a | 1 | 28932326 | 28933219 | 893 | 9 | 0 | 130 | |
| PD14460a | 11 | 55255468 | 55258336 | 2868 | 12 | 0 | 254.166667 | |
| PD14460a | 11 | 55873438 | 55874629 | 1191 | 10 | 0 | 140.6 | |
| PD14460a | 11 | 70164939 | 70165981 | 1042 | 7 | 0 | 149.714286 | |
| PD14460a | 6 | 62258666 | 62262642 | 3976 | 14 | 0 | 289 | |
| PD14460a | 6 | 70390958 | 70396029 | 5071 | 8 | 0 | 971.75 | |
| PD14460a | 6 | 71856701 | 71857899 | 1198 | 12 | 0 | 103 | |
| PD14461a | 11 | 76548880 | 76551051 | 2171 | 15 | 0 | 172.733333 | |
| PD14465a | 11 | 73154788 | 73155745 | 957 | 9 | 0 | 108.333333 | |
| PD14467a | 1 | 243600344 | 243605854 | 5510 | 18 | 0 | 319.277778 | |
| PD14471a | 19 | 20828307 | 20832384 | 4077 | 6 | 0 | 733.166667 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD14471a | 8 | 134285153 | 134285432 | 279 | 6 | 0 | 88.3333333 | yes |
| PD14471a | 8 | 136020027 | 136020988 | 961 | 6 | 0 | 188.5 | yes |
| PD17981a | 11 | 70502868 | 70503135 | 267 | 7 | 0 | 41.1428571 | |
| PD17994a | 11 | 1265891 | 1269834 | 3943 | 6 | 0 | 911 | |
| PD18149a | 12 | 40025099 | 40025518 | 419 | 9 | 0 | 47.4444444 | |
| PD18149a | 12 | 40737658 | 40738120 | 462 | 7 | 0 | 69.2857143 | |
| PD18149a | 12 | 69731899 | 69732494 | 595 | 11 | 0 | 54.5454545 | |
| PD18149a | 12 | 78630412 | 78631235 | 823 | 6 | 0 | 147 | |
| PD18149a | 2 | 47387540 | 47387742 | 202 | 6 | 0 | 42.3333333 | |
| PD18247a | 12 | 120376975 | 120382115 | 5140 | 7 | 0 | 844.428571 | |
| PD18247a | 12 | 123258973 | 123263023 | 4050 | 7 | 0 | 588.714286 | |
| PD18247a | 15 | 45077427 | 45080717 | 3290 | 10 | 0 | 674.4 | |
| PD18247a | 17 | 55282371 | 55288858 | 6487 | 15 | 0 | 457.133333 | |
| PD18247a | 19 | 49052717 | 49055880 | 3163 | 7 | 0 | 566.285714 | |
| PD18247a | 2 | 33163170 | 33164046 | 876 | 6 | 0 | 178.666667 | |
| PD18247a | 20 | 41079349 | 41082384 | 3035 | 7 | 0 | 433.714286 | |
| PD18251a | 19 | 57165834 | 57167147 | 1313 | 12 | 0 | 133.166667 | |
| PD18251a | 21 | 17615064 | 17616000 | 936 | 8 | 0 | 117.875 | |
| PD18251a | 7 | 83455924 | 83456783 | 859 | 6 | 0 | 169.833333 | |
| PD18251a | 8 | 33663677 | 33664515 | 838 | 6 | 0 | 179.666667 | |
| PD18257a | 11 | 73903379 | 73905093 | 1714 | 13 | 0 | 160.692308 | |
| PD18264a | 1 | 162071055 | 162074322 | 3267 | 10 | 0 | 357.6 | |
| PD18264a | 12 | 112121375 | 112122781 | 1406 | 8 | 0 | 393 | |
| PD18264a | 14 | 68762961 | 68764038 | 1077 | 6 | 0 | 181.5 | |
| PD18264a | 15 | 89904014 | 89910438 | 6424 | 8 | 0 | 935.875 | |
| PD18264a | 17 | 17882281 | 17882814 | 533 | 7 | 0 | 176 | |

EP 3 452 937 B1

(continued)

| sample | chr | start.bp | end.bp | length.bp | number.bps | number.bps.clustered | avgDist.bp | alternative.kataegis |
|---|---|---|---|---|---|---|---|---|
| PD18264a | 2 | 86688177 | 86694082 | 5905 | 8 | 0 | 748 | |
| PD18264a | 4 | 96607575 | 96609188 | 1613 | 6 | 0 | 349.333333 | |
| PD18264a | 6 | 138335517 | 138337095 | 1578 | 6 | 0 | 528.666667 | |
| PD18264a | 6 | 152048268 | 152052069 | 3801 | 7 | 0 | 882.857143 | |
| PD18734a | 8 | 90288335 | 90290325 | 1990 | 11 | 0 | 283.727273 | |
| PD18734a | 8 | 94482157 | 94490204 | 8047 | 15 | 0 | 592.666667 | |
| PD18734a | 8 | 103269710 | 103270651 | 941 | 8 | 0 | 119.75 | |
| PD18734a | 8 | 114969054 | 114973215 | 4161 | 9 | 0 | 555.888889 | |
| PD18734a | X | 146030679 | 146033947 | 3268 | 11 | 0 | 300.272727 | |
| PD18749a | 1 | 205071453 | 205076122 | 4669 | 8 | 0 | 595.5 | |
| PD18749a | 11 | 46199180 | 46200382 | 1202 | 9 | 0 | 150.333333 | |
| PD18749a | 19 | 13938659 | 13944141 | 5482 | 17 | 0 | 324.411765 | |
| PD18749a | 20 | 36715075 | 36717047 | 1972 | 8 | 0 | 322.375 | |
| PD18754a | 4 | 2527958 | 2535630 | 7672 | 11 | 0 | 744.454545 | |
| PD18769a | 13 | 49523463 | 49523700 | 237 | 6 | 0 | 49.5 | |
| PD4252a | 9 | 94197395 | 94200190 | 2795 | 15 | 0 | 192.666667 | |
| PD4255a | 8 | 61466398 | 61467188 | 790 | 10 | 0 | 91.1 | |
| PD4255a | 8 | 117771237 | 117772947 | 1710 | 11 | 0 | 165.636364 | |
| PD4255a | X | 110457909 | 110458266 | 357 | 6 | 0 | 68.6666667 | |
| PD4833a | 13 | 36398555 | 36400896 | 2341 | 6 | 0 | 399.166667 | |
| PD4833a | 3 | 166676017 | 166677215 | 1198 | 6 | 0 | 201.166667 | |
| PD4833a | 6 | 127648532 | 127651794 | 3262 | 6 | 0 | 607.833333 | |
| PD4836a | 12 | 6501965 | 6503113 | 1148 | 7 | 0 | 185.714286 | |
| PD4844a | 13 | 29763458 | 29764546 | 1088 | 7 | 0 | 159.571429 | |
| PD4844a | 2 | 52625356 | 52626223 | 867 | 15 | 0 | 66.1333333 | |
| PD4844a | 4 | 151040515 | 151046553 | 6038 | 11 | 0 | 599.181818 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|---------------------|
| PD4874a | 10 | 112589872 | 112591462 | 1590 | 8 | 0 | 218 | |
| PD4874a | 11 | 69573036 | 69574485 | 1449 | 6 | 0 | 427.666667 | |
| PD4874a | 11 | 79698372 | 79699063 | 691 | 6 | 0 | 119.833333 | |
| PD4874a | 11 | 79711149 | 79712217 | 1068 | 11 | 0 | 125.363636 | |
| PD4874a | 15 | 90564880 | 90567150 | 2270 | 10 | 0 | 242.6 | |
| PD4874a | 16 | 84757626 | 84769079 | 11453 | 16 | 0 | 766.75 | |
| PD4874a | 17 | 2977667 | 2980265 | 2598 | 8 | 0 | 434.125 | |
| PD4874a | 2 | 223293294 | 223295081 | 1787 | 6 | 0 | 330.166667 | |
| PD4874a | 2 | 229267119 | 229274874 | 7755 | 19 | 0 | 455.842105 | |
| PD4874a | 3 | 94470812 | 94471401 | 589 | 8 | 0 | 76 | |
| PD4874a | 7 | 77095911 | 77104360 | 8449 | 10 | 0 | 883.8 | |
| PD4874a | 8 | 35884174 | 35885446 | 1272 | 17 | 0 | 76.9411765 | |
| PD4874a | 8 | 37296801 | 37297534 | 733 | 9 | 0 | 84.1111111 | |
| PD4875a | 11 | 42609773 | 42611280 | 1507 | 7 | 0 | 623.285714 | |
| PD4875a | 19 | 56270256 | 56290150 | 19894 | 39 | 0 | 514.230769 | |
| PD4875a | 4 | 87940680 | 87945325 | 4645 | 11 | 0 | 430.454545 | |
| PD4875a | 7 | 139479288 | 139480975 | 1687 | 11 | 0 | 188.909091 | |
| PD4955a | X | 31010821 | 31015332 | 4511 | 6 | 0 | 835.833333 | |
| PD4977a | 1 | 32753313 | 32755265 | 1952 | 10 | 0 | 201.5 | |
| PD4977a | 1 | 32805777 | 32808811 | 3034 | 7 | 0 | 501.285714 | |
| PD4977a | 1 | 42815903 | 42820057 | 4154 | 8 | 0 | 595.625 | |
| PD4977a | 1 | 47314825 | 47316305 | 1480 | 6 | 0 | 595.5 | |
| PD4977a | 1 | 150777567 | 150781677 | 4110 | 11 | 0 | 421.818182 | |
| PD4977a | 1 | 167575079 | 167578969 | 3890 | 13 | 0 | 351.692308 | |
| PD4977a | 1 | 217386180 | 217387909 | 1729 | 9 | 0 | 302.333333 | |
| PD4977a | 10 | 78488817 | 78490964 | 2147 | 6 | 0 | 414.166667 | |

307

(continued)

| sample | chr | start.bp | end.bp | length.bp | number.bps | number.bps.clustered | avgDist.bp | alternative.kataegis |
|---|---|---|---|---|---|---|---|---|
| PD4977a | 11 | 1131179 | 1137535 | 6356 | 14 | 0 | 504.714286 | |
| PD4977a | 11 | 72015333 | 72016371 | 1038 | 6 | 0 | 247.5 | |
| PD4977a | 11 | 102490939 | 102494518 | 3579 | 12 | 0 | 308.75 | |
| PD4977a | 11 | 130247259 | 130250215 | 2956 | 12 | 0 | 260.416667 | |
| PD4977a | 12 | 32359473 | 32363266 | 3793 | 7 | 0 | 594.285714 | |
| PD4977a | 12 | 46050374 | 46055502 | 5128 | 6 | 0 | 978 | |
| PD4977a | 12 | 119784832 | 119790445 | 5613 | 10 | 0 | 561.9 | |
| PD4977a | 13 | 20848055 | 20849075 | 1020 | 6 | 0 | 239.166667 | |
| PD4977a | 13 | 96901304 | 96905106 | 3802 | 6 | 0 | 661 | |
| PD4977a | 14 | 38052401 | 38056783 | 4382 | 9 | 0 | 549.888889 | |
| PD4977a | 14 | 60905632 | 60908570 | 2938 | 6 | 0 | 535.166667 | |
| PD4977a | 14 | 91548028 | 91550256 | 2228 | 8 | 0 | 281.25 | |
| PD4977a | 14 | 100236770 | 100239367 | 2597 | 6 | 0 | 472.166667 | |
| PD4977a | 15 | 47323352 | 47324936 | 1584 | 9 | 0 | 179.555556 | |
| PD4977a | 15 | 99647132 | 99650589 | 3457 | 8 | 0 | 445.5 | |
| PD4977a | 16 | 10688949 | 10691168 | 2219 | 6 | 0 | 393.5 | |
| PD4977a | 16 | 12101355 | 12103965 | 2610 | 10 | 0 | 338.9 | |
| PD4977a | 16 | 20652235 | 20657410 | 5175 | 8 | 0 | 678.125 | |
| PD4977a | 17 | 3685953 | 3688223 | 2270 | 6 | 0 | 379.5 | |
| PD4977a | 17 | 56328248 | 56331270 | 3022 | 9 | 0 | 372.444444 | |
| PD4977a | 17 | 59208930 | 59212312 | 3382 | 8 | 0 | 446.625 | |
| PD4977a | 17 | 62641403 | 62645911 | 4508 | 10 | 0 | 511.5 | |
| PD4977a | 17 | 74224703 | 74225305 | 602 | 6 | 0 | 428.5 | |
| PD4977a | 17 | 77864669 | 77868713 | 4044 | 12 | 0 | 386.25 | |
| PD4977a | 18 | 44421512 | 44428067 | 6555 | 14 | 0 | 506.285714 | |
| PD4977a | 18 | 61223992 | 61226055 | 2063 | 6 | 0 | 436.666667 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD4977a | 19 | 19340952 | 19346500 | 5548 | 10 | 0 | 651.3 | |
| PD4977a | 19 | 50651639 | 50654915 | 3276 | 8 | 0 | 410.625 | |
| PD4977a | 2 | 21996313 | 22000175 | 3862 | 13 | 0 | 336.538462 | |
| PD4977a | 2 | 119848089 | 119850978 | 2889 | 7 | 0 | 424 | |
| PD4977a | 2 | 141856304 | 141860665 | 4361 | 16 | 0 | 311.4375 | |
| PD4977a | 2 | 170409648 | 170412378 | 2730 | 10 | 0 | 415.2 | |
| PD4977a | 2 | 190064711 | 190067307 | 2596 | 6 | 0 | 626.333333 | |
| PD4977a | 2 | 204823896 | 204825326 | 1430 | 6 | 0 | 398.666667 | |
| PD4977a | 2 | 208664828 | 208669527 | 4699 | 9 | 0 | 596.888889 | |
| PD4977a | 2 | 221199848 | 221204862 | 5014 | 11 | 0 | 492.454545 | |
| PD4977a | 2 | 225705881 | 225712447 | 6566 | 8 | 0 | 825.875 | |
| PD4977a | 2 | 228062827 | 228066572 | 3745 | 9 | 0 | 567.444444 | |
| PD4977a | 22 | 34535989 | 34538408 | 2419 | 14 | 0 | 178.571429 | |
| PD4977a | 3 | 85015960 | 85018372 | 2412 | 7 | 0 | 389.142857 | |
| PD4977a | 3 | 85123429 | 85126100 | 2671 | 7 | 0 | 440.142857 | |
| PD4977a | 3 | 114009600 | 114012548 | 2948 | 8 | 0 | 374.25 | |
| PD4977a | 3 | 161671820 | 161675869 | 4049 | 7 | 0 | 698.714286 | |
| PD4977a | 3 | 197065435 | 197066294 | 859 | 6 | 0 | 213.166667 | |
| PD4977a | 4 | 12537814 | 12541199 | 3385 | 8 | 0 | 489.25 | |
| PD4977a | 4 | 12545369 | 12547626 | 2257 | 12 | 0 | 199.166667 | |
| PD4977a | 4 | 89546522 | 89548395 | 1873 | 9 | 0 | 214.333333 | |
| PD4977a | 4 | 106939416 | 106942693 | 3277 | 7 | 0 | 744.857143 | |
| PD4977a | 4 | 141714818 | 141716817 | 1999 | 8 | 0 | 263.125 | |
| PD4977a | 4 | 143723229 | 143726409 | 3180 | 8 | 0 | 449.75 | |
| PD4977a | 4 | 162591589 | 162593281 | 1692 | 7 | 0 | 242.285714 | |
| PD4977a | 5 | 11831197 | 11833137 | 1940 | 8 | 0 | 344.25 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD4977a | 5 | 81203071 | 81205553 | 2482 | 7 | 0 | 477.428571 | |
| PD4977a | 5 | 95025816 | 95030922 | 5106 | 20 | 0 | 289.9 | |
| PD4977a | 5 | 127727745 | 127736650 | 8905 | 28 | 0 | 329.678571 | |
| PD4977a | 6 | 1453706 | 1459440 | 5734 | 11 | 0 | 522.909091 | |
| PD4977a | 6 | 37377402 | 37381178 | 3776 | 27 | 0 | 166.777778 | |
| PD4977a | 6 | 37400171 | 37402670 | 2499 | 15 | 0 | 183.466667 | |
| PD4977a | 6 | 138718082 | 138724848 | 6766 | 8 | 0 | 879.375 | |
| PD4977a | 7 | 75301226 | 75306700 | 5474 | 8 | 0 | 684.375 | |
| PD4977a | 7 | 94652772 | 94655408 | 2636 | 6 | 0 | 517.666667 | |
| PD4977a | 7 | 151489024 | 151490509 | 1485 | 6 | 0 | 353.833333 | |
| PD4977a | 8 | 20161895 | 20165627 | 3732 | 8 | 0 | 607.75 | |
| PD4977a | 8 | 29287021 | 29291371 | 4350 | 6 | 0 | 844 | |
| PD4977a | 8 | 62327018 | 62333771 | 6753 | 23 | 0 | 293.695652 | |
| PD4977a | 8 | 101001137 | 101003504 | 2367 | 9 | 0 | 342.555556 | |
| PD4977a | 8 | 116213982 | 116219110 | 5128 | 8 | 0 | 810.5 | |
| PD4977a | 9 | 33370093 | 33378285 | 8192 | 20 | 0 | 410.2 | |
| PD4977a | 9 | 37559338 | 37566206 | 6868 | 9 | 0 | 763.777778 | |
| PD4977a | 9 | 124723630 | 124729293 | 5663 | 11 | 0 | 521.636364 | |
| PD4977a | 9 | 129502056 | 129503111 | 1055 | 6 | 0 | 196.166667 | |
| PD4977a | 9 | 137391244 | 137397913 | 6669 | 13 | 0 | 580.461538 | |
| PD4977a | X | 46532387 | 46541088 | 8701 | 12 | 0 | 748.75 | |
| PD5925a | 15 | 77633626 | 77642237 | 8611 | 10 | 0 | 937 | |
| PD5925a | 3 | 59039469 | 59044106 | 4637 | 6 | 0 | 835.5 | |
| PD5932a | 4 | 66643706 | 66643832 | 126 | 7 | 0 | 60.5714286 | |
| PD5934a | 18 | 22670285 | 22672685 | 2400 | 6 | 0 | 492.5 | |
| PD5942a | 11 | 40330486 | 40331154 | 668 | 10 | 0 | 151.6 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD5942a | 19 | 5376375 | 5379753 | 3378 | 9 | 0 | 380.333333 | |
| PD5942a | 19 | 12140444 | 12141488 | 1044 | 7 | 0 | 290.857143 | |
| PD5944a | 2 | 217349796 | 217351677 | 1881 | 6 | 0 | 437.5 | |
| PD6046a | 11 | 77274872 | 77275158 | 286 | 7 | 0 | 719 | |
| PD6046a | 12 | 32785801 | 32788025 | 2224 | 15 | 0 | 154.333333 | |
| PD6046a | 17 | 35351367 | 35352546 | 1179 | 8 | 0 | 181.625 | |
| PD6046a | 6 | 52985738 | 52986732 | 994 | 7 | 0 | 162 | |
| PD6046a | 7 | 65881683 | 65885841 | 4158 | 8 | 0 | 523.75 | |
| PD6047a | 11 | 90275937 | 90277739 | 1802 | 6 | 0 | 490.833333 | |
| PD6047a | 11 | 96614145 | 96615719 | 1574 | 6 | 0 | 274.333333 | |
| PD6047a | 17 | 12247607 | 12248871 | 1264 | 13 | 0 | 99.2307692 | |
| PD6047a | 6 | 279403 | 281902 | 2499 | 19 | 0 | 133.473684 | |
| PD6047a | 6 | 288866 | 294421 | 5555 | 16 | 0 | 353.4375 | |
| PD6047a | 6 | 15872571 | 15874807 | 2236 | 6 | 0 | 418.833333 | |
| PD6047a | 7 | 66611744 | 66613242 | 1498 | 7 | 0 | 221.571429 | |
| PD6047a | X | 16603493 | 16606630 | 3137 | 9 | 0 | 352.111111 | |
| PD6047a | X | 102564500 | 102568929 | 4429 | 12 | 0 | 418 | |
| PD6415a | 3 | 84136786 | 84141965 | 5179 | 20 | 0 | 277.65 | |
| PD6415a | 3 | 154330983 | 154331893 | 910 | 7 | 0 | 162.428571 | |
| PD6416a | 4 | 157977201 | 157978931 | 1730 | 6 | 0 | 292.333333 | |
| PD6416a | 7 | 53977246 | 53978178 | 932 | 6 | 0 | 181.5 | |
| PD6727b | 1 | 61397719 | 61398545 | 826 | 9 | 0 | 102.777778 | |
| PD6727b | 1 | 116120058 | 116120338 | 280 | 6 | 0 | 53.5 | |
| PD6727b | 1 | 204295520 | 204296749 | 1229 | 12 | 0 | 127.416667 | |
| PD6727b | 10 | 25516981 | 25523878 | 6897 | 18 | 0 | 383.888889 | |
| PD6727b | 10 | 25533701 | 25536426 | 2725 | 15 | 0 | 189 | |

(continued)

| sample | chr | start.bp | end.bp | length.bp | number.bps | number.bps.clustered | avgDist.bp | alternative.kataegis |
|---|---|---|---|---|---|---|---|---|
| PD6727b | 10 | 28116919 | 28117742 | 823 | 8 | 0 | 125.125 | |
| PD6727b | 10 | 30265729 | 30268786 | 3057 | 8 | 0 | 697.5 | |
| PD6727b | 10 | 34482151 | 34485211 | 3060 | 11 | 0 | 323.181818 | |
| PD6727b | 12 | 75398425 | 75400043 | 1618 | 7 | 0 | 249.714286 | |
| PD6727b | 14 | 38536420 | 38539205 | 2785 | 12 | 0 | 282.666667 | |
| PD6727b | 14 | 39808096 | 39808895 | 799 | 11 | 0 | 155.727273 | |
| PD6727b | 4 | 37339591 | 37344559 | 4968 | 7 | 0 | 734.571429 | |
| PD6727b | 4 | 104179663 | 104180578 | 915 | 6 | 0 | 275.833333 | |
| PD6727b | 4 | 108169447 | 108170932 | 1485 | 6 | 0 | 294.166667 | |
| PD6727b | 4 | 108826663 | 108831845 | 5182 | 25 | 0 | 208.8 | |
| PD6727b | 4 | 120758717 | 120766308 | 7591 | 23 | 0 | 346.608696 | |
| PD6727b | 8 | 113764273 | 113764678 | 405 | 7 | 0 | 77.1428571 | |
| PD6729a2 | 10 | 20314878 | 20315639 | 761 | 6 | 0 | 349 | |
| PD6729a2 | 16 | 27667174 | 27667368 | 194 | 6 | 0 | 38.6666667 | |
| PD6731a2 | 1 | 178777771 | 178779254 | 1483 | 6 | 0 | 308.333333 | |
| PD6731a2 | 1 | 178789480 | 178794259 | 4779 | 12 | 0 | 425.166667 | |
| PD6731a2 | 2 | 17270933 | 17283317 | 12384 | 17 | 0 | 735.588235 | |
| PD6731a2 | 2 | 48565614 | 48570465 | 4851 | 6 | 0 | 843 | |
| PD6731a2 | 4 | 2316572 | 2347522 | 30950 | 70 | 0 | 442.628571 | |
| PD6731a2 | 5 | 120413257 | 120417169 | 3912 | 13 | 0 | 303.461538 | |
| PD6731a2 | 9 | 2246860 | 2265556 | 18696 | 27 | 0 | 741.037037 | |
| PD6732b | 1 | 78443393 | 78443790 | 397 | 7 | 0 | 64 | |
| PD6732b | 8 | 50160172 | 50160454 | 282 | 6 | 0 | 56.3333333 | |
| PD6733b | 17 | 8155554 | 8160641 | 5087 | 8 | 0 | 790.75 | |
| PD6733b | 2 | 157410651 | 157414752 | 4101 | 10 | 0 | 416.9 | |
| PD6733b | 5 | 7116179 | 7116440 | 261 | 7 | 0 | 43.5714286 | |

(continued)

| sample | chr | start.bp | end.bp | length.bp | number.bps | number.bps.clustered | avgDist.bp | alternative.kataegis |
|---|---|---|---|---|---|---|---|---|
| PD6733b | 5 | 25993450 | 25995049 | 1599 | 12 | 0 | 145.5 | |
| PD7066a | 10 | 65391158 | 65391884 | 726 | 7 | 0 | 158 | |
| PD7067a | 1 | 187850157 | 187853744 | 3587 | 7 | 0 | 530.285714 | |
| PD7067a | 11 | 97064788 | 97067752 | 2964 | 7 | 0 | 485 | |
| PD7067a | 20 | 13452844 | 13454118 | 1274 | 6 | 0 | 229.666667 | |
| PD7067a | 5 | 13614019 | 13619350 | 5331 | 18 | 0 | 374.833333 | |
| PD7067a | 5 | 13854337 | 13856835 | 2498 | 7 | 0 | 490 | |
| PD7067a | 7 | 16647804 | 16648266 | 462 | 7 | 0 | 366.714286 | |
| PD7067a | 8 | 43298806 | 43299539 | 733 | 10 | 0 | 75.7 | |
| PD7067a | 8 | 121178703 | 121179191 | 488 | 6 | 0 | 103.833333 | |
| PD7067a | 8 | 131146656 | 131148870 | 2214 | 6 | 0 | 416.5 | |
| PD7067a | X | 46333321 | 46333884 | 563 | 6 | 0 | 95 | |
| PD7069a | 1 | 32270413 | 32279094 | 8681 | 19 | 0 | 486.052632 | |
| PD7069a | 11 | 85301139 | 85303370 | 2231 | 11 | 0 | 212 | |
| PD7211a | 2 | 35964243 | 35965460 | 1217 | 12 | 0 | 108.583333 | |
| PD7211a | 2 | 171229100 | 171231072 | 1972 | 6 | 0 | 386 | |
| PD7211a | 2 | 181413152 | 181415950 | 2798 | 16 | 0 | 177.75 | |
| PD7211a | 3 | 80125955 | 80127256 | 1301 | 6 | 0 | 230.333333 | yes |
| PD7211a | 3 | 80300433 | 80302545 | 2112 | 6 | 0 | 359.666667 | yes |
| PD7217a | 3 | 131269693 | 131274731 | 5038 | 14 | 0 | 393.928571 | |
| PD7217a | 3 | 143878310 | 143880043 | 1733 | 6 | 0 | 293 | |
| PD7217a | 4 | 6728126 | 6741734 | 13608 | 20 | 0 | 778.45 | |
| PD7217a | 6 | 53379682 | 53389407 | 9725 | 15 | 0 | 783 | |
| PD7220a | 2 | 150560596 | 150569812 | 9216 | 17 | 0 | 566.058824 | |
| PD7250a | 6 | 46938865 | 46939567 | 702 | 11 | 0 | 71.9090909 | |
| PD7341a | 14 | 36031072 | 36031350 | 278 | 9 | 0 | 33.7777778 | |

| sample | chr | start.bp | end.bp | length.bp | number.bps | number.bps.clustered | avgDist.bp | alternative.kataegis |
|--------|-----|----------|--------|-----------|------------|---------------------|-----------|---------------------|
| PD7341a | 17 | 25782779 | 25792018 | 9239 | 12 | 0 | 773.75 | |
| PD7341a | 22 | 50126544 | 50127000 | 456 | 7 | 0 | 67.4285714 | |
| PD7341a | 6 | 79099260 | 79102979 | 3719 | 10 | 0 | 982.9 | |
| PD7341a | 6 | 102146602 | 102147214 | 612 | 7 | 0 | 187.428571 | |
| PD7344a | 10 | 25785591 | 25794839 | 9248 | 21 | 0 | 690.619048 | |
| PD7426a | 11 | 132789187 | 132792896 | 3709 | 9 | 0 | 433.555556 | |
| PD7426a | 3 | 135200446 | 135203068 | 2622 | 12 | 0 | 221.25 | |
| PD7426a | 3 | 149270729 | 149274164 | 3435 | 6 | 0 | 574.5 | |
| PD7426a | 3 | 194655925 | 194658020 | 2095 | 7 | 0 | 345.285714 | |
| PD7428a | 2 | 121079637 | 121081159 | 1522 | 11 | 0 | 154.636364 | |
| PD8621a | 6 | 7769345 | 7772929 | 3584 | 12 | 0 | 306.166667 | |
| PD8660a2 | 1 | 93661425 | 93662192 | 767 | 8 | 0 | 124.375 | |
| PD8660a2 | 1 | 164804030 | 164804999 | 969 | 8 | 0 | 305.625 | |
| PD8660a2 | 16 | 244347 | 244564 | 217 | 7 | 0 | 37.7142857 | |
| PD8660a2 | 18 | 9691147 | 9693211 | 2064 | 7 | 0 | 302 | |
| PD8660a2 | 18 | 14612698 | 14615140 | 2442 | 13 | 0 | 205.923077 | |
| PD8660a2 | 19 | 46688343 | 46689059 | 716 | 6 | 0 | 137.833333 | |
| PD8660a2 | 2 | 58194096 | 58195404 | 1308 | 6 | 0 | 256.833333 | |
| PD8660a2 | 2 | 158132572 | 158133553 | 981 | 11 | 0 | 113.545455 | |
| PD8660a2 | 21 | 21056385 | 21058138 | 1753 | 7 | 0 | 251.142857 | |
| PD8660a2 | 4 | 118788423 | 118789502 | 1079 | 6 | 0 | 238.166667 | |
| PD8660a2 | 6 | 75561168 | 75561353 | 185 | 6 | 0 | 36 | |
| PD8660a2 | 8 | 36271521 | 36273076 | 1555 | 10 | 0 | 162.2 | |
| PD8830a | 12 | 6468568 | 6469887 | 1319 | 6 | 0 | 240 | |
| PD8830a | 9 | 77788941 | 77792666 | 3725 | 6 | 0 | 837.666667 | |
| PD8832a | X | 55569515 | 55570007 | 492 | 6 | 0 | 124 | |

(continued)

| sample | chr | start.bp | end.bp | length.bp | number.bps | number.bps.clustered | avgDist.bp | alternative.kataegis |
|---|---|---|---|---|---|---|---|---|
| PD8969a | 1 | 183056724 | 183058722 | 1998 | 10 | 0 | 204.7 | |
| PD8969a | 1 | 184621741 | 184622851 | 1110 | 13 | 0 | 95.9230769 | |
| PD8969a | 1 | 202941273 | 202943975 | 2702 | 18 | 0 | 155.555556 | |
| PD8969a | 12 | 1242951 | 1244409 | 1458 | 13 | 0 | 162.307692 | |
| PD8969a | 18 | 5434882 | 5435620 | 738 | 7 | 0 | 250.857143 | |
| PD8969a | 19 | 10984556 | 10984732 | 176 | 6 | 0 | 31.6666667 | |
| PD8969a | 7 | 80412860 | 80417949 | 5089 | 8 | 0 | 750.625 | |
| PD8969a | 9 | 23767846 | 23778070 | 10224 | 30 | 0 | 358.133333 | |
| PD8969a | 9 | 24921812 | 24923755 | 1943 | 6 | 0 | 331.833333 | |
| PD8969a | 9 | 108893635 | 108894465 | 830 | 6 | 0 | 144 | |
| PD8982a | 11 | 62700312 | 62701447 | 1135 | 7 | 0 | 211.571429 | |
| PD8982a | 14 | 34109850 | 34110931 | 1081 | 10 | 0 | 116 | |
| PD8982a | 5 | 60930167 | 60933306 | 3139 | 10 | 0 | 327.1 | |
| PD8982a | 7 | 65827437 | 65828420 | 983 | 12 | 0 | 103.75 | |
| PD8982a | 8 | 76811834 | 76814061 | 2227 | 16 | 0 | 155.625 | |
| PD8982a | 8 | 78130483 | 78131438 | 955 | 12 | 0 | 86.25 | |
| PD8982a | 8 | 88021446 | 88022210 | 764 | 10 | 0 | 83.6 | |
| PD8982a | 8 | 94127275 | 94128586 | 1311 | 11 | 0 | 122.090909 | |
| PD8982a | 8 | 112574588 | 112578027 | 3439 | 10 | 0 | 515.7 | |
| PD8984a | 7 | 82556488 | 82561104 | 4616 | 16 | 0 | 298.875 | |
| PD9004a | 4 | 46604894 | 46606326 | 1432 | 7 | 0 | 221.142857 | |
| PD9004a | 6 | 22568969 | 22571417 | 2448 | 19 | 0 | 130.263158 | |
| PD9004a | 7 | 7664598 | 7673134 | 8536 | 10 | 0 | 864.6 | |
| PD9004a | 8 | 115477322 | 115477900 | 578 | 7 | 0 | 115 | |
| PD9004a | 8 | 131999493 | 132006013 | 6520 | 9 | 0 | 735.111111 | |
| PD9064a | 18 | 60081331 | 60082788 | 1457 | 6 | 0 | 255.666667 | |

(continued)

| sample | chr | start.bp | end.bp | length.bp | number.bps | number.bps.clustered | avgDist.bp | alternative.kataegis |
|---|---|---|---|---|---|---|---|---|
| PD9064a | 3 | 180925908 | 180929348 | 3440 | 6 | 0 | 580.166667 | |
| PD9568a | 18 | 22123579 | 22126087 | 2508 | 13 | 0 | 197.923077 | |
| PD9571a | 10 | 122786258 | 122792032 | 5774 | 7 | 0 | 840.857143 | |
| PD9571a | 12 | 84959893 | 84960772 | 879 | 7 | 0 | 154.428571 | |
| PD9571a | 6 | 106661290 | 106662009 | 719 | 7 | 0 | 106 | |
| PD9585a | 16 | 30709643 | 30710756 | 1113 | 7 | 0 | 184.142857 | |
| PD9585a | 5 | 44553283 | 44554585 | 1302 | 8 | 0 | 232 | |
| PD9585a | 6 | 64562562 | 64575278 | 12716 | 23 | 0 | 552.956522 | |
| PD9585a | 6 | 69822346 | 69826060 | 3714 | 16 | 0 | 236 | |
| PD9595a | 11 | 18928121 | 18929465 | 1344 | 8 | 0 | 168.375 | |
| PD9595a | 22 | 33242056 | 33247608 | 5552 | 27 | 0 | 206.925926 | |
| PD9595a | 22 | 33284104 | 33285808 | 1704 | 14 | 0 | 121.785714 | |
| PD9595a | 3 | 7773311 | 7780869 | 7558 | 8 | 0 | 953.25 | |
| PD9696a | X | 9450201 | 9451893 | 1692 | 15 | 0 | 119.466667 | |
| PD9702a | 2 | 129354779 | 129357343 | 2564 | 15 | 0 | 185.4 | |
| PD9702a | 4 | 53875075 | 53876249 | 1174 | 6 | 0 | 204.5 | |
| PD10014a | 3 | 137074163 | 137078060 | 3897 | 9 | 0 | 436.888889 | |
| PD10014a | 3 | 137093055 | 137093792 | 737 | 6 | 0 | 125.833333 | |
| PD11326a | 1 | 175323834 | 175326464 | 2630 | 22 | 0 | 128.454545 | |
| PD11326a | 10 | 50099543 | 50100397 | 854 | 9 | 0 | 96.4444444 | |
| PD11326a | 16 | 13926866 | 13929410 | 2544 | 7 | 0 | 383.571429 | |
| PD11327a | 14 | 34387950 | 34388494 | 544 | 12 | 0 | 46.75 | |
| PD11327a | 4 | 65197356 | 65202759 | 5403 | 7 | 0 | 853.142857 | |
| PD11327a | 4 | 139538368 | 139545399 | 7031 | 13 | 0 | 623.153846 | |
| PD11752a | 6 | 62984792 | 62985327 | 535 | 6 | 0 | 142.666667 | |
| PD11755a | 5 | 71855937 | 71857079 | 1142 | 10 | 0 | 118.1 | |

(continued)

| sample | chr | start.bp | end.bp | length.bp | number.bps | number.bps.clustered | avgDist.bp | alternative.kataegis |
|---|---|---|---|---|---|---|---|---|
| PD13296a | 11 | 31636936 | 31639649 | 2713 | 11 | 0 | 251.181818 | |
| PD13296a | 11 | 55696364 | 55696911 | 547 | 8 | 0 | 75.125 | |
| PD13296a | 2 | 142992030 | 142993219 | 1189 | 7 | 0 | 176.714286 | |
| PD13297a | 7 | 142637908 | 142640150 | 2242 | 19 | 0 | 126.736842 | |
| PD13298a | 10 | 71449315 | 71452638 | 3323 | 9 | 0 | 376.444444 | |
| PD13298a | 11 | 125735483 | 125736103 | 620 | 9 | 0 | 93.3333333 | |
| PD13298a | 12 | 2063536 | 2067748 | 4212 | 11 | 0 | 430.181818 | |
| PD13298a | 19 | 4193355 | 4196685 | 3330 | 9 | 0 | 385.222222 | |
| PD13298a | 5 | 52559243 | 52562334 | 3091 | 8 | 0 | 438.875 | |
| PD13298a | 8 | 38683494 | 38686275 | 2781 | 7 | 0 | 440.285714 | |
| PD13299a | 6 | 28122429 | 28129661 | 7232 | 9 | 0 | 870.444444 | |
| PD13311a | 22 | 35133362 | 35139120 | 5758 | 9 | 0 | 639.888889 | |
| PD13418a | 1 | 120529680 | 120530332 | 652 | 6 | 0 | 114.166667 | |
| PD13418a | 10 | 62295424 | 62299009 | 3585 | 12 | 0 | 317.5 | |
| PD13418a | 10 | 73361473 | 73367102 | 5629 | 7 | 0 | 804.571429 | |
| PD13418a | 8 | 51803301 | 51805836 | 2535 | 6 | 0 | 604.166667 | |
| PD13428a | 1 | 119903560 | 119905512 | 1952 | 6 | 0 | 378 | |
| PD13428a | 8 | 77917097 | 77921488 | 4391 | 19 | 0 | 277.315789 | |
| PD18020a | 13 | 63283448 | 63285191 | 1743 | 13 | 0 | 175.769231 | |
| PD18020a | 13 | 65794978 | 65795777 | 799 | 7 | 0 | 132 | |
| PD18020a | 7 | 100291716 | 100295865 | 4149 | 10 | 0 | 449.3 | |
| PD18020a | 7 | 100300078 | 100309460 | 9382 | 27 | 0 | 349.62963 | |
| PD18024a | 2 | 183139529 | 183143060 | 3531 | 9 | 0 | 399.444444 | |
| PD18031a | 18 | 3906597 | 3910424 | 3827 | 10 | 0 | 472.1 | |
| PD18031a | 18 | 4384758 | 4385956 | 1198 | 10 | 0 | 146 | |
| PD18031a | 6 | 135676963 | 135677495 | 532 | 6 | 0 | 111 | |

(continued)

| sample | chr | start.bp | end.bp | length.bp | number.bps | number.bps.clustered | avgDist.bp | alternative.kataegis |
|---|---|---|---|---|---|---|---|---|
| PD18031a | 8 | 37373479 | 37374749 | 1270 | 14 | 0 | 91 | |
| PD18037a | 21 | 33359167 | 33360467 | 1300 | 6 | 0 | 226.5 | |
| PD18037a | 8 | 5123283 | 5124246 | 963 | 7 | 0 | 305.285714 | |
| PD6684a | 11 | 76537013 | 76540480 | 3467 | 12 | 0 | 321.333333 | |
| PD8619a | 11 | 65314002 | 65315751 | 1749 | 10 | 0 | 202.7 | |
| PD8619a | 11 | 81623695 | 81624433 | 738 | 7 | 0 | 114.857143 | |
| PD8619a | 11 | 81993345 | 81997442 | 4097 | 8 | 0 | 868.375 | |
| PD8619a | 11 | 82427563 | 82428635 | 1072 | 11 | 0 | 132.363636 | |
| PD8619a | 11 | 84011044 | 84011873 | 829 | 6 | 0 | 222.166667 | |
| PD8619a | 18 | 37409873 | 37411188 | 1315 | 6 | 0 | 236 | |
| PD8619a | 3 | 86949132 | 86950950 | 1818 | 9 | 0 | 204.777778 | |
| PD8619a | 6 | 24300564 | 24302250 | 1686 | 8 | 0 | 214.625 | |
| PD8619a | 6 | 39015211 | 39018195 | 2984 | 6 | 0 | 506.666667 | |
| PD8619a | 6 | 71322362 | 71323358 | 996 | 6 | 0 | 186.833333 | |
| PD8619a | 6 | 150253398 | 150260884 | 7486 | 9 | 0 | 982.888889 | |
| PD8619a | 8 | 42162346 | 42168762 | 6416 | 7 | 0 | 980.428571 | |
| PD8619a | 9 | 24209474 | 24210666 | 1192 | 8 | 0 | 170.625 | |
| PD10010a | 1 | 31844650 | 31845215 | 565 | 8 | 0 | 131.375 | |
| PD10010a | 1 | 154646437 | 154649179 | 2742 | 14 | 0 | 452.5 | |
| PD10010a | 1 | 168897301 | 168900672 | 3371 | 28 | 0 | 123 | |
| PD10010a | 1 | 178125744 | 178140910 | 15166 | 28 | 0 | 541.964286 | |
| PD10010a | 1 | 178829501 | 178831265 | 1764 | 11 | 0 | 245.181818 | |
| PD10010a | 1 | 178982085 | 178984379 | 2294 | 10 | 0 | 245.6 | |
| PD10010a | 10 | 73091487 | 73094222 | 2735 | 8 | 0 | 459.125 | |
| PD10010a | 16 | 30366979 | 30368268 | 1289 | 11 | 0 | 129.181818 | |
| PD10010a | 2 | 95828923 | 95830605 | 1682 | 7 | 0 | 416 | |

| sample | chr | start.bp | end.bp | length.bp | number.bps | number.bps.clustered | avgDist.bp | alternative.kataegis |
|---|---|---|---|---|---|---|---|---|
| PD13630a | 8 | 51118237 | 51123172 | 4935 | 7 | 0 | 717.857143 | |
| PD11344a | 1 | 12558167 | 12559537 | 1370 | 7 | 0 | 252.714286 | |
| PD11344a | 1 | 22680381 | 22685301 | 4920 | 6 | 0 | 848.833333 | |
| PD11344a | 1 | 229942214 | 229946277 | 4063 | 8 | 0 | 610 | |
| PD11344a | 10 | 45788083 | 45790224 | 2141 | 6 | 0 | 363.333333 | |
| PD11344a | 13 | 105615067 | 105618723 | 3656 | 6 | 0 | 719.5 | |
| PD11344a | 14 | 65623012 | 65632874 | 9862 | 16 | 0 | 717.625 | |
| PD11344a | 15 | 44257887 | 44261138 | 3251 | 7 | 0 | 517.571429 | |
| PD11344a | 15 | 64562802 | 64563682 | 880 | 6 | 0 | 275.166667 | |
| PD11344a | 16 | 14070451 | 14077885 | 7434 | 8 | 0 | 974.125 | |
| PD11344a | 16 | 70439883 | 70441537 | 1654 | 7 | 0 | 254.428571 | |
| PD11344a | 16 | 72436590 | 72442288 | 5698 | 19 | 0 | 300.210526 | |
| PD11344a | 17 | 37833092 | 37836699 | 3607 | 11 | 0 | 433.272727 | |
| PD11344a | 20 | 20195265 | 20195789 | 524 | 8 | 0 | 71.875 | |
| PD11344a | 20 | 25101278 | 25102083 | 805 | 8 | 0 | 101.5 | |
| PD11344a | 20 | 25112304 | 25113243 | 939 | 16 | 0 | 59.25 | |
| PD11344a | 3 | 134798014 | 134799555 | 1541 | 13 | 0 | 127.230769 | |
| PD11344a | 3 | 191458399 | 191464087 | 5688 | 7 | 0 | 816 | |
| PD11344a | 4 | 174085505 | 174089247 | 3742 | 6 | 0 | 626.166667 | |
| PD11344a | 8 | 74686121 | 74692689 | 6568 | 16 | 0 | 412 | |
| PD11344a | 8 | 117164741 | 117168052 | 3311 | 14 | 0 | 265.714286 | |
| PD11344a | X | 114614480 | 114620450 | 5970 | 14 | 0 | 462.571429 | |
| PD11344a | X | 135985440 | 135987642 | 2202 | 6 | 0 | 420.666667 | |
| PD11345a | 17 | 28598544 | 28598761 | 217 | 6 | 0 | 46 | |
| PD11345a | 17 | 78166132 | 78168806 | 2674 | 8 | 0 | 341.75 | |
| PD11345a | 2 | 72823308 | 72824451 | 1143 | 8 | 0 | 146.75 | |

(continued)

| sample | chr | start.bp | end.bp | length.bp | number.bps | number.bps.clustered | avgDist.bp | alternative.kataegis |
|---|---|---|---|---|---|---|---|---|
| PD11345a | 20 | 48961796 | 48964515 | 2719 | 8 | 0 | 355.5 | |
| PD11345a | 20 | 51849100 | 51854918 | 5818 | 11 | 0 | 617.727273 | |
| PD11345a | 20 | 52991546 | 52991891 | 345 | 7 | 0 | 69.2857143 | |
| PD11345a | 20 | 53349873 | 53354121 | 4248 | 8 | 0 | 678 | |
| PD11345a | 20 | 53835789 | 53843303 | 7514 | 20 | 0 | 555.85 | |
| PD11345a | 20 | 53923023 | 53929664 | 6641 | 24 | 0 | 291.125 | |
| PD11345a | 20 | 62907900 | 62908985 | 1085 | 13 | 0 | 92.4615385 | |
| PD11345a | 8 | 107944078 | 107949929 | 5851 | 12 | 0 | 489.583333 | |
| PD11346a | 1 | 239125753 | 239126033 | 280 | 6 | 0 | 51.1666667 | |
| PD11346a | 8 | 83273204 | 83274201 | 997 | 6 | 0 | 190.5 | |
| PD11346a | 8 | 124047170 | 124053009 | 5839 | 11 | 0 | 565.363636 | |
| PD11347a | 11 | 71028806 | 71033024 | 4218 | 26 | 0 | 166.307692 | |
| PD11347a | 8 | 28654822 | 28659059 | 4237 | 12 | 0 | 361.333333 | |
| PD11347a | 8 | 59613359 | 59617170 | 3811 | 14 | 0 | 278.785714 | |
| PD11347a | 8 | 118150466 | 118154098 | 3632 | 12 | 0 | 308.083333 | |
| PD11348a | X | 35654192 | 35654962 | 770 | 7 | 0 | 306.857143 | |
| PD11349a | 1 | 53481199 | 53481492 | 293 | 6 | 0 | 63.5 | |
| PD11349a | 1 | 59263765 | 59265683 | 1918 | 7 | 0 | 274.142857 | |
| PD11349a | 1 | 62463975 | 62465064 | 1089 | 8 | 0 | 139 | |
| PD11349a | 1 | 62893654 | 62895019 | 1365 | 9 | 0 | 164.666667 | |
| PD11349a | 1 | 63134497 | 63143462 | 8965 | 25 | 0 | 359.4 | |
| PD11349a | 1 | 242797369 | 242799300 | 1931 | 11 | 0 | 183.454545 | |
| PD11349a | 10 | 26359848 | 26360815 | 967 | 10 | 0 | 458.2 | |
| PD11349a | 12 | 72615980 | 72616456 | 476 | 7 | 0 | 143.285714 | |
| PD11349a | 13 | 40912407 | 40916857 | 4450 | 6 | 0 | 742.333333 | |
| PD11349a | 13 | 51281564 | 51284778 | 3214 | 7 | 0 | 504.285714 | |

| sample | chr | start.bp | end.bp | length.bp | number.bps | number.bps.clustered | avgDist.bp | alternative.kataegis |
|---|---|---|---|---|---|---|---|---|
| PD11349a | 13 | 60350926 | 60357152 | 6226 | 18 | 0 | 393.277778 | |
| PD11349a | 13 | 73290441 | 73294267 | 3826 | 12 | 0 | 319 | |
| PD11349a | 4 | 62465476 | 62467256 | 1780 | 6 | 0 | 309.5 | |
| PD11349a | 4 | 64607270 | 64607687 | 417 | 9 | 0 | 73.1111111 | |
| PD11349a | 4 | 66223719 | 66225067 | 1348 | 7 | 0 | 210.142857 | |
| PD11349a | 4 | 66435801 | 66438247 | 2446 | 19 | 0 | 181.894737 | |
| PD11349a | 4 | 165799717 | 165800443 | 726 | 10 | 0 | 79.7 | |
| PD11349a | 6 | 84642644 | 84647831 | 5187 | 14 | 0 | 376.785714 | |
| PD11349a | 6 | 84812833 | 84815638 | 2805 | 8 | 0 | 358.875 | |
| PD11349a | 6 | 102836981 | 102838388 | 1407 | 13 | 0 | 112.307692 | |
| PD11349a | 6 | 102847187 | 102850803 | 3616 | 17 | 0 | 220.529412 | |
| PD11349a | 6 | 107653020 | 107653209 | 189 | 8 | 0 | 28 | |
| PD11349a | X | 65453056 | 65455465 | 2409 | 6 | 0 | 404 | |
| PD11465a | 1 | 61188234 | 61189630 | 1396 | 10 | 0 | 201.6 | |
| PD11465a | 17 | 56078036 | 56079211 | 1175 | 11 | 0 | 109.818182 | |
| PD11465a | 5 | 46101284 | 46105217 | 3933 | 18 | 0 | 221.388889 | |
| PD11465a | 6 | 26175024 | 26176744 | 1720 | 16 | 0 | 119.0625 | |
| PD11465a | 6 | 26200419 | 26201591 | 1172 | 7 | 0 | 247.571429 | |
| PD13162a | 16 | 34774414 | 34774931 | 517 | 9 | 0 | 59.4444444 | |
| PD13162a | 16 | 72152471 | 72152647 | 176 | 7 | 0 | 32.5714286 | |
| PD13163a | 11 | 46255638 | 46256868 | 1230 | 8 | 0 | 176 | |
| PD13163a | 6 | 127389820 | 127399200 | 9380 | 11 | 0 | 863.818182 | |
| PD13163a | 9 | 32964856 | 32965775 | 919 | 8 | 0 | 121.5 | |
| PD13164a | 17 | 34049324 | 34054058 | 4734 | 9 | 0 | 910.111111 | |
| PD13164a | 17 | 36783663 | 36784467 | 804 | 12 | 0 | 67.25 | yes |
| PD13164a | 17 | 37418944 | 37419205 | 261 | 8 | 0 | 59.875 | yes |

(continued)

| sample | chr | start.bp | end.bp | length.bp | number.bps | number.bps.clustered | avgDist.bp | alternative.kataegis |
|---|---|---|---|---|---|---|---|---|
| PD13164a | 17 | 59700360 | 59700738 | 378 | 9 | 0 | 63 | |
| PD13164a | 17 | 63479262 | 63479779 | 517 | 9 | 0 | 60.7777778 | |
| PD13165a | 3 | 22869191 | 22871014 | 1823 | 17 | 0 | 114.705882 | |
| PD13166a | 6 | 506664 | 508600 | 1936 | 11 | 0 | 179.545455 | |
| PD13166a | 6 | 605625 | 608734 | 3109 | 27 | 0 | 117.592593 | |
| PD13167a | 1 | 172406962 | 172410284 | 3322 | 8 | 0 | 462.625 | |
| PD13167a | 1 | 173130396 | 173130948 | 552 | 7 | 0 | 100.857143 | |
| PD13167a | 1 | 173453739 | 173455076 | 1337 | 7 | 0 | 207.428571 | |
| PD13167a | 11 | 123994427 | 123995119 | 692 | 8 | 0 | 565.75 | |
| PD13167a | 17 | 52533589 | 52534578 | 989 | 7 | 0 | 161.428571 | |
| PD13167a | 18 | 14632240 | 14632458 | 218 | 6 | 0 | 42 | |
| PD13167a | 5 | 111392703 | 111393834 | 1131 | 13 | 0 | 89.0769231 | |
| PD13602a | 16 | 81739429 | 81743179 | 3750 | 9 | 0 | 512 | |
| PD13602a | 20 | 56864954 | 56869929 | 4975 | 23 | 0 | 227.478261 | |
| PD13602a | 4 | 6486492 | 6489891 | 3399 | 11 | 0 | 502.454545 | |
| PD13603a | 1 | 41574987 | 41577251 | 2264 | 17 | 0 | 136.882353 | |
| PD13603a | 1 | 61923084 | 61924326 | 1242 | 9 | 0 | 153.333333 | |
| PD13603a | 1 | 62332256 | 62333041 | 785 | 8 | 0 | 104.25 | |
| PD13603a | 21 | 20461175 | 20462647 | 1472 | 14 | 0 | 109.857143 | |
| PD13603a | 6 | 44451326 | 44452258 | 932 | 6 | 0 | 160 | |
| PD13604a | 1 | 39616867 | 39618745 | 1878 | 7 | 0 | 403 | |
| PD13604a | 1 | 151348552 | 151350468 | 1916 | 7 | 0 | 313.428571 | |
| PD13604a | 1 | 224490266 | 224494652 | 4386 | 6 | 0 | 907.833333 | |
| PD13604a | 12 | 40947725 | 40950377 | 2652 | 7 | 0 | 420.714286 | |
| PD13604a | 13 | 98897288 | 98899302 | 2014 | 6 | 0 | 391.166667 | |
| PD13604a | 17 | 3882249 | 3885333 | 3084 | 9 | 0 | 344.444444 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD13604a | 17 | 3889479 | 3890969 | 1490 | 8 | 0 | 251.25 | |
| PD13604a | 17 | 7745258 | 7749879 | 4621 | 7 | 0 | 862.428571 | |
| PD13604a | 17 | 7758450 | 7767118 | 8668 | 21 | 0 | 430.47619 | |
| PD13604a | 17 | 27527474 | 27529823 | 2349 | 7 | 0 | 340.428571 | |
| PD13604a | 17 | 74366100 | 74370539 | 4439 | 6 | 0 | 778.666667 | |
| PD13604a | 19 | 50267949 | 50272970 | 5021 | 7 | 0 | 816 | |
| PD13604a | 2 | 9619108 | 9621797 | 2689 | 6 | 0 | 468.333333 | |
| PD13604a | 2 | 71069147 | 71071055 | 1908 | 6 | 0 | 361.166667 | |
| PD13604a | 2 | 100372928 | 100379139 | 6211 | 13 | 0 | 488.307692 | |
| PD13604a | 2 | 208777151 | 208778780 | 1629 | 7 | 0 | 335 | |
| PD13604a | 3 | 83852210 | 83852711 | 501 | 7 | 0 | 140.571429 | |
| PD13604a | 3 | 83861962 | 83862973 | 1011 | 9 | 0 | 112.666667 | |
| PD13604a | 3 | 131153843 | 131156379 | 2536 | 8 | 0 | 322.875 | |
| PD13604a | 3 | 174566304 | 174571490 | 5186 | 10 | 0 | 544.4 | |
| PD13604a | 5 | 51195051 | 51196309 | 1258 | 12 | 0 | 126.916667 | |
| PD13604a | 6 | 31661301 | 31663505 | 2204 | 7 | 0 | 359 | |
| PD13604a | 7 | 104946989 | 104948612 | 1623 | 6 | 0 | 370 | |
| PD13604a | X | 24988776 | 24990267 | 1491 | 6 | 0 | 266.666667 | |
| PD13606a | 11 | 61938045 | 61939129 | 1084 | 6 | 0 | 223.166667 | |
| PD13606a | 12 | 86951187 | 86952735 | 1548 | 9 | 0 | 172.333333 | |
| PD13606a | 12 | 90382797 | 90385733 | 2936 | 15 | 0 | 215 | |
| PD13606a | 21 | 10638638 | 10639129 | 491 | 6 | 0 | 85.1666667 | |
| PD13606a | 3 | 24239684 | 24241402 | 1718 | 6 | 0 | 290.666667 | |
| PD13606a | 5 | 60476390 | 60479535 | 3145 | 7 | 0 | 526.142857 | |
| PD13607a | 16 | 34812527 | 34820141 | 7614 | 15 | 0 | 524.866667 | |
| PD13607a | 8 | 32487855 | 32491539 | 3684 | 19 | 0 | 195.894737 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD13608a | 1 | 20566380 | 20572882 | 6502 | 9 | 0 | 864.333333 | |
| PD13608a | 10 | 114683922 | 114691119 | 7197 | 8 | 0 | 963 | |
| PD13609a | 11 | 48324678 | 48338304 | 13626 | 28 | 0 | 515.25 | |
| PD13609a | 11 | 134931708 | 134932269 | 561 | 6 | 0 | 96.1666667 | |
| PD13609a | 14 | 36641145 | 36650511 | 9366 | 19 | 0 | 542.947368 | |
| PD13609a | 14 | 76882542 | 76885640 | 3098 | 9 | 0 | 345.777778 | |
| PD13609a | 15 | 53439519 | 53442252 | 2733 | 8 | 0 | 343.125 | |
| PD13609a | 16 | 54118236 | 54133127 | 14891 | 24 | 0 | 704.25 | |
| PD13609a | 19 | 36970569 | 36977194 | 6625 | 8 | 0 | 833.5 | |
| PD13609a | 22 | 24696682 | 24700245 | 3563 | 8 | 0 | 581.625 | |
| PD13609a | 22 | 24772440 | 24783954 | 11514 | 25 | 0 | 463.32 | |
| PD13609a | 6 | 5089866 | 5092180 | 2314 | 6 | 0 | 389 | |
| PD13609a | 6 | 109585915 | 109588994 | 3079 | 9 | 0 | 531.888889 | |
| PD13609a | 9 | 2362294 | 2367132 | 4838 | 7 | 0 | 743 | |
| PD13609a | 9 | 16551713 | 16555046 | 3333 | 7 | 0 | 510.285714 | |
| PD13609a | 9 | 22681763 | 22685623 | 3860 | 8 | 0 | 549.25 | |
| PD13609a | 9 | 27454683 | 27460335 | 5652 | 16 | 0 | 392.8125 | |
| PD13609a | 9 | 29590010 | 29591622 | 1612 | 6 | 0 | 364.333333 | |
| PD13609a | 9 | 30720253 | 30730247 | 9994 | 17 | 0 | 633.764706 | |
| PD18045a | 12 | 73741897 | 73742853 | 956 | 15 | 0 | 64.6666667 | |
| PD18045a | 13 | 111494943 | 111517176 | 22233 | 27 | 0 | 832.555556 | |
| PD18045a | 13 | 111532799 | 111537280 | 4481 | 7 | 0 | 950.428571 | |
| PD18045a | 17 | 4271086 | 4275950 | 4864 | 6 | 0 | 927.833333 | |
| PD18045a | 20 | 53981737 | 53983254 | 1517 | 9 | 0 | 169.444444 | |
| PD18045a | 4 | 44499265 | 44502239 | 2974 | 6 | 0 | 497.833333 | |
| PD18045a | 4 | 63417138 | 63421337 | 4199 | 6 | 0 | 704.166667 | |

(continued)

| sample | chr | start.bp | end.bp | length.bp | number.bps | number.bps.clustered | avgDist.bp | alternative.kataegis |
|---|---|---|---|---|---|---|---|---|
| PD18045a | 6 | 139419812 | 139420079 | 267 | 6 | 0 | 109.5 | |
| PD18045a | 8 | 71976812 | 71985627 | 8815 | 14 | 0 | 629.714286 | |
| PD18045a | 8 | 96446659 | 96447230 | 571 | 8 | 0 | 72.5 | |
| PD18045a | 8 | 139529105 | 139533726 | 4621 | 21 | 0 | 232.809524 | |
| PD18045a | 9 | 115980642 | 115997374 | 16732 | 21 | 0 | 857.428571 | |
| PD18046a | 17 | 18673300 | 18673675 | 375 | 7 | 0 | 54.7142857 | |
| PD18047a | 16 | 25442772 | 25450226 | 7454 | 16 | 0 | 470.5 | |
| PD18047a | 16 | 31161852 | 31163710 | 1858 | 7 | 0 | 295.142857 | |
| PD18047a | 17 | 35905460 | 35907183 | 1723 | 9 | 0 | 196.222222 | |
| PD18047a | 17 | 39159362 | 39166018 | 6656 | 12 | 0 | 573.75 | |
| PD18050a | 17 | 63268833 | 63270714 | 1881 | 7 | 0 | 275.571429 | |
| PD18050a | 18 | 26210450 | 26212596 | 2146 | 14 | 0 | 154.214286 | |
| PD18050a | 19 | 32744450 | 32750041 | 5591 | 16 | 0 | 429.5 | |
| PD18050a | 19 | 36357459 | 36359203 | 1744 | 14 | 0 | 227.5 | |
| PD18050a | 6 | 9309197 | 9311869 | 2672 | 7 | 0 | 390.857143 | |
| PD18050a | 6 | 50051404 | 50053849 | 2445 | 7 | 0 | 349.428571 | |
| PD18050a | 8 | 38137859 | 38140259 | 2400 | 22 | 0 | 109.727273 | |
| PD18188a | 17 | 33398309 | 33399668 | 1359 | 9 | 0 | 197 | |
| PD18188a | 17 | 39434011 | 39435499 | 1488 | 9 | 0 | 175.888889 | |
| PD18188a | 8 | 29734847 | 29735337 | 490 | 7 | 0 | 101.571429 | |
| PD18189a | 3 | 157265584 | 157269017 | 3433 | 24 | 0 | 143.833333 | |
| PD18189a | 3 | 160041468 | 160041825 | 357 | 7 | 0 | 52.8571429 | |
| PD18189a | 6 | 71022689 | 71025198 | 2509 | 30 | 0 | 112.466667 | |
| PD18189a | 8 | 96375836 | 96379594 | 3758 | 19 | 0 | 202.631579 | |
| PD6048a | 11 | 68148872 | 68149973 | 1101 | 8 | 0 | 215.375 | |
| PD6048a | 15 | 25112281 | 25113472 | 1191 | 8 | 0 | 149 | |

(continued)

| sample | chr | start.bp | end.bp | length.bp | number.bps | number.bps.clustered | avgDist.bp | alternative.kataegis |
|---|---|---|---|---|---|---|---|---|
| PD6048a | 17 | 47678460 | 47679838 | 1378 | 6 | 0 | 258 | |
| PD6048a | 17 | 70694167 | 70694507 | 340 | 7 | 0 | 81.8571429 | |
| PD6048a | 3 | 8057806 | 8058659 | 853 | 12 | 0 | 72.8333333 | |
| PD6048a | 6 | 144175959 | 144178614 | 2655 | 7 | 0 | 564.857143 | |
| PD6405a | 1 | 80321952 | 80323492 | 1540 | 7 | 0 | 330 | |
| PD6405a | 1 | 212357740 | 212360641 | 2901 | 9 | 0 | 329.111111 | |
| PD6405a | 10 | 53817908 | 53822270 | 4362 | 11 | 0 | 454.545455 | |
| PD6405a | 12 | 78164617 | 78171219 | 6602 | 18 | 0 | 384.333333 | |
| PD6405a | 14 | 104175339 | 104177501 | 2162 | 10 | 0 | 251.7 | |
| PD6405a | 14 | 104587627 | 104588458 | 831 | 10 | 0 | 146.9 | |
| PD6405a | 14 | 104595470 | 104596251 | 781 | 7 | 0 | 112.571429 | |
| PD6405a | 17 | 34984601 | 34989357 | 4756 | 9 | 0 | 559.666667 | |
| PD6405a | 17 | 35129191 | 35129843 | 652 | 8 | 0 | 107.5 | |
| PD6405a | 17 | 37795041 | 37796676 | 1635 | 6 | 0 | 285.333333 | |
| PD6405a | 17 | 37996452 | 37996565 | 113 | 6 | 0 | 29.8333333 | |
| PD6405a | 17 | 52635725 | 52641177 | 5452 | 10 | 0 | 556.1 | |
| PD6405a | 17 | 55164068 | 55164543 | 475 | 9 | 0 | 130.22222 | |
| PD6405a | 17 | 55555926 | 55556016 | 90 | 6 | 0 | 22 | |
| PD6405a | 20 | 35587262 | 35588434 | 1172 | 6 | 0 | 195.666667 | |
| PD6405a | 6 | 34722525 | 34728086 | 5561 | 10 | 0 | 596.8 | |
| PD6405a | 7 | 76208162 | 76210935 | 2773 | 7 | 0 | 406.714286 | |
| PD7202a | 13 | 34796466 | 34796665 | 199 | 8 | 0 | 29.25 | |
| PD7202a | 13 | 60295006 | 60297544 | 2538 | 6 | 0 | 449.333333 | |
| PD7202a | 13 | 60935095 | 60937920 | 2825 | 21 | 0 | 135.952381 | |
| PD7202a | 14 | 81485258 | 81492027 | 6769 | 13 | 0 | 526.846154 | |
| PD7202a | 15 | 22932197 | 22932933 | 736 | 10 | 0 | 73.8 | |

326

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD7202a | 15 | 30110244 | 30111739 | 1495 | 8 | 0 | 191.875 | |
| PD7202a | 17 | 49993702 | 49995339 | 1637 | 8 | 0 | 260 | |
| PD7202a | 17 | 50118727 | 50128678 | 9951 | 15 | 0 | 962.066667 | |
| PD7202a | 17 | 52783126 | 52784863 | 1737 | 6 | 0 | 315.833333 | |
| PD7202a | 17 | 52854633 | 52854987 | 354 | 6 | 0 | 123.5 | |
| PD7202a | 17 | 56509248 | 56512555 | 3307 | 12 | 0 | 566 | |
| PD7202a | 17 | 56613674 | 56620839 | 7165 | 9 | 0 | 841.777778 | |
| PD7202a | 17 | 58713808 | 58718924 | 5116 | 8 | 0 | 766 | |
| PD7202a | 17 | 67660920 | 67661518 | 598 | 7 | 0 | 112 | |
| PD7202a | 6 | 77968289 | 77968866 | 577 | 9 | 0 | 64.2222222 | |
| PD7203a | 13 | 82243346 | 82245908 | 2562 | 7 | 0 | 392 | |
| PD7203a | 17 | 31420648 | 31422913 | 2265 | 8 | 0 | 385 | |
| PD7203a | 8 | 76740840 | 76742036 | 1196 | 13 | 0 | 101.384615 | |
| PD7203a | 8 | 77042443 | 77044855 | 2412 | 10 | 0 | 258.5 | |
| PD7205a | 1 | 69019737 | 69022526 | 2789 | 10 | 0 | 300.2 | |
| PD7205a | 1 | 69050668 | 69061940 | 11272 | 22 | 0 | 514.545455 | |
| PD7205a | 12 | 51787305 | 51793560 | 6255 | 15 | 0 | 432.866667 | |
| PD7205a | 12 | 89664510 | 89669679 | 5169 | 11 | 0 | 479.818182 | |
| PD7205a | 13 | 110229553 | 110231894 | 2341 | 17 | 0 | 346.294118 | |
| PD7205a | 13 | 113165106 | 113166036 | 930 | 10 | 0 | 100.2 | |
| PD7205a | 14 | 28667258 | 28670236 | 2978 | 12 | 0 | 271.5 | |
| PD7205a | 14 | 29201918 | 29202738 | 820 | 7 | 0 | 127.142857 | |
| PD7205a | 17 | 38479925 | 38481600 | 1675 | 6 | 0 | 307.333333 | |
| PD7205a | 4 | 177436409 | 177439425 | 3016 | 7 | 0 | 444 | |
| PD7304a | 5 | 161884058 | 161886450 | 2392 | 12 | 0 | 202.833333 | |
| PD7305a | 1 | 107026444 | 107031006 | 4562 | 15 | 0 | 351.866667 | |

| sample | chr | start.bp | end.bp | length.bp | number.bps | number.bps.clustered | avgDist.bp | alternative.kataegis |
|--------|-----|----------|--------|-----------|------------|---------------------|------------|----------------------|
| PD7305a | 1 | 107752077 | 107766499 | 14422 | 37 | 0 | 411.189189 | |
| PD7305a | 1 | 111007272 | 111008184 | 912 | 8 | 0 | 127.125 | |
| PD7305a | 1 | 111261813 | 111262047 | 234 | 7 | 0 | 36.2857143 | |
| PD7305a | 1 | 114308741 | 114309732 | 991 | 13 | 0 | 78.6923077 | |
| PD7305a | 11 | 67385140 | 67385821 | 681 | 7 | 0 | 115.428571 | |
| PD7305a | 17 | 45735504 | 45735861 | 357 | 9 | 0 | 55.4444444 | |
| PD7305a | 17 | 45919541 | 45925237 | 5696 | 10 | 0 | 592.6 | |
| PD7305a | 17 | 46661408 | 46662970 | 1562 | 7 | 0 | 247 | |
| PD7305a | 17 | 53888346 | 53893601 | 5255 | 8 | 0 | 670 | |
| PD7305a | 17 | 67452502 | 67461903 | 9401 | 11 | 0 | 856.363636 | |
| PD7305a | 17 | 73574153 | 73575312 | 1159 | 6 | 0 | 208 | |
| PD7305a | 20 | 40430490 | 40431267 | 777 | 8 | 0 | 99.375 | |
| PD7305a | 20 | 53975355 | 53980793 | 5438 | 10 | 0 | 556.9 | |
| PD7305a | 20 | 54528410 | 54533765 | 5355 | 11 | 0 | 498.545455 | |
| PD7305a | 20 | 56436391 | 56440742 | 4351 | 19 | 0 | 230.789474 | |
| PD7305a | 20 | 56614428 | 56615116 | 688 | 8 | 0 | 97.5 | |
| PD7305a | 20 | 57328204 | 57336505 | 8301 | 19 | 0 | 440.789474 | |
| PD7305a | 20 | 60706951 | 60708837 | 1886 | 6 | 0 | 360.666667 | |
| PD7306a | 1 | 94601958 | 94606915 | 4957 | 18 | 0 | 312.444444 | |
| PD7306a | 11 | 44027873 | 44028385 | 512 | 6 | 0 | 98.6666667 | |
| PD7306a | 11 | 69625123 | 69625812 | 689 | 6 | 0 | 361.833333 | |
| PD7306a | 17 | 37752860 | 37755092 | 2232 | 8 | 0 | 338.375 | |
| PD7306a | 17 | 47411477 | 47413130 | 1653 | 12 | 0 | 162.75 | |
| PD7306a | 17 | 50641568 | 50647939 | 6371 | 11 | 0 | 583 | |
| PD7306a | 17 | 54563940 | 54568905 | 4965 | 19 | 0 | 268.947368 | |
| PD7306a | 17 | 70533709 | 70537954 | 4245 | 6 | 0 | 974.833333 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD7306a | 20 | 43541602 | 43542710 | 1108 | 6 | 0 | 185.333333 | |
| PD7306a | 4 | 53892771 | 53893224 | 453 | 7 | 0 | 71.5714286 | |
| PD7307a | 11 | 23611824 | 23613369 | 1545 | 9 | 0 | 171.777778 | |
| PD7307a | 15 | 83345501 | 83347801 | 2300 | 6 | 0 | 411.333333 | |
| PD7307a | 17 | 17620774 | 17622784 | 2010 | 9 | 0 | 236.111111 | |
| PD7307a | 17 | 25960409 | 25961363 | 954 | 12 | 0 | 92.3333333 | |
| PD7307a | 17 | 31527702 | 31529200 | 1498 | 6 | 0 | 394.5 | |
| PD7307a | 17 | 47108883 | 47112856 | 3973 | 9 | 0 | 466.777778 | |
| PD7307a | 6 | 123869805 | 123873493 | 3688 | 9 | 0 | 431.333333 | |
| PD7307a | 6 | 129347424 | 129348235 | 811 | 8 | 0 | 153 | |
| PD8995a | 2 | 212872724 | 212876930 | 4206 | 10 | 0 | 538.8 | |
| PD8995a | 2 | 212884389 | 212893076 | 8687 | 11 | 0 | 836.545455 | |
| PD8995a | 3 | 67094886 | 67100224 | 5338 | 6 | 0 | 966.666667 | |
| PD8996a | 17 | 49262772 | 49263064 | 292 | 6 | 0 | 50.3333333 | |
| PD8996a | 17 | 52977634 | 52979192 | 1558 | 6 | 0 | 262.666667 | |
| PD8997a | 21 | 24106218 | 24106644 | 426 | 8 | 0 | 99 | |
| PD8997a | 8 | 38899181 | 38903724 | 4543 | 7 | 0 | 817.714286 | |
| PD8998a | 15 | 94032626 | 94034161 | 1535 | 6 | 0 | 301 | |
| PD8998a | 16 | 27744933 | 27749395 | 4462 | 8 | 0 | 606.25 | |
| PD8998a | 21 | 32544998 | 32549511 | 4513 | 9 | 0 | 545.111111 | |
| PD9009a | 1 | 14680666 | 14681039 | 373 | 8 | 0 | 47.75 | |
| PD9009a | 1 | 14816342 | 14820555 | 4213 | 41 | 0 | 103.804878 | |
| PD9009a | 1 | 23948647 | 23951229 | 2582 | 16 | 0 | 173.6875 | |
| PD9009a | 1 | 59825736 | 59828209 | 2473 | 8 | 0 | 309.625 | |
| PD9009a | 17 | 50119281 | 50121195 | 1914 | 13 | 0 | 150.307692 | |
| PD9009a | 20 | 14183769 | 14188433 | 4664 | 8 | 0 | 681.375 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|---------------------|
| PD9009a | 4 | 18980970 | 18981210 | 240 | 6 | 0 | 44.3333333 | |
| PD9009a | 5 | 144873691 | 144875691 | 2000 | 15 | 0 | 133.866667 | yes |
| PD9009a | 8 | 102792300 | 102794989 | 2689 | 9 | 0 | 427.666667 | |
| PD4826a | 17 | 21806694 | 21807059 | 365 | 7 | 0 | 55.7142857 | |
| PD4826a | 17 | 41557398 | 41558580 | 1182 | 12 | 0 | 117.083333 | |
| PD4826a | 17 | 69544619 | 69544912 | 293 | 8 | 0 | 44.5 | |
| PD4826a | 20 | 20512126 | 20512464 | 338 | 12 | 0 | 358.5 | |
| PD4826a | 3 | 128328591 | 128329328 | 737 | 7 | 0 | 106.714286 | |
| PD4826a | 3 | 158941042 | 158943643 | 2601 | 18 | 0 | 148 | yes |
| PD7249a | 11 | 104555426 | 104555594 | 168 | 6 | 0 | 28.5 | |
| PD7249a | 2 | 195002001 | 195008020 | 6019 | 12 | 0 | 562.833333 | |
| PD7249a | 5 | 121637378 | 121650119 | 12741 | 16 | 0 | 895.6875 | |
| PD7249a | 8 | 87663709 | 87664993 | 1284 | 7 | 0 | 187.285714 | |
| PD4962a | 11 | 16993478 | 16999332 | 5854 | 6 | 0 | 995.5 | |
| .PD4962a | 2 | 178233749 | 178236120 | 2371 | 10 | 0 | 353.8 | |
| PD4841a | 8 | 139393476 | 139398931 | 5455 | 8 | 0 | 695.125 | |
| PD11751a | 1 | 217631360 | 217631855 | 495 | 6 | 0 | 138.333333 | |
| PD11751a | 8 | 132652684 | 132658097 | 5413 | 32 | 0 | 171.75 | |
| PD4264a | 6 | 16411059 | 16412527 | 1468 | 6 | 0 | 249 | |
| PD4978a | 1 | 177313932 | 177314341 | 409 | 6 | 0 | 83.3333333 | |
| PD4978a | 11 | 70149303 | 70152974 | 3671 | 18 | 0 | 209.888889 | |
| PD4978a | 11 | 81967257 | 81971061 | 3804 | 12 | 0 | 366.083333 | |
| PD4978a | 14 | 37730960 | 37731940 | 980 | 11 | 0 | 89.2727273 | |
| PD4978a | 15 | 22309772 | 22311424 | 1652 | 12 | 0 | 196.916667 | |
| PD4978a | 15 | 34761565 | 34772766 | 11201 | 15 | 0 | 948 | |
| PD4978a | 15 | 54358364 | 54360826 | 2462 | 10 | 0 | 250.7 | |

(continued)

| sample | chr | start.bp | end.bp | length.bp | number.bps | number.bps.clustered | avgDist.bp | alternative.kataegis |
|---|---|---|---|---|---|---|---|---|
| PD4978a | 15 | 56767150 | 56769214 | 2064 | 6 | 0 | 525.5 | |
| PD4978a | 17 | 59549592 | 59549900 | 308 | 6 | 0 | 66.5 | |
| PD4978a | 6 | 62550817 | 62555392 | 4575 | 18 | 0 | 263.666667 | |
| PD4978a | 6 | 62571071 | 62576994 | 5923 | 12 | 0 | 517.5 | |
| PD4978a | 8 | 110796201 | 110797062 | 861 | 8 | 0 | 123.125 | |
| 3PD4978a | 8 | 111254683 | 111261445 | 6762 | 20 | 0 | 366.45 | |
| PD4978a | 8 | 111488395 | 111492437 | 4042 | 14 | 0 | 329 | |
| PD4978a | 8 | 129409394 | 129417668 | 8274 | 24 | 0 | 348.041667 | |
| PD4978a | 8 | 129583179 | 129584740 | 1561 | 6 | 0 | 273 | |
| PD4978a | X | 13881416 | 13888089 | 6673 | 10 | 0 | 682.1 | |
| PD4192a | 1 | 40804072 | 40804420 | 348 | 7 | 0 | 52.5714286 | |
| PD4192a | 17 | 37497963 | 37504290 | 6327 | 16 | 0 | 414.125 | |
| PD4192a | 8 | 39132136 | 39133025 | 889 | 10 | 0 | 117 | |
| PD4198a | 10 | 117593343 | 117594233 | 890 | 7 | 0 | 154.428571 | |
| PD4198a | 16 | 23596050 | 23597189 | 1139 | 6 | 0 | 217.5 | |
| PD4198a | 17 | 21217367 | 21219887 | 2520 | 9 | 0 | 327.111111 | |
| PD4198a | 18 | 28269178 | 28270127 | 949 | 12 | 0 | 214.583333 | |
| PD4198a | 5 | 55991666 | 55996280 | 4614 | 11 | 0 | 439.636364 | |
| PD4199a | 1 | 52686688 | 52687615 | 927 | 6 | 0 | 168 | |
| PD4199a | 1 | 58209707 | 58216325 | 6618 | 18 | 0 | 369.666667 | |
| PD4199a | 1 | 58812060 | 58812393 | 333 | 6 | 0 | 69.8333333 | |
| PD4199a | 11 | 70771423 | 70785748 | 14325 | 16 | 0 | 941.875 | |
| PD4199a | 12 | 65423354 | 65429139 | 5785 | 9 | 0 | 819 | |
| PD4199a | 12 | 113224526 | 113228606 | 4080 | 7 | 0 | 688.714286 | |
| PD4199a | 12 | 115316317 | 115318646 | 2329 | 7 | 0 | 453.285714 | |
| PD4199a | 13 | 52101969 | 52102671 | 702 | 6 | 0 | 166.5 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD4199a | 15 | 61376416 | 61382676 | 6260 | 13 | 0 | 644.692308 | |
| PD4199a | 17 | 37950062 | 37956171 | 6109 | 10 | 0 | 631.3 | |
| PD4199a | 19 | 42850684 | 42863524 | 12840 | 26 | 0 | 536.461538 | |
| PD4199a | 19 | 47527944 | 47529863 | 1919 | 9 | 0 | 248.888889 | |
| PD4199a | 2 | 145022271 | 145026906 | 4635 | 6 | 0 | 914 | |
| PD4199a | 2 | 145046961 | 145056596 | 9635 | 11 | 0 | 947.545455 | |
| PD4199a | 2 | 203678364 | 203682010 | 3646 | 9 | 0 | 434.333333 | |
| PD4199a | 3 | 72919435 | 72923713 | 4278 | 11 | 0 | 389 | |
| PD4199a | 5 | 67020108 | 67022469 | 2361 | 24 | 0 | 99.5 | |
| PD4199a | 6 | 109633393 | 109647143 | 13750 | 15 | 0 | 971.266667 | |
| PD4199a | 8 | 108218788 | 108219861 | 1073 | 13 | 0 | 85.0769231 | |
| PD4199a | 8 | 128408328 | 128409078 | 750 | 6 | 0 | 135.5 | |
| PD4199a | 8 | 128424655 | 128425012 | 357 | 7 | 0 | 78.1428571 | |
| PD3904a | 11 | 20543226 | 20545796 | 2570 | 14 | 0 | 194.785714 | |
| PD3904a | 5 | 173624991 | 173626298 | 1307 | 12 | 0 | 115.5 | |
| PD3904a | 8 | 37612092 | 37614750 | 2658 | 7 | 0 | 500 | |
| PD3945a | 2 | 34262723 | 34270846 | 8123 | 28 | 0 | 293.607143 | |
| PD4005a | 10 | 37886268 | 37888672 | 2404 | 17 | 0 | 188.823529 | |
| PD4005a | 10 | 38201375 | 38202885 | 1510 | 23 | 0 | 65.7826087 | |
| PD4005a | 10 | 121312223 | 121315937 | 3714 | 6 | 0 | 758.833333 | |
| PD4006a | 4 | 56247520 | 56249256 | 1736 | 6 | 0 | 391.833333 | |
| PD4088a | 17 | 35928719 | 35931600 | 2881 | 6 | 0 | 523.5 | |
| PD4088a | 17 | 49990940 | 49991400 | 460 | 6 | 0 | 463.666667 | yes |
| PD4088a | 17 | 74077973 | 74082287 | 4314 | 9 | 0 | 494.666667 | |
| PD4088a | 18 | 44833362 | 44837104 | 3742 | 7 | 0 | 539.285714 | |
| PD4088a | 3 | 167385435 | 167388852 | 3417 | 11 | 0 | 374.454545 | |

(continued)

| sample | chr | start.bp | end.bp | length.bp | number.bps | number.bps.clustered | avgDist.bp | alternative.kataegis |
|---|---|---|---|---|---|---|---|---|
| PD4103a | 10 | 24506101 | 24507243 | 1142 | 17 | 0 | 69.8823529 | |
| PD4103a | 10 | 24724139 | 24726563 | 2424 | 10 | 0 | 244.3 | |
| PD4103a | 10 | 57287594 | 57289397 | 1803 | 7 | 0 | 278.142857 | |
| PD4103a | 10 | 57324574 | 57329065 | 4491 | 28 | 0 | 160.607143 | |
| PD4103a | 11 | 66515178 | 66517155 | 1977 | 10 | 0 | 273.3 | |
| PD4103a | 12 | 66727147 | 66731227 | 4080 | 6 | 0 | 745.833333 | |
| PD4103a | 12 | 66847929 | 66856770 | 8841 | 15 | 0 | 638.266667 | |
| PD4103a | 12 | 67633093 | 67634276 | 1183 | 6 | 0 | 209.166667 | |
| PD4103a | 12 | 68454072 | 68455076 | 1004 | 8 | 0 | 128.625 | |
| PD4103a | 12 | 70986204 | 70987207 | 1003 | 8 | 0 | 225.625 | |
| PD4103a | 12 | 71421650 | 71422455 | 805 | 8 | 0 | 469.5 | |
| PD4103a | 12 | 71722858 | 71723706 | 848 | 7 | 0 | 140 | |
| PD4103a | 12 | 71850868 | 71852522 | 1654 | 11 | 0 | 180.545455 | |
| PD4103a | 12 | 71862785 | 71867824 | 5039 | 9 | 0 | 560.666667 | |
| PD4103a | 12 | 73437605 | 73441423 | 3818 | 12 | 0 | 320.333333 | |
| PD4103a | 12 | 73453918 | 73455816 | 1898 | 8 | 0 | 240.25 | |
| PD4103a | 12 | 73480719 | 73482074 | 1355 | 9 | 0 | 177.111111 | |
| PD4103a | 13 | 49412784 | 49414195 | 1411 | 10 | 0 | 146.8 | |
| PD4103a | 17 | 7493744 | 7497152 | 3408 | 17 | 0 | 244 | |
| PD4103a | 20 | 31473686 | 31477501 | 3815 | 19 | 0 | 241.736842 | |
| PD4103a | 3 | 73651973 | 73652224 | 251 | 9 | 0 | 28.5555556 | |
| PD4103a | 3 | 73664736 | 73666636 | 1900 | 6 | 0 | 335.833333 | |
| PD4103a | 3 | 74093225 | 74095404 | 2179 | 12 | 0 | 195.083333 | |
| PD4103a | 4 | 56434977 | 56436213 | 1236 | 6 | 0 | 233.5 | |
| PD4103a | 4 | 74625253 | 74625956 | 703 | 14 | 0 | 54.5 | |
| PD4103a | 4 | 75558729 | 75560994 | 2265 | 7 | 0 | 326.714286 | |

(continued)

| sample | chr | start.bp | end.bp | length.bp | number.bps | number.bps.clustered | avgDist.bp | alternative.kataegis |
|---|---|---|---|---|---|---|---|---|
| PD4103a | 4 | 76987866 | 76989496 | 1630 | 8 | 0 | 228.5 | |
| PD4103a | 4 | 88912682 | 88924232 | 11550 | 14 | 0 | 827.071429 | |
| PD4103a | 4 | 126024367 | 126026054 | 1687 | 11 | 0 | 165.818182 | |
| PD4103a | 4 | 126199379 | 126199723 | 344 | 7 | 0 | 223.142857 | |
| PD4103a | 4 | 164421998 | 164424529 | 2531 | 12 | 0 | 227.416667 | |
| PD4103a | 6 | 158152504 | 158157605 | 5101 | 27 | 0 | 207.814815 | |
| PD4103a | 8 | 91702658 | 91704432 | 1774 | 8 | 0 | 226.25 | |
| PD4103a | 8 | 91903511 | 91904653 | 1142 | 6 | 0 | 320.166667 | |
| PD4103a | X | 32346273 | 32347889 | 1616 | 10 | 0 | 256.3 | |
| PD4116a | 11 | 78824898 | 78825767 | 869 | 6 | 0 | 203.833333 | |
| PD4107a | 12 | 10505415 | 10508972 | 3557 | 12 | 0 | 312 | |
| PD4107a | 6 | 126233460 | 126239477 | 6017 | 80 | 0 | 77.7125 | |
| PD4107a | 6 | 126274977 | 126279808 | 4831 | 46 | 0 | 105.891304 | |
| PD4107a | 6 | 126373413 | 126375422 | 2009 | 8 | 0 | 347.375 | |
| PD4107a | 6 | 126392343 | 126394154 | 1811 | 14 | 0 | 131.071429 | |
| PD4107a | 6 | 126431071 | 126437393 | 6322 | 150 | 0 | 43.5866667 | |
| PD4107a | 6 | 130419476 | 130423199 | 3723 | 53 | 0 | 72.8679245 | |
| PD4107a | 6 | 130433898 | 130438324 | 4426 | 46 | 0 | 98 | |
| PD4107a | 6 | 130483574 | 130489124 | 5550 | 36 | 0 | 167.027778 | |
| PD4107a | 6 | 131811055 | 131818990 | 7935 | 24 | 0 | 331.541667 | |
| PD4107a | 6 | 132397037 | 132401366 | 4329 | 10 | 0 | 455.5 | |
| PD4107a | 6 | 132599539 | 132603467 | 3928 | 43 | 0 | 92.9534884 | |
| PD4107a | 6 | 133710541 | 133716520 | 5979 | 15 | 0 | 401 | |
| PD4107a | 6 | 134015725 | 134024884 | 9159 | 13 | 0 | 759.153846 | |
| PD4107a | 6 | 134117245 | 134118849 | 1604 | 6 | 0 | 368.166667 | |
| PD4107a | 6 | 135258087 | 135262041 | 3954 | 6 | 0 | 677.166667 | |

(continued)

| sample | chr | start.bp | end.bp | length.bp | number.bps | number.bps.clustered | avgDist.bp | alternative.kataegis |
|---|---|---|---|---|---|---|---|---|
| PD4107a | 6 | 138011038 | 138017781 | 6743 | 9 | 0 | 755.888889 | |
| PD4109a | 11 | 4233884 | 4234819 | 935 | 9 | 0 | 134.666667 | |
| PD4109a | 14 | 103259043 | 103265053 | 6010 | 11 | 0 | 554.909091 | |
| PD4109a | 19 | 15712217 | 15715746 | 3529 | 6 | 0 | 660 | |
| PD4109a | 3 | 188845719 | 188851678 | 5959 | 19 | 0 | 401.315789 | |
| PD4109a | 8 | 17689007 | 17689461 | 454 | 9 | 0 | 53.7777778 | |
| PD4248a | 1 | 185320365 | 185322998 | 2633 | 10 | 0 | 364.4 | |
| PD4248a | 10 | 123200692 | 123201268 | 576 | 6 | 0 | 321.5 | |
| PD4248a | 10 | 130763945 | 130767205 | 3260 | 12 | 0 | 283.833333 | |
| PD22357a | 17 | 35905947 | 35906827 | 880 | 6 | 0 | 268.5 | |
| PD22357a | 17 | 47482809 | 47488670 | 5861 | 13 | 0 | 452.384615 | |
| PD22357a | 17 | 47751173 | 47754422 | 3249 | 6 | 0 | 620 | |
| PD22357a | 3 | 86620244 | 86620800 | 556 | 7 | 0 | 113.571429 | |
| PD22358a | 11 | 30201686 | 30202247 | 561 | 7 | 0 | 107.285714 | |
| PD22358a | 11 | 38952534 | 38953254 | 720 | 7 | 0 | 141.714286 | |
| PD22358a | 11 | 46834936 | 46837533 | 2597 | 11 | 0 | 237.272727 | |
| PD22360a | 6 | 51703268 | 51708818 | 5550 | 8 | 0 | 895.75 | |
| PD22362a | 1 | 109796276 | 109804741 | 8465 | 15 | 0 | 598 | |
| PD22362a | 1 | 118627249 | 118627829 | 580 | 6 | 0 | 105.5 | |
| PD22362a | 11 | 20467417 | 20468147 | 730 | 9 | 0 | 133.444444 | |
| PD22362a | 12 | 65580595 | 65583222 | 2627 | 7 | 0 | 381.428571 | |
| PD22362a | 13 | 53017306 | 53017509 | 203 | 7 | 0 | 435.857143 | |
| PD22362a | 17 | 45184344 | 45184647 | 303 | 6 | 0 | 262.5 | |
| PD22362a | 17 | 57822584 | 57823605 | 1021 | 7 | 0 | 187.428571 | |
| PD22362a | 17 | 63538190 | 63538763 | 573 | 6 | 0 | 96.3333333 | |
| PD22362a | 17 | 65208541 | 65209190 | 649 | 6 | 0 | 153.166667 | |

(continued)

| sample | chr | start.bp | end.bp | length.bp | number.bps | number.bps.clustered | avgDist.bp | alternative.kataegis |
|---|---|---|---|---|---|---|---|---|
| PD22362a | 5 | 44966620 | 44968072 | 1452 | 13 | 0 | 168.230769 | |
| PD22362a | 8 | 36764954 | 36765862 | 908 | 6 | 0 | 152.5 | |
| PD22362a | 8 | 94389589 | 94404522 | 14933 | 22 | 0 | 679.136364 | |
| PD22366a | 11 | 59593416 | 59598374 | 4958 | 6 | 0 | 851 | |
| PD22366a | 16 | 48395300 | 48395826 | 526 | 8 | 0 | 107.25 | |
| PD22366a | 2 | 102876959 | 102877234 | 275 | 6 | 0 | 49.1666667 | |
| PD22366a | 9 | 90227224 | 90230421 | 3197 | 7 | 0 | 629.142857 | |
| PD23550a | 1 | 163384415 | 163389190 | 4775 | 30 | 0 | 163.1 | |
| PD23550a | 17 | 50346694 | 50347257 | 563 | 7 | 0 | 137.428571 | |
| PD23550a | 17 | 52669132 | 52672299 | 3167 | 6 | 0 | 530.666667 | |
| PD23550a | 17 | 54856600 | 54857475 | 875 | 6 | 0 | 157.5 | |
| PD23550a | 17 | 57280892 | 57282726 | 1834 | 7 | 0 | 606.142857 | |
| PD23550a | 17 | 59080903 | 59082729 | 1826 | 9 | 0 | 203.777778 | |
| PD23550a | 17 | 60004444 | 60006029 | 1585 | 8 | 0 | 861.875 | |
| PD23550a | 17 | 61076518 | 61077022 | 504 | 6 | 0 | 118.333333 | |
| PD23550a | 4 | 61999310 | 62000172 | 862 | 7 | 0 | 134.857143 | |
| PD23550a | 4 | 69647471 | 69650365 | 2894 | 6 | 0 | 489.833333 | |
| PD23550a | 4 | 71849795 | 71850315 | 520 | 9 | 0 | 84.3333333 | |
| PD23550a | 4 | 72845093 | 72845906 | 813 | 6 | 0 | 189 | |
| PD23550a | 4 | 76087312 | 76090666 | 3354 | 8 | 0 | 424 | |
| PD23550a | 4 | 77345797 | 77347135 | 1338 | 6 | 0 | 229 | |
| PD23550a | 8 | 53332809 | 53334361 | 1552 | 14 | 0 | 111.071429 | |
| PD23550a | 8 | 53680449 | 53680976 | 527 | 11 | 0 | 48.5454545 | |
| PD23558a | 5 | 79673908 | 79678589 | 4681 | 8 | 0 | 762.875 | |
| PD23559a | 11 | 74456858 | 74457121 | 263 | 6 | 0 | 46.3333333 | |
| PD23559a | 21 | 44252865 | 44255399 | 2534 | 6 | 0 | 484.833333 | |

(continued)

| sample | chr | start.bp | end.bp | length.bp | number.bps | number.bps.clustered | avgDist.bp | alternative.kataegis |
|---|---|---|---|---|---|---|---|---|
| PD23559a | 3 | 185135660 | 185136548 | 888 | 8 | 0 | 113.5 | |
| PD23559a | 4 | 88813183 | 88814509 | 1326 | 15 | 0 | 92.8666667 | |
| PD23559a | 9 | 33624449 | 33625225 | 776 | 13 | 0 | 66.4615385 | |
| PD23559a | X | 39646861 | 39647241 | 380 | 9 | 0 | 43 | |
| PD23560a | 1 | 212713143 | 212713684 | 541 | 7 | 0 | 232.428571 | |
| PD23560a | 10 | 33445082 | 33450845 | 5763 | 14 | 0 | 421.142857 | |
| PD23560a | 20 | 59162317 | 59165523 | 3206 | 23 | 0 | 152.130435 | |
| PD23560a | 7 | 137050469 | 137051757 | 1288 | 10 | 0 | 131.2 | |
| PD23561a | 14 | 92092648 | 92095032 | 2384 | 6 | 0 | 401.833333 | |
| PD23561a | 15 | 42000493 | 42008018 | 7525 | 15 | 0 | 573.733333 | |
| PD23561a | 15 | 65292306 | 65294976 | 2670 | 7 | 0 | 387.857143 | |
| PD23561a | 15 | 76390693 | 76394978 | 4285 | 10 | 0 | 616.6 | |
| PD23561a | 17 | 29323682 | 29324020 | 338 | 8 | 0 | 52.375 | |
| PD23561a | 19 | 39290823 | 39292826 | 2003 | 6 | 0 | 375.333333 | |
| PD23561a | 2 | 185166242 | 185167720 | 1478 | 9 | 0 | 194.777778 | |
| PD23561a | 2 | 228684154 | 228685585 | 1431 | 7 | 0 | 209 | |
| PD23561a | 21 | 21328164 | 21330387 | 2223 | 7 | 0 | 405.142857 | |
| PD23561a | 22 | 41852921 | 41856953 | 4032 | 7 | 0 | 614.285714 | |
| PD23561a | 22 | 46171582 | 46173250 | 1668 | 6 | 0 | 309.833333 | |
| PD23561a | 3 | 47674037 | 47675743 | 1706 | 7 | 0 | 363 | |
| PD23561a | 3 | 52626846 | 52629155 | 2309 | 8 | 0 | 334.625 | |
| PD23561a | 3 | 123782901 | 123786682 | 3781 | 9 | 0 | 465.444444 | |
| PD23561a | 4 | 2899829 | 2902467 | 2638 | 8 | 0 | 355.625 | |
| PD23561a | 5 | 59443374 | 59448894 | 5520 | 14 | 0 | 439.071429 | |
| PD23565a | 8 | 33660699 | 33661862 | 1163 | 6 | 0 | 214.333333 | |
| PD23570a | 12 | 51820576 | 51840658 | 20082 | 37 | 0 | 545.945946 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD23574a | 12 | 43456578 | 43458462 | 1884 | 7 | 0 | 305.142857 | |
| PD24182a | 12 | 18356634 | 18357223 | 589 | 6 | 0 | 136 | |
| PD24182a | 12 | 98340542 | 98341646 | 1104 | 12 | 0 | 107.083333 | |
| PD24182a | 6 | 120133346 | 120137101 | 3755 | 9 | 0 | 443.666667 | |
| PD24190a | 1 | 67081758 | 67082420 | 662 | 10 | 0 | 83.3 | |
| PD24191a | X | 51566574 | 51567439 | 865 | 6 | 0 | 190.166667 | |
| PD24192a | 12 | 40704173 | 40710117 | 5944 | 28 | 0 | 212.321429 | |
| PD24192a | 19 | 9054566 | 9055845 | 1279 | 7 | 0 | 206.142857 | |
| PD24192a | 4 | 20025799 | 20033326 | 7527 | 10 | 0 | 781.5 | |
| PD24192a | 4 | 158886973 | 158891653 | 4680 | 8 | 0 | 657.875 | |
| PD24194a | 1 | 159528091 | 159534290 | 6199 | 15 | 0 | 473.4 | |
| PD24194a | 10 | 37591730 | 37596168 | 4438 | 10 | 0 | 446.4 | |
| PD24194a | 11 | 18191497 | 18192997 | 1500 | 6 | 0 | 270 | |
| PD24194a | 15 | 41182389 | 41185679 | 3290 | 6 | 0 | 666.666667 | |
| PD24194a | 17 | 36298437 | 36307961 | 9524 | 11 | 0 | 876.727273 | |
| PD24194a | 17 | 48520735 | 48521405 | 670 | 7 | 0 | 98.4285714 | |
| PD24194a | 19 | 48366757 | 48369310 | 2553 | 6 | 0 | 565.833333 | |
| PD24194a | 2 | 8460735 | 8464026 | 3291 | 6 | 0 | 637.666667 | |
| PD24194a | 20 | 365019 | 365721 | 702 | 7 | 0 | 142.428571 | |
| PD24194a | 22 | 42422893 | 42423505 | 612 | 6 | 0 | 415 | |
| PD24194a | 3 | 193566684 | 193568394 | 1710 | 6 | 0 | 291.833333 | |
| PD24194a | 5 | 10903252 | 10907307 | 4055 | 10 | 0 | 406.1 | |
| PD24194a | 9 | 99239768 | 99241860 | 2092 | 6 | 0 | 505.5 | |
| PD24196a | 12 | 43821803 | 43822980 | 1177 | 14 | 0 | 111.142857 | |
| PD24196a | 13 | 107772680 | 107772939 | 259 | 7 | 0 | 43.5714286 | |
| PD24196a | 13 | 110232250 | 110232619 | 369 | 6 | 0 | 72.3333333 | |

(continued)

| sample | chr | start.bp | end.bp | length.bp | number.bps | number.bps.clustered | avgDist.bp | alternative.kataegis |
|---|---|---|---|---|---|---|---|---|
| PD24196a | 21 | 32117077 | 32127283 | 10206 | 21 | 0 | 489.380952 | |
| PD24196a | 21 | 34621726 | 34625601 | 3875 | 14 | 0 | 315.785714 | |
| PD24199a | 10 | 123589449 | 123591664 | 2215 | 22 | 0 | 104.772727 | |
| PD24199a | 11 | 72230100 | 72232525 | 2425 | 15 | 0 | 207.333333 | |
| PD24199a | 11 | 78814843 | 78816804 | 1961 | 8 | 0 | 369.375 | |
| PD24199a | 11 | 111459112 | 111462945 | 3833 | 6 | 0 | 650.666667 | |
| PD24199a | 14 | 29394525 | 29396273 | 1748 | 7 | 0 | 329.714286 | |
| PD24199a | 17 | 48261555 | 48263822 | 2267 | 8 | 0 | 283.625 | |
| PD24199a | 17 | 62496317 | 62497751 | 1434 | 6 | 0 | 295.333333 | |
| PD24199a | 6 | 72234294 | 72234600 | 306 | 6 | 0 | 61.3333333 | |
| PD24199a | 7 | 94057047 | 94057187 | 140 | 7 | 0 | 20.4285714 | |
| PD24199a | 8 | 127087295 | 127095561 | 8266 | 17 | 0 | 749.882353 | |
| PD24201a | 10 | 57220571 | 57220969 | 398 | 8 | 0 | 53.625 | |
| PD24201a | 19 | 42614788 | 42616915 | 2127 | 12 | 0 | 197.666667 | |
| PD24201a | 19 | 42672093 | 42672717 | 624 | 9 | 0 | 74 | |
| PD24201a | 7 | 62457378 | 62459636 | 2258 | 8 | 0 | 300 | |
| PD24206a | 1 | 150249704 | 150252208 | 2504 | 8 | 0 | 537.125 | |
| PD24206a | 16 | 48138439 | 48141161 | 2722 | 9 | 0 | 317.777778 | |
| PD24206a | 21 | 35700722 | 35700959 | 237 | 9 | 0 | 29.5555556 | |
| PD24206a | 8 | 51153911 | 51154922 | 1011 | 9 | 0 | 122.555556 | |
| PD24206a | 8 | 55022427 | 55024543 | 2116 | 6 | 0 | 352.833333 | |
| PD24207a | 18 | 20723062 | 20727804 | 4742 | 7 | 0 | 678.571429 | |
| PD24207a | 4 | 123819848 | 123822133 | 2285 | 11 | 0 | 269.181818 | |
| PD24208a | 16 | 15256107 | 15258988 | 2881 | 9 | 0 | 378.111111 | |
| PD24208a | 5 | 106953919 | 106962833 | 8914 | 14 | 0 | 679.428571 | |
| PD24208a | 5 | 178868265 | 178871649 | 3384 | 6 | 0 | 603 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD24208a | 7 | 141058772 | 141059618 | 846 | 9 | 0 | 108.555556 | |
| PD24208a | X | 92572401 | 92573159 | 758 | 6 | 0 | 302 | |
| PD24209a | 1 | 33798074 | 33799729 | 1655 | 12 | 0 | 140.833333 | |
| PD24209a | 1 | 33808412 | 33811799 | 3387 | 20 | 0 | 186.7 | |
| PD24209a | 1 | 235052399 | 235055945 | 3546 | 6 | 0 | 662.833333 | |
| PD24209a | 17 | 17908445 | 17909855 | 1410 | 6 | 0 | 242 | |
| PD24209a | 17 | 43800825 | 43804992 | 4167 | 12 | 0 | 407.083333 | |
| PD24209a | 3 | 179267749 | 179272519 | 4770 | 8 | 0 | 639.375 | |
| PD24212a | 6 | 17840357 | 17844076 | 3719 | 7 | 0 | 601.142857 | |
| PD24212a | 6 | 23639654 | 23642182 | 2528 | 18 | 0 | 143.333333 | |
| PD24212a | 6 | 23781612 | 23789502 | 7890 | 23 | 0 | 348.304348 | |
| PD24214a | 17 | 64237691 | 64238416 | 725 | 7 | 0 | 215.571429 | |
| PD24216a | 6 | 152937909 | 152941642 | 3733 | 10 | 0 | 375.4 | |
| PD24217a | 10 | 24632886 | 24636773 | 3887 | 6 | 0 | 705.666667 | |
| PD24217a | 10 | 32739554 | 32742688 | 3134 | 6 | 0 | 523.5 | |
| PD24217a | 14 | 30992066 | 30996322 | 4256 | 18 | 0 | 281.722222 | |
| PD24217a | 17 | 53006019 | 53008727 | 2708 | 10 | 0 | 375.9 | |
| PD24217a | 17 | 69205630 | 69206605 | 975 | 7 | 0 | 148.571429 | |
| PD24217a | 20 | 8477328 | 8484024 | 6696 | 10 | 0 | 741 | |
| PD24217a | 20 | 8501394 | 8503970 | 2576 | 7 | 0 | 433.428571 | |
| PD24217a | 3 | 100358852 | 100360715 | 1863 | 13 | 0 | 145.923077 | |
| PD24217a | 7 | 15965676 | 15966006 | 330 | 7 | 0 | 51.5714286 | |
| PD24217a | 7 | 17092566 | 17096466 | 3900 | 13 | 0 | 302.615385 | |
| PD24217a | 7 | 24749782 | 24750614 | 832 | 6 | 0 | 170.166667 | |
| PD24217a | 7 | 28941391 | 28944975 | 3584 | 8 | 0 | 459.125 | |
| PD24218a | 14 | 42647690 | 42652146 | 4456 | 11 | 0 | 412.545455 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD24219a | 11 | 58805528 | 58806011 | 483 | 7 | 0 | 90.4285714 | |
| PD24220a | 16 | 50934145 | 50936189 | 2044 | 8 | 0 | 269.625 | |
| PD24220a | 17 | 25540272 | 25542713 | 2441 | 7 | 0 | 350.857143 | |
| PD24220a | 17 | 29309060 | 29310034 | 974 | 11 | 0 | 100.818182 | |
| PD24220a | 17 | 52683668 | 52687816 | 4148 | 17 | 0 | 245.529412 | |
| PD24220a | 17 | 71483725 | 71484197 | 472 | 8 | 0 | 159.5 | |
| PD24220a | 18 | 13340948 | 13346666 | 5718 | 8 | 0 | 727.625 | |
| PD24220a | 5 | 137930266 | 137933191 | 2925 | 9 | 0 | 411 | |
| PD24220a | 8 | 115398176 | 115400711 | 2535 | 7 | 0 | 368 | |
| PD24220a | 8 | 116456661 | 116457067 | 406 | 6 | 0 | 295.833333 | |
| PD24220a | 9 | 1061794 | 1063750 | 1956 | 8 | 0 | 501.375 | |
| PD24223a | 11 | 74456858 | 74457121 | 263 | 6 | 0 | 46.3333333 | |
| PD24223a | 3 | 185135640 | 185136265 | 625 | 6 | 0 | 130.5 | yes |
| PD24224a | 2 | 143483073 | 143483467 | 394 | 6 | 0 | 70.6666667 | |
| PD24224a | 3 | 174166237 | 174167597 | 1360 | 9 | 0 | 163.222222 | |
| PD24224a | 3 | 175091679 | 175096843 | 5164 | 17 | 0 | 304.941176 | |
| PD24224a | 7 | 76555718 | 76556685 | 967 | 7 | 0 | 142 | |
| PD24225a | 17 | 37906457 | 37906584 | 127 | 6 | 0 | 22.1666667 | |
| PD24225a | 17 | 56745049 | 56751140 | 6091 | 23 | 0 | 277.086957 | |
| PD24225a | 17 | 59708663 | 59709828 | 1165 | 14 | 0 | 90.5714286 | |
| PD24304a | 2 | 63007610 | 63010479 | 2869 | 9 | 0 | 331.333333 | |
| PD24308a | 11 | 41242921 | 41246480 | 3559 | 8 | 0 | 449.875 | |
| PD24308a | 2 | 79763858 | 79764760 | 902 | 12 | 0 | 105.333333 | |
| PD24308a | 8 | 107359943 | 107361382 | 1439 | 14 | 0 | 110.428571 | |
| PD24322a | 17 | 35382130 | 35384731 | 2601 | 10 | 0 | 295.3 | |
| PD24322a | 17 | 38573789 | 38574718 | 929 | 10 | 0 | 94.6 | |

(continued)

| sample | chr | start.bp | end.bp | length.bp | number.bps | number.bps.clustered | avgDist.bp | alternative.kataegis |
|---|---|---|---|---|---|---|---|---|
| PD24322a | 3 | 135005352 | 135006584 | 1232 | 11 | 0 | 121 | |
| PD24322a | 4 | 181555209 | 181557340 | 2131 | 16 | 0 | 143.5 | |
| PD24325a | 10 | 8270897 | 8280412 | 9515 | 15 | 0 | 694.466667 | |
| PD24325a | 10 | 14066753 | 14068534 | 1781 | 20 | 0 | 93.6 | |
| PD24325a | 13 | 60532158 | 60538338 | 6180 | 38 | 0 | 162.736842 | |
| PD24325a | 16 | 6872234 | 6872820 | 586 | 9 | 0 | 78.4444444 | |
| PD24325a | 4 | 95961331 | 95962631 | 1300 | 17 | 0 | 77.8235294 | |
| PD24326a | 1 | 14780657 | 14785376 | 4719 | 17 | 0 | 327.117647 | |
| PD24326a | 10 | 8890828 | 8892472 | 1644 | 6 | 0 | 328.833333 | |
| PD24326a | 10 | 35157349 | 35161451 | 4102 | 6 | 0 | 695.666667 | |
| PD24326a | 12 | 23574508 | 23577923 | 3415 | 6 | 0 | 707 | |
| PD24326a | 12 | 44590598 | 44594486 | 3888 | 7 | 0 | 884.142857 | |
| PD24326a | 12 | 55724531 | 55726812 | 2281 | 9 | 0 | 306 | |
| PD24326a | 12 | 122273707 | 122276799 | 3092 | 9 | 0 | 441 | |
| PD24326a | 13 | 75770681 | 75774948 | 4267 | 14 | 0 | 452.5 | |
| PD24326a | 15 | 40207013 | 40210147 | 3134 | 6 | 0 | 534.333333 | |
| PD24326a | 15 | 41452178 | 41455135 | 2957 | 21 | 0 | 144.904762 | |
| PD24326a | 16 | 2430765 | 2437337 | 6572 | 9 | 0 | 939.111111 | |
| PD24326a | 16 | 50412860 | 50414530 | 1670 | 6 | 0 | 278.5 | |
| PD24326a | 16 | 68459957 | 68461230 | 1273 | 6 | 0 | 251.166667 | |
| PD24326a | 17 | 14930788 | 14935286 | 4498 | 10 | 0 | 472.2 | |
| PD24326a | 19 | 5209291 | 5214213 | 4922 | 6 | 0 | 925.333333 | |
| PD24326a | 19 | 38001505 | 38006852 | 5347 | 10 | 0 | 730 | |
| PD24326a | 2 | 43224230 | 43225220 | 990 | 8 | 0 | 128.375 | |
| PD24326a | 2 | 112831505 | 112833647 | 2142 | 7 | 0 | 347.857143 | |
| PD24326a | 2 | 152255509 | 152260541 | 5032 | 8 | 0 | 635 | |

(continued)

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD24326a | 2 | 231136728 | 231142079 | 5351 | 9 | 0 | 607.444444 | |
| PD24326a | 21 | 37583054 | 37584437 | 1383 | 7 | 0 | 323.714286 | |
| PD24326a | 22 | 35357808 | 35362662 | 4854 | 8 | 0 | 671.75 | |
| PD24326a | 22 | 35863448 | 35866673 | 3225 | 6 | 0 | 585.666667 | |
| PD24326a | 3 | 42954826 | 42957925 | 3099 | 9 | 0 | 371.111111 | |
| PD24326a | 5 | 127257543 | 127261338 | 3795 | 8 | 0 | 489.625 | |
| PD24326a | 5 | 138421058 | 138427968 | 6910 | 22 | 0 | 324.818182 | |
| PD24326a | 6 | 152028452 | 152033321 | 4869 | 7 | 0 | 741.571429 | |
| PD24326a | 7 | 16784317 | 16787655 | 3338 | 11 | 0 | 347.272727 | |
| PD24326a | 7 | 17229209 | 17235066 | 5857 | 7 | 0 | 842.285714 | |
| PD24326a | 9 | 97634432 | 97636059 | 1627 | 6 | 0 | 378.333333 | |
| PD24327a | 11 | 70749655 | 70752674 | 3019 | 14 | 0 | 218.714286 | |
| PD24327a | 11 | 71494864 | 71495388 | 524 | 8 | 0 | 193.75 | |
| PD24327a | 13 | 21942716 | 21944933 | 2217 | 6 | 0 | 417.166667 | |
| PD24327a | 13 | 26893260 | 26901667 | 8407 | 29 | 0 | 315.827586 | |
| PD24327a | 13 | 26906266 | 26914319 | 8053 | 14 | 0 | 621 | |
| PD24327a | 13 | 26920618 | 26923882 | 3264 | 6 | 0 | 638.666667 | |
| PD24327a | 13 | 27228329 | 27232004 | 3675 | 6 | 0 | 666.333333 | |
| PD24327a | 13 | 27238522 | 27262768 | 24246 | 40 | 0 | 622.05 | |
| PD24327a | 13 | 27403127 | 27410423 | 7296 | 10 | 0 | 835.7 | |
| PD24327a | 13 | 27417107 | 27423745 | 6638 | 14 | 0 | 481.785714 | |
| PD24327a | 13 | 45632647 | 45651296 | 18649 | 28 | 0 | 673.642857 | |
| PD24327a | 13 | 47600038 | 47632602 | 32564 | 48 | 0 | 679.916667 | |
| PD24327a | 13 | 53693670 | 53713805 | 20135 | 25 | 0 | 829.72 | |
| PD24327a | 13 | 54715112 | 54726827 | 11715 | 18 | 0 | 651.222222 | |
| PD24327a | 13 | 73281789 | 73284647 | 2858 | 13 | 0 | 238.692308 | |

| sample | chr | start.bp | end.bp | length.bp | number.bps | number.bps.clustered | avgDist.bp | alternative.kataegis |
|--------|-----|----------|--------|-----------|------------|----------------------|------------|----------------------|
| PD24327a | 14 | 82374557 | 82378697 | 4140 | 6 | 0 | 727.166667 | |
| PD24327a | 20 | 35843293 | 35843747 | 454 | 7 | 0 | 88.2857143 | |
| PD24327a | 20 | 35850084 | 35852413 | 2329 | 8 | 0 | 368 | |
| PD24327a | 20 | 38971349 | 38974992 | 3643 | 9 | 0 | 406.888889 | |
| PD24327a | 20 | 49670592 | 49671966 | 1374 | 8 | 0 | 289.375 | |
| PD24327a | 20 | 52003568 | 52010555 | 6987 | 18 | 0 | 398.888889 | |
| PD24327a | 6 | 24132796 | 24134046 | 1250 | 9 | 0 | 163.555556 | |
| PD24327a | 6 | 24920254 | 24921265 | 1011 | 6 | 0 | 303.666667 | |
| PD24327a | 6 | 34258231 | 34259402 | 1171 | 10 | 0 | 126.8 | |
| PD24327a | 6 | 78723089 | 78724558 | 1469 | 22 | 0 | 70.0454545 | |
| PD24332a | 12 | 88613787 | 88614341 | 554 | 6 | 0 | 155.666667 | |
| PD24333a | 1 | 49532246 | 49532306 | 60 | 6 | 0 | 11 | |
| PD24333a | 11 | 121263613 | 121267536 | 3923 | 6 | 0 | 870.5 | |
| PD24333a | 4 | 132831264 | 132831658 | 394 | 13 | 0 | 34.7692308 | |
| PD24333a | 5 | 171652272 | 171653441 | 1169 | 12 | 0 | 129.166667 | |
| PD24333a | 5 | 178446225 | 178446657 | 432 | 12 | 0 | 70.5833333 | |
| PD24335a | 2 | 1805922 | 1811906 | 5984 | 18 | 0 | 358.388889 | |
| PD24335a | 4 | 126913779 | 126918024 | 4245 | 12 | 0 | 371.75 | |
| PD24335a | 4 | 144604010 | 144615173 | 11163 | 15 | 0 | 782.466667 | |
| PD24335a | 8 | 57405706 | 57408827 | 3121 | 7 | 0 | 492.857143 | |
| PD11388a | 12 | 4073350 | 4076093 | 2743 | 6 | 0 | 469.833333 | |
| PD11388a | 17 | 61905191 | 61907420 | 2229 | 25 | 0 | 89.6 | |
| PD11388a | 8 | 98044900 | 98045928 | 1028 | 6 | 0 | 179 | |

**Table 10D**

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD10010 | 10 | 12 | 1 | 8 | 20 | 57 | 108 | A |
| PD10011 | 107 | 11 | 9 | 0 | 15 | 1 | 143 | F |
| PD10014 | 46 | 52 | 62 | 0 | 56 | 6 | 222 | D |
| PD11326 | 26 | 79 | 182 | 4 | 57 | 11 | 359 | D |
| PD11327 | 11 | 67 | 52 | 20 | 49 | 76 | 275 | D |
| PD11336 | 8 | 1 | 1 | 13 | 2 | 50 | 75 | A |
| PD11337 | 5 | 29 | 2 | 26 | 5 | 11 | 78 | B |
| PD11338 | 0 | 1 | 0 | 0 | 0 | 0 | 1 | E |
| PD11339 | 1 | 17 | 0 | 23 | 4 | 17 | 62 | C |
| PD11340 | 8 | 71 | 8 | 99 | 109 | 54 | 349 | B |
| PD11341 | 0 | 31 | 1 | 0 | 7 | 1 | 40 | E |
| PD11342 | 3 | 4 | 0 | 5 | 0 | 5 | 17 | C |
| PD11343 | 3 | 40 | 1 | 50 | 9 | 19 | 122 | B |
| PD11344 | 2 | 20 | 1 | 70 | 3 | 68 | 164 | C |
| PD11345 | 26 | 123 | 5 | 65 | 21 | 210 | 450 | A |
| PD11346 | 11 | 56 | 5 | 153 | 6 | 240 | 471 | C |
| PD11347 | 0 | 17 | 1 | 41 | 10 | 77 | 146 | C |
| PD11348 | 7 | 14 | 3 | 41 | 6 | 59 | 130 | C |
| PD11349 | 12 | 120 | 9 | 86 | 57 | 167 | 451 | A |
| PD11352 | 1 | 1 | 0 | 0 | 0 | 0 | 2 | F |
| PD11355 | 0 | 36 | 0 | 0 | 5 | 2 | 43 | E |
| PD11357 | 0 | 7 | 0 | 1 | 2 | 8 | 18 | A |
| PD11359 | 0 | 1 | 0 | 0 | 0 | 0 | 1 | E |
| PD11360 | 4 | 15 | 2 | 35 | 6 | 25 | 87 | C |
| PD11361 | 0 | 5 | 0 | 0 | 0 | 0 | 5 | E |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD11364 | 3 | 9 | 0 | 8 | 2 | 1 | 23 | B |
| PD11365 | 15 | 6 | 0 | 0 | 6 | 1 | 28 | F |
| PD11366 | 1 | 29 | 0 | 0 | 1 | 1 | 32 | E |
| PD11367 | 3 | 42 | 1 | 40 | 4 | 14 | 104 | B |
| PD11368 | 37 | 53 | 1 | 59 | 22 | 161 | 333 | A |
| PD11369 | 2 | 38 | 3 | 89 | 17 | 159 | 308 | C |
| PD11370 | 0 | 3 | 0 | 0 | 0 | 0 | 3 | E |
| PD11372 | 0 | 5 | 0 | 36 | 6 | 10 | 57 | B |
| PD11374 | 5 | 24 | 1 | 17 | 7 | 15 | 69 | B |
| PD11375 | 0 | 5 | 0 | 0 | 1 | 0 | 6 | E |
| PD11376 | 0 | 8 | 3 | 9 | 2 | 42 | 64 | A |
| PD11379 | 39 | 32 | 1 | 9 | 19 | 15 | 115 | F |
| PD11380 | 1 | 5 | 1 | 2 | 5 | 17 | 31 | A |
| PD11381 | 0 | 15 | 1 | 11 | 2 | 1 | 30 | B |
| PD11383 | 0 | 4 | 0 | 0 | 0 | 0 | 4 | E |
| PD11384 | 0 | 4 | 1 | 0 | 0 | 0 | 5 | E |
| PD11385 | 3 | 15 | 1 | 29 | 1 | 27 | 76 | C |
| PD11386 | 0 | 14 | 1 | 0 | 0 | 0 | 15 | E |
| PD11388 | 1 | 11 | 4 | 44 | 5 | 91 | 156 | C |
| PD11389 | 1 | 0 | 0 | 0 | 0 | 0 | 1 | F |
| PD11391 | 1 | 0 | 0 | 0 | 1 | 0 | 2 | F |
| PD11393 | 4 | 36 | 3 | 21 | 2 | 91 | 157 | A |
| PD11394 | 0 | 1 | 0 | 17 | 1 | 6 | 25 | B |
| PD11395 | 0 | 4 | 0 | 3 | 1 | 16 | 24 | A |
| PD11396 | 1 | 26 | 1 | 10 | 3 | 5 | 46 | E |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD11397 | 4 | 38 | 3 | 21 | 9 | 25 | 100 | B |
| PD11398 | 4 | 8 | 0 | 26 | 2 | 4 | 44 | B |
| PD11399 | 0 | 1 | 1 | 74 | 4 | 46 | 126 | C |
| PD11402 | 0 | 8 | 0 | 6 | 0 | 17 | 31 | A |
| PD11462 | 5 | 15 | 2 | 15 | 9 | 102 | 148 | A |
| PD11464 | 26 | 10 | 4 | 86 | 0 | 28 | 154 | B |
| PD11465 | 55 | 20 | 3 | 36 | 21 | 73 | 208 | A |
| PD11740 | 2 | 13 | 0 | 0 | 0 | 0 | 15 | E |
| PD11741 | 3 | 6 | 0 | 11 | 2 | 12 | 34 | C |
| PD11742 | 57 | 117 | 86 | 6 | 54 | 15 | 335 | D |
| PD11743 | 23 | 20 | 3 | 9 | 9 | 19 | 83 | F |
| PD11744 | 0 | 1 | 0 | 0 | 0 | 0 | 1 | E |
| PD11745 | 4 | 34 | 2 | 18 | 9 | 87 | 154 | A |
| PD11748 | 43 | 62 | 232 | 0 | 61 | 3 | 401 | D |
| PD11750 | 24 | 27 | 127 | 4 | 32 | 5 | 219 | D |
| PD11751 | 40 | 82 | 238 | 3 | 137 | 13 | 513 | D |
| PD11752 | 4 | 55 | 0 | 25 | 44 | 117 | 245 | A |
| PD11753 | 0 | 5 | 0 | 0 | 4 | 0 | 9 | G |
| PD11755 | 8 | 29 | 77 | 0 | 15 | 2 | 131 | D |
| PD11756 | 0 | 7 | 0 | 4 | 0 | 6 | 17 | E |
| PD11757 | 11 | 14 | 56 | 0 | 12 | 1 | 94 | D |
| PD11760 | 2 | 20 | 1 | 33 | 6 | 13 | 75 | B |
| PD11761 | 3 | 10 | 2 | 18 | 4 | 33 | 70 | C |
| PD11762 | 1 | 12 | 0 | 0 | 2 | 1 | 16 | E |
| PD11765 | 17 | 18 | 4 | 0 | 3 | 1 | 43 | F |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD11766 | 0 | 26 | 0 | 35 | 3 | 17 | 81 | B |
| PD11767 | 0 | 3 | 0 | 0 | 2 | 0 | 5 | G |
| PD11769 | 1 | 6 | 1 | 10 | 2 | 34 | 54 | A |
| PD11816 | 2 | 22 | 0 | 14 | 10 | 9 | 57 | B |
| PD11818 | 11 | 45 | 2 | 152 | 0 | 91 | 301 | C |
| PD11819 | 0 | 0 | 0 | 0 | 1 | 0 | 1 | G |
| PD13162 | 15 | 44 | 3 | 20 | 8 | 46 | 136 | A |
| PD13163 | 6 | 53 | 2 | 24 | 12 | 75 | 172 | A |
| PD13164 | 2 | 12 | 2 | 8 | 3 | 58 | 85 | A |
| PD13165 | 124 | 80 | 4 | 21 | 28 | 67 | 324 | F |
| PD13166 | 7 | 58 | 6 | 59 | 4 | 39 | 173 | B |
| PD13167 | 5 | 83 | 4 | 60 | 1 | 89 | 242 | C |
| PD13168 | 1 | 18 | 2 | 55 | 4 | 97 | 177 | C |
| PD13296 | 6 | 107 | 1000 | 1 | 89 | 18 | 1221 | D |
| PD13297 | 61 | 68 | 122 | 0 | 63 | 7 | 321 | D |
| PD13298 | 11 | 43 | 3 | 127 | 22 | 27 | 233 | B |
| PD13299 | 14 | 41 | 125 | 2 | 87 | 8 | 277 | D |
| PD13302 | 5 | 15 | 2 | 128 | 6 | 111 | 267 | C |
| PD13306 | 0 | 18 | 0 | 0 | 0 | 0 | 18 | E |
| PD13307 | 5 | 29 | 1 | 6 | 1 | 36 | 78 | A |
| PD13310 | 1 | 0 | 0 | 0 | 0 | 0 | 1 | F |
| PD13311 | 12 | 22 | 87 | 0 | 22 | 2 | 145 | D |
| PD13312 | 9 | 5 | 1 | 0 | 5 | 1 | 21 | F |
| PD13418 | 0 | 37 | 1 | 0 | 9 | 2 | 49 | E |
| PD13419 | 0 | 3 | 0 | 3 | 0 | 19 | 25 | A |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD13420 | 0 | 5 | 0 | 5 | 0 | 6 | 16 | C |
| PD13422 | 2 | 6 | 0 | 0 | 2 | 0 | 10 | E |
| PD13424 | 8 | 12 | 8 | 91 | 8 | 89 | 216 | C |
| PD13425 | 22 | 139 | 11 | 217 | 49 | 179 | 617 | C |
| PD13427 | 0 | 2 | 0 | 0 | 0 | 0 | 2 | E |
| PD13428 | 4 | 17 | 0 | 3 | 5 | 20 | 49 | A |
| PD13602 | 17 | 79 | 14 | 22 | 59 | 40 | 231 | G |
| PD13603 | 6 | 60 | 5 | 85 | 8 | 144 | 308 | C |
| PD13604 | 1 | 6 | 3 | 13 | 36 | 90 | 149 | A |
| PD13605 | 4 | 41 | 1 | 40 | 5 | 72 | 163 | C |
| PD13606 | 6 | 47 | 3 | 92 | 11 | 177 | 336 | C |
| PD13607 | 5 | 21 | 0 | 4 | 5 | 5 | 40 | E |
| PD13608 | 5 | 26 | 3 | 93 | 5 | 42 | 174 | B |
| PD13609 | 14 | 179 | 2 | 182 | 10 | 91 | 478 | B |
| PD13619 | 0 | 2 | 0 | 0 | 0 | 0 | 2 | E |
| PD13620 | 8 | 106 | 0 | 14 | 17 | 10 | 155 | E |
| PD13622 | 32 | 50 | 2 | 45 | 42 | 85 | 256 | A |
| PD13623 | 0 | 4 | 1 | 0 | 5 | 0 | 10 | G |
| PD13625 | 20 | 67 | 5 | 48 | 24 | 79 | 243 | C |
| PD13626 | 5 | 14 | 0 | 9 | 1 | 9 | 38 | B |
| PD13627 | 8 | 42 | 67 | 0 | 61 | 6 | 184 | D |
| PD13629 | 0 | 2 | 0 | 0 | 0 | 0 | 2 | E |
| PD13630 | 1 | 44 | 3 | 7 | 5 | 25 | 85 | E |
| PD13631 | 0 | 4 | 0 | 7 | 2 | 6 | 19 | C |
| PD13752 | 9 | 26 | 1 | 5 | 10 | 8 | 59 | E |

| Samples | Number of SV in samples | | | | | | Total | Cluster Group |
|---------|---------------------------|--|--|--|--|--|-------|---------------|
| | Rearrangement Signature1 | Rearrangement Signature2 | Rearrangement Signature3 | Rearrangement Signature4 | Rearrangement Signature5 | Rearrangement Signature6 | | |
| PD13753 | 2 | 5 | 0 | 0 | 3 | 0 | 10 | G |
| PD13754 | 0 | 13 | 1 | 0 | 5 | 1 | 20 | E |
| PD13755 | 0 | 0 | 0 | 0 | 1 | 0 | 1 | G |
| PD13757 | 2 | 7 | 0 | 2 | 1 | 12 | 24 | A |
| PD13758 | 0 | 7 | 0 | 10 | 2 | 6 | 25 | B |
| PD13760 | 0 | 0 | 0 | 1 | 0 | 8 | 9 | A |
| PD13761 | 0 | 0 | 1 | 0 | 0 | 0 | 1 | D |
| PD13762 | 0 | 0 | 1 | 0 | 0 | 0 | 1 | D |
| PD13763 | 0 | 10 | 1 | 7 | 4 | 46 | 68 | A |
| PD13764 | 18 | 216 | 9 | 511 | 16 | 219 | 989 | B |
| PD13765 | 3 | 25 | 2 | 16 | 34 | 29 | 109 | G |
| PD13766 | 0 | 57 | 2 | 0 | 6 | 2 | 67 | E |
| PD13767 | 0 | 7 | 1 | 14 | 2 | 1 | 25 | B |
| PD13768 | 0 | 1 | 0 | 0 | 0 | 0 | 1 | E |
| PD13770 | 5 | 5 | 0 | 12 | 4 | 8 | 34 | C |
| PD13771 | 50 | 50 | 10 | 14 | 128 | 28 | 280 | G |
| PD14432 | 0 | 13 | 0 | 0 | 0 | 0 | 13 | E |
| PD14433 | 0 | 0 | 0 | 7 | 0 | 47 | 54 | A |
| PD14435 | 27 | 63 | 2 | 43 | 6 | 59 | 200 | C |
| PD14437 | 32 | 129 | 0 | 21 | 9 | 26 | 217 | E |
| PD14439 | 0 | 5 | 0 | 0 | 0 | 0 | 5 | E |
| PD14441 | 0 | 2 | 0 | 20 | 1 | 8 | 31 | B |
| PD14442 | 0 | 1 | 0 | 0 | 1 | 0 | 2 | G |
| PD14450 | 0 | 4 | 1 | 23 | 0 | 18 | 46 | C |
| PD14453 | 43 | 125 | 7 | 46 | 25 | 72 | 318 | E |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD14454 | 9 | 14 | 1 | 18 | 6 | 82 | 130 | A |
| PD14456 | 0 | 1 | 0 | 11 | 0 | 15 | 27 | C |
| PD14457 | 9 | 54 | 4 | 75 | 6 | 35 | 183 | B |
| PD14459 | 0 | 8 | 0 | 8 | 1 | 13 | 30 | C |
| PD14460 | 0 | 8 | 1 | 22 | 2 | 24 | 57 | C |
| PD14461 | 0 | 16 | 0 | 10 | 2 | 9 | 37 | B |
| PD14462 | 0 | 7 | 1 | 3 | 0 | 18 | 29 | A |
| PD14465 | 4 | 35 | 1 | 32 | 4 | 23 | 99 | B |
| PD14467 | 0 | 15 | 1 | 15 | 3 | 18 | 52 | C |
| PD14468 | 0 | 13 | 0 | 0 | 2 | 0 | 15 | E |
| PD14471 | 2 | 37 | 1 | 19 | 6 | 49 | 114 | A |
| PD14472 | 0 | 7 | 0 | 0 | 0 | 0 | 7 | E |
| PD14473 | 3 | 5 | 0 | 21 | 0 | 9 | 38 | B |
| PD17973 | 2 | 12 | 0 | 0 | 0 | 1 | 15 | E |
| PD17981 | 2 | 2 | 0 | 7 | 4 | 30 | 45 | A |
| PD17991 | 0 | 3 | 0 | 0 | 0 | 0 | 3 | E |
| PD17994 | 2 | 4 | 0 | 0 | 0 | 0 | 6 | E |
| PD18017 | 28 | 5 | 0 | 2 | 6 | 18 | 59 | F |
| PD18020 | 20 | 91 | 153 | 15 | 131 | 36 | 446 | D |
| PD18022 | 2 | 27 | 1 | 34 | 0 | 49 | 113 | C |
| PD18024 | 96 | 75 | 382 | 2 | 98 | 13 | 666 | D |
| PD18031 | 4 | 10 | 2 | 35 | 17 | 25 | 93 | C |
| PD18037 | 9 | 71 | 6 | 4 | 81 | 26 | 197 | G |
| PD18045 | 20 | 153 | 5 | 180 | 18 | 194 | 570 | C |
| PD18046 | 3 | 31 | 0 | 0 | 4 | 1 | 39 | E |

(continued)

| Samples | Number of SV in samples | | | | | | Total | Cluster Group |
| --- | --- | --- | --- | --- | --- | --- | --- | --- |
| | Rearrangement Signature1 | Rearrangement Signature2 | Rearrangement Signature3 | Rearrangement Signature4 | Rearrangement Signature5 | Rearrangement Signature6 | | |
| PD18047 | 0 | 6 | 4 | 27 | 3 | 49 | 89 | C |
| PD18048 | 47 | 32 | 4 | 24 | 43 | 49 | 199 | F |
| PD18049 | 3 | 26 | 0 | 3 | 5 | 13 | 50 | E |
| PD18050 | 17 | 97 | 2 | 126 | 6 | 51 | 299 | B |
| PD18100 | 0 | 8 | 0 | 0 | 0 | 0 | 8 | E |
| PD18101 | 2 | 7 | 1 | 0 | 0 | 0 | 10 | E |
| PD18116 | 0 | 8 | 0 | 0 | 0 | 1 | 9 | E |
| PD18149 | 0 | 5 | 0 | 2 | 4 | 11 | 22 | A |
| PD18188 | 2 | 6 | 0 | 35 | 2 | 7 | 52 | B |
| PD18189 | 12 | 34 | 0 | 3 | 12 | 11 | 72 | E |
| PD18247 | 1 | 0 | 0 | 0 | 1 | 0 | 2 | F |
| PD18251 | 20 | 60 | 2 | 15 | 9 | 20 | 126 | E |
| PD18257 | 1 | 23 | 2 | 7 | 3 | 15 | 51 | E |
| PD18258 | 1 | 7 | 0 | 0 | 0 | 0 | 8 | E |
| PD18259 | 2 | 33 | 0 | 0 | 39 | 4 | 78 | G |
| PD18264 | 4 | 14 | 1 | 5 | 7 | 9 | 40 | E |
| PD18269 | 0 | 2 | 0 | 0 | 0 | 0 | 2 | E |
| PD18728 | 0 | 1 | 1 | 0 | 0 | 0 | 2 | D |
| PD18730 | 0 | 4 | 0 | 8 | 0 | 8 | 20 | C |
| PD18733 | 0 | 9 | 0 | 15 | 2 | 7 | 33 | B |
| PD18734 | 4 | 2 | 1 | 153 | 4 | 154 | 318 | C |
| PD18748 | 0 | 7 | 0 | 10 | 0 | 1 | 18 | B |
| PD18749 | 0 | 1 | 0 | 0 | 1 | 0 | 2 | G |
| PD18751 | 0 | 10 | 1 | 35 | 3 | 22 | 71 | C |
| PD18754 | 0 | 6 | 0 | 8 | 5 | 2 | 21 | B |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD18756 | 0 | 2 | 0 | 0 | 0 | 0 | 2 | E |
| PD18768 | 0 | 2 | 0 | 0 | 0 | 0 | 2 | E |
| PD18769 | 2 | 10 | 0 | 0 | 7 | 1 | 20 | G |
| PD18771 | 0 | 1 | 0 | 5 | 0 | 2 | 8 | B |
| PD18775 | 0 | 9 | 1 | 0 | 0 | 0 | 10 | E |
| PD18776 | 0 | 0 | 0 | 6 | 1 | 6 | 13 | C |
| PD22251 | 3 | 10 | 1 | 16 | 2 | 7 | 39 | B |
| PD22355 | 50 | 19 | 337 | 0 | 67 | 1 | 474 | D |
| PD22357 | 6 | 68 | 5 | 41 | 9 | 146 | 275 | A |
| PD22358 | 5 | 11 | 0 | 2 | 30 | 13 | 61 | G |
| PD22359 | 0 | 20 | 2 | 1 | 4 | 7 | 34 | E |
| PD22360 | 7 | 48 | 42 | 0 | 53 | 5 | 155 | G |
| PD22361 | 5 | 50 | 1 | 39 | 0 | 34 | 129 | B |
| PD22362 | 3 | 57 | 1 | 62 | 0 | 36 | 159 | B |
| PD22363 | 10 | 41 | 2 | 0 | 72 | 7 | 132 | G |
| PD22364 | 6 | 11 | 3 | 2 | 2 | 16 | 40 | A |
| PD22365 | 6 | 27 | 0 | 1 | 2 | 11 | 47 | E |
| PD22366 | 30 | 80 | 81 | 3 | 57 | 11 | 262 | D |
| PD23550 | 1 | 16 | 4 | 133 | 3 | 113 | 270 | C |
| PD23554 | 5 | 8 | 95 | 0 | 22 | 1 | 131 | D |
| PD23558 | 0 | 64 | 2 | 1 | 63 | 18 | 148 | G |
| PD23559 | 33 | 7 | 0 | 0 | 40 | 4 | 84 | F |
| PD23560 | 1 | 55 | 0 | 1 | 7 | 12 | 76 | E |
| PD23561 | 0 | 6 | 1 | 7 | 6 | 55 | 75 | A |
| PD23562 | 19 | 41 | 110 | 5 | 37 | 9 | 221 | D |

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD23563 | 32 | 68 | 49 | 0 | 60 | 7 | 216 | D |
| PD23564 | 2 | 1 | 1 | 0 | 5 | 0 | 9 | G |
| PD23565 | 7 | 74 | 3 | 26 | 23 | 42 | 175 | E |
| PD23566 | 41 | 52 | 99 | 0 | 67 | 6 | 265 | D |
| PD23567 | 5 | 36 | 145 | 5 | 34 | 30 | 255 | D |
| PD23569 | 4 | 12 | 2 | 0 | 3 | 1 | 22 | E |
| PD23570 | 0 | 13 | 0 | 9 | 1 | 4 | 27 | B |
| PD23574 | 30 | 59 | 118 | 8 | 100 | 10 | 325 | D |
| PD23577 | 47 | 17 | 101 | 0 | 31 | 2 | 198 | D |
| PD23578 | 39 | 23 | 125 | 0 | 31 | 1 | 219 | D |
| PD23579 | 3 | 2 | 0 | 0 | 3 | 1 | 9 | F |
| PD24182 | 5 | 53 | 90 | 2 | 39 | 6 | 195 | D |
| PD24186 | 6 | 39 | 9 | 0 | 53 | 6 | 113 | G |
| PD24189 | 0 | 2 | 2 | 0 | 4 | 0 | 8 | G |
| PD24190 | 29 | 33 | 12 | 55 | 16 | 81 | 226 | C |
| PD24191 | 3 | 21 | 2 | 0 | 28 | 3 | 57 | G |
| PD24192 | 1 | 88 | 3 | 0 | 121 | 12 | 225 | G |
| PD24193 | 0 | 2 | 0 | 0 | 0 | 0 | 2 | E |
| PD24194 | 4 | 26 | 2 | 5 | 5 | 9 | 51 | E |
| PD24195 | 9 | 1 | 0 | 0 | 4 | 0 | 14 | F |
| PD24196 | 2 | 43 | 7 | 17 | 16 | 58 | 143 | A |
| PD24197 | 9 | 42 | 209 | 0 | 53 | 5 | 318 | D |
| PD24199 | 4 | 27 | 2 | 63 | 8 | 28 | 132 | B |
| PD24200 | 1 | 6 | 33 | 0 | 6 | 1 | 47 | D |
| PD24201 | 49 | 111 | 153 | 0 | 68 | 8 | 389 | D |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD24202 | 41 | 8 | 216 | 1 | 22 | 4 | 292 | D |
| PD24204 | 0 | 4 | 0 | 0 | 1 | 0 | 5 | E |
| PD24205 | 0 | 47 | 1 | 0 | 32 | 4 | 84 | G |
| PD24206 | 6 | 61 | 1 | 19 | 7 | 10 | 104 | E |
| PD24207 | 6 | 86 | 3 | 135 | 7 | 110 | 347 | C |
| PD24208 | 25 | 20 | 8 | 0 | 7 | 1 | 61 | F |
| PD24209 | 21 | 3 | 2 | 8 | 4 | 36 | 74 | A |
| PD24212 | 4 | 51 | 8 | 69 | 57 | 47 | 236 | B |
| PD24214 | 2 | 19 | 1 | 87 | 8 | 96 | 213 | C |
| PD24215 | 21 | 11 | 5 | 1 | 17 | 9 | 64 | F |
| PD24216 | 39 | 3 | 0 | 0 | 15 | 1 | 58 | F |
| PD24217 | 8 | 20 | 3 | 51 | 0 | 28 | 110 | B |
| PD24218 | 1 | 14 | 1 | 9 | 6 | 64 | 95 | A |
| PD24219 | 5 | 58 | 1 | 6 | 9 | 21 | 100 | E |
| PD24220 | 2 | 15 | 3 | 109 | 11 | 243 | 383 | C |
| PD24221 | 0 | 6 | 2 | 0 | 4 | 0 | 12 | G |
| PD24223 | 1 | 8 | 0 | 0 | 2 | 1 | 12 | E |
| PD24224 | 2 | 15 | 1 | 11 | 3 | 10 | 42 | B |
| PD24225 | 0 | 5 | 0 | 8 | 2 | 16 | 31 | C |
| PD24302 | 1 | 4 | 0 | 0 | 0 | 0 | 5 | E |
| PD24303 | 8 | 2 | 147 | 0 | 8 | 0 | 165 | D |
| PD24304 | 3 | 25 | 33 | 0 | 27 | 3 | 91 | D |
| PD24306 | 15 | 4 | 82 | 0 | 6 | 0 | 107 | D |
| PD24307 | 0 | 1 | 0 | 8 | 0 | 40 | 49 | A |
| PD24308 | 22 | 69 | 1 | 45 | 21 | 62 | 220 | C |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD24314 | 9 | 26 | 5 | 16 | 17 | 68 | 141 | A |
| PD24318 | 10 | 12 | 1 | 19 | 17 | 35 | 94 | A |
| PD24320 | 7 | 21 | 0 | 5 | 3 | 25 | 61 | A |
| PD24322 | 26 | 35 | 2 | 5 | 4 | 26 | 98 | F |
| PD24325 | 79 | 52 | 5 | 4 | 51 | 8 | 199 | F |
| PD24326 | 4 | 12 | 1 | 7 | 2 | 35 | 61 | A |
| PD24327 | 2 | 55 | 2 | 110 | 5 | 74 | 248 | C |
| PD24329 | 2 | 56 | 0 | 13 | 2 | 21 | 94 | E |
| PD24332 | 6 | 28 | 1 | 7 | 4 | 33 | 79 | A |
| PD24333 | 5 | 24 | 7 | 0 | 12 | 2 | 50 | G |
| PD24335 | 5 | 116 | 2 | 206 | 3 | 103 | 435 | B |
| PD24336 | 4 | 22 | 0 | 46 | 1 | 7 | 80 | B |
| PD24337 | 88 | 63 | 271 | 0 | 35 | 3 | 460 | D |
| PD3851 | 0 | 3 | 0 | 14 | 1 | 14 | 32 | C |
| PD3890 | 14 | 13 | 112 | 0 | 26 | 1 | 166 | D |
| PD3904 | 14 | 41 | 4 | 8 | 55 | 39 | 161 | G |
| PD3905 | 6 | 29 | 52 | 7 | 39 | 7 | 140 | D |
| PD3945 | 11 | 16 | 2 | 0 | 68 | 6 | 103 | G |
| PD3989 | 2 | 5 | 2 | 0 | 5 | 1 | 15 | G |
| PD4005 | 26 | 28 | 101 | 0 | 26 | 2 | 183 | D |
| PD4006 | 104 | 22 | 158 | 0 | 63 | 5 | 352 | D |
| PD4069 | 0 | 2 | 0 | 0 | 1 | 0 | 3 | E |
| PD4072 | 0 | 0 | 0 | 0 | 1 | 0 | 1 | G |
| PD4076 | 0 | 0 | 0 | 0 | 2 | 0 | 2 | G |
| PD4085 | 2 | 28 | 0 | 6 | 2 | 27 | 65 | A |

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD4086 | 12 | 12 | 5 | 0 | 19 | 2 | 50 | F |
| PD4088 | 5 | 51 | 2 | 49 | 1 | 63 | 171 | C |
| PD4103 | 12 | 162 | 4 | 302 | 32 | 101 | 613 | B |
| PD4107 | 15 | 14 | 92 | 39 | 71 | 59 | 290 | D |
| PD4109 | 44 | 53 | 0 | 0 | 75 | 9 | 181 | F |
| PD4115 | 0 | 56 | 2 | 0 | 63 | 7 | 128 | G |
| PD4116 | 4 | 80 | 5 | 18 | 92 | 56 | 255 | G |
| PD4192 | 2 | 62 | 3 | 5 | 7 | 22 | 101 | E |
| PD4194 | 0 | 0 | 0 | 3 | 1 | 19 | 23 | A |
| PD4198 | 5 | 54 | 2 | 80 | 12 | 67 | 220 | C |
| PD4199 | 8 | 30 | 1 | 13 | 12 | 22 | 86 | E |
| PD4225 | 0 | 32 | 0 | 13 | 0 | 1 | 46 | E |
| PD4248 | 15 | 38 | 2 | 14 | 12 | 59 | 140 | A |
| PD4252 | 38 | 52 | 0 | 10 | 32 | 18 | 150 | F |
| PD4255 | 66 | 117 | 8 | 66 | 37 | 17 | 311 | B |
| PD4261 | 0 | 8 | 0 | 10 | 3 | 2 | 23 | B |
| PD4264 | 0 | 15 | 1 | 36 | 1 | 18 | 71 | B |
| PD4266 | 0 | 0 | 0 | 1 | 1 | 5 | 7 | A |
| PD4315 | 15 | 56 | 6 | 69 | 5 | 56 | 207 | C |
| PD4604 | 9 | 93 | 5 | 9 | 55 | 31 | 202 | G |
| PD4605 | 4 | 42 | 2 | 15 | 4 | 31 | 98 | E |
| PD4606 | 0 | 44 | 0 | 0 | 3 | 1 | 48 | E |
| PD4607 | 1 | 4 | 0 | 10 | 0 | 2 | 17 | B |
| PD4613 | 2 | 2 | 0 | 8 | 2 | 9 | 23 | C |
| PD4826 | 18 | 69 | 2 | 101 | 10 | 92 | 292 | C |

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD4833 | 21 | 19 | 434 | 0 | 64 | 2 | 540 | D |
| PD4836 | 7 | 15 | 5 | 6 | 43 | 5 | 81 | G |
| PD4841 | 493 | 65 | 11 | 10 | 25 | 4 | 608 | F |
| PD4844 | 19 | 80 | 11 | 0 | 95 | 11 | 216 | G |
| PD4845 | 9 | 21 | 25 | 5 | 80 | 15 | 155 | G |
| PD4847 | 97 | 24 | 12 | 17 | 36 | 67 | 253 | F |
| PD4872 | 5 | 25 | 6 | 10 | 35 | 10 | 91 | G |
| PD4874 | 6 | 107 | 9 | 23 | 63 | 14 | 222 | G |
| PD4875 | 5 | 33 | 2 | 3 | 82 | 14 | 139 | G |
| PD4876 | 7 | 43 | 0 | 0 | 41 | 4 | 95 | G |
| PD4951 | 4 | 14 | 0 | 0 | 13 | 2 | 33 | G |
| PD4952 | 20 | 93 | 3 | 33 | 138 | 47 | 334 | G |
| PD4953 | 41 | 28 | 2 | 17 | 42 | 13 | 143 | F |
| PD4954 | 12 | 16 | 2 | 2 | 77 | 18 | 127 | G |
| PD4955 | 17 | 25 | 2 | 0 | 53 | 5 | 102 | G |
| PD4956 | 80 | 28 | 20 | 0 | 99 | 9 | 236 | F |
| PD4957 | 4 | 27 | 0 | 1 | 30 | 12 | 74 | G |
| PD4958 | 2 | 41 | 4 | 0 | 50 | 6 | 103 | G |
| PD4959 | 6 | 15 | 0 | 11 | 51 | 20 | 103 | G |
| PD4962 | 10 | 43 | 2 | 12 | 5 | 14 | 86 | E |
| PD4965 | 1 | 6 | 0 | 21 | 2 | 3 | 33 | B |
| PD4967 | 0 | 1 | 0 | 1 | 1 | 6 | 9 | A |
| PD4968 | 8 | 22 | 2 | 123 | 24 | 35 | 214 | B |
| PD4969 | 1 | 7 | 0 | 2 | 0 | 8 | 18 | A |
| PD4970 | 5 | 1 | 0 | 4 | 2 | 19 | 31 | A |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD4971 | 4 | 197 | 4 | 134 | 1 | 44 | 384 | B |
| PD4975 | 0 | 17 | 1 | 0 | 52 | 5 | 75 | G |
| PD4976 | 3 | 74 | 2 | 4 | 0 | 34 | 117 | E |
| PD4977 | 1 | 3 | 0 | 0 | 10 | 1 | 15 | G |
| PD4978 | 7 | 38 | 4 | 278 | 3 | 227 | 557 | C |
| PD4980 | 41 | 20 | 3 | 2 | 7 | 15 | 88 | F |
| PD4981 | 0 | 2 | 0 | 0 | 0 | 0 | 2 | E |
| PD4985 | 0 | 2 | 0 | 0 | 0 | 0 | 2 | E |
| PD4986 | 0 | 5 | 0 | 0 | 4 | 0 | 9 | G |
| PD5925 | 1 | 30 | 15 | 5 | 37 | 4 | 92 | G |
| PD5928 | 2 | 27 | 1 | 0 | 46 | 4 | 80 | G |
| PD5930 | 4 | 10 | 97 | 5 | 20 | 4 | 140 | D |
| PD5932 | 46 | 36 | 167 | 3 | 55 | 13 | 320 | D |
| PD5934 | 89 | 52 | 231 | 4 | 66 | 11 | 453 | D |
| PD5935 | 87 | 61 | 122 | 0 | 60 | 5 | 335 | D |
| PD5936 | 0 | 17 | 1 | 28 | 6 | 79 | 131 | A |
| PD5937 | 2 | 3 | 2 | 0 | 2 | 0 | 9 | D |
| PD5942 | 9 | 46 | 4 | 10 | 25 | 67 | 161 | A |
| PD5944 | 10 | 11 | 0 | 0 | 3 | 1 | 25 | F |
| PD5945 | 21 | 63 | 251 | 0 | 94 | 6 | 435 | D |
| PD5946 | 2 | 24 | 1 | 240 | 3 | 133 | 403 | C |
| PD5947 | 8 | 16 | 2 | 42 | 6 | 76 | 150 | C |
| PD5948 | 41 | 95 | 164 | 27 | 107 | 55 | 489 | D |
| PD5950 | 7 | 34 | 0 | 11 | 162 | 15 | 229 | G |
| PD5951 | 8 | 30 | 2 | 11 | 2 | 63 | 116 | A |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD5953 | 0 | 9 | 3 | 0 | 7 | 1 | 20 | G |
| PD5956 | 181 | 3 | 118 | 38 | 156 | 196 | 692 | F |
| PD5959 | 0 | 43 | 5 | 133 | 2 | 126 | 309 | C |
| PD5960 | 0 | 23 | 4 | 0 | 32 | 3 | 62 | G |
| PD5961 | 3 | 56 | 0 | 24 | 1 | 19 | 103 | E |
| PD5964 | 5 | 2 | 1 | 41 | 5 | 15 | 69 | B |
| PD6016 | 0 | 1 | 0 | 0 | 0 | 0 | 1 | E |
| PD6041 | 0 | 2 | 0 | 0 | 0 | 0 | 2 | E |
| PD6042 | 9 | 89 | 1 | 8 | 20 | 35 | 162 | E |
| PD6043 | 44 | 16 | 53 | 0 | 95 | 8 | 216 | G |
| PD6044 | 2 | 17 | 2 | 23 | 4 | 13 | 61 | B |
| PD6045 | 0 | 2 | 1 | 7 | 0 | 23 | 33 | A |
| PD6046 | 3 | 46 | 2 | 34 | 6 | 121 | 212 | A |
| PD6047 | 20 | 36 | 3 | 32 | 15 | 37 | 143 | C |
| PD6048 | 11 | 36 | 8 | 87 | 13 | 187 | 342 | C |
| PD6404 | 21 | 57 | 1 | 11 | 17 | 32 | 139 | E |
| PD6405 | 10 | 59 | 6 | 51 | 28 | 59 | 213 | C |
| PD6406 | 22 | 41 | 96 | 0 | 25 | 2 | 186 | D |
| PD6409 | 36 | 37 | 39 | 1 | 39 | 10 | 162 | D |
| PD6410 | 7 | 26 | 121 | 3 | 25 | 11 | 193 | D |
| PD6411 | 34 | 48 | 53 | 0 | 47 | 4 | 186 | D |
| PD6412 | 6 | 25 | 3 | 0 | 44 | 4 | 82 | G |
| PD6413 | 17 | 16 | 254 | 0 | 32 | 1 | 320 | D |
| PD6414 | 2 | 3 | 1 | 0 | 1 | 0 | 7 | F |
| PD6415 | 42 | 64 | 168 | 20 | 62 | 7 | 363 | D |

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD6416 | 0 | 26 | 0 | 6 | 13 | 26 | 71 | A |
| PD6417 | 2 | 48 | 2 | 57 | 1 | 27 | 137 | B |
| PD6418 | 1 | 1 | 0 | 0 | 0 | 0 | 2 | F |
| PD6422 | 21 | 128 | 4 | 81 | 42 | 84 | 360 | B |
| PD6466 | 0 | 18 | 2 | 22 | 1 | 17 | 60 | C |
| PD6684 | 16 | 51 | 144 | 0 | 78 | 7 | 296 | D |
| PD6711 | 0 | 10 | 0 | 1 | 0 | 9 | 20 | A |
| PD6719 | 6 | 51 | 1 | 19 | 6 | 25 | 108 | E |
| PD6720 | 6 | 40 | 0 | 13 | 2 | 12 | 73 | E |
| PD6721 | 0 | 16 | 0 | 6 | 1 | 22 | 45 | A |
| PD6722 | 26 | 20 | 104 | 0 | 43 | 3 | 196 | D |
| PD6727 | 23 | 63 | 8 | 67 | 26 | 84 | 271 | C |
| PD6728 | 55 | 14 | 6 | 14 | 7 | 19 | 115 | F |
| PD6729 | 4 | 25 | 4 | 20 | 40 | 27 | 120 | G |
| PD6730 | 12 | 15 | 3 | 3 | 41 | 24 | 98 | G |
| PD6731 | 14 | 25 | 29 | 0 | 28 | 3 | 99 | D |
| PD6732 | 29 | 64 | 9 | 49 | 11 | 17 | 179 | B |
| PD6733 | 7 | 38 | 0 | 2 | 24 | 21 | 92 | G |
| PD7066 | 268 | 37 | 2 | 0 | 10 | 4 | 321 | F |
| PD7067 | 7 | 78 | 2 | 124 | 7 | 7 | 225 | B |
| PD7069 | 3 | 49 | 2 | 11 | 7 | 71 | 143 | A |
| PD7199 | 4 | 7 | 1 | 7 | 1 | 49 | 69 | A |
| PD7201 | 2 | 1 | 0 | 1 | 0 | 8 | 12 | A |
| PD7202 | 5 | 137 | 8 | 114 | 14 | 174 | 452 | C |
| PD7203 | 2 | 54 | 3 | 68 | 6 | 43 | 176 | B |

EP 3 452 937 B1

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD7204 | 0 | 4 | 2 | 29 | 0 | 9 | 44 | B |
| PD7205 | 13 | 84 | 9 | 272 | 24 | 116 | 518 | B |
| PD7206 | 7 | 33 | 1 | 53 | 4 | 29 | 127 | B |
| PD7207 | 0 | 0 | 0 | 1 | 0 | 5 | 6 | A |
| PD7209 | 0 | 0 | 1 | 0 | 0 | 0 | 1 | D |
| PD7210 | 0 | 1 | 0 | 0 | 0 | 0 | 1 | E |
| PD7211 | 25 | 24 | 458 | 8 | 70 | 19 | 604 | D |
| PD7214 | 0 | 15 | 0 | 0 | 0 | 0 | 15 | E |
| PD7215 | 36 | 8 | 165 | 3 | 21 | 26 | 259 | D |
| PD7217 | 2 | 39 | 0 | 3 | 66 | 14 | 124 | G |
| PD7218 | 0 | 1 | 0 | 0 | 0 | 0 | 1 | E |
| PD7219 | 17 | 9 | 2 | 5 | 4 | 33 | 70 | A |
| PD7220 | 2 | 10 | 1 | 2 | 11 | 14 | 40 | A |
| PD7221 | 1 | 6 | 0 | 0 | 2 | 0 | 9 | E |
| PD7238 | 3 | 21 | 1 | 15 | 2 | 3 | 45 | B |
| PD7240 | 2 | 76 | 3 | 19 | 22 | 27 | 149 | E |
| PD7243 | 18 | 27 | 1 | 55 | 7 | 95 | 203 | C |
| PD7248 | 59 | 69 | 138 | 4 | 90 | 7 | 367 | D |
| PD7249 | 18 | 33 | 3 | 56 | 10 | 61 | 181 | C |
| PD7250 | 11 | 64 | 169 | 6 | 81 | 13 | 344 | D |
| PD7304 | 40 | 66 | 0 | 20 | 3 | 25 | 154 | F |
| PD7305 | 11 | 28 | 2 | 144 | 4 | 140 | 329 | C |
| PD7306 | 7 | 40 | 5 | 157 | 26 | 175 | 410 | C |
| PD7307 | 14 | 60 | 4 | 110 | 12 | 127 | 327 | C |
| PD7316 | 33 | 53 | 60 | 0 | 37 | 4 | 187 | D |

EP 3 452 937 B1

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD7321 | 18 | 37 | 344 | 0 | 50 | 3 | 452 | D |
| PD7322 | 2 | 17 | 0 | 30 | 1 | 20 | 70 | C |
| PD7341 | 5 | 67 | 3 | 71 | 11 | 87 | 244 | C |
| PD7344 | 4 | 13 | 0 | 3 | 12 | 5 | 37 | G |
| PD7426 | 86 | 85 | 224 | 2 | 75 | 13 | 485 | D |
| PD7428 | 11 | 41 | 292 | 0 | 39 | 3 | 386 | D |
| PD8609 | 1 | 19 | 2 | 21 | 5 | 12 | 60 | B |
| PD8610 | 4 | 35 | 1 | 9 | 56 | 36 | 141 | G |
| PD8611 | 52 | 52 | 233 | 0 | 74 | 5 | 416 | D |
| PD8612 | 23 | 9 | 0 | 1 | 1 | 9 | 43 | F |
| PD8614 | 1 | 6 | 0 | 5 | 0 | 5 | 17 | B |
| PD8615 | 5 | 20 | 0 | 25 | 1 | 25 | 76 | C |
| PD8617 | 1 | 34 | 0 | 2 | 4 | 17 | 58 | E |
| PD8618 | 0 | 6 | 0 | 0 | 1 | 0 | 7 | E |
| PD8619 | 20 | 34 | 4 | 69 | 12 | 109 | 248 | C |
| PD8620 | 0 | 1 | 0 | 0 | 1 | 0 | 2 | G |
| PD8621 | 19 | 18 | 179 | 0 | 51 | 4 | 271 | D |
| PD8622 | 0 | 21 | 0 | 4 | 5 | 19 | 49 | A |
| PD8623 | 0 | 24 | 0 | 0 | 5 | 1 | 30 | E |
| PD8652 | 35 | 42 | 195 | 0 | 107 | 8 | 387 | D |
| PD8660 | 92 | 173 | 19 | 129 | 138 | 39 | 590 | F |
| PD8828 | 0 | 10 | 0 | 0 | 1 | 0 | 11 | E |
| PD8830 | 13 | 18 | 1 | 25 | 47 | 24 | 128 | G |
| PD8832 | 11 | 141 | 67 | 22 | 105 | 31 | 377 | G |
| PD8964 | 9 | 64 | 3 | 3 | 150 | 18 | 247 | G |

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD8965 | 27 | 62 | 2 | 1 | 3 | 10 | 105 | E |
| PD8969 | 6 | 82 | 2 | 0 | 31 | 4 | 125 | E |
| PD8973 | 0 | 16 | 1 | 8 | 4 | 54 | 83 | A |
| PD8977 | 2 | 16 | 1 | 6 | 2 | 6 | 33 | E |
| PD8978 | 7 | 30 | 2 | 3 | 75 | 16 | 133 | G |
| PD8979 | 13 | 193 | 11 | 336 | 10 | 179 | 742 | B |
| PD8980 | 16 | 176 | 94 | 62 | 61 | 136 | 545 | D |
| PD8981 | 2 | 23 | 2 | 67 | 4 | 74 | 172 | C |
| PD8982 | 175 | 112 | 24 | 37 | 82 | 82 | 512 | F |
| PD8984 | 26 | 50 | 6 | 0 | 73 | 8 | 163 | G |
| PD8995 | 3 | 25 | 1 | 17 | 10 | 61 | 117 | A |
| PD8996 | 2 | 55 | 2 | 28 | 2 | 112 | 201 | A |
| PD8997 | 0 | 112 | 3 | 115 | 12 | 96 | 338 | B |
| PD8998 | 7 | 35 | 1 | 30 | 6 | 16 | 95 | B |
| PD8999 | 4 | 12 | 1 | 45 | 4 | 19 | 85 | B |
| PD9000 | 15 | 77 | 2 | 1 | 98 | 15 | 208 | G |
| PD9001 | 0 | 5 | 0 | 18 | 1 | 13 | 37 | C |
| PD9002 | 5 | 32 | 4 | 54 | 11 | 52 | 158 | C |
| PD9004 | 3 | 109 | 76 | 6 | 79 | 11 | 284 | G |
| PD9009 | 2 | 30 | 1 | 7 | 6 | 46 | 92 | A |
| PD9063 | 0 | 17 | 1 | 44 | 9 | 13 | 84 | B |
| PD9064 | 36 | 33 | 287 | 0 | 70 | 3 | 429 | D |
| PD9065 | 5 | 10 | 0 | 12 | 0 | 1 | 28 | B |
| PD9067 | 1 | 8 | 0 | 0 | 0 | 0 | 9 | E |
| PD9193 | 2 | 2 | 3 | 107 | 1 | 191 | 306 | C |

364

(continued)

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD9464 | 36 | 85 | 3 | 48 | 21 | 156 | 349 | A |
| PD9467 | 3 | 0 | 0 | 6 | 2 | 22 | 33 | A |
| PD9539 | 8 | 37 | 1 | 101 | 11 | 64 | 222 | C |
| PD9541 | 1 | 23 | 1 | 2 | 2 | 15 | 44 | E |
| PD9544 | 0 | 1 | 1 | 0 | 0 | 0 | 2 | D |
| PD9567 | 0 | 37 | 1 | 33 | 10 | 37 | 118 | C |
| PD9568 | 23 | 37 | 26 | 1 | 113 | 15 | 215 | G |
| PD9569 | 10 | 29 | 1 | 4 | 6 | 14 | 64 | E |
| PD9570 | 0 | 2 | 0 | 0 | 0 | 0 | 2 | E |
| PD9571 | 112 | 53 | 10 | 29 | 50 | 62 | 316 | F |
| PD9572 | 1 | 7 | 0 | 3 | 1 | 5 | 17 | E |
| PD9573 | 2 | 8 | 0 | 11 | 1 | 18 | 40 | C |
| PD9574 | 0 | 1 | 1 | 7 | 0 | 12 | 21 | C |
| PD9575 | 34 | 23 | 16 | 0 | 22 | 3 | 98 | F |
| PD9576 | 29 | 149 | 198 | 19 | 94 | 22 | 511 | D |
| PD9577 | 0 | 43 | 1 | 2 | 3 | 11 | 60 | E |
| PD9578 | 0 | 2 | 0 | 0 | 0 | 0 | 2 | E |
| PD9579 | 6 | 13 | 0 | 14 | 2 | 12 | 47 | C |
| PD9582 | 2 | 30 | 2 | 149 | 2 | 60 | 245 | B |
| PD9584 | 13 | 1 | 2 | 17 | 3 | 110 | 146 | A |
| PD9585 | 2 | 73 | 389 | 8 | 54 | 7 | 533 | D |
| PD9589 | 3 | 7 | 2 | 0 | 4 | 1 | 17 | G |
| PD9591 | 0 | 5 | 0 | 5 | 0 | 5 | 15 | B |
| PD9592 | 31 | 35 | 53 | 0 | 100 | 9 | 228 | G |
| PD9593 | 0 | 11 | 1 | 6 | 1 | 4 | 23 | B |

| Samples | Number of SV in samples | | | | | | Total | Cluster Group |
|---|---|---|---|---|---|---|---|---|
| | Rearrangement Signature1 | Rearrangement Signature2 | Rearrangement Signature3 | Rearrangement Signature4 | Rearrangement Signature5 | Rearrangement Signature6 | | |
| PD9595 | 14 | 37 | 101 | 11 | 46 | 14 | 223 | D |
| PD9597 | 0 | 2 | 0 | 6 | 1 | 4 | 13 | C |
| PD9599 | 14 | 54 | 2 | 23 | 10 | 28 | 131 | E |
| PD9600 | 15 | 19 | 0 | 0 | 3 | 1 | 38 | F |
| PD9604 | 40 | 21 | 0 | 4 | 40 | 14 | 119 | F |
| PD9605 | 11 | 16 | 0 | 4 | 9 | 21 | 61 | A |
| PD9694 | 2 | 8 | 1 | 15 | 4 | 21 | 51 | C |
| PD9696 | 42 | 35 | 201 | 0 | 51 | 3 | 332 | D |
| PD9702 | 189 | 38 | 196 | 0 | 45 | 1 | 469 | D |
| PD9752 | 7 | 87 | 3 | 51 | 15 | 131 | 294 | A |
| PD9754 | 4 | 4 | 1 | 10 | 4 | 23 | 46 | A |
| PD9755 | 0 | 0 | 1 | 20 | 2 | 36 | 59 | C |
| PD9756 | 6 | 2 | 0 | 22 | 4 | 18 | 52 | C |
| PD9759 | 4 | 22 | 0 | 9 | 8 | 11 | 54 | E |
| PD9760 | 4 | 43 | 1 | 15 | 8 | 70 | 141 | A |
| PD9761 | 1 | 1 | 0 | 0 | 0 | 0 | 2 | F |
| PD9842 | 0 | 0 | 0 | 0 | 1 | 0 | 1 | G |
| PD9845 | 0 | 7 | 0 | 9 | 2 | 11 | 29 | C |
| PD9847 | 2 | 1 | 1 | 0 | 0 | 0 | 4 | F |

**Table 11**

*REFERENCES*

1 Stratton, M. R., Campbell, P. J. & Futreal, P. A. The cancer genome. Nature 458, 719-724, doi:10.1038/nature07943 (2009).

2 Nik-Zainal, S. et al. Mutational processes molding the genomes of 21 breast cancers. Cell 149, 979-993, doi:10.1016/j.cell.2012.04.024 (2012).

3 Nik-Zainal, S. et al. The life history of 21 breast cancers. Cell 149, 994-1007, doi:10.1016/j.cell.2012.04.023 (2012).

4 Hicks, J. et al. Novel patterns of genome rearrangement and their association with survival in breast cancer. Genome research 16, 1465-1479, doi:10.1101/gr.5460106 (2006).

5 Bergamaschi, A. et al. Extracellular matrix signature identifies breast cancer subgroups with different clinical outcome. The Journal of pathology 214, 357-367, doi:10.1002/path.2278 (2008).

6 Ching, H. C., Naidu, R., Seong, M. K., Har, Y. C. & Taib, N. A. Integrated analysis of copy number and loss of heterozygosity in primary breast carcinomas using high-density SNP array. International journal of oncology 39, 621-633, doi:10.3892/ijo.2011.1081 (2011).

7 Fang, M. et al. Genomic differences between estrogen receptor (ER)-positive and ER-negative human breast carcinoma identified by single nucleotide polymorphism array comparative genome hybridization analysis. Cancer 117, 2024-2034, doi:10.1002/cncr.25770 (2011).

8 Curtis, C. et al. The genomic and transcriptomic architecture of 2,000 breast tumours reveals novel subgroups. Nature 486, 346-352, doi:10.1038/nature10983 (2012).

9 Pleasance, E. D. et al. A comprehensive catalogue of somatic mutations from a human cancer genome. Nature 463, 191-196, doi:10.1038/nature08658 (2010).

10 Pleasance, E. D. et al. A small-cell lung cancer genome with complex signatures of tobacco exposure. Nature 463, 184-190, doi:10.1038/nature08629 (2010).

11 Banerji, S. et al. Sequence analysis of mutations and translocations across breast cancer subtypes. Nature 486, 405-409, doi:10.1038/nature11154 (2012).

12 Ellis, M. J. et al. Whole-genome analysis informs breast cancer response to aromatase inhibition. Nature 486, 353-360, doi:10.1038/nature11143 (2012).

13 Shah, S. P. et al. The clonal and mutational evolution spectrum of primary triple-negative breast cancers. Nature 486, 395-399, doi:10.1038/nature10933 (2012).

14 Stephens, P. J. et al. The landscape of cancer genes and mutational processes in breast cancer. Nature 486, 400-404, doi:10.1038/nature11017 (2012).

15 Cancer Genome Atlas, N. Comprehensive molecular portraits of human breast tumours. Nature 490, 61-70, doi:10.1038/nature11412 (2012).

16 Wu, Y. M. et al. Identification of targetable FGFR gene fusions in diverse cancers. Cancer discovery 3, 636-647, doi:10.1158/2159-8290.CD-13-0050 (2013).

17 Giacomini, C. P. et al. Breakpoint analysis of transcriptional and genomic profiles uncovers novel gene fusions spanning multiple human cancer types. PLoS genetics 9, e1003464, doi:10.1371/journal.pgen.1003464 (2013).

18 Robinson, D. R. et al. Functionally recurrent rearrangements of the MAST kinase and Notch gene families in breast cancer. Nature medicine 17, 1646-1651, doi:10.1038/nm.2580 (2011).

19 Karlsson, J. et al. Activation of human telomerase reverse transcriptase through gene fusion in clear cell sarcoma of the kidney. Cancer letters 357, 498-501, doi:10.1016/j.canlet.2014.11.057 (2015).

20 Khurana, E. et al. Integrative annotation of variants from 1092 humans: application to cancer genomics. Science 342, 1235587, doi:10.1126/science.1235587 (2013).

21 West, J. A. et al. The long noncoding RNAs NEAT1 and MALAT1 bind active chromatin sites. Molecular cell 55, 791-802, doi:10.1016/j.molcel.2014.07.012 (2014).

22 Huang, F. W. et al. Highly recurrent TERT promoter mutations in human melanoma. Science 339, 957-959, doi:10.1126/science.1229259 (2013).

23 Vinagre, J. et al. Frequency of TERT promoter mutations in human cancers. Nature communications 4, 2185, doi:10.1038/ncomms3185 (2013).

24 Alexandrov, L. B. et al. Signatures of mutational processes in human cancer. Nature 500, 415-421, doi:10.1038/nature12477 (2013).

25 Alexandrov, L. B., Nik-Zainal, S., Wedge, D. C., Campbell, P. J. & Stratton, M. R. Deciphering signatures of mutational processes operative in human cancer. Cell reports 3, 246-259, doi:10.1016/j.celrep.2012.12.008 (2013).

26 Lawrence, M. S. et al. Discovery and saturation analysis of cancer genes across 21 tumour types. Nature 505, 495-501, doi:10.1038/nature12912 (2014).

27 Natrajan, R. et al. Characterization of the genomic features and expressed fusion genes in micropapillary carcinomas of the breast. The Journal of pathology 232, 553-565, doi:10.1002/path.4325 (2014).

28 Kalyana-Sundaram, S. et al. Gene fusions associated with recurrent amplicons represent a class of passenger aberrations in breast cancer. Neoplasia 14, 702-708 (2012).

29 Tubio, J. M. Somatic structural variation and cancer. Briefings in functional genomics, doi:10.1093/bfgp/elv016 (2015).

30 Weinhold, N., Jacobsen, A., Schultz, N., Sander, C. & Lee, W. Genome-wide analysis of noncoding regulatory mutations in cancer. Nature genetics 46, 1160-1165, doi:10.1038/ng.3101 (2014).

31 Ussery, D. W., Binnewies, T. T., Gouveia-Oliveira, R., Jarmer, H. & Hallin, P. F. Genome update: DNA repeats in bacterial genomes. Microbiol-Sgm 150, 3519-3521, doi:Doi 10.1099/Mic.0.27628-0 (2004).

32 Lu, S. et al. Short Inverted Repeats Are Hotspots for Genetic Instability: Relevance to Cancer Genomes. Cell reports, doi:10.1016/j.celrep.2015.02.039 (2015).

33 Voineagu, I., Narayanan, V., Lobachev, K. S. & Mirkin, S. M. Replication stalling at unstable inverted repeats: interplay between DNA hairpins and fork stabilizing proteins. Proceedings of the National Academy of Sciences of the United States of America 105, 9936-9941, doi:10.1073/pnas.0804510105 (2008).

34 Wojcik, E. A. et al. Direct and inverted repeats elicit genetic instability by both exploiting and eluding DNA double-strand break repair systems in mycobacteria. PloS one 7, e51064, doi:10.1371/journal.pone.0051064 (2012).

35 Pearson, C. E., Zorbas, H., Price, G. B. & Zannis-Hadjopoulos, M. Inverted repeats, stem-loops, and cruciforms: significance for initiation of DNA replication. Journal of cellular biochemistry 63, 1-22, doi:10.1002/(SICI)1097-4644(199610)63:1<1::AID-JCB1>3.0.CO;2-3 (1996).

36 Kozak, M. Interpreting cDNA sequences: some insights from studies on translation. Mammalian genome : official journal of the International Mammalian Genome Society 7, 563-574 (1996).

37 Helleday, T., Eshtad, S. & Nik-Zainal, S. Mechanisms underlying mutational signatures in human cancers. Nature reviews. Genetics 15, 585-598, doi:10.1038/nrg3729 (2014).

38 Birkbak, N. J. et al. Telomeric allelic imbalance indicates defective DNA repair and sensitivity to DNA-damaging agents. Cancer discovery 2, 366-375, doi:10.1158/2159-8290.CD-11-0206 (2012).

39 Abkevich, V. et al. Patterns of genomic loss of heterozygosity predict homologous recombination repair defects in epithelial ovarian cancer. British journal of cancer 107, 1776-1782, doi:10.1038/bjc.2012.451 (2012).

40 Popova, T. et al. Ploidy and large-scale genomic instability consistently identify basal-like breast carcinomas with BRCA1/2 inactivation. Cancer research 72, 5454-5462, doi:10.1158/0008-5472.CAN-12-1470 (2012).

41 Puente, X. S. et al. Whole-genome sequencing identifies recurrent mutations in chronic lymphocytic leukaemia. Nature 475, 101-105, doi:10.1038/nature10113 (2011).

42 Morganella, S. A., L.B.; . The topography of mutational processes in breast cancer. Submitted (2015).

43 Fong, P. C. et al. Inhibition of poly(ADP-ribose) polymerase in tumors from BRCA mutation carriers. The New England journal of medicine 361, 123-134, doi:10.1056/NEJMoa0900212 (2009).

44 Forster, M. D. et al. Treatment with olaparib in a patient with PTEN-deficient endometrioid endometrial cancer. Nature reviews. Clinical oncology 8, 302-306, doi:10.1038/nrclinonc.2011.42 (2011).

45 Turner, N., Tutt, A. & Ashworth, A. Targeting the DNA repair defect of BRCA tumours. Current opinion in pharmacology 5, 388-393, doi:10.1016/j.coph.2005.03.006 (2005).

46 Waddell, N. et al. Whole genomes redefine the mutational landscape of pancreatic cancer. Nature 518, 495-501, doi:10.1038/nature14169 (2015).

47 Kozarewa, I. et al. Amplification-free Illumina sequencing-library preparation facilitates improved mapping and assembly of (G+C)-biased genomes. Nature methods 6, 291-295, doi:10.1038/nmeth.1311 (2009).

48 Li, H. & Durbin, R. Fast and accurate short read alignment with Burrows-Wheeler transform. Bioinformatics 25, 1754-1760, doi:10.1093/bioinformatics/btp324 (2009).

49 Ye, K., Schulz, M. H., Long, Q., Apweiler, R. & Ning, Z. Pindel: a pattern growth approach to detect break points of large deletions and medium sized insertions from pairedend short reads. Bioinformatics 25, 2865-2871, doi:10.1093/bioinformatics/btp394 (2009).

50 Zerbino, D. R. & Birney, E. Velvet: algorithms for de novo short read assembly using de Bruijn graphs. Genome research 18, 821-829, doi:10.1101/gr.074492.107 (2008).

51 Van Loo, P. et al. Allele-specific copy number analysis of tumors. Proceedings of the National Academy of Sciences of the United States of America 107, 16910-16915, doi:10.1073/pnas.1009843107 (2010).

52 Greenman, C., Wooster, R., Futreal, P. A., Stratton, M. R. & Easton, D. F. Statistical analysis of pathogenicity of somatic mutations in cancer. Genetics 173, 2187-2198, doi:10.1534/genetics.105.044677 (2006).

53 Lawrence, M. S. et al. Mutational heterogeneity in cancer and the search for new cancer-associated genes. Nature 499, 214-218, doi:10.1038/nature12213 (2013).

54 Sun, L., Craiu, R. V., Paterson, A. D. & Bull, S. B. Stratified false discovery control for large-scale hypothesis testing with application to genome-wide association studies. Genetic epidemiology 30, 519-530, doi:10.1002/gepi.20164 (2006).

55 Consortium, E. P. An integrated encyclopedia of DNA elements in the human genome. Nature 489, 57-74, doi:10.1038/nature11247 (2012).

56 Wilkerson, M. D. & Hayes, D. N. ConsensusClusterPlus: a class discovery tool with confidence assessments and item tracking. Bioinformatics 26, 1572-1573, doi:btq170 [pii]10.1093/bioinformatics/btq170 (2010).

57 Zhang, H., Meltzer, P. & Davis, S. RCircos: an R package for Circos 2D track plots. BMC bioinformatics 14, 244, doi:10.1186/1471-2105-14-244 (2013).

58. Alexandrov, L. B. et al. A mutational signature in gastric cancer suggests therapeutic strategies. Nat. Commun. 6:8683 doi: 10.1038/ncomms9683 (2015).

59. Raine, K.M., Hinton, J., Butler, A.P., Teague, J.W., Davies, H.,Tarpey, P., Nik-Zainal, S. and Campbell, P.J. 2015. cgpPindel: Identifying somatically acquired insertion and deletion events from paired end sequencing. Curr. Protoc. Bioinform. 52:15.7.1-15.7.12. doi: 10.1002/0471250953.bi1507s52.

60. Ye, K., Schulz, M.H., Long, Q., Apweiler, R., and Ning, Z. 2009. Pindel: A pattern growth approach to detect break points of large deletions and medium sized insertions from pairedend short reads. Bioinformatics (Oxford, England) 25:2865-2871. doi: 10.1093/bioinformatics/btp394.

**Claims**

1. A computer-implemented method of determining a prognosis of a tumour or determining the suitability of a treatment for a tumour, the method including:

   characterising a DNA sample obtained from said tumour, by performing three or more of the following steps a) to e):

   a) determining a catalogue of base substitution signatures which are present in the sample;
   b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
   c) determining a catalogue of insertion/deletion signatures which are present in the sample
   d) determining the overall copy number profile in the sample
   e) identifying putative driver mutations present in the sample
   and

   based on said determinations and identifications, constructing an interpreted profile of the tumour,
   wherein the three or more steps a) to e) include at least steps a), b) and c); steps a), b) and d); steps a), b) and e); steps b), c) and d); steps b), d) and e); or steps b), c) and e); and
   determining the prognosis or suitability of the treatment from the interpreted profile.

2. A computer-implemented method of selecting a patient for a clinical trial of a cancer therapy, the method including:

   characterising a DNA sample obtained from a tumour in said patient, by performing three or more of the following steps a) to e):

   a) determining a catalogue of base substitution signatures which are present in the sample;
   b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
   c) determining a catalogue of insertion/deletion signatures which are present in the sample
   d) determining the overall copy number profile in the sample
   e) identifying putative driver mutations present in the sample
   and

   based on said determinations and identifications, constructing an interpreted profile of the tumour,
   wherein the three or more steps a) to e) include at least steps a), b) and c); steps a), b) and d); steps a), b) and e);

steps b), c) and d); steps b), d) and e); or steps b), c) and e); and
determining whether or not the patient is suitable for the clinical trial on the basis of the profile constructed for that patient.

3. A computer-implemented method of classifying a plurality of patients undergoing treatment for cancer, or participating in a clinical trial, the method including allocating patients to groups based on a profile constructed from a DNA sample obtained from a tumour in each patient by a method comprising the steps of:

characterising a DNA sample obtained from said tumour, by performing three or more of the following steps a) to e):

a) determining a catalogue of base substitution signatures which are present in the sample;
b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
c) determining a catalogue of insertion/deletion signatures which are present in the sample
d) determining the overall copy number profile in the sample
e) identifying putative driver mutations present in the sample
and

based on said determinations and identifications, constructing an interpreted profile of the tumour,
wherein the three or more steps a) to e) include at least steps a), b) and c); steps a), b) and d); steps a), b) and e); steps b), c) and d); steps b), d) and e); or steps b), c) and e).

4. A computer-implemented method of classifying patients who have completed a clinical trial or course of treatment, the method including:

characterising a DNA sample obtained from a tumour in each of said patient by performing three or more of the following steps a) to e):

a) determining a catalogue of base substitution signatures which are present in the sample;
b) determining a catalogue of rearrangement signatures which are present in the sample, using a rearrangement classification that classifies rearrangements: (i) between clustered and non-clustered, (ii) between deletions, inversions, translocations and tandem duplications, and (iii) by size of the deletions, inversions and tandem duplications;
c) determining a catalogue of insertion/deletion signatures which are present in the sample
d) determining the overall copy number profile in the sample
e) identifying putative driver mutations present in the sample
and

based on said determinations and identifications, constructing an interpreted profile of the tumour,
wherein the three or more steps a) to e) include at least steps a), b) and c); steps a), b) and d); steps a), b) and e); steps b), c) and d); steps b), d) and e); or steps b), c) and e); and correlating the interpreted profile obtained for each patient with the clinical outcome of the trial or treatment.

5. The method according to any one of the preceding claims, further including the steps of:
f) identifying putative recurrently mutated non-coding sites, and
using the identified putative recurrently mutated non-coding sites in constructing the interpreted profile, and optionally further including the step of:

g) obtaining a biologically useful over-arching summary of the sample, and
using the obtained summary in constructing the interpreted profile.

6. The method according to any one of the preceding claims, wherein the method comprises identifying whether the sample has a high or low likelihood of being homologous recombination (HR)-deficient by performing the steps of: determining the presence or absence of a plurality of base substitution signatures, rearrangement signatures and insertion/deletion (indel) signatures in the sample and copy number profiles for the sample; generating, from the presence or absence of said plurality of base substitution signatures, rearrangement signatures and indel signatures in the sample and the copy number profiles for the sample, a probabilistic score; and based on said probabilistic score, identifying whether said sample has a high or low likelihood of being homologous recombination (HR)-deficient.

7. The method according to any one of the preceding claims further including the steps of:

h) identifying the presence of mis-match repair (MMR) deficiency in the sample by using the presence or absence of base substitution signatures and indel signatures, and
using this identification in constructing the interpreted profile.

8. The method according to any one of the preceding claims further including the steps of:

i) identifying mutational characteristics in the sample that are informative of pathophysiological processes that are targetable including signatures relating to the immunological responses or to other DNA damage response processes, and
using the identified characteristics in constructing the interpreted profile.

9. The method according to any one of the preceding claims wherein the catalogue of base substitution signatures is obtained by:

cataloguing the somatic mutations in said sample to produce a mutational catalogue for that sample;
determining the contributions of known mutational signatures to said mutational catalogue by determining a scalar factor for each of a plurality of said known mutational signatures which together minimize a function representing the difference between the mutations in said catalogue and the mutations expected from a combination of said plurality of known mutational signatures scaled by said scalar factors; and
if the scalar factor corresponding to any one of said mutational signatures exceeds a predetermined threshold, including said mutation signature in the catalogue of base substitution signatures for the sample.

10. The method according to any one of the preceding claims wherein the catalogue of rearrangement signatures is obtained by:

cataloguing the somatic mutations in said sample to produce a rearrangement catalogue for that sample which classifies identified rearrangement mutations in the sample using said rearrangement classification;
determining the contributions of known rearrangement signatures to said rearrangement catalogue by computing the cosine similarity between the rearrangement mutations in said catalogue and the known rearrangement signatures; and
if the number or proportion of rearrangements in the rearrangement catalogue which are determined to be associated with one of said rearrangement signatures exceeds a predetermined threshold, including said rearrangement signature in the catalogue of rearrangement signatures for the sample.

11. The method according to any one of the preceding claims wherein the method includes all of the steps a) to e).

12. A computer program product containing non-transitory memory storing a computer program which, when run on a computer, performs the method of any one of the preceding claims.

13. A computer having a processor, wherein the processor is configured to perform the method of any one or more of claims 1 to 11.

**Patentansprüche**

1. Computerimplementiertes Verfahren zur Bestimmung der Prognose eines Tumors oder zur Bestimmung der Eignung einer Behandlung für einen Tumor, wobei das Verfahren Folgendes umfasst:

Charakterisieren einer DNA-Probe, die von dem Tumor erhalten wurde, durch Ausführen von drei oder mehr der folgenden Schritte a) bis e):

a) Bestimmen eines Katalogs von Basensubstitutionssignaturen, die in der Probe vorhanden sind;

b) Bestimmen eines Katalogs von Umordnungssignaturen, die in der Probe vorhanden sind, unter Verwendung einer Umordnungsklassifikation, die Umordnungen wie folgt klassifiziert: (i) nach geclustert und nicht geclustert, (ii) nach Deletionen, Inversionen, Translokationen und Tandemduplikationen und (iii) nach Größe der Deletionen, Inversionen und Tandemduplikationen;

c) Bestimmen eines Katalogs von Insertions-/Deletionssignaturen, die in der Probe vorhanden sind;

d) Bestimmen des Gesamtkopienzahlprofils in der Probe;

e) Identifizieren mutmaßlicher Treibermutationen in der Probe; und

basierend auf den Bestimmungen und Identifizierungen, Erstellen eines interpretierten Profils des Tumors, wobei die drei oder mehr Schritte a) bis e) zumindest die Schritte a), b) und c); die Schritte a), b) und d); die Schritte a), b) und e); die Schritte b), c) und d); die Schritte b), d) und e); oder die Schritte b), c) und e) umfassen; und Bestimmen der Prognose oder Eignung der Behandlung anhand des interpretierten Profils.

2. Computerimplementiertes Verfahren zur Auswahl eines Patienten für eine klinische Studie einer Krebstherapie, wobei das Verfahren Folgendes umfasst:

Charakterisieren einer DNA-Probe, die von einem Tumor in dem Patienten erhalten wurde, durch Ausführen von drei oder mehr der folgenden Schritte a) bis e):

a) Bestimmen eines Katalogs von Basensubstitutionssignaturen, die in der Probe vorhanden sind;

b) Bestimmen eines Katalogs von Umordnungssignaturen, die in der Probe vorhanden sind, unter Verwendung einer Umordnungsklassifikation, die Umordnungen wie folgt klassifiziert: (i) nach geclustert und nicht geclustert, (ii) nach Deletionen, Inversionen, Translokationen und Tandemduplikationen und (iii) nach Größe der Deletionen, Inversionen und Tandemduplikationen;

c) Bestimmen eines Katalogs von Insertions-/Deletionssignaturen, die in der Probe vorhanden sind;

d) Bestimmen des Gesamtkopienzahlprofils in der Probe;

e) Identifizieren mutmaßlicher Treibermutationen in der Probe; und

basierend auf den Bestimmungen und Identifizierungen, Erstellen eines interpretierten Profils des Tumors, wobei die drei oder mehr Schritte a) bis e) zumindest die Schritte a), b) und c); die Schritte a), b) und d); die Schritte a), b) und e); die Schritte b), c) und d); die Schritte b), d) und e); oder die Schritte b), c) und e) umfassen; und Bestimmen, ob der Patient für die klinische Studie geeignet ist oder nicht, basierend auf dem für den Patienten erstellen Profil.

3. Computerimplementiertes Verfahren zur Klassifizierung einer Vielzahl von Patienten, die eine Krebsbehandlung erhalten oder an einer klinischen Studie teilnehmen, wobei das Verfahren das Zuordnen der Patienten zu Gruppen basierend auf einem Profil, das anhand einer DNA-Probe erstellt wurde, die von einem Tumor in jedem Patienten erhalten wurde, durch ein Verfahren umfasst, das die folgenden Schritte umfasst:

Charakterisieren einer DNA-Probe, die von dem Tumor erhalten wurde, durch Ausführen von drei oder mehr der folgenden Schritte a) bis e):

a) Bestimmen eines Katalogs von Basensubstitutionssignaturen, die in der Probe vorhanden sind;

b) Bestimmen eines Katalogs von Umordnungssignaturen, die in der Probe vorhanden sind, unter Verwendung einer Umordnungsklassifikation, die Umordnungen wie folgt klassifiziert: (i) nach geclustert und nicht geclustert, (ii) nach Deletionen, Inversionen, Translokationen und Tandemduplikationen und (iii) nach Größe der Deletionen, Inversionen und Tandemduplikationen;

c) Bestimmen eines Katalogs von Insertions-/Deletionssignaturen, die in der Probe vorhanden sind;

d) Bestimmen des Gesamtkopienzahlprofils in der Probe;

e) Identifizieren mutmaßlicher Treibermutationen in der Probe; und

basierend auf den Bestimmungen und Identifizierungen, Erstellen eines interpretierten Profils des Tumors, wobei die drei oder mehr Schritte a) bis e) zumindest die Schritte a), b) und c); die Schritte a), b) und d); die Schritte a), b) und e); die Schritte b), c) und d); die Schritte b), d) und e); oder die Schritte b), c) und e) umfassen.

4. Computerimplementiertes Verfahren zur Klassifizierung von Patienten, die eine klinische Studie oder einen Behandlungsablauf abgeschlossen haben, wobei das Verfahren Folgendes umfasst:

Charakterisieren einer DNA-Probe, die von einem Tumor in jedem der Patienten erhalten wurde, durch Ausführen von drei oder mehr der folgenden Schritte a) bis e):

a) Bestimmen eines Katalogs von Basensubstitutionssignaturen, die in der Probe vorhanden sind;
b) Bestimmen eines Katalogs von Umordnungssignaturen, die in der Probe vorhanden sind, unter Verwendung einer Umordnungsklassifikation, die Umordnungen wie folgt klassifiziert: (i) nach geclustert und nicht geclustert, (ii) nach Deletionen, Inversionen, Translokationen und Tandemduplikationen und (iii) nach Größe der Deletionen, Inversionen und Tandemduplikationen;
c) Bestimmen eines Katalogs von Insertions-/Deletionssignaturen, die in der Probe vorhanden sind;
d) Bestimmen des Gesamtkopienzahlprofils in der Probe;
e) Identifizieren mutmaßlicher Treibermutationen in der Probe; und

basierend auf den Bestimmungen und Identifizierungen, Erstellen eines interpretierten Profils des Tumors, wobei die drei oder mehr Schritte a) bis e) zumindest die Schritte a), b) und c); die Schritte a), b) und d); die Schritte a), b) und e); die Schritte b), c) und d); die Schritte b), d) und e); oder die Schritte b), c) und e) umfassen; und Korrelieren des interpretierten Profils, das für jeden Patienten erhalten wurde, mit dem klinischen Ergebnis der Studie oder Behandlung.

5. Verfahren nach einem der vorangegangenen Ansprüche, das weiters folgende Schritte umfasst:
f) Identifizieren mutmaßlich wiederholt mutierter nichtkodierender Stellen und
Verwenden der identifizierten mutmaßlich wiederholt mutierten nichtkodierenden Stellen beim Erstellen eines konstruierten Profils, und gegebenenfalls weiters folgenden Schritt umfasst:

g) Erhalten einer biologisch nützlichen übergreifenden Zusammenfassung der Probe und
Verwenden der erhaltenen Zusammenfassung beim Erstellen des interpretierten Profils.

6. Verfahren nach einem der vorangegangenen Ansprüche, wobei das Verfahren das Identifizieren umfasst, ob die Probe eine hohe oder geringe Wahrscheinlichkeit aufweist, defizient bezüglich homologer Rekombination (HR) zu sein, indem die folgenden Schritte ausgeführt werden: Bestimmen der Gegenwart oder Abwesenheit einer Vielzahl von Basensubstitutionssignaturen, Umordnungssignaturen und Insertions-/Deletions- (Indel-) Signaturen in der Probe und eines Kopienzahlprofils für die Probe; Erstellen, anhand der Gegenwart oder Abwesenheit der Vielzahl von Basensubstitutionssignaturen, von Umordnungssignaturen und Indel-Signaturen in der Probe und von Kopienzahlprofilen für die Probe, eines Wahrscheinlichkeitswerts; und basierend auf dem Wahrscheinlichkeitswert, Identifizieren, ob die Probe eine hohe oder geringe Wahrscheinlichkeit aufweist, defizient bezüglich homologer Rekombination (HR) zu sein.

7. Verfahren nach einem der vorangegangenen Ansprüche, das weiters folgende Schritte umfasst:

h) Identifizieren der Gegenwart einer Fehlpaarungsreparatur- (MMR-) Defizienz in der Probe anhand der Gegenwart oder Abwesenheit von Basensubstitutionssignaturen und Indel-Signaturen und
Verwenden dieser Identifizierung beim Erstellen des interpretierten Profils.

8. Verfahren nach einem der vorangegangenen Ansprüche, das weiters folgenden Schritt umfasst:

i) Identifizieren von Mutationseigenschaften in der Probe, die Informationen über pathophysiologische Prozesse liefern, die als Ziel dienen können, einschließlich Signaturen in Verbindung mit immunologischen Antworten oder mit anderen Antwortprozessen auf DNA-Schädigungen, und
Verwenden der identifizierten Eigenschaften beim Erstellen des interpretierten Profils.

9. Verfahren nach einem der vorangegangenen Ansprüche, wobei der Katalog von Basensubstitutionssignaturen durch Folgendes erhalten wird:

Katalogisieren der somatischen Mutationen in der Probe, um einen Mutationskatalog für diese Probe zu erstellen;
Bestimmen der Beiträge bekannter Mutationssignaturen zu dem Mutationskatalog durch Bestimmen eines Skalarfaktors für jede aus einer Vielzahl der bekannten Mutationssignaturen, die zusammen eine Funktion minimieren, die den Unterschied zwischen den Mutationen im Katalog und den Mutationen darstellt, die aus einer Kombination der Vielzahl von bekannten Mutationssignaturen skaliert durch die Skalarfaktoren erwartet werden;

und

wenn der Skalarfaktor, der einer beliebigen der Mutationssignaturen entspricht, über einer vorbestimmten Schwelle liegt, Aufnehmen der Mutationssignatur in den Katalog von Basensubstitutionssignaturen für die Probe.

**10.** Verfahren nach einem der vorangegangenen Ansprüche, wobei der Katalog von Umordnungssignaturen durch Folgendes erhalten wird:

Katalogisieren der somatischen Mutationen in der Probe, um einen Umordnungskatalog für die Probe zu erstellen, der identifizierte Umordnungsmutationen in der Probe anhand der Umordnungsklassifikation klassifiziert;

Bestimmen der Beiträge bekannter Umordnungssignaturen zum Umordnungskatalog durch Berechnen der Kosinus-Ähnlichkeit zwischen den Umordnungsmutationen im Katalog und den bekannten Umordnungssignaturen; und

wenn die Anzahl oder der Anteil an Umordnungen im Umordnungskatalog, von denen bestimmt wird, dass sie mit einer der Umordnungssignaturen assoziiert sind, über einer vorbestimmten Schwelle liegt, Aufnehmen der Umordnungssignatur in den Katalog von Umordnungssignaturen für die Probe.

**11.** Verfahren nach einem der vorangegangenen Ansprüche, wobei das Verfahren alle Schritte a) bis e) umfasst.

**12.** Computerprogrammprodukt, das einen nichttransitorischen Speicher enthält, in dem ein Computerprogramm gespeichert ist, das ein Verfahren nach einem der vorangegangenen Ansprüche ausführt, wenn es auf einem Computer laufen gelassen wird.

**13.** Computer mit einem Prozessor, wobei der Prozessor ausgelegt ist, ein Verfahren nach einem der Ansprüche 1 bis 11 auszuführen.

## Revendications

**1.** Procédé mis en œuvre par ordinateur de détermination d'un pronostic d'une tumeur ou de détermination de la pertinence d'un traitement pour une tumeur, le procédé comprenant :

la caractérisation d'un échantillon d'ADN obtenu à partir de ladite tumeur, en réalisant au moins trois des étapes a) à e) suivantes :

a) détermination d'un catalogue de signatures de substitutions de bases qui sont présentes dans l'échantillon ;
b) détermination d'un catalogue de signatures de réarrangements qui sont présentes dans l'échantillon à l'aide d'une classification de réarrangements qui classifie les réarrangements : (i) entre regroupés et non regroupés, (ii) entre délétions, inversions, translocations et duplications en tandem, et (iii) par taille des délétions, inversions et duplications en tandem ;
c) détermination d'un catalogue de signatures d'insertions/délétions qui sont présentes dans l'échantillon
d) détermination du profil du nombre de copies global dans l'échantillon
e) identification des mutations conductrices putatives présentes dans l'échantillon
et

sur la base desdites déterminations et identifications, la construction d'un profil interprété de la tumeur,
dans lequel les au moins trois étapes a) à e) comprennent au moins les étapes a), b) et c) ; les étapes a), b) et d) ;
les étapes a), b) et e) ; les étapes b), c) et d) ; les étapes b), d) et e) ; ou les étapes b), c) et e) ; et
la détermination du pronostic ou de la pertinence du traitement à partir du profil interprété.

**2.** Procédé mis en œuvre par ordinateur de sélection d'un patient pour un essai clinique d'une thérapie contre le cancer, le procédé comprenant :

la caractérisation d'un échantillon d'ADN obtenu à partir d'une tumeur dudit patient, en réalisant au moins trois des étapes a) à e) suivantes :

a) détermination d'un catalogue de signatures de substitutions de bases qui sont présentes dans l'échantillon ;

b) détermination d'un catalogue de signatures de réarrangements qui sont présentes dans l'échantillon à l'aide d'une classification de réarrangements qui classifie les réarrangements : (i) entre regroupés et non regroupés, (ii) entre délétions, inversions, translocations et duplications en tandem, et (iii) par taille des délétions, inversions et duplications en tandem ;

c) détermination d'un catalogue de signatures d'insertions/délétions qui sont présentes dans l'échantillon

d) détermination du profil du nombre de copies global dans l'échantillon

e) identification des mutations conductrices putatives présentes dans l'échantillon

et

sur la base desdites déterminations et identifications, la construction d'un profil interprété de la tumeur,

dans lequel les au moins trois étapes a) à e) comprennent au moins les étapes a), b) et c) ; les étapes a), b) et d) ; les étapes a), b) et e) ; les étapes b), c) et d) ; les étapes b), d) et e) ; ou les étapes b), c) et e) ; et

la détermination si le patient est approprié ou non pour l'essai clinique sur la base du profil construit pour ce patient.

**3.** Procédé mis en œuvre par ordinateur de classification d'une pluralité de patients soumis à un traitement pour le cancer, ou participant à un essai clinique, le procédé comprenant la répartition des patients en groupes sur la base d'un profil construit à partir d'un échantillon d'ADN obtenu à partir d'une tumeur chez chaque patient par un procédé comprenant les étapes consistant à :

caractériser un échantillon d'ADN obtenu à partir de ladite tumeur, en réalisant au moins trois des étapes a) à e) suivantes :

a) détermination d'un catalogue de signatures de substitutions de bases qui sont présentes dans l'échantillon ;

b) détermination d'un catalogue de signatures de réarrangements qui sont présentes dans l'échantillon à l'aide d'une classification de réarrangements qui classifie les réarrangements : (i) entre regroupés et non regroupés, (ii) entre délétions, inversions, translocations et duplications en tandem, et (iii) par taille des délétions, inversions et duplications en tandem ;

c) détermination d'un catalogue de signatures d'insertions/délétions qui sont présentes dans l'échantillon

d) détermination du profil du nombre de copies global dans l'échantillon

e) identification des mutations conductrices putatives présentes dans l'échantillon

et

sur la base desdites déterminations et identifications, construire un profil interprété de la tumeur,

dans lequel les au moins trois étapes a) à e) comprennent au moins les étapes a), b) et c) ; les étapes a), b) et d) ; les étapes a), b) et e) ; les étapes b), c) et d) ; les étapes b), d) et e) ; ou les étapes b), c) et e).

**4.** Procédé mis en œuvre par ordinateur de classification de patients ayant terminé un essai clinique ou un traitement, le procédé comprenant :

la caractérisation d'un échantillon d'ADN obtenu à partir d'une tumeur dudit patient, en réalisant au moins trois des étapes a) à e) suivantes :

a) détermination d'un catalogue de signatures de substitutions de bases qui sont présentes dans l'échantillon ;

b) détermination d'un catalogue de signatures de réarrangements qui sont présentes dans l'échantillon à l'aide d'une classification de réarrangements qui classifie les réarrangements : (i) entre regroupés et non regroupés, (ii) entre délétions, inversions, translocations et duplications en tandem, et (iii) par taille des délétions, inversions et duplications en tandem ;

c) détermination d'un catalogue de signatures d'insertions/délétions qui sont présentes dans l'échantillon

d) détermination du profil du nombre de copies global dans l'échantillon

e) identification des mutations conductrices putatives présentes dans l'échantillon

et

sur la base desdites déterminations et identifications, la construction d'un profil interprété de la tumeur,

dans lequel les au moins trois étapes a) à e) comprennent au moins les étapes a), b) et c) ; les étapes a), b) et d) ; les étapes a), b) et e) ; les étapes b), c) et d) ; les étapes b), d) et e) ; ou les étapes b), c) et e) ; et la corrélation du profil interprété obtenu pour chaque patient avec les résultats cliniques de l'essai ou du traitement.

5. Procédé selon l'une quelconque des revendications précédentes, comprenant en outre les étapes consistant à :
f) identifier les sites non codants putatifs mutés de manière récurrente, et
utiliser les sites non codants putatifs mutés de manière récurrente identifiés pour construire le profil interprété, et éventuellement comprenant en outre l'étape consistant à :
g) obtenir un résumé primordial biologiquement utile de l'échantillon, et utiliser le résumé obtenu pour construire le profil interprété.

6. Procédé selon l'une quelconque des revendications précédentes, dans lequel le procédé comprend l'identification si l'échantillon a une probabilité faible ou élevée d'être déficient en recombinaison homologue (HR) en effectuant les étapes consistant à : déterminer la présence ou l'absence d'une pluralité de signatures de substitutions de bases, de signatures de réarrangements et de signatures d'insertions/délétions (indel) dans l'échantillon et des profils du nombre de copies pour l'échantillon ; générer, à partir de la présence ou de l'absence de ladite pluralité de signatures de substitutions de bases, de signatures de réarrangements et de signatures d'indel dans l'échantillon et des profils du nombre de copies pour l'échantillon, un score probabiliste ; et sur la base dudit score probabiliste, identifier si ledit échantillon a une probabilité faible ou élevée d'être déficient en recombinaison homologue (HR).

7. Procédé selon l'une quelconque des revendications précédentes comprenant en outre les étapes consistant à :

h) identifier la présence d'un déficit de réparation des mésappariements (MMR) dans l'échantillon en utilisant la présence ou l'absence de signatures de substitutions de bases et de signatures d'indel, et
utiliser cette identification pour construire le profil interprété.

8. Procédé selon l'une quelconque des revendications précédentes comprenant en outre les étapes consistant à :

i) identifier les caractéristiques mutationnelles dans l'échantillon qui renseignent sur les processus physiopathologiques qui sont ciblables, y compris les signatures relatives aux réponses immunologiques ou à d'autres processus de réponse aux dommages de l'ADN, et
utiliser les caractéristiques identifiées pour construire le profil interprété.

9. Procédé selon l'une quelconque des revendications précédentes dans lequel le catalogue de signatures de substitutions de bases est obtenu par :

catalogage des mutations somatiques dans ledit échantillon pour produire un catalogue mutationnel pour cet échantillon ;
détermination des contributions des signatures mutationnelles connues audit catalogue mutationnel en déterminant un facteur scalaire pour chacune d'une pluralité desdites signatures mutationnelles connues qui minimisent ensemble une fonction représentant la différence entre les mutations dans ledit catalogue et les mutations attendues d'une combinaison de ladite pluralité de signatures mutationnelles connues mises à l'échelle par lesdits facteurs scalaires ; et
si le facteur scalaire correspondant à l'une quelconque desdites signatures mutationnelles dépasse un seuil prédéterminé, inclusion de ladite signature mutationnelle dans le catalogue de signatures de substitutions de bases pour l'échantillon.

10. Procédé selon l'une quelconque des revendications précédentes dans lequel le catalogue de signatures de réarrangements est obtenu par :

catalogage des mutations somatiques dans ledit échantillon pour produire un catalogue de réarrangements pour cet échantillon qui classe les mutations de réarrangements identifiées dans l'échantillon en utilisant ladite classification de réarrangements ;
détermination des contributions des signatures de réarrangements connues audit catalogue de réarrangements en calculant la similarité cosinus entre les mutations de réarrangements dans ledit catalogue et les signatures de réarrangements connues ; et
si le nombre ou la proportion de réarrangements dans le catalogue de réarrangements qui sont déterminés comme étant associés à l'une desdites signatures de réarrangements dépasse un seuil défini, inclusion de ladite signature de réarrangements dans le catalogue de signatures de réarrangements pour l'échantillon.

11. Procédé selon l'une quelconque des revendications précédentes dans lequel le procédé comprend toutes les étapes a) à e).

12. Produit programme informatique contenant une mémoire non transitoire stockant un programme informatique qui, lorsqu'il est exécuté sur un ordinateur, exécute le procédé selon l'une quelconque des revendications précédentes.

13. Ordinateur équipé d'un processeur, dans lequel le processeur est configuré pour exécuter le procédé selon l'une quelconque ou plusieurs des revendications 1 à 11.

Figure 1

Figure 2

Figure 3

Figure 4

Figure 5

Figure 6

Figure 7

Figure 8

Figure 9

A

| chr | start | end | length | number of breakpoints | number of samples | known fragile site | nearby cancer gene |
|---|---|---|---|---|---|---|---|
| 1 | 735890 | 1491917 | 756027 | 25 | 13 | FRA1A | |
| 1 | 66670535 | 67015462 | 344927 | 52 | 21 | | |
| 12 | 11843027 | 12728445 | 885418 | 91 | 35 | | ETV6 |
| 8 | 128355027 | 129238954 | 883927 | 114 | 58 | | MYC |
| 5 | 44222786 | 44992104 | 769318 | 49 | 22 | | |
| 5 | 106572301 | 107085560 | 513259 | 36 | 18 | FRA5F | |
| 7 | 92014640 | 92358020 | 343380 | 31 | 13 | FRA7E | CDK6 |
| 11 | 33671711 | 34956790 | 1285079 | 66 | 31 | FRA11E FRA11E-narrow | LMO2 |
| 11 | 64715221 | 65348449 | 633228 | 64 | 31 | FRA11H | |

B

# Figure 10

Figure 11

Figure 12

**A**

**B**

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013096843 A
- EP 2017060279 W

- EP 2017060294 W


### Non-patent literature cited in the description

- **ALEXANDROV et al.** *Cell Reports*, 01 January 2013, vol. 3 (1), 246-259
- **STRATTON, M. R. ; CAMPBELL, P. J. ; FUTREAL, P. A.** The cancer genome. *Nature*, 2009, vol. 458, 719-724
- **NIK-ZAINAL, S. et al.** Mutational processes molding the genomes of 21 breast cancers. *Cell*, 2012, vol. 149, 979-993
- **NIK-ZAINAL, S. et al.** The life history of 21 breast cancers. *Cell*, 2012, vol. 149, 994-1007
- **HICKS, J. et al.** Novel patterns of genome rearrangement and their association with survival in breast cancer. *Genome research*, 2006, vol. 16, 1465-1479
- **BERGAMASCHI, A. et al.** Extracellular matrix signature identifies breast cancer subgroups with different clinical outcome. *The Journal of pathology*, 2008, vol. 214, 357-367
- **CHING, H. C. ; NAIDU, R. ; SEONG, M. K. ; HAR, Y. C. ; TAIB, N. A.** Integrated analysis of copy number and loss of heterozygosity in primary breast carcinomas using high-density SNP array. *International journal of oncology*, 2011, vol. 39, 621-633
- **FANG, M. et al.** Genomic differences between estrogen receptor (ER)-positive and ER-negative human breast carcinoma identified by single nucleotide polymorphism array comparative genome hybridization analysis. *Cancer*, 2011, vol. 117, 2024-2034
- **CURTIS, C. et al.** The genomic and transcriptomic architecture of 2,000 breast tumours reveals novel subgroups. *Nature*, 2012, vol. 486, 346-352
- **PLEASANCE, E. D. et al.** A comprehensive catalogue of somatic mutations from a human cancer genome. *Nature*, 2010, vol. 463, 191-196
- **PLEASANCE, E. D. et al.** A small-cell lung cancer genome with complex signatures of tobacco exposure. *Nature*, 2010, vol. 463, 184-190
- **BANERJI, S. et al.** Sequence analysis of mutations and translocations across breast cancer subtypes. *Nature*, 2012, vol. 486, 405-409

- **ELLIS, M. J. et al.** Whole-genome analysis informs breast cancer response to aromatase inhibition. *Nature*, 2012, vol. 486, 353-360
- **SHAH, S. P. et al.** The clonal and mutational evolution spectrum of primary triple-negative breast cancers. *Nature*, 2012, vol. 486, 395-399
- **STEPHENS, P. J. et al.** The landscape of cancer genes and mutational processes in breast cancer. *Nature*, 2012, vol. 486, 400-404
- Cancer Genome Atlas, N. Comprehensive molecular portraits of human breast tumours. *Nature*, 2012, vol. 490, 61-70
- **WU, Y. M. et al.** Identification of targetable FGFR gene fusions in diverse cancers. *Cancer discovery*, 2013, vol. 3, 636-647
- **GIACOMINI, C. P. et al.** Breakpoint analysis of transcriptional and genomic profiles uncovers novel gene fusions spanning multiple human cancer types. *PLoS genetics*, 2013, vol. 9, e1003464
- **ROBINSON, D. R. et al.** Functionally recurrent rearrangements of the MAST kinase and Notch gene families in breast cancer. *Nature medicine*, 2011, vol. 17, 1646-1651
- **KARLSSON, J. et al.** Activation of human telomerase reverse transcriptase through gene fusion in clear cell sarcoma of the kidney. *Cancer letters*, 2015, vol. 357, 498-501
- **KHURANA, E. et al.** Integrative annotation of variants from 1092 humans: application to cancer genomics. *Science*, 2013, vol. 342, 1235587
- **WEST, J. A. et al.** The long noncoding RNAs NEAT1 and MALAT1 bind active chromatin sites. *Molecular cell*, 2014, vol. 55, 791-802
- **HUANG, F. W. et al.** Highly recurrent TERT promoter mutations in human melanoma. *Science*, 2013, vol. 339, 957-959
- **VINAGRE, J. et al.** Frequency of TERT promoter mutations in human cancers. *Nature*, 2013
- **ALEXANDROV, L. B. et al.** Signatures of mutational processes in human cancer. *Nature*, 2013, vol. 500, 415-421

- **ALEXANDROV, L. B.** ; **NIK-ZAINAL, S.** ; **WEDGE, D. C.** ; **CAMPBELL, P. J.** ; **STRATTON, M. R.** Deciphering signatures of mutational processes operative in human cancer. *Cell reports*, 2013, vol. 3, 246-259
- **LAWRENCE, M. S. et al.** Discovery and saturation analysis of cancer genes across 21 tumour types. *Nature*, 2014, vol. 505, 495-501
- **NATRAJAN, R. et al.** Characterization of the genomic features and expressed fusion genes in micropapillary carcinomas of the breast. *The Journal of pathology*, 2014, vol. 232, 553-565
- **KALYANA-SUNDARAM, S. et al.** Gene fusions associated with recurrent amplicons represent a class of passenger aberrations in breast cancer. *Neoplasia*, 2012, vol. 14, 702-708
- **TUBIO, J.** M. Somatic structural variation and cancer. *Briefings in functional genomics*, 2015
- **WEINHOLD, N.** ; **JACOBSEN, A.** ; **SCHULTZ, N.** ; **SANDER, C.** ; **LEE, W.** Genome-wide analysis of noncoding regulatory mutations in cancer. *Nature genetics*, 2014, vol. 46, 1160-1165
- **USSERY, D. W.** ; **BINNEWIES, T. T.** ; **GOUVEIA-OLIVEIRA, R.** ; **JARMER, H.** ; **HALLIN, P. F.** Genome update: DNA repeats in bacterial genomes. *Microbiol-Sgm*, 2004, vol. 150, 3519-3521
- **LU, S. et al.** Short Inverted Repeats Are Hotspots for Genetic Instability: Relevance to Cancer Genomes. *Cell reports*, 2015
- **VOINEAGU, I.** ; **NARAYANAN, V.** ; **LOBACHEV, K. S.** ; **MIRKIN, S. M.** Replication stalling at unstable inverted repeats: interplay between DNA hairpins and fork stabilizing proteins. *Proceedings of the National Academy of Sciences of the United States of America*, 2008, vol. 105, 9936-9941
- **WOJCIK, E. A. et al.** Direct and inverted repeats elicit genetic instability by both exploiting and eluding DNA double-strand break repair systems in mycobacteria. *PloS one*, 2012, vol. 7, e51064
- **PEARSON, C. E.** ; **ZORBAS, H.** ; **PRICE, G. B.** ; **ZANNIS-HADJOPOULOS, M.** Inverted repeats, stem-loops, and cruciforms: significance for initiation of DNA replication. *Journal of cellular biochemistry*, 1996, vol. 63, 1-22
- **KOZAK, M.** Interpreting cDNA sequences: some insights from studies on translation. *Mammalian genome : official journal of the International Mammalian Genome Society*, 1996, vol. 7, 563-574
- **HELLEDAY, T.** ; **ESHTAD, S.** ; **NIK-ZAINAL, S.** Mechanisms underlying mutational signatures in human cancers. *Nature reviews. Genetics*, 2014, vol. 15, 585-598
- **BIRKBAK, N. J. et al.** Telomeric allelic imbalance indicates defective DNA repair and sensitivity to DNA-damaging agents. *Cancer discovery*, 2012, vol. 2, 366-375
- **ABKEVICH, V. et al.** Patterns of genomic loss of heterozygosity predict homologous recombination repair defects in epithelial ovarian cancer. *British journal of cancer*, 2012, vol. 107, 1776-1782
- **POPOVA, T. et al.** Ploidy and large-scale genomic instability consistently identify basal-like breast carcinomas with BRCA1/2 inactivation. *Cancer research*, 2012, vol. 72, 5454-5462
- **PUENTE, X. S. et al.** Whole-genome sequencing identifies recurrent mutations in chronic lymphocytic leukaemia. *Nature*, 2011, vol. 475, 101-105
- **MORGANELLA, S. A.** ; **L.B.** The topography of mutational processes in breast cancer. *Submitted*, 2015
- **FONG, P. C. et al.** Inhibition of poly(ADP-ribose) polymerase in tumors from BRCA mutation carriers. *The New England journal of medicine*, 2009, vol. 361, 123-134
- **FORSTER, M. D. et al.** Treatment with olaparib in a patient with PTEN-deficient endometrioid endometrial cancer. *Nature reviews. Clinical oncology*, 2011, vol. 8, 302-306
- **TURNER, N.** ; **TUTT, A.** ; **ASHWORTH, A.** Targeting the DNA repair defect of BRCA tumours. *Current opinion in pharmacology*, 2005, vol. 5, 388-393
- **WADDELL, N. et al.** Whole genomes redefine the mutational landscape of pancreatic cancer. *Nature*, 2015, vol. 518, 495-501
- **KOZAREWA, I. et al.** Amplification-free Illumina sequencing-library preparation facilitates improved mapping and assembly of (G+C)-biased genomes. *Nature methods*, 2009, vol. 6, 291-295
- **LI, H.** ; **DURBIN, R.** Fast and accurate short read alignment with Burrows-Wheeler transform. *Bioinformatics*, 2009, vol. 25, 1754-1760
- **YE, K.** ; **SCHULZ, M. H.** ; **LONG, Q.** ; **APWEILER, R.** ; **NING, Z.** Pindel: a pattern growth approach to detect break points of large deletions and medium sized insertions from pairedend short reads.. *Bioinformatics*, 2009, vol. 25, 2865-2871
- **ZERBINO, D. R.** ; **BIRNEY, E.** Velvet: algorithms for de novo short read assembly using de Bruijn graphs. *Genome research*, 2008, vol. 18, 821-829
- **VAN LOO, P. et al.** Allele-specific copy number analysis of tumors. *Proceedings of the National Academy of Sciences of the United States of America*, 2010, vol. 107, 16910-16915
- **GREENMAN, C.** ; **WOOSTER, R.** ; **FUTREAL, P. A.** ; **STRATTON, M. R.** ; **EASTON, D. F.** Statistical analysis of pathogenicity of somatic mutations in cancer. *Genetics*, 2006, vol. 173, 2187-2198
- **LAWRENCE, M. S. et al.** Mutational heterogeneity in cancer and the search for new cancer-associated genes. *Nature*, 2013, vol. 499, 214-218

- **SUN, L.** ; **CRAIU, R. V.** ; **PATERSON, A. D.** ; **BULL, S. B.** Stratified false discovery control for large-scale hypothesis testing with application to genome-wide association studies. *Genetic epidemiology*, 2006, vol. 30, 519-530
- **CONSORTIUM, E. P.** An integrated encyclopedia of DNA elements in the human genome. *Nature*, 2012, vol. 489, 57-74
- **WILKERSON, M. D.** ; **HAYES, D. N.** ConsensusClusterPlus: a class discovery tool with confidence assessments and item tracking. *Bioinformatics*, 2010, vol. 26, 1572-1573
- **ZHANG, H.** ; **MELTZER, P.** ; **DAVIS, S.** RCircos: an R package for Circos 2D track plots. *BMC bioinformatics*, 2013, vol. 14, 244

- **ALEXANDROV, L. B. et al.** A mutational signature in gastric cancer suggests therapeutic strategies. *Nat. Commun.*, 2015
- **RAINE, K.M.** ; **HINTON, J.** ; **BUTLER, A.P.** ; **TEAGUE, J.W.** ; **DAVIES, H.** ; **TARPEY, P.** ; **NIK-ZAINAL, S.** ; **CAMPBELL, P.J.** cgpPindel: Identifying somatically acquired insertion and deletion events from paired end sequencing. *Curr. Protoc. Bioinform.*, 2015, vol. 52, 15.7.1-15.7.12
- **YE, K.** ; **SCHULZ, M.H.** ; **LONG, Q.** ; **APWEILER, R.** ; **NING, Z.** Pindel: A pattern growth approach to detect break points of large deletions and medium sized insertions from pairedend short reads. *Bioinformatics (Oxford, England)*, 2009, vol. 25, 2865-2871